# EUROPEAN PATENT SPECIFICATION

(11) **EP 2 981 608 B1**
(45) Date of publication and mention of the grant of the patent: **11.07.2018**
(21) Application number: 14716272.1
(22) Date of filing: 03.04.2014
(51) Int. Cl.: C12N 7/00, A01N 63/00

(54) **NOVEL GENOTYPES OF CHRYSODEIXIS CHALCITES SINGLE NUCLEOPOLYHEDROVIRUS (CHCHSNPV), A PROCEDURE FOR ITS PRODUCTION AND USE AS A BIOLOGICAL CONTROL AGENT**
NEUE GENOTYPEN DES CHRYSODEIXIS CHALCITES NUCLEOPOLYHEDROVIRUS (CHCHSNPV), HERSTELLUNGSVERFAHREN UND VERWENDUNG ALS MITTEL ZUR BIOLOGISCHEN SCHÄDLINGSBEKÄMPFUNG
GÉNOTYPES NOUVEAUX DU CHRYSODEIXIS CHALCITES NUCLEOPOLYHEDROVIRUS,PROCÉDURE DE PRODUCTION ET UTILISATION COMME AGENT DE LUTTE BIOLOGIQUE

(30) Priority: 04.04.2013 ES 201330487
(43) Date of publication of application: 10.02.2016
(73) Proprietor: Universidad Pública De Navarra, 31006 Pamplona, Navarra (ES); Consejo Superior De Investigaciones Científicas (CSIC), 28006 Madrid (ES); Instituto Canario de Investigaciones Agrarias (ICIA), 38270 San Cristóbal de la Laguna (Tenerife) (ES); Instituto De Ecología, A.C., 91070 Xalapa, Veracruz (MX)
(72) Inventor: CABALLERO MURILLO, Primitivo, E-31192 Mutilva (Navarra) (ES); BERNAL RODRÍGUEZ, Alexandra, E-31192 Mutilva (Navarra) (ES); SIMÓN DE GOÑI, Oihane, E-31192 Mutilva (Navarra) (ES); CARNERO HERNÁNDEZ, Aurelio, E-38270 San Cristóbal de la Laguna (Tenerife) (ES); HERNÁNDEZ SUÁREZ, Estrella, E-38270 San Cristóbal de la Laguna (Tenerife) (ES); WILLIAMS, Trevor, 91070 Xalapa, Veracruz (MX)
(74) Representative: Elzaburu S.L.P.
(86) International application number: PCT/EP2014/056762
(87) International publication number: WO 2014/161974

(56) References cited:
- WO-A2-2007/122278
- M. M. VAN OERS: "Genome sequence of Chrysodeixis chalcites nucleopolyhedrovirus, a baculovirus with two DNA photolyase genes", JOURNAL OF GENERAL VIROLOGY, vol. 86, no. 7, 1 July 2005 (2005-07-01), pages 2069-2080, XP055124460, ISSN: 0022-1317, DOI: 10.1099/vir.0.80964-0 cited in the application
- Tomás Cabello García ET AL: "Caracterización bioquímica de un nucleopoliedrovirus de "Chrysodeixis chalcites" autóctono de España", Boletín de sanidad vegetal. Plagas, 1 January 2000 (2000-01-01), pages 637-644, XP055124508, Retrieved from the Internet: URL:http://dialnet.unirioja.es/servlet/oai art?codigo=114281 cited in the application
- ALEXANDRA BERNAL ET AL: "Stage-specific insecticidal characteristics of a nucleopolyhedrovirus isolate from Chrysodeixis?chalcites enhanced by optical brighteners", PEST MANAGEMENT SCIENCE, vol. 70, no. 5, 22 August 2013 (2013-08-22), pages 798-804, XP055124429, ISSN: 1526-498X, DOI: 10.1002/ps.3617
- A. BERNAL ET AL: "A Chrysodeixis chalcites Single-Nucleocapsid Nucleopolyhedrovirus Population from the Canary Islands Is Genotypically Structured To Maximize Survival", APPLIED AND ENVIRONMENTAL MICROBIOLOGY, vol. 79, no. 24, 4 October 2013 (2013-10-04), pages 7709-7718, XP055124432, ISSN: 0099-2240, DOI: 10.1128/AEM.02409-13
- A. BERNAL ET AL: "Complete Genome Sequences of Five Chrysodeixis chalcites Nucleopolyhedrovirus Genotypes from a Canary Islands Isolate", GENOME ANNOUNCEMENTS, vol. 1, no. 5, 24 October 2013 (2013-10-24), pages e00873-13, XP055124433, DOI: 10.1128/genomeA.00873-13

### SEQUENCE LISTING

<110> UNIVERSIDAD PÚBLICA DE NAVARRA Consejo Superior De Investigaciones Cientificas Instituto Canario de Investigaciones Agrarias (ICIA) Instituto de Ecologia, A.C.
<120> Novel genotypes of Chrysodeixis chalcites single nucleopolyhedrovirus (ChchSNPV), a procedure for its production and use as a biological control agent
<130> PCT-760
<150> ES201330487
   <151> 2013-04-04
<160> 19
<170> BiSSAP 1.0
<210> 1
   <211> 20
   <212> DNA
   <213> Artificial Sequence
<220>
   <221> source
   <222> 1..20
   <223> /isolate="Holland isolate, ChchSNPV-NL, GenBank AY864330" /mol_type="DNA" /note="Primer: Primer F-Hoar for Hoar gene" /organism="Chrysodeixis chalcites nucleopolyhedrovirus"
<400> 1
   ttgttgtatg cagcattgta 20
<210> 2
   <211> 20
   <212> DNA
   <213> Artificial Sequence
<220>
   <221> source
   <222> 1..20
   <223> /isolate="Holland isolate, ChchSNPV-NL, GenBank AY864330" /mol_type="DNA" /note="Primer R-Hoar for hoar gene" /organism="Chrysodeixis chalcites nucleopolyhedrovirus"
<400> 2
   agtaaatatg gctactgcag 20
<210> 3
   <211> 20
   <212> DNA
   <213> Artificial Sequence
<220>
   <221> source
   <222> 1..20
   <223> /isolate="Holland isolate, ChchSNPV-NL, GenBank AY864330" /mol_type="DNA" /note="Primer: Primer F-Brod for bro-d gene" /organism="Chrysodeixis chalcites nucleopolyhedrovirus"
<400> 3
   tatagtataa tattaaactc 20
<210> 4
   <211> 20
   <212> DNA
   <213> Artificial Sequence
<220>
   <221> source
   <222> 1..20
   <223> /isolate="Holland isolate, ChchSNPV-NL, GenBank AY864330" /mol_type="DNA" /note="Primer R-Brod for bro-d gene" /organism="Chrysodeixis chalcites nucleopolyhedrovirus"
<400> 4
   gtcatattcg agtcgtatcc 20
<210> 5
   <211> 756
   <212> DNA
   <213> Chrysodeixis chalcites nucleopolyhedrovirus
<220>
   <221> source
   <222> 1..756
   <223> /isolate="ChchSNPV-TF1" /mol_type="DNA" /organism="Chrysodeixis chalcites nucleopolyhedrovirus" /strain="ChchSNPV-TF1-A"
<220>
   <221> misc_feature
   <222> 1..756
   <223> /gene="Hoar" /note="genotype identifier fragment, viral genome nucleotides 3444-4199"
<220>
   <221> primer_bind
   <222> 737..756
   <223> /PCR_conditions="Primer R-Hoar: AGTAAATATGGCTACTGCAG"
<220>
   <221> primer_bind
   <222> 1..20
   <223> /PCR_conditions="Primer F-Hoar: TTGTTGTATGCAGCATTGTA"
<400> 5
   ttgttgtatg cagcattgta tccgccatcg tatccgttgt ttccgttgtt ataaccattg      60
   ttgtaattgt tgtagccatt atatccatta ttgtatccat tattgtatcc attgttatat     120
   ccattgttat atcctccgtt attgtatcct ccattattat atcttccgtt gttgtatccg     180
   ttattgtatg accaatcagc catcggcatc ataaaattat gcatcggtgg tgctggcatc     240
   atcatcggtg gcggagctga catcatcggc attggttgag ctggcatcgg aataaactca     300
   gtatcgaacg ttaaagcttc tggtatcaaa gccgctaaag ccggagtcgc tggagctgac     360
   gttggcatct gagtcgttgg agctgatgcc ggtgtagctg gtaccgacgt tggcatctga     420
   gtcgttggag ctgatgccgg tgtagctggt accgacgttg gcatctgagt cgttggagct     480
   gatgccggtg tagctggagc cgctgtagct ggagtcgctg gagccggtgt agctggagcc     540
   ggtgtagctg gagccggtgt agctggagcc ggtgtagctg gagccggtgt agctggagcc     600
   ggtgtagctt ccgtcggcgg ctgatcagtg gtcattagtt gtgaagataa tgattctatc     660
   ggcgaggttg gttgcagtgt ggaaaccgct gattcttcaa ctcccaattg tgttaattgg     720
   gattctatag atttttctgc agtagccata tttact                               756
<210> 6
   <211> 921
   <212> DNA
   <213> Chrysodeixis chalcites nucleopolyhedrovirus
<220>
   <221> source
   <222> 1..921
   <223> /isolate="ChchSNPV-TF1" /mol_type="DNA" /organism="Chrysodeixis chalcites nucleopolyhedrovirus" /strain=" ChchSNPV-TFl-B"
<220>
   <221> misc_feature
   <222> 1..921
   <223> /gene="Hoar" /note="Genotype identifier fragment, viral genome nucleotides
   3444-4364"
<220>
   <221> primer_bind
   <222> 1..20
   <223> /PCR_conditions="Primer F-Hoar: TTGTTGTATGCAGCATTGTA"
<220>
   <221> primer_bind
   <222> 902..921
   <223> /PCR_conditions="Primer R-Hoar: AGTAAATATGGCTACTGCAG"
<400> 6
   ttgttgtatg cagcattgta tccgccatcg tatccgttgt ttccgttgtt ataaccattg      60
   ttgtaattgt tgtagccatt atatccatta ttgtatccat tattgtatcc attgttatat     120
   ccattgttat atcctccgtt attgtatcct ccattattat atcttccgtt gttgtatccg     180
   ttattgtatg accaatcagc catcggcatc ataaaattat gcatcggtgg tgctggcatc     240
   atcatcggtg gcggagctga catcatcggc attggttgag ctggcatcgg aataaactca     300
   gtatcgaacg ttaaagcttc tggtatcaaa gccgctaaag ccggagtcgc tggagctgac     360
   gttggcatct gagtcgttgg agctgatgcc ggtgtagctg gtaccgacgt tggcatctga     420
   gtcgttggag ctgatgccgg tgtagctggt accgacgttg gcatctgagt cgttggagct     480
   gatgccggtg tagctggagc cgctgtagct ggagtcgctg gagccggtgt agctggagcc     540
   ggtgtagctg gagccggtgt agctggagcc ggtgtagctg gagccggtgt agctggagcc     600
   ggtgtagctg gagccggtgt agctggagcc ggtgtagctg gagccggtgt agctggagcc     660
   ggtgtagctg gagccggtgt agctggagcc ggtgtagctg gagccggtgt agctggagcc     720
   ggtgtagctg gagccggtgt agctggagcc ggtgtagctg gagccggtgt agcttccgtc     780
   ggcggctgat cagtggtcat tagttgtgaa gataatgatt ctatcggcga ggttggttgc     840
   agtgtggaaa ccgctgattc ttcaactccc aattgtgtta attgggattc tatagatttt     900
   tctgcagtag ccatatttac t                                               921
<210> 7
   <211> 651
   <212> DNA
   <213> Chrysodeixis chalcites nucleopolyhedrovirus
<220>
   <221> source
   <222> 1..651
   <223> /isolate=" ChchSNPV-TF1" /mol_type="DNA" /organism="Chrysodeixis chalcites nucleopolyhedrovirus" /strain=" ChchSNPV-TFl-C"
<220>
   <221> misc_feature
   <222> 1..651
   <223> /gene="Hoar" /note="Genotype identifier fragment, viral genome nucleotides 3443-4093"
<220>
   <221> primer_bind
   <222> 1..20
   <223> /PCR_conditions="Primer F-Hoar: TTGTTGTATGCAGCATTGTA"
<220>
   <221> primer_bind
   <222> 632..651
   <223> /PCR_conditions="Primer R-Hoar: AGTAAATATGGCTACTGCAG"
<400> 7
   ttgttgtatg cagcattgta tccgccatcg tatccgttgt ttccgttgtt ataaccattg      60
   ttgtaattgt tgtagccatt atatccatta ttgtatccat tattgtatct attgttatat     120
   cctccgttat tgtatcctcc attattatat cctccattat tatatcttcc gttgttgtat     180
   ccgttattgt atgaccaatc agccatcggc atcataaaat tatgcatcgg tggtgctggc     240
   atcatcatcg gtggcggagc tgacatcatc ggcattggtt gagctggcat cggaataaac     300
   tcagtatcga acgttaaagc ttctggtatc aaagccgcta aagccggagt cgctggagct     360
   gacgttggca tctgagtcgc tggagctggt atcggagtcg ctgacgttgg tatcgtagtc     420
   gctggagtcg ctggagtcgc tgtagctgga gccgccggtg tagctggagt cgctggagtc     480
   gctgtagctg gagccggtgt agcttccgtc ggcggctgat cagtggtcat tagttgtgaa     540
   gataatgatt ctatcggcga ggttggttgc agtgtggaaa ccgctgattc ttcaactccc     600
   aattgtgtta attgggattc tatagatttt tctgcagtag ccatatttac t              651
<210> 8
   <211> 1743
   <212> DNA
   <213> Chrysodeixis chalcites nucleopolyhedrovirus
<220>
   <221> source
   <222> 1..1743
   <223> /isolate=" ChchSNPV-TF1" /mol_type="DNA" /organism="Chrysodeixis chalcites nucleopolyhedrovirus" /strain=" ChchSNPV-TFl-A"
<220>
   <221> misc_feature
   <222> 1..1743
   <223> /gene="Bro-d" /note="Genotype identifier fragment, viral genome nucleotides 113281-115023"
<220>
   <221> primer_bind
   <222> 1..20
   <223> /PCR_conditions="Primer F-Brod: TATAGTATAATATTAAACTC"
<220>
   <221> primer_bind
   <222> 1724..1743
   <223> /PCR_conditions="Primer R-Brod: GTCATATTCGAGTCGTATCC"
<400> 8
   tatagtataa tattaaactc attttactcg acgattggga ttaaattaaa cagaattaat      60
   tttttctaag tgttttattt gcacattaca aaaactatgt caatcattta ttctatagcc     120
   aaggtatttg aagacatggg attctcattt ggtggagcaa cgttgaaata tctaatattg     180
   aacacgtttt taacaaaaaa gttttgacaa ttgttcatgg tgtgcaccac ctgttcaggc     240
   gaataagttt cccctgcgtt agagtgtcga cgctgagggg tatcaggcac taaatccttt     300
   gacattcctt cgactattcc ctcgactatc gcgttaatac gttccttagc ctcgatgctt     360
   ggcgtgagac atttagacac gcaatcatct tcgctagtaa agtccaacgc cttaaactct     420
   tcgatcatct tcgagtgtat gttcttgtta cgtttacaca tttccatgtc agctttatac     480
   ttatcgcgaa gctcaatttc atttaaaacc tccatttcgg ttctcagttt gttggtgtat     540
   cggaggccgt aaaatatgtg cggctgatct gtgcgcacct tgagccacac agtcacggga     600
   ttaggacact tgagctgtaa aaacttttta gagtctttga gccaggcgta ccgacgcgaa 660
   ttggttagtt tgggtcgctt ggcgtttact tcgggcaatg tggcttcttc ctggtatcgt 720
   tgaatggctc tgtcctgttg ttttatctca ctgtattggt ttcgaccgat acgaatgcgt 780
   tttctgccgt cgacggtgcg ctcgtagcca gtgatgtact cttccttgtc gggtcgctcg 840
   gtaagcgcag gcactactct tccgctgatg ctagtcagtg tttgacgaag cttttcattc 900
   tcatcgatgt tgtccttcgc caacaaagca ttgactgcaa attgagtcat ggacatgttg 960
   gcttgagtgg ccatatcctt catgagcagc tgcattttgt attcgcgctc tttgtactcg 1020
   ctcatttgat tttcgtaatg gcgcttcatc tccgaaatgg aggcgtcgta cttggcaatc 1080
   gtggtgtttg cctgcgacag ctccaacttt aattgtagcg attccatttg agcttctgcc 1140
   aattgtctgt cataattcac gacattggct gtcgtcggaa tcgagtattt accagttttc 1200
   ctcaattccg gcaacacttc ttcgaatagc cagcgttgaa attcttccgc agcaggtagt 1260
   ttagacttca taatcaaagc gtaaacgcct gcctcactga tgaatactgt gtttggctgc 1320
   cagtttgcag gtaattgcga ttcggctgat gtcgtttcat ctttagtttg cttcgtttca 1380
   agggggcgat aatccatcgc ctcctctgtc gatgtagctt taagggggtc ctgatttagg 1440
   acctccttga ttctttgaga tgaccgcgaa gtcgtaggta tttgaccgtt agtcgatgcc 1500
   gttgcaagag agttctgttt tagaaccttt tttatctctt gccaggtccg acgccattga 1560
   ggttttatat ggtcatataa agcttttccg ggtcttttat accccaaaaa ctgggcaatg 1620
   gcatgaccgc cgtacataaa tacatctttc tcgacttcga caatccacac atcgcaagcg 1680
   acgccgccga tctcgcattt tttgttgacg agagacatga tttggatacg actcgaatat 1740
   gac 1743
<210> 9
   <211> 962
   <212> DNA
   <213> Chrysodeixis chalcites nucleopolyhedrovirus
<220>
   <221> source
   <222> 1..962
   <223> /isolate=" ChchSNPV-TF1" /mol_type="DNA" /organism="Chrysodeixis chalcites nucleopolyhedrovirus" /strain=" ChchSNPV-TFl-B"
<220>
   <221> misc_feature
   <222> 1..962
   <223> /gene="Bro-d" /note="Genotype identifier fragment, viral genome nucleotides 113542-114413"
<220>
   <221> primer_bind
   <222> 1..20
   <223> /PCR_conditions="Primer F-Brod: TATAGTATAATATTAAACTC"
<220>
   <221> primer_bind
   <222> 943..962
   <223> /PCR_conditions="Primer R-Brod: GTCATATTCGAGTCGTATCC"
<400> 9
   tatagtataa tattaaactc attttactcg acgatcgggc atttgtacac aatgaaacaa      60
   attattttcc aatatatttt ttattgtgta caatcttttt taatcaaagt caattatcct     120
   gtattaattg taaaatattt tgaatattat ggttttggtt attacataaa gaagattcaa     180
   tacaatatag ttttactaac tgttttaatt ttaattttgc atctgaatat tctttattgc     240
   tattaaattg cacccattca cctctaagct tacgtgattt atattcattt aacaataaag     300
   attctattac atatccttga gtcccggtat cttcataaac atcatattct agtaaatcaa     360
   atggtcttcc acaattaagc tgtttaagtc tattatcaag tgaagtagtg gtgccgattt     420
   tgtaaatatg tttctgttta tattgttcat ttgtgaccat ataaacaaat gatttgactg     480
   gtacgtaggt tgataattgt cgattgtggt acgattcaaa tgaagacgtt aaactattag     540
   ttttacgaag ctgtggcaac acttcttcaa atagccattt ttgaaataag tctgcttttg     600
   gtagtctaca tcgcactatt aatgcgtata cacctgcttc actgataaac actgtgtttg     660
   gctgccagtt tgctggtaac tgcgactcgg ctgatgtcgt tttatcttta gcttgcttcg     720
   tttcaagggg atcctgatta tttaggatct ttatctcttg ccaggtccga cgccattgag     780
   gttttatatg gtcatataaa gcttttccgg gtcttttata ccccaaaaac tgggcaatgg     840
   catgaccgcc gtacataaat acatctttct cgactttgac aatccacaca tcgcaagcga     900
   cgccgccgat ctcgcatttt ttgttgacga gagacatgat ttggatacga ctcgaatatg     960
   ac                                                                    962
<210> 10
   <211> 1725
   <212> DNA
   <213> Chrysodeixis chalcites nucleopolyhedrovirus
<220>
   <221> source
   <222> 1..1725
   <223> /isolate=" ChchSNPV-TF1" /mol_type="DNA" /organism="Chrysodeixis chalcites nucleopolyhedrovirus" /strain=" ChchSNPV-TFl-C"
<220>
   <221> misc_feature
   <222> 1..1725
   <223> /gene="Bro-d" /note="Genotype identifier fragment, viral genome nucleotides 113662-115386"
<220>
   <221> primer_bind
   <222> 1..20
   <223> /PCR_conditions="Primer F-Brod: TATAGTATAATATTAAACTC"
<220>
   <221> primer_bind
   <222> 1706..1725
   <223> /PCR_conditions="Primer R-Brod: GTCATATTCGAGTCGTATCC"
<400> 10
   tatagtataa tattaaactc attttactcg acgattggga ttaaattaaa cagaattaat      60
   ttttttctaa gtgttttatt tgcacattac aaaaactatg tcaatcattt attctatagc      120
   caaggtattt gaagacatgg gattctcatt tggtggagca acgttgaaat atctaatatt     180
   gaacacgttt ttaacaaaaa agttttgaca attgttcatg gtgtgcacca cctgttcagg     240
   cgaataagtt tcccctgcgt tagagtgtcg acgctgaggg gtatcaggca ctaaatcctt     300
   tgacattcct tcgactattc cctcgactat cgcgttaata cgttccttag cctcaatgct     360
   tggcgtgaga catttagaca cgcaatcatc ttcgctagta aagtccaacg ccttaaactc     420
   ttcgatcatc ttcgagtgta tgttcttgtt acgtttacac atttccatgt cagctttata     480
   cttatcgcga agctcaattt catttaaaac ctccatttcg gttctcagtt tgttggtgta     540
   tcgaaggccg taaaatatgt gcggctgatc tgtgcgcacc ttgagccaca cagtcacggg     600
   attaggacac ttgagctgta aaaacttttt agagtctttg agccaggcgt accgacgcga     660
   attggttagt ttgggtcgct tggcgtttac ttcgggcaat gtggcttctt cctggtatcg     720
   ttgaatggct ctgtcctgtt gttttatctc actgtattgg tttcgaccta tacgaatgcg     780
   ttttctgccg tcgacggtgc gctcgtagcc agtgatgtac tcttccttgt cgggtcgctc     840
   ggtaagcgca ggaactactc ttccgctgat gctagtcagt gtttgacgaa gcttttcatt     900
   ctcatcgatg ttgtccttcg ccaacaaagc attgactgca aattgagtca tggacatgtt     960
   ggcttgagtg gccatatcct tcatgagcag ctgcattttg tattcgcgct ctttgtactc    1020
   gctcatttga ttttcgtaat ggcgcttcat ctccgaaatg gaggcgtcgt acttggcaat    1080
   cgtagtgttt gcctgcgcaa tcgtggtgtt tgcctgcgca atcgtggtgt ttgcctgcga    1140
   cagctccaac tttaattgta gcgattccat ttgagcttct gccaattgtt tgtcataatt    1200
   cacgacattg gctgtcgtcg gaatccagtt ttcctcaatt ccggcaacac ttcttcgaat    1260
   agccagcgtt gaaattcttc cgcagcaggt agtttagact tcataatcaa agcgtaaacg    1320
   cctgcctcac tgatgaatac tgtgtttggc tgccagtttg caggtaattg cgattcggct    1380
   gatgtcgttt catctttagt ttgcttcgtt tcaagggggt cctgatttag gattctttga    1440
   gatgaccgcg aagtcgtagg tatttgaccg ttagtcgatg ccgttgcaag agagttctgt    1500
   tttaaaacct cctttatctc ttgccaggtc cgacgccatt gaggttttat atggtcatat    1560
   aaagcttttc cgggtctttt ataccccaaa aattgagcaa tggcatgacc gccgtacata 1620
   aatacatctt tctcgacttc gacaatccac acatcgcaag cgacgctgcc gatcttgcat 1680
   tttttgttga cgagagacat gatttggata cgactcgaat atgac 1725
<210> 11
   <211> 3636
   <212> DNA
   <213> Chrysodeixis chalcites nucleopolyhedrovirus
<220>
   <221> source
   <222> 1..3636
   <223> /isolate=" ChchSNPV-TF1" /mol_type="DNA" /note="Nucleotides 2966 to 6601 of the genome" /organism="Chrysodeixis chalcites nucleopolyhedrovirus" /strain=" ChchSNPV-TFl-A"
<220>
   <221> misc_feature
   <222> 1..3636
   <223> /gene="Chch4 Hoar" /note="Region of variability, complement to CDS"
<400> 11
   ttattctatt ttttgaactg tagttataag aggaattatt gtggtaagac cacaatcctc 60
   aacaatctgg acgtcgtcat cgctgtcatc ggccgaagca ggaggtaaaa tactggataa 120
   tgtgtctttg gccaattgtt gataatctat agcagtctgc tgcagctgca ccggttcttc 180
   tttaacaacg tctagaacga aatccgtagg cacgtacggt ttaccgcgag cagttagatt 240
   taacttgcgc aaattttcat tattgcaagt atagcgataa gtgaccagtt ttgtcgacgg 300
   aggaggctca ttagatgaat ttattgcctc cggtgtgatt acacgcaacg cccgtgaatg 360
   ctctggaaca gttgaattca taggctggtt aaaatcagga ttattgaagg aggccggact 420
   tatagtatcg tacattggag atataaaact cgaaaattga tttaacccgg ggtatccgtt 480
   gttgtatgca gcattgtatc cgccatcgta tccgttgttt ccgttgttat aaccattgtt 540
   gtaattgttg tagccattat atccattatt gtatccatta ttgtatccat tgttatatcc 600
   attgttatat cctccgttat tgtatcctcc attattatat cttccgttgt tgtatccgtt 660
   attgtatgac caatcagcca tcggcatcat aaaattatgc atcggtggtg ctggcatcat 720
   catcggtggc ggagctgaca tcatcggcat tggttgagct ggcatcggaa taaactcagt 780
   atcgaacgtt aaagcttctg gtatcaaagc cgctaaagcc ggagtcgctg gagctgacgt 840
   tggcatctga gtcgttggag ctgatgccgg tgtagctggt accgacgttg gcatctgagt 900
   cgttggagct gatgccggtg tagctggtac cgacgttggc atctgagtcg ttggagctga 960
   tgccggtgta gctggagccg ctgtagctgg agtcgctgga gccggtgtag ctggagccgg 1020
   tgtagctgga gccggtgtag ctggagccgg tgtagctgga gccggtgtag ctggagccgg 1080
   tgtagcttcc gtcggcggct gatcagtggt cattagttgt gaagataatg attctatcgg 1140
   cgaggttggt tgcagtgtgg aaaccgctga ttcttcaact cccaattgtg ttaattggga 1200
   ttctatagat ttttctgcag tagccatatt tactcgcgaa ggagacgaaa cttcagtatt 1260
   attgttatca acatcatcgt catcatcatc atcatcacta ctattgctac tactatcact 1320
   attatcacca tcgttattgt catcatcact actatcacta ctatcaccac tatcactact 1380
   attattatta gtttgattat caatctgttc gtcttgttga ttttcgtctt gttcttcttg 1440
   ttcttcttgt tcttcttgtt cttcttgttc ttcttgttga ttttcgtctt gttcttcttg 1500
   ttgattgttt tgttgttgat tttcgtcttg ttgattgtcg tcttgttttt gttcattttc 1560
   ttgaactaat actggtcttg cagcaggttt tattgtagca gttggtttct caataattat 1620
   ctcagactgt ggtagtacag caggctcaga ttgaggtggc tgagtggcgg tagtagatgg 1680
   aacatcatca ttattatcat cattattgcc atcattatta ctggggccag gcgattgaga 1740
   tgacgctgga atatcatcag cgggcgcttg atcatcagcg ggcgcttgat catcagcggg 1800
   tgcttgatca tcaacgggcg gcggcgatgg cgtccatcgc atccaacgtg gtggcggcgg 1860
   tggagattca tatcggaatg aattaatttg tttggtttga tagcaaaaaa tacattcatt 1920
   gtatttcatc atactatcgt aggtacagca tatgcataat atatgttcac agtgtggata 1980
   cacaaatcct ttatctccat tgttgcaact tatgcacggt tcagtcataa tcttcagctt 2040
   gttgtttctc atgtatgtat atagtacaga actgagtcta gatgattttt ctcgaagtgc 2100
   aatggcttcg gtttgaagcg catgagttat tatcgtatcc agtagataga tattcttttt 2160
   tatctccgca tctacggtga tagtgtgaaa agaagagcca atattcttga atttgtcgtg 2220
   catcagacca catttgatca ttaattcttg tctagtgcta gtattaacgt tttcgaaaat 2280
   cgattgtata aacgaaaact tagcattctc atcctcggaa acagtataga attttttata 2340
   ttcagataaa cattgcataa tatgctctaa aacgcattgc ttggttagta ttgtcaagta 2400
   agtctctaat tgcttgcgaa caggtatata gtttaacgat tggatatgca agaataaatc 2460
   ttcataacgg ttagagaaaa agtgtctaga tttatatgtg atgccagtaa acttgaattg 2520
   tttgttaata cagcacacat agcctacgcg ctggttattt ttagagttta aatttaaata 2580
   gataaacact tttttctcga ccgtattatg gtgcaggtga atattgttat gattgttact 2640
   gatcatatct ccagtgggta atattatagc agtagtatta gatgggtttt cattatctac 2700
   cacatctacc acgggagttt cttgtttttg acgcttgctg ttagacgcag catcactatc 2760
   actctcatta tcgatatcac gcttaggtgt ttcttttttc tttagtttct tcagtatttt 2820
   tttagttttt tttccctttc cttttccttt acctttttca attttttcat tttcattatc 2880
   actgtcactt tcactaataa ttttacgttt gcgtaattcg atttcttctt cttcttgatc 2940
   ttgtccttta tttttttctt ttttaatttt ttcataatca ctgtcattat cactttcact 3000
   aataatttta cgtttttctt tttcttgatc ttgttgttct tgatcttgtt gttgttgatc 3060
   ttgttcttga tcttgttgtt gtttagatgc gctggcgata acgttatcgc cagcagccga 3120
   ttctactgtc acgccatttg aatctacgtc gtgagtcaat tcatccatct tcagtatttt 3180
   tttagtcttt tttccctttc cttttccttt accttttcct ttttcatttt ttttatattc 3240
   actgtcatta tcactttcat taataatttt acgtttgcgt aattcgattt cttcttcttg 3300
   atcttgttta gatgtgctgg taatcacgtt atcgccagtc acgccatttg aatctacgtc 3360
   ttgagtcaat tcatctaatt ctttttgaat tttgttaatt tttgacgaat aattttcatt 3420
   tattttatca aaatttttat attctttaaa aatttcttca tcagattcgg taaatggtat 3480
   gggttcaagg aaccctatta taggtttttt catttcacgc cgaatctctt cttcagaaaa 3540
   attaaacctc cgtagagttt cctctttcaa ttctgcattc gatagcgacg cagtatattt 3600
   cattttctca aatttagatt taaatttccg tatcat 3636
<210> 12
   <211> 3804
   <212> DNA
   <213> Chrysodeixis chalcites nucleopolyhedrovirus
<220>
   <221> source
   <222> 1..3804
   <223> /isolate="ChchSNPV-TF1" /mol_type="DNA" /note="Nucleotides 2966 to 6769 of the genome" /organism="Chrysodeixis chalcites nucleopolyhedrovirus" /strain=" ChchSNPV-TFl-B"
<220>
   <221> misc_feature
   <222> 1..3804
   <223> /gene="Chch4 Hoar" /note="Region of variability, complement to CDS"
<400> 12
   ttattctatt ttttgaactg tagttataag aggaattatt gtggtaagac cacaatcctc 60
   aacaatctgg acgtcgtcat cgctgtcatc ggccgaagca ggaggtaaaa tactggataa 120
   tgtgtctttg gccaattgtt gataatctat agcagtctgc tgcagctgca ccggttcttc 180
   tttaacaacg tctagaacga aatccgtagg cacgtacggt ttaccgcgag cagttagatt 240
   taacttgcgc aaattttcat tattgcaagt atagcgataa gtgaccagtt ttgtcgacgg 300
   aggaggctca ttagatgaat ttattgcctc cggtgtgatt acacgcaacg cccgtgaatg 360
   ctctggaaca gttgaattca taggctggtt aaaatcagga ttattgaagg aggccggact 420
   tatagtatcg tacattggag atataaaact cgaaaattga tttaacccgg ggtatccgtt 480
   gttgtatgca gcattgtatc cgccatcgta tccgttgttt ccgttgttat aaccattgtt 540
   gtaattgttg tagccattat atccattatt gtatccatta ttgtatccat tgttatatcc 600
   attgttatat cctccgttat tgtatcctcc attattatat cttccgttgt tgtatccgtt 660
   attgtatgac caatcagcca tcggcatcat aaaattatgc atcggtggtg ctggcatcat 720
   catcggtggc ggagctgaca tcatcggcat tggttgagct ggcatcggaa taaactcagt 780
   atcgaacgtt aaagcttctg gtatcaaagc cgctaaagcc ggagtcgctg gagctgacgt 840
   tggcatctga gtcgttggag ctgatgccgg tgtagctggt accgacgttg gcatctgagt 900
   cgttggagct gatgccggtg tagctggtac cgacgttggc atctgagtcg ttggagctga 960
   tgccggtgta gctggagccg ctgtagctgg agtcgctgga gccggtgtag ctggagccgg 1020
   tgtagctgga gccggtgtag ctggagccgg tgtagctgga gccggtgtag ctggagccgg 1080
   tgtagctgga gccggtgtag ctggagccgg tgtagctgga gccggtgtag ctggagccgg 1140
   tgtagctgga gccggtgtag ctggagccgg tgtagctgga gccggtgtag ctggagccgg 1200
   tgtagctgga gccggtgtag ctggagccgg tgtagctgga gccggtgtag cttccgtcgg 1260
   cggctgatca gtggtcatta gttgtgaaga taatgattct atcggcgagg ttggttgcag 1320
   tgtggaaacc gctgattctt caactcccaa ttgtgttaat tgggattcta tagatttttc 1380
   tgcagtagcc atatttactc gcgaaggaga cgaaacttca gtattattgt tatcaacatc 1440
   atcgtcatca tcatcatcat cactactatt gctactacta tcactattat caccatcgtt 1500
   attgtcatca tcactactat cactactatc accactatca ctactattat tattagtttg 1560
   attatcaatc tgttcgtctt gttgattttc gtcttgttct tcttgttctt cttgttcttc 1620
   ttgttcttct tgttcttctt gttcttcttg ttcttcttgt tcttcttgtt gattgttttg 1680
   ttgttgattt tcgtcttgtt gattgtcgtc ttgtttttgt tcattttctt gaactaatac 1740
   tggtcttgca gcaggtttta ttgtagcagt tggtttctca ataattatct cagactgtgg 1800
   tagtacagca ggctcagatt gaggtggctg agtggcggta gtagatggaa catcatcatt 1860
   attatcatca ttattgccat cattattact ggggccaggc gattgagatg acgctggaat 1920
   atcatcagcg ggcgcttgat catcagcggg cgcttgatca tcagcgggtg cttgatcatc 1980
   aacgggcggc ggcgatggcg tccatcgcat ccaacgtggt ggcggcggtg gagattcata 2040
   tcggaatgaa ttaatttgtt tggtttgata gcaaaaaata cattcattgt atttcatcat 2100
   actatcgtag gtacagcata tgcataatat atgttcacag tgtggataca caaatccttt 2160
   atctccattg ttgcaactta tgcacggttc agtcataatc ttcagcttgt tgtttctcat 2220
   gtatgtatat agtacagaac tgagtctaga tgatttttct cgaagtgcaa tggcttcggt 2280
   ttgaagcgca tgagttatta tcgtatccag tagatagata ttctttttta tctccgcatc 2340
   tacggtgata gtgtgaaaag aagagccaat attcttgaat ttgtcgtgca tcagaccaca 2400
   tttgatcatt aattcttgtc tagtgctagt attaacgttt tcgaaaatcg attgtataaa 2460
   cgaaaactta gcattctcat cctcggaaac agtatagaat tttttatatt cagataaaca 2520
   ttgcataata tgctctaaaa cgcattgctt ggttagtatt gtcaagtaag tctctaattg 2580
   cttgcgaaca ggtatatagt ttaacgattg gatatgcaag aataaatctt cataacggtt 2640
   agagaaaaag tgtctagatt tatatgtgat gccagtaaac ttgaattgtt tgttaataca 2700
   gcacacatag cctacgcgct ggttattttt agagtttaaa tttaaataga taaacacttt 2760
   tttctcgacc gtattatggt gcaggtgaat attgttatga ttgttactga tcatatctcc 2820
   agtgggtaat attatagcag tagtattaga tgggttttca ttatctacca catctaccac 2880
   gggagtttct tgtttttgac gcttgctgtt agacgcagca tcactatcac tctcattatc 2940
   gatatcacgc ttaggtgttt ctttttcttt tagtttcttc agtatttttt tagttttttt 3000
   tccctttcct tttcctttac ctttttcaat tttttcattt tcattatcac tgtcactttc 3060
   actaataatt ttacgtttgc gtaattcgat ttcttcttct tcttgatctt gtcctttatt 3120
   tttttctttt ttaatttttt cataatcact gtcattatca ctttcactaa taattttacg 3180
   tttttctttt tcttgatctt gttgttcttg atcttgttgt tgttgatctt gttcttgatc 3240
   ttgttgttgt ttagatgcgc tggcgataac gttatcgcca gcagccgatt ctactgtcac 3300
   gccatttgaa tctacgtcgt gagtcaattc atccatcttc agtatttttt tagtcttttt 3360
   tccctttcct tttcctttac cttttccttt ttcatttttt ttatattcac tgtcattatc 3420
   actttcatta ataattttac gtttgcgtaa ttcgatttct tcttcttgat cttgtttaga 3480
   tgtgctggta atcacgttat cgccagtcac gccatttgaa tctacgtctt gagtcaattc 3540
   atctaattct ttttgaattt tgttaatttt tgacgaataa ttttcattta ttttatcaaa 3600
   atttttatat tctttaaaaa tttcttcatc agattcggta aatggtatgg gttcaaggaa 3660
   ccctattata ggttttttca tttcacgccg aatctcttct tcagaaaaat taaacctccg 3720
   tagagtttcc tctttcaatt ctgcattcga tagcgacgca gtatatttca ttttctcaaa 3780
   tttagattta aatttccgta tcat 3804
<210> 13
   <211> 3900
   <212> DNA
   <213> Chrysodeixis chalcites nucleopolyhedrovirus
<220>
   <221> source
   <222> 1..3900
   <223> /isolate="ChchSNPV-TF1" /mol_type="DNA" /note="Nucleotides 2965 to 6864 of the genome" /organism="Chrysodeixis chalcites nucleopolyhedrovirus" /strain="ChchSNPV-TF1-C"
<220>
   <221> misc_feature
   <222> 1..3900
   <223> /gene="Chch4 Hoar" /note="Region of variability, complement to CDS"
<400> 13
   ttattctatt ttttgaactg tagttataag aggaagtatt gtggtaagac cacaatcctc      60
   aacaatctgg acgtcgtcat cgctgtcatt ggccgaagca ggaggtaaaa tactggataa     120
   tgtgtctttg gccaattgtt gataatctat agcagtctgc tgcagctgca ccggttcttc     180
   tttaacaacg tctagaacga aatccgtagg cacgtacggt ttaccgcgag cagttagatt     240
   taacttgcgc aaattttcat tattgcaagt atagcgataa gtgaccagtt ttgtcgacgg     300
   aggaggctca ttagatgaat ttattgcctc cggtgtgatt acacgcaacg cccgtgaatg     360
   ctctggaaca gttgaattca taggctggtt aaaatcagga ttattgaagg aggccggact     420
   tatagtatcg tacattggag atataaaact cgaaaattga tttaacccgg ggtatccgtt     480
   gttgtatgca gcattgtatc cgccatcgta tccgttgttt ccgttgttat aaccattgtt     540
   gtaattgttg tagccattat atccattatt gtatccatta ttgtatctat tgttatatcc     600
   tccgttattg tatcctccat tattatatcc tccattatta tatcttccgt tgttgtatcc     660
   gttattgtat gaccaatcag ccatcggcat cataaaatta tgcatcggtg gtgctggcat     720
   catcatcggt ggcggagctg acatcatcgg cattggttga gctggcatcg gaataaactc     780
   agtatcgaac gttaaagctt ctggtatcaa agccgctaaa gccggagtcg ctggagctga     840
   cgttggcatc tgagtcgctg gagctggtat cggagtcgct gacgttggta tcgtagtcgc     900
   tggagtcgct ggagtcgctg tagctggagc cgccggtgta gctggagtcg ctggagtcgc     960
   tgtagctgga gccggtgtag cttccgtcgg cggctgatca gtggtcatta gttgtgaaga    1020
   taatgattct atcggcgagg ttggttgcag tgtggaaacc gctgattctt caactcccaa    1080
   ttgtgttaat tgggattcta tagatttttc tgcagtagcc atatttactc gcgaaggaga    1140
   cgaaacttca gtattattgt tatcaacatc atcgtcatca tcatcatcat cactactatt    1200
   gctactacta tcactattat caccatcgtt attgtcatca tcactactat cactactatc    1260
   accactatca ctactattat tattagtttg attatcaatc tgttcgtctt gttgattttc    1320
   gtcttgttct tcttgttctt cttgttcttc ttgttcttct tgttcttctt gttcttcttg    1380
   ttcttcttgt tcttcttgtt cttcttgntg attttcgtct tgntnatttt cttcttgttc    1440
   ttcttgttct tcttgttctt cttgttcttc ttgttcttct tgttgatttt cgtcttgttg    1500
   attttcttct tgttcttctt gttcttcttg ttcttcttgt tcttcttgtt cttcttgttg    1560
   attttcgtct tgttgatttt cttcttgttc ttcttgttct tcttgttctt cttgttcttc    1620
   ttgttcttct tgttcttctt gttgattttc gtcttgttga ttttcttctt gttcttcttg    1680
   ttcttcttgt tcttcttgtt cttcttgttc ttcttgttga ttttcgtctt gttgattttc 1740
   ttcttgttct tcttgttctt cttgttcttc ttgttcttct tgttcttctt gttcttcttg 1800
   ttgattttcg tcttgttgat tttcgtcttg ttcttcttgt tgattgtttt gttgttgatt 1860
   ttcgtcttgt tgattgtcgt cttgtttttg ttcattttct tgaactaata ctggtcttgc 1920
   agcaggtttt attgtagcag ttggtttctc aataattatc tcagactgtg gtagtacagc 1980
   attattatca ttaatatcgc catcattatt actggggcca ggcgattgag atgacgctgg 2040
   aatatcatca gcgggcgctt gatcatcagc gggtgcttga tcatcaacgg gcggcggcga 2100
   tggcgtccat cgcatccgcg gcggtggaga ttcatatcgg aatgaattaa tttgtttggt 2160
   ttgatagcaa aaaatacatt cattgtattt catcatacta tcgtaggtac agcatatgca 2220
   taatatatgt tcacagtgtg gatacacaaa tcctttatct ccattgttgc aacttatgca 2280
   cggttcagtc ataatcttca gcttgttgtt tctcatgtat gtatatagta cagaactgag 2340
   tctagatgat ttttctcgaa gtgcaatggc ttcggtttga agcgcatgag ttattatcgt 2400
   atccagtaga tagatattct tttttatctc cgcatctacg gtgatagtgt gaaaagaaga 2460
   gccaatattc ttgaatttgt cgtgcatcag accacatttg atcattaatt cttgtctagt 2520
   gctagtatta acgttttcga aaatcgattg tataaacgaa aacttagcat tctcatcctc 2580
   ggaaacagta tagaattttt tatattcaga taaacattgc ataatatgct ctaaaacgca 2640
   ttgcttggtt agtattgtca agtaagtctc taattgcttg cgaacaggta tatagtttaa 2700
   cgattggata tgcaagaata aatcttcata acggttagag aaaaagtgtc tagatttata 2760
   tgtgatgcca gtaaacttga attgtttgtt aatacagcac acatagccta cgcgctggtt 2820
   atttttagag tttaaattta aatagataaa cacttttttc tcgaccgtat tatggtgcag 2880
   gtgaatattg ttatgattgt tactgatcat atctccagtg ggtaatatta tagcagtagt 2940
   attagatggg ttttcattat ctaccacatc taccacggga gtttcttgtt tttgacgctt 3000
   gctgttagac gcagcatcac tatcactctc attatcgata tcacgcttag gtgtttcttt 3060
   tttctttagt ttcttcagta tttttttagt tttttttccc tttccttttc ctttaccttt 3120
   ttcaattttt tcattttcat tatcactgtc actttcacta ataattttac gtttgcgtaa 3180
   ttcgatttct tcttcttctt gatcttgtcc tttatttttt tcttttttaa ttttttcata 3240
   atcactgtca ttatcacttt cactaataat tttacgtttt tctttttctt gatcttgttg 3300
   ttcttgatct tgttgttgtt gatcttgttc ttgatcttgt tgttgtttag atgcgctggc 3360
   gataacgtta tcgccagcag ccgattctac tgtcacgcca tttgaatcta cgtcgtgagt 3420
   caattcatcc atcttcagta tttttttagt cttttttccc tttccttttc ctttaccttt 3480
   tcctttttca ttttttttat attcactgtc attatcactt tcattaataa ttttacgttt 3540
   gcgtaattcg attttttctt cttgatcttg tttagatgtg ctggtaatca cgttatcgcc 3600
   agtcacgcca tttgaatcta cgtcttgagt caattcatct aattcttttt gaatttcgtt 3660
   tatttttgac aaattatttt catttatttt atcaaaattt tcatattctt taaaaatttc 3720
   ttcatcagat tcggtaaatg gcatgggttc aagaaaacct attaaaggtt ttttcatttc 3780
   acgccgaatc tcttcttcag taaaattaaa cctccgtagg gtttcctctt tcaattctgc 3840
   attcgatagc gacgcagtat atttcatttt ctcaaattta gatttaaatt tccgtatcat 3900
<210> 14
   <211> 1611
   <212> DNA
   <213> Chrysodeixis chalcites nucleopolyhedrovirus
<220>
   <221> source
   <222> 1..1611
   <223> /isolate="ChchSNPV-TF1" /mol_type="DNA" /note="Nucleotides 113388 to 114998 of the genome" /organism="Chrysodeixis chalcites nucleopolyhedrovirus" /strain="ChchSNPV-TF1-A"
<220>
   <221> misc_feature
   <222> 1..1611
   <223> /gene="Chch114, Bro-d" /note="Region of variability, complement to CDS"
<400> 14
   ttattctata gccaaggtat ttgaagacat gggattctca tttggtggag caacgttgaa 60
   atatctaata ttgaacacgt ttttaacaaa aaagttttga caattgttca tggtgtgcac 120
   cacctgttca ggcgaataag tttcccctgc gttagagtgt cgacgctgag gggtatcagg 180
   cactaaatcc tttgacattc cttcgactat tccctcgact atcgcgttaa tacgttcctt 240
   agcctcgatg cttggcgtga gacatttaga cacgcaatca tcttcgctag taaagtccaa 300
   cgccttaaac tcttcgatca tcttcgagtg tatgttcttg ttacgtttac acatttccat 360
   gtcagcttta tacttatcgc gaagctcaat ttcatttaaa acctccattt cggttctcag 420
   tttgttggtg tatcggaggc cgtaaaatat gtgcggctga tctgtgcgca ccttgagcca 480
   cacagtcacg ggattaggac acttgagctg taaaaacttt ttagagtctt tgagccaggc 540
   gtaccgacgc gaattggtta gtttgggtcg cttggcgttt acttcgggca atgtggcttc 600
   ttcctggtat cgttgaatgg ctctgtcctg ttgttttatc tcactgtatt ggtttcgacc 660
   gatacgaatg cgttttctgc cgtcgacggt gcgctcgtag ccagtgatgt actcttcctt 720
   gtcgggtcgc tcggtaagcg caggcactac tcttccgctg atgctagtca gtgtttgacg 780
   aagcttttca ttctcatcga tgttgtcctt cgccaacaaa gcattgactg caaattgagt 840
   catggacatg ttggcttgag tggccatatc cttcatgagc agctgcattt tgtattcgcg 900
   ctctttgtac tcgctcattt gattttcgta atggcgcttc atctccgaaa tggaggcgtc 960
   gtacttggca atcgtggtgt ttgcctgcga cagctccaac tttaattgta gcgattccat 1020
   ttgagcttct gccaattgtc tgtcataatt cacgacattg gctgtcgtcg gaatcgagta 1080
   tttaccagtt ttcctcaatt ccggcaacac ttcttcgaat agccagcgtt gaaattcttc 1140
   cgcagcaggt agtttagact tcataatcaa agcgtaaacg cctgcctcac tgatgaatac 1200
   tgtgtttggc tgccagtttg caggtaattg cgattcggct gatgtcgttt catctttagt 1260
   ttgcttcgtt tcaagggggc gataatccat cgcctcctct gtcgatgtag ctttaagggg 1320
   gtcctgattt aggacctcct tgattctttg agatgaccgc gaagtcgtag gtatttgacc 1380
   gttagtcgat gccgttgcaa gagagttctg ttttagaacc ttttttatct cttgccaggt 1440
   ccgacgccat tgaggtttta tatggtcata taaagctttt ccgggtcttt tataccccaa 1500
   aaactgggca atggcatgac cgccgtacat aaatacatct ttctcgactt cgacaatcca 1560
   cacatcgcaa gcgacgccgc cgatctcgca ttttttgttg acgagagaca t 1611
<210> 15
   <211> 828
   <212> DNA
   <213> Chrysodeixis chalcites nucleopolyhedrovirus
<220>
   <221> source
   <222> 1..828
   <223> /isolate="ChchSNPV-TF1" /mol_type="DNA" /organism="Chrysodeixis chalcites nucleopolyhedrovirus" /strain="ChchSNPV-TF1-B"
<220>
   <221> misc_feature
   <222> 1..828
   <223> /gene="Chch114, Bro-d" /note="Region of variability, complement to CDS"
<400> 15
   tcaattatcc tgtattaatt gtaaaatatt ttgaatatta tggttttggt tattacataa 60
   agaagattca atacaatata gttttactaa ctgttttaat tttaattttg catctgaata 120
   ttctttattg ctattaaatt gcacccattc acctctaagc ttacgtgatt tatattcatt 180
   taacaataaa gattctatta catatccttg agtcccggta tcttcataaa catcatattc 240
   tagtaaatca aatggtcttc cacaattaag ctgtttaagt ctattatcaa gtgaagtagt 300
   ggtgccgatt ttgtaaatat gtttctgttt atattgttca tttgtgacca tataaacaaa 360
   tgatttgact ggtacgtagg ttgataattg tcgattgtgg tacgattcaa atgaagacgt 420
   taaactatta gttttacgaa gctgtggcaa cacttcttca aatagccatt tttgaaataa 480
   gtctgctttt ggtagtctac atcgcactat taatgcgtat acacctgctt cactgataaa 540
   cactgtgttt ggctgccagt ttgctggtaa ctgcgactcg gctgatgtcg ttttatcttt      600
   agcttgcttc gtttcaaggg gatcctgatt atttaggatc tttatctctt gccaggtccg      660
   acgccattga ggttttatat ggtcatataa agcttttccg ggtcttttat accccaaaaa      720
   ctgggcaatg gcatgaccgc cgtacataaa tacatctttc tcgactttga caatccacac      780
   atcgcaagcg acgccgccga tctcgcattt tttgttgacg agagacat                   828
<210> 16
   <211> 420
   <212> DNA
   <213> Chrysodeixis chalcites nucleopolyhedrovirus
<220>
   <221> source
   <222> 1..420
   <223> /isolate="ChchSNPV-TF1" /mol_type="DNA" /note="Nucleotides 114861 to 115280 of the genome" /organism="Chrysodeixis chalcites nucleopolyhedrovirus" /strain="ChchSNPV-TF1-C"
<220>
   <221> misc_feature
   <222> 1..420
   <223> /gene="Chch114, Bro-d" /note="Region of variability, complement to CDS"
<400> 16
   tcacgacatt ggctgtcgtc ggaatccagt tttcctcaat tccggcaaca cttcttcgaa       60
   tagccagcgt tgaaattctt ccgcagcagg tagtttagac ttcataatca aagcgtaaac      120
   gcctgcctca ctgatgaata ctgtgtttgg ctgccagttt gcaggtaatt gcgattcggc      180
   tgatgtcgtt tcatctttag tttgcttcgt ttcaaggggg tcctgattta ggattctttg      240
   agatgaccgc gaagtcgtag gtatttgacc gttagtcgat gccgttgcaa gagagttctg      300
   ttttaaaacc tcctttatct cttgccaggt ccgacgccat tgaggtttta tatggtcata      360
   taaagctttt ccgggtcttt tataccccaa aaattgagca atggcatgac cgccgtacat      420
<210> 17
   <211> 149684
   <212> DNA
   <213> Chrysodeixis chalcites nucleopolyhedrovirus
<220>
   <221> source
   <222> 1..149684
   <223> /isolate="ChchSNPV-TF1" /isolation_source="Canary Islands, Spain" /mol_type="DNA" /note="Genome of ChchSNPV-TFl-A" /organism="Chrysodeixis chalcites nucleopolyhedrovirus" /strain="ChchSNPV-TF1-A"
<400> 17
   atgtatacac gttacagcta taacccttct ctgggccgca cctacgttta cgacaacaaa 60
   tactacaaaa atttgggtgc cgtaatcaag aatgccaagc gtaagaagca ctacgccgaa 120
   catgaattag aagaaaaaac ccttgatccc ctagacaact acttggtagc tgaagatcct 180
   ttcctgggac ccggtaagaa ccaaaaactc actttgttta aagagatccg taatgtaaag 240
   cccgatacca tgaagcttgt cgttaactgg agcggcaaag agtttctcag ggaaacttgg 300
   acccgcttca tggaggacag cttccccatc gttaacgacc aagaaatcat ggacgttttc 360
   ctagtagtta acatgcgccc gacaagaccc aatcgttgct tcaaattctt agcccaacac 420
   gctttacgtt gcgaccccga ttatgttcct cacgaggtga ttagaatcgt agagccgtct 480
   tgggtaggca gcaacaacga atacagaatt agtctggcca agaaaggcgg tggctgccca 540
   atcatgaacc ttcactctga gtacaccaac tcgtttgaag agtttattgc tcgcgtgatc 600
   tgggagaact tctacaagcc catagtttac gtaggaaccg attccgccga ggaagaggag 660
   attcttcttg aagtgtcttt agtctttaaa attaaggaat tcgctcccga cgcgcctctg 720
   tattccggcc cggcatacta agccgtagat tcgttgtcga ctttttcgtt aatttctgtt 780
   aaaagtcttt ctgcttgttt aatgtcttgt ctgctaacgt gttggctatt tattaatctc 840
   gacacattag ctgacatgcg taagatttga tcgttttcgt ttccgctatt ctgaactcga 900
   ttcatagtgt ttaaatactt atttttgacg gtttccacct cattgtcgct gatccaatca 960
   ttattgtctt cggaaaacga atcctcatta ccggtgctct cggctagcgc ttttactctt 1020
   tttgccaggg ccaaatgcaa tactgaaggg gcagtttcaa caatcggttc tttgtccgcg 1080
   ggaatttttt ttacattttt taaaattgta ccagatcgaa tcgcttccat taactgcgaa 1140
   tgtgcgtcta ttggcggtga cgcaactgtt gtgggcttta acgacggttt cgagggcaaa 1200
   ttttcttctc cgacttcttt aaacatttga aacatgggcg gcggtggcgg tggcggtggc 1260
   ggtggaatcg aagtcagtgg catcggcggt ggcggcggtg gcggtggaat cgaagacatc 1320
   ggcggcaaag aaggtggcgg tggtatcgta atatgggttt gcgtcgacga caatggtgat 1380
   tttaaatttt tttcctgagt agaatatatt tttgttgttt cttgtatctt ttgtgtctcg 1440
   gtgattggca taggaatcga tggtgtcgac accgattctg gggtagatga cgccaacgac 1500
   ccagcatttt gattatccag agtcaagatt tgattgaaga tcgaatcgat tttggccgtc 1560
   ggtttttcga cttcttcttg atactgttta tagagtttaa aaaaagtttt cattaaattc 1620
   tctatcttta aaagttcgtt ttcgtggtta attttatcga taatgtcgaa taaaatagtt 1680
   ttatagcggc tgttggcgtc gacgcgggtc accatatttt cgagttgcag aattttattt 1740
   ttcatgcgca cgttatctgg tagaacggca ggtccgatag tgtcccggac gttggccgca 1800
   acatcgatag tgattttagc tgtattttgg taaatcgatt ccgccagacg tagatatttt 1860
   atagccagag ctctgtccat gacgactctg tctccgatga cggcattgcc aaagtcgagt 1920
   ctgcgcacga aatctgtcgg aggttgataa ttaagacttt gtaagtattc tctcacggtg 1980
   acctgtgtca tttcagtcgt cgatcaatat ggacggtgtg ctcgaagaga tgagtgactt 2040
   tttaagcgaa ctcgtgccag aaactaagat taaagtggcc gaaggtaaat ttggcaaagt 2100
   gtccgtatgg agacacaagg acactcaaaa attattttta cagaaagaaa ttactccaga 2160
   gaatttcaat ggtatcgagg tgatggtcca cgacttaatg aagaataaca agttttttat 2220
   taaactttat tatttcacga cgacacttaa aactcacttg cttattatgg attacatcaa 2280
   aggaggtgac ttatgggatc tccttcgaca agagcataaa ctagaggaaa tggaaacaaa 2340
   attaataatc atgcaattgg tagaagcgct ttattatctt cattcacatc aatttataca 2400
   taacgatata aagctagaga atataatata ctcaagatat agacagattt atctatgtga 2460
   ttatggccta tgtaaatcag tcaaaacgga atcgtgctat gacggaacta tggattattt 2520
   ttcaccagaa aaaatcaaag gacatgaata taattttagt tttgattggt gggctgttgg 2580
   tattttgact tacgagctga ttacaggtca tcatccatat aaaaactatt acgatgaaga 2640
   tttgacagta aaaatgttac atcatagaca gcagcacaaa aaattaaaat ttaactctaa 2700
   attaagtaaa gttgttgtaa tgtttataga tggtatgtta aaatacaact ataactatag 2760
   actgaataat gtttatgaca taatgaaaca agagtttatt aatattgaca ttaatttatt 2820
   aaaataaaat tatattacta catacatttt tttgttgttt taaatttatt tacttttttt 2880
   gtatttttta gatttaattg atacatttta aatttattga aatataaaat ataacataca 2940
   cattttgttg tttttattat atacattatt ctattttttg aactgtagtt ataagaggaa 3000
   ttattgtggt aagaccacaa tcctcaacaa tctggacgtc gtcatcgctg tcatcggccg 3060
   aagcaggagg taaaatactg gataatgtgt ctttggccaa ttgttgataa tctatagcag 3120
   tctgctgcag ctgcaccggt tcttctttaa caacgtctag aacgaaatcc gtaggcacgt 3180
   acggtttacc gcgagcagtt agatttaact tgcgcaaatt ttcattattg caagtatagc 3240
   gataagtgac cagttttgtc gacggaggag gctcattaga tgaatttatt gcctccggtg 3300
   tgattacacg caacgcccgt gaatgctctg gaacagttga attcataggc tggttaaaat 3360
   caggattatt gaaggaggcc ggacttatag tatcgtacat tggagatata aaactcgaaa 3420
   attgatttaa cccggggtat ccgttgttgt atgcagcatt gtatccgcca tcgtatccgt 3480
   tgtttccgtt gttataacca ttgttgtaat tgttgtagcc attatatcca ttattgtatc 3540
   cattattgta tccattgtta tatccattgt tatatcctcc gttattgtat cctccattat 3600
   tatatcttcc gttgttgtat ccgttattgt atgaccaatc agccatcggc atcataaaat 3660
   tatgcatcgg tggtgctggc atcatcatcg gtggcggagc tgacatcatc ggcattggtt 3720
   gagctggcat cggaataaac tcagtatcga acgttaaagc ttctggtatc aaagccgcta 3780
   aagccggagt cgctggagct gacgttggca tctgagtcgt tggagctgat gccggtgtag 3840
   ctggtaccga cgttggcatc tgagtcgttg gagctgatgc cggtgtagct ggtaccgacg 3900
   ttggcatctg agtcgttgga gctgatgccg gtgtagctgg agccgctgta gctggagtcg 3960
   ctggagccgg tgtagctgga gccggtgtag ctggagccgg tgtagctgga gccggtgtag 4020
   ctggagccgg tgtagctgga gccggtgtag cttccgtcgg cggctgatca gtggtcatta 4080
   gttgtgaaga taatgattct atcggcgagg ttggttgcag tgtggaaacc gctgattctt 4140
   caactcccaa ttgtgttaat tgggattcta tagatttttc tgcagtagcc atatttactc 4200
   gcgaaggaga cgaaacttca gtattattgt tatcaacatc atcgtcatca tcatcatcat 4260
   cactactatt gctactacta tcactattat caccatcgtt attgtcatca tcactactat 4320
   cactactatc accactatca ctactattat tattagtttg attatcaatc tgttcgtctt 4380
   gttgattttc gtcttgttct tcttgttctt cttgttcttc ttgttcttct tgttcttctt 4440
   gttgattttc gtcttgttct tcttgttgat tgttttgttg ttgattttcg tcttgttgat 4500
   tgtcgtcttg tttttgttca ttttcttgaa ctaatactgg tcttgcagca ggttttattg 4560
   tagcagttgg tttctcaata attatctcag actgtggtag tacagcaggc tcagattgag 4620
   gtggctgagt ggcggtagta gatggaacat catcattatt atcatcatta ttgccatcat 4680
   tattactggg gccaggcgat tgagatgacg ctggaatatc atcagcgggc gcttgatcat 4740
   cagcgggcgc ttgatcatca gcgggtgctt gatcatcaac gggcggcggc gatggcgtcc 4800
   atcgcatcca acgtggtggc ggcggtggag attcatatcg gaatgaatta atttgtttgg 4860
   tttgatagca aaaaatacat tcattgtatt tcatcatact atcgtaggta cagcatatgc 4920
   ataatatatg ttcacagtgt ggatacacaa atcctttatc tccattgttg caacttatgc 4980
   acggttcagt cataatcttc agcttgttgt ttctcatgta tgtatatagt acagaactga 5040
   gtctagatga tttttctcga agtgcaatgg cttcggtttg aagcgcatga gttattatcg 5100
   tatccagtag atagatattc ttttttatct ccgcatctac ggtgatagtg tgaaaagaag 5160
   agccaatatt cttgaatttg tcgtgcatca gaccacattt gatcattaat tcttgtctag 5220
   tgctagtatt aacgttttcg aaaatcgatt gtataaacga aaacttagca ttctcatcct 5280
   cggaaacagt atagaatttt ttatattcag ataaacattg cataatatgc tctaaaacgc 5340
   attgcttggt tagtattgtc aagtaagtct ctaattgctt gcgaacaggt atatagttta 5400
   acgattggat atgcaagaat aaatcttcat aacggttaga gaaaaagtgt ctagatttat 5460
   atgtgatgcc agtaaacttg aattgtttgt taatacagca cacatagcct acgcgctggt 5520
   tatttttaga gtttaaattt aaatagataa acactttttt ctcgaccgta ttatggtgca 5580
   ggtgaatatt gttatgattg ttactgatca tatctccagt gggtaatatt atagcagtag 5640
   tattagatgg gttttcatta tctaccacat ctaccacggg agtttcttgt ttttgacgct 5700
   tgctgttaga cgcagcatca ctatcactct cattatcgat atcacgctta ggtgtttctt 5760
   ttttctttag tttcttcagt atttttttag ttttttttcc ctttcctttt cctttacctt 5820
   tttcaatttt ttcattttca ttatcactgt cactttcact aataatttta cgtttgcgta 5880
   attcgatttc ttcttcttct tgatcttgtc ctttattttt ttctttttta attttttcat 5940
   aatcactgtc attatcactt tcactaataa ttttacgttt ttctttttct tgatcttgtt 6000
   gttcttgatc ttgttgttgt tgatcttgtt cttgatcttg ttgttgttta gatgcgctgg 6060
   cgataacgtt atcgccagca gccgattcta ctgtcacgcc atttgaatct acgtcgtgag 6120
   tcaattcatc catcttcagt atttttttag tcttttttcc ctttcctttt cctttacctt 6180
   ttcctttttc atttttttta tattcactgt cattatcact ttcattaata attttacgtt 6240
   tgcgtaattc gatttcttct tcttgatctt gtttagatgt gctggtaatc acgttatcgc 6300
   cagtcacgcc atttgaatct acgtcttgag tcaattcatc taattctttt tgaattttgt 6360
   taatttttga cgaataattt tcatttattt tatcaaaatt tttatattct ttaaaaattt 6420
   cttcatcaga ttcggtaaat ggtatgggtt caaggaaccc tattataggt tttttcattt 6480
   cacgccgaat ctcttcttca gaaaaattaa acctccgtag agtttcctct ttcaattctg 6540
   cattcgatag cgacgcagta tatttcattt tctcaaattt agatttaaat ttccgtatca 6600
   ttatgatgat cacaattaac cagtatagca cgtatagaaa aattatactg aacaagtttt 6660
   aaataaccaa cacatatata ataaaagata aaaaaattat gtgattagat agagatttca 6720
   tatcatatca taaatatgat atagctcgag ataaactttt ttgtcgtgca aaaaagtact 6780
   ctaaagtgta gtatatttac agcatattgt acagtgtaga ctattctagt taaatagtct 6840
   acgattagta gattttgttc tgtatattga tgccgatacg ataacgaact tttttgtagt 6900
   gcaaaaaaat acgataacga ttatcgatag cctatttgat tagatatagc catgtttgtt 6960
   atctttcgat ataaagtaat atgtttaata aaatatatta gtaaaatata tcagtcgtgc 7020
   cgggtaacac aaatatcgat cgaaagtgat aaaatgccga aactcaagaa aattccaaaa 7080
   aaaccagtag atgaccacct aggaatcctc agatatttca agattatcag aaagccagtt 7140
   tgtgctataa acgaattacc tcctaaagaa gatcaaaaaa aagaagacga catcatcatt 7200
   catctttcta gtgacgcacc tgataattca ttcaattttg attttgaact tagtgacgac 7260
   tcaagcgata gtgactctga cgattcaagc gatagtgact ctgattcgtc taacgcactt 7320
   agtggcctag ataaggtttt gttgaattcg tctaataata atgctgctga caagaatgct 7380
   atctttatat gtcctaacac tcaaaagtta aacagcagtg gtagtgccat aataggcatg 7440
   aatcgtccta atttgactat agaagattca gataatagca atgatgagga agtttatgta 7500
   cctacttcgc ggcggccaat aaaacctgca gcaaattatc agaagataaa gcagcaacta 7560
   aagcagcaac tcgaccaaat attatatcgt ttgtgagtga taacaatgga acattatcta 7620
   atcaataatc aataaaagat taatattgtg tacgtgatgt ttttatttga ttataactga 7680
   tatgtcaggt ataaaagcgc tcaaatcgca gtcaggacca tcacaagtat ttgtgccttg 7740
   ctcccgagca gtcctcctct atactttttg ttgtgcaaac tacaattttt tttataattc 7800
   atcatgatga tgtattacat caagcaagac attgaacatc taatgtctgt tttgctgaag 7860
   gaaaaaccca aacgtgaaat ggtgttaaac gagcggaatt cggagaaaag ttacttgctc 7920
   agcagaatac ccaaacctac tttgttaaag gataacaaag tgtttaatct ggtggccgat 7980
   atctttcatg aaagtaaaga agagcttaat cataaagggg cggaaatcgt gtacacatca 8040
   tcggcttcgg tgcctagtgt gtcagaagct aactctgact ctgattctga ttcggatgac 8100
   gatgatgaca ctttagaaaa atttgtgaag atcaagaaaa atgtaaatgc tgttttctcg 8160
   cggaccgtta aacaacttaa aaaacaaaac aaactaataa aaaaatttta ttcagccatg 8220
   aataatgttg gagagggtga gtcagaagaa acgagacgtt taaataacag aagaatgata 8280
   gcctacaaaa acaacggctt gagcattgcc aatgttggtg atatagtaga taatctattc 8340
   agggcttatg ttgaaatgac ttcaggtatc gtacatttaa taaataatat aaatgaattt 8400
   gtgatagata gaccatatag tgatttcttc gatggtaaag aaccatatag tgatttcttc 8460
   gatggtcaag aaaatcttcc tgatgagaat aactccaatc atcaggaaga tcaagcggga 8520
   gatctaacag atccagaaga ttcaaatcaa gaggaagact ccaacaatcg tcaggaagac 8580
   tccaacaatc gtcaggaaga ctctaacaat tatcaaaaaa atcaagcgga aaatctaaca 8640
   gacccagaag attccaacta tcaggaagat atagaaaatc aagctgagtc aagcggcttt 8700
   gtgccaaggg attcaatttt ctataatatg tatggtgatc aagatgatga caatgaaagt 8760
   gtagcatatg atgacattgc agatgttaat gaagtcaata acatttcaga aaatgatgtt 8820
   tctgaaataa atattagcaa aatgaatatg caagacgaac accaggaaga tatagaggaa 8880
   gatcaacaag aaaaagataa aaatcaacaa gataaaaagg aaaatcaaca agataaagat 8940
   aaagatcaac aacaagaaac aaaacgtgcc tatcaagagt gggctttcaa cgattatcag 9000
   acatcatcac acaggtccga tagcatctca agttctaatg atgactctgg cagtgtcgac 9060
   aatacaactg ctaaaagaaa cagtaaattt gtatttaaaa cttcttctcg tgctcgtcgt 9120
   gacatacgcc gcgacaacac agtgcgtcaa gctctcaaat tttaatattt ttgttttgta 9180
   tacttttaaa tctttttgtt gttttatata atttcatata agcttttgtt atgttttttt 9240
   tgccaattta ttataataaa caaacacaaa ccaaaactaa tttttttctt tagatttttt 9300
   tagaaatagc attttttctt tacaccttta taaacaatag ctcacgtctt gcttaactga 9360
   cagggttaca attaacttaa ctgccagggt tcgattccct aaaactctat aacctttttt 9420
   attaagatac atcaaattaa aatgagtttt tttaatcctc tacgaaaaat aaataaaatt 9480
   tatcctaata caacacaatt taatttagac aacttggctg tagttaataa tgctccaagt 9540
   ggattcaaga atgttcttaa ttctactagc attcaaaatg caggaaataa tcgctttatt 9600
   cccggctata atataggcaa taataatttt atcagtgcag ctgatatgaa taaaataatg 9660
   cgcaataatg atgtcaccag catacgtaat gtttttggta atacaccaac tcaaactgac 9720
   ctaaatggcc tttcattgct gcgtagagca gataatgttc ctgatgcgaa ccttcatagt 9780
   gcggccatgc gtaggaatgc agtaaaacaa aattacccca acaccaatac aaacacacct 9840
   gccggtgtaa gcaatgtgtt agaacaaaat cctcggttaa ctaatcattt acagacatta 9900
   aaaaatgctg gagtggtaag tttgattggt gtcggtgttt acttgacttt tagcgcagcc 9960
   actctaattc aagacataat tgaagccctc aaccgaaccg gaggcagttt ttactatata 10020
   ggaagcgacg gcggagataa catcagcgca tgtttactca tggatagaac ttgccgatta 10080
   caacatcctg aggcaataac tgacgtgaga aaatgcgatt ttgacccttt aatatcgaac 10140
   gcaacagaac tgcagagtat ttgtcaaggc tttaattatg aactagaaaa atccgtatgt 10200
   cgggctagcg accccaacgc caacataaat acacctcaat atgtagatat atccgactta 10260
   gctcccggtc aaactatcgc ctgtgtagaa ccctacaata tgggtgattt aataggagat 10320
   ttaaatttag ataatttatt gggcgaaaac ggcttgatta ataaatcttc aaataaaagt 10380
   caaagtttaa gcgatagttt attgccgttt attgtaatga taggagttat tgcatttata 10440
   attatcattg gatattttgt tatcaaaaga ctgttcaata agcaaactgt gagcgtagaa 10500
   actaatagaa ctcgttgagt ggcttaatca tgtcactttg tcgtctagac gatttacgac 10560
   tgttttattg tcatggcgat tcataataaa acaattatgt gtagcccgaa ggatttttat 10620
   tagactttcg aatgaataca tataattttt atacattaat ttttttataa agattcatat 10680
   cgtaatagta gatacgtttg ggcttgatgg ttttaacttt aacacaaacg ctataaaatt 10740
   ttaaagcaga cacgtcgagt ttgtttctag tgaaaaacat ggcatccgta aaaccgcact 10800
   gagaacaata cagcactata tgttttttta gatacatttt atgtcgagtg cagagaatgc 10860
   aaaatcgctt acacggtata ggaaaatata gtacaaaatt aaaagtagtt ttgtagactt 10920
   ttttttcgac cgaatagaaa taggtgagca gcttctgttc gcccatgctc gcaaaaaaat 10980
   ccatcatttt gctatcgccg aaatatagct gaaaagtgtt gtcatcgcag ccgatcttgc 11040
   tagaagtagt cttgatgatg caatttctgt cagtttctgt aaatagagtt cgaccatatg 11100
   ttcgcaataa aatttttgtg atttgttcgt tgggcttttt tttatccact atatccttta 11160
   ttgtcccaaa gaaatcgtgt cgacccacaa ccgaaatctc ttctgtagta caagtgtgtt 11220
   cgagattaat attgaacaag tagcatttaa aaaagcccaa ttgacacaga gtctctgctt 11280
   cttcgagtat aacatatgga tatatctgaa acaggtcaaa ctggtgttta tatttaaaca 11340
   gttcgtaaca tttagagcag atcaatttaa acttcttgtg tctctttaat tcatcttcaa 11400
   tgtctatgct gttgtcgatg aggcaaaaga gtgagaaatt agtctctcgc ttaaactgtt 11460
   tcgagcattg gttacatgta gccttttcta caatttcttc ttgcttcaaa tctctacaat 11520
   tcatctgtag caaattgttc acttttaatc ttccctgaac gtaatcggac gcaaactgaa 11580
   ttatcgattg tagtagacaa taattcaaat gactcaagaa tttaggtctc aaatccttcg 11640
   gagggctttg aatgatgggc gcctgtctat aatctggatt gttggctcta gtttctcgaa 11700
   ctatattcat caggtggtct atcagcatgg gtttcctttc cgtttttgaa gacattttga 11760
   ctgttgtcgt ggaatacgat tgggtcgtcg atgacatatg accgatgttt agaagcgcac 11820
   gatagcttat gaaaataaat acgagaagcg aacactttat acacgctctg ttgacctcgg 11880
   cgcgaccgta gagttataaa taaacacata atatgcgcgg gaatttgact ctacaaaggc 11940
   gtcacccgtc aagtttatat tattacgaga cgcgagacaa tagcgcgatt catttacatg 12000
   aagatgctca tcctgtctgg tacgtgattt tgtagtcatg gacccagtac aaaagcttat 12060
   ctcatacgat gttgtggtca gtcatcatcg ggcgattgtc atgaacactt ttgattatca 12120
   aaacgatctt tataagcgta tcgcaatgga aagtgcggct gtggccagca ttctattgtc 12180
   accgcgaaac tctgacgatg gaaacagtaa tagtataaat agtttcttga acattagaaa 12240
   caacaatgga gaaaaaggct cgaccgattt cgaaacacag gtatttaaaa attttatttt 12300
   taacgacctg cacaccaacg atttagcgtt gaatcccaag gcccaatttt tagttaaaca 12360
   ggcctcattt aatgtgatag acgagatctt tcaacagaca tataacgaaa aaatcgacaa 12420
   gattctggcg tttgtagatg acactgacga cattcgtatt cccaacgata gatgtttaca 12480
   ttatttgata gcggaaatcg gtaaaatcgt taatgtttta cagcacgtta atcgcatgtc 12540
   aaagtttgaa tatagtatgt acgtatttat gccgtatcta aaacaaataa gaaaaactat 12600
   cattctattc gtcaatgact tttgctgcaa aaaactcgtc gaaaactatt tattaacgct 12660
   cgatgtgatg attgcggact ctttaaaatt actagaaacg attcaaatga tcaataaacg 12720
   tgtagatgtg atgaacgtgt ttctcgatcg acccgtgtac cggtgcaaca tctgcgaaga 12780
   tactagttta gaatcgcgtt tcctcaaacc caacgaatgc tgcggctata gcatctgtaa 12840
   catgtgctat gccaatttat ggaaattttg cactctttat ccagtgtgtc ctgtttgtaa 12900
   gaccagtttc aaatcttcag gctctgcagc ttcttctttg tcgtcatcgt cgtcgaccaa 12960
   gcatcaagct ctattcgaag aataaaatat ataagattta aatatgagtc taatcggcga 13020
   gagacagact ttggaaaaaa agcacatgaa acacttgttt ctggcatcgt attttaatct 13080
   aaacaattat gactatttac cggtcgaaac gaccgctttt attggcgatt atttacgcaa 13140
   taatttcgaa agactagacg actcgactct gttgcaatat attaattatt tgaatagtat 13200
   cgccctcaaa aatctcgtag tcgacaacag tgcagacagt tttaaatatg taaagccaca 13260
   gtttaagttt atctgcggtc gcaacaatat cgacattatc gagttcgatt caaaggtttt 13320
   tattaaaccg aaaaccccaa tctacgctac caatttattc gtaaacgacc ccaaacagtt 13380
   tcgattgatt ctctatcaag aattttcaaa ggtgtttaac gatcgattat ttgtcaacaa 13440
   tggtgacact tactgcataa tcgacggtgg cgaaggctac attttcgaag acgcctacat 13500
   cgattggtgc gggattcgca tgtgtagtgt gcccaaagtc gaaaacgatc aatatccgta 13560
   tcgattgtat ttgatcggag aacccatggc tcaacatttt atcaggaaca atatcacgat 13620
   gctcgcgggc agcgactaca ttctcaaaaa ttttcacaaa ggattgccgc tgtttcgcaa 13680
   caattatcgg gtgattaata gtaaaaaatt tgtgacccga aagcctaatc agctttttgc 13740
   agaaatgcga gcggaactgg acacgcacac gaattatata aaatttattc aacgtgacta 13800
   catttacgat gctaatttcc ctgaagattt gctcgatctc ctcaacgatt acatgaccaa 13860
   tacatctcat tacaagttta tcaccaagtt tatcgagccg ggcatcaaga tgggcaacag 13920
   ttatagcgag attgtggtcg atcgttatgc cgttaataag tatcgaaaat taaacataaa 13980
   aagcgagccg aacacactct tcccggcgct gcgttacaac gatccgtcct atatttttgt 14040
   gagaccagat atcaaacaaa taaagggcac tttgaacgct tttttcgtgc cgaaagaacg 14100
   tttattggtg atcttggcca acagcagcct gttcggatcc accgagctga ttcattttaa 14160
   taaacatcga aacctaaacg agctggtgca tttcgatagg agtttgctgc cgtatagaca 14220
   gttttcgcct ccgcaggtac taacccttga cacgtatatc attgacgcca gtcacaaatt 14280
   gtatttaact aagcacattt ttggtaatac agtgcctgca tatttaatta taagaggaga 14340
   ttacgaaagt tctcaatttc aagatttgaa aaatctgaaa aactcttggg ttttgaatac 14400
   tctacttaag ctgtttatag atccacaata aaataaaata tggaagattt acgttcgcca 14460
   ggaactacga gtcgattcaa cccgaacatg ttcaatccca gcatgttgat gaccattctc 14520
   atcggtctag taattattat actgctgatt atgctcttcc aatcgagcag cgcgggaggc 14580
   ggtagttctc cagcagcggc gcctcaagcg ggctttacga atccgttgaa tgccacgatg 14640
   agagcgaatc cctttgtgaa cacaccacaa cgagcgatgt tgtaaagcgt tctacgagaa 14700
   ttataagaca atataaacaa aatgaaacgt ttcaagtgtc agacagcacc aaaagtgaga 14760
   accgtcaccg aaatcatcaa cagcgatgac aaactacaaa aggaatacga tctcaccgag 14820
   tttgatgtga aaaacctcaa cagtctcgaa agttatgata cgttaaaaat aaaactagtg 14880
   atcgtcaaat atatggccat gttgaatact ttgcagctaa ctcagccgct gctgaccatc 14940
   ttccgagaca gaaacgccac tcgcgagatc gttaccgttg ttttggcctc actcggcttc 15000
   gtacacaatc gcgtaaatcc actagtgaat aattttaatc ggaaaatgga gtttataatt 15060
   gtggaatcga aaaacctgat aattcccgga gaaccgattc tatttcgaca caacgaaaat 15120
   gaagatatcg tttgtattat cgatcgagtc agtatcgtca agatgctcga aaagcaattc 15180
   gacacggaca tgaacgtcag caatattatt caagaacatc aaaaacttaa actcatcaag 15240
   tcgtttacga gtgtaaagaa acgaaaatcg tttgacgatc aggacaatag cctctacatt 15300
   aagctgaacg aaatcgaagc cacccagtac acgactttgc tgttcatcat ggaacacgct 15360
   tacggacact attatattct caaaaactat ggcatctaca attatactca gtcgcttttg 15420
   gatcatacca tttttactca aaaatacaag ccttctttga atgtcaactt tgccaatcta 15480
   cttttgagta aattcagatt taccgtcgag gaatctgagg gcaatcgaag caaatcttgc 15540
   ggcaacaata gtttgtattc ttctaaaaat caactagaaa tattaagtta tagcagcgat 15600
   aaataaaatt catgtggctt ttactagcga tttttattat tgttaaacta ttagtttttc 15660
   ataaaatgca aaatatgcat ttagacgtac acaatcataa actttgtccg aatggatatc 15720
   acggcctcgt aaatgatcct ttcgattgta acgtttatta cagttgtcct caaaaaactc 15780
   aattttattg tcctcccgat gaacaattcg atgtcgagcg acaattatgc ataccaaacg 15840
   atattcatga cgatagctgc atggccagaa aatacaaaag cttacttctc taatttttaa 15900
   taaaactcga cattcgcaca ctagtttttt tttggttaaa cacattcgat cctgacgttt 15960
   tctctgttaa tgcagacatt tggatataca aaagctgcca tttcaatgtc tttcgggttg 16020
   gtgtgagttt tcgaaagtga tgctataaaa tgcgatggta tgttgccgcg gagaatgacc 16080
   agttctttag cgaaataatt ctcaattcca tcgacacata cggtcacgcc tcgcacacgc 16140
   tggaaagaag ctggctcgtg atttttatta actgtaaaaa tgtgccacac ttgtatttcg 16200
   cctttacagt cgacgacggc cggcaacact ggtctcgaaa aattttgatg attcaccaat 16260
   ctgacgcaaa aaaacggtaa tctgacgcaa ctaattacat acgcgtttct aatggcagtg 16320
   cgatcgatcg gcggcaatag acacttcatt cgtattgcaa tattaactcg gtcagcgcgc 16380
   ccgcgttcgt agccgcttac gagtcttgtg gaagcgaaat tgcggttatt accatcgccg 16440
   tcagtgtcat cgctttcgtt tatttgaaac ggtatgatat ttatcgagtt gatcgcattt 16500
   atcacgctaa atgtaattag gctatcttta tcttgaggat agtataaatt gacaatcatt 16560
   ttatttttac ttccagttct cacttacaag cgttgtgatg tcgcatccaa acatcatcag 16620
   tgccatggac aacaacgacg cgtctatcaa atataaaaat tatatcgaca acgccatcaa 16680
   cactcctacg catacgattc tgcagaacgt cagtatggat ttcgacgaca gcaatattct 16740
   ggatttcggc aacgaaaatg atatgaatgt ctacgacaga cgagacagta acagcagtaa 16800
   aattgagaat gatgcttgcg atgaaaactc tcaattttct gatgtcaacg tcaacaataa 16860
   tgcggacaac aatgattata taaaaatcat gaaaactgct accgatgtcg tcgaaaacaa 16920
   gaatgaatat acgaataaac ataaaactgc agtcgtttcg actaaaccat tcaagaaaaa 16980
   tcctaaaaaa aggccatcgt catcgttgac gactacgacg atgacgacga cgacgacgac 17040
   agaaaagaag aacaagtcaa gacctaacag acctcccaat tccactgtaa tcgctgaggg 17100
   tagtattcca ccacaacctg tgataaagcc atcgaagaag cagacagttt ttgtttcgcc 17160
   tttaatcaat agaggaggaa aaaacttgaa tgttttgcgc aacgacaata ataataattt 17220
   caataatgac agtgacgata gcaacggaag cgacagtgaa gatagcgatt ctacgcatcc 17280
   gccgccttcg aaaaagacaa aaatgacatc aaaatcatct aaaatgtcgg tgacgccgca 17340
   acaacaaatg cccgagattt tgaaaattaa tactgccgac aaaaataaag tcaatgacga 17400
   gaaacaaaca gtgaaatata acaaaaaaaa gcaacaatct caagacgctg gtgccgtcgt 17460
   ggtcgtgaaa caacaaaaac ttgataacga atcaacaagt caaacttccg ttaatgatga 17520
   tcaacaacgg tcgaaagatt gcgattctcc aacaaatgac ttgtttgaaa ataaaataat 17580
   ccccaacatg atgaccatgg aaagagacaa taaccgcaag tttgtgcaat atattctcaa 17640
   cgctcacaat tatctgttta tagtatacga aaacaagtat aatgccaaga cttttaacaa 17700
   aaactccaac gcatcgattt ataaaataga gtatgtgaat tgcgtccagt ccatatacaa 17760
   gtattataac gccaattact cgcatatcga tagaacatgc aaagtcgtgt ctttcaatcg 17820
   attcagattc gccatatctg tgaacctttt aaataaaatg cagattgaat tgcctcctac 17880
   ggaacaattt aaaaaggaag acctcaagaa gatttctccg aagaacactt tttgcctatt 17940
   aaatgaagtc aaagatccgg atttcatttc caagctcact aacacattcg gcttggacaa 18000
   tatttatatt cagggtcaac tcactatgct gctctcgtcg attggtgaga atcgggcaaa 18060
   gattttgaat cagcatatca gtgcaatgat tgaagataaa agcctattca ctataccttt 18120
   gcatttgtct cgatccaagg aattggaaga aattgttgat gacgatctga accccaacaa 18180
   cagtaacgtc tcgtcggcct acattcgaga cataatagaa ctctcgaaca aactcaagtt 18240
   taaggctcct attattccgt catatgtcta taaaaccaag gaacaaaaca ttgagaatgt 18300
   tcttagtttt tggatcaaca ctcagaagaa caacaacgag cgcgataaaa ctttggcaaa 18360
   atctctgcag tttacataca agtttaccag tgtcgctcga gtgctcttcg acgaaaccga 18420
   tggcgacgtc aataaactgt ttaaagtgaa gaaagagcct ggatctgtgg caatgattga 18480
   agattatcta caggcttgtg aaaaaatacc caatggcaac aactttatta tgatcaacac 18540
   actcaacgat gaacgtgtga caatcatcaa ggccaaaaat gaattctttt ggatttgtac 18600
   taataatcct aataatttaa ttcactgtat cgatatcatt atggccttta aaaactttaa 18660
   tcatcatttg ctctctttga ttcccagcaa tcgtaaggat ttgaacaatc gtcacagtgg 18720
   attaataaag ctagtggcct atcatttagg tggtgatgtt gacattaatt ttgtacgtgc 18780
   catgactgag aagtttaaat gtaattatct atataaaaaa ttttaaatgt ataatttttc 18840
   ttgtctactg taatttgaat aaatttttaa ggatcgtatt ttgtatagta ttgtagaatt 18900
   agtattatga tcaataaaaa tttttttatt cgtatatcat tctatggttt atttttacct 18960
   tcgaaattat gtccaataag aaaataatca tgtcgacttt gaccgctatc gatatcgata 19020
   acgcattcaa gtattcgact catatgcata ggctgattta tattcacaag tggagatcta 19080
   aatttccaaa catttttatt aattacgaaa ttagagcagc aacgaatgac gattattatg 19140
   tgcccctcgc cctagccgac agggccattg ctgttaaact cgagtttagt aaagagggat 19200
   gcgagagtat ttcttgctat ccctataacg aatatgggcc tgtggatttt aaaacaccta 19260
   cgggctacac tcaaacgtcc gatgtgcccg tacaatacgc tcagccggca tgctatcatt 19320
   tggacagagc ggcggctacg cgacaaggcg cagagaatga agtccaagca cccgaattga 19380
   ggtataccga cggcggtaaa tgtattctag tcgataccct aactaaaatg tacttgaact 19440
   ctccctacat acgaacggac gagcatctga ttcaaggtgt cgatgatgtg cctggattta 19500
   atgtcgtgcc cgacacagat cccctatttc ccgagcgatt caaaggcgaa tttaatgaag 19560
   catactgtcg acgcttcggc aggtctctgg tgaacggcgg ttgctcccta cagtggtggg 19620
   agagtttgat aggattcgtt ttaggcgata ccatttacat tacatttaaa atgctgatca 19680
   ataatgtttt tagcgaattg cgagatttcg attatagtcg accgtcgcca gagttgccga 19740
   caaagcccgt cgtcgattcc gaacgactct taaacgagtg gaaaaaccgc atcgacagcc 19800
   gttctaatct tttggatgaa ataaaatttg ccgaatttaa aacctattcc gatttaacga 19860
   tcagtagaac tcgaaaatta gtttatattg cggaaaaggg atttcatcat gagacgatac 19920
   cgtttaaaca attggaattt cgaaaggtgt cgaccgcttt ctcacgtacg aacatcgaca 19980
   acacagcgtc tttggacgac attattactc aatttctcga ggacaattcg ttactcttgg 20040
   gtctggcaac tagctacgga ttcgatgtca ttttcgatca attgaaggcc atgctcaaaa 20100
   gaattaacac cactctaatt ccagccatga aacggttgct tttggagaca tcgaaacgtg 20160
   tcacggccaa gatgctcggc gaaacttata aagctttcgt ggcccacacc ttcaatcgta 20220
   tcgccattaa aactctatcg accgtagcaa aggcgatgac aagaataacc atcaaagccg 20280
   cgtcggtcgt cggaattttg ttaattattt tcacaatatc tgacctaatt ttagcttttt 20340
   gggatccgtt cggttacagt aatatgttcc ctcgagaatt tccggacgac ttgtcgcgtt 20400
   cgttccttgc ggcctatttc gagtcgatca gcgatggcaa tctagacatg atagaatggc 20460
   tacccgagta ctttgaagac ctcatcgaag aggacgatgt cgccatgttt gagaatctaa 20520
   aatccacatt tgagtatgtg acctctctcg aaataaactc tgacggacaa ctgttggagt 20580
   tcgatagagg cgaaagcatc gaagatttcg acgaaatctc gttaattgga aacgctcttg 20640
   cgtccagcgc tctctacaca cgactcgaat ttctacaata tacggctcga cacaataaaa 20700
   tattatacgg caccgacgac ggtattgtcg tcaacaacgc cggtaataac gttattttgc 20760
   cgacgctttt catttttggc gcagtgatca tcatatttat acagagtcaa gatactattc 20820
   aaaataatat tatggtatcg ctattcatcg tatttctact gattgctgtt tatttaatca 20880
   tacagaattc tttattatac tacttgagtt taaggaaatt tacgagtagg acacaggcta 20940
   aatggtatga taatttatac aactaaatag agattaaata tatttttatt caacaaatcg 21000
   aacgatatga ttacttagga gaggacttct tttttctgag cggaacatcg ggcacgtcgg 21060
   ggatctcggg tatctcgggg ttgagaatct cagtaatgcc gtcgacggct gtctgaagag 21120
   tggcgagact cgcgctttga gcgtcgagtt tctcgtttaa ttctgtgaca tcgggtagat 21180
   tggcgcgaac gtcctcgact gccgactgaa gaacatcaac tttctggctg acggcttgga 21240
   tgtcggcacg aatcaacaac aaaatatttt gagacatgtt ttaatttttt taatatttat 21300
   acgataaatg gatctatact tattatatgt aattttattg agggacaatt aacggaccgt 21360
   ccaatctaat tctgtctatt tcgtattcgc cttccaccaa agagatgctt atctcttttt 21420
   tattgtgaaa aatggcaata tttctaggta gatcgcgata tgttaaacga tttgccgtcg 21480
   tggacaaagc aaactttttc acggtcatat acggcgaaga ataggccatc tgtcgtttac 21540
   cataaaccga catgccatta cgcaacaaac tgacatatac tgttgaatcg taattaattt 21600
   gaccagaaac caatcctttc ggccttatgc ccgacactgg aaacagcaca attccagcgt 21660
   cataatcgtc ataacgacac gtgattacag agaccagacg atcgttaaaa aatatcggtg 21720
   cgcctatcca aatcttatcg gcaatccaca gatcgtctac agcaaaagtg cgcaactgtc 21780
   cgtagatcag gcgattggcg tgtctatgaa aattagtata gacgtgttcc ggctgcactc 21840
   taaacaatat gccgtcgttt agcaagacca tcaaactgtc gttcgagttg tcgatggcgg 21900
   ggaacacaat atcgctggcc accccgggaa aatgatgaag aaccgacagc ggcggctgat 21960
   cgtcaccagt ttttatgtaa ttcgaatcgg aatgcggtcg aatcgtctca attctgacag 22020
   ctttgccgtc gacggcaact acacgaataa ttttttcatt ttcatcaata ctatattcta 22080
   catttaattt tgttctccga gtcgtcgtcg gtgtcgacgc cattgacgct gacgctattg 22140
   tcaacataaa tgacaagata aattgagatt gaatcatttt atataatacc ttatcttgtg 22200
   ttttttctcc taccaatctg ctcattaata gcatatttaa cctctgacat tacgagttga 22260
   gtatatatta attgagatga atctttgtcg attattaacg aacgaaccga cgaaacgaac 22320
   gagccaaaat gcctgttttt aaaggctcta acagcaaaaa aaatggagtg tcgacgagct 22380
   gcacttctac aatcactaaa aatacatgga cggacactag aaacaccatg gtggtcaaga 22440
   gtaatgacga gaatctaagg aaaaaattaa acgaaatcct tcaagccaag aaacaactca 22500
   gcattcagat ggaacattgg gaaagaatca agcgcataac gaaggatccc aaagaaatag 22560
   cagccatcga atgtaagctc aataaaatta gaaatgattt tctaaatttt gggtcaaata 22620
   atttctagtc atggataaaa caaataaaaa gtgtgtgtat aattttaaaa aggagtttta 22680
   ttataatttt catcattaac accaccacca ccaccaccac ctaaatctac atcaataaat 22740
   gtcgcagaat tataccaatc gtcatcgtgt ttgctgactt tggtgtcgcc gacggatgtc 22800
   gaatgataat tttgttttag atcatcaaaa atatgatgac agctctcaaa atcatgtcgt 22860
   tggcgacgac ggtcattgcg cgatctccgc cttggcgcct cagttaattt gcacttgaat 22920
   aagctcccgt ccggccagta gactctacgg ttgacagcaa tcaatttgtt ggcggcataa 22980
   cttgatgtca atacgattcg cttgcgggtg catttgtcgt gtttgatgtc agccttgatt 23040
   ctgccgccgc acacatgatt gataaaattt ttaataaatt ttttatcaac attctcgcca 23100
   ttgacataaa agacgaaaca gtcacgatcg gtatagtcgt acattttttt tccttattat 23160
   tcctcatctt ctgcaggttt tatgtgaaaa aataccatcg gaatgatgga aagagacaat 23220
   ggcttttcct ttagtttggt tccagtcaga gtatgtagcg attcttcctt ttggccttcc 23280
   ttcacacctt ctactattga tcccacaaac attggcactt cttcagaagg cttatcattg 23340
   tcctttagct ttaatatttc atcgaagcgt tctatagtaa aagattcaca tttgaccgtc 23400
   ttgtctagtt tgccagtgct gaagagcagt tgattatgct ttcgagaaat atcaaaaaat 23460
   ttgttcacat tctttgcgta ttctttgccc atgttgaagt agtaaatgtt gctaattttg 23520
   cagtcttttt ctttaaaatg catcgatatg atattgccaa aaatctcatt gtacacatgt 23580
   ctgttggccg aacgtatagc taaagaatcg ccatactgac cacgagtacg tttcaccata 23640
   gcgtaatcga aatagaaaaa cgattttaag ccacccttga cacgcatacc gatctcgtat 23700
   acatttttct tgcttgaccc gttgggctcg acgacgcaaa tctgattcag atattccgta 23760
   gtttccattt gcaaatttag cagtttactc agatgatttt caatgtgtat attgtctcga 23820
   atgaatccaa agcactccgt caaaatgtta tagggcgaaa tgcaatcgat tattttatta 23880
   tttacgtaac gttgagacac aattgtattg atcatgtccg acatccaaga actctcggtc 23940
   ttccataaac aaagggcact atcagaaaca tcaaattcga tgttttgagc gacatcatcg 24000
   gaatcgatgg cggctccggc ggtgacaata ctctcgtact cttccctagg tttcttgctg 24060
   ttcgaatctt gatcgttagt atcgagtttc ctcttcattt cgctggcgct ggcgctggcg 24120
   ttgttgaatc gagatgccat gttggacgac gtgtgtctaa agactgatac gtatgaccga 24180
   tcggtaaacg ctttttatac catataccat taatctatct ttatatattt atctagtaag 24240
   cgtaatcatg gagtatcgtt cgtgttatct tttgagcaat agcgcctttt cctcgaagag 24300
   tggtgtacgt tttaaacagt acaaaaatct gatgaatctt gccaaaggcc ttataccatc 24360
   gtcgatagaa ccaatttcga tcatagaatt ggaaaaattt aaaatgataa tcgagcccga 24420
   caccaattac gtttccaata tacacgattt ccatttttat accgacaata aagatttcac 24480
   tgtcgttcac gttttagatg cgaacactag acagtttttg ggtaaactca ttattaacta 24540
   taatatcgat gacgctgatg atcatggcat tttcgggttg gttgccgatc tcgccaacga 24600
   taattccgac gataacgata acgtttatca agatagttta gatattgaac caatataaag 24660
   ttgaaattca gcaagcgtta ctttattcta gtagtggagc tcgctttgga acagaactaa 24720
   attcattatt cgacatggag tacctgacaa atcattcgat attgaatatg atttccgttt 24780
   attttgaatc attcgatgat tttttgaatc tgcagacggc catgcaactg gatcatttta 24840
   ctttttggaa tctattctat ccgggagcca tcaaaaagaa taatatttac aatagtcaat 24900
   atgccatgtg ctatttcgat ttgctgaaca agcccaaggt ttttagaaaa tacaagaaat 24960
   atgaagataa atttgattta ttggtaaccg acgacgacga gtgtgtgggt cttcatttat 25020
   atctgcaaat gatcgattac gattaccagc accaatacag aaaattgttg ccattgatgc 25080
   acgatcgaca ggccacattc gatgccatgt atgaattatt ttgcaagagt gtcgacattg 25140
   aggatatgtt cagtgatgtg cccaggaaaa atatttctga tccggcaagt gaaagactta 25200
   aatataaata tccgattaat gtatgtaaaa ttttaacatc aagagctaca tacgtgatct 25260
   ttaaagggac cgatttggag ctacgggtaa attttaaaaa atatccaaag ctattggagt 25320
   acattcagaa attgcttacg ttcgaactcg atcctgagga gagatgctgt tttgttaaaa 25380
   gtttactcgg aaacaggagt aaacaattgt gcgattggca taacattttt caatattacg 25440
   gcaagtctga agtgctgtat aaagctttta taaaaaataa tgttttttat aaattagtgt 25500
   ttggcatcga aaaggatatt tttcttcaac aatacatcga gagcaccaat catcttggta 25560
   acttgcgtaa aattctgaag aatgacttta aaaaattgaa gtttaacact gaagtggggc 25620
   tcgataatac atttccagtt ttaaaatatg tcgatttata tgtactcaac aaaatttgtc 25680
   ttgagtttaa gtctgttgaa aaaaacacca aaaagaagat taattttctt ttacagaata 25740
   ttttattcat gtataaatca aaaatattat tagataaaaa aatgttgaag gacgtcaaac 25800
   atcttacaaa cttagctgta aatagaattt taatttataa aagagtcgat ggatattata 25860
   aagacatact tgattataat agaaacgcaa tgattcaaca atttaataac actttgagta 25920
   aaaaaattga caaaaaaatc aataaacact acaatttcaa tttaaaagtt tctcgcaagc 25980
   gtaaattgtc tacatgttca gacatttaac aaatgattgt aatgtggttt tgttacatat 26040
   aattaaattt ttataaaatt tttttttcgt ctattcattt gtttcttcat cctcctcatc 26100
   cattgtttcg aacgaaacgt catcgacatc gtcgagacag tcgtagttat aaaaagtgag 26160
   caattcttta atactcgaaa atccggtcaa atccctaaac aagaattgag cgtttaattt 26220
   ccagaacggc acgctcaaat acgagtaata cagttctaca aaactgatca cacccatttc 26280
   gctgtttaaa aactcgatat aagcggaatt gttgacgttc gacgactgat cgaaaacgta 26340
   ttttcgatga aaatcacatc gaagttcggc atttttatga taacacaaac gacaatggta 26400
   aaatttttcc aactgcaaag cgtttaaata atctcgcgct tcgtccgaga gaatattgat 26460
   ttcttcgatg ctaaaatctt cgtctttctt tcgtttcaac aataatttgt ttctacccat 26520
   atgttgaaga acatcgccca gtttcggagg tagcgacggc atcggtggcg gtggcggtgg 26580
   cggcggtgac aatgacgatg atgtcgttaa cgccaatgtt gcggtagctg gtggagtttt 26640
   tggtcgtatc gatgccatca gttttgaggt caatcgacgc aaatcctcac gaaacgtctc 26700
   gtttttattc atattgtcat gcgcgagcgc caataataga gccttaatga tactatttct 26760
   cgctaaatta tataagtact ttttagtcaa acaaaatgca agtgtttgta aaaacgttaa 26820
   ctggaaaatc gatcaccatc gaattagaat caaacgacac catcgagcaa gtcaaacaaa 26880
   agattaccga caaagaaggg atacctcccg atcaacaaag actcatttac ggcggcaaac 26940
   aactcgaaga tgatagaaac gtcagcgact ataacattca aaaagaatct actatccatc 27000
   tggtcctgag acttagagga ggaaaaatat gattagtttg aatcaaatca taaaaatcgt 27060
   tataacaaaa tctaatttac acggcataca caatgtcaag ttgattgacg aaaaaaacga 27120
   caaaattgat gatttttacc aagccgtttt aatcgtgcct ccgggcatcg gtaaggtatt 27180
   ttaccagggc ctacatgtca ccggtcgtgt actcagaatc gaaggaacca atgtagattt 27240
   gatatatcaa aaagagtaaa aattttttag atttcgtgtg tgtactgtga tatagtgtaa 27300
   aatcctctat ttttcgacat tcaatatact ttgaaatgat gtatagaaag aaataaaaaa 27360
   aattgtgtac atttaagttt tttattttac aattaaacag atgacatttg actctcatca 27420
   aaatcgtcat cgacaatttg ttgatatgtc gatgatgatt tcgatgacga tttgtgttgc 27480
   tgtttagcct tttttacatt caaattaata ttagttttct ggacacgttt tttttgctgt 27540
   tgctgtttag agctgccggc ggcgacatga ttgtcatcat tgttggaggt tttaatcgtt 27600
   aatttatgtt cagacgacga cgacgacggt acaatcgttt ccaataattt tttaaagcaa 27660
   tcgacaccat tctgtatcat gtttcgattc aaacgctcaa aaccgacatc cgatccgtta 27720
   ctgtttttag atagcttgat ttcgtataga aagctttgat taggagtcga tccatcagca 27780
   aacgtttcgt tcatcactcg ataaaactca ttgtataatt cagcacgctt agcgttggag 27840
   gcatcaattt tactagattc ggcaacattc ttgatatcat tttcttcaat gttggcgtca 27900
   gaattagtct gttcggagat gactggcgcc ccatcgactt ctatactttt gcctaatttt 27960
   tccataaaat caaaaattaa aacaaacggc ttgatgttcg aatggctctt cataatattc 28020
   cacatcatgt gactgttggg ccagaccgat tgcttgtttt tcgtgtacca gctattgaat 28080
   aggatatact tgttattgct gatcatcttc tttgttttct ttgcagtccg tttgtcgtgc 28140
   aacaaaataa tggccacggt aaactcgaga ccgttgtcgc catacacagg catcatggtg 28200
   attctatact tgattttctt cttctcgagc agttgaattg tggtattgac attttcaaaa 28260
   ttgttcctgt cgaaaactag tttgtcgctg tatttgtcga agatttcgac gagattatta 28320
   cagtttttgg tggagttggc catatcgatt cgaagattgt ttatattcag tttcgaggga 28380
   tttatttaaa tgaaatattc tttttaattt taaatgatga cactgcacgc gcttgcactg 28440
   cgttcgctgt ccgccaacgg ttatgagtgc tttatcaaga ttattcctta tatagtcgaa 28500
   ttgagtagcg aatccatcat cgaacacatg atcacaaaca ttggtcacgt ctcccgtatg 28560
   cttacgctta ttaatggtct ctcgaaccaa agcgtaaatc tcgcttctag tcaagcagca 28620
   ttgactatat ttttcaggtc ttgaatgttc atcattggaa tggtggcgtt gtcgctgttg 28680
   ttcgacgtga tgttgatgct ggaccgacgc gtggctgctg tcgatttcgc attcgttgtg 28740
   gtcctcgaac gcaaagtcgt cgccattgac gatttccatc gttccaatcg acgattcagt 28800
   tttaaagata aaagaaaaaa tccaaatgaa tttttattac caaacttgtc gtagccgttt 28860
   tcgtcttttt cgcagttgat aacgttactt tcgtcgaatt tagctttgac aaatttaacg 28920
   tattcaaaaa actcacgata attactagaa tcgtacgtga tcaattgctt ctcgttcatg 28980
   tactgaaaat agtcttgtaa cgacacgata tatacgtgtc gaggcaattc tctgttgtgt 29040
   cgtcgagtct cgatatcgac tcgatagtcg ttgcctttgt cgccgccgtt attgagtttg 29100
   acacaatacg tattcggttc tcgagataca ctgattagca aaccgtttaa aatgcccacg 29160
   taaatggcgt atttgtacgt gacgcccgag tctatccatt gcaaaataaa cggccgacgg 29220
   tagacatagg cgctttcgaa aaaggtgcca gtctcctcga taaactcgcg aatagcagtt 29280
   tcatgatcga aaatgtctcg attgtcgcgc tttcctctgg gtatagaaat tttttccaaa 29340
   aagtttgctc tttttagcac ggcactgtcg ctatattcga cggtgctgtc gtacgatcgt 29400
   cgagcacaca gcagcaccgc cttatcgggc tccattatca taaacaggcc tgcacttctc 29460
   atcgtaaata aaattcaact actttatata ttaataatac ttttatttta tataaaacca 29520
   ttattatcac ataaaatctt aacaaacata ttagtacaag acatcaatcc tctcgaagct 29580
   gagacagatc attacaaatt tttaacaaag actgataatc actaaaaatt agttcaaatg 29640
   attgtgcgta gtcatgtgga gaaatctcga tgtcgtttaa ctcgaaaaaa gtattcattg 29700
   acgttaaatt cttgtgattg tccaggaaaa atttttgaat caattttctg ttttgatcat 29760
   cctcgtcgtc gtcgtcgacg tcatctccat cttttctggc gtttgaatcg atgaattcgt 29820
   cgaaaacgta aaggacagcc atctgaactc cacgaacgta cggatttgtg taaatagttt 29880
   tatttaaaca cttgacaatc aaatctgtgc cgatgtagtc ataaatgtca gagtcgtcgt 29940
   acttgtcatg gagaactaga cgaattttat tacaaagctg tctgatgcaa tccaaattta 30000
   aaatttgtat tcctggattg tgctcgaatg tttgcagata atacgcttgc tcgtgcagcc 30060
   cggacagatg tataaaacga aggaatttgg aatacatttc gacttgaaat gacgtacgac 30120
   aacaggcgaa gcaccttcga ctttaaatga tgtacgacaa cgggtgaagc gtctatttat 30180
   atgccactcc actctacaca atcatgataa cgagtctgca tctgatcgac aataatattt 30240
   attcaatcgt tgcttgataa caccacacaa attgtacgct cggaataatt tagccatcaa 30300
   cacaagaata tggtttttgt ccgtggattc gatgtcggtc actcgagtca agtggaaaaa 30360
   ggcgctggcg taattattat tcattaatgc cgtgtagcgt ttaaataaat ctctgctgcc 30420
   gattcttttc aaactcgtag ccaaccatag cgtgtacgag agaatcaacc gtttcttgtg 30480
   gtcgatttcc gcattctcat cgtcgccgac gtacaattga agccaatcgt ccagttcttt 30540
   gcgtatattg tctcggacga atcctttaat tttattttca tttgttccgt tcacatgaac 30600
   atctctcact accgctatca acacttttaa tttcttctgt atattatcga gtttgtcgta 30660
   gagttggttg gagtcgggta atgttgtaaa aaagtcaata tttatttgaa tctgcatcac 30720
   gacactatcg agattataca attcgtactg tacactgtcg gcggccaaac aaattaatcc 30780
   gaaatcaatc atgctgtcgt cgacaatttg atcagagttc gactaaacga tggccgtaat 30840
   gtgatccggt tttgattcgc ttgcctttaa tgaaatcaat cttaatttcg ttatcgttac 30900
   agagtattcg agattgggca gcagctcgtt tgatcgaatt taggcgacgg gtcgacagct 30960
   cgagtggatt atatatttct gatttctcca gggtaaacga gttgagatgg ccacaattga 31020
   accattccag tccaacgatt aaattgatca agaaaataat ttcataatca cagtactcga 31080
   atacgaattt attacaaagg cagtaatagt agaatttgag tgaattatat agatagaata 31140
   ggtagttgcc ctgtttcaga taaaattcaa catctgtcac gaataaaatg ttgataattt 31200
   tttcggaaat taaatgcttt agtttttgta gatatatcaa agagtttttg tcctcgtcga 31260
   gtctgatggc acacacgtta tccatgtacg cgttattgat gcaaacaaat tcttgtctct 31320
   tcaattctct tttcagtgca gcacttttat cgttggcaat ttcgatgacg atatattttt 31380
   gagagctctt gtgtctcttt atcacgtccg aaatggatgt aatcgaagcg aatagttttt 31440
   caaataaatt attctcccag cctctggcgc cgacgctggt cttgtcgtta ttgttctgat 31500
   gtttaaagtc cagcccgctc tgaatcagct ttgtggtgat gcgcacgctc aattgttgtc 31560
   caagagtata atgatcttcg tagcctctct cccacatgac gttacaagcg aaagcaatca 31620
   aatcggacac gttttcttta accaaaatct tgtgtaatcg gtcgaaatcg cctttgtacc 31680
   aacgatcgca cgccaacagt ttcagcaatc tcgattgatg ttcggtcagg tcgtttcctc 31740
   tagtggcatc gatatagtgt tcgatgtcgc gaggactcga tttcaacaga tttgtcatgt 31800
   aaaatacgac caacgtctgt tgtatttgca cgaatccctc gtcgttgact tgtaggcgaa 31860
   gatctgtcgt gtgatataga tcaccatcgc agacattgtt gtcgtcgaca tatgccagat 31920
   aaaaagagat ttcatccgct aaaaatttac aacactgagg cagcaactgc gaagttgtag 31980
   tatagtaacg tgcaaacgcc atgccggtca tgaagtaact atctgtcgat tcgatctaat 32040
   cgctatgatt caaatctatt tcaactgacc tagataaaag agggtttaaa tatctcgata 32100
   aaatgcaatt gagattatta aatttgaagc agtgtcctca agacgtttcg aaaatgttta 32160
   aatttcccaa cgaaatgatg gaagaattct tgatgagacc ggtcattcaa caaggatttc 32220
   aagaactcga cattattggc aagataaaga atacagaacc tcaagttttg atggaaattc 32280
   aacgcctcaa ctcgagcggt atatatggca tgttggccgg aggatatgcc gcttttctat 32340
   tgggcctgac cgacacatac actgatgtcg attacttttg tgaagatatc gaaggtgtct 32400
   atcaatatgt catcgctaac aagaatgatt accgagaaca ttcgcatgta ctgagcagac 32460
   ccgatgccgt cactttggtt tcggaaggga taaagtttta tgtggtcaat catgaacatt 32520
   caaagttgca gataatatcg aggcgcatgt ctatgcgcag aagcatcaat tacaaaggat 32580
   acgccttcta tgcagattgc ttacgctctt tcgatttgcc catctgtaaa aaaggaatat 32640
   ttctatgtcc catcgaatgg gtcgggcaaa tcgagtgcat cgaatacaca aacgattcgc 32700
   tgcgattcat catacaaaat tattctccaa ttaaaccatc gggaaaatat gaagcatcgt 32760
   caaaggcgat aacacgcaag gaaaaataca gcgctagaac tgtacaccac ggattgccta 32820
   ctactctgaa agaaatttgt caagacgcaa tactttatca cagcgatcaa gcaaacactc 32880
   aagattttgt catttattga aaatattgta accaaccaaa aatatattag tttgcttctt 32940
   tattatgttt gcttttcatt attgtttgat tttattttgt gactaataat aaacgaaaat 33000
   taaaaatatg tgtatttttt atttgttgta attaagtcga atgacattga gtaaaatgaa 33060
   aacacaatac tcaaactgca ttatttgtaa aggaatggtt tatgtttata aaaaatacac 33120
   acaatcttta tcgtcacaaa tattttttca taattatcga gccatttata ggaaaaacat 33180
   tggatatcta tgtggtaaat gttatcgttt acatgtaata aaaaaataaa aaaaattgta 33240
   aattaatatg aaaaaaattt attaaatcaa atacatgaaa aacaatttat taattttctt 33300
   ttcaatgttg gctgaagtcg atgctttgtc gtcaaaagtc aattcggcaa tttttttatt 33360
   taagccttta atcaccatct tgagttgctt ttcattttct tttgccagtt tcatcacatc 33420
   ttccatagca agaacgttca ctttcgattg atacgctagt tcgaccaaaa tttcaaatag 33480
   cgaaagtcca tgcatatcca agtcctttaa tacatcaaat atcatgtgat cattaaaaaa 33540
   gttggtatac gcttctgttg ccttttcaaa actaccatct tcagaatcgt cttcgtcgac 33600
   atagtcattg tcgtcatcgt ccgagtcttc gtcgaccgtt tcttttatgc tcacgtcttc 33660
   tttatcaacg tcgactactt cttctttgtt cttgttcgag tcgtcgtcga ttgcgtttcg 33720
   tttaaaaatt ttcttgttgc cggcgaacat gttctcgtcg tcgtcgtcgt aatcgtcatc 33780
   gctgctcatg tcttcgtatg cggcggcaat ttctttttgc aattctatta cgccttcttt 33840
   tttcagaaaa actagtttcg tcttcaacgt ttccaacctc agccatattt tatattcttc 33900
   ttcttggacg accgaatagt caataaattc gacacgtcta ttttttaact ttttgaacat 33960
   atcgttaaat tgttgaacta aaatagtccg ttcgttgaac gttattttta attgttgaga 34020
   cataatatat ttgcgtgcct ttaatattat acattccatc catttcgagg gatgaatatt 34080
   ctctaaatcg agatcgccca ataaaagttc gttagagtgc tttgaattca tctttactat 34140
   tgacttgttt acgcggtcaa aagatccaga agactgtgat attgttcggc tcgaactcct 34200
   cttttatatc atttctttat cgtatcatag tattattgca gtgcaataag ctgcccatga 34260
   ctctgaattc tccgctatta tcgggcagat tagagctacc gcggacacat tgcaggcttt 34320
   cggacaggcg attacccaca aacgattgat tgatagtcca ctgatctaat cgtgttcctt 34380
   cgactttttc gtgtcccgta taattacttt tgataaattc tttaaaaata ttatctggag 34440
   tattattgaa cagcatatag ggtatattaa agattggata tcgcttggcc tcgagatcgt 34500
   ggtgattacc gccgcggacg acatatttag tttctatatc attaaaattc gactgtctcg 34560
   acaagaaaga tatgggcgat agacatatct gcgcgctagt tccgtcttct gccatccatt 34620
   ctgtcaaatc ttcggaaccg ttcatgactc ctttttgtcc gtgtatacca cagattttga 34680
   tgccttgcag gtcacttgtc gacgtgatca tggtgacttt aacgtacacc gtgtcgttgt 34740
   gtacggccat agtagagtct aaacactcga tcctttgatt gaatatctct cgaaagtaaa 34800
   tgtaaatttt cgatagaaaa aaatttttat ttttacatgc ctcgatttta tagcgtttac 34860
   catcatatat ccagccgatt tttacattcg agacgacaac gcctgccata tataaaaaat 34920
   ttcctccttc aacttgtaca aaattgtttt ctttactttt aaaatatttt ataaccggtg 34980
   tctcagtctt tgaatgattg agttttccct ttaatttgtt gattttgttg ttgtacagtc 35040
   tgatcggtaa attgatgtcg ggaatatatg gatcttcggc agtcatcagt ttattgtctc 35100
   gaaccagcgt ccacagtttg aacattttat tattcacttg tattcgcgga ctgacgacca 35160
   cagaattacc tacgggcaaa gtcttcaaaa aaacatcgga atcgagctcc tcgtcgtagc 35220
   agctcacggg catcgcgttt ttcaaattcg tcagcgaaac tatgagtttt ggtacgggaa 35280
   tcgtgctgaa gattcgtgta tagccaaagt aataatattg caccagcttt gacatgaggc 35340
   tagtgacgtc atcgttttcc tctatcttac cgatttttgg gtatttcttc aagatcgaat 35400
   aagaattatg atattcgaaa ggcgtcagca gagtcgtaat ttttttgccc gcgatactaa 35460
   tcactcgctt gatacacacc atgccttcgt ggtgatttac aaagagaatg cggttggcca 35520
   gcagcttgag ttcaatggga ctgtattgcc gtttgagttg ataaaaaatc aattgtaaat 35580
   cgtcaaaacg gcaggcgtat atggtcggtc ggtcgttgaa cgcgattcta atgttttttt 35640
   catcgacatt ggagggatcg ttggcgtaaa ttaatccgtt ggtcagcaat aacttgaatt 35700
   tgtccgcgac taactggtaa tcgacggcgg gaagacgtac gtttcggcag agaaaaaatt 35760
   ttttaccggc cactgtcatt tcgccatgaa aaaaactatc gacaaacttg acaaaatccc 35820
   gttgttgcat gagcatgtct tggcgcatcg tgtcgttagt gattcgcacg acttcgttgc 35880
   cgattcgata tttaagcgga ggaggattaa tctcaatgtt gttgttgctg ctgttgtctt 35940
   gataattaat gaaattcttc ttttgcttgc taaaagtctt cgagacggaa tagattaatt 36000
   tgccgttgac gatggtgtcc acaacttttt tacattcttt gacaaataga atagtttgta 36060
   gttttcttcg ctttatactg ccagttttat tgatatcgcc atcgttttta ttgtcgataa 36120
   attcttcatt tttcgttcta aacgcttgag tgttgtttct ttttcgtttg ttattgcttt 36180
   tattttcgcc caaatcgttg ccatcgtcat cgccgccgct gccgtttact ttattttttt 36240
   tctgtttttc gtcttttgat ttttgtctag caatagagtc gttatcgtcg tcgctgacat 36300
   tgacacaatt tttgtcgtcg attttcatca tattcgtgta ttctttgaga atgggcgcat 36360
   acatcaaatc gagtaaataa tcgtgtttat atatgatttt attagccaaa ctgtcgatgg 36420
   tgtaattgat gtcggatttc attatatttt taatatcctc aactaaatga ttgctctggt 36480
   ccgtattaaa atcgaatata aaatttaaag gttcccattt accgctgcgt ttcagataca 36540
   tttcgagggc ctcgttcagc tccatcgtaa caatatagtc tcgggcgaat acgtctcggg 36600
   cgaaaagcac attatcgtct ttgtcgtata ccagttgaat ggcgcgattg atcttttttt 36660
   ccttgtcaac gttgccgtac aggaacatgc gtttgcagtt tttggaatat tgcttgtcgt 36720
   agaaattatg cagtagaata ttgttgttca tcaggatgtt cggaaaactc aagtttcttc 36780
   cgtcgatcat gaaagtgccg ttcatattgt tgattttata cgtatccttg tggcgaaatt 36840
   tgaggtcgag acgagttcca aaaatcacca atacacattt gtgtaggacg catcttcctt 36900
   gtacatcgac ggcacagcaa aaataagatc tacgctcttg taaatacttt actgtacatt 36960
   cgttttcaga tttattatta cagtttagat agaattcaaa attatattta tttttgagtt 37020
   tatcgtagag attattgaaa tcgacaatca cgtccgtcat cttgaaacta tcggtgatca 37080
   ctaatgatgt cgacaacagc catggatatc aggcctacag cttctattcg acccgtgact 37140
   cgtgcgttta aacgtaaaat ggatcaaaaa actaaaagat atttagaatc ggaagcgatc 37200
   gaggatgatg attatgattt gtcgtcgacg ccaaaagacg aaaatttaat gtcatcgacg 37260
   acatctttgt cgccgccacc ggcgaaacaa gctaaaataa aacgaaaaaa tttatttaaa 37320
   ataaatttgg acacgcagag tttttacgat ttgctttcgc taaaaaccga ttcatccgac 37380
   tttcaaatca agcaacgcgt aaacgaacta atcgactttt attcaacggc aaaagtcgac 37440
   gacagtagtt ttcgagacga tgacgataaa gttgaggtca tcaattctac aaatcaaatt 37500
   ttattaaaaa tttctcaagc ttccatgata ctgaccaacg tccgcgccaa acgcgtctac 37560
   gacaagtata tcgagatcaa atacaagatt agattgtggc aactcaacga attacgaccg 37620
   ttaatcgatg agattttcaa gcatatcgag tcgatattca ccgtctcgag tgattttcaa 37680
   gagtttgatg atatagaacc ggcactcaga aactttgtag ccaacgagct tactcagcga 37740
   cgaagtcgag gcaaacgcct gcgtagcacc accaccaacc gtcttcgcat acaatggaat 37800
   gtcaatctca acgatgagac aaacgccggt gtcgacgaac ggtatttact ggattatttc 37860
   ggtaaatatg gtgagattgt cgggatcgtc atgtgcagtt ctaggcccgg ttgtgcggtc 37920
   atcgaatttg atacgcttcg ttcggtcacc gaagtcatca gcgaagagag tcaactcaaa 37980
   cgatttattg ttcaggatct ctccgaggcg gaattgatca gtagcgatag tgctgctcaa 38040
   ctacaaagta aactagacga actcgaacgt ctatccagtg atttactcag tatacaagag 38100
   aatatcaaca aacaagcgtc ttaaacacgc aatttcgttg tcaatttcga taacaaataa 38160
   aacaaaaggc ataactttaa tagtatttta ttttaaccaa aataaatttt gattacatta 38220
   gtaaaagagt ttctacatat cggacaatcg ggtacgctcc aagcgcactc tatacaagca 38280
   acggcatggc cgcaaggaat atagcaggcg tcaatctctc tgctgaaaca aattttacac 38340
   tcggacttaa acgatgactc ttgaacggtt tcgtttgaag acgtgctggc gatttcctga 38400
   gagatcgcag gcggcgtcgg tcttggcttt ggtattggct tcggtgccgg tataacacac 38460
   gattcactga ttacttgctg cacataatcg ggtcccttga cgaacagtac atagcgacag 38520
   tcgggatacc atcgggcgtg ttgttgccaa ggcaccctat cgcaactcca atcggatagg 38580
   gtacaaccgt cgtagaaaca ttttactttg tcgccgatgc ctgtgtaaaa gaagcccgcc 38640
   tccgctaaat ctatatagtc ggtgcggttg cgaggccagt gacgaaagct gttgattcgc 38700
   ttaactacat tatcgaaatt aggatattta aaaataaact gattctctcg ttcatatgta 38760
   tcgtacttgg cccgtttcgc atctagtttt tttacgaacg gacactgagg agcatatttt 38820
   ttgtgatctc gatcgaccac atcgttttgt tgccatttac aaatctccac tttacagtag 38880
   gcacatcgga cttgatctcc actacctaaa taataaaatc cgttcatcgc caactctatc 38940
   ggcggaacat agggattaat ccacttttca ctatcgaaag tcttaagtct gttgctacag 39000
   cgctgcatgg tttgctcaaa actctccatc ttcgttgccg tagtgttcgt ggcttcgctt 39060
   acaaagtata aatactcaga gataacgacc gtgcgcgtat tatatagctg tgatacgaga 39120
   gcgtgttatc taatcatttt aactacgcag ttgcttctca acgaatcatg aggataagta 39180
   aacgacaaaa atgatgatat ttttttatta tatagctttg ccacctacgc ccgcgacaat 39240
   gcctactatt attttatcgc ctttacaaag gaaaaaacga tttaataaaa gacaaaagac 39300
   tcgttttata cgtatattta aatcaaaaaa aattacaaac aaatctctac cgagattaag 39360
   agttttttaa ctgtttgccg ttacgattct aaacaatgga aagaaacatc gcttacagaa 39420
   aagcttaaaa ctaaaacaat aaaaatatac acttccagtg aggcgacaag gcttgtcgca 39480
   ctgtaaacat ttctcataat gaattttata taattttatc ttggactcgt aactgtacca 39540
   agcgaaacga attctctcgc gccagacatt tttgtctaca atgatactat tattctcaac 39600
   aaaatcaaca tcatcatcat caacatcatc accataactt tttttgtttt tgtcgtagcc 39660
   accaatgttt atagattcat aatagcaaac actaatcaca atatagttta tgagctcttc 39720
   attttcgcta ctgtcgtcca aaggaaataa gcattgagcg cagcacagat gcgattcttt 39780
   attgtttaga gtaaaacaga atttacaaaa aaaattattg tacaatattc gacaattgtc 39840
   ttcgacacta gaatcgctac cgccgctgtc gcttcttttg attgtattca tcaccaatgt 39900
   ctttttaatt tcacaccacc aacgacggta cactagttct tttcgtacag tatcgttagc 39960
   cgttcgatac aaacgagaat atttgataaa gggtttaata agctccatga ttcatgctcg 40020
   aatgcgagcc aaagtcgaat attaacgtct ttatactaca gacaaaacat gttgataacc 40080
   gtcaatcttc aaaacaaaga cgaatatatt tataaaacat tcaaaaggat gtggaacgaa 40140
   tacaaaatcg aatgccaaat ttgtttcgat tgtatagaaa acgatggcgt ggttgccgtc 40200
   accgaatatc gaacgttaaa tttagaaaaa atgtttcatc aatcgtgcct aaagcgctgg 40260
   caacgagaac acaatcggga tccttttaat agaaacgtca agtgttggtt cgattttcca 40320
   ccaaagtctt tggagcagtg ttcggcgctt ctcgagcata tgaaaggatt catcggagac 40380
   caacgtcccg acaaagacta cgccgacgag tataatcgca tcgtaaaaga ggaatctgtc 40440
   atcgatattg aactggattt cgaaaaactt ttgagttact cttgacggag gacaagacgg 40500
   tttactcgaa aacaagataa ttgtaaacgt ttttgacgtg aatatcgatg tctatgactc 40560
   gataatactt gtttgtcgcg gcgacagatg taaattggtt cacatcattg tccggtgtat 40620
   gtttatagaa tattatagat agcagatcca ataaacgata gttgtacatg tctgttagtt 40680
   ttctttgata attgtctatg tgatttcgat attcgtcggg atgaatataa tcatagggca 40740
   atcgagactc tttatattcg agcagatctg ttatgttgct cacgatgtaa ttgacgcaca 40800
   aattgaaaaa attcgtaatg ccgtgcgtga ttttagtctt gccacgagtg tagttgacat 40860
   tcacgatgac gtgagctacg gtcgacacgt tatcgaatat tctcacataa taattgaaca 40920
   aggcttcgct aaatttataa aaattcttac gactcacatc tttgtcgctc aatatggcat 40980
   tcattatggt ggcataatcg ataacattct cgatattgtc ttcgaaagtt aaaaattgtt 41040
   taagcgtatt cgacactcga gaattgatgt tattttgaag cgcatccaaa taaagtttaa 41100
   tgctgtcgat acttttttgg atacacagca tgttgttaca atcgctgggt atttgattta 41160
   gtagaggcgg agtgatgaaa accactacgg gagatgagtt cgatccttcg gttctttgtc 41220
   ttttattcat atcgatgaca ctcgagaccg ccgtcgttag cgacgccgcc ggcaaagtcg 41280
   tgggatcctg cgaaagctgt atcgttcgag acatttcatt ttgacgtctc attatatgtt 41340
   tttccgcctc ttccatttga cgaatatgtt gaattctctg ttgctcttca tcgatcatca 41400
   tcgatcgatc gatatccatt tccatttcgg cctctacaga gctcgttgcc gtagatattg 41460
   ccgtcgccgg cggcggtttc gtggtagatt ttaatttttt cttagcgctt ccatcggtct 41520
   ttgaagactt tctaaatcct aatattgatg acgacgatga agacgatgaa ggcggaggtg 41580
   ataatggcgt ttccgacgct cttttactcg agcgccgatc catttcggta cgaagacgag 41640
   cagtagttta tcaatagacc aaaaacaaat agtcgggata atcgtttaaa gtgactatag 41700
   acctcgaact ggacacttct gttcgtgtgt acttatcata gtaaaaatat ttattatatt 41760
   ccgaatcgaa tttcacgaat ttaaatcgcg aaagctggtg atattcatcg taagacagta 41820
   attttatctt cttacgttca aaaatgtctg catctccatt cgaagcggac gtaatcgaca 41880
   tcattttgaa aaataacctc gagctcgtcg acaacactta tataatttta aatgtcatcg 41940
   atggcaagcg cggtggttcg attaaaccag tgtgtctcgg agagattaac tcctttcaaa 42000
   cccatcaaga ttcaaagtgc tcaatgtccg attcatccgt tacgagcgaa ctgcagagcg 42060
   atcgtacgct ataatgatgt ggtcgaaagc aagttcatta atcacattac gattttcgaa 42120
   tcgttatatc aaaattatga ccgacgtccg tactacatgt gcctgctgaa tcctgctttc 42180
   gagatcgacg aaagaggcga atatctgaac gccaacgaaa tgtacgccta tgttcatctg 42240
   cagcccatcg acgacgaaga agtcttctac ggaatcgacg aagccggaga gagatctatg 42300
   gccacactaa aaaccgcaat taaagcaatt atggactgtt tcgatgtttg tcgagacaaa 42360
   tacattttaa tggtggacga actccaaatt gatgtggttt actcgatatt ccgaactatc 42420
   gtattaccgc agcgaatggt gtttatttac cagacagagc acgcgcccag caataatgat 42480
   gttcaaatat ttagtgtgcc gcaaactgtc gaatcatggg attcgcatat tatttacaaa 42540
   tcgtttctac tctacaacac gatactcaca atgatgctca aacaaaagaa ccccttcaac 42600
   aataacaaga aaaatatttc tgtcgttttt cgtaatctag gcaagtgtcc taataataaa 42660
   gacagagtca agtgttgcga cctaaactat ggaggaaatc cacctggaca tattatgtgt 42720
   cctccgcgtg aaatggtcaa aagaattttc cattattcca aatgggccaa aactccgaac 42780
   aattatcgaa ggtatttcga gttgattaca acaccaccca tcaaccgcag aatgtacgag 42840
   ccggacggac aagtcaattt taattacaac acgggaatgc taataatact cgactggtat 42900
   aattttatcg aagagtttcg cgcatatttt ggcatcaagt aattatgcgc agatcactag 42960
   tatttattga gacacgttat cagacgatgc ttactcttaa aaagtgtgtc atcgcagtgt 43020
   gttatcttat cacagattcc gaacaagtcc tatcgaaatg aataaggtca attttcaact 43080
   taagaaagta atcaacaata ttgttgatac aaaaatgaat aaggaccagt cctcttcggg 43140
   ctactcgtca tcacgatcat tatcgtcggc ggctacatcg aatcagcaga atgctgcaga 43200
   tttctatacc aaacataagt cggatgtggc gtacgtcggt cgcaacacca cctacaatgt 43260
   cgtcggccag cgaaactatg gaaaatattt tgatgaaaaa acctacaaat tttaaaatca 43320
   ctggagggac gggtcttatg cgttgcgtcg acgaaaatga ccaaattgta tactgtttaa 43380
   actgtaagtt tgtagcacca atgtctgtga gctatgaaca ttttatttgg ctccatgaaa 43440
   aatacaactg tatggtgaac aatcgaggac catgcgccta tacaatcagc accagcaacc 43500
   acgatatacc caccgccgat actccgaatc taatcaaact agtcgataaa gattctaccc 43560
   gataattgtg ttcatttcaa atctaaaatt atgtgtatta tagagaattt taataataaa 43620
   gatccaagaa aattttaaaa ttttgcttta tttattttct gcgaaatttt gttctgcgca 43680
   tgtgccaagt ctagtttagt aaatatacgg ccatataaat agagtaaatg tcgacgacgg 43740
   cgcgttagtc gtttgctcga ttattaaaat gacactgtcg acgtctttca gtgctcgttt 43800
   cttagagttt gaacaagttt gtatcgactt gagatatgta acttttatcg atgacatatc 43860
   atgcgacaat atcaacaatt gtgcccacga ttatataata tttttaaatg taaagaaggc 43920
   aatgttttgc aattttaata taactacaga catgtctctg gagactttgg ccgaatatat 43980
   ttacgataat gttcgggtca ccatcgacgg acgtttgcac cgtaagcatt cgattatggc 44040
   cgagtgcgtg cgcttcaacg gagcggacaa caacaattcc atattcatca atatacatgc 44100
   cgaagctcga gttattgtgg ctaaaaaact gttcgacaac gaacgatacc atcaacgcat 44160
   cagtggctat atagatttcg agaatcgatc ggatccaaac gacaaaaaaa aattattgac 44220
   tgacagggaa cgagctctac tggatcgtga atacgaaatc aaaatgctcg gatcgacttg 44280
   aatcatttgt cgtcacggct aactaacgat tacgagacat gatttttgcc ttgtcgtcgt 44340
   cgtcgccgcc ctcgtcttcg tggtaataat catcatcgtc gcggttatcg tcggtgtcgt 44400
   aatctgtatt gtcttcggtg tcgtcttcga tgtcagtata atccgagtct tgtgcttgtt 44460
   gtgtgaatgg aacacgatcg tcctctttta tgtttctatt gtagaaaaat aatttttgtt 44520
   ttgccgatga cgacgaagat gggcgctcgg ctgcaacgac attgccatca ttttcgtcgt 44580
   cgtcgtcgct ttcgaccata tcgtgccgga caatctcttc ggcggcggtc tttggtatcc 44640
   aacggtcgtt gagcttgacg tagtctcgct cgacggcctg tcgtgccata tgtaaagcga 44700
   catcttcatc gccgccgtcg agtttatggt attttttaaa ggtctttaca aataattgtt 44760
   tagctcgcgc cggcatttct tctctaaaaa aggcctcgtt caaatgaaac attttaaaaa 44820
   tacttatcta atctattcgg tatcatagtc agaagcggat tcggattcgc tttcgcttgt 44880
   agatgaagtg tcggtggtgt cgtaatcgtt ggcgttcttt cgagccaccc actcgtcatc 44940
   tttattgtga acatatttac gtttgaccgc ctgccaggcg attttgaagg atatcggttc 45000
   agattttgtt tttgtgtatg cacgattaaa aaattttagg aatattcttt taccgttgta 45060
   cggtagactg tatgtcgaag gtggaagttc tgttatatta ttgtacatta tgatgtaaat 45120
   aaaaattttt ttgatcgaaa tgttttttta tttgtttaac acagtgtaca caacacagat 45180
   acatatatac aaaacattta aacttaatta atatctaaac ctgaagtaaa atttaaacta 45240
   acaacaatat aatttgacta tgaatagaat gagatataaa ctagattcaa gtagaattcg 45300
   atttaaatca aagtcaaatt ataatgaaat tgaagtgaga ttcaactgta aaactcattt 45360
   acgataaaat gcgcaatgag cccattaaat ttgcgtttct tcgtttaata ttagagtctg 45420
   gggtaaagac gtaataacgc tatcatcgta gccggcgtta tagttgacga tagagtcatt 45480
   attgttaccg ttaacgataa tggatgaagt ggatagagga ggagacgata gacttgtttg 45540
   tgtcaacaga tcataaatat cattttcgtt ctttactata taaattttgc tacgttcgtt 45600
   ttgtctcacc atgacgcctt ttttacacaa cgaaacgtac ttgtaatgcg gcaacagggc 45660
   gtctcgtgtt tttttcaata attgtttatg ctcgggcgat gcggccacga aaatcttgac 45720
   aggggcatcg aaatcaatat caagatcata atttttcagt cgaacttcac gcgaacgatt 45780
   ttgccactcg cgagccgtcg cggcatcgtt caactttaat ttaatgtgat ttttttcgta 45840
   atcgctgttc acgatgagtt tatagtttag cgttaggaga tggcaaattt ttttaacgta 45900
   atttgtacga atctttttat tgtacagtct tttatcgtaa attccataga tttcgactgt 45960
   atcgttaaga ttttcttcct cgagacgctt cagtttatta acaatattat cagtgacgtt 46020
   gcgttcaatt tcatttttaa ttagattctt taaaattgga atattaatta gctcggccat 46080
   gttagtttac gtcaaacgta aaaatttaaa ttaaattccc tcttattact agacaaaaag 46140
   tcgatttatt tcacaatgag ctcgtctaca gcaagcacgt ccatcgaatt tatcccgaaa 46200
   aaaccaataa attacgattt attgatcgat ccggccgaca tcgacaatgt gtatttctgt 46260
   aagattaacg attttcgcgt gtttctcaag aacattatag ctgacgttaa gaaaatcaag 46320
   attaatttct ttaatagtct aatcgaacag ctaatttccg tgtatcaaga atgcgaatcg 46380
   cgaaactcgc acaccgaaat gctgagcaga atgctcatgg ccagcaccat cattgtcacc 46440
   gatttgccgt ccaatgtata tttgaaaaaa ttgaaacgca acaaattcac cgacaccatc 46500
   gactatttga ctctgcccaa tttcatactg tgggatcaca actttataat tttcctgaac 46560
   aaagccttca attcgaaaca cgacaacgga ctcgtggaca tcagcggatc gattcaaaag 46620
   attaaactga cccacggcat cataaaggat cagtttcaga gtaaaaacgg ttacgcaggc 46680
   cagtttttat actcgacttt cttgaatacg gcatctttct atgcaaacgt ccaatgtttg 46740
   aatggagcca acgaaatcat accgccgaag agcagcatca aacggtatta tggacgggac 46800
   gtgagcaacg tacgagcctg gacgacgaga catcctaata tatctcagct aagcacccaa 46860
   gtgtcggacg ttcgccagcc cgacgactat aacgactgga acgttaaagt cggtttgggc 46920
   atcttcacgg gcgccaatac cgattgtgat ggagacaaaa aagttatcac gtttctacct 46980
   caacccaatt cgctcatcga tctggaatgt ttattatatg gagacccacg atacaacttt 47040
   atttgtttcg acaaaaacag attatcgttt gtgtcacaac aaatctacta tctataccgc 47100
   aaacagaata atatcaaagc cattttcaat accatgccta tattaaaacg tctgtggaag 47160
   tcgtataaag gtattgtatt ttcacagaaa ttggatccgt tgctaagaga ttgtgcgctc 47220
   gttttgagtt caaacgctag ttatttacta ttcaataaac tgacagagtt aatatccgac 47280
   gaagagatga tttgcggtga cgaagaaatt cataatctac aagggcaatt tacggatatt 47340
   attaaatgtg gcgcaaaagg cagtaaagat ttaataaata gcactcgaac ctacaaaaat 47400
   accaaaataa ccgacattga agaagtcgca ggcagagcaa taaagagtct aaataactac 47460
   atttcttcgc ataatcgagt caaagtcgga ggcggagata tctatcacaa taccaccgtt 47520
   ttacagaatg tctacatgaa aaacgatcac atttgctaca aaaacgattc tgtcaagttg 47580
   gctagcgtct gcttattacc ctcggaattt ctatttccgg aacatttgtt ggatctcttt 47640
   ttgtaatata tttttgtcga taataaaaaa gatgatgcaa tacgattgtc gtttcattat 47700
   aattacaaaa catgatgcaa tattgggcaa tatcaacatc atcatcatta ttcatgcgac 47760
   gcgtgcgtca atcgatgacc ccggatgatc gtcgacctct cgatcgttat cgattcgggt 47820
   cacgcgcgcg cgcgacaaac atccggctga cgcaacattg catcatgttt tcgtcgacgc 47880
   gaaaaacgtg ctcgacggct cggacgatcg cctacggtcg atccgcgatc gaacgatcgc 47940
   gcgtcgccgc gggacgatcg tggatggagt cgacgatgat cgattctcgg acgatcgaat 48000
   taaattttac gcttgtgtca gcgttaaaaa ccataatttt gtagttatta tcaccaatat 48060
   caagtaaaat gtctcgtacg ttgttaacat acaagtttca aaatttttgc agatttttac 48120
   aaacttttac agttacaact atcgcgataa tatccacagt ttcaatgtta aaaactccaa 48180
   tttagttaga aacgaccgga acgtgagctc ggtaaaatta taaaactaaa aactacaaat 48240
   ttttcaaaaa atttttgcat caagttttat tatatcccta acattgaagc caaaaactac 48300
   tagattgatg tcaaaaactt taatgttatc taaaacgacc gggacgtcaa aaaataaaat 48360
   ttctaaattt tttaacagat ttttctaaaa tttttatcct acgtctaaga caaaatactc 48420
   gttttcgtcg ttaaaagcat cgacattgtc gaaatcgtcc gcgacaaaag cttcgtttgg 48480
   cgtacgggct attcatgatc gatcaatcat gcatcgaccc gagacaaacg tcccgtacga 48540
   tttcgatgat attgaagtca cgaggtgtta gagtaacgta cgggctattc atgatcgttc 48600
   aatcattgat cgactcggca ccgatgttat cggaatcgtc acgaacgatt ggcatatttg 48660
   acataatcat tggctttgat attgagtcga ttcgaacgac aaaaaaaaca gaataaaata 48720
   aacaaacaaa atccatcgtc actggcacta tttaaaccgc tttagcgcga cattcgttag 48780
   tatctcgtta tctttcgtgt caactataca catcaaagaa aaatgtctct ctcaaaaata 48840
   aatcatgtcg ctgacaacga aatgcctgcc gctaagagat tcaaagcgta tgataatcaa 48900
   tccgaagaat cgtgtcatga aaaaaataac cattccgacg ttagtgccga cggtaaaatt 48960
   aaacttagat ttggcaacga agacattgca gttgtcacga tggtcgatga cgacggccaa 49020
   ttgtggatgt tggcgaatcc gtttgccaga attttggagt acagtaatgc tccgaaagcg 49080
   atttcaacgt tcgtcagcga taaaaatcaa ttgtgtttcg aaaatcttaa gtcttcccaa 49140
   agcaggcaga cttgtatgac gtcatcactt catccgaaga ctaaattcat caacaaagcg 49200
   ggtttgttcg agctgattca aaactccaag atgccacagg ctcaagaatt taagcaatgg 49260
   atcaactcgg acctgctgcc tacactgtgt cagcaaagag aatacagccc gcataaaaat 49320
   taccaaaaca tgtctctaac aaaaattaat tttgtagacg ataaagaagt tgagacgtat 49380
   acgattgatg tcgatggtga aaaatggatg actgccaacc cgtttgctaa agcgttaaat 49440
   tatagcttac cacatattgc aatttctaag tttgtgacta atgaaaatca gaaaacttat 49500
   gaagaaatta acccgatacg attcacatcg accgatgact catccgttct acctcggaac 49560
   attcaggcca agaccaagtt tatcaatcaa gcgggcgtct tcgagttgat caacgctagc 49620
   acaatgcctg ccgcgaagcg attcaaagcg tggaacacca acgatctgct gcccactctt 49680
   tgtcaacaag gagaatacag catgactgca gacgcgcccg tcgaaataca agagggaatg 49740
   aatgctgttc acgcagccac taacgaaggc cgagaagctc cgtggatgga ggacttgcac 49800
   aaactaagaa actccgtagt acagaaagac aaaataattg aagtcatatc atacgagaac 49860
   aaagaactct ccgtgtcgtt gcgcacttcg aacgaaaaat tgcaagatgc taacgacaaa 49920
   ctaatgtact ttgccagcgc cttggtagac tccaacaatg gattaatgaa agccaacgaa 49980
   cggattgaaa accttgccaa tcgcatggca gacatcgccc aggacgtgat agccaagccg 50040
   agtgaccctc aactattgca ttcgctagct gtgtgcgcct tgggcgaagg ccaatatgcc 50100
   tttgtgagac ctcaaaagcg tagtctgaaa cgtagcttgg accggctttc catcgacgag 50160
   agtcaaattt tatttaaaag taactacgta cccaatgcga tgaacgtact gaacaaagtc 50220
   aaagagagtc tgcctaaaga caagtttaca gctcgtcaca acaagattac gttgctagaa 50280
   gatttgacta gagaggatct agttgaagcg attaactcgt cgatgaccga gcgacaggtg 50340
   gccatttcgg ccaacaaagc gtcgggcaga aaataaaatg tgatcaatca ataaaaatct 50400
   ttttttatat attttacatg ttgactatta ttctaatccc tatgtgcgtt gaatagtttt 50460
   taatgtttgt tgagcagtat tccaatcctt gcgaccattc atgatcgttt aatcatgaat 50520
   cgccacgaca ccaacgttac cgtttttgtc aataccaatg atagatttta atagaaaaat 50580
   tgtggtcatg ttttaataca ataataatat atgttaagat cactctagtc taattaagag 50640
   gttcgaatta aacaacaatt gaaaatgttc atgtacttta cctttctagt actaattata 50700
   ttgggattca tttttgataa aaatgaagga ttctctaatc tattactctt tatgctgctg 50760
   ctgtttgtca tatttataat gttactgcaa atctactaca taaagccaga gtctacgact 50820
   gccgatctac caaccggtaa agctaagagt ataaaaaaga aaagagattt agaaaaagcg 50880
   tttgacgcaa ttataaataa gaacactagt tctctagatt gaaacaaaat ggacttcttt 50940
   aaaaattttg tcaatcaaat ggtcacatcg atgccgatcg tgacaaaagt ggcctacgtc 51000
   aatgtgcaca tcaaaaacta catgaaggag ctggaacggg acgacacgtt tcgccataaa 51060
   tttatcatga tcctacaaat gtttatcaat cgacagatca cagtcgaaga catgtgtcaa 51120
   ataatggatg ccgtcgatgg tttgaaactg actagacagc agattgaata cttggtgaat 51180
   cgagtctact acaacgaaca tattatattg attcttcaga gttatgttcg caatcagcac 51240
   ttggacgatg accagattga cgatttagca catttcctag ttcacgaaat aaataatgcg 51300
   attatatacc aaaaataaaa actttattca tcaaaacaaa tcaacaaatt ttattacatt 51360
   ccccacacaa attgttagga tcataacgaa aaacgtcaac gtcagctgac acgacgacag 51420
   gtctcttggt ctttgtcgat ttcggcggcg gacgcttttt gtatagagaa aactgatact 51480
   gatagttgac gacgatcgat tcatcttcgc tttcgtcgtc gttagagtcg tcactgtcgt 51540
   tatcgtcatc ttcgatggcg tcggtagatt tttttgttgt ttttttggca gcatgtttga 51600
   cgggcttcaa ttgaggaaat ttaacatcgt gctgttgatt ctgcatgtaa tgctgacaga 51660
   ttctaataaa ataatactca aactcggggc gatcgccaaa tatctgaatc agctcgttca 51720
   taaacgtaca caagatgccc atatgcttga gccaactcat acgcttggtc tgatcgaaaa 51780
   gcttgagagg aaaggccttt tgagtcacag tgccgacgac atcggtcagt aagtagggga 51840
   ttctgtctcc gttgccgggt agaaaatcag ttccgcaatt agccaaaatc tctcgacagt 51900
   gcttggcgat ggtgatgggt cgcttgcgct ctggcgcgcc cggagccaat tctttcttct 51960
   ttttgtttgt agtgttttcg ttataactca tgctgaagcc ataatcgcta tagttatcgt 52020
   cctttccgaa attgtcgtaa tgagcgatca tgagctcggt gagcagattg agaccgcatg 52080
   ccacgctatg attgcgcaga tacatgtcga cgatctgtct aaaagacttg cgcataaaca 52140
   gaggcatatc cttttttatc agccacccct tgtatttgat gcgattttcg ctattcagat 52200
   aacaatattt cttctttttc aatagaatca gactggtcat tatgttctct agagccattt 52260
   tatatccgac ccaatcggca ttcagtttat tgagcacata attgtttata attttaccaa 52320
   ttgtctcata gacattttct ttaatttcat tctcatcgaa ggctacgtga ataaacgaag 52380
   agtccgtgtc gccatagatc actttgaaca taatcgatga cagcttgaaa tcgctcaata 52440
   ttctaggatc gtcgctcata ctttgaatct ttttgatggc ctccatcaat ttttcacgtc 52500
   cgatttttgt cacataattg gctaaaggtt taaagaagat accgaaatag ccgtagatgc 52560
   tgttggcgat tcgctttaca gcattctgag ttttatcata taaattatac tgaaatgtgc 52620
   ccacatcgaa cttgtctcgc ttcgttttat atagaattcg caggtccaag agctgtctca 52680
   aaagcttggg attgatcgca ttttgattct tcactagata cacgtaatta tcgtgtccga 52740
   tgaacgtgtt cgataggcaa attccctctt gaatcattat agttaggtag agtgaattaa 52800
   agtctagagt ggcgacccaa cgtttcagtc ccggttgagg cgaaagaact ttaccgccct 52860
   tgtagataca tttttgtttt tggtgacaca gacgaacggc attttgaggt atttcttcga 52920
   ttggacttct ctgcagcaga gtcaagtcga ccatttgcga gtctacggct tcaatgttgc 52980
   cttcactaga gccaccctcc ctctgtttcc atgtgcgttt gcgcccagac gttaccgata 53040
   gatcggcttt attaaaaaaa tacgcatcag gtatttcgcg cttcaatcga tcatcgtaat 53100
   gagtgttgtt caaagctcga taaaaaaaca ctacactcac tttgtgtgaa atgttacgaa 53160
   gaacgtcatc tgtgctcaga tagagcaaca tgcacagcgt atacataaag tccataattt 53220
   ctagcttgag aaagatatcg atgggcagca cgctgtcctg tacgttgtac tcgatcagtt 53280
   tcttgaggca attgttatta tagagatcga gcatttcggc aatgggcaaa tccacctttt 53340
   tttgattcaa ataatgctcg gacacagtgt tcaattgaaa attttccaca ttattctgat 53400
   cagaatctgt actcaaaaat tggtatagat cgacatgagt atagtacgaa aaaaaatgag 53460
   tattcatacg attaccgaat ttatcgcata atattgacgt gtgaatacta atcggctcca 53520
   gatcgtagcg gacgatcttg gtccacgatt ccgatgtggt caatcccacg ctatagctgc 53580
   ggctgtgttg tctgctcgac agaattctag cgcgattgat gataaaaggc aaatcaaatt 53640
   tgtctccgtt gtaatcgaga agacaatcca tattaatggc gggcagcagt ttaaaaaaag 53700
   cctttagcat atttatttcg tttttgaagc gaacacacat gatttccgca gacgggtcct 53760
   tgtcgacatt ttgcatgtcg ttgtcgttat tttcaatgta atagaggcaa attttcgtgt 53820
   ttttgtcgtc gcgcttaacg acaattgata tcgaaatgat gaaattttct tcgggcgtag 53880
   aaaatctttg gccgtttgaa tgtgtttcta tatcgtaaca cgcaatgacc gggacaatct 53940
   cacgagttaa cgagtcgggc gacacgattt cgagtagttc ttcgatgttg tcgatgttat 54000
   tgtaggagca ttgcaaacga tttccgatac atcttagcgt tttcttgaat cgaacgtatt 54060
   gcccttcttt aagttttgtt tgcatatgaa cacgattgat gtcgtttaga aacgaatcta 54120
   gacaatactt gtcaaactgt tcattttctc tacagcgctt gtatttaatc acgttaatgc 54180
   gttcgctgct cactcctcgc aaacccggca tcaccatcgt cttatagctt tcgcagttgt 54240
   tgtagcactt ggaaaaggcg tgattgttat agcattttcg ataggaaaac aaatcacaca 54300
   tggtctccat gtaaaattga taagaccgat tgttgttgtg gccagtgaaa aaaactatca 54360
   aacggttatt attgtacatc attcgtgtta tacggtacac gtccgaggga aagatcttgg 54420
   catttccttg tagccacgta cttctgacag cactttctag ttcctcgtac ttcaaaaggc 54480
   tcaacatcat gaacaggtat cgaccaaagt ataaaaacac tgaagtcaac gctgacactg 54540
   tgcaaaattt attgagaaca atcaacacta tgagtcagcg gtgcaaaact cagtcgtcga 54600
   ctgatgatgt tctgcagcga gttcgctcta ttatactatt gcatcgaccc gatctgatat 54660
   cgcgtgctga tcttcagata ccggagctcg tcaccgaggc tctgataccg tcgtctaatc 54720
   agattacaca caatttcaac tacaagtacg attataatag taacgtgcca aatcctccga 54780
   ttccgccgcc tttaaaccca tttacgtcga atacgtccgg gacgacgacg acgtatcaat 54840
   ccgatcaaaa attaccgttg cagccggtgc agatgtttac attcaatcag actccgtcgg 54900
   cgacgatggc accgccttcg cagtcgaatg ctacttttta tccgcaaacg acgtcgacac 54960
   tgccgccgcc gcaaactttt atatcgattt ccgaggaaga ttcggtcgcg ctcaacgaat 55020
   cataccaaca aactctacga tcaccatcga tcgaatcgta caaacgatta attagcgttt 55080
   tggtggtgat gtctaaaaaa tatattagat cggacgtgtt cgtcattggc ttgaatcgct 55140
   tggaatcgtt cgattctctg ctgagtagcg atctggagga actgatgcag tgtattcaac 55200
   gtaacaccaa ttggtcgata ggcggcggag gtaaagcttt tactactcca gaattatgtc 55260
   agctaatttc gacgatagtg acaggctatt gtcgctttgc cggtaccgtg ctcaaatcgt 55320
   ctagttttaa tctgtcgagt gtaggtagcg tcgagagttt gcaacaacag acggcttcta 55380
   ttcaaagtca attgtcttcg ataatcagtg cgagcgactc tgtcggcttg aataacgatc 55440
   aaataattag attaaataat cagattgaaa tgcttcaatc gacattggtc gaacgagaat 55500
   ctaccattat aactctgaat acaaacataa atattatgac tcaacgcaat caagccctcg 55560
   aatcgaatct ggcaattatc cagtcatcgt ataatagttt ggctagagct ttagatgtta 55620
   atataaacac gtccgctaga ctggaaaatg ccgaaatcgt tgaggcgaca aatcgaatta 55680
   tacagaacaa aattatggag gatagaggtc tactgaaatc atttgagagc gaaaaacaaa 55740
   catacggtca atctgtaaat agattagaga gggaattgaa cgattctcgt gagtatgcaa 55800
   atagattaat gttggaattg aagtcggctc gtgatgatat tatccaatta aaagaggaat 55860
   cgaaccgtaa tgcttcgaag caacaggacg aatctactat taaagaacgc caacaacaag 55920
   aattgtttgt atcggaatta ttcgatttac gcgaagaaaa taaaagtaat caaaatcgaa 55980
   tcgcttcaat gagcatcgaa ttaatgaata ttgccgaaga aaataaagtt ttaaaagata 56040
   aattggcaat tcgagagaag ccagtcgctc gcgccaaacc ctacaataga ccgtcgcaga 56100
   tagaggcgac gacatcgacg gccgtagaaa atcggactcg agctctacag aaaaccatta 56160
   aagatctcga aacgactaat caacaattgc gcaaccaaaa ctctagctta acatcgcaaa 56220
   tgaacaaaaa caaacaatcg gctagcgttc aggcggactg caatcgaatc aggtccgagg 56280
   tagataacgt tcgtcaaaag gtcgaggaat taatgaaaaa tcaggcgaca ttaaacttgc 56340
   aagacattaa tgcgctcaat caaaagactg cagactatta caataccaaa ttgtccgaat 56400
   tgaccagtga aaatcaaagc attagagaca ttctaaacac tgaaatcgat aaacaagtcg 56460
   atatactcaa cgaacgtatc gaccgatcaa aatcaaatat tcttgataaa atcgaaagac 56520
   tatcggaaga atttagtcct gttttagttc gtgcacaatc atcagaaaaa tatgttcaga 56580
   atttgctcgc cgattatgaa tctctgtcta gaaatgtagc caatcagcag ttgtcggaat 56640
   aatacaaaaa aaatgtttat taaatgttta ttaaagttta tttaaaccca caacccaaaa 56700
   atagtacaag atttgtaatt atatgtttat ttatacccac aactctaaaa tagtagcaat 56760
   tttaattaca tcaaaaatta taatacaaaa acacacatta tatagaataa tattgttgtt 56820
   gttcgtgttc ataggcagtg actcctatta tattgtagtt tttcgaaaca ttatcggcga 56880
   caatatagaa ttcgacttca gttatattct cagcaatcat gtcgttgagt tgattcaaat 56940
   ccattttaat tttaaccaat ttctgtaatc gatcctcatt gttgcctttg ttgttgttgc 57000
   cacagaagaa tgttcctttg agcgggtcat tgttgtcttg ttcgaacaaa ttaaatgaaa 57060
   tacttaatcg atcgttgcca tattgttccg cttgaactct agtaatcttg ccgacgatga 57120
   cacgtttgtt ctggcgactc acgcttaaaa tgctgttctt gtcctccttt aacatagcag 57180
   catctttggc tctctcgatt tgagtaatgt ccataatgat aaaagacttg tagttgttgt 57240
   tgctctgatg gcatttgatg cgcatcaaat tgatcattct attttgatta tttacaaaaa 57300
   attccaacaa atcagactcg ttatcgaagc tttgtttgaa ggcggcacta attttctgta 57360
   atgttgcact acattcaatc tgatataatt ttccttcact acgagaacta aaatcgttgg 57420
   taaacgacac tacaaatatc attttgtaca agtcattttc cattaattta aagccaaatt 57480
   gaaatttagc caatacagaa ataatttcac tgtcgtcaaa gtctttctga caaacaaatc 57540
   gtttaacatt gactttctta tcgggctctg aagattcttt aaaatcggta atatagtagc 57600
   gtttcttttc atatgtaatg gttaaattgt atttcttgtt tagcttcatg gcataaaact 57660
   gactggcatc tccataatac tccttgctga cgttgtcgat gagtactttg aataagtaaa 57720
   atgattcatt gttaatagat agagtgtttc tggcgaccaa ctcgcccacg actctattct 57780
   gtcgcattgg gcccgcacta gagcccacac ttccagttga aatcgacata gcatcacttt 57840
   tattattaga ccttttcaga ggtagattaa catcaatctt ctggcgtttg ccgccataaa 57900
   tctcttcgtt atcttcgacg gcacgatcgt tctgtttttg atcgcgtttg cgctttttaa 57960
   tttttttttc ttgttgtttt tgtaatagaa gctgttgttg acgttgttgt tcatcgtctt 58020
   cgtcttcgtc ttcgacgact ttttcaactt cattatcgat attctcttca acgtcagtgg 58080
   tgttgttgta gacgttttcg tcttgttcct cttcatcaaa gtgagaggga gcagtcaccg 58140
   acatattctt gatagacatg gccaataatt cgatacgctg gcgaatgcta aactcggcga 58200
   gggtcgaagt gtctcccgag agtagagaac acgcttggaa ggatttagtt atcgacacgt 58260
   tgatagccag cccgaaagac gattcctaca ggactatgat caataaagcc aatttcgaaa 58320
   actttgacta taagagaccc ttgatttatg atatcaaaac tgatacggtt ctgataaaca 58380
   gcgagtcttt gaacagagca cttaatcgac cgaccggttc gctcgatccg ataaatgtga 58440
   tgtctgcaca cgtatttctc gcttttatat gctcactact gttaattctg ttgacggcac 58500
   tgctttttga tcaaaacgac agagacgaat cgccaattga attgaacgat ggttagatca 58560
   agtttcggtg tcgtcgatgt cggcggtcat tgttaaggtc ggccaaatgg attattattc 58620
   gaatataatc aactctcgac tcgcgccgcc gtcgttctat ctcgagtaca gcaacagaat 58680
   cgagagcttc gtcggtagtt ctttaaccag agaatatata caaaatttag caaagtttgg 58740
   tatatactac gaaagcggca atggtgttta taaatgtgca ttttgtcctc taattatggt 58800
   caaattggac atgagaacgc taaaatatca cacgttttcg ttgtgttcga tggcgacgac 58860
   tatactcgcc ataaacgaaa ctctccgaaa ggaatcgttt cgcaagttca agactgctcg 58920
   acgcatattc aaagaaaacg gcaactcttt ggccgtaaac ggattctatt attatggaaa 58980
   aaatatcgaa attcgatgcg ccggttgtcg tctcacaatc gtcaaattga atcgaaccga 59040
   tcgtgccgaa gatattcatc gaaaatattc atcagaatgc gaatttaaca acaagcctac 59100
   ggctccgccc gccagcgaca gcgacacgga aattgatgtc aaaaacgtca aaaatcacca 59160
   tacagacgtc tacaccgaat cgtatggtaa tactgctgca aaaatttatc ctgttttgga 59220
   ttcgaataat ggtatcgtcc atgacgataa aaccaacaat agcaatgatg tcaacctgtt 59280
   tttaaaatcg aatattgtcg ccgacggtga taaaaataca caatcggttt cgagcgtcgc 59340
   aggagaggac gatcgttatt gtaaaatatg ctttgagaac gagagaaata cgtgcttttt 59400
   accttgtaaa cacgttagca catgcaccga ttgtgcccgc aaatgtaaag tatgttgtat 59460
   atgtagaatg aagataaaag aacgattgga ggtttattta cagtaattta ttgtttattt 59520
   gaactatata accgcaacac gatcgtttcg gacatcattc gaaacaaaat gtctatacaa 59580
   acgggtgaca tagtcgagtc gaacgtggtg acccaattta attcggtcgc ttttattgtt 59640
   aatcatgaga ctcgtcgggt cacgatcatg tcatataaaa atgaacctgt caaattccac 59700
   gtgttcaacc agcacgataa cgatcctgat cttctaaagc ttcaatatca ttatccggga 59760
   ctggcgagca gtatcgaact acctaaaatc actcacatga gtaaagttcg aatcatggta 59820
   ttcgatcagc gacatcggat gattatcgtc gaagagtttc tcctggaaaa actatacttt 59880
   gtgcatcatc attacgctaa atattatgtg tacggtttag tgcctgttgt cgccaagaac 59940
   tttaaacata atctgtatag cggtgcgcct gtctttgatg aaacgggtaa agttttgttg 60000
   tcggtcgtta gcgactgtta tttttcatcg acaacagacg aaaattgtat tatacccctg 60060
   agcggagaac tgagcggatc tcgaggagtg ttgtgtctcg atggccatgt gtggctcaac 60120
   gaaagcggtg acgatttcac attcgacaag acaaacgttg cgaatagaat cgatttgtac 60180
   gtttcgcatg ataaaaaatt tgtgtattta aacatattct ataataacaa tattataaat 60240
   tttattagag ttaaagccaa gtttgttgga aatgtactga ttcgttaaac atcgattaga 60300
   atgaactgat taatttatta ctctattaaa acgtgcactg attagttagg aattgtgtac 60360
   tgatacgtta aatattgatt gtatatttaa agtgtaatga taataaaata aaatcaaatt 60420
   ggttgtgaca agactatttt attcgcgccg atgcggccaa ctcgttgatc ataccgcacg 60480
   aatttacgtt tcttttgacc ctaacgaaac cgttctctcc gtagtcggat ccccacgaat 60540
   ttttcaacac ccagaacggt acaccatttt ctatgccgta gccgacgaga agaacggcat 60600
   gattgaggcc gtagttttta cacgaagtaa ttattcctcc gtaataatct gtgagatcga 60660
   cggcatcgac cgccactgct ataggaccca tttcatgaag tacatctttc aatttatcct 60720
   cactatataa aacatatcta taacaattgt ccacggaaac ttcaaattta tcgctttgga 60780
   gtcgacacgg gccttgtacc gaccgatagg gatagtcttc ttcgtattcc aaaccgccca 60840
   tagccattat ttcctcgtac gcagtgtgta gtagtccgcc ggcgcatccc atgtcgatgg 60900
   tgtcgcaatc gaccaattgc tgttcggata gatcgacatg ttcattatac ttgatggcat 60960
   attgactctc taatccggct atagtagaga aggcccagca cgatccgcaa aacccctggt 61020
   ctttgacgga agtgatcttg ttaaattgtc tccagtcgaa ggtttcttgc gtatatttcg 61080
   agggaccgtc gactatgacg ggctcgcaag agttttttaa agctggcgat cttatgccca 61140
   gacccgtgaa cttggcgatg acttcattct tggtcagatc ggcaaatttg tttatattgt 61200
   aaacggccga atcgttttgt tgattcttaa gattgatgat ttcgagatta gttttaaaga 61260
   ttttataccg ataatcgcgt tcgttgtcgt cggcgtatac ttttttatat tttgtttgaa 61320
   acgtttcaaa atattgcgga gctcgttcta aattatattg taatttaaac tgcggagccg 61380
   ccgtgacata atcgataatg gcgtcgacac cattgtcaaa gccgccggac gcaaagacga 61440
   aaacaaaaaa tagtatgatt tttttcataa ttattcgcta ctcatacaat aaacgataat 61500
   ttcttatttg gacaatacag tcatgatgca ttcggtaatg ttgcatttct aggaaaattg 61560
   atatttataa gacaaaatca aaatgcattt tgtattcatt ggaatcgtat gctacatatt 61620
   aaccatagac ggcggacatg ctgcggctcc cgggattcct gtgatcgatt gggccgacag 61680
   aaattatgcc ttggttcaag tcagtcacga agccacctcc tacgaaaatc tagttaaaaa 61740
   agtcgatcga gttcaaattc cggtctcttg gaacgtttgg agtggcgaac aaggcgatat 61800
   cgcttatgtg ttattcgaca gtaaacaagt atggagcggt gacgcatcca acaaacacgc 61860
   ttccatattt gttagtacag gcggtcgatt taaaatgact gtaaagttgt gcaattcgga 61920
   cggatgcact tcgagtcagc cggtcgatgt cgtgatcgca gacacggacg gctctcatct 61980
   agctccatta tattatgaat atcaggaatt aaatcaaccg ttcgagactc gggtcaaaaa 62040
   taaagtcgtt gccgcatatt ttgtcgaatg gggcgtttat cctagaaact ttcctgtaga 62100
   tcgagtgcct actcctaatc tatcgcatgt tctctatgga tttattccta tatgcggcgg 62160
   cgacggcatt aacgacagtc tcaagaccat aaccggtagt tttgaagctc ttcagcggtc 62220
   gtgttcgggt cgagacgact ttaaagtgtc gattcatgac ccgtgggctg ccgtccagaa 62280
   accacaaaaa caagtaaccg cttggaacga accatacaaa ggtaattttg gtcaattgat 62340
   ggcagccaaa ctggcgaatc ctcacattaa agtgttacct tcgataggcg gctggacatt 62400
   atccgatccg ttcttttatt ttcacgatgt ggccaaacgc aagatttttg tcgattccgt 62460
   catagaattt ttacagacct ggaaattttt cgatggtgtc gacatcgatt gggagtttcc 62520
   cggcgggaaa ggcgccaatc ccaacgtagg cgacgtcgaa cgcgatcgcg aaacgtacac 62580
   gattctgttg agagatttac gcgaagccct cgaccgtctc tcaatcgaaa cgaatcgaac 62640
   gtacgaattg actagcgcga tcagcgccgg cgatgacaaa attgccgtag tcgattacac 62700
   tgaagcgcaa aaatatttgg attatatttt tttaatgaca tacgatttca aaggagcttg 62760
   gtcgaatagc gatttgggat atcaaacgac tctgtacgcg ccttcgttta accctaaaga 62820
   gcattatacg accgattttg ccgttcgtgc tatgctcaag caaaaagtcg aaaacaaaaa 62880
   gattatagtc ggcgtggcca tgtacggtcg aggttggacc ggcgtcggtg actttgtcga 62940
   cgaaaataat ccattttccg gcacggctaa gggaccgatc aaaggtacct gggaggacgg 63000
   agtcgtcgat tatagagaga tttttaattc ttttgtgggt aaagacggca ttaatcgtta 63060
   cgattccatc gcaaaagcgg catacgtctt taataacgcg accggcgatc tcataaccta 63120
   cgacagtcct cagtcggttc tggataaagc aaaatatgtt ttggactata atctgggtgg 63180
   tctgttcgcg tgggagatcg atgctgacaa cggattgctt ctaaacgcaa tgaacagcgg 63240
   tctcggcggt ttaacaattt ttgctagtga aaatgaattt tataaagtga ataatcatta 63300
   atattgcgac aacacattag tcgcatcgat catacaccgg cttcggtgaa attacataaa 63360
   acagtaaagg aaataaaaaa caaatgtgta gtaaaaaatt ttttatttaa atatcttcgt 63420
   tgggcacata actagcgata tatagctcca tgtagccgtc gtctctgttt tcgttgtcga 63480
   gaaaatactt taattttaac aaatgtttct caataatgga tatctcgaca aaagaattga 63540
   tcggattttt agtgtaatta tagaaatatt ttacattaat ctcgatcatg tcggtcggtt 63600
   ctgtagcact atcgatggca caattttctt cagattctcc ttctttcaca acgtaattga 63660
   cattaatttc gtcgcctttg ctgttaaaaa tgattttatc gagatcgatt ttgatgccaa 63720
   cgttaagatt accgagtaaa tccaagtgac gacacaggcg tttcaattcg acactatgca 63780
   tcatggctct gccggtcact tgcaaatcgt tagacatgcc atagtcttga ctatcaaaat 63840
   gatacaactt gtattcgtag gatttcagag atttgccagt cttgatctta aagttgagat 63900
   aatcgtgact gtcgttttcg ccggaccata tagtgagcac gctcgcgtct gtgcacgttt 63960
   ttaaaatctt gcacaatcca ctcatcttga cggatagaga caagtttttc gtaaacgtaa 64020
   aactcttgaa agcttccttc atcattgtaa gcttgatgaa tgaaatcctg ctcgtgtccg 64080
   agcattgcat cgaaattcct tcgctggtga cttctagact cgagaactcg accaccgtct 64140
   caataacttc cattgccttt ttgaaagcaa tcggattctt gaatttagcc tcgaacatct 64200
   tgattgtatc gctgacgcta gttgactatt gacagtaaca aaaaacgatt gtatttatac 64260
   tcgatgacga tcgagataat cgagatatga aatgttatcg caacaaagtt gattattaag 64320
   cgttcactat gattcgttcg attttcgtgt tatatacaat tatcaattgt gttagatccc 64380
   acggatactt gtcctatccg gcggcgagac aatacaaatg ctttagagac aataatttct 64440
   ggtggcccga ggacggagag gcgattcccg atcccgcctg caaacacgca tatcaaacag 64500
   tgtacgccaa ataccgatca gaaggagagc cagccggtgt tgccgccaat gccgcacagt 64560
   acatgtttca gcagtattac gagtacgctg ccgtcgccgg cgccaactat gaagatttcg 64620
   atcacatcaa gcgaaatgta gtatcgcata atttatgcgc ggccggtgcc gattaccgtt 64680
   ccgatccttt cggtgacaaa tccggcatcg acgagcctca tacagagtgg cgagccgata 64740
   ctttttatgt agatcccaag caaaaatata gtcgaggcat cgaaacggtt ctgcatttct 64800
   gtccgaccgc ggttcatcag cccagctact tcgaggtctt tatctctaag cgcgaatatg 64860
   actattccca cgaattgact tggaacgacc tcgagtttat aggcggtaac aactcacagt 64920
   tgatcgacaa cgacgggtcg gatccattgt gcgcacacga atccatatac actatcaacg 64980
   tcgccatacc ctttagaatt aggaaattcg tattgtacgt tcgctggcaa cgcaacgaca 65040
   tagcaggcga aggattttac aactgtgccg atgtcgtctt tgacgattat ttactcacac 65100
   acagacgtgt cagtaagcgg agaaaggcat atagagatct ttaatttatc gaccaaacta 65160
   aaattatttt cgaccaatcg aaatgtattt tcgaccgatt aaatgcgtta tctgttaata 65220
   acaattttat ctacaaacca aagtatataa agctcgggat ggtacgattc gtgcagttat 65280
   tattttcatc atgtcgctga aagtcaacta cgatcaaacg ttcgccgtca gcgctcagca 65340
   aactctggcc aatcattttc aagagtgtag aacaactaat atcggaagac tctgtgatcc 65400
   taaacgaatt ctaaagttga atcaaaccga agagcccgac cacgatgtcg tcaagaaagg 65460
   aggagtggtg tattggatgt ggagagactg tcgcgtacaa gacaattggg ccatgattta 65520
   tgctcagtat ttagcgttta aaaccaaaag tccattatat atagtctatt gcctgccgaa 65580
   attttatcta aattcgactc gacgacaata tcagtttttg attgaaggct tgatcgagct 65640
   gagtgaagag tgtgccgaac tcgatatcac ttttgtgata ctcgacgaca gtgccgatgt 65700
   tgtgctgatc gattgggtgc gcaagcacga tatttgtgct gtcgtttgcg actttaatcc 65760
   tttgcagctt cagataaaat cgaccgttaa tattttacaa aatctgccgg ccgatgtgta 65820
   ctttgctcaa gtcgatgctc ataatgtcgt gccttgttgg ttgactttca ttgccgacag 65880
   acacgattat gatgagttta aaagtaaaat tgatgtagaa ctagaaaact tgttgactcc 65940
   gttttctctt gtcatacaac atccgtacaa atcggtcgtg tctatagaat cttcaaccaa 66000
   tacgtccatc gactggagca atctattgtc gtcacgaaat gtcgatcatt ctgtgaagcg 66060
   aattaaatgg accgaagcag gatacaatgc ggcaatactg agattggcca cgttcattca 66120
   gtgctatatt tataattata aaaattcaat acataatcca gtctccagta aacagagtga 66180
   tttatcacca ttctttcatt ttggatttat atcagcacaa cgagtaattt atcatttacg 66240
   cttttgtgtt acaaaaaaaa gtgtccttca aaaaactgta tttataagaa aacttaaaaa 66300
   taatatagaa aaatttatcg aaaactgttt atatcgaagg gaatttgcag acaatttttg 66360
   ttactttaat ttaaactaca taacttttaa tgcggcttcg ccacaaatca agaggtatat 66420
   agcaaaacat ttaagatact atacatacag cttgaatgaa ttggaatatt cgcaaacaca 66480
   cgacaatata tggaataaag ctcaagaaga tttgcgagaa aacggcaaaa tatatccatt 66540
   catacgcgta tactgggcca aaaagatttt ggagtggacg tcaacaccgg aagaggcttt 66600
   gaatcgtgcg atttacttga atcaaaagta tgctgtcgac ggttgcgatc ccagcggtta 66660
   tgtaggatgt ttgtacgcta tgagcggtct tttgaacgcc ggccaatcta atgtgtttgt 66720
   ctttggcgaa atcaacaata tcaggtcgaa atggctaata aacacgtgca aatataaaga 66780
   ttttacatat agctatactt catttagatt tcaatacatg tttgaataga ttgccgtcat 66840
   ttaagattta attttgtatt acaataaaaa ctcttttttt tcaattataa tttttatttt 66900
   acaacaatac attttaatga ttataataaa aattaatcct atacatgatt gttttattcg 66960
   ttagaacttt tattgtcgat tgcctgcatt atgctgctca catcttgtgc tggcatcgag 67020
   ccattaaaaa tataattgtt accattaaag tagtttttta tacactgatc gtgcacttgt 67080
   tgcggtgaat aaatttctaa agcattttca tatgtctttg gactaccttc gggcttggtc 67140
   tcatttttta tttcgtcgat gaccgcacgc acgatttggc tgactcgttc tttagcttcg 67200
   atgctaggcg tgagacactt gagaatggca tcttcttcgc tcattaatcc gagactttta 67260
   aagtgctcga tagcggtgcg atcgaccagt cgatttttct tcaccatctc gacgtcggcg 67320
   agatatttct gacgcaactc gtcttcgttg agaatttcca tttcggtaca caatttgttt 67380
   acatacttga gtccgtagaa catgtgagga tatttcgatc gtatcacaac ccaaacggcc 67440
   actggattgg cgcatttaat ctgtaaaaat ttttccgaat ccagcagcca ttcgtagcgt 67500
   ttagatcgct ggcgtttcga cggattggat cgttgacgct cgaggaattt atcgcattga 67560
   tcgatctcgt ttcgctggca acggctcata cgaatccgtt ttttaccgtc gacggtgcgt 67620
   tcgtatccag tgacatattc ctctttttcg ggtcgattgc tcagcgatgg cacgacccgt 67680
   tcgctcacac tttccaagtt atttctcatt tgttcgttct cttttatatt gtcttgagcc 67740
   aaaagagtag tgacaccaag ttgcatcata ttcatttggg ccatatggga cattttgttc 67800
   aggtccaatt gcatcttgag cgcttgttct ttgtactcgg agatttgacg ttcgtaattt 67860
   attttcattt cacagatagt gttattcttg tcggcaattg tcagatcctt ctcggcgatg 67920
   actgtattct tctcggcgat aatcaaattt ttctccatca cttccatttt taattccatc 67980
   aactgttttt cctttgccca aattgccgat ttgccttcgt ttgtgacttg atgaattata 68040
   tccattccct cttgtatttc gacgggcgcg tctgcagtca tgctgtattc tccttgttga 68100
   caaagagtgg gcagcagatc gttggtgttc cacgctttga atcgcttcgc ggcaggcatt 68160
   gtgctagcgt tgatcaactc gaagacgccc gcttgattga taaacttggt cttggcctga 68220
   atgttccgag gtagaacgga tgagtcatcg gtcgatgtga atcgtatcgg gttaatttct 68280
   tcataagttt tctgattttc attagtcaca aacttagaaa ttgcaatatg tggtaagcta 68340
   taatttaacg ctttagcaaa cgggttggca gtcatccatt tttcaccatc gacatcaatc 68400
   gtatacgtct caacttcttt atcgtctaca aaattaattt ttgttagaga catgttttgg 68460
   taatttttat gcgggctgta ttctctttgc tgacacagtg taggcagcag gtccgagttg 68520
   atccattgct taaattcttg agcctgtggc atcttggagt tttgaatcag ctcgaacaaa 68580
   cccgctttgt tgatgaattt agtcttcgga tgaagtgatg acgtcataca agtctgcctg 68640
   ctttgggaag acttaagatt ttcgaaacac aattgatttt tatcgctgac gaacgttgaa 68700
   atcgctttcg gagcattact gtactccaaa attctggcaa acggattcgc caacatccat 68760
   ctctcgccgc tttcgtcgac gacgcaaacg acttcgagat tttcgttgcc gaagctagtc 68820
   atgattttgg atgtagtcat tttaaaaaaa ttaaaaagtg attatgacaa actgacacaa 68880
   tgtatctcta ccgatgtcaa gcgactacta attttttaaa aaataatata tatatatata 68940
   taactagtgt ttaaaaaaat atattgtatg tcattaaagt acatgacaat cgactcaaaa 69000
   atcatgtaaa agacatcaaa taacatgaca atgtaatgaa ttaaacaaca agtttttata 69060
   aaatttattt atttattaaa tttatagtgt aatctgcttg gatagtatgt cgttgttgtt 69120
   gttgatctac ataatgttgc agttgagttc gttatcatcg cgagacatgg ttcgccttct 69180
   cttattagcc aaataactag ctaaatggct gtcaatgtca tatttattat tctggttata 69240
   atttttgttg tcaattttcg catctttatt agtctggttg attatcttgc ccaattcgtt 69300
   atttctcgac gacaaattgc gtataatatt ttgcatctga gcttcgcgat tttgatattc 69360
   aagaattcgc ttttgatatt tgtttaatat catatccttt tgtttgttgt ctttcatgac 69420
   gtttactgtt ttttgttctg aatccagtag tttatgcttc atattccgta taacttgatc 69480
   ttgagcttct ttatttttct gatcttcgac atccatagcg tttcgagtaa ttttttcttg 69540
   atttttgttt acaaaatatt gaccatctgt acacaatttg atgagcacgg tcgaagcgag 69600
   ccattgacgt aaatcgatcg ctttttgaat ctgagaccct tttattaact cgagcacgcc 69660
   cgcgagattg acgaaaatcg tctcggttcc gcaccatttc gcacgttcgg gatcctgctt 69720
   cataaatgtg ccgacgctga tagcaagctt caaaaacttt aacttcttcc ggttatcttc 69780
   atcgacaatc ttcaaaatgc ttctatttac gccaatctcg agaatcgaag ccagttgagt 69840
   accacaaaac cactcctctt cgtacaaatc gacgacggag atgacgtgtt taaaatcgtc 69900
   tttaaaaaac ttggtaatga ctttagaatt catgattttc taccgattat tgctgacaaa 69960
   ttgactgaga aagtgaattt acactagctt tatacattag ttatcttgaa tgatatcgcc 70020
   cgataacgat aacaaatcgt cttcttcact aggcatgatt tcgtttgttt gtaaaaatct 70080
   cacacaatct ctgcaagtaa tgatttgatc ataatcttct tttttaatgt ttttgtcgtg 70140
   taatgttgtc gatttcaacg gtacacgatc tatattataa tatttgtgta gatgatctac 70200
   ggtagtggac ggatgagatt gagacatttc tatcgaatac aatctgaatc cagtccaacg 70260
   aatatttgta cgtacactac tatcgatcag ataaaaagtc gtcgaagaga taacgacaga 70320
   taaaatattc tttaaatata ctgcacattt gtatattcca ttattactcg tgtctcgtcg 70380
   acgaaaacgg tagcaataac gatggcagca aacgatcgat tgaagcgaat gttcgaagag 70440
   agtcgtatcg actcatccgg tgagacctgt cacccgtcac gcgttcgtgt cataagccga 70500
   ttacgcgaat tggtcgtcgc cagcgaaggc aaagaagaag gtggtgttgt ttactggatg 70560
   tctcgcgata gcagagttca agacaattgg gccctgatct acgctcaaga actggcgcac 70620
   acggctaaat tgcctcttta tgtggtgttt tgtatgacca agagttttaa taatgcttcg 70680
   atgcgtcaat ttcattttct gatcaaaggt ctcgaagaag ttcgtgtcga atgtcaaaaa 70740
   ctcgacatca cctttgtcat gctcgacggc agcgcggatc tagtgttgaa cgactgggtg 70800
   cgtgaacatg acatttgtgc ggtcgtgtgt gattttaatc cgttacgtac ggtcagagag 70860
   tgggtgagcc gaatccgtga ccaattgccc gatacagtat actttgctca agtcgatgct 70920
   cataatgtag tgccttgctg ggtcgcatcg aacaaacaag aatataacgc tatgttcatg 70980
   cgcaaaaaac taaattctaa actagaatgt tatttaaagc cgtttcctcc tgtcgttcga 71040
   cacgaatata aatccagtgt tgttctcgat ccgtgcacta cgacacacat cgattggcaa 71100
   caactgttgg cttcgcgcga cgctgacgtt tccgtgggac ctgtggattg gatcgagcct 71160
   ggttacatca gcgccttgaa cgtgttggcc ggattcattc gatgtaatct attaaagtac 71220
   aaaatgagtc gcaacaatcc cgtattaaaa acgcagagca acatgtcgcc cttttaccat 71280
   tttggccaaa tatctgttca acgagtaatg ttgcatctca atagccttaa aatcaataaa 71340
   aatgacagtg gtatcgattc aaacatcaac gattacatcg aagagtgttt tgttcgtcgt 71400
   gaattagcag acaatttctg tttctacaat tccaactatg acactttttt aggcgctccg 71460
   aattgggcca aggagactct ggtgattcac aagtccgatt ctaggcaata tatttatagt 71520
   ttattgcaat tcgagcataa tcaaacgcat gatgaactat ggaatgctgc tcagcgacag 71580
   ctacgcaacg agggtaaaat tcacggatac ctacgcatgt actgggccaa aaagattctc 71640
   gagtggactc gaactcccga gcaggcgctc gagtattcta tttatctaaa cgatagatac 71700
   agtctcgacg gtcgcgatcc taacggttat gtcggttgca tgtggagcat ttgcggtctt 71760
   cacgatagaa gctttttaga acgtcaagtg tttggtaaaa ttcgctatat gaatcgtaat 71820
   ggttgcgaac gcaagttcaa tgtgaaacaa tacattatta ataacaaata aatgatttat 71880
   aactattcaa ttgttttatt cgcatcatcc ttgaaaaata gaatcaaact gacgaatccc 71940
   atatttatgt attacgaaat atacaatata agtcacaata acgtttgaac tcgaacaata 72000
   aacacgtttc gcgcttaata ttttttaata aaatatggct caagtcgacg ctggcgtttt 72060
   gactattcag ttcgacatcg gtagcataag caaaggatac agcatagact cgagcgacaa 72120
   agatatcata attgtcacaa aatgtacgcc ggagaagttt atggattatt tatacaaccg 72180
   tactctgctc gtcaataatc acaccaagac tgttgacggc aattcgacta gtgtggacct 72240
   gtacaaagct ttacatggaa tttacacggg tcgctattac tatttgggcg tcttcacgac 72300
   aaaggaagat tttcttaaca ccaatggaac gtacgacatg gaattgtata catttacacg 72360
   cgagttgacc aggctgcgca taaacaacat actaaaaacc atgatgatgt acaaaatcaa 72420
   aggtaacaat ccggacgcgc ccaagttttt gttgatgacc atgtttcatt tggcatacac 72480
   agagtattgg ctgaaaaata acgattttcc cgactgtgta aagctaccga gtctattgca 72540
   aaagtgtaat actcgtaaga agaaagtata tcgtcatttg atgcgaaaca gaagagaaaa 72600
   tacagtaccg cttcaaaggg acattgacta catcaacaat tggcaataca ggttgaagat 72660
   tcaagtcagt caactaccag ctaccaaaaa tcgttacgat gtcctcaaga taataataat 72720
   gtacatgcta ggtgaatcta atttgtatat tcctgaaata aataaagttt aatcattcaa 72780
   cttttatgtt taattttgta gaataagtat ttcaacaaat ttaaataaaa aaatacacaa 72840
   tatgcatata cgatcagtta ttctagccgt agttttgtta ggctatcaat atgccatggc 72900
   ttgtacagaa acaggtcgta attgtaaata cagcgatgaa tgctgtagcg gtgcttgttc 72960
   ggcagtattt ggattttgtt tacatagata gatatacaag tcaaataaaa taatacaaat 73020
   tagcatttta ttaaaatcat ttattttttc tggtatataa aacaatcaga aagacaattt 73080
   ttacaaaaat aatctatata ataattacta attagttgtt ctttggtcaa cacgtgtcca 73140
   cttacgcaac gatctaaacg aatccgatta tcgtcatggg ttacgtatct taaaggcagt 73200
   ttgtattcca aaagtatagc tccataattt ttttgtaagt taagcgtcaa tcctgcatct 73260
   attatgtgat cacgtagcat gcaagccgta ttcaggcttt ttatcttgcg aaagggcctc 73320
   ttgaaattaa tcataagcac cttaaaacac gaatcactca aagacttgac tgttggtttg 73380
   gcacactcga ttcgagtata atcgtacact acacgaatat gtcttgatct atcgcccgta 73440
   tagaaacgat attcggtcct taacgcttcg ggtatatctt ctaagcgaac ctcaatatta 73500
   ttagtattat atactctagc acgcaaacta gattttctac cgcaaatatc gacatcaata 73560
   tgcatgtccc tgaacggttc catcgcgcaa atagaatcga cccgatcgac aatcgccgac 73620
   agaatgaaca aattcgattg catagctgag ttttattcga tatactgatg acatgattta 73680
   aaaataaaaa attatctaat cttaaaagcg tttattaatt tatactacta ttaccgctgc 73740
   tctcgccaat ttcgtccgat tgttcgcgta tttcctccat atcgtttcca ttgttttcat 73800
   cttcgtcgtc ttcgtcgtca tcttcgtcat tatcgtcatc atcgctttcg ttgattccga 73860
   catcgaattt gtttagataa tgctgtgtac tcttccacga ttcgtggttc attaattttg 73920
   cgactttttg cagaggtaca ccattattgt attgattgct gcacaagtag tgtctaatca 73980
   tattcgatct gggtctatcc atctcgacgc cggcttcttc gagaagtcgt ttgaaatctt 74040
   taaatggggt agaagtgttt ttcgagatct gcaagatcgt cggatgctta acgtatatct 74100
   ctcgcgccag ctcgagtggc ttgtttttaa tgttattcaa agcgtttata cggttacgtt 74160
   ttcgtttcag attaatgttg cttcgaatgt tgcccttttt tattagtgta tttaggtttt 74220
   ctatggtcaa ttgacgcgct tcgttgatgc gcattccagt gcctagcatt atacaaaaaa 74280
   cgatagcgcc tctgagcaga ccccgatcgt gtacatactc tccgttcaaa tatttaattt 74340
   ttttctctat acaaacgatc acactgtcga taatcttttt caacactata ttcttttctt 74400
   tatctcggat atttttgagt tctttatcgc ggggcaacat gaccatttta ggaattttat 74460
   actcgggcaa gctcattgcg ttcgaataaa aattgatggt gagctggaga gtttctttgg 74520
   tgaccgatct caattcaagc attcgacgac acagctcctc ggggtccacg attgcttgtt 74580
   gattttcgat agaatcgaac tctcttttta gcgtataatt attgatatca tcgagattac 74640
   gatcgtcgat gagacaatag atgagtttta tcagacgcga cttatagctt tttaatgtgg 74700
   tcggagcgaa gggtttattg aacatgtact gtgaccacat gctgttgctt ttgacctgat 74760
   ccggcgagca tcgctggcgt tcggtggcaa tctcgaatat ctcctcgaat ctttcgtgac 74820
   tttgaatttt acatttccaa ctattgaacg agtactcatt tctaagattt gcggcagcat 74880
   ctacgtcatt catgctagaa taaaataggg tggtttgact atacgttttt ctcgttctct 74940
   tattattaca aagtaatata tggcatacaa tataactaga acacaaaaaa tagaaagcaa 75000
   tgccatcaag accacattta aaattccacc ttcagcagca ttcgcgtacg cagtataatt 75060
   agtattgccg atgtcgttta acaataacga agacgatggt cgagttttat tgataccatc 75120
   gtcctcggtg tcgttggtct tttgtttagg ttttttatat ccattttcat cgatatcgtt 75180
   cacggccaac ttcaacggta tatactctac ttttttttga ttgagagaca tgcgctcgta 75240
   aggcacctct agactcatga tcgtatcacg tgtaattatt tcagttgttc aaattttgtt 75300
   tataacttaa ctgttgaaaa ataaatctac gtaacgcttc gttttgaaag gccaattcag 75360
   tgaggctctg ctgacaggga gttttatacg attccgtgtt tttcgtcgaa gtcaatgcag 75420
   ccgcactctc aaaaagactg gcattgatgc ctttttctgt gctcagatta gcgataaatt 75480
   taaaaatgtc cgtcgatgcg gacaatggtg ccaattgact tatgtttcgt ttcaagctga 75540
   gcacacgtga tcgattacgt tcgtttagag tatacagata atagtcactg ccgttgccgc 75600
   caaacaaatc gtcaataacg ttattgatga tggtgttgat catcaacagc cgaggatagc 75660
   gttgcttgtc gttcatcccg tattcgatag cgttttgaat gttttgcgga atagtggctc 75720
   gctgaagtaa cagagtcatg taatcgtttg caatcttctg gtcgaacggc agcggaatgg 75780
   gggtgtttgt cgcgatcgct tcggctattt gatattcgag ggcgaatccc aattgacgag 75840
   cggcctcttc gacgctgctc gagtgtagag ctgctgcatt attattataa aatttatgag 75900
   cgtacgccgg caaaacatta tagacgaaag aagatctgaa gatgtcgcta gtgatctcgc 75960
   tggatcccaa ctcttgttgc agacgtgcat aatgtttgat gagattttca tcactatcga 76020
   agcgcttggt cacgttaacg tcgaccggtt gggtttcgat acacacgtta cgtatggtac 76080
   tgaccagatc tagcatttga ggcgagagat gcgacatatc gttggtgcta taataacgaa 76140
   taattttacc catatagtcg acacatttat ttaaccacat gtcattgacg gaagacggca 76200
   cggctgccga ggacgtcctc tgttgctgct gattcatagt aataaatgtt acggtcgtga 76260
   ctataataaa tttttgattc gcgtttatgt ggacacacga tgattcgagg atcacttatc 76320
   atataattat tgttgatata caaaatcgct ataataaaaa taaccaaaaa aataaaatac 76380
   cacgaaaacg taaacatatt ggccacaatc acagccaatg ccaaagaaac aaatacagtt 76440
   tgcatgctct ttcgtttaca gagaatactc tcgcaatttt tgaaggcgac gttaaacttg 76500
   ttttcgccat cgataaacgc tcgaagttcg tctttacagc actcatcgca aagcaccaaa 76560
   gccattatca tggtgccgtc agagtgcact atttgaaagg ttttcggctg actgcccgga 76620
   tgtaattcaa acgaatatcc gttcgatata tcgatgtgag catagtaatg cgccaacatg 76680
   gtgccgtagg tcttttttac tctagtttta cacactttta taatgttcaa gtcttcgcta 76740
   tccgttattc cgtcgaacag ataatgaatc aataattccg gatcatattt aatgcgattg 76800
   agggtcgttg tcaagttttt gtcgtttaat tgatagttcg ccgctgctgt cgtcatcgct 76860
   gtcggaatat tccatggcat ctgatccgat tccattaccg ttaattttgt tgttttcgtc 76920
   acgatcttca cgactatcga ctcgtgtgtt ctttcttatc tcgtcgtcct cgtcgttgct 76980
   gtcttcgtcg tcgtcttcgt cttgagattt atttccaaaa gtttttagaa ataattgact 77040
   gtttgttgat tttctcaacg gctcaatgtt gataccgttg acgtcgacgg cagtgatatt 77100
   attgttgatg tcctcgacaa aattgttctt taaatttatc tcgtttttaa gaacagcttc 77160
   cgtcatctta tcagtataga ctattttaaa aatacaagca cctttcttga ggatggtcgg 77220
   tttaatgtta aagagaataa cgttaagatt gttgctcata ttaaaattat agactactac 77280
   ataatcgcct ccgacgatgg tcgacgaacg agactgattt tgaatcaatt tcgctagcag 77340
   ctgagtgtcg aggatgttta aattatatgc gccaactgcc agttttttta agtgatattc 77400
   atcctgaaca ataaacgaaa gaatattttc atcattttct ttatcgagtg tcacatagac 77460
   ggcaatgtcg actgtgccgc tgttgttggt ggccatattt ttacttgtaa tattttctat 77520
   aatatactta tcaatataca atgaattcga cgaacgagaa ttcaataatc gtctcaaagt 77580
   catgaccgag tacataaaaa gaacaaatgc cgaacatccg ctgccggttt cgttaagtta 77640
   tgtctctggc gtcgatcaaa atttttatac agtcactact ttttccactc aaaatttaat 77700
   cgtagaacat atgagcatac atgacgatcg acacgaatat tttaattttt tgactcaaaa 77760
   atatgaaaaa gtcaacaatt atgatgatgt tttagtcgat tcactcgcca ccgtttcgga 77820
   caacaacaga gtcaaatctc attcgagtga tccgacaaaa tatttgttgc gaggcgacga 77880
   cggttggttt gaaatgaatt gccccataga agagcaattc gacgccaacg cttttcgttg 77940
   tgtaccgatt cccgtgtgtc aaaataaaca agtcggagtg cacggtatga cagaaaatct 78000
   actcgacaga ttagtgctga atcatcgaac gcccagagtc gtcgatgacg ataacgttca 78060
   atccgaagaa attcatccta ctatttatct tagatgtgtc gaaggcggat cggccgtcgt 78120
   cgaagagtgt cccaatggat atctttttga cgtttcgcaa ggccgatgtg taatgcgaaa 78180
   cgactgtgaa aatagacccg acggttatgt attatcacaa ttccctgaat ctctaaacat 78240
   cgaccagtac atgatctgtc gtgatggcga tacagtaatt cagtcgtgtc ctgtcggtca 78300
   aatattcgac cgaaggctga tgtcgtgtgt ggaggcgcat ccgtgccaat ttaatggagc 78360
   gggccacacc tacataacga acgatatcgg tcctacgcaa ttttataaat gtcaagattc 78420
   cgaaaattcg attctaatta catgcatcaa tcgggttttt gtcaacgacg aataccaatg 78480
   ttcgggagat tctcgatgct ctgttttcga aaacggcacc ggtacccaaa ttaaagtttt 78540
   tgacgatgaa atcttgtcgt atgaccaagg aattctagtt tgcgacaact ataacgtcat 78600
   caaaaatatc gattgcgatg acacaaatat tttagaaaat cgcatttaca acaatcgttt 78660
   cgtaccgatg atcaatctac caaaagaaat ttattcgata gctgactcga aatgcgtgtc 78720
   gttcgatttt agtttagtta gacaaaataa cgacactttt ggcatcgaat ccgtgcccaa 78780
   tgactataat ataaaattcg agacggcttt tacgggtcgc accagcaatg cggacagact 78840
   aatcgatatc actcgaatag atagtgtggt gtcttcatac gcccgcgact ttaatttcgt 78900
   cggattcaat tttttaaatg aacaagaaat taattgcgtg caaggatctt tatatgatat 78960
   ctttgaaggc cgacgattaa acttgtgcac gagtgatgac aattttagac agattatcat 79020
   tcgtccaaac gagttttata aatcgaaaaa cgtgctactc gaagtcgata gcgactatag 79080
   agagtattgt gccgctcgat tagacaataa tatgagtcaa atattcatat tcgataattt 79140
   tctcgaaacg ttttcggtca atatacgaca attcgacgaa tgtcataata ttctcaataa 79200
   aattcatatc aaatatacta cgattgacgg taaatatact accgtacagc ccgaatataa 79260
   cttcaaaagt gtaaacgacc aaaaaaatat cgaaagatat gcggaaaata tacaaaattc 79320
   cgaggacact ataccggtgc ttttcgaccc gttcgagcac atcgaaaccg taaagcctct 79380
   gttcaatccg tttgcagaga acacgattgc tagagattta cccgattttc cagacttgtt 79440
   cccgccttat cctcctcctc gtcctcctcc gccaccacca ccagtcatac cagagcctga 79500
   accagagccc gaactgactc taaggaacaa atttcttaat tattcttgct tcttttcatt 79560
   gcccacattt aaaatgagtc attgtgatgt cgtcgatgac cacataattg aagctatcgc 79620
   taatattaga cagaacgtac aggtgcacga tgattgtgtc aacgctgaag gcatcaaaaa 79680
   cgtgattaat gcctacgttt atctgggaaa tgacatcggt tgtagaacag aatttaccga 79740
   tgacggcact atacatgtta atcgtgtaaa cgaacccacg aaatttatga atctgaccac 79800
   tcaatcaaac gacggcataa aatacaacca ttggatacat acaaacgatg gaataaatta 79860
   tttggcatgt cctcacaatt tgtacaatag caaccgattt acctgttccg tcgaatcgaa 79920
   cagattctat tacatgcaag atttacagaa ttaaagaaaa aaaactattg tatatttgtg 79980
   taaacgttta atatatatta aaataaatgg tcaaaacaga aatttaattt tattcattca 80040
   ttcattcacc ccgcaataaa gggttctgta acagtcgcgt tggctggagg aaacggtgga 80100
   ataacttctc gcggcaccga aatcggcatg atggatgccc gagatccgtc gtcgggtcta 80160
   agaatttcac ttcccatgat caacggcacc atcatcggat attgttcgta tcgggcgaga 80220
   gatctctgta gagcagtggc gttgccctta aacttgagaa cgttttcgat ctgtaaaaag 80280
   ttttcgttta tgcccgatct atatttgggt tgaacgggat tatagaaggg cacgttggca 80340
   acgagaccgg tgctgtcgaa tcggcacgtg gcacaatttc taaactctaa actctccgtc 80400
   gatatcttca tgatttgagg cgcgacgcac ttgttgatga gattttgaag atagggcggc 80460
   atactggcga ctactcgttc agagatatcg tcattctcat cgtcccaact ataggttcga 80520
   gcgctattgt tcttgacgac cgaacagaat ctcgtgggat ccgtctgcga caggacatga 80580
   gtagtgatcg aatatagatc gtttactatg ttcactagtt ccatttgaaa gctctcgttg 80640
   tatcttaacg attgacacac ttgttcttgt gcttcatagt tgttgtagat gatgtgtaac 80700
   acgagctgtt cgggcaaggg cagtgccata acgttaagca ctgtctgata gtgttctttg 80760
   gtgggaataa gtattcgatc gttgcccacg gctttgtgat tgacgaggct tttgccgacg 80820
   gtacgatagt atgtgttacc ttcgccatcg acgatcggca tcgacatttt ctccatttta 80880
   aatcgaacgg acgcatgaaa atcgcaaatg aaccaaccgt cgtcgacatt cgcatccgga 80940
   gaacacggag agccgtacgt tcgacacaca tcgaacggcc gtaccgcttg gaagatacaa 81000
   taattattca tacggttgct gatgctcccg ctcgacacta gtgccatggc tatcgaaaat 81060
   gaaatctctt attcaataaa tttgagccaa gatttgttgt atattatttt agattcttat 81120
   atttctaaaa agtttattgt gacagaagaa tacactgatt tcgtggacga aaacaacgtg 81180
   cgcactcgac gatatgtcgg cgaacataga ttcgtgagcg tgaagaaaac ttgtaaaagt 81240
   ctcgaaaaaa tagttttcgt cgacaagaat attgtggtgc cgttcgtgaa tcgaattagt 81300
   ttcgaagaac ctccagacac tacgcccacg aacagcttgc gaagaataat aaagtgtaaa 81360
   gtttacaaaa gtgataaatg ccccgactgt gagataaagt tcgagcacgt ctactttaac 81420
   aagaacaaaa ttgacaattt agattcccta atggccacga aacagatggt gctctataat 81480
   ttactacaaa ataaaaacga aactctggtg aagcagtctc atttgggatc ggatgaaata 81540
   ctggccaatc tacgcttgga atatgaatac acgacgggat ttgtcaatag tgacgtttta 81600
   aaattcatgt ctacaatcgt gcgagacttt gacgagattg tagcgtataa gaacatcagt 81660
   cctatgttgc cttatactac gctacagaat catataatct acaggaaatt tgaagatgaa 81720
   aaatgtctaa tggataattt tgaagacatc aaagatgtgt ataaatgggc actaaaactc 81780
   gacggtattc gaggcagagg gtttatcacc aaaaagcaaa taataatttt tatggacgat 81840
   atgcaaattt tttccggacc ttttgattcg ccattcagca tcaacaacgt gattgccttt 81900
   caatgtgaac tcatagaaaa aaccctatat attactgatc ttcttcacgt gttcaaatat 81960
   acgtataata atcgtacaca atacgaatgt tccttggatc catacaatat agatcccgcc 82020
   accgctatcg attgtatcaa tttaatggct aatctctaca acaaaccgtc taacgacgac 82080
   aacgacaatt tcaagattta cacatttaaa aatgaaacga tcagcattaa atttcaaaaa 82140
   ttctatgatc cgccgatcaa atctcttgga tacagcacct tgccgaccga tggatttgtc 82200
   gtgctcgaca cgaacatgag ctacgtaaaa tataaatact ataaaaccgt agaattggag 82260
   tacgataaga aaaaagatgt gtttaaaact ctcgaaagcg atctcgaaca atacatggtc 82320
   accattaatc cgtgcgtggg tgaattgaaa cacgagagca tctacgaagc tatcgtccac 82380
   gataatcata taaatgtcat caaatatcgt cccgatagat tgattccgaa ttgatttaga 82440
   cacaatttcg gagtgatcga ctttgttttt gaaaaatata aataaataaa taaatttaca 82500
   aacagaatta aaattggttt tattcgataa tgtattacat gtgtaacgac tttttgtatt 82560
   cggcccatgt catcctctca aagtgtttaa caggcatcat attcctttgt atgtaacgat 82620
   aatcgtttat atgattgtga aagaccattg tggcgtataa agttccgtgt ttcattaatg 82680
   tgttttgatt agaatttttg atggtaatct cgtcgaccat gatgattttc tcaccaaatt 82740
   tctctcgatt caaactaatt tcgatgcgtt ctatagaaaa tattaaaaat cctttaatac 82800
   ccaaataatg ttctcgacac attgcacagt ttaatttaaa aaataaatta taaaatatgg 82860
   tcttcatttg ttggagattg gcggtgacaa tttcgtaagt tagcttgtct cggctgtcga 82920
   tcatgtcgtc gacgacgata gcgagaaaat gaatggtatc ccagattgtg gtgaaggaat 82980
   atgaatattt tttgggttga aacgcccgca aattgagctc ttcggtcttt tcgataaata 83040
   acacttgcat ctgttgcaaa tcaaaggtgc tcggcaactc taaagcccag ttcttcagct 83100
   gttcgatctc gttcgcttgt tcatctttgt aggtgatcac gcaagctatg ctatataaat 83160
   aagtcagttc tgtcgctaga atttcggcaa tttctctcga ttgacaggtt cgtattcggt 83220
   ctatgtgacg aaaagcgtac agaaaaaaac tatctttata acgagaaaac aaaggagtca 83280
   acggtatcat gattaacgat tctgaaaaaa ctatttatct atatctatcg gatatgccga 83340
   acggtattca gaacgacaag gccgacgatg gcgatataat ctattttgag ggaattatcg 83400
   agtgtttcga cgacgagacc gccgataaat ttggcttttt cgctgagctg aagaaagagg 83460
   aagccttatt tatgaaaaaa acattttacg atctcatcga acacaacaac ggcaactatt 83520
   gcaaaaatca tgtactaatc gacgcgctca ttatgtataa gacgtatgcg gaattggtgg 83580
   acgaatcggc atttggcgtg aacatactca attattgtgt agaatacttg acacacgttt 83640
   tcaagttatt tcgcttgcag agtcgcataa ttgttgtact gccgacacac gtagactggc 83700
   agcaagataa tttaagtgct cttttgaaac atttactaca gtccacgctg attgaaatcg 83760
   tttcaaaatg atcggaactc tagtattgat aatcatagtg ttggcggtgt tgtacttttt 83820
   gtttatcaac aacaaactga acatgaactc gatcaacgaa tcttcgccga gtttagcgga 83880
   cagcagcaat tccctgcaga ccgacccgca gaccggacaa ttgagcgtca agttcaatag 83940
   tcccaaaatc aagtcccttc gcgtgttaca cggagaaaac aaaattagta aaatctacgt 84000
   cagcgaaaaa ccattgacat acaacgaaat catcgacgaa ggcaacagat ccgtgggcac 84060
   gaattgtgtt tttctaggaa ctcttcttga tacatcatca tcatcatcat caccatcagc 84120
   ggcaaatgcg actacaacga ctcgcactac taccaatttc gatgtgaaac aattcaaaaa 84180
   cgtgttcata gtcttcaaaa atctcgaatc gagcaagatt aaagagtcta caaacatgac 84240
   tcgttacgaa gccgaaggaa tggtgtattg tttaatcgat tcaactacaa cgaccgtgcc 84300
   cgatctaaga gacgtgtcgt acccgattgt agtgtacacg accaatgcaa acgcgcagct 84360
   caagctcaag gaatgggact acacacagct caacgatgct ggaactctct tcctaaagaa 84420
   tgaaaaatct tttaggctac agtaatttac cgataaatcc taactatgaa ttgaaagata 84480
   gactacttgt aatttatgat aaaggaaaca catgttaaaa taataaaaaa atttttgttt 84540
   aatctctata tttttctaat aaatatacaa aattcaaaca cattttttat tcaaatctag 84600
   cataaaaatt ctggtctatg tatgttgaat tgttccttgt tccaggccat gtcgatgctg 84660
   tagaagcatt tttcatcttc tttatagtac tttttatatt tacgtttaaa agcactaaac 84720
   aatgtggcaa ttcgttgcga accgttggcg tttcgtttgc tgaccaacag atcgtgtagc 84780
   atattctcga cgtagggaac ggcaagctcg atcatcttct cgatctttgt ttcgtcaatc 84840
   attttggctc ccggttttac ttcgactttg agcacgtaga ttaaagcgtt caacggacta 84900
   ttgttcacgt ccagacacag tagattgtga ttgtgtatag gatcgtgttt aatgatattt 84960
   ttatagggaa cataaccatc tttggcgttt cgcttgtaca tgagtacgtg agataggaag 85020
   attcgaaccg gtttcgccaa atcttcattg tagtttttct ccatcggata gagtttattt 85080
   ttaatgtgcc aatatataga cccgttgaat ggtaaatttt cttcaaagag atgatcagta 85140
   taaatgacgg caaatctatt gcgcacaccc ttgtcatagt ccgagatgtg cagcggttta 85200
   ttattgacga tgagcaattt ataattagcc tcatactttt gactgccctg aaactttcga 85260
   cagaccgaat tgctcttggt cgagtctgcg gtactcttga agaaagaatc gtcacacacc 85320
   ttcatttcat tgatcacgta cagctgagag atcattttat cggcctccat ttcgttggtg 85380
   tccttcttgg atagagtgta cttgtcgctg tcgtgtttat gcactacgac tatatactcg 85440
   agcagttcga aaaagctcga ttttcccgag cccggcttgc cgttcaagta gatgcagcac 85500
   ttttcataat cagagggtat gcccatgctc gccccgaaat gcatcataac caaagagttt 85560
   atatagttaa aattggtaaa ccctcggaaa tagagatatc ccctgacgat ttgctttaca 85620
   aaagtcgaat tatactcgtc gagaccgagt ttggtcataa agattctcat ataaaatctg 85680
   actaaccacg agttgaggtc atcgttgggt ctgcgtatga tcaatttgtc ccaccataga 85740
   ttccacctct ttaaaatgtg ccatgtgtta caatagttgt tgtaaaaatt aattaaattc 85800
   tctttagatt cttcattttc gacatctttc aaactgtcgt ttatccggtt atcgtcagcg 85860
   aaatcgtcat tatcgtcatg attgttttga tttatacacg atctagacga caagacttgt 85920
   gttttgagtg tctcgatgaa atcgtctggt gacgacaatt ccgtaataaa attatcaata 85980
   aaatcgattc tatcaatctt cgagtacaaa taatcgatta ttgattcgcg atgttcattg 86040
   aaaaattcta gattattgta gagtaaaatg ttttcatggt ttttcttcag tgaccatttc 86100
   aagttgtctt ttaagacttt aaaatataaa ccagtcgagt agagtagaga ccaagacagc 86160
   tcgatgatgc ttcgtgtttt cgaaaacaac tccatgatta gagcgattct aaaagcacgc 86220
   gaattaatct caatattcat cagacattgg cactttttct tttgttgtgc cgatttttgc 86280
   tccagacact ctgaacactt tagattgatc atcagatcgg tcaattttgt attgctcaga 86340
   tagacaccaa tcgtgatcaa ttcgctatga tccgaatgcc aaatttcacg aaatacatca 86400
   ttcagagcac tctgttcgcg gagcttgcat ttcaagcaat tgttgagagc caatgctatg 86460
   gacatgttgt tttttaaagt ccgaatatct ctacagattt tagccatatg atatgtcttg 86520
   taaattacat tttcgttctt gccattgtcc atcataaatt gtatgacacg ttcgggaaga 86580
   taggtttttt cctcttttcg ggtcaacgat tttatcagag tattgcccag aagaaaagga 86640
   caactgctat ggtaatcgtt aatgaacaga ttgtaaacac cctcttcagt gaaatataaa 86700
   tatttccagt tgttaaactt gacgctaggc agtgttatgc cgttgacaga ctcagtcaac 86760
   ttgtggagat cctccttttc cttgacggca atgtaatgct ttccgttata tacgagaatg 86820
   gtattggccg atttgatttt attaaaaaat ccactacata acacttcggc agtagcgcca 86880
   cagctttttg cgagatcgta tgtatagtcc caggttttgt ttttgtaata aatcaaatag 86940
   tgaatagcgt aataatgggc caacgatacg cacggatcca cattcaagaa cacggcccaa 87000
   tgattacaga aacgaataaa gacttttggc gtcaatctgt agtatggctc gcaacgcttc 87060
   ttcgccaact cgaaattaat tcccttgtct ccgaaaatac tctcgaccac taattctaga 87120
   tacagtttta tgtcggtctt gcaaaactcg agttcttcaa ccttggagat ggctctccat 87180
   ataacgatta taatgtaatc gaaattcata aaattgctgt cgataaaata attttggaga 87240
   actctagtat ctgccaaatc ggtggctgtc aatacttcag ccatattttc gattatagtt 87300
   tccacacaat cgagtatttg ctgctgaaat ttgtccaaat tctcactgct gggagtgata 87360
   ttcttgtggg ctttcaattt ctccttgcca atcaaggcgg ctggcgtttc aaattccttg 87420
   tcaaacgcac gtagattgtt tagattgatc tgaatcatgt gtttatactt ttctgccgtg 87480
   atacatttcg ataaatttat atcgtctcta atgtagtcga aaggattttt attggaaaac 87540
   actaatctat ccttgactct gcagacgcct tcgcctccgt cgttcatcgc cagatgaaac 87600
   atgatttcgt cactatcgtc gaatctactg cggccgttga caaacatggt cctgttgtct 87660
   tcgtagctct tgatcgacaa ctccacattg aggaaatctt caggattgaa cacaaacaaa 87720
   tttacgtttc ctagcgattt gttgtgaatc aggggtattc tgttgccaat gtcgatgtcg 87780
   tgcttcatca gtaaatagtg gcgccatcca tagtaagata taggaatatt gggccatatg 87840
   taatagctgc cgactctatt acactcgttg ccgaatccgt tttgattgct gttaaaaaat 87900
   tcttcaaagt tgatgtcatc tctgatacga tcgtacaagt ccacgtgaat aaagggattc 87960
   accatcaaat aaaagtaatt gtcttcgaca aaccagtcgt gcggcttcat cgaagacaga 88020
   tcgtgagtag tggcgcacga gagtcgttgt tgagtttccg tgtgtccgtc gattaacatc 88080
   aacactagtt tttcaaaatt ttcataatta tcgatctgca atctttgctc ggtcgattga 88140
   tttacaataa cgatactatc gacattgact gtattgttgt cgccgataga tttaaaaatg 88200
   ttatcgaaaa tagtattaga tctaatcggt agtgctgcca ttttaggtct actgattttt 88260
   ttaatggcgc ttctgtttat aaatccttat cggcgcgaca tagaaagatt agtgagagat 88320
   cacaaacaaa ctttacaatt tggcgcttac attgacattt tcgatttgag cacacgacaa 88380
   caaatcgaac gactgtttat aataaaaccc gaaaacgtaa tattgtacaa tacgaacggt 88440
   agtctatttt attacttggc atcgaccagt ttattgtgtc caaacgagtt ttctttggtg 88500
   cgcttcactc gacccgaaat tgtagcattt aacgagaacg gtcaagccaa taccgtctgc 88560
   accaatgtaa acagtttagt tatcgttgaa cactttatga ctctcaagaa caacatcgcc 88620
   gacgacagaa tacttttgtc cgtagacgaa atccattaca caattattga tatcataaat 88680
   ttactcattc gttcgggata tgtatttcta caataactgc aaaattttca acaatatcgt 88740
   tgacgatgac cacgacaaag tcgtcgacaa tcgtttcaat aatcgtttca ataatagttt 88800
   caagtgtttc gatggttaat ttttaattta attacacaat gaccacaaac taaacgataa 88860
   tgtttgcaat tttaaagata tttaccacaa ataaagcgat tgcggctaca atgtcgaaga 88920
   catcgacaga tatctaaacc gatgcggtcg tcaaaattat aaagactatg accacgaata 88980
   taaagacaac ggacgtgatt tcaaagacag actcgccatt tgtaatcttt tacaaattag 89040
   gttagaggcg ttagatataa agtatttagt ttcatttttg ctacagtctt taacgctcag 89100
   caatcgagat gggacatttt ttaatccaac gagaatcgat ctttgacgat ttgatcgaat 89160
   ctagtgtcgg ctttcaagat gtggacaaat tcgactttat taaagacatt atcgtcaata 89220
   ccgatgtgct ggagagggat tgtgccgatt ttatggatcc taatttagaa tttaatataa 89280
   cggctccaat ctacgaattg tgtcatgaaa gtttacttaa aatcatttgg attgccaagt 89340
   acggaccgaa ctgtttttta ccagagtttt ggactttttt tattagaaaa tataacggaa 89400
   aatgctataa atgtgacaat cgtgcacagt tacgttatcc caagtcttgt aactattgcg 89460
   attccgatat caggcaattt tattatcatg acgatggaat aatattcgaa gaactagtca 89520
   atgacgataa taactattgt tcttcatgtc gaaaggctct attcgacata gtggatgtgt 89580
   atcaatttaa tcattccatc tgtagatgtg attattaaat aaaaaatttt tttatatatc 89640
   acaccattaa catgcgtttg tcgtcgacga ttctgatcga tcacggagaa aaatcttgtt 89700
   ggatgttgat cgatgtcaaa aaacgtaaaa tcgattcttt aaacgacaat aacgacaata 89760
   tcgtcaaaga cagcggcgtg caagataaac tctgcgtatt tggtgaaaat aaccacaaaa 89820
   ttatcatcaa cgacggcgac gacgacgacg acaacaacga tggtagattt gtaaaaagga 89880
   gaaaaataaa ataacttttt gaaattttgt gttttattta tactatatat tatttgattt 89940
   gaaccaactt tcgtattcgt caatgttgtc aacgatttcg tcatgataaa tatcccaatc 90000
   gttgatgggc tcgatacatt tgcgaacttt aataaaataa tcatacgaat aatcgttcgt 90060
   tttcaaatcg tcgacgagag aaatcgtttt tatgtagttt attcccgctt ttctgaggta 90120
   atatagcact atccttgggg acttgggaag tttggcatcc tcgtcgttgt tgacgttaac 90180
   atctgaataa aaagcctttt ccacgaaaat gtgtttcgtt ttattatcgg caatcactcg 90240
   tctcgtcatt gtcgacgatg gcgtcgacga gcgttgaccg ccgcagatta cgacatcaaa 90300
   atacggcacg agtctagttt tatccattga ataagtcacg tgctctttgt ttccataaga 90360
   ccaaaggacc aagacaaatc ccttttcttt gagggtaatt aagctgtcgt aaacgtcatc 90420
   gtttcttatt ctgacgtctt cctcgtcggt gatgagagta ttgtcgagat cgaagacgag 90480
   aacgtgcggt atctcccatg aaaagctgtc cgtttgcatc tgataaattt ctaaatagtt 90540
   ttgaacgtac cagtctttga gaaaccaata cattggtatt ttttcattaa ttacatacac 90600
   gtggcctagc gcagacgttt tgaaagccag ttttaaattt actcgaattt cacgcatatt 90660
   gtcttcacat cgtaccacgt gcatgatgta attgttggtg tcgatcgagc gagcgttgaa 90720
   cgactttttg tcaagggcga acacgataaa ctcgaacaga tccaaatgtc gaaaagacac 90780
   cagcgaaagg tcggcgaagt cgcaaacgac aagaatgtgt cttttgacaa gagcggcgcg 90840
   tctgcgaagc acgacccagg tcggtggcgt cataattgtt atgaattatt tttaattttt 90900
   aaagaatttc gaaacaacaa tcaatacaag gagctgatac aatttctcgt cgacaactat 90960
   ccggccaatg tcaagaataa aacttttaat tttgtcaata ctgaacattt atttcattca 91020
   ttgtacgctt atataccggc catcaccaat gtggaaaaag aacgaaagca aattcgtcta 91080
   tcggaagaat gtatccataa actgtttgtt aatactataa acgattttaa actatattca 91140
   gagctattcg acatgataat cattcagaag atgaaagaga cctgtccctg tcaattgctg 91200
   ctacagagaa gagaagaaat caaagtgtat gtaaacacga ttagagataa aaaattcgat 91260
   tctaaaccgc caaagctcaa aaaagagtac atcgacaaca tcatgtacaa gtactcgtta 91320
   aattggaaaa atcttttatt gaaaaaaaaa ttatcagaaa cgagcgacgg ccggggcaac 91380
   accgtgggca ttggcaagag taaaaatgcc aaaaaaaaat taaaactcaa aaagagaaca 91440
   attttgaccg atgatttgat ttatttaaaa aatattaaac attcacaaag attgccagcg 91500
   ataaatggta tgtcgctcaa agtctgtaaa cacagcttta tcactatgga gaagcagatg 91560
   cgagccggcg acgaagccgt ttccttcatt agatactgtc aatactgtgg caaaattgga 91620
   aacaacggta ataatcaata acgtctccgg ctgtatccgt agggattgcg actaccgctc 91680
   gatgagcgtc tgccgcgagg acgtccgggt ctacgacgcc tgcctccgct agagcggcgc 91740
   ctgctggtcg acctgcgtct gtattgtccg ccgcttgagc gccgcctgcc tccgcttgaa 91800
   cgacgcctat actgtccgcc gcttgaacga cgcctgcctc cgctggagcg gcgcctgcct 91860
   ccgctggagc gacgacgccc gcccgatgat cgtcgacgat ataccatttt gttgagttga 91920
   atacaatcgt gtttaaagta ttgtgttttt tattttacac cttatattat tattttagtt 91980
   cgatatagcc ttattagtat tttttacgtt tgttcgaggt gccgctgctg cccaaataga 92040
   tattcaacga atcggtctgc tcgggagttt tgacgagaat tccacatttc tcactatact 92100
   ctcgaatgtt gtccatcacc ttgaaaatgt tgttatagtc gttgacgttg aacttgcaat 92160
   tggacacggc atagttttcc atggtagtat agaatatgga gttggccgca ttatagaaca 92220
   ttcgatgtat agagaaatca tctgtaatct tgagcaactc attaataaaa tcttcgtcgt 92280
   cgcaatacgg aattcgattg ccttcgctcg cattgacata ttgctgcggc tcgagcttgc 92340
   tcagttcgtc tcctcgttcg gtcagcagtt cttcgagagt gcatctcttg tcgcgcgaca 92400
   gagccgaact ctgcagcact atgacgcgag cgaaacgagt cacaggatag ttcatggcct 92460
   tgttcatcgt gctctttaga attttaatat tgccaaagtc gacaatcgac gtaggtagat 92520
   ccagcaaatt tttgagtaaa tctgttaaat gatgcacctc atcgctggtg tagttgggca 92580
   tacattcgta ttcgtccacc atcgagctct ccagtagagc atagagaggt ctgtatttag 92640
   gcgatttggc cagatagatc atgcacgaga cgagatcgct aactttgaaa tcggtcgaac 92700
   tcgtattcgt caatgtatag tgtttgagta ttttctggca atttttgcgg aagagtccca 92760
   tgttggcgct agagctgtcc gccgcgatcg atctaccgcc gccgctgctg ctagagaata 92820
   aactcagagg atttctctgt gcagtcggcg ccatcgtcgt cgtcgacgtc gcaggaaaag 92880
   gacatgaact cgcggccggc ggtgtcgatc ctctctcgaa ttgactagtg agagcagcat 92940
   tattttcgac gtaagacgct tgcgacagtt gagccacggt ggtgagaaag tcttgaaact 93000
   cgtccatcga caggtccatg gtggtgtcgg gatcggcgag tagagtaaaa aattttggcc 93060
   aaatggccat attcatttgt gtgtcgattt tattttttat tcgttcaatt tcgagaaata 93120
   acattacaga actcattttg aaaattgggt acttcttctt attgatttac gattacaaat 93180
   ttattatcat atatattcga catgagctgg agcacgttta tagttttgac cgtattcaaa 93240
   cgaatagact gttcttcgtt cgagatggcc tctaatatag ttttagctat cgcagattgc 93300
   ttgcttatta cacgaatcgc gttacgtttt tgtgggttgg tgtcgttgag aataacgtcc 93360
   gccacggtat tcgtctggtt taaattttgc aaaaattcag ccgaattcat aatcatgtct 93420
   ccatcgtctt cttcgttatc gtccatcgtt cgattcgagt ggaggcgacg actcgtggtt 93480
   cttgccgtgc cgatcagact agccgacgac attgtcgttg agtagatgat acagttcttc 93540
   gtctagttta tatttagtca ccagttgtct tatatagcgt tcggctacaa tgtattcgct 93600
   catcgtttca ctgaataaat cctgtttgat actctgcagt ttgagcataa atttctcaaa 93660
   tttatcgtca tcataattat gaaaaatgaa tcgacagacg tttctcattt ctaattctgc 93720
   agcacttttc gttttattcg tagacgcttc caagtatagt ctcaaaaaga agccggtgaa 93780
   cactatcgaa gcgattttat ttatttttgg tgattttatt ttcgttttga gagccagttc 93840
   agttatgaat cgtttgaacg gagagaacag cttagattcg caagtattgt tgttgcgcaa 93900
   tttccacaaa agttgctcga gatcgccatc gccaagaccg ccggcgacga gacgacattc 93960
   tttgatgaga gaagcgcacg ctttaaagtt caaatagccc gtggtggctt tgtcacataa 94020
   tagattataa aagaattgag taaaagaatt tgtaatcaaa tcgtcagcat tgaacactcc 94080
   attggtgtcg tactcagttt tttgaataat atacaggaaa atgggcagac cgaacatggg 94140
   tcttaaaaat atgtcccatc cgtcctgtat ggcggtatcg aaaacggtca agctctccga 94200
   caaataatta atcttgcagc tgagacattc gattttgtta acggagcata cttcacattt 94260
   cgaattcagc tgcattatcg aaggcgctgg tgtggattta taatattttt gcaagtattg 94320
   cattatagtt ctgaattttg gcacttgact catgaattca tctttgagaa aaacggaaaa 94380
   aatttttttg acatcattat tgtcctgttt tctgtcgaaa tcctgtttca ccacgtcgac 94440
   acatttatta aattcattga agaacgtcaa gcctttgatg ttgacgttag tgctctgatc 94500
   gaaatacttt gaaaataaga atgccagtga atctatttcg gccgtgctca aaagaaccct 94560
   aaaattgaca ttctgaaaag agtcaaattt attgaatctc agagtgtatt cgactaggtt 94620
   cattgcgttg gtcatttttc aaatactaca cttatcagta taccgaaaac atggcctccg 94680
   atgacgatta tgatgaaggc gtcgacatca acaccgccga tgcaaccatc agacataaca 94740
   catttaaaat aaaattcgaa ttcgccaatc tactgtttaa atatctggca tacgttagac 94800
   cggaagaaag aatcaatctc gagtcgtttc gctctcgtct cgacgactta gacattggaa 94860
   ttgagtttga aatctctaat tttactctcg ataacgtgat aaatcaaatg aaaaatttat 94920
   taatttacgt gactcccggc ccatctgtaa atgttgaacc gtccgcgacg agtgtcacca 94980
   ccgcaaatct aattttcacc aacaacgatg acatcgacac gatcaaacta tatctgacaa 95040
   atcttttgca aaacgaaaat ctaaaacctt taacgtatcg atcgttaaac gaattggacc 95100
   agagattgaa gacggaatcc atcatttatg cggacattct ctccgaatta cggctggacg 95160
   ccatcgactg tgaacaaaat acgatactcc agagattttt gaatatttat aaaaattatg 95220
   gaatcgccaa ctgtatcgac acagatctcc ggtattatgt cgaaaagttg cgggatttcg 95280
   accgatccac gctgccgccg acggtggcgg atgcgctcaa aacgatcata tataatatgg 95340
   actcgccgta taaaattcaa gttacgatgg atcgacagga atatctcacg ggcgcgacca 95400
   acgtcgacgt cagggcgctt ttcaacagat ataacgagat tttacccata aaatttgttt 95460
   cgtcagagac gtcggcggta acgccgatgg acgcgacaac atcggcatcg ttcaaacgaa 95520
   aattgggatc ggatcgaaaa aattattcct cgagttccga atacgatgac gataccgacg 95580
   ctattggtgg cgtcggacgc atcattagcg atatcggctc gaccgccgcc gactcttcga 95640
   ctcttcgaaa aaaaagacga aaacgaaaag ccaaaaaaat atctagcgcc tcgacatcga 95700
   agccgatcgc tacaacaaaa ccttcgacga ccacaaccac cgagccgatc atctcgtctt 95760
   cggccaatca aatatttatc gaaggcgttc gacaatcggc aagaccgacg atggctcttc 95820
   cgccattgtt gttgtatatc attcgaatcg taccgaccga tgtgaacgct tcgctattga 95880
   catgtccctc gaacgcgttg agctcgaata tcaacacttt taatttctac ggtaaattgt 95940
   caagaattaa atcgttaaat ctcacgacag tcgacagaaa tgtccatttt ttcgagttgc 96000
   tcgaacctct agcgcattat ggtgtcactc taaacgacat caatcggtcg atttggttca 96060
   tttcaaaggc cggaaactat ttcgaggcga acgcttttaa tttcaataac atcagacgga 96120
   atctggctaa agaaacggac gattcggatc gcatagctct cttcatgata cgctataatt 96180
   ttctatggca ctatagacaa tttataagta aactagtgtc cagtgccata tcaccatata 96240
   aaaaccagaa aatcgttaac gtattgcagg tgtgcagcaa ttcggtcaat agggcgtttg 96300
   ataaagccaa cattagacta aacaactcaa aaatttactc cggtcccgtt gacgatatag 96360
   ttcgtctgat gaacggcatg tttgcggatc tgttgatatg atcgtttatg ttgccttgct 96420
   tgttgtggtg tttgtcgtct ttctgaccgt tctcgttata ttgagtgtta ataaaattca 96480
   attaagacaa atgttgtatt atcaatataa atatataccg gaaccattaa taagattcgt 96540
   caaagtcgat cgactcaaaa cctacgatga cgtgtccttt taacattaaa gtaatcatca 96600
   gcgatagatt ctttacgttt cctcacgatt atgtcgtgcc acaaactgac gtaggcggag 96660
   ctccagtccg caatctagtc gtgtacgtgc cctccgaaga ggacgtgcag tacgtagaca 96720
   agcgcctgtt taccaatttc gattcggtcc tagtctatag acacgaatgg acggatcgag 96780
   tcgagagtcg agctcccaaa aaaaatggca tcgccaccgt cgtctactgg aatccgatcc 96840
   tgcccatcgc cgaggtcggc gtgggcgaaa cacgcgtttt tagcgtttta ttgaccgata 96900
   aactgtttta ttgcaacact atgattgtcg atcccaacgt acccgtttgt cccattcaga 96960
   ttatgaataa atccctacga aactatatac cgatcacggg cgagtgtccg cttcagcatt 97020
   tgaacgtgtt actcgacgat aataaaaata actttttaat atgtttcaat agagagacat 97080
   cgaccgctct caggctattg aacatcaaac ggatcatgac cattttcggt tatagacgag 97140
   tctctgccaa gtattcgttc aatatgcaag attctcatct cgaacagatt tatatcgaac 97200
   tcaagtacga gctgattcgg agactgatga aaggcgacac cacaccgaaa tgcttacaat 97260
   tgaacgtcaa cagcttggag tatgtgaaga gggcgagaga gcttctgatg atacccgact 97320
   ctgctcagac tatagtcaat ttagtcaaaa tgttttcaaa acttattctg ccatatcttt 97380
   tagtgccgga cgtgatcatc aaattgaaca ctatggaccg acagcgtaag gttcgattgt 97440
   tctgtaaaaa cgacagtttt gctataactt catttggacc cgtgcccaac aatatggtcg 97500
   aagataaccc gatggcgttc gattattcgg acatcaatac gcccggacac ttgaatagcg 97560
   tcaaagaaca gatatttacg gcatctcgca tcgaaaacgt gaccatcaca gcggctagat 97620
   acaattactt tttttaaacc acaaataagt cgacatcatg agacgtatgg ctagaactct 97680
   cggcggcagc gacacaaact cgatactcaa ccaggatcaa ctcgaacaat tagtcaaccg 97740
   aaatcagtca tttttacgtg attttctttt ggttatttgt ggtgttgttg tctttattgt 97800
   atgtttatta tttatagtgt taattttcaa tttgaactct tcgatagaag ctagagaacg 97860
   acagaaacgg caacgaaaac agacatttat ggctaatctc gattacagat accgacagcc 97920
   gaacagcagt tttaataaat aatttttttt aacttgtatt ttgttttaat aattgaattt 97980
   aaattgatta ctgtgtgcat tttcgaaata aaagctgttg gaggttttgg tgacggtcga 98040
   gggcacaatt aaatcagaaa tggacatgga accaaaagac aacggttctt caatatcttc 98100
   gaataaaagc tgatgtgtta ggcgagacct gctcgtcgcg ttattgatag ttatttgacg 98160
   agtttcagtg tctttgagta tgacaatagg aaaatctact atacattgga tggtgctcga 98220
   gtttgtggtg atgatgaagg cgttcgtgtc gggcacacga acaaccgtcg tgttatcttc 98280
   atcgagagca tcctcgacca acaaactgaa acaggttatt cctctgccgg tgtttatcaa 98340
   ttcagacgag atgatctgtt cgaccgtttg catgttaatc ggttcggaca atcgagtatc 98400
   aaaattcgat aagcctccta ggttattgtt cacaatgttg tgatgtttgg tcacgactcc 98460
   attcgacact tgattgttag agttactcgt ccaacgagac tcttgagtta aatctttcgt 98520
   taaaaccacg caacgtatgg tagatgaagt tacaggacga atcgcattaa tacgttcgta 98580
   catatggaac atgccgtatc gatgatacag tgtatagctg taatattcga tggataaagc 98640
   atcgaaacga gcgtgcatag ccatgacacc ggcgttttct gtggcgcaca ttgccgtata 98700
   tgcaaaggcc ggatgaaagc tactcgtgct cgggcccgta gtcggtatca ccacttcgcc 98760
   ctgcagattg gtcaacagca aaataccaga ctcgagacct aacatttgcg tacgatacgg 98820
   aattactcga gctccgttcg cccagatttt ccgagtcatc gtccacaacg gagcgtgcct 98880
   attattagtg ggatcggcct cgtaataggc gatttcggga gattggccca ctacggaacc 98940
   gaaatatttg tcgtttcgaa tggttaaaat cttactgaag tctccactga ttacaccatt 99000
   acgatagtca ataaagaatc cgagcacatt agaaaaatga gctccgtttc tagatagaac 99060
   ggctggattg gcaagtccct gtctactgcc gatcagcgag attgaattat aaatattttg 99120
   catattgaca gtcgattcac cgaatagata gttgtagtag tcgaatgtga agaaactgtt 99180
   aatcagatat ccgtaggccc tgacgtcggt gtgatcgaag taggcaaaat cttgatggat 99240
   gccgttaccg gacactacaa tcgggaaatt gatcaatttg agcacgtatt gaacctgatc 99300
   ttcggctttg atttgagcga aagaatagcc tctgagaagc tggccatagc tgtacggtag 99360
   acacattcgc atggcgtttc cggccgtgcg ccaccagcct aatgcaaaag tcggcaacgg 99420
   taaatactca tcgaagacct tgatgaccaa ctcgtcaaga ttataaaagc cccttagcac 99480
   gatacaggta ttttgaaaga cctctggcat agtaattgag aaatgatacc actctgcttg 99540
   tattccccac ggggcttggt tcacaggcgc cgggaacggt agatgcgaaa cgatcgtgaa 99600
   catagccgca taaagatttg cagcaagttg cgaatctaaa tagagcgaat cgttaggatt 99660
   gcggaacctg actccgtagc ctatcagtgt atgtagtaca gtgccaaatt gaggtacgct 99720
   attccaagga tctaggccta cgaaaatgtt accatcgtca gaaaattggc gagtgggatt 99780
   cgctattttt tcggcctttt gcaaaaactt gtattgaaga gtttttatat aataattttc 99840
   aaacgcatcc aaatcggata tgttccttcc ttcggttcct aatgaagacg aaacgtttgc 99900
   cattgtacgg gcaggtaaag gattcgatga aaattgaaat acgaataata tgacgattac 99960
   aacaataatg attgcgaata gaaacgccca catgatgaga tgattagacg acacaggaca 100020
   aacacaaaac aaataaaatt attttcttat aatcccatat ttatttattt caatattctt 100080
   ctaaaatagg cagtttgtag aaaaatttcc agatgaatac gtccatatac ttgatgatag 100140
   gcattcgagt cctatatttt tccttcttta ccactatgct cgtttcgtaa tcatcacagt 100200
   cgatatttcc gatgctatta ttgaaatcgt tgctactgcg atgccagggc ttgacgtctc 100260
   cataatagtt gatcacgtag gcttcgctat ttttacgcaa atgatgatac gatcctgccg 100320
   tccacgcata caaaatggtc aaatgcgtta ttgtcatgtt cgaaatcgcc aacgcttgca 100380
   acagcacagg ctcgtcgaat ccgttgtgga aatgacattt ctgtaaaaat ctattcgtcg 100440
   ggaccaaaag tgatttaatc gtgtttaaaa gcttcaacga cggctcgaac agcaccgtgc 100500
   ccgctttaca taaaatttta ttgtgtttat attctagata ttgattgatt ttacaagcgt 100560
   caattttggt accgaatggc atttttccat agttgtcatc ggccatgtac agagctggag 100620
   tttccaaagc gaacaaatgg tcaatgtttt tgattacgat gtgatcggca tcgaggtaga 100680
   gtattttgtc atagtcggtc aacgaaagac actgccattt tgtgaaggca tgatcgatcc 100740
   aaggcttata catttgattc tgtcgattcg acagcatggg cggacatgaa tacgaaataa 100800
   agtcgaccaa aactactcta tcgtagattc gttcgagagt ttcgactgcg cgatcgctca 100860
   catccctcgt taccatacac accaaatcgt gaacggtatt ggtgtacaat atgcttttgg 100920
   ccaaagctaa agctcctttg acatattcat cgccaagcat aaccaaagtc acgtatgcat 100980
   acattttggt ctttatattt ctaccttaac acaatacatg cgaaccgaat caatctaaat 101040
   tagcgtgcgg tccattcatg atggaagaat catgaatggt cgatgcgatt cgcatcgggt 101100
   cgaacgatca tgaatgattc caccgacgta aacgccatcg gtatcggccg acgttaatat 101160
   aataattcac cgcaacattg tcgtaaattt ccatgacgtt aataatcatt tatctacagt 101220
   ttatataaaa atttttaaaa atttttgaat tttattgaaa gtttgttttg tgatttcgtc 101280
   tacgacgatt atctcgaagt cattgctaac aataaaattg tcaacgacag gtttattgtc 101340
   gacattgtcg gtgacgacat atgtaatggt aattgcgcat ttagtaaaat ctgatgcaat 101400
   aatttaggcg atgcgtaata aaaaatctga tgcaataatt taggtaatgg cgcaataaaa 101460
   aatctgatgc aataattcaa gtaataaaaa tctgatgtaa taatttagat gatgagtaga 101520
   accaatcgat tctgccgatc acgttaataa tgtttccaaa tcgacccaaa aacgattgaa 101580
   ataaacttga gaataacatc actgacataa tcgataacaa aactttcaaa aaagtgcgca 101640
   aaaaattttt gtgtggcgcg gcgcaacaat gtgtcgattt acaatcaaat aacggttttt 101700
   atcatcattc tcgacacaaa tagcctcaac ttggagtttt ataaactcta aaaatgttat 101760
   aatgtcgcgg acaccaaaat gacaatccgt aaattaattt ttattctttc ttcatgaaat 101820
   tcatataaac gaccagttta ttcatgtcga tgatatgtgg atataaagat tattataata 101880
   atactgataa actagggaat ataaagaaaa gtaaagctgt tgattcgtta gtataccagt 101940
   tgccctcaaa tcgaatagtt tatccggatc aatcatggtc aacgatttta atttcgatgt 102000
   tatcgtcaga aataattccg ataattctat aaattttgta aattttgtag tcgacgacaa 102060
   caacccaaaa gccgcgagca gcgatttcgg tgccgtcgat aaaaatttga gagtcgatga 102120
   ctcaatcttc ggtgtcgccg acaacaatat cgatgtaaac agcaacaatc ttggcgtcga 102180
   caatataaaa atttctaatc aagactttca agatataata gattttatga acaagaatgc 102240
   cgaacgaaac agagagttgc gcatgaaacc gtataaaagc gtcgaaaatg cgttgaaaag 102300
   tttcgaaact aacataaatg gtatgaaaga tgatgtgtcc aacatacaag aaaaaattaa 102360
   tagcatggaa actcaactga aatgtatcga agatgatgcc tataacaaac gattctctat 102420
   gagaaacaaa aagactcgct atctgaataa aagggttcgt cagctagaat gtttgacacg 102480
   gactcagctg ttggacctga tcgacaaatg cattcatcct gtagatgttc ttcgattgac 102540
   tgaaattgtt aaaaacaaat tgagataaat aaaaaattac atatattttt gcatttttat 102600
   ttagacacag catttacaat aaattgttta gattgattac aatcaagcaa gcaaaatttt 102660
   acaatacaat ctggattcga aacgaaacaa cattaaacac gtaaaccaat tgtttcgaat 102720
   acatcgcgac tttcaatcgc tttgtagatg tcgaatccgc gcatcttgag gacacgatcc 102780
   aaataggatt tgattgtaat aacaaaagat ttgtgagctc tctcaatgtc gtcagagtca 102840
   aacgtatcaa gtttagcgat tttgctgaaa tttttgacga tttcgtcgag gggaacttga 102900
   gtgagagcat gaatcacatc tctgagctgt ttcaccacac tcatccatgt ctctacattc 102960
   atcggactct gcacaacaaa agatttataa ttgtttaaaa gcagccgatt tctttcaaat 103020
   ccgatcactc tactcatctt catcttgaca tcaatctcga tgtacacggc attttcatat 103080
   tcatcatcat catcatccaa atccatatag taatctctgt cgtcatcgtc atcgtcgagt 103140
   ctaaggtttt gatattcatc cggtatccag catgtgacat aatggaaaca agtctctaaa 103200
   aactcattcg cgacattgtc gctatcaaaa caatatgttg gaataccagc aagctcttcg 103260
   tccacttcat tgcgcactcg aacaatatca aacatgccag tctcttcctg cataacaatc 103320
   tctgccatgg tccacatggc atagtcaatc gacgaattaa acttgtgaat ggtacctctc 103380
   gctgctagca gtattacttc tgtgatgata tttggcacgg aagtcgcttc gatacacatc 103440
   atcagtcgtc cgatcacaca atcgaaaaat ttttttctat tgatcaagtt tatggcatag 103500
   ttttctgtcc cgagcccatg tatatcttcg gaagatttta aatgttcgtc atttttatat 103560
   ttaacttgaa aggcgtcatg aatttttttg aactttaaaa atttgttggc aatttgagga 103620
   ccaaattggt tgagctcttc gtcgacgacg acgagagaac tatcgatgtg ttgttgttgt 103680
   tgttgttttg aagacatgtt ggataatgat agaatcacta cattttcttg acatttatac 103740
   tgggatttca gtgattatca aagggatttc aataattatc agtgtgattt caatgattaa 103800
   tagagataat aaaacgacag gataggatat caaaaataat atttatttgc acattattca 103860
   gattttatga tctttcgtag ttgcttcgga gaaatctcgt agcaatcgtt ttctttgcgg 103920
   cagttgacat ccaatatgtt atgcaggatt ttattaatag ttttttcggc aaacaaacta 103980
   ttcttggcgt acaccgaata atagtgttct ttagggtttt tgatgtcttt attgatggat 104040
   tctatatact ttaaatgctt cttggccaca tcaattttat acttgttcgc ggcttgtagt 104100
   tttctgttcg tgtagatgaa aacggtattc aggctcatca ggcgacgagt cgacgacata 104160
   gaagacatgg aagacaaaga cgatgctgaa gatatcgaag tcaatgaagt ggtcgatgcc 104220
   atggagacca tagaatcttc ggaatgtgaa cttgaaagac tcatgatgaa acagagcgaa 104280
   atagtgcact aatgtttaaa ttttggtata tatcattatt tatacatatc tttaatcttc 104340
   ataatctgat acaaacgtac gattatctga tataaagggc tttatcagat acggaggtaa 104400
   tataaatgta cgatgttgct gcaacgaatg cagtttatgt actgaacata aaacatcatg 104460
   tcatacctgt tgtctctcgg tggagtggcg tgcacgacca aaactacaat tttgagacag 104520
   atcgtccaag acaaaatacc taatgtcgtg gtgcattttg acgactacaa ggaacttcac 104580
   gatcgatacc agtttgatca tcgaatgggc agcgaactgt ttgcagcata tcgcaacgcc 104640
   aacgatacag tgttcaagaa cgattatatg aatgtgcaca tttttgatcg ccagccaatg 104700
   gaatcgctcg tttattcgag tcttcgccac aaaatcaaca aaaatatcag ctactacatg 104760
   tacatcaaca gtgcgaacat gggactgcac gacggctgga agagcgtcat attgcgcacg 104820
   cacccgagca acgatgagct gttcgtcgaa atgatgaaga agcgcgacaa taaaatcgac 104880
   atgtatacca gagggtatat acgcgagcaa aataacaggt tcaatttgtg gtccaaagct 104940
   gttgacgcta ctgaactgta tgtcaatttg cagaataacg atattaaacg attgactcaa 105000
   gaacagaata acattaaaaa ttatatagtc aatcagatct ataatgtcga atgtttggac 105060
   ggtctggtgg tttacaagtt tagattgcct ataatccagg ataggattgc tctattttct 105120
   tatgattatt tactgtggaa tcttttgtcg tttaatcctc agcgacgata cagattcgtg 105180
   gtcgaaaaaa tgattaggct tttggataac aaatttacac taattttcat gtgtcatttg 105240
   aagctgaacg aggtaaacga gcatataaat tatttgtgca agactctaaa tttacctcta 105300
   atcatgatga ttgccactga gaacaacaaa tattgtgtac ctcgatcggg ctgtgtagat 105360
   tatttgacca atctaaatcc taaaatcaat cggacccaat ctcgctacta cggaaatttc 105420
   aatagtagta tggaagaaga ttatgctacc aataatagtg tacaattttt cgatcgactt 105480
   gacttttttc taaataaata aatgaatgaa taaaactcca aatgtttcat tattgtgatc 105540
   attgtcgaaa attttaattc cattatcgtc aattacaatt ctataatcaa tattatcaat 105600
   tttatatgtc acattatcga ttccattgtc ttcaattttg tagtggtttt ctattataat 105660
   tgaaataaag gttttgcaat tttatacgat ggtgttattt ttcttttacg actattcaat 105720
   gccaccgtta aaagcggtga agatgctgta gattttttca gattttcgtc tgtcgtcgac 105780
   tttgtcgtcg tctgtgacga taacgatgat gttgcggtta aagtgatcgg acttttatta 105840
   ttatggctgt atagatcgat cgtttctata aaaatttttg tcgcaatctc tttaccaaaa 105900
   gtgacgagat gatgctcgtt ggtgcgggga ttgtccagat tttcgaggca ctcgttgtag 105960
   tgttggagca tggctcgcgg cgagctcttg agatcgggat cgattccgtc gagtcgcttt 106020
   agagccactt cgaggacggc tctgtagttt ttgaaatagt ctttgccttt gttgagacca 106080
   aatttgaagg cgatcaacaa gagacgcctg ttgaaatctt tatagtcgat cgaatcgtca 106140
   acatatttgg tttggctgaa taatttttta atagtgtcat aattttttgg agatggattc 106200
   ttaaagtatt catcgcgtgc gcccttgacg atggcaagaa tattgtcggg tagcatgcgc 106260
   tcagattcga tcagagcgct acatttgtcg gcgatgagtt gcctagcaaa gtcgtcaatg 106320
   tttatcgaat ccataaccgc gacgagtact taataggtag ctcacaaaca caatctatcg 106380
   ttatctagtc ctgaaattga gcgatccgtc gaggttaaaa ttgtagttgc tcaatttaaa 106440
   gtataatcga gccacgtcca tcgaaccgat tgcttcgata acgtcattca attcagacga 106500
   atgttgaaaa ctgtagacgc aatagcacat tttgtagttg agcagcgtgg acaccaacaa 106560
   ttcgatgaga aactgaaacg ttttattttc ggtgcagtcg ttgatgccgg cgccggcgtg 106620
   taacctgata atggcgtcgg gttcgtcttc gatgtcgttg ttgtgtcgtc tcgtgattct 106680
   attaaacagc atgcccgaat agagtttgag tatcatacga tcgagatgtc cgatatcgga 106740
   ttgacgagaa cccgcatgcg ttttgatgtc gcaagactcg aacatgagta tattgacgta 106800
   gacgatattg tacagactta tcgcgtctgc ccagaatttc gattcgtacg aagcgttttt 106860
   cacgagatta tactgaatga tattgctgac gatataagaa tcgttatcgc taaacaccgg 106920
   tttattattc ttttcaatga aatacgctac cgcatacagc tcgggatatt tcataaaaag 106980
   tatgtcttcg taggaagcgc tatcatcgag agcgaatagg ccgggtcgaa tctcggcata 107040
   gtcgaggatc aggtcgtgcg atttgaaaga cgttcgagta ttatatgcat gtgtcacatc 107100
   aaactgctca atgtttacca tattggggtt gattttctgg ttggatgagc gaattttttc 107160
   tatcgtcgga gccgttgccg cagacggatg gtgtacgata atcggcttga ctaacgattc 107220
   ggcatttaaa cgaataaaat tgaacatgtt tatgaaatag ttgaagaggc atttgagttt 107280
   ttcaatcttt agaggagtat cggccgcttt cagagccgta aaatcgagtc cacgaatact 107340
   atcgaaaaag aaggcatgaa tcaatagaac gaggcaatga tagcggctca tatcggtttc 107400
   gttgcgtatt tcgagagagg cgatgtcagt gttgaggacc acttcaaact cgacatgaaa 107460
   tttaaaatct tgcatctcga catcctgtat cctatttagc agttgaatgt acgcgtcgat 107520
   tcccaaatag cgattcattt ccgcaataaa atcaatttta ggagttgtgg tcggcaacgt 107580
   cgacgacatc gtcggcgctg ttatcgttgt gattgtcgac gatattgatg gatttttatt 107640
   cggaatcgat gcttcggtcg ttttaatcgc ggccgttgtc ggcgacgatg attgatttat 107700
   tatattattc agtagattta gtgtatcttt aatgattttc tcatcttcgc tcatgtaatc 107760
   tatgatttta ttacgaaaat cggcatcgtc gatcacgttt aattttaatt tcaaatcaca 107820
   aaaggtctgt tgatgctgtt taaatctatt ggaaaaattt ataaattgtt gagatatggc 107880
   attgaaatcg ttggcaaacg ccatagtttc ggtgaacgtg accacaatac tgttttgtaa 107940
   tgaaacatat tgatcgatga tacttttttt attcattgtg tatcgcagag tgtataatct 108000
   tatattagga aaaaaaccaa tacaatagta aattcacata tttttttatt tgataaaatt 108060
   tgatttcgaa taacaaacaa tacaaataga gaaaagttat tttatttgat tagaatcgaa 108120
   cgtatcaatt atttaaattt ccataaacat attgatcgag atcgtacatg gattcggcgg 108180
   cagaactaca aaaaggcatg gaatttggaa ggcgaggacc tttgttgtgg ttttcgataa 108240
   aagaagttag aaattcttct gcatatttga gcatgtcgtg ttgagaaaaa tgtcgttctt 108300
   cttcttcttc aatatcatca gcattcagtt gataaacgtc aattatttga ttcctcaaaa 108360
   tgtccaattc tttaggcatg atgtcgtgta gattttccgg taaaacaaca acagtatcgt 108420
   agaacattaa atgttccatt agatcatctt taaaagcaga cagctctcga ttaaagtttt 108480
   ttcttgaata gtagttgagc gtttcgggta gcatgtcaac tatacaatct atgagcatgt 108540
   cgatcaaatg gccggaatta ataaaatgct caccttttga attgtaaacg cacaatttat 108600
   tatctagagc gtagaccgga tccggtgtga atcttttggc cgtttgtccg aattttttaa 108660
   cgtaaacctc aaaatgttta aagaaatctt caattagcgt atccatgtaa ttattatcat 108720
   cataatctcc ttcgtccaaa ttcaaaatga gccatacgcc accgcattcg atcagacgat 108780
   ccagtagctc ttcaaatctg ttttggtctt ccatcgagac gggcaacgcg attttaaagc 108840
   aatcttcatc atcgtcatcg atttttgccg cacattcagc gcaataaact gtagaattga 108900
   gtttattctt ttcaacgact cgaaatgtcc aaacgttggc ttcgtattgt tgatagccat 108960
   atccacaaac ggtacaaacg aaataaaagt cattagtgat aaacttgtaa gcaaacatgt 109020
   tgattaaatt cgcgagtgcg tcgcgagaca cgtcttcggt cgagatgaac atgatttcga 109080
   tatcacagag tttttgtccg tagagtatag actgtaagaa tattacaagt accggtgttt 109140
   atatatattt acagattata gtagataaca aaacgactag ataatgtcat atatattttt 109200
   gtagatggaa actagattgt tgggtatgca caccggtatc gagcgcacca atgatgtttg 109260
   attatagtac attcgagtgt aattcgagtc acagacgcaa tcctcgacac taaaaggtct 109320
   gttgactaga tctaaagaga gatttccgtt gcttacgacg gcatctttat aaccacaaat 109380
   atagggtctc aagtcacccg catcgttgac aatgtcacga taagtactga gacaagtatt 109440
   gtatacgaga atgtcgtcag cgactatggc gtcgacgagt cctcttgatc tgtcgcaaat 109500
   cgattcgatc gaatcgggac ctctgtacgg tatcgaagct gtacaaaagc cctcgacaca 109560
   atcaaattga ttgatggcaa tggcgcaatt gttagtgcat tgtcgcttcg acacgcacgg 109620
   taatctagta tgcgaacaat ctacaatacc gttttgttcg tatacaaact gtagtgcaga 109680
   tttacctgta tttttattgg ctctctctac attttgtatt ttgttttcag tcatcatgtt 109740
   tatagttttc aataaataaa aacatactat tagtaaagca atcaaaatta ttattatacc 109800
   cagcgaaatt gatgaaaagg acgacatgat tgttgcctac aggcttattt gaaaataaaa 109860
   cgatttatat ttataatata gagtttatta caaatcaatt cgataaaatc acgtaaccat 109920
   tgttcaatcc atgattaaac aggctgagca cagtattgaa acattcgtcg catttagtcg 109980
   agtcgtacag cttaaaaagc caagcttcgc aaacgctgca tcgagtctgg ccatccagat 110040
   ccggcgtgtc gaatccgtgt gatatcgaaa ataattgatg gttagatttc aatatgacgt 110100
   tggatagatc ttcagcggta aactttgaag cgcaattgat acagtaggta gcgtctttaa 110160
   aaatgacgca tttcatcacg acacatttgt acgtgtcggc aatgttaata gtaatctcat 110220
   cgacatcaac tccttggttt acgacatcaa ttttcgagtt gtccgccatg acaaaggtgc 110280
   gatggtgtgt aaatacagcc gccaacgtac tgatgctcga gcgacaaacg tatcagcatt 110340
   tatagtcgtc tcgacctaat gataagacaa aatttaaatt tatcgcaact ttatcgacat 110400
   tatcataatc ataatcatcg gcatgtctgg cgaagaggat cagaatattt ttcttgttat 110460
   acgcgaagac ataaaaaatg tcagtattca agttcaagat gttgacgata aggctagcga 110520
   ttctaatgct agtctagtag aaatagaaaa ctctataaaa gcttttccat tcgattcgtt 110580
   ggctgctcaa ttgaatgtga tcaacaccgg tgtacaaacg acaaacacca gtataagtca 110640
   ggctaatgcg caattaaata cacttcaaaa gaacgtcgac gataagctaa ccagtctagg 110700
   tacttcggtg cagggagtga gcgcaaaagt agacgccgga gttgctagtc tcgcttctca 110760
   attcactgcc accaacacta aaatagacgg tgtcggcact aaagtagatt ctggtgttca 110820
   aaccacttca gctcaattga cagcctttca aaaggataca aattctaaaa ttgatactct 110880
   cggcactagt gtcgcatcca tatcgacaca attgacagct tttcagaata gtacaagagc 110940
   tagtttagac aatattacgg ctttgctgaa tgccataatc aaaattctta aacctttagg 111000
   actgttgtag taatgggcat ggacacgtat taaaagcatc aagattattg tgtttaaaaa 111060
   tttaatagaa atcaaaaata cattaaagta aaaaaactat taaattatag taaaatgaaa 111120
   ataaaatcaa gaaaaacatt aaagtcaaaa ttatataata aaaatcaata tagggttaaa 111180
   attattagaa aaaaacaata cattaaaaat taattgattt aagttgtgtc tgctgacata 111240
   aagaatcgaa atgtagttta attcttttta cagctctact ttcgggttta tacatgtgtt 111300
   ctaaccacca attggatatt ttgttgatgt gacgcttgcg ttcacaacgc aaaacctgcg 111360
   tgttaccagg ccacaacact atcgaaccgt caatagactt tatttcgaat gtagtagcat 111420
   tgttggtgaa tagtttctct ctcgggatgg tgtgcacatg agcagcaaat ttatgcatgt 111480
   tacaatattc ctgtataaag tcatcgggga atatgttttc aaagaaaagc ttcacaatta 111540
   aagggtcgtg gtcttcgttt tcttcatctt caatctgttg tttattagca cataatgtat 111600
   ccaaaaccgt atcgaatgtg ctgtgatagc ggttcaaaaa agtcttggtc aatacatact 111660
   cgtatgtaaa gttttcatta tcgaaaatat gaatccaatt tattttgtag ttgatgtcat 111720
   catataggcc cgtgaataac acccttttgt ttagcgtata aatgttcaat aaatcgataa 111780
   acttccataa ttctggatat ttattaatgt ttccgatgac atgttttctc gtttgattct 111840
   tggtacgatc gacaaaagta aagctattat tgtgttttga tgcgtacgat ttaatagcat 111900
   cggtcaacaa catggcaggc caaaaattat tacacggatc gatggtgacc ccttcattat 111960
   tattttgata ccatttatgt acgcactcgt ctacagatcc tttgtcgcaa taaaacgtga 112020
   cctcgtttct attgatccac ttatgtagat tggtcataaa tttctcgaca aaaaaatcgt 112080
   aagacacatc aaacttgatt tgttcgatcg tttccttgag accgggcgtg gtaaagctgt 112140
   ccacgtctcc attgtacacg agccactgtt cgacttcatt attatcggga tatttagaag 112200
   cgaccgcttg gtatattaga cgttcgtatt cgtcgacgat gggattttca tgaataccgg 112260
   tcaaaaatct gagaacctta tggccatagt atgtgatttt gtcgcccgaa ttcaattcct 112320
   caatataatt atatagttcc atagattcct cgtcgtcttc ttttatggta tcgaaaatca 112380
   gataattctc atcgagatat ttccagattt gcatgtcagt ttttgtagta tctttaaaca 112440
   gtaatttaaa ttcttcgaat gattcgagct ctgtcaagtt tttttccgaa agatattgct 112500
   gctttttctt ttcgtgagca acattttcca ttgctctatc taataaagga aaatcttcgg 112560
   gaggtgcatc taacaattct ccatgacatc tatcgaatct ttcatcatga ttccgatttc 112620
   tatgccattt tttttctttt tgatcgaatt cacgggctaa attaggttga actgccgtcg 112680
   gttcgaacaa ctctactgtt gtaaattctt tctccagatt catgtataat aaataataac 112740
   tggtgatata gatgccatac ttttttggct caatcgattc tttctcgaca tcgtggtaat 112800
   tgtaccgatt gatacgtaaa aacgcatttc cgttacaatc cgtgatgatg tcttccacat 112860
   atactttttc gatttcacac gaacatgacg gcacaaacac tttcagattc ttggcgttga 112920
   acgcttcgac cttgtaaatt ttattacaac acaacatgga cacgaccact cgatggatcc 112980
   aatgtccccc gacattatcg cgattagccc gttgaaattt ggcaatttcg gttatttgct 113040
   ccatagtcga gtcacttgac agggacaata aacattttta gctcgcaatc agctttatat 113100
   aaattgataa atatcacatt tcacagatta gatcagatta cggctaagta ctaaagtgct 113160
   atcaacttta gacgcaatta tgttacctga cgagattaga taataaagtg ggacgacaga 113220
   ttagatagta aagtgagcca gattagttat taattttgta tattaaataa agtaaaataa 113280
   tatagtataa tattaaactc attttactcg acgattggga ttaaattaaa cagaattaat 113340
   tttttctaag tgttttattt gcacattaca aaaactatgt caatcattta ttctatagcc 113400
   aaggtatttg aagacatggg attctcattt ggtggagcaa cgttgaaata tctaatattg 113460
   aacacgtttt taacaaaaaa gttttgacaa ttgttcatgg tgtgcaccac ctgttcaggc 113520
   gaataagttt cccctgcgtt agagtgtcga cgctgagggg tatcaggcac taaatccttt 113580
   gacattcctt cgactattcc ctcgactatc gcgttaatac gttccttagc ctcgatgctt 113640
   ggcgtgagac atttagacac gcaatcatct tcgctagtaa agtccaacgc cttaaactct 113700
   tcgatcatct tcgagtgtat gttcttgtta cgtttacaca tttccatgtc agctttatac 113760
   ttatcgcgaa gctcaatttc atttaaaacc tccatttcgg ttctcagttt gttggtgtat 113820
   cggaggccgt aaaatatgtg cggctgatct gtgcgcacct tgagccacac agtcacggga 113880
   ttaggacact tgagctgtaa aaacttttta gagtctttga gccaggcgta ccgacgcgaa 113940
   ttggttagtt tgggtcgctt ggcgtttact tcgggcaatg tggcttcttc ctggtatcgt 114000
   tgaatggctc tgtcctgttg ttttatctca ctgtattggt ttcgaccgat acgaatgcgt 114060
   tttctgccgt cgacggtgcg ctcgtagcca gtgatgtact cttccttgtc gggtcgctcg 114120
   gtaagcgcag gcactactct tccgctgatg ctagtcagtg tttgacgaag cttttcattc 114180
   tcatcgatgt tgtccttcgc caacaaagca ttgactgcaa attgagtcat ggacatgttg 114240
   gcttgagtgg ccatatcctt catgagcagc tgcattttgt attcgcgctc tttgtactcg 114300
   ctcatttgat tttcgtaatg gcgcttcatc tccgaaatgg aggcgtcgta cttggcaatc 114360
   gtggtgtttg cctgcgacag ctccaacttt aattgtagcg attccatttg agcttctgcc 114420
   aattgtctgt cataattcac gacattggct gtcgtcggaa tcgagtattt accagttttc 114480
   ctcaattccg gcaacacttc ttcgaatagc cagcgttgaa attcttccgc agcaggtagt 114540
   ttagacttca taatcaaagc gtaaacgcct gcctcactga tgaatactgt gtttggctgc 114600
   cagtttgcag gtaattgcga ttcggctgat gtcgtttcat ctttagtttg cttcgtttca 114660
   agggggcgat aatccatcgc ctcctctgtc gatgtagctt taagggggtc ctgatttagg 114720
   acctccttga ttctttgaga tgaccgcgaa gtcgtaggta tttgaccgtt agtcgatgcc 114780
   gttgcaagag agttctgttt tagaaccttt tttatctctt gccaggtccg acgccattga 114840
   ggttttatat ggtcatataa agcttttccg ggtcttttat accccaaaaa ctgggcaatg 114900
   gcatgaccgc cgtacataaa tacatctttc tcgacttcga caatccacac atcgcaagcg 114960
   acgccgccga tctcgcattt tttgttgacg agagacatga tttggatacg actcgaatat 115020
   gacgttagat gcaatccgac gataaactaa tcttatcggc acgaaacttg acgactgaat 115080
   gattaaatct ttgacgcgta atcgattttt atagtgccgt cgaggcgtga tctcgcgact 115140
   tttttgtaag tataataata atgaaagcca tttgtgttat cagcggtgac gtgaccggac 115200
   aagtattctt tttacaagag agtcccgagc atctactaaa gatcacgggc tacattttga 115260
   acctacccaa aggcttgcac ggatttcatg ttcacgaatt cggcgacacg agcaacggct 115320
   gcacgtctgc aggcgagcat tttaacccta cgaacatggc tcacggtgcg cccaactctg 115380
   ccgttcgtca cgtcggtgat cttggcaata ttgatgctaa aactcaccaa tctctgacta 115440
   gtatcgataa aatagattcg gtcatgtctt tgtacggacc ccactcgata ttgggcagaa 115500
   gtctagtggt tcatactcat cgtgacgatc tcggcctgac cgatcatcct ttgagtaaaa 115560
   ctacaggcaa ctcgggagga agattgggat gcggaatcat tggcgtatct cgataaaaat 115620
   aattacacac aaaatttctc aaacaaaaaa atttattacc tccaccaaat aataatgata 115680
   ataaagtgaa caaaataata gttacaggaa atttttacaa aaaatcgtcc acatcactat 115740
   gttcgctttc ataaaactga agttcgctag attcatcgga gtcagagtcc aagtccatag 115800
   aaaagaaact acaagtataa tgattacaat atctgcaacg tatagaaggc aaatcgttat 115860
   atctgaggca gcttaggcat tttaaatcgt aagcaaaggg tgacaaaaag aaacgactgt 115920
   acaactcaat caactgcacg gccatgtaat gaggaatata tctagaaaat ttagtaacga 115980
   attcttgtct aaacgctacc cgttcttcga tgttgtgcat atttatcttt ttcttgatag 116040
   aaaaaaaaat tttcttggca cacatcaacg acaagctgtc ttctttgcgc tgacgacaac 116100
   gaatataccg atcacaagct ttagaattca tttcaatgtc ccatcgccag tttaaaacgt 116160
   ttatttgctt gaacattcta aacacatttt cgtaaatcgg gcgtggtaaa tagggcttca 116220
   tcacatcttg aaacttttgt tctacttctt cgagcatcaa gtcatcggga atatactttt 116280
   catagtattc tgtaaacaca ctattattta acgtatttaa caggttctgt gcgcacaaga 116340
   gctgcagaga agatacgttt tggtgtccac gatccatggt ggttgacaga cttgttataa 116400
   tgctttctgg cgacgagacc cgatatttat attataattt atctaatttt atctaatcta 116460
   tacaataaaa caatcaaatt tatcaggaaa aaagtttatt taatcaataa tttttatact 116520
   tgtatccggg agcatatgaa aagctggtag atttttgaaa agccatatcc ttactcgaag 116580
   acgtggagta gcggcaattg caattccacg atttgttgtc caggcaccgt ctacattttt 116640
   cattgtagtt actattaaag ttgttgaaga aaatcactct ggtcgcccac gaagtcatcg 116700
   aaaatgaagt ttcttccggt ttcttcatag tgccggtgga gctgctggtg ctgggcttgt 116760
   tagagccggt ggcgtacgag cctttgccag tcttgcaaat gaggcacatg attgagagtt 116820
   atgattgcaa ctgcactgtt tgcaactgta tgcatttagc tattcagtat gcgcttttat 116880
   agtgtgaaaa aagattagat aacgagataa cacgagataa ctagtagatt agcatttcct 116940
   caacattaaa gttttattag ttgaagatag aatcgaatgc aaaaaatttt gcccaatatc 117000
   tcaagtgaat aaaataatta aattatcagg cgtcaactac aaaattaaat gtttagcgaa 117060
   atattgaaaa tcatcaggcg ttcgcgcaaa ttatcgattg attgtttgct acaataaaaa 117120
   tatcaggaac caaggtaaag agtaaaataa aacataattt aaataataaa agtttttatt 117180
   tagatgggta caaattttat tttagtcaca aacatatcta agaggaattt tatagtgccc 117240
   ggatagacat tttttgttat taagtttgaa tccacagaac tttttttgac ggcaaaaata 117300
   tatgtagttc tacagatggg gcatgaatta tgattttgag accaaatctt gagacatttt 117360
   gtacaaaaca cgtgctcaca gttgttggga tgtgaatagc ttcgacagcc tcgtcgacga 117420
   atcttttgca cacaaatagc acactcgtcg ttcattttca gcacaacatc tcggacgatc 117480
   gacggttttt cgattataat atcgctgact tgcaggacat cgaacaagat tctgttatgt 117540
   ttgtcaaaac taatgtttaa atatttctca tgttttttgt ctacgaattg agcagaatca 117600
   attattttcg ttcttcgttt aaccacatat ccgttggggt tcaaaaaaat tatatagtca 117660
   aaattaccaa tcttagccac aaaacccacg ttagacatgg tgacttttta atgtacaaat 117720
   gaatcgatcg ttaaagagat cgctttaaat agtgtatcga gatttattta tcggtaatca 117780
   tgtatatata cacacataaa gaatacaaca aaatatagga aaaaaaatat ttattattta 117840
   ttaaaaacat ttcattggcc ggtagagccg aaaccgccgc tgcccctgtc ggtgtcgtcg 117900
   agctcgtcga ccatgatcac gttgggtgta tgataggact cgatgatcat ctgcgcaatc 117960
   ttgtcgccct tcttgaaatt atattctttt ttgcttctat tgatgagaca aacaccaacg 118020
   gggccgcgat agtctgtgtc gatgatgccc gcttggatgc cgatgccgtg cttggttgcc 118080
   aggccagaac gctcagcgat gcgaccatat cgtttcggag gcaattgaat ggcgattccg 118140
   atatcgacaa ctgtccattt ttgaggtttc agacacacat cggtgggagt gtagagatcg 118200
   tagccggcgg agcccagagt cgcacgttga ggagcaatcg catctttatt tagcttctta 118260
   aagtacatat tctcgtgttg ttcctgatga tagtattcgt tgtcggaaca atcttcaatg 118320
   ctcaatctcg atcgcttatt ggcggtagac attattttcg cagagaggtg tggtgtgagt 118380
   gtcgtcgtaa gttactataa atatgactga ctgacgaccg gactctgttt atataccaag 118440
   tgaatcgata aatattaatg atatcgtaat atgtcgtcat tgacgagatc aactgattta 118500
   tagtcatcgt atgttcagga cgttgacgtt acaataaaat attttactcg tcgagtcgag 118560
   agtatctatc gttcaataag ttatttataa aattaaccac tattcgttac gatataatgt 118620
   cgttgctttc tagaaaatac caagatgtcg acattgcggc atatctcgat caatcgtatg 118680
   tgctgtgggt cagtgccgat gacgttttac agattttgag gttgcccgtg tccgttcttc 118740
   agaccatagc tccgcgacac aaaaaatgct ggacagattt tcgatgtccc actcagtgca 118800
   gattcgacgc gagcaaagtt tttgtagatc tctacggtct tgggaaccta tgcaatcgtg 118860
   tcaattcgag ctgttccgat tatttgatga ctctattcgt ggccgaagtc tatagagatt 118920
   gcttgcctcg tcgacgatca tcggggtgtc ggccgcgcag acgatcgtcc ggaggtcgca 118980
   gacgctcatc gggatgccga cgaaggtcct caggcggacg aaggcgttca tccggcggtc 119040
   gtaggcgttc gtctggaggt cgtaggcgtt cgtcttgttg cagacctcct caccaccacc 119100
   atcatcacca tcataacgag ttactcgagc gtatttcgcg acaaaacgag attctgctca 119160
   ccacgctcaa ccaactcagt ctaaacaata gtaaccaaca tctcgagctg accaacattc 119220
   taaacgcgat tcgcctgcaa aatgtggcca tagctgctca attaacgcaa gtcatagaaa 119280
   ctctcgacag tcagttgggc gaattgacga cggatttaag acgattattg aatgagctcg 119340
   acgctcgttt cgcagctctc acaacggcac tcaccaatgc catcgctcaa cttcaagact 119400
   cgatcagagc cgaactggtc ggtatcaatt cgatactcaa caatctcacg tccagcgtca 119460
   ccaatattaa cgctactctc aacaatctat tacaggccgt aaacggatta aacattgaag 119520
   gtctcacgac actactcaat accattttag ctttgatcga ggagattcta gggatattga 119580
   cacctgaaat tcctttaaat aaaagatcct aaatatatgg gaataaaaac ataaattatt 119640
   tttaacaaaa attttatttg agcatactaa aaatcttcga gaatttcata tgtttcgttg 119700
   gccgctccga accgcttgta ctcgcccaca cgtttttcaa agaaattagt cttgccctca 119760
   agagatatgt tattcataaa ctcgaaagga tttgcagtgt tgaaatgctt gtcgtagccc 119820
   aactcgaaca acagtcgatc tgccacaaac tcgatgtatt cgcacatgga ttgcgaattc 119880
   atgcccaaca tagcgaccgg taaagcattg gtaaagaaat ccttttcaat gtcgaccgct 119940
   tctacaaaca tactcaacac cttggtcttg gagagcttgt tctcttcggg aatatattta 120000
   ttgtagtata gacaagcaaa gtcggtgtgt agtccttcgt ctctggaaat gagttcgttg 120060
   ctgtaagtca agccgggtag agctcctttt ttcgagtctt tgatatagta gattgcagca 120120
   aaactaccag aaaaaaagac accttcgacg atagcaaaag cgaccatgcg ttcggcgaat 120180
   gtggcagatt gattttcgat ccatttttga gcccaagccg ccttctgctt gacactatct 120240
   atatgctcga tcgcattcaa aatcctgtcg cattcttggg gatctttaat gtattcgacg 120300
   acaagaatgt tatacatttc cgtgtgaaca cactcaatga atgtctgttg cgcatagaaa 120360
   aaactagctt ccaattcggg cacggcggat tgcatgtgct cgatgatgtt agtgttcacg 120420
   atgacgtcgg cggcagcgaa aaacgcgaga acgtgcttga gaaaaaatcg ttctttatcg 120480
   ttcagtttct tgttccaatc gtcaaagtcc ttggacatgt cgatctcttc gactttccag 120540
   tgacaatcga ccgctttctt gtacgattgc cagagatctt cattcacgat gggcaataaa 120600
   gtcttacgcg aaacggacga catcgtcagt gagtaagaag ttttcttcga tacgagacga 120660
   gtgcttgtga acaagtgtcg ctcgagcaaa tgtcaaagtg ataatacaaa tttaacgatc 120720
   gttctcgact cttttataca atcatgatcg aagatgataa ctatgctcta cccgtgctct 120780
   ccttcgataa cgaggcttac aaagtgtcgt ggcaagagat atcgttcgat ccgtacagac 120840
   aagagtattt gtattctata cgagtagaag agctcgaagt tcgttacgtc tattgggacg 120900
   ataacgcgcg tcgaatgaat ctgctgagca ccggtcagtt taagattcgc atcggaaacg 120960
   tgtactccga accggagacg gtcgagttta atgccgacga gcaattattg aaattgactg 121020
   tttattcgcc acagaaaaat tttaattcaa tcaccgtgca cttgaataaa agatatttcg 121080
   tcgacgatcg cagaccgtgg caagttccac gttggatcaa acgtgtcttt ggtccgttat 121140
   aagaaatatt taaattactt caagcgcaat gaacgaatac agtacatcat ttcgagcgta 121200
   ccgagaattg ttgagcatca aacgtgcttt gtggctgtac gtgtcgttgg tggtttgcgg 121260
   ctttgccatt ttcgtcggtt tgagtgttta cgccggcgga gtcatttacg cccaaattga 121320
   agacatttct caaactgttg gcgcgtcagc ccgcagccaa aaagaactac aaagcctcat 121380
   tatagaatct ttcgacaaca atctcgaggc cgacatacaa ttttctttaa taattcgctc 121440
   gagtgcctcg gttcaactga agatcaacac ttatccagca cgaagatcga taacgttttc 121500
   tgaagaacga gtgacctgtc ccggtagtgt ccaatatgaa aactacataa aaacactata 121560
   cgatcgagac actaaacccg atatacataa catttatctg ttctatggag gccatggact 121620
   gggcaaatcg tttgcatctc agcaattggg aaaagccttg agtaaatttg gaaatgtggt 121680
   ggtcgtgtca gtaccgatga ccagtttcgt aggagtggtc ggcttcaaca cgataggcaa 121740
   tgtcatacaa tctatagaat ccgtctgtca cggttgctat gtcatatgga tgtttgacga 121800
   attggattcg tatttgatag ataatcgcga ttctttatac cagagcaaaa gcatcacaga 121860
   atttgccgag tacacgggct ttgtcaagaa tcataatcga atcctagcct ttacaatgaa 121920
   caatggtgag attcttaagc atgactattg gacgcatcaa gatgaaatcg tcaacggcac 121980
   atacgaatac gaacgtgact ttagaaaggc tctcgtcaaa accggcatga gttcgactga 122040
   ttttcttatg gacggacagc tgagcagact gcgatcgttc gtcggcaaca aaatatttaa 122100
   atttaaacga ttcaatagag agacggcaaa gcgattcatc aacatgtatc tacctgacca 122160
   taagcagcta tcgtggaaca gtcaaatcga aaataaactg ttcggtaacg aaacaactca 122220
   gacatatgat gtgagaacgc tggtgatcgc tctcgatgat gtactaaatt taaagaaaat 122280
   aaataaaact taacacattt tataaaaaat tttatttcat tcctctagat tgtataattc 122340
   aaattgaaca aatcgtttaa aatacacata aatctgtacc aacatttggt aaaataatta 122400
   ttttcccaca aatgataggg gaagttgacc tgatgtgtaa tatcatcaat attattaact 122460
   agtctatcgt ttagatataa atgtataatg agattcgagc ccaatgtcgg attcaagaga 122520
   tacaacaagt ttttgagatt cttgatttca aagacagcat tcgagttttt tatataataa 122580
   aaattttgta gttgcatacg actattgaca ggataatatt tttttcctag cagtttccta 122640
   agttttatga ttctattctt tattttgtaa tcttgatagt tgaacacgtt aaacattaga 122700
   cattcgatct gatcggcaat gacattatcg atatattcga cggtaatatt cgtatcgtaa 122760
   agcggcttga tgagatacga taaaggtttg ttcatagccg aattcatggc actataattt 122820
   tttatagtca aatcgagaaa atagacatca aacacgaaat agttcaattc cttgatgtcc 122880
   gatcgtcggt ataaacgcat cgatgactgc gaagtggcga ccatgtgcac gttaaatgag 122940
   tatcgcacta aaaaatattc ttcgttcact ttcgaaacgg tggtcttgaa aggcatccgg 123000
   tgattcaacc gcaattcgac gtcggccaca ttgaattcga cggcttcgtt gacgtacgat 123060
   ttgaaaataa ttacagattt ttgacagtcg tctgtgctgt cgtcgttgtc atcgtcatcc 123120
   atattatacg tttgtttggt gtcgttggcc gcatccttga tgagtttttt gatgtcaaat 123180
   tctcgtaaaa ttttctcgat tacaccaaaa tatttttgtg aaatttttcg taaatttttt 123240
   tcgaggctag ttaacgccac aaatgacgac aaattgtcga tattgactcg atgattgaag 123300
   tcgacgtcgt cgacgtaaat ctcgatgtcg atgtcggcga gaaacgggtt tatttcctcc 123360
   aaatgtgcgg caatggcagc gccgcctttc acgacggtaa atgttttcag agaactgtat 123420
   attttttcta aaatttcttg atgaaatttc ttcgccggtc gattgtcgtc taaaaagccg 123480
   ccgtcgactg ctttattgtt gttttcgtcg actacgacgg tcgggctatc gatacgattc 123540
   aatttttgaa gtactctgtc gctgagaatg tcgttgacga gtttttttga aatgtaaggg 123600
   atggttctat ggtagtgaaa tcgttcgagt atagactcca taatgaaatc aaataacgat 123660
   cgtattaacg tgttaaaaca gcatcggcag cttactatgc cctcgttaat gcgtattatt 123720
   aattcgatcg atacgtctaa gcgcgacgga cgtcaactat tttataacat ttgttataac 123780
   atcataacca gaatcttgag tagatcgact gcaatcgaaa ccatcaaaac gatcgtttcg 123840
   atcattatag aaactgaaaa aactattttc aaccagagta aagttttaaa ctacatcaga 123900
   aactttttga tcgttaacag tgatggtcat aatcttcagt gtactattaa tttaaaatta 123960
   ctagattata ttttaaatag atataacatc gaagaagaaa aagaagaaca aaacgattat 124020
   cgtcatcaat aaaaatgtca tcgctgctca ccgccgaaca gaaagagatt tacgacaaat 124080
   attgctatgt caattatgtg gccatgctaa aacacgacga catgaagctg acgcaaaacg 124140
   aaatcctgag agtggaaaaa atgactcgtg gacaatctga aaatcccctc tggaggctgc 124200
   ttcgactcaa tcgacagacc gcttcgaaca gcaacagcac cagcataatg atttcgccca 124260
   gtgcagcaat gtgtcatggt atcaaagaag aaaaagtagt aaaggacaat aaaaatttaa 124320
   tgaatgaaat tcgtaggtta atcgaaataa ctttaaacac gattgtcaca aaagagattt 124380
   tcgactgtgg catgtttctc tccaagttag gtctgcatgc cgcctctcca gacgcatact 124440
   acgttacgat cgacaatgtc tatgtgccca tcgaaatcaa gtgtccatac acgtacaaag 124500
   acttaacata cgaagatgta cgaaaatctt tacgaaaaaa tccaaactcg aaaaaagaac 124560
   gatatagaat caaaaatact gcattgtcgg tcaacgtgga aggcgtcccg ctgtttattg 124620
   tggaaaaaac cgatccccat tatcgccaaa tgcaacgaca aatgtatgtc atgaatgctc 124680
   ccatctgtgt gtatctagta aaatttaaag attcgtttgt cgcttgtaca gtctatcgag 124740
   attctacttc atacgatgca gagtccagag acgaaagaaa aattttcaac atgtttatca 124800
   atcgaaacaa gagaaatcta atgctaaccg atcagtacaa gcgaaaagcg agcttcgaag 124860
   gcaaacatct accgcaatac agcgagctcg atattcgagc gttgaccaga agcggactct 124920
   atttcgatta tggacaactc gtctgtatat tttgccagat ttgcttcgat gtcgacacac 124980
   cggccaagga aattctggca aaacatacag attgcgcgtc gaatagctac aacggcatcg 125040
   actttcacgt cgaacatcga gcttatatca ataactcgac tcgctataag actttaccat 125100
   acgaacacag gcacttggcc agtgacggct tgtttcttga tcccatcgat tctcaatata 125160
   aaatgttttg ttgtggccta ataataaagg actttaatgt taaacatgtc gtcgaatgtg 125220
   aatatagtat gataattgat gataaataaa atacattaat aaattttaca caatcgtatt 125280
   tattactttt tcctttacca catgtacagt atagagcata acataaatag ttaacaacag 125340
   tagaatacaa caagaggaga gtttaaacat ttcttggcca tcggaccatt cgagaaatct 125400
   gtacactgga tttcctgtgt aatagttata aaacactaaa aagaatccat agaatagtgt 125460
   aaacaacagc ggctgatata catgtttcag gcggacgggc actgacgaaa tacaaacttc 125520
   aaagagcacc acgagcgaat tacaaaaatg tatcacattg ttaacgggcg aactaatgtt 125580
   attgtgatat ttatagacta cagtcactat aaaatatatt gtactagtca gcagattcga 125640
   cgaacaggcc acattaaaga aaaaccattg tacgtttacg taccaaggca atctataact 125700
   atattcggct tcggtgaatc gtccaataaa tgtggtcata gtgccgacga caaacatgac 125760
   caacaatgag atcatcgacc aatgtgaata gtacagaaaa aattccgctt tatctatagc 125820
   tacataatat cccgacgcga ttagcaacga catgatcaat acggatgccg ttctcgtggt 125880
   cagcataatc agttgcgaag aatcgcgcgg agaaaccaaa cagcaagagc taaagcgatc 125940
   tgtaatgtct ccgccccaga tcatgttctg ttgtcgctgc cgtttgccgc ccattaaatg 126000
   gatgctgtcc attctcaacc gaatgagact gtatgtatta aacgtaaaaa attccactta 126060
   attgtgactc gatgttcacg attcgtaccg ttttatacca cagttcgtag gtgtcgtcca 126120
   ttaaaacgat taaaaaaaat ctatataagt attatttaac ataaagtgtg ggtgtcgaca 126180
   tcgacaacga ccttggcacc gaaacctata taattcgaac ggctgccgac gaccgagacg 126240
   agtcgcattc gagcgaacgg acatacgaca tcatttagcg tgtcaaatta ccgcaaaatg 126300
   tggaaatcgt tattagtgtc gttgcttttg tcgatgacgg tggcagagat cgttcatagc 126360
   gcgccagcgc caatgaacac gactttgata tccatcgatg tggacgatca cgacgacggc 126420
   ggcggcgacg tcggcggcgg tttaccgtta aaggccggta ccgaaagatt ggtacagttt 126480
   tttatcaatc acaagtatct gcacatgcat gtcgatggcg tgataaacgg ctcactcgac 126540
   agatttaata atgagacagt gtggagaaga atagcggtac caataccaga ggaaactacg 126600
   gaaaaaacta ctggtatttt gatccaatcg gctagcaatt gccgttatct gtgtatgaac 126660
   gagtgtggct acatgtacag tgccgaaata cccactttcg agtgtatttt acaagaaaaa 126720
   atcgaagaaa gtcgatatca tttaatctat aaggatttta atagacgtca tgcgtacttg 126780
   tcgctgaatt tagaaggaaa attccgtcga agcgtattca aaagcaagga accgatcggc 126840
   gatgccataa tacgcacaaa cgtggccatc gaaacttgga ctggtgaaaa aatttcgaat 126900
   acgtgcggtg agttgattcg tagcaaactc aattatcgca ttcgaaagac atgtcgaagt 126960
   gcattaaaaa aaagactgat cagcaacgaa attccggcca taggagacaa agttggcatc 127020
   gacgacatca taagcgagat gattaacgac aacagcactc tagattctgt tgctgttgcg 127080
   atgccgccgc ctccgccgat acccactcta gaaattatga atagtactgt cgaggcggca 127140
   acaataaatt cttcgagtgt tgcctcgacg aatgcgacta atgcggtggc ggttgtggat 127200
   tatgtcgacg acggtgacga cgacgacgac gacagtgaag atgtcgaaat cactatcgaa 127260
   ggcgtcgacc cgaaaaattt ttatgtgccg gattcgtccg attcgacgct gagcattcat 127320
   ttgctaaact cgactacagc tcggcctccg actactcctg ttcgtcgtcg ccagcccgtg 127380
   atcgccatcg aagacataat cgatagtatg atcaagattc ctgtcgaaac cggcggaaca 127440
   tcggacgttt cggatgcacg aaatgttctt tttaaatatg aaaataattt ctatatcaat 127500
   aaatgtataa acatgtaaat ctgtatattg tagatgtatt tcaattggat tgtaataaag 127560
   actttgaaat caaattttca ttcgtattac atttattcgt agccaaacta cactgagtaa 127620
   ttactatagg tattgagtat ttgagctaaa ttattatact ggctttcggg caccataccg 127680
   attgcatcgc aaatgtgtat agttgtagca tttttagttt caagtacacc aaatactgcg 127740
   ctgaccatta atgccacagg aaattggcct ctaggatatc gagtggcagg actccaaatt 127800
   ttaatccatc ctccttctct gctaaaatag caccaagagt ttgtaaatgt ttctgcggtg 127860
   ccgacttgaa cagtgccgtg tctgcgtata caaagatcgg gttgatgtac gatgcatctg 127920
   cctctacccg gatagaagca tggttgagtc gttcgtctgg ctaactttaa aaacctaaca 127980
   tatatactgt cctttagaga tgagtcgaca aagatttcat gtggactgta agcgatcgaa 128040
   aattttattg tagaaagaat cgaatttgga atcggctgca gcagcctatg gtatctgaca 128100
   tgactaagat tatgcctggt aacgtgagca aaaatatcgt catccgacag taaattgtcg 128160
   cttcttcccc aaaaaaattt atattgaatt tcatgtaacg catcgatggg aatattaaac 128220
   ggctgtatgc acgagttgac aatagaaata ttgtctctcc tattcaaacg agactgtctc 128280
   agcatactcg cgccgccgga cgctattccg aagagattgt cttcgatggg acaattacaa 128340
   aaactcaatt cggttccgtc ttgatcattt ttaaagtact ggagtcgtcc agtcgtacgc 128400
   ctgcctgtga tcggatctat tgaacaagga tcgacaacac aaatggaagt gagtcgtagt 128460
   gtctgtcgat atatcggatc gagaccagga tgttcgatcg aaacaaatcc gtctcgacac 128520
   ggggcctgct caaaaaaagt cctataatcg gtatctcgca cacgcatcgg tcggcaaaat 128580
   ggagtctgtg tctgagtatc taactctgat atgaatcctt cgtcgcagac gcatctcatt 128640
   ggcgattcgt taatgtttag aatacgtccg ttcggttgac atcctacagg cacgttacaa 128700
   tcagaataga gattcagctg agtgactaga cccggcgacg tgcagttgca gagaagcgaa 128760
   tagccaattt cgtttctagt caaaatccaa acgcccgtgt tgggattgca ggcacgagcc 128820
   cgttctctat tgagcgccat gcaatacgat tcgcccggct gtatcgtata ctcgatgtcg 128880
   ttcatagtga ttttagtagg ttcgtcaaaa tattgacagt tggccaaacc ttcacgacaa 128940
   atatcacaat cttgatgtga agtgcatggc gtcaattgag agtgacaaga cactggatta 129000
   tcttcgagta taatctcgga aggaggttct attagaggaa cattcgagtt atcgaagcgt 129060
   tcgagaggat acaggacagg ctcgtccgca tacaccaata cagatatata atttgtcaag 129120
   agtaatacaa ttagaattag tatgacaata gctaatatta aatacatttt gttgacttac 129180
   gaatatataa aaataaaaaa attgtaacgt ttattaatat tttttattgt aatcacaagg 129240
   aaaatagacg gcaggaggac aaaggttcct aatgtttaaa tccgtttcaa gctctatacg 129300
   caaagcgtta tatgtagacc agctccttgt gtgatgatat gtgccatcgt taaactcaac 129360
   gtgtctgata aaatactttc ttctcacaat cttcatgtat aatattttct tgcaccattt 129420
   agcatgtaga attccgatca gtccgttggc tatattatgc tcgtgggcag gcctcatgaa 129480
   cgtgtataaa gcccatacgt ttctcgacca ctcgtgcgaa gccagaaaat cgtatctaag 129540
   cttggtaata gcacgtcttg ttatacgcat accctcttta ttacagatat ggatctcgat 129600
   tgccactatt ctgttgaatc gtttctggtg aaacgtaaac agctcgtggc cattttccaa 129660
   gatcgaaaca ctttccttgt tgtagttatg aatcgaatac aattggtcga tttcaaaaat 129720
   tgaaggcgaa cgcaaaggag acaatatagt tgctagagaa gccattgtat cggtcgaaag 129780
   actgtaatat gatcgccaaa cgaaagtcca tttatactaa tagattaaac ataatcagca 129840
   gataacactt tatctgttga taatggtatc aattttttgg tttatcatgt cgattttgtt 129900
   gccattggtc atgacgagat cgtaagtaaa ttttgtagtg ttttgaattc ggttcaaata 129960
   ggacatggtg tcgtttctaa atttggccat atcgtaaacg atagagtcga atctgtcttc 130020
   gatcgcttga taaataaaag tcaacagctt tttaattgcg gccagttcgt aagctatgct 130080
   gcctgtctgc catagataag ctagcaacag aaccagagcc aacgcagagt agttcatttt 130140
   cgtttccctt attttttttc taatattata ttctcatttt tgtttcctta tttttttcga 130200
   atgattatat ctttaattga tttaaaacta tcgaccatgg tgtcgttact gaatgcaagt 130260
   ttattagtca agtccgcttg catgctcttc actgaatcga gagccgatgt aatgttcgag 130320
   tatagagacg cattttcggt gcgaatgatt tcgcgaatgg tgtcgacggt gtcgtatccg 130380
   acaccattgg agccgagcac attattatta atattgactt tatcgaaatc cggttgatat 130440
   aattgtccgc tattggtgtt taaacattct tgtagtgtac agagctgtgt ttttatctcg 130500
   ttcagaggat caaccacctg actctgtgca cccattatta aatcacaaaa gagtcgcttc 130560
   aatacgaaat actgaggcgg gtggactttt ttactattca aaacgtacga cgagaggtat 130620
   tttcctaatg aaaacacgtg tatatagttt ttattgtttc gcgtcagttt ttgggaagca 130680
   ttaacgttcg tccagagcac tgccttattg aaatttctta ttgtcaccaa gggcgctaac 130740
   agcttagtgg ccgaacctag ctcgacatac ccatctcgat cgtcatcgct attttcaata 130800
   attaccactt ccaaaacctc atcgtcatag tgaaattgct gttgcaatga atctgacatc 130860
   accacgttat tcgtattgtt tggcccgttc attgctgtcg aatcgttgag attgctcgtt 130920
   tcgtacattg tgttaatggc ttaattgtag aattaaaaat agcactagat gattacgttt 130980
   atcgttttaa aaaagtatat taggtcttat cttttaagtt aaattcgtat ttcgaatata 131040
   attttttaaa tacattatga agtctcaaat gttggtgtac gacaacggcg gcctcgccaa 131100
   caccaatcat ttgtatacta atcatcgaca aaaatcttat agtcccgtcg acagaatgca 131160
   cacccgtcgt caatataatt atacaaacta ccttaatgat ttgtcctatt cgccggccgc 131220
   ttggcataaa ctcacgacca acgaaagaac caacgttatc tacgaaaaat gtaagttatc 131280
   tcaatatgat ggggtcgatt accgtagatt acagggacgc gatagctttc ggtgtgatga 131340
   tatattaaag attgtcgcta gcccgcagcc ttctagttat tccacaacta tcaatggaga 131400
   cacatcagca gcaatcgtct cgtcgggaac aacggcaaga ggtgttatta tggacaccat 131460
   tactgtctaa tctcgacgat atcgacaaga atgccgatta tagaatctta attgacgatt 131520
   ttgacataga tatcacgccg tatacagtct ttgaaaatga tggagccaca ataaaaatat 131580
   ctggattgcg tctgtattat ttactcaaaa ataaacgctt gtacgaagaa tcgatggaga 131640
   ctcagtgtag tagtaataaa acttttaaaa agagtctgaa aaaagtgtgc tttacaaagg 131700
   ctatccaagg aggaaaaaat agtgtagttg ctgtcatcaa gagtaaatta cgcttaccgc 131760
   cttgtataca gtctctacta tcagacatcg atgtgcgacc ccgaggaaat aggtttcgaa 131820
   agagattcat tttcaattgt tatattgcta atctcataac gtgtccggcg tgcgacaaac 131880
   agtgcatcgt agacgccatg gctgttctct atctgcacga cgacaagtgc gttcgtgaat 131940
   ttgaaaaact actcaatcgc aataaggacg tgtataagcc gcccagttgc ttaaatatgc 132000
   aaaacaaaga acgtttatgt cctaataaaa ctggcatggg atgtaaagga cgtaatcctc 132060
   tatgcaattt ctaatttata tgtatcgaat aaaaaatttt tcgtttaaaa atgattttta 132120
   tttataaatt ttcaaatata aacgatttga ctgtttctac agaagattca acttgtaaag 132180
   tgcgtcgagt tttctttttg taattaaagt tcttcatatc gagctcttcg ttgagacaca 132240
   gcatggccag ctgaggatta ggatgaatgc tgtcgtaaac tagttccata tcgctatatt 132300
   ttcgtttcat agctctgtgt cgtctggatt ggcccgacag aaaagctatc tgagtagaat 132360
   tgttttcgag agattttacg aaaaccgcca atcttggatg tttagaagaa tcgcgtggaa 132420
   agcgcacgct ttcataacga cacatttgtc tgtatcctcc gtcgtggtcg tcgtcgtcgt 132480
   cgctttgatt atcacgagat ttgaacgata attggcaatc atcggcgaca caattgtcgc 132540
   tagtcgcttc aatattgtga ccatcgactc gataattgtt tctgttgact cgatgattgt 132600
   gtctatccac tcgatatttc tcaagttgac tgtacaattc ttctgcagat atttttttct 132660
   caattttttc aaaattttca cacatttgag ctttgagcgt ttcgatcgct tgataatttt 132720
   tagtcagagt ttctattgta ttagtttcat tgttcctgac aaaagcatca aaaacgcgta 132780
   acatttctgt cattttctct tctatcggca acgacgccaa aggcaacggc ttcttttttg 132840
   tcgacaaatg ttcttctttc atggcatcaa aaacgttttc aatcatccat gtggtaaatt 132900
   caattttatt tttaaaatta acattatcga gaatctgcat cgcgccatgt ttattgatga 132960
   taaaaatcgt tttattgtcg tcattgtctg cgactgctcg agattctttt tgaaaactca 133020
   acagctctcc cacggttttt ttatattgat cattctcgat aaagtttatg gccgcatcga 133080
   tgtcgtcaag tccgatgcct tcggcgaaat cgaccgccaa aatccagatt tcgttctcgt 133140
   aaaatagata gcgcaacaaa aacgaaaatt gatcatcgaa acataatctt tttttattga 133200
   aaatatacga gaatgatttc atgtctcgct gtcgattcct tcgattgtgg tcactttcgt 133260
   cggcataatt gttgtcattg tcgacagaat cgcccgaaaa taataatctc tttataaaat 133320
   taaacatttt attggtaaca cacaagacac atacacacac actttacaat atttggaaag 133380
   aaaacttttg tcctttataa ttgtagctaa aaggtgctct tatctgtgat tgagtacaaa 133440
   acacgtgttt gtcgacagtc ggccagagct tgtgtaatgt agcgacatcg gcgctcttgc 133500
   cacttataga ctcgatggca gatttgtacg agtgaataaa gcttccaggc cagattcgat 133560
   ccagacgaat atctgcaggc ttgacgaata tttggtaata gcgctctcgt agattcttgg 133620
   aggctccgat gcgaaattta tctatacgta accacacatg aatgcctcga ttgcccgagt 133680
   gcatgattcg agctatgctg cttttgaaaa aatttctaaa tgtctccgtc gccactttaa 133740
   ttttcaagtc taaaaggtct gcatcgtcgg cttcgatgtc cacgtcgatg acccattctc 133800
   gaccgtcttc gagcgccttg acgtgaatat cgctgacgtt ttgatcttga ataaattcaa 133860
   atagatctcg agcgttcgta aagtattgtt taggatgata ccaattgacg ccatcggtga 133920
   acgaatactg tctgctgtcg ttgtatgcca cgttattcca cagcttttcg acttgctcga 133980
   atgtgtagag tctaggctga gcgtacatct cggtagaatt gctgtcgacg agtggacgat 134040
   aagtaacatg gcgttacatt tattgtttgc cgttctgata tcgaatgcgc cacgcctcaa 134100
   cagcaccgcg catgacgata tcgatagagt attcgatttg attttggacg agatcaccaa 134160
   aattgaggag aatgaagctg acgattttaa ttatactcgt ttgattttct ttattctttt 134220
   attgataata attattttga tcaagacacg actatatcgc tatttgctat gttgtaaaaa 134280
   ttgtcggaaa aaagcgatga cgattgtgac gattgaacca aaaacgaagg aatctccacc 134340
   gacatcgccg actacggcgg cgctgacaga accgccgatt gttaaacttg aattttcgaa 134400
   aaaaaatatt tccgacgaca taagcctcgt cgacgataaa ggccgcgaac ctgcgattaa 134460
   attgatcgat ttcaagtcta ttgtcgatac cgactagatt atgataaaaa ttgcgacatc 134520
   aattgtcttt tgaatgccgg attcggattt attacgctac gtctgctcgt cacataatta 134580
   tagatttcat cgagattata gcccgtacgt ttggtcaaat agtgtatgac tatggtggcg 134640
   gatcgagaga ttccggcgtg acaatgcacg taaacgcgtc ttttctccag gtcaatcttt 134700
   tgtaacaaaa aattaaaaga tttcaaaaaa tgagattgta tgtcggctcg ttcgttgtca 134760
   tcgatattaa tgtatagata gttgtcgatc gcgtcgatta tgtcggtttt gcccacaaaa 134820
   acgggaggat cgttccagac ggtcaacact gcagtaatat tttcttggcg taaaaatttt 134880
   tcaaatagat ttcgatcgta gatggcaccg cccaaataca gattgtcggt gattttggac 134940
   gcattaatca gtattccgtc tccgtcaccc gctccgactc taatgtaatt catattgcgt 135000
   acgctaaccc gtttccgtgc aaagattcga ataaacacac cgtgtcagtg ggtcagtgtg 135060
   ccatatttaa acaggggccg gacgacacac gacacacatc atcgggtaac ctaaatatcc 135120
   ttaaattaac cacacgaaaa aagtagagaa agtgcacaat gaaaactata atagtgctgt 135180
   cgttaatgat ggtactagtc attctaccgt cgcaaggcgc cagaattttg gcagtgtttc 135240
   caacaccggc gtacagccat cagaccgtct tcaaaaccta cattcgagct ctggccgaaa 135300
   gaggtcatga gattttcgtg atcaaaccat cgaccagagt aaattacgta gatcggtact 135360
   ctgatttgaa tcttttggga aatatcacag agatcgatgc cagtctatcg gaagaatact 135420
   ttaggcggct ggtcaaggcg tcgagcgttt ttcgcaagag aggactcatc tccgatagta 135480
   gcacagtgac ggcacacaac tatctcggct tggtgaggat gatcagcgat cagttcgagt 135540
   tgcccgaagt gaaaaacttt atagcaaacc gcaaggaatt gcaattcgat ctactcatta 135600
   ccgaagcatt tatggactat tctctagtgt tttctcatct ttttgatgat ttgccagtga 135660
   ttcaaatatc ttcaggctat ggagtggcgg aaaattttga aacgatgggt gccgtcagta 135720
   ggcatcctct atactatccg aatctttggc gagacaagtt caccgacttg aacgtctggg 135780
   aaacgatcaa cgaaatctat atggaattac gattgcaaaa cgagtttagt aaactggccg 135840
   acgaacagaa taaaatgctt aaagatcagt tcggcgggag cactcccact gtacaggaat 135900
   tgagaaatcg agtagaatta ctctttatca acacacatgc cgttttcgat aacaatcgac 135960
   cggtaccgcc gagcgtccag tatctgggcg gattgcatct gaaaggaaat acgcaccgtc 136020
   gtcatctcca aggattcgta aaggaatatc tggacaattc tactcagggc gtcgtgtatg 136080
   tcagtttcgg ttcgggtatc aacagtgctg acatggaaaa tgaattcctt caaatgttcc 136140
   tcgatgtgtt tgtcaaatta ccctacaata ttttatggaa gtacgacggc atgatcgagt 136200
   catccagact gccagataac gtgtttatac aggcgtggtt cgatcagttt gaagtattga 136260
   aacatccaaa cgtgaaggca ttcgtgactc aaggcggagt acaatcgacg gacgaggcca 136320
   tagaagcact cgtgccgctg atcggactac caatgatggg cgatcaagca tttaacacta 136380
   ataaatatgc tgaactaggc atcggccgga atttggacac gctcactgta aatacaaaac 136440
   aattggttga cgcaatcgtt gacgcatgcc aaaatccttc gtaccgcaaa catttatcac 136500
   aattgcgcca tattattcgt catcaagcaa tttcaccttt acagaaagcc atatattata 136560
   cggagcacgt gattaacaac cgcggacgta ccatgctcaa aacgaaagct gccaacgtta 136620
   gctatggcga ttacgtcatg tcatatataa ttgttccttt cgtgactgtt tctattatga 136680
   atcatttgcg tcaattgttg agaataaatt tattttaata tgataactgt atttttttat 136740
   gttccaaatg taaaatacta cattttgtac tctattacaa gggaataaaa cctgttattt 136800
   cgttgatcga gtaatctgtc gacactcgat ttgcgcaggc gttgtcggct gcgcggattt 136860
   ggtttataaa cgactcgtgt tcatagtcat gtacactaca actcgaacta gcgccaaatc 136920
   atgtcgacat tgaggaataa atgcttgata cgaagcatac aaatattaga gaaaaattat 136980
   cgacgggtgt ctgtagggga cttgaaaaaa atatcagagg ccatggcgat actacaagag 137040
   tccaacggta aactaacgag aaccctacaa aagatgtctt cgtattacgc agaacgctac 137100
   aagaacaaga tacagaacct tcaaaatacg atcgataaaa aagatcgtca cattaaagat 137160
   ttatacaagc aaatcgacga gaaaaaccaa aaaggagttt ttatagttcg actcgataaa 137220
   tctgtatgtt tcttttcttc tctcgagtcc ctgaataaaa caatcaagca atctgcgtcc 137280
   gtacagaata taaagatttt attatatcga agcgcatgcg acatcaaatc tgaacgatcg 137340
   gtctgcatcg caatagcaaa ggcgaaatac aagaattacg tgagcgttga gaatcgaaag 137400
   attatattcg tcgagagtgc cgatgtggac tcgttcgaat tagatattaa acagatgctc 137460
   acctaaaata tgtaaaatat gtagttaaaa ttttttttag ttaattttta gtggtgtttg 137520
   tgtgtaaaga ggctcgattt tcctagtact ttaaacaatc ttgcctatac aagttaacta 137580
   ttgcttcttc ggatacgctt gggtgttgaa ataatttact cgaatactag aataaaatta 137640
   tttattgata ttaatcgaat agacggacat tacattcgtt gctactatta gaataaaatt 137700
   tatttttgtt aaaatattta tcgtgtttat tcatcctatt agctttatta tttttgttat 137760
   tgttagaaat atatttgact aatgctatcg ttaaagctat gttaaccagt attaaagata 137820
   tacataaaac tcctaaaaat acaataaaat actttaaaat aatactgacg attgttttat 137880
   tattttcctc attctcgctt ttgttgtggt catcattttc tttgctaaag atgtcatttt 137940
   catcgactat aatcaaagtc gatggaggat taccagtgga aactattaaa ggaacaacag 138000
   tactggtacg aggtaaatga gtagtaggaa ttattgttaa aggtgttgtt gttgtacgag 138060
   aacaaggagt aataattttt cttgttacat ttaatatcac atttataggc ttatatacta 138120
   gagatgattt cttgactaca taactaaagc aatacttgtt tggattaaaa ttaaagtgtt 138180
   cgatatactt tttggtataa taatataaat ctaaaggagt attctgatcg atatttagca 138240
   gagacgtaaa caattttttt gaggtctcac cgcctctgct caaaagccgt tcgaaatacg 138300
   gttgattata catggcggcg agatgcacga gtgtgttgtt acgcaaatcg acaggcttca 138360
   ataaattttg taaagaaatc ttctcgtttc gcagtctggg tatgttgttg gcgaacgaac 138420
   gcaacgacag gttgctgttg atgctaggcg gggccacgaa cacttgacat gcatccactc 138480
   gattcatcag attctttaac gtctctgctt cttgtacttc gactgctgat gctgatggag 138540
   acgaacgaat cgaggcgaac ggcagtaatc tcgaccaaaa cgatgaggcg cgacccaatc 138600
   gaattaaagc gctcgtatcg tttcggcaag acgacacccc attataccag taatcgttat 138660
   cgatattgaa aaatttttcc aagacattgt ggtgatcgac agcattgctg ggcatcagat 138720
   atcgaactgc caactttact agtcctttct gtagatattc aaaatcgaat tgcgacaccg 138780
   gttcgtgatt agtctttatt tcgtattgcg ccacaatttt ttcgatcgta ttatctggtc 138840
   gcctgtttct attctgtttg atcagacgga atggtgtcaa aataaatgca caatcgtcaa 138900
   agtcgatctg tatatagttg tggcacacgg taaacgttcg attatcgatc atctgcaaat 138960
   actgttcctg taccgtattc ttggcgatga tcgactcgtc tttgctgttt attttgtcgc 139020
   attttgcgat tctgtttaat ttaaaaacgt taaaatcatt ttctaaagcg gaatcgacat 139080
   cgatcgtata gttttgcttt agaatcatat ccctgaatat gctgggcttc atttcgtaaa 139140
   gaaaagacac gagagcgctt cccatcgagt ataatatatt ggaatcgtaa tcggctttga 139200
   caatctcggc gatggtggcg tttcgatatg cctttaaacc ctcatgatcg cgccaaaaac 139260
   aatcacgatt accgaatctg tcggcggcgc cttcgatata ccacttgggc atgaatcttg 139320
   cttgtagcgt aaacaaaaga cagtgatgaa attcatgacc atatgcgttc ggtagatcgt 139380
   catgttcata gtctctgaag aagacatgag ctcgaatcac acgtttctcg tgcacgtatt 139440
   gagtgatgcc gccgttgttg acgccataat tccagtaagg tcccaatcga acgtattgtt 139500
   tacgatcgtt atgcacgtac atgtcaatgt cggtgaggtt gtggcgatac tgaagctgta 139560
   gtcgtttgta gaaatcgata aaatctttat agacggtatt catttcgagc gaaatcttca 139620
   tgagtacatt atttctagta atgttgtgat gtaaaaaaac tcgtacgggt cccacgtcaa 139680
   atctcgtgtt gaagggatag atgacatctt caattttttc atagagattt ttatcaaact 139740
   tggagtcatc gtaactgatc ttgttgccgg agacgaccaa atttttaaat ttcgacacat 139800
   aacacacgta aatgtcttgt ttgatctcgg ccttcggcat aatcatcagc ggatgctcga 139860
   gggctattcg agacagctga acggcagcgc gatcgatgag ttcggttatt ttgatgtttt 139920
   tctcgaatgt ctctctgatc gaagcgtaag cgattagaca cgttttaaac tgtctatcgt 139980
   tccacatgat gaatgaactg cacaattcgt gaaacgatac aaaagttctt cgcatgttgg 140040
   ccgagttcac gtagcgagta tacaaatcgg cggtcatttc gaatagattc gaggcgacaa 140100
   tctgcatttt gcgagaaatc cgacggtcga cgagagcaat ttttctatta aatttatcta 140160
   gcgaatccag tagagaggct tgagcatcgt actcttgcat ccgagccatt agctgcaacc 140220
   acaccatata tctctctatt tcggatggtg tgttgaatgc aaaatcttga tctttaaaat 140280
   tatctacaag ttttttggca ttacactcga aattggtgaa tttggctcgt atacacgata 140340
   tcaaaagttt attcattttc actttatcat aatggctgta atcgatacac aaaaatgtcg 140400
   gcagaaaaat tattgtcgtg attatcgcgt acttgaaaaa caccaccatt gttacgagcc 140460
   aatacacttt gactgccaat tgaaatagat tttttcgaca catttgtctg ttcttcttta 140520
   aaactgataa tcattctaca agccattcga tcgtccgaga atcgatcatc gtcgactcca 140580
   tccacgatcg tcccgcggcg acgcgcgatc gttcgatcgc ggatcgaccg taggcgatcg 140640
   tccgagccgt cgagcacgtt tttcgcgtcg acgaaaacat gatgcaatgt tgcgtcagcc 140700
   ggatgtttgt cgcgcgcgcg cgtgacccga atcgataacg atcgagaggt cgacgatcat 140760
   ccggggtcat cgattgacgc acgcgtcgca tgaataatga tgatgatgtt gatattgccc 140820
   aatattgcat catgttttgt aattataatg aaacgacaat cgtattgcat catctttttt 140880
   atttttacgt ttccaacgaa tccgcattaa aactcgatga cgcaaaatat aaactcgatt 140940
   ataatgtgat gtccaaattc atcaattatg agggcaaaaa ctttataatc ttgtaaaacg 141000
   actcggacgc tggagagaca tcgactacaa aaattttttg tggttttttt acaaaatttt 141060
   agtctatcat cacagacgtt ttcaatgaaa ttggagtcgt tgatccatat aaaaagaatt 141120
   ttagaaacga ttttgatggt atttactttg aatttatagt tattggcatg catgttgagg 141180
   atacatgaat ctataaaaat ttttaaaatc acgcgcaggc cgtgtattgt gtagaaatta 141240
   ataatctaaa aaagagactt ttactcgatg attgatgtga atcaccacat aaataaagtt 141300
   ttcgacgttg acagttttaa aattttataa aaatttttag cagaaaattt ttattgtttt 141360
   gtcagagccg aaatcatcaa taatagcgtc gagtcgattc acgattaaac gattatgaat 141420
   cgacacgaca tcaacatcgt cgcaaatatc tttgaaaata gtgtcagtaa cgatttcaac 141480
   aataatggtg tcatggcgat tcatgatcgt tttatcatga atggatatga cacatctaag 141540
   aaacgatatt caaaaatcta agactcgaat atctgtaaga tatatcaatc gcatgtctgt 141600
   agcctactat attggtcgtg tcaaaaatgg atcaaaacat taaaaaatta tttattaaag 141660
   aaaccaatct caaggaaaaa cacgaaagtc tggacaaggc ctacagaaaa aaacctaaag 141720
   aagatagaaa cgagagtgca atcgatgaga tgaccacctg cgccgcctat ttgtacggcg 141780
   tggaagagca gctgtttagc cttttgtcga acgtgagtca agagcgcaga atggacttta 141840
   ttaacgatct aaccgaactg gactttagta actacgaaat tgaactatta ttgaacacta 141900
   gatccgatga atatttattg aaaaaatttc atgttcacaa gaagagcgaa aatattcaaa 141960
   agattttcaa aagtaacagt attagattcg ttaaagtctt ggagcagttt gtggtgaaga 142020
   gacgggcata caagaaaaaa cctaaagata ctctactcga agaattggtc attttaaaat 142080
   ccattataat taaacattta tgtataatgg aaaaacttac acaaaaataa aaatctataa 142140
   aattataatc aatcgttttt tatttacaca caattatatt tacatacaca caattatatc 142200
   tatataaaaa tatagcaacg atacggacaa aatacattat ttcgatgaca ttagaaacga 142260
   taatggtggt gctgtcgccg ccgctgtcaa agattgccga tctgtcgtcg gtatagccat 142320
   atacgtgtat gctgcatggt cccatttggg tgtattattg gtgggtacag catagtatgg 142380
   cgactcgttt ttatccacat taatgtagtg tttcacctcg tccttgtcgc tatcgtcgtc 142440
   accatcgaag ctatcatagt cttcttcttt aaaatttata tatgtttctt gtgtttcata 142500
   aaattttttc ttgtcctccc tgcggttttc atcttttata acgccattca ccggtttaat 142560
   ggcgattttt tctacttcat caatcgtgtc ttcagtggat gtcgacttgt ttgttggaca 142620
   gcaactcgtc gcagttgact ttgaatctaa tgatttagtc ttgtacgcta cgtataaata 142680
   tgataaatgt atcaatagaa cgtacgtagg aaataaaatt atagaaaagc gtttggtttt 142740
   tagaaaagtc ggttctccga catagtattc gcttctgcat tttgcgcaat aaatttcgaa 142800
   tttgttgtta agcgtgaaac aattggcgct ttgaatcgca ttattgtcca ttttgacgat 142860
   tccgtcccag cattctttgt cgatatcata atctctctcg ttgacatcga ggccgggaac 142920
   atgaccgtat gtgacgaacc agtccattga aaatagagcg attgtcgtta aaatagtggt 142980
   cgcgccgatg agcggtacag caatttgatc atagaccaaa gtcgagagca cgacaatcaa 143040
   tccataaacc aacagcacac tactaaaatt caccacttga ctgccgtctt cgtaatcgat 143100
   gatgagtcca aatcgtccgt cgttgatccc gacgactccc aaaacgaagg cggtaaatcc 143160
   gcacgcaaaa agcatcactt tggtgtaata ccgtaaatac gcctgcatct tgtaagaaac 143220
   atgcatgtac tactaattat agtagttgtg attatttttc tttttatttt atataaacct 143280
   atctatgccg cccacgtaga gataaaaacc gctcaagatg aatacaacgc caccgtcgac 143340
   gaccgaatcg agtacatgga aggtgtgtta aaaagacgca aatatgtacc gctcgaagct 143400
   ctgcctacga tacgattcaa taccgaacta ggcactataa acgatggcga acgcaagtgt 143460
   atgtcggtgc cggtgtacgt cagttcagat aatattccta attttgattg cacactcatc 143520
   tgcgatgatc ccaccgcagt ttattgtttt gttggcgaat ttgataaatt tcttgtaaac 143580
   ggccagatgc tcacgcgcgg cggctattgt actacaaatt ctgtacctag aaactgcaat 143640
   cgcgagacta gcatcatttt acatagccta aatcaatgga cgtgcatcgc agaggaccca 143700
   cgctactttg ccggccctat gaacatggta caaatcgctg ggcgccagca cgccactaga 143760
   atcgcacccg gcgaaatcac aaaaaatatt gtttacgaca atctactcaa aatggaggtc 143820
   gacgtgtctc gaaacacttt tcgtcgggat tgggacgaat cgatgccgga cggcagccgt 143880
   agattccagg tcatttgtaa tgcattagac gatcgattca atcaaatgtt tgtaaatccc 143940
   atgaatccaa tcgagtgttt acctaataca tgtacgagtg ctagatatgt gcatcgagac 144000
   gttaaaccca atttcgagac gggcgaatgc gaatgtggtg atgttaacgt taccagacta 144060
   actcatatcg tgcccggtga tcgcacttcc atgtgcgcaa gtgttgtcga tacgtttcaa 144120
   acgaccaaca attcacacgt atttagagtg caatgtgtga atatgaacac gttaatcagc 144180
   gactatgatc caaacatgct tctatgtcct ggctcgcaat tcgattccaa taacgatagc 144240
   gcgtttagct atgaattgcc gggcacgtat gttttttctt cgaacggcat acacgaacct 144300
   actcatcggt tctatttaga cacgcgcagt agaatcgact ataatatcgt aaatcctgtc 144360
   ccgacttgaa acctataaaa gtcagtagca tgtatcttta gttttattga gttgctgcga 144420
   aacgatagca atggaccgac aaactacaca aaaactactc gaaaatgaga ataataaaca 144480
   caaaaatgtt ggcatacaac tcaacaaaga agaatttgga agcgacatta aaaaggggaa 144540
   tccgaatttt tatcatatac taaataatac cgaagacctc aaaaaacatg ccaaatgtcg 144600
   cagaaaacta aactttgatt tcatataaag atggaatgga aataaaatca tcatgttatt 144660
   cgggatatct atcaataaaa atataaattc aataaaacat ttttatttat aaaacctaat 144720
   cattatagtt taatcgtact cgatattctc gagtttattc tgtagagcta ataaatgctg 144780
   tggatcgtga tgttttttct tgacaggctt cgggttcatg aattgcattt cacgatcgac 144840
   acgaataacg gtaggatctt cgcgacgaca gcaacacgag aacatagaag acacggtgcc 144900
   cgagcagcaa gtagaataaa ttttgaacac catcagagct agcagactga gtacaatcca 144960
   cacgaaaacc atttttacga catggaaatc ggctccaatc gacgagaaaa aattggacag 145020
   ccacgagttt tcagtatcgt ctagaatttc atcgccggca aaaaccgtat tattgttcat 145080
   tagccgacgt aaatctacca atctatcggt caatttgtgt aaattattgt gctctaaatc 145140
   attattcatt ttcatagtct gcagctgtag agtgtccaga tccttgagca cagcactcaa 145200
   attaaaggct gtatcgatgg gcatttgtaa atttttattt agactgttgt acgtttcgag 145260
   tgtagttaaa aacgtccgtt tcgtgctcaa ttgacaagtg tgcttgccgg tagcgttaat 145320
   gatgccgatt cccgccgaca aaactagagt gtttgccgac accgaattat ctcctttctg 145380
   tagacagtca aagttgatgt cgacctcatt ttcgaggaca tagagccatc gattaaaatc 145440
   agtaatttta tagaagattt cactctcaaa tttaccgatt ctaacatcac aatccttttc 145500
   aaaattgatg tctttgtcgt ttttcaaaaa gatacgaata tcacacagct gcgcttgatt 145560
   agaatcgtgt acgacctgag gcacgtagca gagaatcatg tcgtgtccga gtcgacaaga 145620
   ctcgcgagtg tcgtcgaaac gcacaaaatt cctgcgatcc ttcgatacac ctagatattt 145680
   gctatccggt agaattatgg cacatttaga tttaacgcaa aacggtatgg gcacaatttg 145740
   gtagacatca tatttttgct gattgacgag cggcacttca atgatgaata gtagttttct 145800
   ttctttagtg ataaaaacgt gaatattaat gattctgtcg atgagctcga acatattgtc 145860
   gtagtgcaat ggcacaggcc acgataaacc tttaggtaga taattgctca cattcttcat 145920
   ttcttccagc aatttttcgg cggtaatgac tgcggaactg agtcgtttgt ctttggcgtc 145980
   ctcgacggca tctttaatct ggtcgtaaag actgtctagc ctatcgatct cactacagat 146040
   caaatgaatt ttagtgccca catactcaca tactttttct tctcttcgtt tggcatccaa 146100
   ttcatcttcc accatacgtt caaactgaat caaatgatcg gatatctgtt ttatctgacg 146160
   attgagtgcg ttcgtgccgt tggcgagttt gtgaagttcg tgggcatcgt cactgtccat 146220
   taggccgaac aaatatttat ataggctacc gagagggtcg aatagacctc tcttgcttct 146280
   gctcagcgta tatttcgatg gagtttcgta gttgccagac ttgcggattt ttccatcgat 146340
   atcgttatga agattcacca agttgacgat tttgttcatg atagttttat cgagttcggt 146400
   ctgaataata tttgaactgc tacaattttt aaattgtcta cttttgtaga tgaatttttg 146460
   tagattttta acatgatcga acatgtcttt taatcgcaaa aacacagcgg catgatccat 146520
   ttctacaata aaatgccaag tgttttcgac aaactgcatc tccttgatag gttgaaaata 146580
   caagcccgaa gtgtgcggta aatgctgtac gttaataatt tcgttagccg atattagttc 146640
   tttattggcg tcggttgttg cgcacaacaa catcatcgac actgccaacg agattagcgg 146700
   ctgtgacaat gtcatgttgt ttcgcgtttc tatcgttcac actatactga atatgtcagc 146760
   gaagactttg aggctttata ctcggcaacg cagaatataa ttaatagctt actactatat 146820
   attttttaac cgtcgtctcg ttgccgttgc tatactgtcg gtcgtaaatc aatcgtcaca 146880
   gacaagcgac atgattttcg tggtcattat tttcgtgtgt catacccaaa aatagagtca 146940
   ataaaataca cgtgttatta ttttaaatgc gtcatttatt tatcaagttg acctctacca 147000
   cacaataaca ggataataaa aaaataatga atacaataac aagataataa aataatacaa 147060
   caaaatgtga tgtcatgtgt ctgtttttta accttggcag ctctcgcaac cgatgtagca 147120
   tgcgtcaaca acatccggtt cattgtttgc acgattgatc gccgacgatg aaggcacggt 147180
   gaattgaatc gtgtttgctg cgggtttggt tctcaaatag tacatgcccg tttttaaacc 147240
   cttttcccaa gaatagtcgt gaatggatga taaaacttta taggttggat tggccacaaa 147300
   aagattgagc gattggctct gatcgatgta aggtccccgc tctgcggcca tgtccaacac 147360
   tactttttgt ttaatttccc aagcggtctt gaagagcgcc ttgatattgt gtggaatttc 147420
   atcaatattt tgtacagatc cgttgttttt aataattttt atcatgatat ctgtcgaata 147480
   tacgttggtg tctacgagtt tatcgacgag atgctgattg accacttgat actcgcccga 147540
   cagaactctt cgtacgtaca aattactagt aaacggctcg aacgactcgt tgttacctag 147600
   aatctgtgca gtcgttgccg tcggcatgta agcaatcaat aatgaattgc gtaagccgtg 147660
   catagccatt tctcgcttaa gctcgtccca gttccaaaga tcattcgatt ggttaatatt 147720
   ccacaaatca aactgtaaaa gtccacaact tgcagggctt ccatcgtaag aatcgtatgt 147780
   tccgtgcagt ttagctagtt cgatactttg acttagtgca ccatagtaaa tagtttctgc 147840
   aatcttttta tttaaatctc tagcctcttg gctgtcgtag ggcatatcca aaatggcgaa 147900
   cgtatccgca agtccttgaa taccgatacc gatgggtcgg tgtctgaaat tcgatgcttg 147960
   agcgctagcc aacggataaa aattcaagtc gataatttta ttgagattat tggtaaccac 148020
   tctagataca ttgtagagcg acttaaagtc gaagctttta aactgtttat tggcaaaatt 148080
   atttacggca atcgaggcca agttacacac ggccgtttct cgattgtcgc tatattgaac 148140
   gatttcggca cacagattgc tgtttcgaat cactccctcg tttttttgat tatttttatt 148200
   gttaacgtga tctttaaaca tcatgtatgg agctccggtc tcgacttgag tctcaataat 148260
   ttttctatga agatctaaag ccgaaatttg ttttttaaac ttgccttcgg cctcgtaatt 148320
   ttcgtaaagg tcctcgaatt cttggccata gcaatcgttt aaaccgggac actcgtcggg 148380
   gcacatcaat gaccatttcc ctcctgtctt taccctcttc ataaacagat caggtgtcca 148440
   catggcaaaa agaagatcgc gagccttcat atcctcggca cccatattgc gctttagatc 148500
   caaaaaagta aaaacgtctg cgtgccaagg ctcgatgtat acggtcatgg cgcctttcct 148560
   tttacctcct tgatcgacat ggcgtagcat gctattaaaa acacgcaatg caggtcccaa 148620
   tcccgccgca gttccgttgg tggattcgat tttactattg gtggcgcgaa tttgagtgac 148680
   attcaggccg atgccgcctc cgtgtttaga tatcatggca caatcgttga gtgttttata 148740
   gatgccgtta atactgtcct cttgaatgtt aagcaggtag cacgatgaca tttgaggcat 148800
   tttcatacca gacgaaaata gagttggact agcgtgagtg aacagctttg cccgcatcaa 148860
   tctataggat tcgatagcgc tttcgatgtc atcaccatga atctgtagag ctacacgcat 148920
   cagcatgtgt tgaggcctct cgacgacaat attatcgact ctcatcaaat attttccttc 148980
   gattaaagtc atcagaccaa agtaagtgta atgtttatcg aaatcgtaag aaatgctagc 149040
   attgattgag tctaaatgct ttataactat cgagtagaaa gaatcacaaa gaatcccatt 149100
   tttccagaga atctctacta ctttagagta atcgtcctcg gttttggcat gtagcttgtt 149160
   gactgcgatc acaacggcca gatcagaata aacatagtgc ttgtaagtct gagaggcaca 149220
   cacattagca ctgtattctt caaactcatc ctcgtgtata ttgtcataga ttccgcaaaa 149280
   aattttatcg gcaatatagt taaagtcgat gtattcttta ttttttttca agtcaccact 149340
   tcgagtataa agataagttt tgatgtagtc catatttact ttgatcgact ctcggccgtt 149400
   cctcaaaatc atttgatatg aagaaaacat tttaaaatca ctatacaaaa gtcacgactc 149460
   ggtgtcaagg tgtatgtagt aataataaaa cacatttgat tgtcgagtat atttatatca 149520
   tttaatcgct gaatgatgct cgtcggattg agaaagagat aaaaaattct catattagat 149580
   gcgtttgccg taatcatgtt tcgaccaaaa atttttctat tgatcatgca caacaaaata 149640
   agtatttttc tcctttcgta aaagtttgtg aaaaatcaaa tata 149684
<210> 18
   <211> 149080
   <212> DNA
   <213> Chrysodeixis chalcites nucleopolyhedrovirus
<220>
   <221> source
   <222> 1..149080
   <223> /isolate="ChchSNPV-TF1" /isolation_source="Canary Islands, Spain" /mol_type="DNA" /note="Genome of ChchSNPV-TFl-B" /organism="Chrysodeixis chalcites nucleopolyhedrovirus" /strain="ChchSNPV-TF1-B"
<400> 18
   atgtatacac gttacagcta taacccttct ctgggccgca cctacgttta cgacaacaaa 60
   tactacaaaa atttgggtgc cgtaatcaag aatgccaagc gtaagaagca ctacgccgaa 120
   catgaattag aagaaaaaac ccttgatccc ctagacaact acttggtagc tgaagatcct 180
   ttcctgggac ccggtaagaa ccaaaaactc actttgttta aagagatccg taatgtaaag 240
   cccgatacca tgaagcttgt cgttaactgg agcggcaaag agtttctcag ggaaacttgg 300
   acccgcttca tggaggacag cttccccatc gttaacgacc aagaaatcat ggacgttttc 360
   ctagtagtta acatgcgccc gacaagaccc aatcgttgct tcaaattctt agcccaacac 420
   gctttacgtt gcgaccccga ttatgttcct cacgaggtga ttagaatcgt agagccgtct 480
   tgggtaggca gcaacaacga atacagaatt agtctggcca agaaaggcgg tggctgccca 540
   atcatgaacc ttcactctga gtacaccaac tcgtttgaag agtttattgc tcgcgtgatc 600
   tgggagaact tctacaagcc catagtttac gtaggaaccg attccgccga ggaagaggag 660
   attcttcttg aagtgtcttt agtctttaaa attaaggaat tcgctcccga cgcgcctctg 720
   tattccggcc cggcatacta agccgtagat tcgttgtcga ctttttcgtt aatttctgtt 780
   aaaagtcttt ctgcttgttt aatgtcttgt ctgctaacgt gttggctatt tattaatctc 840
   gacacattag ctgacatgcg taagatttga tcgttttcgt ttccgctatt ctgaactcga 900
   ttcatagtgt ttaaatactt atttttgacg gtttccacct cattgtcgct gatccaatca 960
   ttattgtctt cggaaaacga atcctcatta ccggtgctct cggctagcgc ttttactctt 1020
   tttgccaggg ccaaatgcaa tactgaaggg gcagtttcaa caatcggttc tttgtccgcg 1080
   ggaatttttt ttacattttt taaaattgta ccagatcgaa tcgcttccat taactgcgaa 1140
   tgtgcgtcta ttggcggtga cgcaactgtt gtgggcttta acgacggttt cgagggcaaa 1200
   ttttcttctc cgacttcttt aaacatttga aacatgggcg gcggtggcgg tggcggtggc 1260
   ggtggaatcg aagtcagtgg catcggcggt ggcggcggtg gcggtggaat cgaagacatc 1320
   ggcggcaaag aaggtggcgg tggtatcgta atatgggttt gcgtcgacga caatggtgat 1380
   tttaaatttt tttcctgagt agaatatatt tttgttgttt cttgtatctt ttgtgtctcg 1440
   gtgattggca taggaatcga tggtgtcgac accgattctg gggtagatga cgccaacgac 1500
   ccagcatttt gattatccag agtcaagatt tgattgaaga tcgaatcgat tttggccgtc 1560
   ggtttttcga cttcttcttg atactgttta tagagtttaa aaaaagtttt cattaaattc 1620
   tctatcttta aaagttcgtt ttcgtggtta attttatcga taatgtcgaa taaaatagtt 1680
   ttatagcggc tgttggcgtc gacgcgggtc accatatttt cgagttgcag aattttattt 1740
   ttcatgcgca cgttatctgg tagaacggca ggtccgatag tgtcccggac gttggccgca 1800
   acatcgatag tgattttagc tgtattttgg taaatcgatt ccgccagacg tagatatttt 1860
   atagccagag ctctgtccat gacgactctg tctccgatga cggcattgcc aaagtcgagt 1920
   ctgcgcacga aatctgtcgg aggttgataa ttaagacttt gtaagtattc tctcacggtg 1980
   acctgtgtca tttcagtcgt cgatcaatat ggacggtgtg ctcgaagaga tgagtgactt 2040
   tttaagcgaa ctcgtgccag aaactaagat taaagtggcc gaaggtaaat ttggcaaagt 2100
   gtccgtatgg agacacaagg acactcaaaa attattttta cagaaagaaa ttactccaga 2160
   gaatttcaat ggtatcgagg tgatggtcca cgacttaatg aagaataaca agttttttat 2220
   taaactttat tatttcacga cgacacttaa aactcacttg cttattatgg attacatcaa 2280
   aggaggtgac ttatgggatc tccttcgaca agagcataaa ctagaggaaa tggaaacaaa 2340
   attaataatc atgcaattgg tagaagcgct ttattatctt cattcacatc aatttataca 2400
   taacgatata aagctagaga atataatata ctcaagatat agacagattt atctatgtga 2460
   ttatggccta tgtaaatcag tcaaaacgga atcgtgctat gacggaacta tggattattt 2520
   ttcaccagaa aaaatcaaag gacatgaata taattttagt tttgattggt gggctgttgg 2580
   tattttgact tacgagctga ttacaggtca tcatccatat aaaaactatt acgatgaaga 2640
   tttgacagta aaaatgttac atcatagaca gcagcacaaa aaattaaaat ttaactctaa 2700
   attaagtaaa gttgttgtaa tgtttataga tggtatgtta aaatacaact ataactatag 2760
   actgaataat gtttatgaca taatgaaaca agagtttatt aatattgaca ttaatttatt 2820
   aaaataaaat tatattacta catacatttt tttgttgttt taaatttatt tacttttttt 2880
   gtatttttta gatttaattg atacatttta aatttattga aatataaaat ataacataca 2940
   cattttgttg tttttattat atacattatt ctattttttg aactgtagtt ataagaggaa 3000
   ttattgtggt aagaccacaa tcctcaacaa tctggacgtc gtcatcgctg tcatcggccg 3060
   aagcaggagg taaaatactg gataatgtgt ctttggccaa ttgttgataa tctatagcag 3120
   tctgctgcag ctgcaccggt tcttctttaa caacgtctag aacgaaatcc gtaggcacgt 3180
   acggtttacc gcgagcagtt agatttaact tgcgcaaatt ttcattattg caagtatagc 3240
   gataagtgac cagttttgtc gacggaggag gctcattaga tgaatttatt gcctccggtg 3300
   tgattacacg caacgcccgt gaatgctctg gaacagttga attcataggc tggttaaaat 3360
   caggattatt gaaggaggcc ggacttatag tatcgtacat tggagatata aaactcgaaa 3420
   attgatttaa cccggggtat ccgttgttgt atgcagcatt gtatccgcca tcgtatccgt 3480
   tgtttccgtt gttataacca ttgttgtaat tgttgtagcc attatatcca ttattgtatc 3540
   cattattgta tccattgtta tatccattgt tatatcctcc gttattgtat cctccattat 3600
   tatatcttcc gttgttgtat ccgttattgt atgaccaatc agccatcggc atcataaaat 3660
   tatgcatcgg tggtgctggc atcatcatcg gtggcggagc tgacatcatc ggcattggtt 3720
   gagctggcat cggaataaac tcagtatcga acgttaaagc ttctggtatc aaagccgcta 3780
   aagccggagt cgctggagct gacgttggca tctgagtcgt tggagctgat gccggtgtag 3840
   ctggtaccga cgttggcatc tgagtcgttg gagctgatgc cggtgtagct ggtaccgacg 3900
   ttggcatctg agtcgttgga gctgatgccg gtgtagctgg agccgctgta gctggagtcg 3960
   ctggagccgg tgtagctgga gccggtgtag ctggagccgg tgtagctgga gccggtgtag 4020
   ctggagccgg tgtagctgga gccggtgtag ctggagccgg tgtagctgga gccggtgtag 4080
   ctggagccgg tgtagctgga gccggtgtag ctggagccgg tgtagctgga gccggtgtag 4140
   ctggagccgg tgtagctgga gccggtgtag ctggagccgg tgtagctgga gccggtgtag 4200
   ctggagccgg tgtagcttcc gtcggcggct gatcagtggt cattagttgt gaagataatg 4260
   attctatcgg cgaggttggt tgcagtgtgg aaaccgctga ttcttcaact cccaattgtg 4320
   ttaattggga ttctatagat ttttctgcag tagccatatt tactcgcgaa ggagacgaaa 4380
   cttcagtatt attgttatca acatcatcgt catcatcatc atcatcacta ctattgctac 4440
   tactatcact attatcacca tcgttattgt catcatcact actatcacta ctatcaccac 4500
   tatcactact attattatta gtttgattat caatctgttc gtcttgttga ttttcgtctt 4560
   gttcttcttg ttcttcttgt tcttcttgtt cttcttgttc ttcttgttct tcttgttctt 4620
   cttgttcttc ttgttgattg ttttgttgtt gattttcgtc ttgttgattg tcgtcttgtt 4680
   tttgttcatt ttcttgaact aatactggtc ttgcagcagg ttttattgta gcagttggtt 4740
   tctcaataat tatctcagac tgtggtagta cagcaggctc agattgaggt ggctgagtgg 4800
   cggtagtaga tggaacatca tcattattat catcattatt gccatcatta ttactggggc 4860
   caggcgattg agatgacgct ggaatatcat cagcgggcgc ttgatcatca gcgggcgctt 4920
   gatcatcagc gggtgcttga tcatcaacgg gcggcggcga tggcgtccat cgcatccaac 4980
   gtggtggcgg cggtggagat tcatatcgga atgaattaat ttgtttggtt tgatagcaaa 5040
   aaatacattc attgtatttc atcatactat cgtaggtaca gcatatgcat aatatatgtt 5100
   cacagtgtgg atacacaaat cctttatctc cattgttgca acttatgcac ggttcagtca 5160
   taatcttcag cttgttgttt ctcatgtatg tatatagtac agaactgagt ctagatgatt 5220
   tttctcgaag tgcaatggct tcggtttgaa gcgcatgagt tattatcgta tccagtagat 5280
   agatattctt ttttatctcc gcatctacgg tgatagtgtg aaaagaagag ccaatattct 5340
   tgaatttgtc gtgcatcaga ccacatttga tcattaattc ttgtctagtg ctagtattaa 5400
   cgttttcgaa aatcgattgt ataaacgaaa acttagcatt ctcatcctcg gaaacagtat 5460
   agaatttttt atattcagat aaacattgca taatatgctc taaaacgcat tgcttggtta 5520
   gtattgtcaa gtaagtctct aattgcttgc gaacaggtat atagtttaac gattggatat 5580
   gcaagaataa atcttcataa cggttagaga aaaagtgtct agatttatat gtgatgccag 5640
   taaacttgaa ttgtttgtta atacagcaca catagcctac gcgctggtta tttttagagt 5700
   ttaaatttaa atagataaac acttttttct cgaccgtatt atggtgcagg tgaatattgt 5760
   tatgattgtt actgatcata tctccagtgg gtaatattat agcagtagta ttagatgggt 5820
   tttcattatc taccacatct accacgggag tttcttgttt ttgacgcttg ctgttagacg 5880
   cagcatcact atcactctca ttatcgatat cacgcttagg tgtttctttt ttctttagtt 5940
   tcttcagtat ttttttagtt ttttttccct ttccttttcc tttacctttt tcaatttttt 6000
   cattttcatt atcactgtca ctttcactaa taattttacg tttgcgtaat tcgatttctt 6060
   cttcttcttg atcttgtcct ttattttttt cttttttaat tttttcataa tcactgtcat 6120
   tatcactttc actaataatt ttacgttttt ctttttcttg atcttgttgt tcttgatctt 6180
   gttgttgttg atcttgttct tgatcttgtt gttgtttaga tgcgctggcg ataacgttat 6240
   cgccagcagc cgattctact gtcacgccat ttgaatctac gtcgtgagtc aattcatcca 6300
   tcttcagtat ttttttagtc ttttttccct ttccttttcc tttacctttt cctttttcat 6360
   tttttttata ttcactgtca ttatcacttt cattaataat tttacgtttg cgtaattcga 6420
   tttcttcttc ttgatcttgt ttagatgtgc tggtaatcac gttatcgcca gtcacgccat 6480
   ttgaatctac gtcttgagtc aattcatcta attctttttg aattttgtta atttttgacg 6540
   aataattttc atttatttta tcaaaatttt tatattcttt aaaaatttct tcatcagatt 6600
   cggtaaatgg tatgggttca aggaacccta ttataggttt tttcatttca cgccgaatct 6660
   cttcttcaga aaaattaaac ctccgtagag tttcctcttt caattctgca ttcgatagcg 6720
   acgcagtata tttcattttc tcaaatttag atttaaattt ccgtatcatt atgatgatca 6780
   caattaacca gtatagcacg tatagaaaaa ttatactgaa caagttttaa ataaccaaca 6840
   catatataat aaaagataaa aaaattatgt gattagatag agatttcata tcatatcata 6900
   aatatgatat agctcgagat aaactttttt gtcgtgcaaa aaagtactct aaagtgtagt 6960
   atatttacag catattgtac agtgtagact attctagtta aatagtctac gattagtaga 7020
   ttttgttctg tatattgatg ccgatacgat aacgaacttt tttgtagtgc aaaaaaatac 7080
   gataacgatt atcgatagcc tatttgatta gatatagcca tgtttgttat ctttcgatat 7140
   aaagtaatat gtttaataaa atatattagt aaaatatatc agtcgtgccg ggtaacacaa 7200
   atatcgatcg aaagtgataa aatgccgaaa ctcaagaaaa ttccaaaaaa accagtagat 7260
   gaccacctag gaatcctcag atatttcaag attatcagaa agccagtttg tgctataaac 7320
   gaattacctc ctaaagaaga tcaaaaaaaa gaagacgaca tcatcattca tctttctagt 7380
   gacgcacctg ataattcatt caattttgat tttgaactta gtgacgactc aagcgatagt 7440
   gactctgacg attcaagcga tagtgactct gattcgtcta acgcacttag tggcctagat 7500
   aaggttttgt tgaattcgtc taataataat gctgctgaca agaatgctat ctttatatgt 7560
   cctaacactc aaaagttaaa cagcagtggt agtgccataa taggcatgaa tcgtcctaat 7620
   ttgactatag aagattcaga taatagcaat gatgaggaag tttatgtacc tacttcgcgg 7680
   cggccaataa aacctgcagc aaattatcag aagataaagc agcaactaaa gcagcaactc 7740
   gaccaaatat tatatcgttt gtgagtgata acaatggaac attatctaat caataatcaa 7800
   taaaagatta atattgtgta cgtgatgttt ttatttgatt ataactgata tgtcaggtat 7860
   aaaagcgctc aaatcgcagt caggaccatc acaagtattt gtgccttgct cccgagcagt 7920
   cctcctctat actttttgtt gtgcaaacta caattttttt tataattcat catgatgatg 7980
   tattacatca agcaagacat tgaacatcta atgtctgttt tgctgaagga aaaacccaaa 8040
   cgtgaaatgg tgttaaacga gcggaattcg gagaaaagtt acttgctcag cagaataccc 8100
   aaacctactt tgttaaagga taacaaagtg tttaatctgg tggccgatat ctttcatgaa 8160
   agtaaagaag agcttaatca taaaggggcg gaaatcgtgt acacatcatc ggcttcggtg 8220
   cctagtgtgt cagaagctaa ctctgactct gattctgatt cggatgacga tgatgacact 8280
   ttagaaaaat ttgtgaagat caagaaaaat gtaaatgctg ttttctcgcg gaccgttaaa 8340
   caacttaaaa aacaaaacaa actaataaaa aaattttatt cagccatgaa taatgttgga 8400
   gagggtgagt cagaagaaac gagacgttta aataacagaa gaatgatagc ctacaaaaac 8460
   aacggcttga gcattgccaa tgttggtgat atagtagata atctattcag ggcttatgtt 8520
   gaaatgactt caggtatcgt acatttaata aataatataa atgaatttgt gatagataga 8580
   ccatatagtg atttcttcga tggtaaagaa ccatatagtg atttcttcga tggtcaagaa 8640
   aatcttcctg atgagaataa ctccaatcat caggaagatc aagcgggaga tctaacagat 8700
   ccagaagatt caaatcaaga ggaagactcc aacaatcgtc aggaagactc caacaatcgt 8760
   caggaagact ctaacaatta tcaaaaaaat caagcggaaa atctaacaga cccagaagat 8820
   tccaactatc aggaagatat agaaaatcaa gctgagtcaa gcggctttgt gccaagggat 8880
   tcaattttct ataatatgta tggtgatcaa gatgatgaca atgaaagtgt agcatatgat 8940
   gacattgcag atgttaatga agtcaataac atttcagaaa atgatgtttc tgaaataaat 9000
   attagcaaaa tgaatatgca agacgaacac caggaagata tagaggaaga tcaacaagaa 9060
   aaagataaaa atcaacaaga taaaaaggaa aatcaacaag ataaagataa agatcaacaa 9120
   caagaaacaa aacgtgccta tcaagagtgg gctttcaacg attatcagac atcatcacac 9180
   aggtccgata gcatctcaag ttctaatgat gactctggca gtgtcgacaa tacaactgct 9240
   aaaagaaaca gtaaatttgt atttaaaact tcttctcgtg ctcgtcgtga catacgccgc 9300
   gacaacacag tgcgtcaagc tctcaaattt taatattttt gttttgtata cttttaaatc 9360
   tttttgttgt tttatataat ttcatataag cttttgttat gttttttttg ccaatttatt 9420
   ataataaaca aacacaaacc aaaactaatt tttttcttta gattttttta gaaatagcat 9480
   tttttcttta cacctttata aacaatagct cacgtcttgc ttaactgaca gggttacaat 9540
   taacttaact gccagggttc gattccctaa aactctataa ccttttttat taagatacat 9600
   caaattaaaa tgagtttttt taatcctcta cgaaaaataa ataaaattta tcctaataca 9660
   acacaattta atttagacaa cttggctgta gttaataatg ctccaagtgg attcaagaat 9720
   gttcttaatt ctactagcat tcaaaatgca ggaaataatc gctttattcc cggctataat 9780
   ataggcaata ataattttat cagtgcagct gatatgaata aaataatgcg caataatgat 9840
   gtcaccagca tacgtaatgt ttttggtaat acaccaactc aaactgacct aaatggcctt 9900
   tcattgctgc gtagagcaga taatgttcct gatgcgaacc ttcatagtgc ggccatgcgt 9960
   aggaatgcag taaaacaaaa ttaccccaac accaatacaa acacacctgc cggtgtaagc 10020
   aatgtgttag aacaaaatcc tcggttaact aatcatttac agacattaaa aaatgctgga 10080
   gtggtaagtt tgattggtgt cggtgtttac ttgactttta gcgcagccac tctaattcaa 10140
   gacataattg aagccctcaa ccgaaccgga ggcagttttt actatatagg aagcgacggc 10200
   ggagataaca tcagcgcatg tttactcatg gatagaactt gccgattaca acatcctgag 10260
   gcaataactg acgtgagaaa atgcgatttt gaccctttaa tatcgaacgc aacagaactg 10320
   cagagtattt gtcaaggctt taattatgaa ctagaaaaat ccgtatgtcg ggctagcgac 10380
   cccaacgcca acataaatac acctcaatat gtagatatat ccgacttagc tcccggtcaa 10440
   actatcgcct gtgtagaacc ctacaatatg ggtgatttaa taggagattt aaatttagat 10500
   aatttattgg gcgaaaacgg cttgattaat aaatcttcaa ataaaagtca aagtttaagc 10560
   gatagtttat tgccgtttat tgtaatgata ggagttattg catttataat tatcattgga 10620
   tattttgtta tcaaaagact gttcaataag caaactgtga gcgtagaaac taatagaact 10680
   cgttgagtgg cttaatcatg tcactttgtc gtctagacga tttacgactg ttttattgtc 10740
   atggcgattc ataataaaac aattatgtgt agcccgaagg atttttatta gactttcgaa 10800
   tgaatacata taatttttat acattaattt ttttataaag attcatatcg taatagtaga 10860
   tacgtttggg cttgatggtt ttaactttaa cacaaacgct ataaaatttt aaagcagaca 10920
   cgtcgagttt gtttctagtg aaaaacatgg catccgtaaa accgcactga gaacaataca 10980
   gcactatatg tttttttaga tacattttat gtcgagtgca gagaatgcaa aatcgcttac 11040
   acggtatagg aaaatatagt acaaaattaa aagtagtttt gtagactttt ttttcgaccg 11100
   aatagaaata ggtgagcagc ttctgttcgc ccatgctcgc aaaaaaatcc atcattttgc 11160
   tatcgccgaa atatagctga aaagtgttgt catcgcagcc gatcttgcta gaagtagtct 11220
   tgatgatgca atttctgtca gtttctgtaa atagagttcg accatatgtt cgcaataaaa 11280
   tttttgtgat ttgttcgttg ggcttttttt tatccactat atcctttatt gtcccaaaga 11340
   aatcgtgtcg acccacaacc gaaatctctt ctgtagtaca agtgtgttcg agattaatat 11400
   tgaacaagta gcatttaaaa aagcccaatt gacacagagt ctctgcttct tcgagtataa 11460
   catatggata tatctgaaac aggtcaaact ggtgtttata tttaaacagt tcgtaacatt 11520
   tagagcagat caatttaaac ttcttgtgtc tctttaattc atcttcaatg tctatgctgt 11580
   tgtcgatgag gcaaaagagt gagaaattag tctctcgctt aaactgtttc gagcattggt 11640
   tacatgtagc cttttctaca atttcttctt gcttcaaatc tctacaattc atctgtagca 11700
   aattgttcac ttttaatctt ccctgaacgt aatcggacgc aaactgaatt atcgattgta 11760
   gtagacaata attcaaatga ctcaagaatt taggtctcaa atccttcgga gggctttgaa 11820
   tgatgggcgc ctgtctataa tctggattgt tggctctagt ttctcgaact atattcatca 11880
   ggtggtctat cagcatgggt ttcctttccg tttttgaaga cattttgact gttgtcgtgg 11940
   aatacgattg ggtcgtcgat gacatatgac cgatgtttag aagcgcacga tagcttatga 12000
   aaataaatac gagaagcgaa cactttatac acgctctgtt gacctcggcg cgaccgtaga 12060
   gttataaata aacacataat atgcgcggga atttgactct acaaaggcgt cacccgtcaa 12120
   gtttatatta ttacgagacg cgagacaata gcgcgattca tttacatgaa gatgctcatc 12180
   ctgtctggta cgtgattttg tagtcatgga cccagtacaa aagcttatct catacgatgt 12240
   tgtggtcagt catcatcggg cgattgtcat gaacactttt gattatcaaa acgatcttta 12300
   taagcgtatc gcaatggaaa gtgcggctgt ggccagcatt ctattgtcac cgcgaaactc 12360
   tgacgatgga aacagtaata gtataaatag tttcttgaac attagaaaca acaatggaga 12420
   aaaaggctcg accgatttcg aaacacaggt atttaaaaat tttattttta acgacctgca 12480
   caccaacgat ttagcgttga atcccaaggc ccaattttta gttaaacagg cctcatttaa 12540
   tgtgatagac gagatctttc aacagacata taacgaaaaa atcgacaaga ttctggcgtt 12600
   tgtagatgac actgacgaca ttcgtattcc caacgataga tgtttacatt atttgatagc 12660
   ggaaatcggt aaaatcgtta atgttttaca gcacgttaat cgcatgtcaa agtttgaata 12720
   tagtatgtac gtatttatgc cgtatctaaa acaaataaga aaaactatca ttctattcgt 12780
   caatgacttt tgctgcaaaa aactcgtcga aaactattta ttaacgctcg atgtgatgat 12840
   tgcggactct ttaaaattac tagaaacgat tcaaatgatc aataaacgtg tagatgtgat 12900
   gaacgtgttt ctcgatcgac ccgtgtaccg gtgcaacatc tgcgaagata ctagtttaga 12960
   atcgcgtttc ctcaaaccca acgaatgctg cggctatagc atctgtaaca tgtgctatgc 13020
   caatttatgg aaattttgca ctctttatcc agtgtgtcct gtttgtaaga ccagtttcaa 13080
   atcttcaggc tctgcagctt cttctttgtc gtcatcgtcg tcgaccaagc atcaagctct 13140
   attcgaagaa taaaatatat aagatttaaa tatgagtcta atcggcgaga gacagacttt 13200
   ggaaaaaaag cacatgaaac acttgtttct ggcatcgtat tttaatctaa acaattatga 13260
   ctatttaccg gtcgaaacga ccgcttttat tggcgattat ttacgcaata atttcgaaag 13320
   actagacgac tcgactctgt tgcaatatat taattatttg aatagtatcg ccctcaaaaa 13380
   tctcgtagtc gacaacagtg cagacagttt taaatatgta aagccacagt ttaagtttat 13440
   ctgcggtcgc aacaatatcg acattatcga gttcgattca aaggttttta ttaaaccgaa 13500
   aaccccaatc tacgctacca atttattcgt aaacgacccc aaacagtttc gattgattct 13560
   ctatcaagaa ttttcaaagg tgtttaacga tcgattattt gtcaacaatg gtgacactta 13620
   ctgcataatc gacggtggcg aaggctacat tttcgaagac gcctacatcg attggtgcgg 13680
   gattcgcatg tgtagtgtgc ccaaagtcga aaacgatcaa tatccgtatc gattgtattt 13740
   gatcggagaa cccatggctc aacattttat caggaacaat atcacgatgc tcgcgggcag 13800
   cgactacatt ctcaaaaatt ttcacaaagg attgccgctg tttcgcaaca attatcgggt 13860
   gattaatagt aaaaaatttg tgacccgaaa gcctaatcag ctttttgcag aaatgcgagc 13920
   ggaactggac acgcacacga attatataaa atttattcaa cgtgactaca tttacgatgc 13980
   taatttccct gaagatttgc tcgatctcct caacgattac atgaccaata catctcatta 14040
   caagtttatc accaagttta tcgagccggg catcaagatg ggcaacagtt atagcgagat 14100
   tgtggtcgat cgttatgccg ttaataagta tcgaaaatta aacataaaaa gcgagccgaa 14160
   cacactcttc ccggcgctgc gttacaacga tccgtcctat atttttgtga gaccagatat 14220
   caaacaaata aagggcactt tgaacgcttt tttcgtgccg aaagaacgtt tattggtgat 14280
   cttggccaac agcagcctgt tcggatccac cgagctgatt cattttaata aacatcgaaa 14340
   cctaaacgag ctggtgcatt tcgataggag tttgctgccg tatagacagt tttcgcctcc 14400
   gcaggtacta acccttgaca cgtatatcat tgacgccagt cacaaattgt atttaactaa 14460
   gcacattttt ggtaatacag tgcctgcata tttaattata agaggagatt acgaaagttc 14520
   tcaatttcaa gatttgaaaa atctgaaaaa ctcttgggtt ttgaatactc tacttaagct 14580
   gtttatagat ccacaataaa ataaaatatg gaagatttac gttcgccagg aactacgagt 14640
   cgattcaacc cgaacatgtt caatcccagc atgttgatga ccattctcat cggtctagta 14700
   attattatac tgctgattat gctcttccaa tcgagcagcg cgggaggcgg tagttctcca 14760
   gcagcggcgc ctcaagcggg ctttacgaat ccgttgaatg ccacgatgag agcgaatccc 14820
   tttgtgaaca caccacaacg agcgatgttg taaagcgttc tacgagaatt ataagacaat 14880
   ataaacaaaa tgaaacgttt caagtgtcag acagcaccaa aagtgagaac cgtcaccgaa 14940
   atcatcaaca gcgatgacaa actacaaaag gaatacgatc tcaccgagtt tgatgtgaaa 15000
   aacctcaaca gtctcgaaag ttatgatacg ttaaaaataa aactagtgat cgtcaaatat 15060
   atggccatgt tgaatacttt gcagctaact cagccgctgc tgaccatctt ccgagacaga 15120
   aacgccactc gcgagatcgt taccgttgtt ttggcctcac tcggcttcgt acacaatcgc 15180
   gtaaatccac tagtgaataa ttttaatcgg aaaatggagt ttataattgt ggaatcgaaa 15240
   aacctgataa ttcccggaga accgattcta tttcgacaca acgaaaatga agatatcgtt 15300
   tgtattatcg atcgagtcag tatcgtcaag atgctcgaaa agcaattcga cacggacatg 15360
   aacgtcagca atattattca agaacatcaa aaacttaaac tcatcaagtc gtttacgagt 15420
   gtaaagaaac gaaaatcgtt tgacgatcag gacaatagcc tctacattaa gctgaacgaa 15480
   atcgaagcca cccagtacac gactttgctg ttcatcatgg aacacgctta cggacactat 15540
   tatattctca aaaactatgg catctacaat tatactcagt cgcttttgga tcataccatt 15600
   tttactcaaa aatacaagcc ttctttgaat gtcaactttg ccaatctact tttgagtaaa 15660
   ttcagattta ccgtcgagga atctgagggc aatcgaagca aatcttgcgg caacaatagt 15720
   ttgtattctt ctaaaaatca actagaaata ttaagttata gcagcgataa ataaaattca 15780
   tgtggctttt actagcgatt tttattattg ttaaactatt agtttttcat aaaatgcaaa 15840
   atatgcattt agacgtacac aatcataaac tttgtccgaa tggatatcac ggcctcgtaa 15900
   atgatccttt cgattgtaac gtttattaca gttgtcctca aaaaactcaa ttttattgtc 15960
   ctcccgatga acaattcgat gtcgagcgac aattatgcat accaaacgat attcatgacg 16020
   atagctgcat ggccagaaaa tacaaaagct tacttctcta atttttaata aaactcgaca 16080
   ttcgcacact agtttttttt tggttaaaca cattcgatcc tgacgttttc tctgttaatg 16140
   cagacatttg gatatacaaa agctgccatt tcaatgtctt tcgggttggt gtgagttttc 16200
   gaaagtgatg ctataaaatg cgatggtatg ttgccgcgga gaatgaccag ttctttagcg 16260
   aaataattct caattccatc gacacatacg gtcacgcctc gcacacgctg gaaagaagct 16320
   ggctcgtgat ttttattaac tgtaaaaatg tgccacactt gtatttcgcc tttacagtcg 16380
   acgacggccg gcaacactgg tctcgaaaaa ttttgatgat tcaccaatct gacgcaaaaa 16440
   aacggtaatc tgacgcaact aattacatac gcgtttctaa tggcagtgcg atcgatcggc 16500
   ggcaatagac acttcattcg tattgcaata ttaactcggt cagcgcgccc gcgttcgtag 16560
   ccgcttacga gtcttgtgga agcgaaattg cggttattac catcgccgtc agtgtcatcg 16620
   ctttcgttta tttgaaacgg tatgatattt atcgagttga tcgcatttat cacgctaaat 16680
   gtaattaggc tatctttatc ttgaggatag tataaattga caatcatttt atttttactt 16740
   ccagttctca cttacaagcg ttgtgatgtc gcatccaaac atcatcagtg ccatggacaa 16800
   caacgacgcg tctatcaaat ataaaaatta tatcgacaac gccatcaaca ctcctacgca 16860
   tacgattctg cagaacgtca gtatggattt cgacgacagc aatattctgg atttcggcaa 16920
   cgaaaatgat atgaatgtct acgacagacg agacagtaac agcagtaaaa ttgagaatga 16980
   tgcttgcgat gaaaactctc aattttctga tgtcaacgtc aacaataatg cggacaacaa 17040
   tgattatata aaaatcatga aaactgctac cgatgtcgtc gaaaacaaga atgaatatac 17100
   gaataaacat aaaactgcag tcgtttcgac taaaccattc aagaaaaatc ctaaaaaaag 17160
   gccatcgtca tcgttgacga ctacgacgat gacgacgacg acgacgacag aaaagaagaa 17220
   caagtcaaga cctaacagac ctcccaattc cactgtaatc gctgagggta gtattccacc 17280
   acaacctgtg ataaagccat cgaagaagca gacagttttt gtttcgcctt taatcaatag 17340
   aggaggaaaa aacttgaatg ttttgcgcaa cgacaataat aataatttca ataatgacag 17400
   tgacgatagc aacggaagcg acagtgaaga tagcgattct acgcatccgc cgccttcgaa 17460
   aaagacaaaa atgacatcaa aatcatctaa aatgtcggtg acgccgcaac aacaaatgcc 17520
   cgagattttg aaaattaata ctgccgacaa aaataaagtc aatgacgaga aacaaacagt 17580
   gaaatataac aaaaaaaagc aacaatctca agacgctggt gccgtcgtgg tcgtgaaaca 17640
   acaaaaactt gataacgaat caacaagtca aacttccgtt aatgatgatc aacaacggtc 17700
   gaaagattgc gattctccaa caaatgactt gtttgaaaat aaaataatcc ccaacatgat 17760
   gaccatggaa agagacaata accgcaagtt tgtgcaatat attctcaacg ctcacaatta 17820
   tctgtttata gtatacgaaa acaagtataa tgccaagact tttaacaaaa actccaacgc 17880
   atcgatttat aaaatagagt atgtgaattg cgtccagtcc atatacaagt attataacgc 17940
   caattactcg catatcgata gaacatgcaa agtcgtgtct ttcaatcgat tcagattcgc 18000
   catatctgtg aaccttttaa ataaaatgca gattgaattg cctcctacgg aacaatttaa 18060
   aaaggaagac ctcaagaaga tttctccgaa gaacactttt tgcctattaa atgaagtcaa 18120
   agatccggat ttcatttcca agctcactaa cacattcggc ttggacaata tttatattca 18180
   gggtcaactc actatgctgc tctcgtcgat tggtgagaat cgggcaaaga ttttgaatca 18240
   gcatatcagt gcaatgattg aagataaaag cctattcact atacctttgc atttgtctcg 18300
   atccaaggaa ttggaagaaa ttgttgatga cgatctgaac cccaacaaca gtaacgtctc 18360
   gtcggcctac attcgagaca taatagaact ctcgaacaaa ctcaagttta aggctcctat 18420
   tattccgtca tatgtctata aaaccaagga acaaaacatt gagaatgttc ttagtttttg 18480
   gatcaacact cagaagaaca acaacgagcg cgataaaact ttggcaaaat ctctgcagtt 18540
   tacatacaag tttaccagtg tcgctcgagt gctcttcgac gaaaccgatg gcgacgtcaa 18600
   taaactgttt aaagtgaaga aagagcctgg atctgtggca atgattgaag attatctaca 18660
   ggcttgtgaa aaaataccca atggcaacaa ctttattatg atcaacacac tcaacgatga 18720
   acgtgtgaca atcatcaagg ccaaaaatga attcttttgg atttgtacta ataatcctaa 18780
   taatttaatt cactgtatcg atatcattat ggcctttaaa aactttaatc atcatttgct 18840
   ctctttgatt cccagcaatc gtaaggattt gaacaatcgt cacagtggat taataaagct 18900
   agtggcctat catttaggtg gtgatgttga cattaatttt gtacgtgcca tgactgagaa 18960
   gtttaaatgt aattatctat ataaaaaatt ttaaatgtat aatttttctt gtctactgta 19020
   atttgaataa atttttaagg atcgtatttt gtatagtatt gtagaattag tattatgatc 19080
   aataaaaatt tttttattcg tatatcattc tatggtttat ttttaccttc gaaattatgt 19140
   ccaataagaa aataatcatg tcgactttga ccgctatcga tatcgataac gcattcaagt 19200
   attcgactca tatgcatagg ctgatttata ttcacaagtg gagatctaaa tttccaaaca 19260
   tttttattaa ttacgaaatt agagcagcaa cgaatgacga ttattatgtg cccctcgccc 19320
   tagccgacag ggccattgct gttaaactcg agtttagtaa agagggatgc gagagtattt 19380
   cttgctatcc ctataacgaa tatgggcctg tggattttaa aacacctacg ggctacactc 19440
   aaacgtccga tgtgcccgta caatacgctc agccggcatg ctatcatttg gacagagcgg 19500
   cggctacgcg acaaggcgca gagaatgaag tccaagcacc cgaattgagg tataccgacg 19560
   gcggtaaatg tattctagtc gataccctaa ctaaaatgta cttgaactct ccctacatac 19620
   gaacggacga gcatctgatt caaggtgtcg atgatgtgcc tggatttaat gtcgtgcccg 19680
   acacagatcc cctatttccc gagcgattca aaggcgaatt taatgaagca tactgtcgac 19740
   gcttcggcag gtctctggtg aacggcggtt gctccctaca gtggtgggag agtttgatag 19800
   gattcgtttt aggcgatacc atttacatta catttaaaat gctgatcaat aatgttttta 19860
   gcgaattgcg agatttcgat tatagtcgac cgtcgccaga gttgccgaca aagcccgtcg 19920
   tcgattccga acgactctta aacgagtgga aaaaccgcat cgacagccgt tctaatcttt 19980
   tggatgaaat aaaatttgcc gaatttaaaa cctattccga tttaacgatc agtagaactc 20040
   gaaaattagt ttatattgcg gaaaagggat ttcatcatga gacgataccg tttaaacaat 20100
   tggaatttcg aaaggtgtcg accgctttct cacgtacgaa catcgacaac acagcgtctt 20160
   tggacgacat tattactcaa tttctcgagg acaattcgtt actcttgggt ctggcaacta 20220
   gctacggatt cgatgtcatt ttcgatcaat tgaaggccat gctcaaaaga attaacacca 20280
   ctctaattcc agccatgaaa cggttgcttt tggagacatc gaaacgtgtc acggccaaga 20340
   tgctcggcga aacttataaa gctttcgtgg cccacacctt caatcgtatc gccattaaaa 20400
   ctctatcgac cgtagcaaag gcgatgacaa gaataaccat caaagccgcg tcggtcgtcg 20460
   gaattttgtt aattattttc acaatatctg acctaatttt agctttttgg gatccgttcg 20520
   gttacagtaa tatgttccct cgagaatttc cggacgactt gtcgcgttcg ttccttgcgg 20580
   cctatttcga gtcgatcagc gatggcaatc tagacatgat agaatggcta cccgagtact 20640
   ttgaagacct catcgaagag gacgatgtcg ccatgtttga gaatctaaaa tccacatttg 20700
   agtatgtgac ctctctcgaa ataaactctg acggacaact gttggagttc gatagaggcg 20760
   aaagcatcga agatttcgac gaaatctcgt taattggaaa cgctcttgcg tccagcgctc 20820
   tctacacacg actcgaattt ctacaatata cggctcgaca caataaaata ttatacggca 20880
   ccgacgacgg tattgtcgtc aacaacgccg gtaataacgt tattttgccg acgcttttca 20940
   tttttggcgc agtgatcatc atatttatac agagtcaaga tactattcaa aataatatta 21000
   tggtatcgct attcatcgta tttctactga ttgctgttta tttaatcata cagaattctt 21060
   tattatacta cttgagttta aggaaattta cgagtaggac acaggctaaa tggtatgata 21120
   atttatacaa ctaaatagag attaaatata tttttattca acaaatcgaa cgatatgatt 21180
   acttaggaga ggacttcttt tttctgagcg gaacatcggg cacgtcgggg atctcgggta 21240
   tctcggggtt gagaatctca gtaatgccgt cgacggctgt ctgaagagtg gcgagactcg 21300
   cgctttgagc gtcgagtttc tcgtttaatt ctgtgacatc gggtagattg gcgcgaacgt 21360
   cctcgactgc cgactgaaga acatcaactt tctggctgac ggcttggatg tcggcacgaa 21420
   tcaacaacaa aatattttga gacatgtttt aattttttta atatttatac gataaatgga 21480
   tctatactta ttatatgtaa ttttattgag ggacaattaa cggaccgtcc aatctaattc 21540
   tgtctatttc gtattcgcct tccaccaaag agatgcttat ctctttttta ttgtgaaaaa 21600
   tggcaatatt tctaggtaga tcgcgatatg ttaaacgatt tgccgtcgtg gacaaagcaa 21660
   actttttcac ggtcatatac ggcgaagaat aggccatctg tcgtttacca taaaccgaca 21720
   tgccattacg caacaaactg acatatactg ttgaatcgta attaatttga ccagaaacca 21780
   atcctttcgg ccttatgccc gacactggaa acagcacaat tccagcgtca taatcgtcat 21840
   aacgacacgt gattacagag accagacgat cgttaaaaaa tatcggtgcg cctatccaaa 21900
   tcttatcggc aatccacaga tcgtctacag caaaagtgcg caactgtccg tagatcaggc 21960
   gattggcgtg tctatgaaaa ttagtataga cgtgttccgg ctgcactcta aacaatatgc 22020
   cgtcgtttag caagaccatc aaactgtcgt tcgagttgtc gatggcgggg aacacaatat 22080
   cgctggccac cccgggaaaa tgatgaagaa ccgacagcgg cggctgatcg tcaccagttt 22140
   ttatgtaatt cgaatcggaa tgcggtcgaa tcgtctcaat tctgacagct ttgccgtcga 22200
   cggcaactac acgaataatt ttttcatttt catcaatact atattctaca tttaattttg 22260
   ttctccgagt cgtcgtcggt gtcgacgcca ttgacgctga cgctattgtc aacataaatg 22320
   acaagataaa ttgagattga atcattttat ataatacctt atcttgtgtt ttttctccta 22380
   ccaatctgct cattaatagc atatttaacc tctgacatta cgagttgagt atatattaat 22440
   tgagatgaat ctttgtcgat tattaacgaa cgaaccgacg aaacgaacga gccaaaatgc 22500
   ctgtttttaa aggctctaac agcaaaaaaa atggagtgtc gacgagctgc acttctacaa 22560
   tcactaaaaa tacatggacg gacactagaa acaccatggt ggtcaagagt aatgacgaga 22620
   atctaaggaa aaaattaaac gaaatccttc aagccaagaa acaactcagc attcagatgg 22680
   aacattggga aagaatcaag cgcataacga aggatcccaa agaaatagca gccatcgaat 22740
   gtaagctcaa taaaattaga aatgattttc taaattttgg gtcaaataat ttctagtcat 22800
   ggataaaaca aataaaaagt gtgtgtataa ttttaaaaag gagttttatt ataattttca 22860
   tcattaacac caccaccacc accaccacct aaatctacat caataaatgt cgcagaatta 22920
   taccaatcgt catcgtgttt gctgactttg gtgtcgccga cggatgtcga tgaataattt 22980
   tgttttagat catcaaaaat atgatgacag ctctcaaaat catgtcgttg gcgacgacgg 23040
   tcattgcgcg atctccgcct tggcgcctca gttaatttgc acttgaataa gctcccgtcc 23100
   ggccagtaga ctctacggtt gacagcaatc aatttgttgg cggcataact tgatgtcaat 23160
   acgattcgct tgcgggtgca tttgtcgtgt ttgatgtcag ccttgattct gccgccgcac 23220
   acatgattga taaaattttt aataaatttt ttatcaacat tctcgccatt gacataaaag 23280
   acgaaacagt cacgatcggt atagtcgtac attttttttc cttattattc ctcatcttct 23340
   gcaggtttta tgtgaaaaaa taccatcgga atgatggaaa gagacaatgg cttttccttt 23400
   agtttggttc cagtcagagt atgtagcgat tcttcctttt ggccttcctt cacaccttct 23460
   actattgatc ccacaaacat tggcacttct tcagaaggct tatcattgtc ctttagcttt 23520
   aatatttcat cgaagcgttc tatagtaaaa gattcacatt tgaccgtctt gtctagtttg 23580
   ccagtgctga agagcagttg attatgcttt cgagaaatat caaaaaattt gttcacattc 23640
   tttgcgtatt ctttgcccat gttgaagtag taaatgttgc taattttgca gtctttttct 23700
   ttaaaatgca tcgatatgat attgccaaaa atctcattgt acacatgtct gttggccgaa 23760
   cgtatagcta aagaatcgcc atactgacca cgagtacgtt tcaccatagc gtaatcgaaa 23820
   tagaaaaacg attttaagcc acccttgaca cgcataccga tctcgtatac atttttcttg 23880
   cttgacccgt tgggctcgac gacgcaaatc tgattcagat attccgtagt ttccatttgc 23940
   aaatttagca gtttactcag atgattttca atgtgtatat tgtctcgaat gaatccaaag 24000
   cactccgtca aaatgttata gggcgaaatg caatcgatta ttttattatt tacgtaacgt 24060
   tgagacacaa ttgtattgat catgtccgac atccaagaac tctcggtctt ccataaacaa 24120
   agggcactat cagaaacatc aaattcgatg ttttgagcga catcatcgga atcgatggcg 24180
   gctccggcgg tgacaatact ctcgtactct tccctaggtt tcttgctgtt cgaatcttga 24240
   tcgttagtat cgagtttcct cttcatttcg ctggcgctgg cgctggcgtt gttgaatcga 24300
   gatgccatgt tggacgacgt gtgtctaaag actgatacgt atgaccgatc ggtaaacgct 24360
   ttttatacca tataccatta atctatcttt atatatttat ctagtaagcg taatcatgga 24420
   gtatcgttcg tgttatcttt tgagcaatag cgccttttcc tcgaagagtg gtgtacgttt 24480
   taaacagtac aaaaatctga tgaatcttgc caaaggcctt ataccatcgt cgatagaacc 24540
   aatttcgatc atagaattgg aaaaatttaa aatgataatc gagcccgaca ccaattacgt 24600
   ttccaatata cacgatttcc atttttatac cgacaataaa gatttcactg tcgttcacgt 24660
   tttagatgcg aacactagac agtttttggg taaactcatt attaactata atatcgatga 24720
   cgctgatgat catggcattt tcgggttggt tgccgatctc gccaacgata attccgacga 24780
   taacgataac gtttatcaag atagtttaga tattgaacca atataaagtt gaaattcagc 24840
   aagcgttact ttattctagt agtggagctc gctttggaac agaactaaat tcattattcg 24900
   acatggagta cctgacaaat cattcgatat tgaatatgat ttccgtttat tttgaatcat 24960
   tcgatgattt tttgaatctg cagacggcca tgcaactgga tcattttact ttttggaatc 25020
   tattctatcc gggagccatc aaaaagaata atatttacaa tagtcaatat gccatgtgct 25080
   atttcgattt gctgaacaag cccaaggttt ttagaaaata caagaaatat gaagataaat 25140
   ttgatttatt ggtaaccgac gacgacgagt gtgtgggtct tcatttatat ctgcaaatga 25200
   tcgattacga ttaccagcac caatacagaa aattgttgcc attgatgcac gatcgacagg 25260
   ccacattcga tgccatgtat gaattatttt gcaagagtgt cgacattgag gatatgttca 25320
   gtgatgtgcc caggaaaaat atttctgatc cggcaagtga aagacttaaa tataaatatc 25380
   cgattaatgt atgtaaaatt ttaacatcaa gagctacata cgtgatcttt aaagggaccg 25440
   atttggagct acgggtaaat tttaaaaaat atccaaagct attggagtac attcagaaat 25500
   tgcttacgtt cgaactcgat cctgaggaga gatgctgttt tgttaaaagt ttactcggaa 25560
   acaggagtaa acaattgtgc gattggcata acatttttca atattacggc aagtctgaag 25620
   tgctgtataa agcttttata aaaaataatg ttttttataa attagtgttt ggcatcgaaa 25680
   aggatatttt tcttcaacaa tacatcgaga gcaccaatca tcttggtaac ttgcgtaaaa 25740
   ttctgaagaa tgactttaaa aaattgaagt ttaacactga agtggggctc gataatacat 25800
   ttccagtttt aaaatatgtc gatttatatg tactcaacaa aatttgtctt gagtttaagt 25860
   ctgttgaaaa aaacaccaaa aagaagatta attttctttt acagaatatt ttattcatgt 25920
   ataaatcaaa aatattatta gataaaaaaa tgttgaagga cgtcaaacat cttacaaact 25980
   tagctgtaaa tagaatttta atttataaaa gagtcgatgg atattataaa gacatacttg 26040
   attataatag aaacgcaatg attcaacaat ttaataacac tttgagtaaa aaaattgaca 26100
   aaaaaatcaa taaacactac aatttcaatt taaaagtttc tcgcaagcgt aaattgtcta 26160
   catgttcaga catttaacaa atgattgtaa tgtggttttg ttacatataa ttaaattttt 26220
   ataaaatttt tttttcgttc tattcatttg tttcttcatc ctcctcatcc attgtttcga 26280
   acgaaacgtc atcgacatcg tcgagacagt cgtagttata aaaagtgagc aattctttaa 26340
   tactcgaaaa tccggtcaaa tccctaaaca agaattgagc gtttaatttc cagaacggca 26400
   cgctcaaata cgagtaatac agttctacaa aactgatcac acccatttcg ctgtttaaaa 26460
   actcgatata agcggaattg ttgacgttcg acgactgatc gaaaacgtat tttcgatgaa 26520
   aatcacatcg aagttcggca tttttatgat aacacaaacg acaatggtaa aatttttcca 26580
   actgcaaagc gtttaaataa tctcgcgctt cgtccgagag aatattgatt tcttcgatgc 26640
   taaaatcttc gtctttcttt cgtttcaaca ataatttgtt tctacccata tgttgaagaa 26700
   catcgcccag tttcggaggt agcgacggca tcggtggcgg tggcggtggc ggcggtgaca 26760
   atgacgatga tgtcgttaac gccaatgttg cggtagctgg tggagttttt ggtcgtatcg 26820
   atgccatcag ttttgaggtc aatcgacgca aatcctcacg aaacgtctcg tttttattca 26880
   tattgtcatg cgcgagcgcc aataatagag ccttaatgat actatttctc gctaaattat 26940
   ataagtactt tttagtcaaa caaaatgcaa gtgtttgtaa aaacgttaac tggaaaatcg 27000
   atcaccatcg aattagaatc aaacgacacc atcgagcaag tcaaacaaaa gattaccgac 27060
   aaagaaggga tacctcccga tcaacaaaga ctcatttacg gcggcaaaca actcgaagat 27120
   gatagaaacg tcagcgacta taacattcaa aaagaatcta ctatccatct ggtcctgaga 27180
   cttagaggag gaaaaatatg attagtttga atcaaatcat aaaaatcgtt ataacaaaat 27240
   ctaatttaca cggcatacac aatgtcaagt tgattgacga aaaaaacgac aaaattgatg 27300
   atttttacca agccgtttta atcgtgcctc cgggcatcgg taaggtattt taccagggcc 27360
   tacatgtcac cggtcgtgta ctcagaatcg aaggaaccaa tgtagatttg atatatcaaa 27420
   aagagtaaaa attttttaga tttcgtgtgt gtactgtgat atagtgtaaa atcctctatt 27480
   tttcgacatt caatatactt tgaaatgatg tatagaaaga aataaaaaaa attgtgtaca 27540
   tttaagtttt tttattttac aattaaacag atgacatttg actctcatca aaatcgtcat 27600
   cgacaatttg ttgatatgtc gatgatgatt tcgatgacga tttgtgttgc tgtttagcct 27660
   tttttacatt caaattaata ttagttttct ggacacgttt tttttgctgt tgctgtttag 27720
   agctgccggc ggcgacatga ttgtcatcat tgttggaggt tttaatcgtt aatttatgtt 27780
   cagacgacga cgacgacggt acaatcgttt ccaataattt tttaaagcaa tcgacaccat 27840
   tctgtatcat gtttcgattc aaacgctcaa aaccgacatc cgatccgtta ctgtttttag 27900
   atagcttgat ttcgtataga aagctttgat taggagtcga tccatcagca aacgtttcgt 27960
   tcatcactcg ataaaactca ttgtataatt cagcacgctt agcgttggag gcatcaattt 28020
   tactagattc ggcaacattc ttgatatcat tttcttcaat gttggcgtca gaattagtct 28080
   gttcggagat gactggcgcc ccatcgactt ctatactttt gcctaatttt tccataaaat 28140
   caaaaattaa aacaaacggc ttgatgttcg aatggctctt cataatattc cacatcatgt 28200
   gactgttggg ccagaccgat tgcttgtttt tcgtgtacca gctattgaat aggatatact 28260
   tgttattgct gatcatcttc tttgttttct ttgcagtccg tttgtcgtgc aacaaaataa 28320
   tggccacggt aaactcgaga ccgttgtcgc catacacagg catcatggtg attctatact 28380
   tgattttctt cttctcgagc agttgaattg tggtattgac attttcaaaa ttgttcctgt 28440
   cgaaaactag tttgtcgctg tatttgtcga agatttcgac gagattatta cagtttttgg 28500
   tggagttggc catatcgatt cgaagattgt ttatattcag tttcgaggga tttatttaaa 28560
   tgaaatattc tttttaattt taaatgatga cactgcacgc gcttgcactg cgttcgctgt 28620
   ccgccaacgg ttatgagtgc tttatcaaga ttattcctta tatagtcgaa ttgagtagcg 28680
   aatccatcat cgaacacatg atcacaaaca ttggtcacgt ctcccgtatg cttacgctta 28740
   ttaatggtct ctcgaaccaa agcgtaaatc tcgcttctag tcaagcagca ttgactatat 28800
   ttttcaggtc ttgactgttc atcattggaa tggtggcgtt gtcgctgttg ttcgacgtga 28860
   tgttgatgct ggaccgacgc gtggctgctg tcgatttcgc attcgttgtg gtcctcgaac 28920
   gcaaagtcgt cgccattgac gatttccatc gttccaatcg acgattcagt tttaaagata 28980
   aaagaaaaaa tccaaatgaa tttttattac caaacttgtc gtagccgttt tcgtcttttt 29040
   cgcagttgat aacgttactt tcgtcgaatt tagctttgac aaatttaacg tattcaaaaa 29100
   actcacgata attactagaa tcgtacgtga tcaattgctt ctcgttcatg tactgaaaat 29160
   agtcttgtaa cgacacgata tatacgtgtc gaggcaattc tctgttgtgt cgtcgagtct 29220
   cgatatcgac tcgatagtcg ttgcctttgt cgccgccgtt attgagtttg acacaatacg 29280
   tattcggttc tcgagataca ctgattagca aaccgtttaa aatgcccacg taaatggcgt 29340
   atttgtacgt gacgcccgag tctatccatt gcaaaataaa cggccgacgg tagacatagg 29400
   cgctttcgaa aaaggtgcca gtctcctcga taaactcgcg aatagcagtt tcatgatcga 29460
   aaatgtctcg attgtcgcgc tttcctctgg gtatagaaat tttttccaaa aagtttgctc 29520
   tttttagcac ggcactgtcg ctatattcga cggtgctgtc gtacgatcgt cgagcacaca 29580
   gcagcaccgc cttatcgggc tccattatca taaacaggcc tgcacttctc atcgtaaata 29640
   aaattcaact actttatata ttaataatac ttttatttta tataaaacca ttattatcac 29700
   ataaaatctt aacaaacata ttagtacaag acatcaatcc tctcgaagct gagacagatc 29760
   attacaaatt tttaacaaag actgataatc actaaaaatt agttcaaatg attgtgcgta 29820
   gtcatgtgga gaaatctcga tgtcgtttaa ctcgaaaaaa gtattcattg acgttaaatt 29880
   cttgtgattg tccaggaaaa atttttgaat caattttctg ttttgatcat cctcgtcgtc 29940
   gtcgtcgacg tcatctccat cttttctggc gtttgaatcg atgaattcgt cgaaaacgta 30000
   aaggacagcc atctgaactc cacgaacgta cggatttgtg taaatagttt tatttaaaca 30060
   cttgacaatc aaatctgtgc cgatgtagtc ataaatgtca gagtcgtcgt acttgtcatg 30120
   gagaactaga cgaattttat tacaaagctg tctgatgcaa tccaaattta aaatttgtat 30180
   tcctggattg tgctcgaatg tttgcagata atacgcttgc tcgtgcagcc cggacagatg 30240
   tataaaacga aggaatttgg aatacatttc gacttgaaat gacgtacgac aacaggcgaa 30300
   gcaccttcga ctttaaatga tgtacgacaa cgggtgaagc gtctatttat atgccactcc 30360
   actctacaca atcatgataa cgagtctgca tctgatcgac aataatattt attcaatcgt 30420
   tgcttgataa caccacacaa attgtacgct cggaataatt tagccatcaa cacaagaata 30480
   tggtttttgt ccgtggattc gatgtcggtc actcgagtca agtggaaaaa ggcgctggcg 30540
   taattattat tcattaatgc cgtgtagcgt ttaaataaat ctctgctgcc gattcttttc 30600
   aaactcgtag ccaaccatag cgtgtacgag agaatcaacc gtttcttgtg gtcgatttcc 30660
   gcattctcat cgtcgccgac gtacaattga agccaatcgt ccagttcttt gcgtatattg 30720
   tctcggacga atcctttaat tttattttca tttgttccgt tcacatgaac atctctcact 30780
   accgctatca acacttttaa tttcttctgt atattatcga gtttgtcgta gagttggttg 30840
   gagtcgggta atgttgtaaa aaagtcaata tttatttgaa tctgcatcac gacactatcg 30900
   agattataca attcgtactg tacactgtcg gcggccaaac aaattaatcc gaaatcaatc 30960
   atgctgtcgt cgacaatttg atcagagttc gactaaacga tggccgtaat gtgatccggt 31020
   tttgattcgc ttgcctttaa tgaaatcaat cttaatttcg ttatcgttac agagtattcg 31080
   agattgggca gcagctcgtt tgatcgaatt taggcgacgg gtcgacagct cgagtggatt 31140
   atatatttct gatttctcca gggtaaacga gttgagatgg ccacaattga accattccag 31200
   tccaacgatt aaattgatca agaaaataat ttcataatca cagtactcga atacgaattt 31260
   attacaaagg cagtaatagt agaatttgag tgaattatat agatagaata ggtagttgcc 31320
   ctgtttcaga taaaattcaa catctgtcac gaataaaatg ttgataattt tttcggaaat 31380
   taaatgcttt agtttttgta gatatatcaa agagtttttg tcctcgtcga gtctgatggc 31440
   acacacgtta tccatgtacg cgttattgat gcaaacaaat tcttgtctct tcaattctct 31500
   tttcagtgca gcacttttat cgttggcaat ttcgatgacg atatattttt gagagctctt 31560
   gtgtctcttt atcacgtccg aaatggatgt aatcgaagcg aatagttttt caaataaatt 31620
   attctcccag cctctggcgc cgacgctggt cttgtcgtta ttgttctgat gtttaaagtc 31680
   cagcccgctc tgaatcagct ttgtggtgat gcgcacgctc aattgttgtc caagagtata 31740
   atgatcttcg tagcctctct cccacatgac gttacaagcg aaagcaatca aatcggacac 31800
   gttttcttta accaaaatct tgtgtaatcg gtcgaaatcg cctttgtacc aacgatcgca 31860
   cgccaacagt ttcagcaatc tcgattgatg ttcggtcagg tcgtttcctc tagtggcatc 31920
   gatatagtgt tcgatgtcgc gaggactcga tttcaacaga tttgtcatgt aaaatacgac 31980
   caacgtctgt tgtatttgca cgaatccctc gtcgttgact tgtaggcgaa gatctgtcgt 32040
   gtgatataga tcaccatcgc agacattgtt gtcgtcgaca tatgccagat aaaaagagat 32100
   ttcatccgct aaaaatttac aacactgagg cagcaactgc gaagttgtag tatagtaacg 32160
   tgcaaacgcc atgccggtca tgaagtaact atctgtcgat tcgatctaat cgctatgatt 32220
   caaatctatt tcaactgacc tagataaaag agggtttaaa tatctcgata aaatgcaatt 32280
   gagattatta aatttgaagc agtgtcctca agacgtttcg aaaatgttta aatttcccaa 32340
   cgaaatgatg gaagaattct tgatgagacc ggtcattcaa caaggatttc aagaactcga 32400
   cattattggc aagataaaga atacagaacc tcaagttttg atggaaattc aacgcctcaa 32460
   ctcgagcggt atatatggca tgttggccgg aggatatgcc gcttttctat tgggcctgac 32520
   cgacacatac actgatgtcg attacttttg tgaagatatc gaaggtgtct atcaatatgt 32580
   catcgctaac aagaatgatt accgagaaca ttcgcatgta ctgagcagac ccgatgccgt 32640
   cactttggtt tcggaaggga taaagtttta tgtggtcaat catgaacatt caaagttgca 32700
   gataatatcg aggcgcatgt ctatgcgcag aagcatcaat tacaaaggat acgccttcta 32760
   tgcagattgc ttacgctctt tcgatttgcc catctgtaaa aaaggaatat ttctatgtcc 32820
   catcgaatgg gtcgggcaaa tcgagtgcat cgaatacaca aacgattcgc tgcgattcat 32880
   catacaaaat tattctccaa ttaaaccatc gggaaaatat gaagcatcgt caaaggcgat 32940
   aacacgcaag gaaaaataca gcgctagaac tgtacaccac ggattgccta ctactctgaa 33000
   agaaatttgt caagacgcaa tactttatca cagcgatcaa gcaaacactc aagattttgt 33060
   catttattga aaatattgta accaaccaaa aatatattag tttgcttctt tattatgttt 33120
   gcttttcatt attgtttgat tttattttgt gactaataat aaacgaaaat taaaaatatg 33180
   tgtatttttt atttgttgta attaagtcga atgacattga gtaaaatgaa aacacaatac 33240
   tcaaactgca ttatttgtaa aggaatggtt tatgtttata aaaaatacac acaatcttta 33300
   tcgtcacaaa tattttttca taattatcga gccatttata ggaaaaacat tggatatcta 33360
   tgtggtaaat gttatcgttt acatgtaata aaaaaataaa aaaattgtaa attaatatga 33420
   aaaaaattta ttaaatcaaa tacatgaaaa acaatttatt aattttcttt tcaatgttgg 33480
   ctgaagtcga tgctttgtcg tcaaaagtca attcggcaat ttttttattt aagcctttaa 33540
   tcaccatctt gagttgcttt tcattttctt ttgccagttt catcacatct tccatagcaa 33600
   gaacgttcac tttcgattga tacgctagtt cgaccaaaat ttcaaatagc gaaagtccat 33660
   gcatatccaa gtcctttaat acatcaaata tcatgtgatc attaaaaaag ttggtatacg 33720
   cttctgttgc cttttcaaaa ctaccatctt cagaatcgtc ttcgtcgaca tagtcattgt 33780
   cgtcatcgtc cgagtcttcg tcgaccgttt cttttatgct cacgtcttct ttatcaacgt 33840
   cgactacttc ttctttgttc ttgttcgagt cgtcgtcgat tgcgtttcgt ttaaaaattt 33900
   tcttgttgcc ggcgaacatg ttctcgtcgt cgtcgtcgta atcgtcatcg ctgctcatgt 33960
   cttcgtatgc ggcggcaatt tctttttgca attctattac gccttctttt ttcagaaaaa 34020
   ctagtttcgt cttcaacgtt tccaacctca gccatatttt atattcttct tcttggacga 34080
   ccgaatagtc aataaattcg acacgtctat tttttaactt tttgaacata tcgttaaatt 34140
   gttgaactaa aatagtccgt tcgttgaacg ttatttttaa ttgttgagac ataatatatt 34200
   tgcgtgcctt taatattata cattccatcc atttcgaggg atgaatattc tctaaatcga 34260
   gatcgcccaa taaaagttcg ttagagtgct ttgaattcat ctttactatt gacttgttta 34320
   cgcggtcaaa agatccagaa gactgtgata ttgttcggct cgaactcctc ttttatatca 34380
   tttctttatc gtatcatagt attattgcag tgcaataagc tgcccatgac tctgaattct 34440
   ccgctattat cgggcagatt agagctaccg cggacacatt gcaggctttc ggacaggcga 34500
   ttacccacaa acgattgatt gatagtccac tgatctaatc gtgttccttc gactttttcg 34560
   tgtcccgtat aattactttt gataaattct ttaaaaatat tatctggagt attattgaac 34620
   agcatatagg gtatattaaa gattggatat cgcttggcct cgagatcgtg gtgattaccg 34680
   ccgcggacga catatttagt ttctatatca ttaaaattcg actgtctcga caagaaagat 34740
   atgggcgata gacatatctg cgcgctagtt ccgtcttctg ccatccattc tgtcaaatct 34800
   tcggaaccgt tcatgactcc tttttgtccg tgtataccac agattttgat gccttgcagg 34860
   tcacttgtcg acgtgatcat ggtgacttta acgtacaccg tgtcgttgtg tacggccata 34920
   gtagagtcta aacactcgat cctttgattg aatatctctc gaaagtaaat gtaaattttc 34980
   gatagaaaaa aatttttatt tttacatgcc tcgattttat agcgtttacc atcatatatc 35040
   cagccgattt ttacattcga gacgacaacg cctgccatat ataaaaaatt tcctccttca 35100
   acttgtacaa aattgttttc tttactttta aaatatttta taaccggtgt ctcagtcttt 35160
   gaatgattga gttttccctt taatttgttg attttgttgt tgtacagtct gatcggtaaa 35220
   ttgatgtcgg gaatatatgg atcttcggca gtcatcagtt tattgtctcg aaccagcgtc 35280
   cacagtttga acattttatt attcacttgt attcgcggac tgacgaccac agaattacct 35340
   acgggcaaag tcttcaaaaa aacatcggaa tcgagctcct cgtcgtagca gctcacgggc 35400
   atcgcgtttt tcaaattcgt cagcgaaact atgagttttg gtacgggaat cgtgctgaag 35460
   attcgtgtat agccaaagta ataatattgc accagctttg acatgaggct agtgacgtca 35520
   tcgttttcct ctatcttacc gatttttggg tatttcttca agatcgaata agaattatga 35580
   tattcgaaag gcgtcagcag agtcgtaatt tttttgcccg cgatactaat cactcgcttg 35640
   atacacacca tgccttcgtg gtgatttaca aagagaatgc ggttggccag cagcttgagt 35700
   tcaatgggac tgtattgccg tttgagttga taaaaaatca attgtaaatc gtcaaaacgg 35760
   caggcgtata tggtcggtcg gtcgttgaac gcgattctaa tgtttttttc atcgacattg 35820
   gagggatcgt tggcgtaaat taatccgttg gtcagcaata acttgaattt gtccgcgact 35880
   aactggtaat cgacggcggg aagacgtacg tttcggcaga gaaaaaattt tttaccggcc 35940
   actgtcattt cgccatgaaa aaaactatcg acaaacttga caaaatcccg ttgttgcatg 36000
   agcatgtctt ggcgcatcgt gtcgttagtg attcgcacga cttcgttgcc gattcgatat 36060
   ttaagcggag gaggattaat ctcaatgttg ttgttgctgc tgttgtcttg ataattaatg 36120
   aaattcttct tttgcttgct aaaagtcttc gagacggaat agattaattt gccgttgacg 36180
   atggtgtcca caactttttt acattctttg acaaatagaa tagtttgtag ttttcttcgc 36240
   tttatactgc cagttttatt gatatcgcca tcgtttttat tgtcgataaa ttcttcattt 36300
   ttcgttctaa acgcttgagt gttgtttctt tttcgtttgt tattgctttt attttcgccc 36360
   aaatcgttgc catcgtcatc gccgccgctg ccgtttactt tatttttttt ctgtttttcg 36420
   tcttttgatt tttgtctagc aatagagtcg ttatcgtcgt cgctgacatt gacacaattt 36480
   ttgtcgtcga ttttcatcat attcgtgtat tctttgagaa tgggcgcata catcaaatcg 36540
   agtaaataat cgtgtttata tatgatttta ttagccaaac tgtcgatggt gtaattgatg 36600
   tcggatttca ttatattttt aatatcctca actaaatgat tgctctggtc cgtattaaaa 36660
   tcgaatataa aatttaaagg ttcccattta ccgctgcgtt tcagatacat ttcgagggcc 36720
   tcgttcagct ccatcgtaac aatatagtct cgggcgaata cgtctcgggc gaaaagcaca 36780
   ttatcgtctt tgtcgtatac cagttgaatg gcgcgattga tctttttttc cttgtcaacg 36840
   ttgccgtaca ggaacatgcg tttgcagttt ttggaatatt gcttgtcgta gaaattatgc 36900
   agtagaatat tgttgttcat caggatgttc ggaaaactca agtttcttcc gtcgatcatg 36960
   aaagtgccgt tcatattgtt gattttatac gtatccttgt ggcgaaattt gaggtcgaga 37020
   cgagttccaa aaatcaccaa tacacatttg tgtaggacgc atcttccttg tacatcgacg 37080
   gcacagcaaa aataagatct acgctcttgt aaatacttta ctgtacattc gttttcagat 37140
   ttattattac agtttagata gaattcaaaa ttatatttat ttttgagttt atcgtagaga 37200
   ttattgaaat cgacaatcac gtccgtcatc ttgaaactat cggtgatcac taatgatgtc 37260
   gacaacagcc atggatatca ggcctacagc ttctattcga cccgtgactc gtgcgtttaa 37320
   acgtaaaatg gatcaaaaaa ctaaaagata tttagaatcg gaagcgatcg aggatgatga 37380
   ttatgatttg tcgtcgacgc caaaagacga aaatttaatg tcatcgacga catctttgtc 37440
   gccgccaccg gcgaaacaag ctaaaataaa acgaaaaaat ttatttaaaa taaatttgga 37500
   cacgcagagt ttttacgatt tgctttcgct aaaaaccgat tcatccgact ttcaaatcaa 37560
   gcaacgcgta aacgaactaa tcgactttta ttcaacggca aaagtcgacg acagtagttt 37620
   tcgagacgat gacgataaag ttgaggtcat caattctaca aatcaaattt tattaaaaat 37680
   ttctcaagct tccatgatac tgaccaacgt ccgcgccaaa cgcgtctacg acaagtatat 37740
   cgagatcaaa tacaagatta gattgtggca actcaacgaa ttacgaccgt taatcgatga 37800
   gattttcaag catatcgagt cgatattcac cgtctcgagt gattttcaag agtttgatga 37860
   tatagaaccg gcactcagaa actttgtagc caacgagctt actcagcgac gaagtcgagg 37920
   caaacgcctg cgtagcacca ccaccaaccg tcttcgcata caatggaatg tcaatctcaa 37980
   cgatgagaca aacgccggtg tcgacgaacg gtatttactg gattatttcg gtaaatatgg 38040
   tgagattgtc gggatcgtca tgtgcagttc taggcccggt tgtgcggtca tcgaatttga 38100
   tacgcttcgt tcggtcaccg aagtcatcag cgaagagagt caactcaaac gatttattgt 38160
   tcaggatctc tccgaggcgg aattgatcag tagcgatagt gctgctcaac tacaaagtaa 38220
   actagacgaa ctcgaacgtc tatccagtga tttactcagt atacaagaga atatcaacaa 38280
   acaagcgtct taaacacgca atttcgttgt caatttcgat aacaaataaa acaaaaggca 38340
   taactttaat agtattttat tttaaccaaa ataaattttg attacattag taaaagagtt 38400
   tctacatatc ggacaatcgg gtacgctcca agcgcactct atacaagcaa cggcatggcc 38460
   gcaaggaata tagcaggcgt caatctctct gctgaaacaa attttacact cggacttaaa 38520
   cgatgactct tgaacggttt cgtttgaaga cgtgctggcg atttcctgag agatcgcagg 38580
   cggcgtcggt cttggctttg gtattggctt cggtgccggt ataacacacg attcactgat 38640
   tacttgctgc acataatcgg gtcccttgac gaacagtaca tagcgacagt cgggatacca 38700
   tcgggcgtgt tgttgccaag gcaccctatc gcaactccaa tcggataggg tacaaccgtc 38760
   gtagaaacat tttactttgt cgccgatgcc tgtgtaaaag aagcccgcct ccgctaaatc 38820
   tatatagtcg gtgcggttgc gaggccagtg acgaaagctg ttgattcgct taactacatt 38880
   atcgaaatta ggatatttaa aaataaactg attctctcgt tcatatgtat cgtacttggc 38940
   ccgtttcgca tctagttttt ttacgaacgg acactgagga gcatattttt tgtgatctcg 39000
   atcgaccaca tcgttttgtt gccatttaca aatctccact ttacagtagg cacatcggac 39060
   ttgatctcca ctacctaaat aataaaatcc gttcatcgcc aactctatcg gcggaacata 39120
   gggattaatc cacttttcac tatcgaaagt cttaagtctg ttgctacagc gctgcatggt 39180
   ttgctcaaaa ctctccatct tcgttgccgt agtgttcgtg gcttcgctta caaagtataa 39240
   atactcagag ataacgaccg tgcgcgtatt atatagctgt gatacgagag cgtgttatct 39300
   aatcatttta actacgcagt tgcttctcaa cgaatcatga ggataagtaa acgacaaaaa 39360
   tgatgatatt tttttattat atagctttgc cacctacgcc cgcgacaatg cctactatta 39420
   ttttatcgcc tttacaaagg aaaaaacgat ttaataaaag acaaaagact cgttttatac 39480
   gtatatttaa atcaaaaaaa attacaaaca aatctctacc gagattaaga gttttttaac 39540
   tgtttgccgt tacgattcta aacaatggaa agaaacatcg cttacagaaa agcttaaaac 39600
   taaaacaata aaaatataca cttccagtga ggcgacaagg cttgtcgcac tgtaaacatt 39660
   tctcataatg aattttatat aattttatct tggactcgta actgtaccaa gcgaaacgaa 39720
   ttctctcgcg ccagacattt ttgtctacaa tgatactatt attctcaaca aaatcaacat 39780
   catcatcatc aacatcatca ccataacttt ttttgttttt gtcgtagcca ccaatgttta 39840
   tagattcata atagcaaaca ctaatcacaa tatagtttat gagctcttca ttttcgctac 39900
   tgtcgtccaa aggaaataag cattgagcgc agcacagatg cgattcttta ttgtttagag 39960
   taaaacagaa tttacaaaaa aaattattgt acaatattcg acaattgtct tcgacactag 40020
   aatcgctacc gccgctgtcg cttcttttga ttgtattcat caccaatgtc tttttaattt 40080
   cacaccacca acgacggtac actagttctt ttcgtacagt atcgttagcc gttcgataca 40140
   aacgagaata tttgataaag ggtttaataa gctccatgat tcatgctcga atgcgagcca 40200
   aagtcgaata ttaacgtctt tatactacag acaaaacatg ttgataaccg tcaatcttca 40260
   aaacaaagac gaatatattt ataaaacatt caaaaggatg tggaacgaat acaaaatcga 40320
   atgccaaatt tgtttcgatt gtatagaaaa cgatggcgtg gttgccgtca ccgaatatcg 40380
   aacgttaaat ttagaaaaaa tgtttcatca atcgtgccta aagcgctggc aacgagaaca 40440
   caatcgggat ccttttaata gaaacgtcaa gtgttggttc gattttccac caaagtcttt 40500
   ggagcagtgt tcggcgcttc tcgagcatat gaaaggattc atcggagacc aacgtcccga 40560
   caaagactac gccgacgagt ataatcgcat cgtaaaagag gaatctgtca tcgatattga 40620
   actggatttc gaaaaacttt tgagttactc ttgacggagg acaagacggt ttactcgaaa 40680
   acaagataat tgtaaacgtt tttgacgtga atatcgatgt ctatgactcg ataatacttg 40740
   tttgtcgcgg cgacagatgt aaattggttc acatcattgt ccggtgtatg tttatagaat 40800
   attatagata gcagatccaa taaacgatag ttgtacatgt ctgttagttt tctttgataa 40860
   ttgtctatgt gatttcgata ttcgtcggga tgaatataat catagggcaa tcgagactct 40920
   ttatattcga gcagatctgt tatgttgctc acgatgtaat tgacgcacaa attgaaaaaa 40980
   ttcgtaatgc cgtgcgtgat tttagtcttg ccacgagtgt agttgacatt cacgatgacg 41040
   tgagctacgg tcgacacgtt atcgaatatt ctcacataat aattgaacaa ggcttcgcta 41100
   aatttataaa aattcttacg actcacatct ttgtcgctca atatggcatt cattatggtg 41160
   gcataatcga taacattctc gatattgtct tcgaaagtta aaaattgttt aagcgtattc 41220
   gacactcgag aattgatgtt attttgaagc gcatccaaat aaagtttaat gctgtcgata 41280
   cttttttgga tacacagcat gttgttacaa tcgctgggta tttgatttag tagaggcgga 41340
   gtgatgaaaa ccactacggg agatgagttc gatccttcgg ttctttgtct tttattcata 41400
   tcgatgacac tcgagaccgc cgtcgttagc gacgccgccg gcaaagtcgt gggatcctgc 41460
   gaaagctgta tcgttcgaga catttcattt tgacgtctca ttatatgttt ttccgcctct 41520
   tccatttgac gaatatgttg aattctctgt tgctcttcat cgatcatcat cgatcgatcg 41580
   atatccattt ccatttcggc ctctacagag ctcgttgccg tagatattgc cgtcgccggc 41640
   ggcggtttcg tggtagattt taattttttc ttagcgcttc catcggtctt tgaagacttt 41700
   ctaaatccta atattgatga cgacgatgaa gacgatgaag gcggaggtga taatggcgtt 41760
   tccgacgctc ttttactcga gcgccgatcc atttcggtac gaagacgagc agtagtttat 41820
   caatagacca aaaacaaata gtcgggataa tcgtttaaag tgactataga cctcgaactg 41880
   gacacttctg ttcgtgtgta cttatcatag taaaaatatt tattatattc cgaatcgaat 41940
   ttcacgaatt taaatcgcga aagctggtga tattcatcgt aagacagtaa ttttatcttc 42000
   ttacgttcaa aaatgtctgc atctccattc gaagcggacg taatcgacat cattttgaaa 42060
   aataacctcg agctcgtcga caacacttat ataattttaa atgtcatcga tggcaagcgc 42120
   ggtggttcga ttaaaccagt gtgtctcgga gagattaact cctttcaaac ccatcaagat 42180
   tcaaagtgct caatgtccga ttcatccgtt acgagcgaac tgcagagcga tcgtacgcta 42240
   taatgatgtg gtcgaaagca agttcattaa tcacattacg attttcgaat cgttatatca 42300
   aaattatgac cgacgtccgt actacatgtg cctgctgaat cctgctttcg agatcgacga 42360
   aagaggcgaa tatctgaacg ccaacgaaat gtacgcctat gttcatctgc agcccatcga 42420
   cgacgaagaa gtcttctacg gaatcgacga agccggagag agatctatgg ccacactaaa 42480
   aaccgcaatt aaagcaatta tggactgttt cgatgtttgt cgagacaaat acattttaat 42540
   ggtggacgaa ctccaaattg atgtggttta ctcgatattc cgaactatcg tattaccgca 42600
   gcgaatggtg tttatttacc agacagagca cgcgcccagc aataatgatg ttcaaatatt 42660
   tagtgtgccg caaactgtcg aatcatggga ttcgcatatt atttacaaat cgtttctact 42720
   ctacaacacg atactcacaa tgatgctcaa acaaaagaac cccttcaaca ataacaagaa 42780
   aaatatttct gtcgtttttc gtaatctagg caagtgtcct aataataaag acagagtcaa 42840
   gtgttgcgac ctaaactatg gaggaaatcc acctggacat attatgtgtc ctccgcgtga 42900
   aatggtcaaa agaattttcc attattccaa atgggccaaa actccgaaca attatcgaag 42960
   gtatttcgag ttgattacaa caccacccat caaccgcaga atgtacgagc cggacggaca 43020
   agtcaatttt aattacaaca cgggaatgct aataatactc gactggtata attttatcga 43080
   agagtttcgc gcatattttg gcatcaagta attatgcgca gatcactagt atttattgag 43140
   acacgttatc agacgatgct tactcttaaa aagtgtgtca tcgcagtgtg ttatcttatc 43200
   acagattccg aacaagtcct atcgaaatga ataaggtcaa ttttcaactt aagaaagtaa 43260
   tcaacaatat tgttgataca aaaatgaata aggaccagtc ctcttcgggc tactcgtcat 43320
   cacgatcatt atcgtcggcg gctacatcga atcagcagaa tgctgcagat ttctatacca 43380
   aacataagtc ggatgtggcg tacgtcggtc gcaacaccac ctacaatgtc gtcggccagc 43440
   gaaactatgg aaaatatttt gatgaaaaaa cctacaaatt ttaaaatcac tggagggacg 43500
   ggtcttatgc gttgcgtcga cgaaaatgac caaattgtat actgtttaaa ctgtaagttt 43560
   gtagcaccaa tgtctgtgag ctatgaacat tttatttggc tccatgaaaa atacaactgt 43620
   atggtgaaca atcgaggacc atgcgcctat acaatcagca ccagcaacca cgatataccc 43680
   accgccgata ctccgaatct aatcaaacta gtcgataaag attctacccg ataattgtgt 43740
   tcatttcaaa tctaaaatta tgtgtattat agagaatttt aataataaag atccaagaaa 43800
   attttaaaat tttgctttat ttattttctg cgaaattttg ttctgcgcat gtgccaagtc 43860
   tagtttagta aatatacggc catataaata gagtaaatgt cgacgacggc gcgttagtcg 43920
   tttgctcgat tattaaaatg acactgtcga cgtctttcag tgctcgtttc ttagagtttg 43980
   aacaagtttg tatcgacttg agatatgtaa cttttatcga tgacatatca tgcgacaata 44040
   tcaacaattg tgcccacgat tatataatat ttttaaatgt aaagaaggca atgttttgca 44100
   attttaatat aactacagac atgtctctgg agactttggc cgaatatatt tacgataatg 44160
   ttcgggtcac catcgacgga cgtttgcacc gtaagcattc gattatggcc gagtgcgtgc 44220
   gcttcaacgg agcggacaac aacaattcca tattcatcaa tatacatgcc gaagctcgag 44280
   ttattgtggc taaaaaactg ttcgacaacg aacgatacca tcaacgcatc agtggctata 44340
   tagatttcga gaatcgatcg gatccaaacg acaaaaaaaa attattgact gacagggaac 44400
   gagctctact ggatcgtgaa tacgaaatca aaatgctcgg atcgacttga atcatttgtc 44460
   gtcacggcta actaacgatt acgagacatg atttttgcct tgtcgtcgtc gtcgccgccc 44520
   tcgtcttcgt ggtaataatc atcatcgtcg cggttatcgt cggtgtcgta atctgtattg 44580
   tcttcggtgt cgtcttcgat gtcagtataa tccgagtctt gtgcttgttg tgtgaatgga 44640
   acacgatcgt cctcttttat gtttctattg tagaaaaata atttttgttt tgccgatgac 44700
   gacgaagatg ggcgctcggc tgcaacgaca ttgccatcat tttcgtcgtc gtcgtcgctt 44760
   tcgaccatat cgtgccggac aatctcttcg gcggcggtct ttggtatcca acggtcgttg 44820
   agcttgacgt agtctcgctc gacggcctgt cgtgccatat gtaaagcgac atcttcatcg 44880
   ccgccgtcga gtttatggta ttttttaaag gtctttacaa ataattgttt agctcgcgcc 44940
   ggcatttctt ctctaaaaaa ggcctcgttc aaatgaaaca ttttaaaaat acttatctaa 45000
   tctattcggt atcatagtca gaagcggatt cggattcgct ttcgcttgta gatgaagtgt 45060
   cggtggtgtc gtaatcgttg gcgttctttc gagccaccca ctcgtcatct ttattgtgaa 45120
   catatttacg tttgaccgcc tgccaggcga ttttgaagga tatcggttca gattttgttt 45180
   ttgtgtatgc acgattaaaa aattttagga atattctttt accgttgtac ggtagactgt 45240
   atgtcgaagg tggaagttct gttatattat tgtacattat gatgtaaata aaaatttttt 45300
   tgatcgaaat gtttttttat ttgtttaaca cagtgtacac aacacagata catatataca 45360
   aaacatttaa acttaattaa tatctaaacc tgaagtaaaa tttaaactaa caacaatata 45420
   atttgactat gaatagaatg agatataaac tagattcaag tagaattcga tttaaatcaa 45480
   agtcaaatta taatgaaatt gaagtgagat tcaactgtaa aactcattta cgataaaatg 45540
   cgcaatgagc ccattaaatt tgcgtttctt cgtttaatat tagagtctgg ggtaaagacg 45600
   taataacgct atcatcgtag ccggcgttat agttgacgat agagtcatta ttgttaccgt 45660
   taacgataat ggatgaagtg gatagaggag gagacgatag acttgtttgt gtcaacagat 45720
   cataaatatc attttcgttc tttactatat aaattttgct acgttcgttt tgtctcacca 45780
   tgacgccttt tttacacaac gaaacgtact tgtaatgcgg caacagggcg tctcgtgttt 45840
   ttttcaataa ttgtttatgc tcgggcgatg cggccacgaa aatcttgaca ggggcatcga 45900
   aatcaatatc aagatcataa tttttcagtc gaacttcacg cgaacgattt tgccactcgc 45960
   gagccgtcgc ggcatcgttc aactttaatt taatgtgatt tttttcgtaa tcgctgttca 46020
   cgatgagttt atagtttagc gttaggagat ggcaaatttt tttaacgtaa tttgtacgaa 46080
   tctttttatt gtacagtctt ttatcgtaaa ttccatagat ttcgactgta tcgttaagat 46140
   tttcttcctc gagacgcttc agtttattaa caatattatc agtgacgttg cgttcaattt 46200
   catttttaat tagattcttt aaaattggaa tattaattag ctcggccatg ttagtttacg 46260
   tcaaacgtaa aaatttaaat taaattccct cttattacta gacaaaaagt cgatttattt 46320
   cacaatgagc tcgtctacag caagcacgtc catcgaattt atcccgaaaa aaccaataaa 46380
   ttacgattta ttgatcgatc cggccgacat cgacaatgtg tatttctgta agattaacga 46440
   ttttcgcgtg tttctcaaga acattatagc tgacgttaag aaaatcaaga ttaatttctt 46500
   taatagtcta atcgaacagc taatttccgt gtatcaagaa tgcgaatcgc gaaactcgca 46560
   caccgaaatg ctgagcagaa tgctcatggc cagcaccatc attgtcaccg atttgccgtc 46620
   caatgtatat ttgaaaaaat tgaaacgcaa caaattcacc gacaccatcg actatttgac 46680
   tctgcccaat ttcatactgt gggatcacaa ctttataatt ttcctgaaca aagccttcaa 46740
   ttcgaaacac gacaacggac tcgtggacat cagcggatcg attcaaaaga ttaaactgac 46800
   ccacggcatc ataaaggatc agtttcagag taaaaacggt tacgcaggcc agtttttata 46860
   ctcgactttc ttgaatacgg catctttcta tgcaaacgtc caatgtttga atggagccaa 46920
   cgaaatcata ccgccgaaga gcagcatcaa acggtattat ggacgggacg tgagcaacgt 46980
   acgagcctgg acgacgagac atcctaatat atctcagcta agcacccaag tgtcggacgt 47040
   tcgccagccc gacgactata acgactggaa cgttaaagtc ggtttgggca tcttcacggg 47100
   cgccaatacc gattgtgatg gagacaaaaa agttatcacg tttctacctc aacccaattc 47160
   gctcatcgat ctggaatgtt tattatatgg agacccacga tacaacttta tttgtttcga 47220
   caaaaacaga ttatcgtttg tgtcacaaca aatctactat ctataccgca aacagaataa 47280
   tatcaaagcc attttcaata ccatgcctat attaaaacgt ctgtggaagt cgtataaagg 47340
   tattgtattt tcacagaaat tggatccgtt gctaagagat tgtgcgctcg ttttgagttc 47400
   aaacgctagt tatttactat tcaataaact gacagagtta atatccgacg aagagatgat 47460
   ttgcggtgac gaagaaattc ataatctaca agggcaattt acggatatta ttaaatgtgg 47520
   cgcaaaaggc agtaaagatt taataaatag cactcgaacc tacaaaaata ccaaaataac 47580
   cgacattgaa gaagtcgcag gcagagcaat aaagagtcta aataactaca tttcttcgca 47640
   taatcgagtc aaagtcggag gcggagatat ctatcacaat accaccgttt tacagaatgt 47700
   ctacatgaaa aacgatcaca tttgctacaa aaacgattct gtcaagttgg ctagcgtctg 47760
   cttattaccc tcggaatttc tatttccgga acatttgttg gatctctttt tgtaatatat 47820
   ttttgtcgat aataaaaaag atgatgcaat acgattgtcg tttcattata attacaaaac 47880
   atgatgcaat attgggcaat atcaacatca tcatcattat tcatgcgacg cgtgcgtcaa 47940
   tcgatgaccc cggatgatcg tcgacctctc gatcgttatc gattcgggtc acgcgcgcgc 48000
   gcgacaaaca tccggctgac gcaacattgc atcatgtttt cgtcgacgcg aaaaacgtgc 48060
   tcgacggctc ggacgatcgc ctacggtcga tccgcgatcg aacgatcgcg cgtcgccgcg 48120
   ggacgatcgt ggatggagtc gacgatgatc gattctcgga cgatcgaatt aaattttacg 48180
   cttgtgtcag cgttaaaaac cataattttg tagttattat caccaatatc aagtaaaatg 48240
   tctcgtacgt tgttaacata caagtttcaa aatttttgca gatttttaca aacttttaca 48300
   gttacaacta tcgcgataat atccacagtt tcaatgttaa aaactccaat ttagttagaa 48360
   acgaccggaa cgtgagctcg gtaaaattat aaaactaaaa actacaaatt tttcaaaaaa 48420
   tttttgcatc aagttttatt atatccctaa cattgaagcc aaaaactact agattgatgt 48480
   caaaaacttt aatgttatct aaaacgaccg ggacgtcaaa aaataaaatt tctaaatttt 48540
   ttaacagatt tttctaaaat ttttatccta cgtctaagac aaaatactcg ttttcgtcgt 48600
   taaaagcatc gacattgtcg aaatcgtccg cgacaaaagc ttcgtttggc gtacgggcta 48660
   ttcatgatcg atcaatcatg catcgacccg agacaaacgt cccgtacgat ttcgatgata 48720
   ttgaagtcac gaggtgttag agtaacgtac gggctattca tgatcgttca atcattgatc 48780
   gactcggcac cgatgttatc ggaatcgtca cgaacgattg gcatatttga cataatcatt 48840
   ggctttgata ttgagtcgat tcgaacgaca aaaaaaacag aataaaataa acaaacaaaa 48900
   tccatcgtca ctggcactat ttaaaccgct ttagcgcgac attcgttagt atctcgttat 48960
   ctttcgtgtc aactatacac atcaaagaaa aatgtctctc tcaaaaataa atcatgtcgc 49020
   tgacaacgaa atgcctgccg ctaagagatt caaagcgtat gataatcaat ccgaagaatc 49080
   gtgtcatgaa aaaaataacc attccgacgt tagtgccgac ggtaaaatta aacttagatt 49140
   tggcaacgaa gacattgcag ttgtcacgat ggtcgatgac gacggccaat tgtggatgtt 49200
   ggcgaatccg tttgccagaa ttttggagta cagtaatgct ccgaaagcga tttcaacgtt 49260
   cgtcagcgat aaaaatcaat tgtgtttcga aaatcttaag tcttcccaaa gcaggcagac 49320
   ttgtatgacg tcatcacttc atccgaagac taaattcatc aacaaagcgg gtttgttcga 49380
   gctgattcaa aactccaaga tgccacaggc tcaagaattt aagcaatgga tcaactcgga 49440
   cctgctgcct acactgtgtc agcaaagaga atacagcccg cataaaaatt accaaaacat 49500
   gtctctaaca aaaattaatt ttgtagacga taaagaagtt gagacgtata cgattgatgt 49560
   cgatggtgaa aaatggatga ctgccaaccc gtttgctaaa gcgttaaatt atagcttacc 49620
   acatattgca atttctaagt ttgtgactaa tgaaaatcag aaaacttatg aagaaattaa 49680
   cccgatacga ttcacatcga ccgatgactc atccgttcta cctcggaaca ttcaggccaa 49740
   gaccaagttt atcaatcaag cgggcgtctt cgagttgatc aacgctagca caatgcctgc 49800
   cgcgaagcga ttcaaagcgt ggaacaccaa cgatctgctg cccactcttt gtcaacaagg 49860
   agaatacagc atgactgcag acgcgcccgt cgaaatacaa gagggaatga atgctgttca 49920
   cgcagccact aacgaaggcc gagaagctcc gtggatggag gacttgcaca aactaagaaa 49980
   ctccgtagta cagaaagaca aaataattga agtcatatca tacgagaaca aagaactctc 50040
   cgtgtcgttg cgcacttcga acgaaaaatt gcaagatgct aacgacaaac taatgtactt 50100
   tgccagcgcc ttggtagact ccaacaatgg attaatgaaa gccaacgaac ggattgaaaa 50160
   ccttgccaat cgcatggcag acatcgccca ggacgtgata gccaagccga gtgaccctca 50220
   actattgcat tcgctagctg tgtgcgcctt gggcgaaggc caatatgcct ttgtgagacc 50280
   tcaaaagcgt agtctgaaac gtagcttgga ccggctttcc atcgacgaga gtcaaatttt 50340
   atttaaaagt aactacgtac ccaatgcgat gaacgtactg aacaaagtca aagagagtct 50400
   gcctaaagac aagtttacag ctcgtcacaa caagattacg ttgctagaag atttgactag 50460
   agaggatcta gttgaagcga ttaactcgtc gatgaccgag cgacaggtgg ccattttcgc 50520
   caacaaagcg tcgggcagaa aataaaatgt gatcaatcaa taaaaatctt tttttatata 50580
   ttttacatgt tgactattat tctaatccct atgtgcgttg aatagttttt aatgtttgtt 50640
   gagcagtatt ccaatccttg cgaccattca tgatcgttta atcatgaatc gccacgacac 50700
   caacgttacc gtttttgtca ataccaatga tagattttaa tagaaaaatt gtggtcatgt 50760
   tttaatacaa taataatata tgttaagatc actctagtct aattaagagg ttcgaattaa 50820
   acaacaattg aaaatgttca tgtactttac ctttctagta ctaattatat tgggattcat 50880
   ttttgataaa aatgaaggat tctctaatct attactcttt atgctgctgc tgtttgtcat 50940
   atttataatg ttactgcaaa tctactacat aaagccagag tctacgactg ccgatctacc 51000
   aaccggtaaa gctaagagta taaaaaagaa aagagattta gaaaaagcgt ttgacgcaat 51060
   tataaataag aacactagtt ctctagattg aaacaaaatg gacttcttta aaaattttgt 51120
   caatcaaatg gtcacatcga tgccgatcgt gacaaaagtg gcctacgtca atgtgcacat 51180
   caaaaactac atgaaggagc tggaacggga cgacacgttt cgccataaat ttatcatgat 51240
   cctacaaatg tttatcaatc gacagatcac agtcgaagac atgtgtcaaa taatggatgc 51300
   cgtcgatggt ttgaaactga ctagacagca gattgaatac ttggtgaatc gagtctacta 51360
   caacgaacat attatattga ttcttcagag ttatgttcgc aatcagcact tggacgatga 51420
   ccagattgac gatttagcac atttcctagt tcacgaaata aataatgcga ttatatacca 51480
   aaaataaaaa ctttattcat caaaacaaat caacaaattt tattacattc cccacacaaa 51540
   ttgttaggat cataacgaaa aacgtcaacg tcagctgaca cgacgacagg tctcttggtc 51600
   tttgtcgatt tcggcggcgg acgctttttg tatagagaaa actgatactg atagttgacg 51660
   acgatcgatt catcttcgct ttcgtcgtcg ttagagtcgt cactgtcgtt atcgtcatct 51720
   tcgatggcgt cggtagattt ttttgttgtt tttttggcag catgtttgac gggcttcaat 51780
   tgaggaaatt taacatcgtg ctgttgattc tgcatgtaat gctgacagat tctaataaaa 51840
   taatactcaa actcggggcg atcgccaaat atctgaatca gctcgttcat aaacgtacac 51900
   aagatgccca tatgcttgag ccaactcata cgcttggtct gatcgaaaag cttgagagga 51960
   aaggcctttt gagtcacagt gccgacgaca tcggtcagta agtaggggat tctgtctccg 52020
   ttgccgggta gaaaatcagt tccgcaatta gccaaaatct ctcgacagtg cttggcgatg 52080
   gtgatgggtc gcttgcgctc tggcgcgccc ggagccaatt ctttcttctt tttgtttgta 52140
   gtgttttcgt tataactcat gctgaagcca taatcgctat agttatcgtc ctttccgaaa 52200
   ttgtcgtaat gagcgatcat gagctcggtg agcagattga gaccgcatgc cacgctatga 52260
   ttgcgcagat acatgtcgac gatctgtcta aaagacttgc gcataaacag aggcatatcc 52320
   ttttttatca gccacccctt gtatttgatg cgattttcgc tattcagata acaatatttc 52380
   ttctttttca atagaatcag actggtcatt atgttctcta gagccatttt atatccgacc 52440
   caatcggcat tcagtttatt gagcacataa ttgtttataa ttttaccaat tgtctcatag 52500
   acattttctt taatttcatt ctcatcgaag gctacgtgaa taaacgaaga gtccgtgtcg 52560
   ccatagatca ctttgaacat aatcgatgac agcttgaaat cgctcaatat tctaggatcg 52620
   tcgctcatac tttgaatctt tttgatggcc tccatcaatt tttcacgtcc gatttttgtc 52680
   acataattgg ctaaaggttt aaagaagata ccgaaatagc cgtagatgct gttggcgatt 52740
   cgctttacag cattctgagt tttatcatat aaattatact gaaatgtgcc cacatcgaac 52800
   ttgtctcgct tcgttttata tagaattcgc aggtccaaga gctgtctcaa aagcttggga 52860
   ttgatcgcat tttgattctt cactagatac acgtaattat cgtgtccgat gaacgtgttc 52920
   gataggcaaa ttccctcttg aatcattata gttaggtaga gtgaattaaa gtctagagtg 52980
   gcgacccaac gtttcagtcc cggttgaggc gaaagaactt taccgccctt gtagatacat 53040
   ttttgttttt ggtgacacag acgaacggca ttttgaggta tttcttcgat tggacttctc 53100
   tgcagcagag tcaagtcgac catttgcgag tctacggctt caatgttgcc ttcactagag 53160
   ccaccctccc tctgtttcca tgtgcgtttg cgcccagacg ttaccgatag atcggcttta 53220
   ttaaaaaaat acgcatcagg tatttcgcgc ttcaatcgat catcgtaatg agtgttgttc 53280
   aaagctcgat aaaaaaacac tacactcact ttgtgtgaaa tgttacgaag aacgtcatct 53340
   gtgctcagat agagcaacat gcacagcgta tacataaagt ccataatttc tagcttgaga 53400
   aagatatcga tgggcagcac gctgtcctgt acgttgtact cgatcagttt cttgaggcaa 53460
   ttgttattat agagatcgag catttcggca atgggcaaat ccaccttttt ttgattcaaa 53520
   taatgctcgg acacagtgtt caattgaaaa ttttccacat tattctgatc agaatctgta 53580
   ctcaaaaatt ggtatagatc gacatgagta tagtacgaaa aaaaatgagt attcatacga 53640
   ttaccgaatt tatcgcataa tattgacgtg tgaatactaa tcggctccag atcgtagcgg 53700
   acgatcttgg tccacgattc cgatgtggtc aatcccacgc tatagctgcg gctgtgttgt 53760
   ctgctcgaca gaattctagc gcgattgatg ataaaaggca aatcaaattt gtctccgttg 53820
   taatcgagaa gacaatccat attaatggcg ggcagcagtt taaaaaaagc ctttagcata 53880
   tttatttcgt ttttgaagcg aacacacatg atttccgcag acgggtcctt gtcgacattt 53940
   tgcatgtcgt tgtcgttatt ttcaatgtaa tagaggcaaa ttttcgtgtt tttgtcgtcg 54000
   cgcttaacga caattgatat cgaaatgatg aaattttctt cgggcgtaga aaatctttgg 54060
   ccgtttgaat gtgtttctat atcgtaacac gcaatgaccg ggacaatctc acgagttaac 54120
   gagtcgggcg acacgatttc gagtagttct tcgatgttgt cgatgttatt gtaggagcat 54180
   tgcaaacgat ttccgataca tcttagcgtt ttcttgaatc gaacgtattg cccttcttta 54240
   agttttgttt gcatatgaac acgattgatg tcgtttagaa acgaatctag acaatacttg 54300
   tcaaactgtt cattttctct acagcgcttg tatttaatca cgttaatgcg ttcgctgctc 54360
   actcctcgca aacccggcat caccatcgtc ttatagcttt cgcagttgtt gtagcacttg 54420
   gaaaaggcgt gattgttata gcattttcga taggaaaaca aatcacacat ggtctccatg 54480
   taaaattgat aagaccgatt gttgttgtgg ccagtgaaaa aaactatcaa acggttatta 54540
   ttgtacatca ttcgtgttat acggtacacg tccgagggaa agatcttggc atttccttgt 54600
   agccacgtac ttctgacagc actttctagt tcctcgtact tcaaaaggct caacatcatg 54660
   aacaggtatc gaccaaagta taaaaacact gaagtcaacg ctgacactgt gcaaaattta 54720
   ttgagaacaa tcaacactat gagtcagcgg tgcaaaactc agtcgtcgac tgatgatgtt 54780
   ctgcagcgag ttcgctctat tatactattg catcgacccg atctgatatc gcgtgctgat 54840
   cttcagatac cggagctcgt caccgaggct ctgataccgt cgtctaatca gattacacac 54900
   aatttcaact acaagtacga ttataatagt aacgtgccaa atcctccgat tccgccgcct 54960
   ttaaacccat ttacgtcgaa tacgtccggg acgacgacga cgtatcaatc cgatcaaaaa 55020
   ttaccgttgc agccggtgca gatgtttaca ttcaatcaga ctccgtcggc gacgatggca 55080
   ccgccttcgc agtcgaatgc tactttttat ccgcaaacga cgtcgacact gccgccgccg 55140
   caaactttta tatcgatttc cgaggaagat tcggtcgcgc tcaacgaatc ataccaacaa 55200
   actctacgat caccatcgat cgaatcgtac aaacgattaa ttagcgtttt ggtggtgatg 55260
   tctaaaaaat atattagatc ggacgtgttc gtcattggct tgaatcgctt ggaatcgttc 55320
   gattctctgc tgagtagcga tctggaggaa ctgatgcagt gtattcaacg taacaccaat 55380
   tggtcgatag gcggcggagg taaagctttt actactccag aattatgtca gctaatttcg 55440
   acgatagtga caggctattg tcgctttgcc ggtaccgtgc tcaaatcgtc tagttttaat 55500
   ctgtcgagtg taggtagcgt cgagagtttg caacaacaga cggcttctat tcaaagtcaa 55560
   ttgtcttcga taatcagtgc gagcgactct gtcggcttga ataacgatca aataattaga 55620
   ttaaataatc agattgaaat gcttcaatcg acattggtcg aacgagaatc taccattata 55680
   actctgaata caaacataaa tattatgact caacgcaatc aagccctcga atcgaatctg 55740
   gcaattatcc agtcatcgta taatagtttg gctagagctt tagatgttaa tataaacacg 55800
   tccgctagac tggaaaatgc cgaaatcgtt gaggcgacaa atcgaattat acagaacaaa 55860
   attatggagg atagaggtct actgaaatca tttgagagcg aaaaacaaac atacggtcaa 55920
   tctgtaaata gattagagag ggaattgaac gattctcgtg agtatgcaaa tagattaatg 55980
   ttggaattga agtcggctcg tgatgatatt atccaattaa aagaggaatc gaaccgtaat 56040
   gcttcgaagc aacaggacga atctactatt aaagaacgcc aacaacaaga attgtttgta 56100
   tcggaattat tcgatttacg cgaagaaaat aaaagtaatc aaaatcgaat cgcttcaatg 56160
   agcatcgaat taatgaatat tgccgaagaa aataaagttt taaaagataa attggcaatt 56220
   cgagagaagc cagtcgctcg cgccaaaccc tacaatagac cgtcgcagat agaggcgacg 56280
   acatcgacgg ccgtagaaaa tcggactcga gctctacaga aaaccattaa agatctcgaa 56340
   acgactaatc aacaattgcg caaccaaaac tctagcttaa catcgcaaat gaacaaaaac 56400
   aaacaatcgg ctagcgttca ggcggactgc aatcgaatca ggtccgaggt agataacgtt 56460
   cgtcaaaagg tcgaggaatt aatgaaaaat caggcgacat taaacttgca agacattaat 56520
   gcgctcaatc aaaagactgc agactattac aataccaaat tgtccgaatt gaccagtgaa 56580
   aatcaaagca ttagagacat tctaaacact gaaatcgata aacaagtcga tatactcaac 56640
   gaacgtatcg accgatcaaa atcaaatatt cttgataaaa tcgaaagact atcggaagaa 56700
   tttagtcctg ttttagttcg tgcacaatca tcagaaaaat atgttcagaa tttgctcgcc 56760
   gattatgaat ctctgtctag aaatgtagcc aatcagcagt tgtcggaata atacaaaaaa 56820
   aatgtttatt aaatgtttat taaagtttat ttaaacccac aacccaaaaa tagtacaaga 56880
   tttgtaatta tatgtttatt tatacccaca actctaaaat agtagcaatt ttaattacat 56940
   caaaaattat aatacaaaaa cacacattat atagaataat attgttgttg ttcgtgttca 57000
   taggcagtga ctcctattat attgtagttt ttcgaaacat tatcggcgac aatatagaat 57060
   tcgacttcag ttatattctc agcaatcatg tcgttgagtt gattcaaatc cattttaatt 57120
   ttaaccaatt tctgtaatcg atcctcattg ttgcctttgt tgttgttgcc acagaagaat 57180
   gttcctttga gcgggtcatt gttgtcttgt tcgaacaaat taaatgaaat acttaatcga 57240
   tcgttgccat attgttccgc ttgaactcta gtaatcttgc cgacgatgac acgtttgttc 57300
   tggcgactca cgcttaaaat gctgttcttg tcctccttta acatagcagc atctttggct 57360
   ctctcgattt gagtaatgtc cataatgata aaagacttgt agttgttgtt gctctgatgg 57420
   catttgatgc gcatcaaatt gatcattcta ttttgattat ttacaaaaaa ttccaacaaa 57480
   tcagactcgt tatcgaagct ttgtttgaag gcggcactaa ttttctgtaa tgttgcacta 57540
   cattcaatct gatataattt tccttcacta cgagaactaa aatcgttggt aaacgacact 57600
   acaaatatca ttttgtacaa gtcattttcc attaatttaa agccaaattg aaatttagcc 57660
   aatacagaaa taatttcact gtcgtcaaag tctttctgac aaacaaatcg tttaacattg 57720
   actttcttat cgggctctga agattcttta aaatcggtaa tatagtagcg tttcttttca 57780
   tatgtaatgg ttaaattgta tttcttgttt agcttcatgg cataaaactg actggcatct 57840
   ccataatact ccttgctgac gttgtcgatg agtactttga ataagtaaaa tgattcattg 57900
   ttaatagata gagtgtttct ggcgaccaac tcgcccacga ctctattctg tcgcattggg 57960
   cccgcactag agcccacact tccagttgaa atcgacatag catcactttt attattagac 58020
   cttttcagag gtagattaac atcaatcttc tggcgtttgc cgccataaat ctcttcgtta 58080
   tcttcgacgg cacgatcgtt ctgtttttga tcgcgtttgc gctttttaat ttttttttct 58140
   tgttgttttt gtaatagaag ctgttgttga cgttgttgtt catcgtcttc gtcttcgtct 58200
   tcgacgactt tttcaacttc attatcgata ttctcttcaa cgtcagtggt gttgttgtag 58260
   acgttttcgt cttgttcctc ttcatcaaag tgagagggag cagtcaccga catattcttg 58320
   atagacatgg ccaataattc gatacgctgg cgaatgctaa actcggcgag ggtcgaagtg 58380
   tctcccgaga gtagagaaca cgcttggaag gatttagtta tcgacacgtt gatagccagc 58440
   ccgaaagacg attcctacag gactatgatc aataaagcca atttcgaaaa ctttgactat 58500
   aagagaccct tgatttatga tatcaaaact gatacggttc tgataaacag cgagtctttg 58560
   aacagagcac ttaatcgacc gaccggttcg ctcgatccga taaatgtgat gtctgcacac 58620
   gtatttctcg cttttatatg ctcactactg ttaattctgt tgacggcact gctttttgat 58680
   caaaacgaca gagacgaatc gccaattgaa ttgaacgatg gttagatcaa gtttcggtgt 58740
   cgtcgatgtc ggcggtcatt gttaaggtcg gccaaatgga ttattattcg aatataatca 58800
   actctcgact cgcgccgccg tcgttctatc tcgagtacag caacagaatc gagagcttcg 58860
   tcggtagttc tttaaccaga gaatatatac aaaatttagc aaagtttggt atatactacg 58920
   aaagcggcaa tggtgtttat aaatgtgcat tttgtcctct aattatggtc aaattggaca 58980
   tgagaacgct aaaatatcac acgttttcgt tgtgttcgat ggcgacgact atactcgcca 59040
   taaacgaaac tctccgaaag gaatcgtttc gcaagttcaa gactgctcga cgcatattca 59100
   aagaaaacgg caactctttg gccgtaaacg gattctatta ttatggaaaa aatatcgaaa 59160
   ttcgatgcgc cggttgtcgt ctcacaatcg tcaaattgaa tcgaaccgat cgtgccgaag 59220
   atattcatcg aaaatattca tcagaatgcg aatttaacaa caagcctacg gctccgcccg 59280
   ccagcgacag cgacacggaa attgatgtca aaaacgtcaa aaatcaccat acagacgtct 59340
   acaccgaatc gtatggtaat actgctgcaa aaatttatcc tgttttggat tcgaataatg 59400
   gtatcgtcca tgacgataaa accaacaata gcaatgatgt caacctgttt ttaaaatcga 59460
   atattgtcgc cgacggtgat aaaaatacac aatcggtttc gagcgtcgca ggagaggacg 59520
   atcgttattg taaaatatgc tttgagaacg agagaaatac gtgcttttta ccttgtaaac 59580
   acgttagcac atgcaccgat tgtgcccgca aatgtaaagt atgttgtata tgtagaatga 59640
   agataaaaga acgattggag gtttatttac agtaatttat tgtttatttg aactatataa 59700
   ccgcaacacg atcgtttcgg acatcattcg aaacaaaatg tctatacaaa cgggtgacat 59760
   agtcgagtcg aacgtggtga cccaatttaa ttcggtcgct tttattgtta atcatgagac 59820
   tcgtcgggtc acgatcatgt catataaaaa tgaacctgtc aaattccacg tgttcaacca 59880
   gcacgataac gatcctgatc ttctaaagct tcaatatcat tatccgggac tggcgagcag 59940
   tatcgaacta cctaaaatca ctcacatgag taaagttcga atcatggtat tcgatcagcg 60000
   acatcggatg attatcgtcg aagagtttct cctggaaaaa ctatactttg tgcatcatca 60060
   ttacgctaaa tattatgtgt acggtttagt gcctgttgtc gccaagaact ttaaacataa 60120
   tctgtatagc ggtgcgcctg tctttgatga aacgggtaaa gttttgttgt cggtcgttag 60180
   cgactgttat ttttcatcga caacagacga aaattgtatt atacccctga gcggagaact 60240
   gagcggatct cgaggagtgt tgtgtctcga tggccatgtg tggctcaacg aaagcggtga 60300
   cgatttcaca ttcgacaaga caaacgttgc gaatagaatc gatttgtacg tttcgcatga 60360
   taaaaaattt gtgtatttaa acatattcta taataacaat attataaatt ttattagagt 60420
   taaagccaag tttgttggaa atgtactgat tcgttaaaca tcgattagaa tgaactgatt 60480
   aatttattac tctattaaaa cgtgcactga ttagttagga attgtgtact gatacgttaa 60540
   atattgattg tatatttaaa gtgtaatgat aataaaataa aatcaaattg gttgtgacaa 60600
   gactatttta ttcgcgccga tgcggccaac tcgttgatca taccgcacga atttacgttt 60660
   cttttgaccc taacgaaacc gttctctccg tagtcggatc cccacgaatt tttcaacacc 60720
   cagaacggta caccattttc tatgccgtag ccgacgagaa gaacggcatg attgaggccg 60780
   tagtttttac acgaagtaat tattcctccg taataatctg tgagatcgac ggcatcgacc 60840
   gccactgcta taggacccat ttcatgaagt acatctttca atttatcctc actatataaa 60900
   acatatctat aacaattgtc cacggaaact tcaaatttat cgctttggag tcgacacggg 60960
   ccttgtaccg accgataggg atagtcttct tcgtattcca aaccgcccat agccattatt 61020
   tcctcgtacg cagtgtgtag tagtccgccg gcgcatccca tgtcgatggt gtcgcaatcg 61080
   accaattgct gttcggatag atcgacatgt tcattatact tgatggcata ttgactctct 61140
   aatccggcta tagtagagaa ggcccagcac gatccgcaaa acccctggtc tttgacggaa 61200
   gtgatcttgt taaattgtct ccagtcgaag gtttcttgcg tatatttcga gggaccgtcg 61260
   actatgacgg gctcgcaaga gttttttaaa gctggcgatc ttatgcccag acccgtgaac 61320
   ttggcgatga cttcattctt ggtcagatcg gcaaatttgt ttatattgta aacggccgaa 61380
   tcgttttgtt gattcttaag attgatgatt tcgagattag ttttaaagat tttataccga 61440
   taatcgcgtt cgttgtcgtc ggcgtatact tttttatatt ttgtttgaaa cgtttcaaaa 61500
   tattgcggag ctcgttctaa attatattgt aatttaaact gcggagccgc cgtgacataa 61560
   tcgataatgg cgtcgacacc attgtcaaag ccgccggacg caaagacgaa aacaaaaaat 61620
   agtatgattt ttttcataat tattcgctac tcatacaata aacgataatt tcttatttgg 61680
   acaatacagt catgatgcat tcggtaatgt tgcatttcta ggaaaattga tatttataag 61740
   acaaaatcaa aatgcatttt gtattcattg gaatcgtatg ctacatatta accatagacg 61800
   gcggacatgc tgcggctccc gggattcctg tgatcgattg ggccgacaga aattatgcct 61860
   tggttcaagt cagtcacgaa gccacctcct acgaaaatct agttaaaaaa gtcgatcgag 61920
   ttcaaattcc ggtctcttgg aacgtttgga gtggcgaaca aggcgatatc gcttatgtgt 61980
   tattcgacag taaacaagta tggagcggtg acgcatccaa caaacacgct tccatatttg 62040
   ttagtacagg cggtcgattt aaaatgactg taaagttgtg caattcggac ggatgcactt 62100
   cgagtcagcc ggtcgatgtc gtgatcgcag acacggacgg ctctcatcta gctccattat 62160
   attatgaata tcaggaatta aatcaaccgt tcgagactcg ggtcaaaaat aaagtcgttg 62220
   ccgcatattt tgtcgaatgg ggcgtttatc ctagaaactt tcctgtagat cgagtgccta 62280
   ctcctaatct atcgcatgtt ctctatggat ttattcctat atgcggcggc gacggcatta 62340
   acgacagtct caagaccata accggtagtt ttgaagctct tcagcggtcg tgttcgggtc 62400
   gagacgactt taaagtgtcg attcatgacc cgtgggctgc cgtccagaaa ccacaaaaac 62460
   aagtaaccgc ttggaacgaa ccatacaaag gtaattttgg tcaattgatg gcagccaaac 62520
   tggcgaatcc tcacattaaa gtgttacctt cgataggcgg ctggacatta tccgatccgt 62580
   tcttttattt tcacgatgtg gccaaacgca agatttttgt cgattccgtc atagaatttt 62640
   tacagacctg gaaatttttc gatggtgtcg acatcgattg ggagtttccc ggcgggaaag 62700
   gcgccaatcc caacgtaggc gacgtcgaac gcgatcgcga aacgtacacg attctgttga 62760
   gagatttacg cgaagccctc gaccgtctct caatcgaaac gaatcgaacg tacgaattga 62820
   ctagcgcgat cagcgccggc gatgacaaaa ttgccgtagt cgattacact gaagcgcaaa 62880
   aatatttgga ttatattttt ttaatgacat acgatttcaa aggagcttgg tcgaatagcg 62940
   atttgggata tcaaacgact ctgtacgcgc cttcgtttaa ccctaaagag cattatacga 63000
   ccgattttgc cgttcgtgct atgctcaagc aaaaagtcga aaacaaaaag attatagtcg 63060
   gcgtggccat gtacggtcga ggttggaccg gcgtcggtga ctttgtcgac gaaaataatc 63120
   cattttccgg cacggctaag ggaccgatca aaggtacctg ggaggacgga gtcgtcgatt 63180
   atagagagat ttttaattct tttgtgggta aagacggcat taatcgttac gattccatcg 63240
   caaaagcggc atacgtcttt aataacgcga ccggcgatct cataacctac gacagtcctc 63300
   agtcggttct ggataaagca aaatatgttt tggactataa tctgggtggt ctgttcgcgt 63360
   gggagatcga tgctgacaac ggattgcttc taaacgcaat gaacagcggt ctcggcggtt 63420
   taacaatttt tgctagtgaa aatgaatttt ataaagtgaa taatcattaa tattgcgaca 63480
   acacattagt cgcatcgatc atacaccggc ttcggtgaaa ttacataaaa cagtaaagga 63540
   aataaaaaac aaatgtgtag taaaaaaatt tttttattta aatatcttcg ttgggcacat 63600
   aactagcgat atatagctcc atgtagccgt cgtctctgtt ttcgttgtcg agaaaatact 63660
   ttaattttaa caaatgtttc tcaataatgg atatctcgac aaaagaattg atcggatttt 63720
   tagtgtaatt atagaaatat tttacattaa tctcgatcat gtcggtcggt tctgtagcac 63780
   tatcgatggc acaattttct tcagattctc cttctttcac aacgtaattg acattaattt 63840
   cgtcgccttt gctgttaaaa atgattttat cgagatcgat tttgatgcca acgttaagat 63900
   taccgagtaa atccaagtga cgacacaggc gtttcaattc gacactatgc atcatggctc 63960
   tgccggtcac ttgcaaatcg ttagacatgc catagtcttg actatcaaaa tgatacaact 64020
   tgtattcgta ggatttcaga gatttgccag tcttgatctt aaagttgaga taatcgtgac 64080
   tgtcgttttc gccggaccat atagtgagca cgctcgcgtc tgtgcacgtt tttaaaatct 64140
   tgcacaatcc actcatcttg acggatagag acaagttttt cgtaaacgta aaactcttga 64200
   aagcttcctt catcattgta agcttgatga atgaaatcct gctcgtgtcc gagcattgca 64260
   tcgaaattcc ttcgctggtg acttctagac tcgagaactc gaccaccgtc tcaataactt 64320
   ccattgcctt tttgaaagca atcggattct tgaatttagc ctcgaacatc ttgattgtat 64380
   cgctgacgct agttgactat tgacagtaac aaaaaacgat tgtatttata ctcgatgacg 64440
   atcgagataa tcgagatatg aaatgttatc gcaacaaagt tgattattaa gcgttcacta 64500
   tgattcgttc gattttcgtg ttatatacaa ttatcaattg tgttagatcc cacggatact 64560
   tgtcctatcc ggcggcgaga caatacaaat gctttagaga caataatttc tggtggcccg 64620
   aggacggaga ggcgattccc gatcccgcct gcaaacacgc atatcaaaca gtgtacgcca 64680
   aataccgatc agaaggagag ccagccggtg ttgccgccaa tgccgcacag tacatgtttc 64740
   agcagtatta cgagtacgct gccgtcgccg gcgccaacta tgaagatttc gatcacatca 64800
   agcgaaatgt agtatcgcat aatttatgcg cggccggtgc cgattaccgt tccgatcctt 64860
   tcggtgacaa atccggcatc gacgagcctc atacagagtg gcgagccgat actttttatg 64920
   tagatcccaa gcaaaaatat agtcgaggca tcgaaacggt tctgcatttc tgtccgaccg 64980
   cggttcatca gcccagctac ttcgaggtct ttatctctaa gcgcgaatat gactattccc 65040
   acgaattgac ttggaacgac ctcgagttta taggcggtaa caactcacag ttgatcgaca 65100
   acgacgggtc ggatccattg tgcgcacacg aatccatata cactatcaac gtcgccatac 65160
   cctttagaat taggaaattc gtattgtacg ttcgctggca acgcaacgac atagcaggcg 65220
   aaggatttta caactgtgcc gatgtcgtct ttgacgatta tttactcaca cacagacgtg 65280
   tcagtaagcg gagaaaggca tatagagatc tttaatttat cgaccaaact aaaattattt 65340
   tcgaccaatc gaaatgtatt ttcgaccgat taaatgcgtt atctgttaat aacaatttta 65400
   tctacaaacc aaagtatata aagctcggga tggtacgatt cgtgcagtta ttattttcat 65460
   catgtcgctg aaagtcaact acgatcaaac gttcgccgtc agcgctcagc aaactctggc 65520
   caatcatttt caagagtgta gaacaactaa tatcggaaga ctctgtgatc ctaaacgaat 65580
   tctaaagttg aatcaaaccg aagagcccga ccacgatgtc gtcaagaaag gaggagtggt 65640
   gtattggatg tggagagact gtcgcgtaca agacaattgg gccatgattt atgctcagta 65700
   tttagcgttt aaaaccaaaa gtccattata tatagtctat tgcctgccga aattttatct 65760
   aaattcgact cgacgacaat atcagttttt gattgaaggc ttgatcgagc tgagtgaaga 65820
   gtgtgccgaa ctcgatatca cttttgtgat actcgacgac agtgccgatg ttgtgctgat 65880
   cgattgggtg cgcaagcacg atatttgtgc tgtcgtttgc gactttaatc ctttgcagct 65940
   tcagataaaa tcgaccgtta atattttaca aaatctgccg gccgatgtgt actttgctca 66000
   agtcgatgct cataatgtcg tgccttgttg gttgactttc attgccgaca gacacgatta 66060
   tgatgagttt aaaagtaaaa ttgatgtaga actagaaaac ttgttgactc cgttttctct 66120
   tgtcatacaa catccgtaca aatcggtcgt gtctatagaa tcttcaacca atacgtccat 66180
   cgactggagc aatctattgt cgtcacgaaa tgtcgatcat tctgtgaagc gaattaaatg 66240
   gaccgaagca ggatacaatg cggcaatact gagattggcc acgttcattc agtgctatat 66300
   ttataattat aaaaattcaa tacataatcc agtctccagt aaacagagtg atttatcacc 66360
   attctttcat tttggattta tatcagcaca acgagtaatt tatcatttac gcttttgtgt 66420
   tacaaaaaaa agtgtccttc aaaaaactgt atttataaga aaacttaaaa ataatataga 66480
   aaaatttatc gaaaactgtt tatatcgaag ggaatttgca gacaattttt gttactttaa 66540
   tttaaactac ataactttta atgcggcttc gccacaaatc aagaggtata tagcaaaaca 66600
   tttaagatac tatacataca gcttgaatga attggaatat tcgcaaacac acgacaatat 66660
   atggaataaa gctcaagaag atttgcgaga aaacggcaaa atatatccat tcatacgcgt 66720
   atactgggcc aaaaagattt tggagtggac gtcaacaccg gaagaggctt tgaatcgtgc 66780
   gatttacttg aatcaaaagt atgctgtcga cggttgcgat cccagcggtt atgtaggatg 66840
   tttgtacgct atgagcggtc ttttgaacgc cggccaatct aatgtgtttg tctttggcga 66900
   aatcaacaat atcaggtcga aatggctaat aaacacgtgc aaatataaag attttacata 66960
   tagctatact tcatttagat ttcaatacat gtttgaatag attgccgtca tttaagattt 67020
   aattttgtat tacaataaaa actctttttt ttcaattata atttttattt tacaacaata 67080
   cattttaatg attataataa aaattaatcc tatacatgat tgttttattc gttagaactt 67140
   ttattgtcga ttgcctgcat tatgctgctc acatcttgtg ctggcatcga gccattaaaa 67200
   atataattgt taccattaaa gtagtttttt atacactgat cgtgcacttg ttgcggtgaa 67260
   taaatttcta aagcattttc atatgtcttt ggactacctt cgggcttggt ctcatttttt 67320
   atttcgtcga tgaccgcacg cacgatttgg ctgactcgtt ctttagcttc gatgctaggc 67380
   gtgagacact tgagaatggc atcttcttcg ctcattaatc cgagactttt aaagtgctcg 67440
   atagcggtgc gatcgaccag tcgatttttc ttcaccatct cgacgtcggc gagatatttc 67500
   tgacgcaact cgtcttcgtt gagaatttcc atttcggtac acaatttgtt tacatacttg 67560
   agtccgtaga acatgtgagg atatttcgat cgtatcacaa cccaaacggc cactggattg 67620
   gcgcatttaa tctgtaaaaa tttttccgaa tccagcagcc attcgtagcg tttagatcgc 67680
   tggcgtttcg acggattgga tcgttgacgc tcgaggaatt tatcgcattg atcgatctcg 67740
   tttcgctggc aacggctcat acgaatccgt tttttaccgt cgacggtgcg ttcgtatcca 67800
   gtgacatatt cctctttttc gggtcgattg ctcagcgatg gcacgacccg ttcgctcaca 67860
   ctttccaagt tatttctcat ttgttcgttc tcttttatat tgtcttgagc caaaagagta 67920
   gtgacaccaa gttgcatcat attcatttgg gccatatggg acattttgtt caggtccaat 67980
   tgcatcttga gcgcttgttc tttgtactcg gagatttgac gttcgtaatt tattttcatt 68040
   tcacagatag tgttattctt gtcggcaatt gtcagatcct tctcggcgat gactgtattc 68100
   ttctcggcga taatcaaatt tttctccatc acttccattt ttaattccat caactgtttt 68160
   tcctttgccc aaattgccga tttgccttcg tttgtgactt gatgaattat atccattccc 68220
   gcttgtattt cgacgggcgc gtctgcagtc atgctgtatt ctccttgttg acaaagagtg 68280
   ggcagcagat cgttggtgtt ccacgctttg aatcgcttcg cggcaggcat tgtgctggcg 68340
   ttgatcaact cgaagacgcc tgcacgattg atgaattttg tgttcgattt gatgttccga 68400
   ggtagaacgg atgagtcatc ggtcgatgtg aatcgtatcg ggttaatttc ttcataagtt 68460
   ttctgatttt cattagtcac aaacttagaa attgcaatat gtggtaagct ataatttaac 68520
   gctttagcaa acgggttggc agtcatccat ttttcaccat cgacatcaat cgtatacgtc 68580
   tcaacttctt tatcgtctac aaaattaatt tttgttagag acatgttttg gtaattttta 68640
   tgcgggctgt attctctttg ctgacacagt gtaggcagca ggtccgagtt gatccattgc 68700
   ttaaattctt gagcctgtgg catcttggag ttttgaatca gctcgaacaa acccgctttg 68760
   ttgatgaatt tagtcttcgg atgaagtgat gacgtcatac aagtctgcct gctttgggaa 68820
   gacttaagat tttcgaaaca caattgattt ttatcgctga cgaacgttga aatcgctttc 68880
   ggagcattac tgtactccaa aattctggca aacggattcg ccaacatcca tctctcgccg 68940
   ctttcgtcga cgacgcaaac gacttcgaga ttttcgttgc cgaagctagt catgattttg 69000
   gatgtagtca ttttaaaaaa attaaaaagt gattatgaca aactgacaca atgtatctct 69060
   accgatgtca agcgactact aattttttaa aaaataatat atatatatat ataactagtg 69120
   tttaaaaaaa tatattgtat gtcattaaag tacatgacaa tcgactcaaa aatcatgtaa 69180
   aagacatcaa ataacatgac aatgtaatga attaaacaac aagtttttat aaaatttatt 69240
   tatttattaa atttatagtg taatctgctt ggatagtatg tcgttgttgt tgttgatcta 69300
   cataatgttg cagttgagtt cgttatcatc gcgagacatg gttcgccttc tcttattagc 69360
   caaataacta gctaaatggc tgtcaatgtc atatttatta ttctggttat aatttttgtt 69420
   gtcaattttc gcatctttat tagtctggtt gattatcttg cccaattcgt tatttctcga 69480
   cgacaaattg cgtataatat tttgcatctg agcttcgcga ttttgatatt caagaattcg 69540
   cttttgatat ttgtttaata tcatatcctt ttgtttgttg tctttcatga cgtttactgt 69600
   tttttgttct gaatccagta gtttatgctt catattccgt ataacttgat cttgagcttc 69660
   tttatttttc tgatcttcga catccatagc gtttcgagta atttttcctt gatttttgtt 69720
   tacaaaatat tgaccatctg tacacaattt gatgagcacg gtcgaagcga gccattgacg 69780
   taaatcgatc gctttttgaa tctgagaccc ttttattaac tcgagcacgc ccgcgagatt 69840
   gacgaaaatc gtctcggttc cgcaccattt cgcacgttcg ggatcctgct tcataaatgt 69900
   gccgacgctg atagcaagct tcaaaaactt taacttcttc cggttatctt catcgacaat 69960
   cttcaaaatg cttctattta cgccaatctc gagaatcgaa gccagttgag taccacaaaa 70020
   ccactcctct tcgtacaaat cgacgacgga gatgacgtgt ttaaaatcgt ctttaaaaaa 70080
   cttggtaatg actttagaat tcatgatttt ctaccgatta ttgctgacaa attgactgag 70140
   aaagtgaatt tacactagct ttatacatta gttatcttga atgatatcgc ccgataacga 70200
   taacaaatcg tcttcttcac taggcatgat ttcgtttgtt tgtaaaaatc tcacacaatc 70260
   tctgcaagta atgatttgat cataatcttc ttttttaatg tttttgtcgt gtaatgttgt 70320
   cgatttcaac ggtacacgat ctatattata atatttgtgt agatgatcta cggtagtgga 70380
   cggatgagat tgagacattt ctatcgaata caatctgaat ccagtccaac gaatatttgt 70440
   acgtacacta ctatcgatca gataaaaagt cgtcgaagag ataacgacag ataaaatatt 70500
   ctttaaatat actgcacatt tgtatattcc attattactc gtgtctcgtc gacgaaaacg 70560
   gtagcaataa cgatggcagc aaacgatcga ttgaagcgaa tgttcgaaga gagtcgtatc 70620
   gactcatccg gtgagacctg tcacccgtca cgcgttcgtg tcataagccg attacgcgaa 70680
   ttggtcgtcg ccagcgaagg caaagaagaa ggtggtgttg tttactggat gtctcgcgat 70740
   agcagagttc aagacaattg ggccctgatc tacgctcaag aactggcgca cacggctaaa 70800
   ttgcctcttt atgtggtgtt ttgtatgacc aagagtttta ataatgcttc gatgcgtcaa 70860
   tttcattttc tgatcaaagg tctcgaagaa gttcgtgtcg aatgtcaaaa actcgacatc 70920
   acctttgtca tgctcgacgg cagcgcggat ctagtgttga acgactgggt gcgtgaacat 70980
   gacatttgtg cggtcgtgtg tgattttaat ccgttacgta cggtcagaga gtgggtgagc 71040
   cgaatccgtg accaattgcc cgatacagta tactttgctc aagtcgatgc tcataatgta 71100
   gtgccttgct gggtcgcatc gaacaaacaa gaatataacg ctatgttcat gcgcaaaaaa 71160
   ctaaattcta aactagaatg ttatttaaag ccgtttcctc ctgtcgttcg acacgaatat 71220
   aaatccagtg ttgttctcga tccgtgcact acgacacaca tcgattggca acaactgttg 71280
   gcttcgcgcg acgctgacgt ttccgtggga cctgtggatt ggatcgagcc tggttacatc 71340
   agcgccttga acgtgttggc cggattcatt cgatgtaatc tattaaagta caaaatgagt 71400
   cgcaacaatc ccgtattaaa aacgcagagc aacatgtcgc ccttttacca ttttggccaa 71460
   atatctgttc aacgagtaat gttgcatctc aatagcctta aaatcaataa aaatgacagt 71520
   ggtatcgatt caaacatcaa cgattacatc gaagagtgtt ttgttcgtcg tgaattagca 71580
   gacaatttct gtttctacaa ttccaactat gacacttttt taggcgctcc gaattgggcc 71640
   aaggagactc tggtgattca caagtccgat tctaggcaat atatttatag tttattgcaa 71700
   ttcgagcata atcaaacgca tgatgaacta tggaatgctg ctcagcgaca gctacgcaac 71760
   gagggtaaaa ttcacggata cctacgcatg tactgggcca aaaagattct cgagtggact 71820
   cgaactcccg agcaggcgct cgagtattct atttatctaa acgatagata cagtctcgac 71880
   ggtcgcgatc ctaacggtta tgtcggttgc atgtggagca tttgcggtct tcacgataga 71940
   agctttttag aacgtcaagt gtttggtaaa attcgctata tgaatcgtaa tggttgcgaa 72000
   cgcaagttca atgtgaaaca atacattatt aataacaaat aaatgattta taactattca 72060
   attgttttat tcgcatcatc cttgaaaaat agaatcaaac tgacgaatcc catatttatg 72120
   tattacgaaa tatacaatat aagtcacaat aacgtttgaa ctcgaacaat aaacacgttt 72180
   cgcgcttaat attttttaat aaaatatggc tcaagtcgac gctggcgttt tgactattca 72240
   gttcgacatc ggtagcataa gcaaaggata cagcatagac tcgagcgaca aagatatcat 72300
   aattgtcaca aaatgtacgc cggagaagtt tatggattat ttatacaacc gtactctgct 72360
   cgtcaataat cacaccaaga ctgttgacgg caattcgact agtgtggacc tgtacaaagc 72420
   tttacatgga atttacacgg gtcgctatta ctatttgggc gtcttcacga caaaggaaga 72480
   ttttcttaac accaatggaa cgtacgacat ggaattgtat acatttacac gcgagttgac 72540
   caggctgcgc ataaacaaca tactaaaaac catgatgatg tacaaaatca aaggtaacaa 72600
   tccggacgcg cccaagtttt tgttgatgac catgtttcat ttggcataca cagagtattg 72660
   gctgaaaaat aacgattttc ccgactgtgt aaagctaccg agtctattgc aaaagtgtaa 72720
   tactcgtaag aagaaagtat atcgtcattt gatgcgaaac agaagagaaa atacagtacc 72780
   gcttcaaagg gacattgact acatcaacaa ttggcaatac aggttgaaga ttcaagtcag 72840
   tcaactacca gctaccaaaa atcgttacga tgtcctcaag ataataataa tgtacatgct 72900
   aggtgaatct aatttgtata ttcctgaaat aaataaagtt taatcattca acttttatgt 72960
   ttaattttgt agaataagta tttcaacaaa tttaaataaa aaaatacaca atatgcatat 73020
   acgatcagtt attctagccg tagttttgtt aggctatcaa tatgccatgg cttgtacaga 73080
   aacaggtcgt aattgtaaat acagcgatga atgctgtagc ggtgcttgtt cggcagtatt 73140
   tggattttgt ttacatagat agatatacaa gtcaaataaa ataatacaaa ttagcatttt 73200
   attaaaatca tttatttttt ctggtatata aaacaatcag aaagacaatt tttacaaaaa 73260
   taatctatat aataattact aattagttgt tctttggtca acacgtgtcc acttacgcaa 73320
   cgatctaaac gaatccgatt atcgtcatgg gttacgtatc ttaaaggcag tttgtattcc 73380
   aaaagtatag ctccataatt tttttgtaag ttaagcgtca atcctgcatc tattatgtga 73440
   tcacgtagca tgcaagccgt attcaggctt tttatcttgc gaaagggcct cttgaaatta 73500
   atcataagca ccttaaaaca cgaatcactc aaagacttga ctgttggttt ggcacactcg 73560
   attcgagtat aatcgtacac tacacgaata tgtcttgatc tatcgcccgt atagaaacga 73620
   tattcggtcc ttaacgcttc gggtatatct tctaagcgaa cctcaatatt attagtatta 73680
   tatactctag cacgcaaact agattttcta ccgcaaatat cgacatcaat atgcatgtcc 73740
   ctgaacggtt ccatcgcgca aatagaatcg acccgatcga caatcgccga cagaatgaac 73800
   aaattcgatt gcatagctga gttttattcg atatactgat gacatgattt aaaaataaaa 73860
   aattatctaa tcttaaaagc gtttattaat ttatactact attaccgctg ctctcgccaa 73920
   tttcgtccga ttgttcgcgt atttcctcca tatcgtttcc attgttttca tcttcgtcgt 73980
   cttcgtcgtc atcttcgtca ttatcgtcat catcgctttc gttgattccg acatcgaatt 74040
   tgtttagata atgctgtgta ctcttccacg attcgtggtt cattaatttt gcgacttttt 74100
   gcagaggtac accattattg tattgattgc tgcacaagta gtgtctaatc atattcgatc 74160
   tgggtctatc catctcgacg ccggcttctt cgagaagtcg tttgaaatct ttaaatgggg 74220
   tagaagtgtt tttcgagatc tgcaagatcg tcggatgctt aacgtatatc tctcgcgcca 74280
   gctcgagtgg cttgttttta atgttattca aagcgtttat acggttacgt tttcgtttca 74340
   gattaatgtt gcttcgaatg ttgccctttt ttattagtgt atttaggttt tctatggtca 74400
   attgacgcgc ttcgttgatg cgcattccag tgcctagcat tatacaaaaa acgatagcgc 74460
   ctctgagcag accccgatcg tgtacatact ctccgttcaa atatttaatt tttttctcta 74520
   tacaaacgat cacactgtcg ataatctttt tcaacactat attcttttct ttatctcgga 74580
   tatttttgag ttctttatcg cggggcaaca tgaccatttt aggaatttta tactcgggca 74640
   agctcattgc gttcgaataa aaattgatgg tgagctggag agtttctttg gtgaccgatc 74700
   tcaattcaag cattcgacga cacagctcct cggggtccac gattgcttgt tgattttcga 74760
   tagaatcgaa ctctcttttt agcgtataat tattgatatc atcgagatta cgatcgtcga 74820
   tgagacaata gatgagtttt atcagacgcg acttatagct ttttaatgtg gtcggagcga 74880
   agggtttatt gaacatgtac tgtgaccaca tgctgttgct tttgacctga tccggcgagc 74940
   atcgctggcg ttcggtggca atctcgaata tctcctcgaa tctttcgtga ctttgaattt 75000
   tacatttcca actattgaac gagtactcat ttctaagatt tgcggcagca tctacgtcat 75060
   tcatgctaga ataaaatagg gtggtttgac tatacgtttt tctcgttctc ttattattac 75120
   aaagtaatat atggcataca atataactag aacacaaaaa atagaaagca atgccatcaa 75180
   gaccacattt aaaattccac cttcagcagc attcgcgtac gcagtataat tagtattgcc 75240
   gatgtcgttt aacaataacg aagacgatgg tcgagtttta ttgataccat cgtcctcggt 75300
   gtcgttggtc ttttgtttag gttttttata tccattttca tcgatatcgt tcacggccaa 75360
   cttcaacggt atatactcta cttttttttg attgagagac atgcgctcgt aaggcacctc 75420
   tagactcatg atcgtatcac gtgtaattat ttcagttgtt caaattttgt ttataactta 75480
   actgttgaaa aataaatcta cgtaacgctt cgttttgaaa ggccaattca gtgaggctct 75540
   gctgacaggg agttttatac gattccgtgt ttttcgtcga agtcaatgca gccgcactct 75600
   caaaaagact ggcattgatg cctttttctg tgctcagatt agcgataaat ttaaaaatgt 75660
   ccgtcgatgc ggacaatggt gccaattgac ttatgtttcg tttcaagctg agcacacgtg 75720
   atcgattacg ttcgtttaga gtatacagat aatagtcact gccgttgccg ccaaacaaat 75780
   cgtcaataac gttattgatg atggtgttga tcatcaacag ccgaggatag cgttgcttgt 75840
   cgttcatccc gtattcgata gcgttttgaa tgttttgcgg aatagtggct cgctgaagta 75900
   acagagtcat gtaatcgttt gcaatcttct ggtcgaacgg cagcggaatg ggggtgtttg 75960
   tcgcgatcgc ttcggctatt tgatattcga gggcgaatcc caattgacga gcggcctctt 76020
   cgacgctgct cgagtgtaga gctgctgcat tattattata aaatttatga gcgtacgccg 76080
   gcaaaacatt atagacgaaa gaagatctga agatgtcgct agtgatctcg ctggatccca 76140
   actcttgttg cagacgtgca taatgtttga tgagattttc atcactatcg aagcgcttgg 76200
   tcacgttaac gtcgaccggt tgggtttcga tacacacgtt acgtatggta ctgaccagat 76260
   ctagcatttg aggcgagaga tgcgacatat cgttggtgct ataataacga ataattttac 76320
   ccatatagtc gacacattta tttaaccaca tgtcattgac ggaagacggc acggctgccg 76380
   aggacgtcct ctgttgctgc tgattcatag taataaatgt tacggtcgtg actataataa 76440
   atttttgatt cgcgtttatg tggacacacg atgattcgag gatcacttat catataatta 76500
   ttgttgatat acaaaatcgc tataataaaa ataaccaaaa aaataaaata ccacgaaaac 76560
   gtaaacatat tggccacaat cacagccaat gccaaagaaa caaatacagt ttgcatgctc 76620
   tttcgtttac agagaatact ctcgcaattt ttgaaggcga cgttaaactt gttttcgcca 76680
   tcgataaacg ctcgaagttc gtctttacag cactcatcgc aaagcaccaa agccattatc 76740
   atggtgccgt cagagtgcac tatttgaaag gttttcggct gactgcccgg atgtaattca 76800
   aacgaatatc cgttcgatat atcgatgtga gcatagtaat gcgccaacat ggtgccgtag 76860
   gtctttttta ctctagtttt acacactttt ataatgttca agtcttcgct atccgttatt 76920
   ccgtcgaaca gataatgaat caataattcc ggatcatatt taatgcgatt gagggtcgtt 76980
   gtcaagtttt tgtcgtttaa ttgatagttc gccgctgctg tcgtcatcgc tgtcggaata 77040
   ttccatggca tctgatccga ttccattacc gttaattttg ttgttttcgt cacgatcttc 77100
   acgactatcg actcgtgtgt tctttcttat ctcgtcgtcc tcgtcgttgc tgtcttcgtc 77160
   gtcgtcttcg tcttgagatt tatttccaaa agtttttaga aataattgac tgtttgttga 77220
   ttttctcaac ggctcaatgt tgataccgtt gacgtcgacg gcagtgatat tattgttgat 77280
   gtcctcgaca aaattgttct ttaaatttat ctcgttttta agaacagctt ccgtcatctt 77340
   atcagtatag actattttaa aaatacaagc acctttcttg aggatggtcg gtttaatgtt 77400
   aaagagaata acgttaagat tgttgctcat attaaaatta tagactacta cataatcgcc 77460
   tccgacgatg gtcgacgaac gagactgatt ttgaatcaat ttcgctagca gctgagtgtc 77520
   gaggatgttt aaattatatg cgccaactgc cagttttttt aagtgatatt catcctgaac 77580
   aataaacgaa agaatatttt catcattttc tttatcgagt gtcacataga cggcaatgtc 77640
   gactgtgccg ctgttgttgg tggccatatt tttacttgta atattttcta taatatactt 77700
   atcaatatac aatgaattcg acgaacgaga attcaataat cgtctcaaag tcatgaccga 77760
   gtacataaaa agaacaaatg ccgaacatcc gctgccggtt tcgttaagtt atgtctctgg 77820
   cgtcgatcaa aatttttata cagtcactac tttttccact caaaatttaa tcgtagaaca 77880
   tatgagcata catgacgatc gacacgaata ttttaatttt ttgactcaaa aatatgaaaa 77940
   agtcaacaat tatgatgatg ttttagtcga ttcactcgcc accgtttcgg acaacaacag 78000
   agtcaaatct cattcgagtg atccgacaaa atatttgttg cgaggcgacg acggttggtt 78060
   tgaaatgaat tgccccatag aagagcaatt cgacgccaac gcttttcgtt gtgtaccgat 78120
   tcccgtgtgt caaaataaac aagtcggagt gcacggtatg acagaaaatc tactcgacag 78180
   attagtgctg aatcatcgaa cgcccagagt cgtcgatgac gataacgttc aatccgaaga 78240
   aattcatcct actatttatc ttagatgtgt cgaaggcgga tcggccgtcg tcgaagagtg 78300
   tcccaatgga tatctttttg acgtttcgca aggccgatgt gtaatgcgaa acgactgtga 78360
   aaatagaccc gacggttatg tattatcaca attccctgaa tctctaaaca tcgaccagta 78420
   catgatctgt cgtgatggcg atacagtaat tcagtcgtgt cctgtcggtc aaatattcga 78480
   ccgaaggctg atgtcgtgtg tggaggcgca tccgtgccaa tttaatggag cgggccacac 78540
   ctacataacg aacgatatcg gtcctacgca attttataaa tgtcaagatt ccgaaaattc 78600
   gattctaatt acatgcatca atcgggtttt tgtcaacgac gaataccaat gttcgggaga 78660
   ttctcgatgc tctgttttcg aaaacggcac cggtacccaa attaaagttt ttgacgatga 78720
   aatcttgtcg tatgaccaag gaattctagt ttgcgacaac tataacgtca tcaaaaatat 78780
   cgattgcgat gacacaaata ttttagaaaa tcgcatttac aacaatcgtt tcgtaccgat 78840
   gatcaatcta ccaaaagaaa tttattcgat agctgactcg aaatgcgtgt cgttcgattt 78900
   tagtttagtt agacaaaata acgacacttt tggcatcgaa tccgtgccca atgactataa 78960
   tataaaattc gagacggctt ttacgggtcg caccagcaat gcggacagac taatcgatat 79020
   cactcgaata gatagtgtgg tgtcttcata cgcccgcgac tttaatttcg tcggattcaa 79080
   ttttttaaat gaacaagaaa ttaattgcgt gcaaggatct ttatatgata tctttgaagg 79140
   ccgacgatta aacttgtgca cgagtgatga caattttaga cagattatca ttcgtccaaa 79200
   cgagttttat aaatcgaaaa acgtgctact cgaagtcgat agcgactata gagagtattg 79260
   tgccgctcga ttagacaata atatgagtca aatattcata ttcgataatt ttctcgaaac 79320
   gttttcggtc aatatacgac aattcgacga atgtcataat attctcaata aaattcatat 79380
   caaatatact acgattgacg gtaaatatac taccgtacag cccgaatata acttcaaaag 79440
   tgtaaacgac caaaaaaata tcgaaagata tgcggaaaat atacaaaatt ccgaggacac 79500
   tataccggtg cttttcgacc cgttcgagca catcgaaacc gtaaagcctc tgttcaatcc 79560
   gtttgcagag aacacgattg ctagagattt acccgatttt ccagacttgt tcccgcctta 79620
   tcctcctcct cgtcctcctc cgccaccacc accagtcata ccagagcctg aaccagagcc 79680
   cgaactgact ctaaggaaca aatttcttaa ttattcttgc ttcttttcat tgcccacatt 79740
   taaaatgagt cattgtgatg tcgtcgatga ccacataatt gaagctatcg ctaatattag 79800
   acagaacgta caggtgcacg atgattgtgt caacgctgaa ggcatcaaaa acgtgattaa 79860
   tgcctacgtt tatctgggaa atgacatcgg ttgtagaaca gaatttaccg atgacggcac 79920
   tatacatgtt aatcgtgtaa acgaacccac gaaatttatg aatctgacca ctcaatcaaa 79980
   cgacggcata aaatacaacc attggataca tacaaacgat ggaataaatt atttggcatg 80040
   tcctcacaat ttgtacaata gcaaccgatt tacctgttcc gtcgaatcga acagattcta 80100
   ttacatgcaa gatttacaga attaaagaaa aaaaactatt gtatatttgt gtaaacgttt 80160
   aatatatatt aaaataaatg gtcaaaacag aaatttaatt ttattcattc attcattcac 80220
   cccgcaataa agggttctgt aacagtcgcg ttggctggag gaaacggtgg aataacttct 80280
   cgcggcaccg aaatcggcat gatggatgcc cgagatccgt cgtcgggtct aagaatttca 80340
   cttcccatga tcaacggcac catcatcgga tattgttcgt atcgggcgag agatctctgt 80400
   agagcagtgg cgttgccctt aaacttgaga acgttttcga tctgtaaaaa gttttcgttt 80460
   atgcccgatc tatatttggg ttgaacggga ttatagaagg gcacgttggc aacgagaccg 80520
   gtgctgtcga atcggcacgt ggcacaattt ctaaactcta aactctccgt cgatatcttc 80580
   atgatttgag gcgcgacgca cttgttgatg agattttgaa gatagggcgg catactggcg 80640
   actactcgtt cagagatatc gtcattctca tcgtcccaac tataggttcg agcgctattg 80700
   ttcttgacga ccgaacagaa tctcgtggga tccgtctgcg acaggacatg agtagtgatc 80760
   gaatatagat cgtttactat gttcactagt tccatttgaa agctctcgtt gtatcttaac 80820
   gattgacaca cttgttcttg tgcttcatag ttgttgtaga tgatgtgtaa cacgagctgt 80880
   tcgggcaagg gcagtgccat aacgttaagc actgtctgat agtgttcttt ggtgggaata 80940
   agtattcgat cgttgcccac ggctttgtga ttgacgaggc ttttgccgac ggtacgatag 81000
   tatgtgttac cttcgccatc gacgatcggc atcgacattt tctccatttt aaatcgaacg 81060
   gacgcatgaa aatcgcaaat gaaccaaccg tcgtcgacat tcgcatccgg agaacacgga 81120
   gagccgtacg ttcgacacac atcgaacggc cgtaccgctt ggaagataca ataattattc 81180
   atacggttgc tgatgctccc gctcgacact agtgccatgg ctatcgaaaa tgaaatctct 81240
   tattcaataa atttgagcca agatttgttg tatattattt tagattctta tatttctaaa 81300
   aagtttattg tgacagaaga atacactgat ttcgtggacg aaaacaacgt gcgcactcga 81360
   cgatatgtcg gcgaacatag attcgtgagc gtgaagaaaa cttgtaaaag tctcgaaaaa 81420
   atagttttcg tcgacaagaa tattgtggtg ccgttcgtga atcgaattag tttcgaagaa 81480
   cctccagaca ctacgcccac gaacagcttg cgaagaataa taaagtgtaa agtttacaaa 81540
   agtgataaat gccccgactg tgagataaag ttcgagcacg tctactttaa caagaacaaa 81600
   attgacaatt tagattccct aatggccacg aaacagatgg tgctctataa tttactacaa 81660
   aataaaaacg aaactctggt gaagcagtct catttgggat cggatgaaat actggccaat 81720
   ctacgcttgg aatatgaata cacgacggga tttgtcaata gtgacgtttt aaaattcatg 81780
   tctacaatcg tgcgagactt tgacgagatt gtagcgtata agaacatcag tcctatgttg 81840
   ccttatacta cgctacagaa tcatataatc tacaggaaat ttgaagatga aaaatgtcta 81900
   atggataatt ttgaagacat caaagatgtg tataaatggg cactaaaact cgacggtatt 81960
   cgaggcagag ggtttatcac caaaaagcaa ataataattt ttatggacga tatgcaaatt 82020
   ttttccggac cttttgattc gccattcagc atcaacaacg tgattgcctt tcaatgtgaa 82080
   ctcatagaaa aaaccctata tattactgat cttcttcacg tgttcaaata tacgtataat 82140
   aatcgtacac aatacgaatg ttccttggat ccatacaata tagatcccgc caccgctatc 82200
   gattgtatca atttaatggc taatctctac aacaaaccgt ctaacgacga caacgacaat 82260
   ttcaagattt acacatttaa aaatgaaacg atcagcatta aatttcaaaa attctatgat 82320
   ccgccgatca aatctcttgg atacagcacc ttgccgaccg atggatttgt cgtgctcgac 82380
   acgaacatga gctacgtaaa atataaatac tataaaaccg tagaattgga gtacgataag 82440
   aaaaaagatg tgtttaaaac tctcgaaagc gatctcgaac aatacatggt caccattaat 82500
   ccgtgcgtgg gtgaattgaa acacgagagc atctacgaag ctatcgtcca cgataatcat 82560
   ataaatgtca tcaaatatcg tcccgataga ttgattccga attgatttag acacaatttc 82620
   ggagtgatcg actttgtttt tgaaaaatat aaataaataa ataaatttac aaacagaatt 82680
   aaaattggtt ttattcgata atgtattaca tgtgtaacga ctttttgtat tcggcccatg 82740
   tcatcctctc aaagtgttta acaggcatca tattcctttg tatgtaacga taatcgttta 82800
   tatgattgtg aaagaccatt gtggcgtata aagttccgtg tttcattaat gtgttttgat 82860
   tagaattttt gatggtaatc tcgtcgacca tgatgatttt ctcaccaaat ttctctcgat 82920
   tcaaactaat ttcgatgcgt tctatagaaa atattaaaaa tcctttaata cccaaataat 82980
   gttctcgaca cattgcacag tttaatttaa aaaataaatt ataaaatatg gtcttcattt 83040
   gttggagatt ggcggtgaca atttcgtaag ttagcttgtc tcggctgtcg atcatgtcgt 83100
   cgacgacgat agcgagaaaa tgaatggtat cccagattgt ggtgaaggaa tatgaatatt 83160
   ttttgggttg aaacgcccgc aaattgagct cttcggtctt ttcgataaat aacacttgca 83220
   tctgttgcaa atcaaaggtg ctcggcaact ctaaagccca gttcttcagc tgttcgatct 83280
   cgttcgcttg ttcatctttg taggtgatca cgcaagctat gctatataaa taagtcagtt 83340
   ctgtcgctag aatttcggca atttctctcg attgacaggt tcgtattcgg tctatgtgac 83400
   gaaaagcgta cagaaaaaaa ctatctttat aacgagaaaa caaaggagtc aacggtatca 83460
   tgattaacga ttctgaaaaa actatttatc tatatctatc ggatatgccg aacggtattc 83520
   agaacgacaa ggccgacgat ggcgatataa tctattttga gggaattatc gagtgtttcg 83580
   acgacgagac cgccgataaa tttggctttt tcgctgagct gaagaaagag gaagccttat 83640
   ttatgaaaaa aacattttac gatctcatcg aacacaacaa cggcaactat tgcaaaaatc 83700
   atgtactaat cgacgcgctc attatgtata agacgtatgc ggaattggtg gacgaatcgg 83760
   catttggcgt gaacatactc aattattgtg tagaatactt gacacacgtt ttcaagttat 83820
   ttcgcttgca gagtcgcata attgttgtac tgccgacaca cgtagactgg cagcaagata 83880
   atttaagtgc tcttttgaaa catttactac agtccacgct gattgaaatc gtttcaaaat 83940
   gatcggaact ctagtattga taatcatagt gttggcggtg ttgtactttt tgtttatcaa 84000
   caacaaactg aacatgaact cgatcaacga atcttcgccg agtttagcgg acagcagcaa 84060
   ttccctgcag accgacccgc agaccggaca attgagcgtc aagttcaata gtcccaaaat 84120
   caagtccctt cgcgtgttac acggagaaaa caaaattagt aaaatctacg tcagcgaaaa 84180
   accattgaca tacaacgaaa tcatcgacga aggcaacaga tccgtgggca cgaattgtgt 84240
   ttttctagga actcttcttg atacatcatc atcatcatca tcaccatcag cggcaaatgc 84300
   gactacaacg actcgcacta ctaccaattt cgatgtgaaa caattcaaaa acgtgttcat 84360
   agtcttcaaa aatctcgaat cgagcaagat taaagagtct acaaacatga ctcgttacga 84420
   agccgaagga atggtgtatt gtttaatcga ttcaactaca acgaccgtgc ccgatctaag 84480
   agacgtgtcg tacccgattg tagtgtacac gaccaatgca aacgcgcagc tcaagctcaa 84540
   ggaatgggac tacacacagc tcaacgatgc tggaactctc ttcctaaaga atgaaaaatc 84600
   ttttaggcta cagtaattta ccgataaatc ctaactatga attgaaagat agactacttg 84660
   taatttatga taaaggaaac acatgttaaa ataataaaaa aatttttgtt taatctctat 84720
   atttttctaa taaatataca aaattcaaac acatttttta ttcaaatcta gcataaaaat 84780
   tctggtctat gtatgttgaa ttgttccttg ttccaggcca tgtcgatgct gtagaagcat 84840
   ttttcatctt ctttatagta ctttttatat ttacgtttaa aagcactaaa caatgtggca 84900
   attcgttgcg aaccgttggc gtttcgtttg ctgaccaaca gatcgtgtag catattctcg 84960
   acgtagggaa cggcaagctc gatcatcttc tcgatctttg tttcgtcaat cattttggct 85020
   cccggtttta cttcgacttt gagcacgtag attaaagcgt tcaacggact attgttcacg 85080
   tccagacaca gtagattgtg attgtgtata ggatcgtgtt taatgatatt tttataggga 85140
   acataaccat ctttggcgtt tcgcttgtac atgagtacgt gagataggaa gattcgaacc 85200
   ggtttcgcca aatcttcatt gtagtttttc tccatcggat agagtttatt tttaatgtgc 85260
   caatatatag acccgttgaa tggtaaattt tcttcaaaga gatgatcagt ataaatgacg 85320
   gcaaatctat tgcgcacacc cttgtcatag tccgagatgt gcagcggttt attattgacg 85380
   atgagcaatt tataattagc ctcatacttt tgactgccct gaaactttcg acagaccgaa 85440
   ttgctcttgg tcgagtctgc ggtactcttg aagaaagaat cgtcacacac cttcatttca 85500
   ttgatcacgt acagctgaga gatcatttta tcggcctcca tttcgttggt gtccttcttg 85560
   gatagagtgt acttgtcgct gtcgtgttta tgcactacga ctatatactc gagcagttcg 85620
   aaaaagctcg attttcccga gcccggcttg ccgttcaagt agatgcagca cttttcataa 85680
   tcagagggta tgcccatgct cgccccgaaa tgcatcataa ccaaagagtt tatatagtta 85740
   aaattggtaa accctcggaa atagagatat cccctgacga tttgctttac aaaagtcgaa 85800
   ttatactcgt cgagaccgag tttggtcata aagattctca tataaaatct gactaaccac 85860
   gagttgaggt catcgttggg tctgcgtatg atcaatttgt cccaccatag attccacctc 85920
   tttaaaatgt gccatgtgtt acaatagttg ttgtaaaaat taattaaatt ctctttagat 85980
   tcttcatttt cgacatcttt caaactgtcg tttatccggt tatcgtcagc gaaatcgtca 86040
   ttatcgtcat gattgttttg atttatacac gatctagacg acaagacttg tgttttgagt 86100
   gtctcgatga aatcgtctgg tgacgacaat tccgtaataa aattatcaat aaaatcgatt 86160
   ctatcaatct tcgagtacaa ataatcgatt attgattcgc gatgttcatt gaaaaattct 86220
   agattattgt agagtaaaat gttttcatgg tttttcttca gtgaccattt caagttgtct 86280
   tttaagactt taaaatataa accagtcgag tagagtagag accaagacag ctcgatgatg 86340
   cttcgtgttt tcgaaaacaa ctccatgatt agagcgattc taaaagcacg cgaattaatc 86400
   tcaatattca tcagacattg gcactttttc ttttgttgtg ccgatttttg ctccagacac 86460
   tctgaacact ttagattgat catcagatcg gtcaattttg tattgctcag atagacacca 86520
   atcgtgatca attcgctatg atccgaatgc caaatttcac gaaatacatc attcagagca 86580
   ctctgttcgc ggagcttgca tttcaagcaa ttgttgagag ccaatgctat ggacatgttg 86640
   ttttttaaag tccgaatatc tctacagatt ttagccatat gatatgtctt gtaaattaca 86700
   ttttcgttct tgccattgtc catcataaat tgtatgacac gttcgggaag ataggttttt 86760
   tcctcttttc gggtcaacga ttttatcaga gtattgccca gaagaaaagg acaactgcta 86820
   tggtaatcgt taatgaacag attgtaaaca ccctcttcag tgaaatataa atatttccag 86880
   ttgttaaact tgacgctagg cagtgttatg ccgttgacag actcagtcaa cttgtggaga 86940
   tcctcctttt ccttgacggc aatgtaatgc tttccgttat atacgagaat ggtattggcc 87000
   gatttgattt tattaaaaaa tccactacat aacacttcgg cagtagcgcc acagcttttt 87060
   gcgagatcgt atgtatagtc ccaggttttg tttttgtaat aaatcaaata gtgaatagcg 87120
   taataatggg ccaacgatac gcacggatcc acattcaaga acacggccca atgattacag 87180
   aaacgaataa agacttttgg cgtcaatctg tagtatggct cgcaacgctt cttcgccaac 87240
   tcgaaattaa ttcccttgtc tccgaaaata ctctcgacca ctaattctag atacagtttt 87300
   atgtcggtct tgcaaaactc gagttcttca accttggaga tggctctcca tataacgatt 87360
   ataatgtaat cgaaattcat aaaattgctg tcgataaaat aattttggag aactctagta 87420
   tctgccaaat cggtggctgt caatacttca gccatatttt cgattatagt ttccacacaa 87480
   tcgagtattt gctgctgaaa tttgtccaaa ttctcactgc tgggagtgat attcttgtgg 87540
   gctttcaatt tctccttgcc aatcaaggcg gctggcgttt caaattcctt gtcaaacgca 87600
   cgtagattgt ttagattgat ctgaatcatg tgtttatact tttctgccgt gatacatttc 87660
   gataaattta tatcgtctct aatgtagtcg aaaggatttt tattggaaaa cactaatcta 87720
   tccttgactc tgcagacgcc ttcgcctccg tcgttcatcg ccagatgaaa catgatttcg 87780
   tcactatcgt cgaatctact gcggccgttg acaaacatgg tcctgttgtc ttcgtagctc 87840
   ttgatcgaca actccacatt gaggaaatct tcaggattga acacaaacaa atttacgttt 87900
   cctagcgatt tgttgtgaat caggggtatt ctgttgccaa tgtcgatgtc gtgcttcatc 87960
   agtaaatagt ggcgccatcc atagtaagat ataggaatat tgggccatat gtaatagctg 88020
   ccgactctat tacactcgtt gccgaatccg ttttgattgc tgttaaaaaa ttcttcaaag 88080
   ttgatgtcat ctctgatacg atcgtacaag tccacgtgaa taaagggatt caccatcaaa 88140
   taaaagtaat tgtcttcgac aaaccagtcg tgcggcttca tcgaagacag atcgtgagta 88200
   gtggcgcacg agagtcgttg ttgagtttcc gtgtgtccgt cgattaacat caacactagt 88260
   ttttcaaaat tttcataatt atcgatctgc aatctttgct cggtcgattg atttacaata 88320
   acgatactat cgacattgac tgtattgttg tcgccgatag atttaaaaat gttatcgaaa 88380
   atagtattag atctaatcgg tagtgctgcc attttaggtc tactgatttt tttaatggcg 88440
   cttctgttta taaatcctta tcggcgcgac atagaaagat tagtgagaga tcacaaacaa 88500
   actttacaat ttggcgctta cattgacatt ttcgatttga gcacacgaca acaaatcgaa 88560
   cgactgttta taataaaacc cgaaaacgta atattgtaca atacgaacgg tagtctattt 88620
   tattacttgg catcgaccag tttattgtgt ccaaacgagt tttctttggt gcgcttcact 88680
   cgacccgaaa ttgtagcatt taacgagaac ggtcaagcca ataccgtctg caccaatgta 88740
   aacagtttag ttatcgttga acactttatg actctcaaga acaacatcgc cgacgacaga 88800
   atacttttgt ccgtagacga aatccattac acaattattg atatcataaa tttactcatt 88860
   cgttcgggat atgtatttct acaataactg caaaattttc aacaatatcg ttgacgatga 88920
   ccacgacaaa gtcgtcgaca atcgtttcaa taatcgtttc aataatagtt tcaagtgttt 88980
   cgatggttaa tttttaattt aattacacaa tgaccacaaa ctaaacgata atgtttgcaa 89040
   ttttaaagat atttaccaca aataaagcga ttgcggctac aatgtcgaag acatcgacag 89100
   atatctaaac cgatgcggtc gtcaaaatta taaagactat gaccacgaat ataaagacaa 89160
   cggacgtgat ttcaaagaca gactcgccat ttgtaatctt ttacaaatta ggttagaggc 89220
   gttagatata aagtatttag tttcattttt gctacagtct ttaacgctca gcaatcgaga 89280
   tgggacattt tttaatccaa cgagaatcga tctttgacga tttgatcgaa tctagtgtcg 89340
   gctttcaaga tgtggacaaa ttcgacttta ttaaagacat tatcgtcaat accgatgtgc 89400
   tggagaggga ttgtgccgat tttatggatc ctaatttaga atttaatata acggctccaa 89460
   tctacgaatt gtgtcatgaa agtttactta aaatcatttg gattgccaag tacggaccga 89520
   actgtttttt accagagttt tggacttttt ttattagaaa atataacgga aaatgctata 89580
   aatgtgacaa tcgtgcacag ttacgttatc ccaagtcttg taactattgc gattccgata 89640
   tcaggcaatt ttattatcat gacgatggaa taatattcga agaactagtc aatgacgata 89700
   ataactattg ttcttcatgt cgaaaggctc tattcgacat agtggatgtg tatcaattta 89760
   atcattccat ctgtagatgt gattattaaa taaaaatttt tttatatatc acaccattaa 89820
   catgcgtttg tcgtcgacga ttctgatcga tcacggagaa aaatcttgtt ggatgttgat 89880
   cgatgtcaaa aaacgtaaaa tcgattcttt aaacgacaat aacgacaata tcgtcaaaga 89940
   cagcggcgtg caagataaac tctgcgtatt tggtgaaaat aaccacaaaa ttatcatcaa 90000
   cgacggcgac gacgacgacg acaacaacga tggtagattt gtaaaaagga gaaaaataaa 90060
   ataacttttt gaaattttgt gttttattta tactatatat tatttgattt gaaccaactt 90120
   tcgtattcgt caatgttgtc aacgatttcg tcatgataaa tatcccaatc gttgatgggc 90180
   tcgatacatt tgcgaacttt aataaaataa tcatacgaat aatcgttcgt tttcaaatcg 90240
   tcgacgagag aaatcgtttt tatgtagttt attcccgctt ttctgaggta atatagcact 90300
   atccttgggg acttgggaag tttggcatcc tcgtcgttgt tgacgttaac atctgaataa 90360
   aaagcctttt ccacgaaaat gtgtttcgtt ttattatcgg caatcactcg tctcgtcatt 90420
   gtcgacgatg gcgtcgacga gcgttgaccg ccgcagatta cgacatcaaa atacggcacg 90480
   agtctagttt tatccattga ataagtcacg tgctctttgt ttccataaga ccaaaggacc 90540
   aagacaaatc ccttttcttt gagggtaatt aagctgtcgt aaacgtcatc gtttcttatt 90600
   ctgacgtctt cctcgtcggt gatgagagta ttgtcgagat cgaagacgag aacgtgcggt 90660
   atctcccatg aaaagctgtc cgtttgcatc tgataaattt ctaaatagtt ttgaacgtac 90720
   cagtctttga gaaaccaata cattggtatt ttttcattaa ttacatacac gtggcctagc 90780
   gcagacgttt tgaaagccag ttttaaattt actcgaattt cacgcatatt gtcttcacat 90840
   cgtaccacgt gcatgatgta attgttggtg tcgatcgagc gagcgttgaa cgactttttg 90900
   tcaagggcga acacgataaa ctcgaacaga tccaaatgtc gaaaagacac cagcgaaagg 90960
   tcggcgaagt cgcaaacgac aagaatgtgt cttttgacaa gagcggcgcg tctgcgaagc 91020
   acgacccagg tcggtggcgt cataattgtt atgaattatt tttaattttt aaagaatttc 91080
   gaaacaacaa tcaatacaag gagctgatac aatttctcgt cgacaactat ccggccaatg 91140
   tcaagaataa aacttttaat tttgtcaata ctgaacattt atttcattca ttgtacgctt 91200
   atataccggc catcaccaat gtggaaaaag aacgaaagca aattcgtcta tcggaagaat 91260
   gtatccataa actgtttgtt aatactataa acgattttaa actatattca gagctattcg 91320
   acatgataat cattcagaag atgaaagaga cctgtccctg tcaattgctg ctacagagaa 91380
   gagaagaaat caaagtgtat gtaaacacga ttagagataa aaaattcgat tctaaaccgc 91440
   caaagctcaa aaaagagtac atcgacaaca tcatgtacaa gtactcgtta aattggaaaa 91500
   atcttttatt gaaaaaaaaa ttatcagaaa cgagcgacgg ccggggcaac accgtgggca 91560
   ttggcaagag taaaaatgcc aaaaaaaaat taaaactcaa aaagagaaca attttgaccg 91620
   atgatttgat ttatttaaaa aatattaaac attcacaaag attgccagcg ataaatggta 91680
   tgtcgctcaa agtctgtaaa cacagcttta tcactatgga gaagcagatg cgagccggcg 91740
   acgaagccgt ttccttcatt agatactgtc aatactgtgg caaaattgga aacaacggta 91800
   ataatcaata acgtctccgg ctgtatccgt agggattgcg actaccgctc gatgagcgtc 91860
   tgccgcgagg acgtccgggt ctacgacgcc tgcctccgct agagcggcgc ctgctggtcg 91920
   acctgcgtct gtattgtccg ccgcttgagc gccgcctgcc tccgcttgaa cgacgcctat 91980
   actgtccgcc gcttgaacga cgcctgcctc cgctggagcg gcgcctgcct ccgctggagc 92040
   gacgacgccc gcccgatgat cgtcgacgat ataccatttt gttgagttga atacaatcgt 92100
   gtttaaagta ttgtgttttt tattttacac cttatattat tattttagtt cgatatagcc 92160
   ttattagtat tttttacgtt tgttcgaggt gccgctgctg cccaaataga tattcaacga 92220
   atcggtctgc tcgggagttt tgacgagaat tccacatttc tcactatact ctcgaatgtt 92280
   gtccatcacc ttgaaaatgt tgttatagtc gttgacgttg aacttgcaat tggacacggc 92340
   atagttttcc atggtagtat agaatatgga gttggccgca ttatagaaca ttcgatgtat 92400
   agagaaatca tctgtaatct tgagcaactc attaataaaa tcttcgtcgt cgcaatacgg 92460
   aattcgattg ccttcgctcg cattgacata ttgctgcggc tcgagcttgc tcagttcgtc 92520
   tcctcgttcg gtcagcagtt cttcgagagt gcatctcttg tcgcgcgaca gagccgaact 92580
   ctgcagcact atgacgcgag cgaaacgagt cacaggatag ttcatggcct tgttcatcgt 92640
   gctctttaga attttaatat tgccaaagtc gacaatcgac gtaggtagat ccagcaaatt 92700
   tttgagtaaa tctgttaaat gatgcacctc atcgctggtg tagttgggca tacattcgta 92760
   ttcgtccacc atcgagctct ccagtagagc atagagaggt ctgtatttag gcgatttggc 92820
   cagatagatc atgcacgaga cgagatcgct aactttgaaa tcggtcgaac tcgtattcgt 92880
   caatgtatag tgtttgagta ttttctggca atttttgcgg aagagtccca tgttggcgct 92940
   agagctgtcc gccgcgatcg atctaccgcc gccgctgctg ctagagaata aactcagagg 93000
   atttctctgt gcagtcggcg ccatcgtcgt cgtcgacgtc gcaggaaaag gacatgaact 93060
   cgcggccggc ggtgtcgatc ctctctcgaa ttgactagtg agagcagcat tattttcgac 93120
   gtaagacgct tgcgacagtt gagccacggt ggtgagaaag tcttgaaact cgtccatcga 93180
   caggtccatg gtggtgtcgg gatcggcgag tagagtaaaa aattttggcc aaatggccat 93240
   attcatttgt gtgtcgattt tattttttat tcgttcaatt tcgagaaata acattacaga 93300
   actcattttg aaaattgggt acttcttctt attgatttac gattacaaat ttattatcat 93360
   atatattcga catgagctgg agcacgttta tagttttgac cgtattcaaa cgaatagact 93420
   gttcttcgtt cgagatggcc tctaatatag ttttagctat cgcagattgc ttgcttatta 93480
   cacgaatcgc gttacgtttt tgtgggttgg tgtcgttgag aataacgtcc gccacggtat 93540
   tcgtctggtt taaattttgc aaaaattcag ccgaattcat aatcatgtct ccatcgtctt 93600
   cttcgttatc gtccatcgtt cgattcgagt ggaggcgacg actcgtggtt cttgccgtgc 93660
   cgatcagact agccgacgac attgtcgttg agtagatgat acagttcttc gtctagttta 93720
   tatttagtca ccagttgtct tatatagcgt tcggctacaa tgtattcgct catcgtttca 93780
   ctgaataaat cctgtttgat actctgcagt ttgagcataa atttctcaaa tttatcgtca 93840
   tcataattat gaaaaatgaa tcgacagacg tttctcattt ctaattctgc agcacttttc 93900
   gttttattcg tagacgcttc caagtatagt ctcaaaaaga agccggtgaa cactatcgaa 93960
   gcgattttat ttatttttgg tgattttatt ttcgttttga gagccagttc agttatgaat 94020
   cgtttgaacg gagagaacag cttagattcg caagtattgt tgttgcgcaa tttccacaaa 94080
   agttgctcga gatcgccatc gccaagaccg ccggcgacga gacgacattc tttgatgaga 94140
   gaagcgcacg ctttaaagtt caaatagccc gtggtggctt tgtcacataa tagattataa 94200
   aagaattgag taaaagaatt tgtaatcaaa tcgtcagcat tgaacactcc attggtgtcg 94260
   tactcagttt tttgaataat atacaggaaa atgggcagac cgaacatggg tcttaaaaat 94320
   atgtcccatc cgtcctgtat ggcggtatcg aaaacggtca agctctccga caaataatta 94380
   atcttgcagc tgagacattc gattttgtta acggagcata cttcacattt cgaattcagc 94440
   tgcattatcg aaggcgctgg tgtggattta taatattttt gcaagtattg cattatagtt 94500
   ctgaattttg gcacttgact catgaattca tctttgagaa aaacggaaaa aatttttttg 94560
   acatcattat tgtcctgttt tctgtcgaaa tcctgtttca ccacgtcgac acatttatta 94620
   aattcattga agaacgtcaa gcctttgatg ttgacgttag tgctctgatc gaaatacttt 94680
   gaaaataaga atgccagtga atctatttcg gccgtgctca aaagaaccct aaaattgaca 94740
   ttctgaaaag agtcaaattt attgaatctc agagtgtatt cgactaggtt cattgcgttg 94800
   gtcatttttc aaatactaca cttatcagta taccgaaaac atggcctccg atgacgatta 94860
   tgatgaaggc gtcgacatca acaccgccga tgcaaccatc agacataaca catttaaaat 94920
   aaaattcgaa ttcgccaatc tactgtttaa atatctggca tacgttagac cggaagaaag 94980
   aatcaatctc gagtcgtttc gctctcgtct cgacgactta gacattggaa ttgagtttga 95040
   aatctctaat tttactctcg ataacgtgat aaatcaaatg aaaaatttat taatttacgt 95100
   gactcccggc ccatctgtaa atgttgaacc gtccgcgacg agtgtcacca ccgcaaatct 95160
   aattttcacc aacaacgatg acatcgacac gatcaaacta tatctgacaa atcttttgca 95220
   aaacgaaaat ctaaaacctt taacgtatcg atcgttaaac gaattggacc agagattgaa 95280
   gacggaatcc atcatttatg cggacattct ctccgaatta cggctggacg ccatcgactg 95340
   tgaacaaaat acgatactcc agagattttt gaatatttat aaaaattatg gaatcgccaa 95400
   ctgtatcgac acagatctcc ggtattatgt cgaaaagttg cgggatttcg accgatccac 95460
   gctgccgccg acggtggcgg atgcgctcaa aacgatcata tataatatgg actcgccgta 95520
   taaaattcaa gttacgatgg atcgacagga atatctcacg ggcgcgacca acgtcgacgt 95580
   cagggcgctt ttcaacagat ataacgagat tttacccata aaatttgttt cgtcagagac 95640
   gtcggcggta acgccgatgg acgcgacaac atcggcatcg ttcaaacgaa aattgggatc 95700
   ggatcgaaaa aattattcct cgagttccga atacgatgac gataccgacg ctattggtgg 95760
   cgtcggacgc atcattagcg atatcggctc gaccgccgcc gactcttcga ctcttcgaaa 95820
   aaaaagacga aaacgaaaag ccaaaaaaat atctagcgcc tcgacatcga agccgatcgc 95880
   tacaacaaaa ccttcgacga ccacaaccac cgagccgatc atctcgtctt cggccaatca 95940
   aatatttatc gaaggcgttc gacaatcggc aagaccgacg atggctcttc cgccattgtt 96000
   gttgtatatc attcgaatcg taccgaccga tgtgaacgct tcgctattga catgtccctc 96060
   gaacgcgttg agctcgaata tcaacacttt taatttctac ggtaaattgt caagaattaa 96120
   atcgttaaat ctcacgacag tcgacagaaa tgtccatttt ttcgagttgc tcgaacctct 96180
   agcgcattat ggtgtcactc taaacgacat caatcggtcg atttggttca tttcaaaggc 96240
   cggaaactat ttcgaggcga acgcttttaa tttcaataac atcagacgga atctggctaa 96300
   agaaacggac gattcggatc gcatagctct cttcatgata cgctataatt ttctatggca 96360
   ctatagacaa tttataagta aactagtgtc cagtgccata tcaccatata aaaaccagaa 96420
   aatcgttaac gtattgcagg tgtgcagcaa ttcggtcaat agggcgtttg ataaagccaa 96480
   cattagacta aacaactcaa aaatttactc cggtcccgtt gacgatatag ttcgtctgat 96540
   gaacggcatg tttgcggatc tgttgatatg atcgtttatg ttgccttgct tgttgtggtg 96600
   tttgtcgtct ttctgaccgt tctcgttata ttgagtgtta ataaaattca attaagacaa 96660
   atgttgtatt atcaatataa atatataccg gaaccattaa taagattcgt caaagtcgat 96720
   cgactcaaaa cctacgatga cgtgtccttt taacattaaa gtaatcatca gcgatagatt 96780
   ctttacgttt cctcacgatt atgtcgtgcc acaaactgac gtaggcggag ctccagtccg 96840
   caatctagtc gtgtacgtgc cctccgaaga ggacgtgcag tacgtagaca agcgcctgtt 96900
   taccaatttc gattcggtcc tagtctatag acacgaatgg acggatcgag tcgagagtcg 96960
   agctcccaaa aaaaatggca tcgccaccgt cgtctactgg aatccgatcc tgcccatcgc 97020
   cgaggtcggc gtgggcgaaa cacgcgtttt tagcgtttta ttgaccgata aactgtttta 97080
   ttgcaacact atgattgtcg atcccaacgt acccgtttgt cccattcaga ttatgaataa 97140
   atccctacga aactatatac cgatcacggg cgagtgtccg cttcagcatt tgaacgtgtt 97200
   actcgacgat aataaaaata actttttaat atgtttcaat agagagacat cgaccgctct 97260
   caggctattg aacatcaaac ggatcatgac cattttcggt tatagacgag tctctgccaa 97320
   gtattcgttc aatatgcaag attctcatct cgaacagatt tatatcgaac tcaagtacga 97380
   gctgattcgg agactgatga aaggcgacac cacaccgaaa tgcttacaat tgaacgtcaa 97440
   cagcttggag tatgtgaaga gggcgagaga gcttctgatg atacccgact ctgctcagac 97500
   tatagtcaat ttagtcaaaa tgttttcaaa acttattctg ccatatcttt tagtgccgga 97560
   cgtgatcatc aaattgaaca ctatggaccg acagcgtaag gttcgattgt tctgtaaaaa 97620
   cgacagtttt gctataactt catttggacc cgtgcccaac aatatggtcg aagataaccc 97680
   gatggcgttc gattattcgg acatcaatac gcccggacac ttgaatagcg tcaaagaaca 97740
   gatatttacg gcatctcgca tcgaaaacgt gaccatcaca gcggctagat acaattactt 97800
   tttttaaacc acaaataagt cgacatcatg agacgtatgg ctagaactct cggcggcagc 97860
   gacacaaact cgatactcaa ccaggatcaa ctcgaacaat tagtcaaccg aaatcagtca 97920
   tttttacgtg attttctttt ggttatttgt ggtgttgttg tctttattgt atgtttatta 97980
   tttatagtgt taattttcaa tttgaactct tcgatagaag ctagagaacg acagaaacgg 98040
   caacgaaaac agacatttat ggctaatctc gattacagat accgacagcc gaacagcagt 98100
   tttaataaat aattttttta acttgtattt tgttttaata attgaattta aattgattac 98160
   tgtgtgcatt ttcgaaataa aagctgttgg aggttttggt gacggtcgag ggcacaatta 98220
   aatcagaaat ggacatggaa ccaaaagaca acggttcttc aatatcttcg aataaaagct 98280
   gatgtgttag gcgagacctg ctcgtcgcgt tattgatagt tatttgacga gtttcagtgt 98340
   ctttgagtat gacaatagga aaatctacta tacattggat ggtgctcgag tttgtggtga 98400
   tgatgaaggc gttcgtgtcg ggcacacgaa caaccgtcgt gttatcttca tcgagagcat 98460
   cctcgaccaa caaactgaaa caggttattc ctctgccggt gtttatcaat tcagacgaga 98520
   tgatctgttc gaccgtttgc atgttaatcg gttcggacaa tcgagtatca aaattcgata 98580
   agcctcctag gttattgttc acaatgttgt gatgtttggt cacgactcca ttcgacactt 98640
   gattgttaga gttactcgtc caacgagact cttgagttaa atctttcgtt aaaaccacgc 98700
   aacgtatggt agatgaagtt acaggacgaa tcgcattaat acgttcgtac atatggaaca 98760
   tgccgtatcg atgatacagt gtatagctgt aatattcgat ggataaagca tcgaaacgag 98820
   cgtgcatagc catgacaccg gcgttttctg tggcgcacat tgccgtatat gcaaacgccg 98880
   gatgaaagct actcgtgctc gggcccgtag tcggtatcac cacttcgccc tgcagattgg 98940
   tcaacagcaa aataccagac tcgagaccta gcatttgcgt acgatacgga attactcgag 99000
   ctccgttcgc ccagattttc cgagtcatcg tccacaacgg agcgtgccta ttattagtgg 99060
   gatcggcctc gtaataggcg atttcgggag attggcccac tacggaaccg aaatatttgt 99120
   cgtttcgaat ggttaaaatc ttactgaagt ctccactgat tacaccatta cgatagtcaa 99180
   taaagaatcc gagcacatta gaaaaatgag ctccgtttct agatagaacg gctggattgg 99240
   caagtccctg tctactgccg atcagcgaga ttgaattata aatattttgc atattgacag 99300
   tcgattcacc gaatagatag ttgtagtagt cgaatgtgaa gaaactgtta atcagatatc 99360
   cgtaggccct gacgtcggtg tgatcgaagt aggcgaaatc ttgatggatg ccgttaccgg 99420
   acactacaat cgggaaattg atcaatttga gcacgtattg aacctgatct tcggctttga 99480
   tttgagcgaa agaatagcct ctgagaagct ggccataact gtacggtaga cacattcgca 99540
   tggcgtttcc ggccgtgcgc caccagccca atgcaaaagt cggcaacggt aaatactcat 99600
   cgaagacctt gatgaccaac tcgtcaagat tataaaagcc ccttagcacg atacaggtat 99660
   tttgaaagac ctctggcata gtaattgaga aatgatacca ctctgcttgt attccccacg 99720
   gggcttggtt cacaggcgcc gggaacggta gatgcgaaac gatcgtgaac atagccgcat 99780
   aaagatttgc agcaagttgc gaatctaaat agagcgaatc gttaggattg cggaacctga 99840
   ctccgtagcc tatcagtgta tgtagtacag tgccaaattg aggtacacta ttccaaggat 99900
   ctaggcctac gaaaatgtta ccatcgtcag aaaattggcg agtgggattc gctatttttt 99960
   cggccttttg caaaaacttg tattgaagag tttttatata ataattttca aacgcatcca 100020
   aatcggatat gttccttcct tcggttccta atgaagacga aacgtttgcc attgtacggg 100080
   caggtaaagg attcgatgaa aattgaaata cgaataatat gacgattaca acaataatga 100140
   ttgcgaatag aaacgcccac atgatgagat gattagacga cacaggacaa acacaaaaca 100200
   aataaaatta ttttcttata atcccatatt tatttatttc aatattcttc taaaataggc 100260
   agtttgtaga aaaatttcca gatgaatacg tccatatact tgatgatagg cattcgagtc 100320
   ctatattttt ccttctttac cactatgctc gtttcgtaat catcacagtc gatatttccg 100380
   atgctattat tgaaatcgtt gctactgcga tgccagggct tgacgtctcc ataatagttg 100440
   atcacgtagg cttcgctatt tttacgcaaa tgatgatacg atcctgccgt ccacgcatac 100500
   aaaatggtca aatgcgttat tgtcatgttc gaaatcgcca acgcttgcaa cagcacaggc 100560
   tcgtcgaatc cgttgtggaa atgacatttc tgtaaaaatc tattcgtcgg gaccaaaagt 100620
   gatttaatcg tgtttaaaag cttcaacgac ggctcgaaca gcaccgtgcc cgctttacat 100680
   aaaattttat tgtgtttata ttctagatat tgattgattt tacaagcgtc aattttggta 100740
   ccgaatggca tttttccata gttgtcatcg gccatgtaca gagctggagt ttccaaagcg 100800
   aacaaatggt caatgttttt gattacgatg tgatcggcat cgaggtagag tattttgtca 100860
   tagtcggtca acgaaagaca ctgccatttt gtgaaggcat gatcgatcca aggcttatac 100920
   atttgattct gtcgattcga cagcatgggc ggacatgaat acgaaataaa gtcgaccaaa 100980
   actactctat cgtagattcg ttcgagagtt tcgactgcgc gatcgctcac atccctcgtt 101040
   accatacaca ccaaatcgtg aacggtattg gtgtacaata tgcttttggc caaagctaaa 101100
   gctcctttga catattcatc gccaagcata accaaagtca cgtatgcata cattttggtc 101160
   tttatatttc taccttaaca caatacgtcc gaaccgaatc aatctaaatt agcgtgcggt 101220
   ccattcatga tggaagaatc atgaatggtc gatgcgaatc gcatcgggtc gaacgatcat 101280
   gaatgattcc accgacgtaa acgccatcgg tatcggccga cgttaatata ataattcacc 101340
   gcaacattgt cgtaaatttc catgacacta acgttaataa tcatttatct acagtttata 101400
   taaaaatttt aaaaattttt gaattttatt gaaagtttgt tttgtgattt tgtctacgac 101460
   gattatctcg aagtcattgc caacaataaa cctgtcgttg acaattttat tgtcgacatt 101520
   gtcggtgacg acatatgtaa tggtaattgc gcatttagta atgcaataat ttaggcgatg 101580
   cgcaataaaa aatctgatgc aataatttag gcgatgcgca aaatctgatg caataattca 101640
   agtggtggcg taataaaaaa tatgatgtaa taatttagat gatgagtaga accaatcgat 101700
   tctgccgatc acgttaaaaa tgtttccaat cgacccaaaa acgattgaaa taaacttgag 101760
   aataacatca ctgacataat cgataacaaa actttcaaaa aagtgcgcaa aaaatttttg 101820
   tgtggcgcgg cgcaacaatg tgtcgattta caatcaaata acggttttta tcatcattct 101880
   cgacacaaat agcctcaact tggggtttta taaactctaa aaatgttata atgtcgcgga 101940
   caccaaaatg acaatccgta aattaatttt tattctttct tcatgaaatt catataaacg 102000
   accagtttat tcatgtcgat gatatgtgga tatgaagatt attataataa tactgataaa 102060
   ctagggaata taaagaaaag taaagctgtt gattcgttag tataccagtt gccctcaaat 102120
   cgaatagttt atccggatca atcatggtca acgattttaa tttcgatgtt atcgtcagaa 102180
   ataattccga taattctata aattttgtaa attttgtagt cgacgacaac aacccaaaag 102240
   ccgcgagcag cgatttcggt gccgtcgata aaaatttgag agtcgatgac tcaatcttcg 102300
   gtgtcgccga caacaatatc gatgtaaaca gcaacaatct tggcgtcgac aatataaaaa 102360
   tttctaatca agactttcaa gatataatag attttatgaa caagaatgcc gaacgaaaca 102420
   gagagttgcg catgaaaccg tataaaagcg tcgaaaatgc gttgaaaaat ttcgaaacta 102480
   acataaatgg catgaaagat gatgtgtcca acatacaaga aaaaattaat agcatggaaa 102540
   ctcaactgaa atgtatcgaa gatgatgcct ataacaaacg attctctatg agaaacaaaa 102600
   agactcgcta tctgaataaa agggttcgtc agctagaatg tttgacacgg actcagctgt 102660
   tggacctgat cgacaaatgc attcatcctg tagatgttct tcgattgact gaaattgtta 102720
   aaaacaaatt gagataaata aaaaattaca tatatttttg catttttatt tagacacagc 102780
   atttacaata aattgtttag attgattaca atcaagcaag caaaatttta caatacaatc 102840
   tggattcgaa acgaaacaac attaaacacg taaaccaatt gtttcgaata catcgcgact 102900
   ttcaatcgct ttgtagatgt cgaatccgcg catcttgagg acacgatcca aataggattt 102960
   gattgtaata acaaaagatt tgtgagctct ctcaatgtcg tcagagtcaa acgtatcaag 103020
   tttagcgatt ttgctgaaat ttttgacgat ttcgtcgagg ggaacttgag tgagagcatg 103080
   aatcacatct ctgagctgtt tcaccacact catccatgtc tctacattca tcggactctg 103140
   cacaacaaaa gatttataat tgtttaaaag cagccgattt ctttcaaatc cgatcactct 103200
   actcatcttc atcttgacat caatctcgat gtacacggca ttttcatatt catcatcatc 103260
   atcatccaaa tccatatagt aatctctgtc gtcatcgtca tcgtcgagtc taaggttttg 103320
   atattcatcc ggtatccagc atgtgacata atggaaacaa gtctctaaaa actcattcgc 103380
   gacattgtcg ctatcaaaac aatatgttgg aataccagca agctcttcgt ccacttcatt 103440
   gcgcactcga acaatatcaa acatgccagt ctcttcctgc ataacaatct ctgccatggt 103500
   ccacatggca tagtcaatcg acgaattaaa cttgtgaatg gtacctctcg ctgctagcag 103560
   tattacttct gtgatgatat tcggcacgga agtcgcttcg atacacatca tcagtcgtcc 103620
   gatcacacaa tcgaaaaatt tttttctatt gatcaagttt atggcatagt tttctgtccc 103680
   gagcccatgt atatcttcgg aagattttaa atgttcgtca tttttatatt taacttgaaa 103740
   ggcgtcatga atttttttga actttaaaaa tttgttggca atttgaggac caaattggtt 103800
   gagctcttcg tcgacgacga cgagagaatt atcgatgtgt tgttgttgtt gttgttttga 103860
   agacatgttg gataatgata gaattactac attttcttga catttatact gggatttcag 103920
   tgattatcaa agggatttca ataattatca gtgtgatttc aatgattaat agagataata 103980
   aaacgacagg ataggatatc aaaaataata tttatttgca cattattcag attttatgat 104040
   ctttcgtagt tgcttcggag aaatctcgta gcaatcgttt tctttgcggc agttgacatc 104100
   caatatgtta tgcaggattt tattaatagt tttttcggca aacaaactat tcttggcgta 104160
   caccgaataa tagtgttctt tagggttttt gatgtcttta ttgatggatt ctatatactt 104220
   taaatgcttc ttggccacat caattttata cttgttcgcg gcttgtagtt ttctgttcgt 104280
   gtagatgaaa acggtattca ggctcatcag gcgacgagtc gacgacatag aagacatgga 104340
   agacaaagac gatgctgaag atatcgaagt caatgaagtg gtcgatgcca tggagaccat 104400
   agaatcttcg gaatgtgaac ttgaaagact catgatgaaa cagagcgaaa tagtgcacta 104460
   atgtttaaat tttggtatat atcattattt atacatatct ttaatcttca taatctgata 104520
   caaacgtacg atttatctga tataaagggc tttatcagat acggaggtaa tataaatgta 104580
   cgatgttgct gcaacgaatg cagtttatgt actgaacata aaacatcatg tcatacctgt 104640
   tgtctctcgg tggagtggcg tgcacgacca aaactacaat tttgagacag atcgtccaag 104700
   acaaaatacc taatgtcgtg gtgcattttg acgactacaa ggaacttcac gatcgatacc 104760
   agtttgatca tcgaatgggc agcgaactgt ttgcagcata tcgcaacgcc aacgatacag 104820
   tgttcaagaa cgattatatg aatgtgcaca tttttgatcg ccagccaatg gaatcgctcg 104880
   tttattcgag tcttcgccac aaaatcaaca aaaatatcag ctactacatg tacatcaaca 104940
   gtgtgaacat gggactgcac gacggctgga agagcgtcat attgcgcacg cacccgagca 105000
   acgatgagct gttcgtcgaa atgatgaaga agcgcgacaa taaaatcgac atgtatacca 105060
   gagggtatat acgcgagcaa aataacaggt tcaatttgtg gtccaaagct gttgacgcta 105120
   ctgaactgta tgtcaatttg cagaataacg atattaaacg attgactcaa gaacagaata 105180
   acattaaaaa ttatatagtc aatcagatct ataatgtcga atgtttggac ggtctggtgg 105240
   tttacaagtt tagattgcct ataatccagg ataggattgc tctattttct tatgattatt 105300
   tactgtggaa tcttttgtcg tttaatcctc agcgacgata cagattcgtg gtcgaaaaaa 105360
   tgattaggct tttggataac aaatttacac taattttcat gtgtcatttg aagctgaacg 105420
   aggtaaacga gcatataaat tatttgtgca agactctaaa tttacctcta atcatgatga 105480
   ttgccactga gaacaacaaa tattgtgtac ctcgatcggg ctgtgtagat tatttgacca 105540
   atctaaatcc taaaatcaat cggacccaat ctcgctacta cggaaatttc aatagtagta 105600
   tggaagaaga ttatgctacc aataatagtg tacaattttt cgatcgactt gacttttttc 105660
   taaataaata aatgaatgaa taaaactcca aatgtttcat tattgtgatc attgtcgaaa 105720
   attttaattc cattatcgtc aattacaatt ctataatcaa tattatcaat tttatatgtc 105780
   acattatcga ttccactgtc ttcaattttg tagtggtttt ctattataat tgaaataaag 105840
   gttttgcaat tttatacgat ggtgttattt ttcttttacg actattcaat gccaccgtta 105900
   aaagcggtga agatgctgta gattttttca gattttcgtc tgtcgtcgac tttgtcgtcg 105960
   tctgtgacga taacgatgat gttgcggtta aagtgatcgg acttttatta ttatggctgt 106020
   atagatcgat cgtttctata aaaatttttg tcgcaatctc tttaccaaaa gtgacgagat 106080
   gatgctcgtt ggtgcgggga ttgtccagat tttcgaggca ctcgttgtag tgttggagca 106140
   tggctcgcgg cgagctcttg agatcgggat cgattccgtc gagtcgcttt agagccactt 106200
   cgaggacggc tctgtagttt ttgaaatagt ctttgccttt gttgagacca aatttgaagg 106260
   cgatcaacaa gagacgcctg ttgaaatctt tatagtcgat cgaatcgtca acatatttgg 106320
   tttggctgaa taatttttta atagtgtcat aattttttgg agatggattc ttaaagtatt 106380
   catcgcgtgc gcccttgacg atggcaagaa tattgtcggg tagcatgcgc tcagattcga 106440
   tcagagcgct acatttgtcg gcgatgagtt gcctagcaaa gtcgtcaatg tttatcgaat 106500
   ccataaccgc gacgagtact taataggtag ctcacaaaca caatctatcg ttatctagtc 106560
   ctgaaattga gcgatccgtc gaggttaaaa ttgtagttgc tcaatttaaa gtataatcga 106620
   gccacgtcca tcgaaccgat tgcttcgata acgtcattca attcagacga atgttgaaaa 106680
   ctgtagacgc aatagcacat tttgtagttg agcagcgtgg acaccaacaa ttcgatgaga 106740
   aactgaaacg ttttattttc ggtgcagtcg ttgatgccgg cgccggcgtg taacctgatt 106800
   atggcgtcgg gttcgtcttc gatgtcgttg ttgtgtcgtc tcgtgattct attaaacagc 106860
   atgcccgaat agagtttgag tatcatacga tcgagatgtc cgatatcgga ttgacgagaa 106920
   cccgcatgcg ttttgatgtc gcaagactcg aacatgagta tattgacgta gacgatattg 106980
   tacagactta tcgcgtctgc ccagaatttc gattcgtacg aagcgttttt cacgagatta 107040
   tactgaatga tattgctgac gatataagaa tcgttatcgc taaacaccgg tttattattc 107100
   ttttcaatga aatacgctac cgcatacagc tcgggatatt tcataaaaag tatgtcttcg 107160
   taggaagcgc tatcatcgag agcgaatagg ccgggtcgaa tctcggcata gtcgaggatc 107220
   aggtcgtgcg acttgaaaga cgttcgagta ttatatgcat gtgtcacatc aaactgctca 107280
   atgtttacca tattggggtt gattttctgg ttggatgagc gaattttttc tatcgtcgga 107340
   gccgttgccg cagacggatg gtgtacgata atcggcttga ctaacgattc ggcacttaaa 107400
   cgaataaaat taaacatgtt tatgaaatag ttgaagaggc atttgagttt ttcaattttt 107460
   agaggagtat cggccgcttt cagagccgta aaatcgagtc cacgaatact atcgaaaaag 107520
   aaggcatgaa tcaatagaac gaggcaatga tagcggctca tatcggtttc gttgcgtatt 107580
   tcgagagagg cgatgtcagt gttgaggacc acttcaaact cgacatgaaa tttaaaatct 107640
   tgcatctcga catcctgtat cctatttagc agttgaatgt acgcgtcgat tcccaaatag 107700
   cgattcattt ccgcaataaa atcaatttta ggagttttgg tcggcaacgt cgacgacaac 107760
   gtcggcgctg ttatcgttgt gattgtcgac gatattgatg gatttttatt cggaatcgat 107820
   gcttcggtcg ttttaatcgc ggccgttgtc ggcgacgatg attgatttat tatattattc 107880
   agtagattta gtgtatcttt aatgattttc tcatcttcgc tcatgtaatc tatgatttta 107940
   ttacgaaaat cggcatcgtc gatcacgttt aattttaatt tcaaatcaca aaaggtctgt 108000
   tgatgctgtt taaatctatt ggaaaaattt ataaattgtt gagatgtggc attgaaatcg 108060
   ttggcaaacg ccatagtttc ggtgaacgtg accacaatac tgttttgtaa tgaaacatat 108120
   tgatcgatga tacttttttt attcattgtg tatcgcagag tgtataatct tatattagga 108180
   aaaaaaccaa tacaatagta aattcacata ttttttattt gataaaattt gatttcgaat 108240
   aacaaacaat acaaatagag aaaagttatt ttatttgatt aaaatcgaac gtatcaatta 108300
   tttaaatttc cataaacata ttgatcgaga tcgtacatgg attcggcggc agaactacaa 108360
   aaaggcatgg aatttggaag gcgaggacct ttgttgtggt tttcgataaa agaagttaga 108420
   aattcttctg catatttgag catgtcgtgt tgagaaaaat gtcgttcttc ttcttcttca 108480
   atatcatcag cattcagttg ataaacgtca attatttgat tcctcaaaat gtccaattct 108540
   ttaggcatga tgtcgtgtag attttccggt aaaacaacaa cagtatcgta gaacattaaa 108600
   tgctccatta gatcatcttt aaaagcagac agctctcgat taaagttttt tcttgaatag 108660
   tagttgagcg tttcgggtag catgtcaact atacaatcta tgagcatgtc gatcaaatgg 108720
   ccggaattaa taaaatgctc accttttgaa ttgtaaacgc acaatttatt atctagagcg 108780
   tagaccggat ccggtgtgaa tcttttggcc gtttgtccga attttttaac gtaaacctca 108840
   aaatgtttaa agaaatcttc aattagcgta tccatgtaat tattatcatc ataatctcct 108900
   tcgtccaaat tcaaaatgag ccatacgcca ccgcattcga tcagacgatc cagtagctct 108960
   tcaaatctgt tttggtcttc catcgagacg ggcaacgcga ttttaaagca atcttcatca 109020
   tcgtcatcga tttttgccgc acattcagcg caataaactg tagaattgag tttattcttt 109080
   tcaacgactc gaaatgtcca aacgttggct tcgtattgtt gatagccata tccacaaacg 109140
   gtacaaacga aataaaagtc attagtgata aacttgtaag caaacatgtt gattagattc 109200
   gcgagtgcgt cgcgagacac gtcttcggtc gagataaaca tgatttcgat atcacagagt 109260
   ttttgtccgt agagtataga ctgtaaaaat attacaagta ccggtgttta tatatattta 109320
   cagattatag tagataacaa aacgactaga tgatgtcata tatatttttg tagatggaaa 109380
   ctagattgtt gggtatgcac accggtatcg agcgcaccaa tgatgtttga ttatagtaca 109440
   ttcgagtgta attcgagtca cagacgcaat cctcgacact aaaaggtctg ttgactagat 109500
   ctaaagagag atttccgttg cttacgacgg catctttata accacaaata tagggtctca 109560
   agtcacccga atcgttgaca atgtcacgat aagtactgag acaagtattg tatacgagaa 109620
   tgtcgtcagc gactatggcg tcgacgagtc ctcttgatct gtcgcaaatc gattcgatcg 109680
   aatcgggacc tctgtacggt atcgaagctg tacaaaagcc ctcgacacaa tcaaattgat 109740
   tgatagcaat ggcgcaattg ttagtgcatt gtcgcttcga cacgcacggt aatctagtat 109800
   gcgaacaatc tacaataccg ttttgttcgt atacaaactg tagtgcagat ttacctgtat 109860
   ttttattggc tctctctaca ttttgtattt tgttttcagt catcatgttt atagttttca 109920
   ataaataaaa acatactatt agtaaagcaa tcaaaattat tattataccc agcgaaattg 109980
   atgaaaagga cgacatgatt gttgcctaca ggcttatttg aaaataaaac gatttatatt 110040
   tataatatag agtttattac aaatcaattc gataaaatca cgtaaccatt gttcaatcca 110100
   tgattaaaca ggctgagcac agtattgaaa cattcgtcgc atttagtcga atcgtacagc 110160
   ttaaaaagcc aagcttcgca aacgctgcat cgagtctggc catccagatc cggcgtgtcg 110220
   aatccgtgtg atatcgaaaa taattgatgg ttagatttca atatgacgtt ggatagatct 110280
   tcagcggtaa actttgaagc gcaattgata cagtaggtag cgtctttaaa aatgacgcat 110340
   ttcatcacga cacatttgta cgtgtcggca atgttaatag taatctcatc gacatcaact 110400
   ccttggttta cgacatcaat tttcgagttg tccgccatga caaaggtgcg atggtgtggg 110460
   aatacagccg ctaacgtact gatgctcgag cgacaaacgt atcagcattt atagtcgtct 110520
   cgacctaatg ataagacaaa atttaaattt atcgcaactt tatcgacatt atcataatca 110580
   taatcatcgg catgtctggc gaagaggatc agaatatttt tcttgttata cgcgaagaca 110640
   taaaaaatgt cagtattcaa gttcaagatg ttgacgataa ggctagcgat tctaatgcta 110700
   gtctagtaga aatagaaaac tctataaaag cttttccatt cgattcgttg gctgctcaat 110760
   tgaatgtgat caacaccggt gtacaaacga caaacaccag tataagtcag gctaatgcgc 110820
   aattaaatac acttcaaaag aacgtcgacg ataagctaac cagtctaggt acttcggtgc 110880
   agggagtgag cgcaaaagta gacgccggag ttgctagtct cgcttctcaa ttcactgcca 110940
   ccaacactaa aatagacggt gtcggcacta aagtagattc tggtgttcaa accacttcag 111000
   ctcaattgac agcctttcaa aaggatacaa attctaaaat tgatactctc ggcactagtg 111060
   tcgcatccat atcgacacaa ttgacagctt ttcagaatag tacaagagct agtttagaca 111120
   atattacggc tttgctgaat gccataatca aaattcttaa acctttagga ctgttgtagt 111180
   aatgggcatg gacacgtatt aaaagcatca agattattgt gtttaaatat ttaatagaaa 111240
   tcaaaaatac attaaagtaa aaaaactatt aaattatagt aaaatgaaaa taaaatcaag 111300
   aaaaacatta aagtcaaaat tatataataa aaatcaatat agggttaaaa ttattagaaa 111360
   aaaaacaata cattaaaaat taattgattt aagttgtgtc tgctgacata aagaatcgaa 111420
   atgtagttta attcttttta cagctctact ttcgggttta tacatgtgtt ctaaccacca 111480
   attggatatt ttgttgatgt gacgcttgcg ttcacaacgc aaaacctgcg tgttaccagg 111540
   ccacaacact atcgaaccgt caatagactt tatttcgaat gtagtagcat tgttggtgaa 111600
   tagtttctct ctcgggatgg tgtgcacatg agcagcaaat ttatgcatgt tacaatattc 111660
   ctgtataaag tcatcgggga atatgttttc aaagaaaagc ttcacaatta aagggtcgtg 111720
   gtcttcgttt tcttcatctt caatctgttg tttattagca cataatgtat ccaaaaccgt 111780
   atcgaatgtg ctgtgatagc ggttcaaaaa agtcttggtc aatacatact cgtatgtaaa 111840
   gttttcatta tcgaaaatat gaatccaatt tattttgtag ttgatgtcat catataggcc 111900
   cgtgaataac acccttttgt ttagcgtata aatgttcaat aaatcgataa acttccataa 111960
   ttctggatat ttattaatgt ttccgatgac atgttttctc gtttgattct tggtacgatc 112020
   gacaaaagta aagctattat tgtgttttga tgcgtacgat ttaatagcat cggtcaacaa 112080
   catggcaggc caaaaattat tacacggatc gatggtgacc ccttcattat tattttgata 112140
   ccatttatgt acgcactcgt ctacagatcc tttgtcgcaa taaaacgtga cctcgtttct 112200
   attgatccac ttatgtagat tggtcataaa tttctcgaca aaaaaatcgt aagacacatc 112260
   aaacttgatt tgttcgatcg tttccttgag accgggcgtg gtaaagctgt ccacgtctcc 112320
   attgtacacg agccactgtt cgacttcatt attatcggga tatttagaag cgaccgcttg 112380
   gtatattaga cgttcgtatt cgtcgacgat gggattttca tgaataccgg tcaaaaatct 112440
   gagaacctta tggccatagt atgtgatttt gtcgcccgaa ttcaattcct caatataatt 112500
   atatagttcc atagattcct cgtcgtcttc ttttatggta tcgaaaatca gataattctc 112560
   gtcgagatat ttccagattt gcatatcagt ttttgtagta tctttaaaca gtagtttaaa 112620
   ttcttcgaat gattcgagct ctgtcaagtt tttttccgaa agatattgct gcttttttct 112680
   ttcgtgagca acattttcca ttgctctatc taataaagga aaatcttcgg gaggtgcatc 112740
   taacaattct ccatgatatc tatcgaatct ttcatcatga ttccgatttc tatgccattt 112800
   tttttctttt tgatcgaatt cacgggccaa attgggttga actgccgtcg gttcgtacaa 112860
   ctctactgtt gtaaattctt tctccagatt catgtataat aaataataac tggtgatata 112920
   gatgccatac ctttttggct cagtcgattc tttctcgaca tcgtggtaat tgtaccgatt 112980
   gatacgtaaa aacgcatttc cgttacaatc cgtgatgatg tcttccacat atactttttc 113040
   gatttcacac gaacatgacg gcacaaacac tttcagattc ttggcgttaa acgcttcgac 113100
   cttgtaaatt ttattacaac acaacatgga cacgaccact cgatggatcc aatgtccccc 113160
   gacattatcg cgattagccc gttgaaattt ggcaatttcg gttatttgct ccatagtcga 113220
   gtcgcttgac agggacaata aacattttta gctcgcaatc agctttatat aaattgataa 113280
   atatcacatt tcacagatta gatcagatta cggctaagta ctaaagatgc tatcaacttt 113340
   agacgcaatt atgttacctg acgagattag ataataaagt gggacgacag attagatagt 113400
   aaagtgagcc agattagtta ttaattttgt atattaaata aagtaaaata atatagtata 113460
   atattaaact cattttactc gacgatcggg catttgtaca caatgaaaca aattattttc 113520
   caatatattt tttattgtgt acaatctttt ttaatcaaag tcaattatcc tgtattaatt 113580
   gtaaaatatt ttgaatatta tggttttggt tattacataa agaagattca atacaatata 113640
   gttttactaa ctgttttaat tttaattttg catctgaata ttctttattg ctattaaatt 113700
   gcacccattc acctctaagc ttacgtgatt tatattcatt taacaataaa gattctatta 113760
   catatccttg agtcccggta tcttcataaa catcatattc tagtaaatca aatggtcttc 113820
   cacaattaag ctgtttaagt ctattatcaa gtgaagtagt ggtgccgatt ttgtaaatat 113880
   gtttctgttt atattgttca tttgtgacca tataaacaaa tgatttgact ggtacgtagg 113940
   ttgataattg tcgattgtgg tacgattcaa atgaagacgt taaactatta gttttacgaa 114000
   gctgtggcaa cacttcttca aatagccatt tttgaaataa gtctgctttt ggtagtctac 114060
   atcgcactat taatgcgtat acacctgctt cactgataaa cactgtgttt ggctgccagt 114120
   ttgctggtaa ctgcgactcg gctgatgtcg ttttatcttt agcttgcttc gtttcaaggg 114180
   gatcctgatt atttaggatc tttatctctt gccaggtccg acgccattga ggttttatat 114240
   ggtcatataa agcttttccg ggtcttttat accccaaaaa ctgggcaatg gcatgaccgc 114300
   cgtacataaa tacatctttc tcgactttga caatccacac atcgcaagcg acgccgccga 114360
   tctcgcattt tttgttgacg agagacatga tttggatacg actcgaatat gacgttagat 114420
   gcaatccgac gataaactaa tcttatcggc acgaaacttg acgactgaat gattaaatct 114480
   ttgacgcgta atcgattttt atagtgccgt cgaggcgtga tctcgcgact tttttgtaag 114540
   tataataata atgaaagcca tttgtgttat cagcggtgac gtgaccggac aagtattctt 114600
   tttacaagag agtcccgagc atctactaaa gatcacgggc tacattttga acctacccaa 114660
   aggcttgcac ggatttcatg ttcacgaatt cggcgacacg agcaacggct gcacgtctgc 114720
   aggcgagcat tttaacccta cgaacatggc tcacggtgcg cccaactctg ccgttcgtca 114780
   cgtcggtgat cttggcaata ttgatgctaa aactcaccaa tctctgacta gtatcgataa 114840
   aatagattcg gtcatgtctt tgtacggacc ccactcgata ttgggcagaa gtctagtggt 114900
   tcatactcat cgtgacgatc tcggcctgac cgatcatcct ttgagtaaaa ctacaggcaa 114960
   ctcgggagga agattgggat gcggaatcat tggcgtatct cgataaaaat aattacacac 115020
   aaaatttctc aaacaaaaaa atttattacc tccaccaaat aataatgata ataaagtgaa 115080
   caaaattata gttacaggaa atttttacaa aaaatcgtcc acatcactat gttcgctttc 115140
   gtaaaactga agttcgctag attcatcgga gtcggagtcc aagtccatag aaaagaaact 115200
   acaagtataa tgattacaat atctgcaacg tatagaaggc aaatcgttat atctgaggca 115260
   gcttaggcat tttaaatcgt aagcaaaggg tgacaaaaag aaacgactgt acaactcaat 115320
   caactgcacg gccatgtaat gaggaatata tctagaaaat ttagtaacga attcttgtct 115380
   aaacgctacc cgttcttcga tgttgtgcat atttatcttt ttcttgatag aaaaaaaaat 115440
   tttcttggca cacatcaacg acaagctgtc ttctttgcgc tgacgacaac gaatataccg 115500
   atcacaagct ttagaattca tttcaatgtc ccatcgccag tttaaaacgt ttatttgttt 115560
   gaacattcta aacacatttt cgtaaatcgg gcgtggtaaa tagggcttca tcacatcttg 115620
   aaacttttgt tctacttctt cgagcatcaa gtcatcggga atatactttt catagtattc 115680
   tgtaaacaca ctattattta acgtattcaa caggttctgt gcgcacaaga gctgcagaga 115740
   agatacgttt tggtgtccac gatccatggt ggttgacaga cttgttataa tgctttctgg 115800
   cgacgagacc cgatatttat attataattt atctaatttt atctaatcta tacaataaaa 115860
   caatcaaatt tatcaggaaa aaagtttatt taatcaataa tttttatact tgtatccggg 115920
   agcatatgaa aagctggtag atttttgaaa agccatatcc ttactcgaag acgtggagta 115980
   gcggcaattg caattccacg atttgttgtc caggcaccgt ctacattttt cattgtagtt 116040
   actattaaag ttgttgaaga aaatcactct ggtcgcccac gaagtcatcg aaaatgaagt 116100
   ttcttccggt ttcttcatag tgccggtgga gctgctggtg ctgggcttgt tagagccggt 116160
   ggcgtacgag cctttgccag tcttgcaaat gaggcacatg attgagagtt atgattgcaa 116220
   ctgcactgtt tgcaactgta tgcatttagc tattcagtat gcgcttttat agtgtgaaaa 116280
   aagattagat aacgagataa cacgagataa ctagtagatt agcatttcct caacattaaa 116340
   gttttattag ttgaagatag aatcgaatgc aaaaaatttt gcccaatatc tcaagtgaat 116400
   aaaataatta aattatcagg cgtcaactac aaaattaaat gtttagcgaa atattgaaaa 116460
   tcatcaggcg ttcgcgcaaa ttatcgattg attgtttgct acaataaaaa tatcaggaac 116520
   caaggtaaag agtaaaataa aacataattt aaataataaa agtttttatt tagatgggta 116580
   caaattttat tttagtcaca aacatatcta agaggaattt tatagtgccc ggatagacat 116640
   tttttgttat taagtttgaa tccacagaac tttttttgac ggcaaaaata tatgtagttc 116700
   tacagatggg gcatgaatta tgattttgag accaaatctt gagacatttt gtacaaaaca 116760
   cgtgctcaca gttgttggga tgtgaatagc ttcgacagcc tcgtcgacga atcttttgca 116820
   cacaaatagc acactcgtcg ttcattttca gcacaacatc gcggacgatc gacggttttt 116880
   caattataat atcgctgact tgcaggacat cgaacaagat tctgttatgt ttgtcaaaac 116940
   taatgtttaa atatttctca tgtttcttgt ctacgaattg agcagaatca attattttcg 117000
   ttcttcgttt aaccacatat ccgttggggt tcaaaaaaat tatatagtca aaattaccaa 117060
   tcttagccac aaaacccacg ttagacatgg tgacttttta atgtacaaat gaatcgatcg 117120
   ttaaagagat cgctttaaat agtgtatcga gatttattta tcggtaatca tgtatactac 117180
   acacacataa agaatacaac aaaatatagg aaaaaaatat ttattattta ttaaaaacat 117240
   ttcattggcc ggtagagccg aaaccgccgc tgcccctgtc ggtgtcgtcg agctcgtcga 117300
   ccatgatcac gttgggtgta tgataggact cgatgatcat ctgcgcaatc ttgtcgcctt 117360
   tcttgaaatt atactctttt ttgcttctat tgatgagaca aacaccaacg gggccgcgat 117420
   agtctgtgtc gattacgccc gcttggatgc cgatgccgtg cttggttgcc aggccagaac 117480
   gctcagcgat gcgaccatat cgtttcggag gcaattgaat ggcgattccg atatcgacaa 117540
   ctgtccattt ttgaggtttc agacacacat cggtgggagt gtagagatcg tagccggcgg 117600
   agcccagagt cgcacgttga ggagcaatcg catctttatt tagcttctta aagtacatat 117660
   tctcgtgttg ttcctgatga tagtattcgt tgtcggaaca atcttcaatg ctcaatctcg 117720
   atcgcttatt ggcggtagac attattttcg cagagaggtg tggtgtgagt gtcgtcgtaa 117780
   gttactataa atatgactga ctgacgaccg gactctgttt atataccaag tgaatcgata 117840
   aatattaatg atatcgtaat atgtcgtcat tgacgagatc aactgattta tagtcatcgt 117900
   atgttcagga cgttgacgtt acaataaaat attttactcg tcgagtcgag agtatctatc 117960
   gttcaataag ttatttataa aattaaccac tattcgttac gatataatgt cgttgctttc 118020
   tagaaaatac caagatgtcg acattgcggc atatctcgat caatcgtatg tgctgtgggt 118080
   cagtgccgat gacgttttac agattttgag gttgcccgtg tccgttcttc agaccatagc 118140
   tccgcgacac aaaaaatgct ggacagattt tcgatgtccc actcagtgca gattcgacgc 118200
   gagcaaagtt tttgtagatc tctacggtct tgggaaccta tgcaatcgtg tcaattcgag 118260
   ctgttccgat tatttgatga ctctattcgt ggccgaagtc tatagagatt gcttgcctcg 118320
   tcgacgatca tcgggatgtc ggccgcgcag acgatcgtcc ggaggtcgca gacgctcatc 118380
   gggatgccga cgaaggtcct caggcggacg aaggcgttca tccggcggtc gtaggcgttc 118440
   gtctggaggt cgtaggcgtt cgtcttgttg cagacctcct caccaccatt atcatcacca 118500
   tcataacgag ttactcgagc gtatttcgcg acaaaacgag attctgctca ccacgctcaa 118560
   ccaactcagt ctaaacaata gtaaccaaca tctcgagctg accaacattc taaacgcgat 118620
   tcgcctgcaa aatgtggcca tagctgctca attaacgcaa gtcatagaaa ctctcgacag 118680
   tcagttgggc gaattgacga cggatttaag acgattattg aatgagctcg acgctcgttt 118740
   cgctgctctc acaacggcac tcaccaatgc catcgctcaa cttcaagact cgatcagagc 118800
   cgaactggtc ggtatcaatt cgatactcaa caatctcacg tccagcgtca ccaatattaa 118860
   cgctactctc aacaatctat tacaggccgt aaacggatta aacattgaag gtctcacgac 118920
   actactcaat accattttag ctttgatcga ggagattcta gggatattga cacctgaaat 118980
   tcctttaaat aaaagatcct aaatatatgg gaataaaaaa cataaattat ttttaacaaa 119040
   aattttattt gagcatacta aaaatcttcg agaatttcat atgtttcgtt ggccgctccg 119100
   aaccgcttgt actcgcccac acgtttttca aagaaattag tcttgccctc aagagatatg 119160
   ttattcataa actcgaaagg atttgcagtg ttgaaatgct tgtcgtagcc caactcgaac 119220
   aacagtcgat ctgccacaaa ctcgatgtat tcgcacatgg attgcgaatt catgcccaac 119280
   atagcgaccg gtaaagcatt ggtaaagaaa tccttttcaa tgtcgaccgc ttctacaaac 119340
   atactcaaca ccttggtctt ggagagcttg ttctcttcgg gaatatattt attgtagtat 119400
   agacaagcaa agtcggtgtg tagtccttcg tctctggaaa tgagttcgtt gctgtaagtc 119460
   aagccgggta gagctccttt tttcgagtct ttgatatagt agattgcagc aaaactacca 119520
   gaaaaaaaga caccttcgac gatagcaaaa gcgaccatgc gttcggcgaa tgtggcagat 119580
   tgattttcga tccatttttg agcccaagcc gccttctgct tgacactatc tatatgctcg 119640
   atcgcattca aaatcctgtc gcattcttgg ggatctttaa tgtattcgac gacaagaatg 119700
   ttatacattt ccgtgtgaac acactcaatg aatgtctgtt gcgcatagaa aaaactagct 119760
   tccaattcgg gcacggcgga ttgcatgtgc tcgatgatgt tagtgttcac gatgacgtcg 119820
   gcggcagcga aaaacgcgag aacgtgcttg agaaaaaatc gttctttatc gttcagtttc 119880
   ttgttccaat cgtcaaagtc cttggacatg tcgatctctt cgactttcca gtgacaatcg 119940
   accgctttct tgtacgattg ccagagatct tcattcacga tgggcaataa agtcttacgc 120000
   gaaacggacg acatcgtcag tgagtaagaa gttttcttcg atacgagacg agtgcttgtg 120060
   aacaagtgcc gctcgagcaa atgtcaaagt gataatacaa atttaacgat cgttctcgac 120120
   tcttttatac aatcatgatc gaagatgata actatgctct acccgtgctc tccttcgata 120180
   acgaggctta caaagtgtcg tggcaagaga tatcgttcga tccgtacaga caagagtatt 120240
   tgtattctat acgagtagaa gagctcgaag ttcgttacgt ctattgggac gataacgcgc 120300
   gtcgaatgaa tctgctgagc accggtcagt ttaagattcg catcggaaac gtgtactccg 120360
   aaccggagac ggtcgagttt aatgccgacg agcaattatt gaaattgact gtttattcgc 120420
   cacagaaaaa ttttaattca atcaccgtgc acttgaataa aagatatttc gtcgacgatc 120480
   gcagaccgtg gcaagttcca cgttggatca aacgtgtctt tggtccgtta taagaaatat 120540
   ttaaattact tcaagcgcaa tgaacgaata cagtacatca tttcgagcgt accgagaatt 120600
   gttgagcatc aaacgtgctt tgtggctgta cgtgtcgttg gtggtttgcg gctttgccat 120660
   tttcgtcggt ttgagtgttt acgccggcgg agtcatttac gcccaaattg aagacatttc 120720
   tcaaactgtt ggcgcgtcag cccgcagcca aaaagaacta caaagcctca ttatagaatc 120780
   tttcgacaac aatctcgagg ccgacataca attttcttta ataattcgct cgagtgcctc 120840
   ggttcaactg aagatcaaca cttatccagc acgaagatcg ataacgtttt ctgaagaacg 120900
   agtgacctgt cccggtagtg tccaatatga aaactacata aaaacactat acgatcgaga 120960
   cactaaaccc gatatacata acatttatct gttctatgga ggccatggac tgggcaaatc 121020
   gtttgcatct cagcaattgg gaaaagcctt gagtaaattt ggaaatgtgg tggtcgtgtc 121080
   agtaccgatg accagtttcg taggagtggt cggcttcaac acgataggca atgtcataca 121140
   atctatagaa tccgtctgtc acggttgcta tgtcatatgg atgtttgacg aattggattc 121200
   gtatttgata gataatcgcg attctttata ccagagcaaa agcatcacag aatttgccga 121260
   gtacacgggc tttgtcaaga atcataatcg aatcctagcc tttacaatga acaatggtga 121320
   gattcttaag catgactatt ggacgcatca agatgaaatc gtcaacggca catacgaata 121380
   cgaacgtgac tttagaaagg ctctcgtcaa aaccggcatg agttcgactg attttcttat 121440
   ggacggacag ctgagcagac tgcgatcgtt cgtcggcaac aaaatattta aatttaaacg 121500
   attcaataga gagacggcaa agcgattcat caacatgtat ctacctgacc ataagcagct 121560
   atcgtggaac agtcaaatcg aaaataaact gttcggtaac gaaacaactc agacatatga 121620
   tgtgagaacg ctggtgatcg ctctcgatga tgtactaaat ttaaagaaaa taaataaaac 121680
   ttaacacatt ttataaaaaa ttttatttca ttcctctaga ttgtataatt caaattgaac 121740
   aaatcgttta aaatacacat aaatctgtac caacatttgg taaaataatt attttcccac 121800
   aaatgatagg ggaagttgac ctgatgtgta atatcatcaa tattattaac tagtctatcg 121860
   tttagatata aatgtataat gagattcgag cccaatgtcg gattcaagag atacaacaag 121920
   tttttgagat tcttgatttc aaagacagca ttcgagtttt ttatataata aaaattttgt 121980
   agttgcatac gactattgac aggataatat ttttttccta gcagtttcct aagttttatg 122040
   attctattct ttattttgta atcttgatag ttgaacacgt taaacattag acattcgatc 122100
   tgatcggcaa tgacattatc gatatattcg acggtaatat tcgtatcgta aagcggcttg 122160
   atgagatacg ataaaggttt gttcatagcc gaattcatgg cactataatt ttttatagtc 122220
   aaatcgagaa aatagacatc aaacacgaaa tagttcaatt ccttgatgtc cgatcgtcgg 122280
   tataaacgca tcgatgactg cgaagtggcg accatgtgca cgttaaatga gtatcgcact 122340
   aaaaaatatt cttcgttcac tttcgaaacg gtggtcttga aaggcatccg gtgattcaac 122400
   cgcaattcga cgtcggccac attgaattcg acggcttcgt tgacgtacga tttgaaaata 122460
   attacagatt tttgacagtc gtctgtgctg tcgtcgttgt catcgtcatc catattatac 122520
   gtttgtttgg tgtcgttggc cgcatccttg atgagttttt tgatgtcaaa ttctcgtaaa 122580
   attttctcga ttacaccaaa atatttttgt gaaatttttc gtaaattttt ttcgaggcta 122640
   gttaacgcca caaatgacga caaattgtcg atattgactc gatgattgaa gtcgacgtcg 122700
   tcgacgtaaa tctcgatgtc gatgtcggcg agaaacgggt ttatttcctc caaatgtgcg 122760
   gcaatggcag cgccgccttt cacgacggta aatgttttca gagaactgta tattttttct 122820
   aaaatttctt gatgaaattt cttcgccggt cgattgtcgt ctaaaaagcc gccgtcgact 122880
   gctttattgt tgttttcgtc gactacgacg gtcgggctat cgatacgatt caatttttga 122940
   agtactctgt cgctgagaat gtcgttgacg agtttttttg aaatgtaagg gatggttcta 123000
   tggtagtgaa atcgttcgag tatagactcc ataatgaaat caaataacga tcgtattaac 123060
   gtgttaaaac agcatcggca gcttactatg ccctcgttaa tgcgtattat taattcgatc 123120
   gatacgtcta agcgcgacgg acgtcaacta ttttataaca tttgttataa catcataacc 123180
   agaatcttga gtagatcgac tgcaatcgaa accatcaaaa cgatcgtttc gatcattata 123240
   gaaactgaaa aaactatttt caaccagagt aaagttttaa actacatcag aaactttttg 123300
   atcgttaaca gtgatggtca taatcttcag tgtactatta atttaaaatt actagattat 123360
   attttaaata gatataacat cgaagaagaa aaagaagaac aaaacgatta tcgtcatcaa 123420
   taaaaatgtc atcgctgctc accgccgaac agaaagagat ttacgacaaa tattgctatg 123480
   tcaattatgt ggccatgcta aaacacgacg acatgaagct gacgcaaaac gaaatcctga 123540
   gagtggaaaa aatgactcgt ggacaatctg aaaatcccct ctggaggctg cttcgactca 123600
   atcgacagac cgcttcgaac agcaacagca ccagcataat gatttcgccc agtgcagcaa 123660
   tgtgtcatgg tatcaaagaa gaaaaagtag taaaggacaa taaaaattta atgaatgaaa 123720
   ttcgtaggtt aatcgaaata actttaaaca cgattgtcac aaaagagatt ttcgactgtg 123780
   gcatgtttct ctccaagtta ggtctgcatg ccgcctctcc agacgcatac tacgttacga 123840
   tcgacaatgt ctatgtgccc atcgaaatca agtgtccata cacgtacaaa gacttaacat 123900
   acgaagatgt acgaaaatct ttacgaaaaa atccaaactc gaaaaaagaa cgatatagaa 123960
   tcaaaaatac tgcattgtcg gtcaacgtgg aaggcgtccc gctgtttatt gtggaaaaaa 124020
   ccgatcccca ttatcgccaa atgcaacgac aaatgtatgt catgaatgct cccatctgtg 124080
   tgtatctagt aaaatttaaa gattcgtttg tcgcttgtac agtctatcga gattctactt 124140
   catacgatgc agagtccaga gacgaaagaa aaattttcaa catgtttatc aatcgaaaca 124200
   agagaaatct aatgctaacc gatcagtaca agcgaaaagc gagcttcgaa ggcaaacatc 124260
   taccgcaata cagcgagctc gatattcgag cgttgaccag aagcggactc tatttcgatt 124320
   atggacaact cgtctgtata ttttgccaga tttgcttcga tgtcgacaca ccggccaagg 124380
   aaattctggc aaaacataca gattgcgcgt cgaatagcta caacggcatc gactttcacg 124440
   tcgaacatcg agcttatatc aataactcga ctcgctataa gactttacca tacgaacaca 124500
   ggcacttggc cagtgacggc ttgtttcttg atcccatcga ttctcaatat aaaatgtttt 124560
   gttgtggcct aataataaag gactttaatg ttaaacatgt cgtcgaatgt gaatatagta 124620
   tgataattga tgataaataa aatacattaa taaattttac acaatcgtat ttattacttt 124680
   ttcctttacc acatgtacag tatagagcat aacataaata gttaacaaca gtagaataca 124740
   acaagaggag agtttaaaca tttcttggcc atcggaccat tcgagaaatc tgtacactgg 124800
   atttcctgtg taatagttat aaaacactaa aaagaatcca tagaatagtg taaacaacag 124860
   cggctgatat acatgtttca ggcggacggg cactgacgaa atacaaactt caaagagcac 124920
   cacgagcgaa ttacaaaaat gtatcacatt gttaacgggc gaactaatgt tattgtgata 124980
   tttatagact acagtcacta taaaatatat tgtactagtc agcagattcg acgaacaggc 125040
   cacattaaag aaaaaccatt gtacgtttac gtaccaaggc aatctataac tatattcggc 125100
   ttcggtgaat cgtccaataa atgtggtcat agtgccgacg acaaacatga ccaacaatga 125160
   gatcatcgac caatgtgaat agtacagaaa aaattccgct ttatctatag ctacataata 125220
   tcccgacgcg attagcaacg acatgatcaa tacggatgcc gttctcgtgg tcagcataat 125280
   cagttgcgaa taatcgcgcg gagaaaccaa acagcaagag ctaaagcgat ctgtaatgtc 125340
   tccgccccag atcatgttct gttgtcgctg ccgtttgccg cccattaaat ggatgctgtc 125400
   cattctcaac cgaatgagac tgtatgtatt aaacgtaaaa aattccactt aattgtgact 125460
   cgatgttcac gattcgtacc gttttatacc acagttcgta ggtgtcgtcc attaaaacga 125520
   ttaaaaaaaa tctatataag tattatttaa cataaagtgt gggtgtcgac atcgacaacg 125580
   accttggcac cgaaacctat ataattcgaa cggctgccga cgaccgagac gagtcgcatt 125640
   cgagcgaacg gacatacgac atcatttagc gtgtcaaatt accgcaaaat gtggaaatcg 125700
   ttattagtgt cgttgctttt gtcgatgacg gtggcagaga tcgttcatag cgcgccagcg 125760
   ccaatgaaca cgactttgat atccatcgat gtggacgatc acgacgacgg cggcggcgac 125820
   gtcggcggcg gtttaccgtt aaaggccggt accgaaagat tggtacagtt ttttatcaat 125880
   cacaagtatc tgcacatgca tgtcgatggc gtgataaacg gctcactcga cagatttaat 125940
   aatgagacag tgtggagaag aatagcggta ccaataccag aggaaactac ggaaaaaact 126000
   actggtattt tgatccaatc ggctagcaat tgccgttatc tgtgtatgaa cgagtgtggc 126060
   tacatgtaca gtgccgaaat acccactttc gagtgtattt tacaagaaaa aatcgaagaa 126120
   agtcgatatc atttaatcta taaggatttt aatagacgtc atgcgtactt gtcgctgaat 126180
   ttagaaggaa aattccgtcg aagcgtattc aaaagcaagg aaccgatcgg cgatgccata 126240
   atacgcacaa acgtggccat cgaaacttgg actggtgaaa aaatttcgaa tacgtgcggt 126300
   gagttgattc gtagcaaact caattatcgc attcgaaaga catgtcgaag tgcattaaaa 126360
   aaaagactga tcagcaacga aattccggcc ataggagaca aagttggcat cgacgacatc 126420
   ataagcgaga tgattaacga caacagcact ctagattctg ttgctgttgc gatgccgccg 126480
   cctccgccga tacccactct agaaattatg aatagtactg tcgaggcggc aacaataaat 126540
   tcttcgagtg ttgcctcgac gaatgcgact aatgcggtgg cggttgtgga ttatgtcgac 126600
   gacggtgacg acgacgacga cgacagtgaa gatgtcgaaa tcactatcga aggcgtcgac 126660
   ccgaaaaatt tttatgtgcc ggattcgtcc gattcgacgc tgagcattca tttgctaaac 126720
   tcgactacag ctcggcctcc gactactcct gttcgtcgtc gccagcccgt gatcgccatc 126780
   gaagacataa tcgatagtat gatcaagatt cctgtcgaaa ccggcggaac atcggacgtt 126840
   tcggatgcac gaaatgttct ttttaaatat gaaaataatt tctatatcaa taaatgtata 126900
   aacatgtaaa tctgtatatt gtagatgtat ttcaattgga ttgtaataaa gactttgaaa 126960
   tcaaattttc attcgtatta catttattcg tagccaaact acactgagta attactatag 127020
   gtattgagta tttgagctaa attattatac tggctttcgg gcaccatacc gattgcatcg 127080
   caaatgtgta tagttgtagc atttttagtt tcaagtacac caaatactgc gctgaccatt 127140
   aatgccacag gaaattggcc tctaggatat cgagtggcag gactccaaat tttaatccat 127200
   cctccttctc tgctaaaata gcaccaagag tttgtaaatg tttctgcggt gccgacttga 127260
   acagtgccgt gtctgcgtat acaaagatcg ggttgatgta cgatgcatct gcctctaccc 127320
   ggatagaagc atggttgagt cgttcgtctg gctaacttta aaaacctaac atatatactg 127380
   tcctttagag atgagtcgac aaagatttca tgtggactgt aagcgatcga aaattttatt 127440
   gtagaaagaa tcgaatttgg aatcggctgc agcagcctat ggtatctgac atgactaaga 127500
   ttatgcctgg taacgtgagc aaaaatatcg tcatccgaca gtaaattgtc gcttcttccc 127560
   caaaaaaatt tatattgaat ttcatgtaac gcatcgatgg gaatattaaa cggctgtatg 127620
   cacgagttga caatagaaat attgtctctc ctattcaaac gagactgtct cagcatactc 127680
   gcgccgccgg acgctattcc gaagagattg tcttcgatgg gacaattaca aaaactcaat 127740
   tcggttccgt cttgatcatt tttaaagtac tggagtcgtc cagtcgtacg cctgcctgtg 127800
   atcggatcta ttgaacaagg atcgacaaca caaatggaag tgagtcgtag tgtctgtcga 127860
   tatatcggat cgagaccagg atgttcgatc gaaacaaatc cgtctcgaca cggggcctgc 127920
   tcaaaaaaag tcctataatc ggtatctcgc acacgcatcg gtcggcaaaa tggagtctgt 127980
   gtctgagtat ctaactctga tatgaatcct tcgtcgcaga cgcatctcat tggcgattcg 128040
   ttaatgttta gaatacgtcc gttcggttga catcctacag gcacgttaca atcagaatag 128100
   agattcagct gagtgactag acccggcgac gtgcagttgc agagaagcga atagccaatt 128160
   tcgtttctag tcaaaatcca aacgcccgtg ttgggattgc aggcacgagc ccgttctcta 128220
   ttgagcgcca tgcaatacga ttcgcccggc tgtatcgtat actcgatgtc gttcatagtg 128280
   attttagtag gttcgtcaaa atattgacag ttggccaaac cttcacgaca aatatcacaa 128340
   tcttgatgtg aagtgcatgg cgtcaattga gagtgacaag acactggatt atcttcgagt 128400
   ataatctcgg aaggaggttc tattagagga acattcgagt tatcgaagcg ttcgagagga 128460
   tacaggacag gctcgtccgc atacaccaat acagatatat aatttgtcaa gagtaataca 128520
   attagaatta gtatgacaat agctaatatt aaatacattt tgttgactta cgaatatata 128580
   aaaataaaaa aattgtaacg tttattaata ttttttattg taatcacaag gaaaatagac 128640
   ggcaggagga caaaggttcc taatgtttaa atccgtttca agctctatac gcaaagcgtt 128700
   atatgtagac cagctccttg tgtgatgata tgtgccatcg ttaaactcaa cgtgtctgat 128760
   aaaatacttt cttctcacaa tcttcatgta taatattttc ttgcaccatt tagcatgtag 128820
   aattccgatc agtccgttgg ctatattatg ctcgtgggca ggcctcatga acgtgtataa 128880
   agcccatacg tttctcgacc actcgtgcga agccagaaaa tcgtatctaa gcttggtaat 128940
   agcacgtctt gttatacgca taccctcttt attacagata tggatctcga ttgccactat 129000
   tctgttgaat cgtttctggt gaaacgtaaa cagctcgtgg ccattttcca agatcgaaac 129060
   actttccttg ttgtagttat gaatcgaata caattggtcg atttcaaaaa ttgaaggcga 129120
   acgcaaagga gacaatatag ttgctagaga agccattgta tcggtcgaaa gactgtaata 129180
   tgatcgccaa acgaaagtcc atttatacta atagattaaa cataatcagc agataacact 129240
   ttatctgttg ataatggtat caattttttg gtttatcatg tcgattttgt tgccattggt 129300
   catgacgaga tcgtaagtaa attttgtagt gttttgaatt cggttcaaat aggacatggt 129360
   gtcgtttcta aatttggcca tatcgtaaac gatagagtcg aatctgtctt cgatcgcttg 129420
   ataaataaaa gtcaacagct ttttaattgc ggccagttcg taagctatgc tgcctgtctg 129480
   ccatagataa gctagcaaca gaaccagagc caacgcagag tagttcattt tcgtttccct 129540
   tatttttttt ctaatattat attctcattt ttgtttcctt atttttttcg aatgattata 129600
   tctttaattg atttaaaact atcgaccatg gtgtcgttac tgaatgcaag tttattagtc 129660
   aagtccgctt gcatgctctt cactgaatcg agagccgatg taatgttcga gtatagagac 129720
   gcattttcgg tgcgaatgat ttcgcgaatg gtgtcgacgg tgtcgtatcc gacaccattg 129780
   gagccgagca cattattatt aatattgact ttatcgaaat ccggttgata taattgtccg 129840
   ctattggtgt ttaaacattc ttgtagtgta cagagctgtg tttttatctc gttcagagga 129900
   tcaaccacct gactctgtgc acccattatt aaatcacaaa agagtcgctt caatacgaaa 129960
   tactgaggcg ggtggacttt tttactattc aaaacgtacg acgagaggta ttttcctaat 130020
   gaaaacacgt gtatatagtt tttattgttt cgcgtcagtt tttgggaagc attaacgttc 130080
   gtccagagca ctgccttatt gaaatttctt attgtcacca agggcgctaa cagcttagtg 130140
   gccgaaccta gctcgacata cccatctcga tcgtcatcgc tattttcaat aattaccact 130200
   tccaaaacct catcgtcata gtgaaattgc tgttgcaatg aatctgacat caccacgtta 130260
   ttcgtattgt ttggcccgtt cattgctgtc gaatcgttga gattgctcgt ttcgtacatt 130320
   gtgttaatgg cttaattgta gaattaaaaa tagcactaga tgattacgtt tatcgtttta 130380
   aaaaagtata ttaggtctta tcttttaagt taaattcgta tttcgaatat aattttttaa 130440
   atacattatg aagtctcaaa tgttggtgta cgacaacggc ggcctcgcca acaccaatca 130500
   tttgtatact aatcatcgac aaaaatctta tagtcccgtc gacagaatgc acacccgtcg 130560
   tcaatataat tatacaaact accttaatga tttgtcctat tcgccggccg cttggcataa 130620
   actcacgacc aacgaaagaa ccaacgttat ctacgaaaaa tgtaagttat ctcaatatga 130680
   tggggtcgat taccgtagat tacagggacg cgatagcttt cggtgtgatg atatattaaa 130740
   gattgtcgct agcccgcagc cttctagtta ttccacaact atcaatggag acacatcagc 130800
   agcaatcgtc tcgtcgggaa caacggcaag aggtgttatt atggacacca ttactgtcta 130860
   atctcgacga tatcgacaag aatgccgatt atagaatctt aattgacgat tttgacatag 130920
   atatcacgcc gtatacagtc tttgaaaatg atggagccac aataaaaata tctggattgc 130980
   gtctgtatta tttactcaaa aataaacgct tgtacgaaga atcgatggag actcagtgta 131040
   gtagtaataa aacttttaaa aagagtctga aaaaagtgtg ctttacaaag gctatccaag 131100
   gaggaaaaaa tagtgtagtt gctgtcatca agagtaaatt acgcttaccg ccttgtatac 131160
   agtctctact atcagacatc gatgtgcgac cccgaggaaa taggtttcga aagagattca 131220
   ttttcaattg ttatattgct aatctcataa cgtgtccggc gtgcgacaaa cagtgcatcg 131280
   tagacgccat ggctgttctc tatctgcacg acgacaagtg cgttcgtgaa tttgaaaaac 131340
   tactcaatcg caataaggac gtgtataagc cgcccagttg cttaaatatg caaaacaaag 131400
   aacgtttatg tcctaataaa actggcatgg gatgtaaagg acgtaatcct ctatgcaatt 131460
   tctaatttat atgtatcgaa taaaaaattt ttcgtttaaa aatgattttt atttataaat 131520
   tttcaaatat aaacgatttg actgtttcta cagaagattc aacttgtaaa gtgcgtcgag 131580
   ttttcttttt gtaattaaag ttcttcatat cgagctcttc gttgagacac agcatggcca 131640
   gctgaggatt aggatgaatg ctgtcgtaaa ctagttccat atcgctatat tttcgtttca 131700
   tagctctgtg tcgtctggat tggcccgaca gaaaagctat ctgagtagaa ttgttttcga 131760
   gagattttac gaaaaccgcc aatcttggat gtttagaaga atcgcgtgga aagcgcacgc 131820
   tttcataacg acacatttgt ctgtatcctc cgtcgtggtc gtcgtcgtcg tcgctttgat 131880
   tatcacgaga tttgaacgat aattggcaat catcggcgac acaattgtcg ctagtcgctt 131940
   caatattgtg accatcgact cgataattgt ttctgttgac tcgatgattg tgtctatcca 132000
   ctcgatattt ctcaagttga ctgtacaatt cttctgcaga tatttttttc tcaatttttt 132060
   caaaattttc acacatttga gctttgagcg tttcgatcgc ttgataattt ttagtcagag 132120
   tttctattgt attagtttca ttgttcctga caaaagcatc aaaaacgcgt aacatttctg 132180
   tcattttctc ttctatcggc aacgacgcca aaggcaacgg cttctttttt gtcgacaaat 132240
   gttcttcttt catggcatca aaaacgtttt caatcatcca tgtggtaaat tcaattttat 132300
   ttttaaaatt aacattatcg agaatctgca tcgcgccatg tttattgatg ataaaaatcg 132360
   ttttattgtc gtcattgtct gcgactgctc gagattcttt ttgaaaactc aacagctctc 132420
   ccacggtttt tttatattga tcattctcga taaagtttat ggccgcatcg atgtcgtcaa 132480
   gtccgatgcc ttcggcgaaa tcgaccgcca aaatccagat ttcgttctcg taaaatagat 132540
   agcgcaacaa aaacgaaaat tgatcatcga aacataatct ttttttattg aaaatatacg 132600
   agaatgattt catgtctcgc tgtcgattcc ttcgattgtg gtcactttcg tcggcataat 132660
   tgttgtcatt gtcgacagaa tcgcccgaaa ataataatct ctttataaaa ttaaacattt 132720
   tattggtaac acacaagaca catacacaca cactttacaa tatttggaaa gaaaactttt 132780
   gtcctttata attgtagcta aaaggtgctc ttatctgtga ttgagtacaa aacacgtgtt 132840
   tgtcgacagt cggccagagc ttgtgtaatg tagcgacatc ggcgctcttg ccacttatag 132900
   actcgatggc agatttgtac gagtgaataa agcttccagg ccagattcga tccagacgaa 132960
   tatctgcagg cttgacgaat atttggtaat agcgctctcg tagattcttg gaggctccga 133020
   tgcgaaattt atctatacgt aaccacacat gaatgcctcg attgcccgag tgcatgattc 133080
   gagctatgct gcttttgaaa aaatttctaa atgtctccgt cgccacttta attttcaagt 133140
   ctaaaaggtc tgcatcgtcg gcttcgatgt ccacgtcgat gacccattct cgaccgtctt 133200
   cgagcgcctt gacgtgaata tcgctgacgt tttgatcttg aataaattca aatagatctc 133260
   gagcgttcgt aaagtattgt ttaggatgat accaattgac gccatcggtg aacgaatact 133320
   gtctgctgtc gttgtatgcc acgttattcc acagcttttc gacttgctcg aatgtgtaga 133380
   gtctaggctg agcgtacatc tcggtagaat tgctgtcgac gagtggacga taagtaacat 133440
   ggcgttacat ttattgtttg ccgttctgat atcgaatgcg ccacgcctca acagcaccgc 133500
   gcatgacgat atcgatagag tattcgattt gattttggac gagatcacca aaattgagga 133560
   gaatgaagct gacgatttta attatactcg tttgattttc tttattcttt tactgataat 133620
   aattattttg atcaagacac gactatatcg ctatttgcta tgctgtaaaa attgtcggaa 133680
   aaaagcgatg acgattgaac caaaaacgaa ggaatctcca ccgacatcgc cgactacggc 133740
   ggcgccgaca gaaccgccga ttgttaaact tgaattttcg aaaaaaaata tttccgacga 133800
   cataagcgtc gtcgacgata aaggccgcga acctgcgatt aaattaatca atttcaagtc 133860
   tattgtcgat accgactaga ttatgataat tatgatagaa attgcgacat caattgtctt 133920
   ttgaatgccg gattcggatt tattacgcta cgtctgctcg tcacataatt atagatttca 133980
   tcgagattat agcccgtacg tttggtcaaa tagtgtatga ctatggtggc ggatcgagag 134040
   attccggcgt gacaatgcac gtaaacgcgt cttttctcca ggtcaatctt ttgtaacaaa 134100
   aaattaaaag atttcaaaaa atgagattgt atgtcggctc gttcgttgtc atcgatatta 134160
   atgtatagat agttgtcgat cgcgtcgatt atgtcggttt tgcccacaaa aacgggagga 134220
   tcgttccaga cggtcaacac tgcagtaata ttttcttggc gtaaaaattt ttcaaataga 134280
   tttcgatcgt agatggcacc gcccaaatac agattgtcgg tgattttgga cgcattaatc 134340
   agtattccgt ctccgtcacc cgctccgact ctaatgtaat tcatattgcg tacgctaacc 134400
   cgttttcgtg caaagattcg aataaacaca ccgtgtcagt gggtcagtgt gccatattta 134460
   aacaggggcc ggacgacaca cgacacacat catcgggtga cctaaatatc cttaaattaa 134520
   ctacacgaaa aaagtagaga aagtgcacaa tgaaaactat aatagtgctg tcgttaatga 134580
   tgatattagt cattctaccg tcgcaaggcg ccagaatttt ggcagtgttt ccaacaccgg 134640
   cgtacagcca tcagaccgtc ttcaaaacct acattcgagc tctggccgaa agaggtcatg 134700
   agattttcgt gatcaaacca tcgaccagag taaattacgt agatcggtac tctgatttga 134760
   atcttttggg aaatatcaca gagatcgatg ccagtctatc ggaagaatac tttaggcggc 134820
   tggtcaaggc gtcgagcgtt tttcgcaaga gaggactcat ctccgatagt agcacagtga 134880
   cggcacacaa ctatctcggc ttggtgagga tgatcagcga tcagttcgag ttgcccgaag 134940
   tgaaaaactt tatagcaaac cgcaaggaat tgcagttcga tctactcatt accgaagcat 135000
   ttatggacta ttctctagtg ttttctcatc tttttgatga tttgccagtg attcaaatat 135060
   cttcaggcta tggagtggcg gaaaattttg aaacgatggg tgccgtcagt aggcatcctc 135120
   tatactatcc gaatctttgg cgagacaagt tcaccgactt gaacgtctgg gaaacgatca 135180
   acgaaatcta tatggaatta cgattgcaaa acgagtttag taaactggcc gacgaacaga 135240
   ataaaatgct taaagatcag ttcggcggga gcactcccac tgtacaggaa ttgagaaatc 135300
   gagtagaatt actctttatc aacacacatg ccgttttcga taacaatcga ccggtaccgc 135360
   cgagcgtcca gtatctgggc ggattgcatc tgaaaggaaa tacgcaccgt cgtcatctcc 135420
   aaggattcgt aaaggaatat ctggacaatt ctactcaggg cgtcgtgtat gtcagtttcg 135480
   gttcgggtat caacagtgct gacatggaaa atgaattcct tcaaatgttc ctcgatgtgt 135540
   ttgtcaaatt accctacaat attttatgga agtacgacgg catgatcgag tcatccagac 135600
   tgccagataa cgtgtttata caggcgtggt tcgatcagtt tgaagtattg aaacatccaa 135660
   acgtgaaggc attcgtgact caaggcggag tacaatcgac ggacgaggcc atagaagcac 135720
   tcgtgccgct gatcggacta ccaatgatgg gcgatcaagc atttaacact aataaatatg 135780
   ctgaactagg catcggccgg aatttggaca cgctcactgt aaatacaaaa caattggttg 135840
   acgcaatcgt tgacgcatgc caaaatcctt cgtaccgcaa acatttatca caattgcgcc 135900
   atattattcg tcatcaagca atttcacctt tacagaaagc catatattat acggagcacg 135960
   tgattaacaa ccgcggacgt accatgctca aaacgaaagc tgccaacgtt agctatggcg 136020
   attacgtcat gtcatatata attgttcctt tcgtgactgt ttctattatg aatcatttgc 136080
   gtcaattgtt gagaataaat ttattttaat atgataactg tattttttta tgttccaaat 136140
   gtaaaatact acattttgta ctctattaca agggaataaa acctgttatt tcgttgatcg 136200
   agtaatctgt cgacactcga tttgcgcagg cgttgtcggc tgcgcggatt tggtttataa 136260
   acgactcgtg ttcatagtca tgtacactac aactcgaact agcgccaaat catgtcgaca 136320
   ttgaggaata aatgcttgat acgaagcata caaatattag agaaaaatta tcgacgggtg 136380
   tctgtagggg acttgaaaaa aatatcagag gccatggcga tactacaaga gtccaacggt 136440
   aaactaacga gaaccctaca aaagatgtct tcgtattacg cagaacgcta caagaacaag 136500
   atacagaacc ttcaaaatac gatcgataaa aaagatcgtc acattaaaga tttatacaag 136560
   caaatcgacg agaaaaacca aaaaggagtt tttatagttc gactcgataa atctgtatgt 136620
   ttcttttctt ctctcgagtc cctgaataaa acaatcaagc aatctgcgtc cgtacagaat 136680
   ataaagattt tattatatcg aagcgcatgc gacatcaaat ctgaacgatc ggtctgcatc 136740
   gcaatagcaa aggcgaaata caagaattac gtgagcgttg agaatcgaaa gattatattc 136800
   gtcgagagtg ccgatgtgga ctcgttcgaa ttagatatta aacagatgct cacctaaaat 136860
   atgtaaaata tgtagtaaaa ttttttttag ttaattttta gtggtgtttg tgtgtaaaga 136920
   ggctcgattt tcctagtact ttaaacaatc ttgcctatac aagttaacta ttgcttcttc 136980
   ggatacgctt gggtgttgaa ataatttact cgaatactag aataaaatta tttattgata 137040
   ttaatcgaat agacggacat tacattcgtt gctactatta gaataaaatt tatttttgtt 137100
   aaaatattta tcgtgtttat tcatcctatt agctttatta tttttgttat tgttagaaat 137160
   atatttgact aatgctatcg ttaaagctat gttaaccagt attaaagata tacataaaac 137220
   tcctaaaaat acaataaaat actttaaaat aatactgacg attgttttat tattttcctc 137280
   attctcgctt ttgttgtggt catcattttc tttgctaaag atgtcatttt catcgactat 137340
   aatcaaagtc gatggaggat taccagtgga aactattaaa ggaacaacag tactggtacg 137400
   aggtaaatga gtagtaggaa ttattgttaa aggtgttgtt gttgtacgag aacaaggagt 137460
   aataattttt cttgttacat ttaatatcac atttataggc ttatatacta gagatgattt 137520
   cttgactaca taactaaagc aatacttgtt tggattaaaa ttaaagtgtt cgatatactt 137580
   tttggtataa taatataaat ctaaaggagt attctgatcg atatttagca gagacgtaaa 137640
   caattttttt gaggtctcac cgcctctgct caaaagccgt tcgaaatacg gttgattata 137700
   catggcggcg agatgcacga gtgtgttgtt acgcaaatcg acaggcttca ataaattttg 137760
   taaagaaatc ttctcgtttc gcagtctggg tatgttgttg gcgaacgaac gcaacgacag 137820
   gttgctgttg atgctaggcg gggccacgaa cacttgacat gcatccactc gattcatcag 137880
   attctttaac gtctctgctt cttgtacttc gactgctgat gctgatggag acgaacgaat 137940
   cgaggcgaac ggcagtaatc tcgaccaaaa cgatgaggcg cgacccaatc gaattaaagc 138000
   gctcgtatcg tttcggcaag acgacacccc attataccag taatcgttat cgatattgaa 138060
   aaatttttcc aagacattgt ggtgatcgac agcattgctg ggcatcagat atcgaactgc 138120
   caactttact agtcctttct gtagatattc aaaatcgaat tgcgacaccg gttcgtgatt 138180
   agtctttatt tcgtattgcg ccacaatttt ttcgatcgta ttatctggtc gcctgtttct 138240
   attctgtttg atcagacgga atggtgtcaa aataaatgca caatcgtcaa agtcgatctg 138300
   tatatagttg tggcacacgg taaacgttcg attatcgatc atctgcaaat actgttcctg 138360
   taccgtattc ttggcgatga tcgactcgtc tttgctgttt attttgtcgc attttgcgat 138420
   tctgtttaat ttaaaaacgt taaaatcatt ttctaaagcg gaatcgacat cgatcgtata 138480
   gttttgcttt agaatcatat ccctgaatat gctgggcttc atttcgtaaa gaaaagacac 138540
   gagagcgctt cccatcgagt ataatatatt ggaatcgtaa tcggctttga caatctcggc 138600
   gatggtggcg tttcgatatg cctttaaacc ctcatgatcg cgccaaaaac aatcacgatt 138660
   accgaatctg tcggcggcgc cttcgatata ccacttgggc atgaatcttg cttgtagcgt 138720
   aaacaaaaga cagtgatgaa attcatgacc atatgcgttc ggtagatcgt catgttcata 138780
   gtctctgaag aagacatgag ctcgaatcac acgtttctcg tgcacgtatt gagtgatgcc 138840
   gccgttgttg acgccataat tccagtaagg tcccaatcga acgtattgtt tacgatcgtt 138900
   atgcacgtac atgtcaatgt cggtgaggtt gtggcgatac tgaagctgta gtcgtttgta 138960
   gaaatcgata aaatctttat agacggtatt catttcgagc gaaatcttca tgagtacatt 139020
   atttctagta atgttgtgat gtaaaaaaac tcgtacgggt cccacgtcaa atctcgtgtt 139080
   gaagggatag atgacatctt caattttttc atagagattt ttatcaaact tggagtcatc 139140
   gtaactgatc ttgttgccgg agacgaccaa atttttaaat ttcgacacat aacacacgta 139200
   aatgtcttgt ttgatctcgg ccttcggcat aatcatcagc ggatgctcga gggctattcg 139260
   agacaactga acggcagcgc gatcgatgag ttcggttatt ttgatgtttt tctcgaatgt 139320
   ctctctgatc gaagcgtaag cgactagaca cgttttaaac tgtctatcgt tccacatgat 139380
   gaatgaactg cacaattcgt gaaacgatac aaaagttctt cgcatgttgg ccgagttcac 139440
   gtagcgagta tacaaatcgg cggtcatttc gaatagattc gaggcgacaa tctgcatttt 139500
   gcgagaaatc cgacggtcga cgagagcaat ttttctatta aatttatcta gcgaatccag 139560
   tagagaggct tgagcatcgt actcttgcat ccgagccatt agctgcaacc acaccatata 139620
   tctctctatt tcggatggtg tgttgaatgc aaaatcttga tctttaaaat tatctacaag 139680
   ttttttggca ttacactcga aattgatgaa tttggctcgt atacacgata tcaaaagttt 139740
   attcattttc actttatcat aatggctgta atcgatacac aaaaatgtcg gcagaaaaat 139800
   tattgtcgtg attatcgcgt acttgaaaaa caccaccatt gttacgagcc aatacacttt 139860
   gactgccaat tgaaataaat tttttcgaca catttgtctg ttttccttta aaactgataa 139920
   tcattctaca agccattcga tcgtccgaga atcgatcatc gtcgactcca tccacgccga 139980
   cgatcgtccc gcggcgacgc gcgatcgatc gatcatgaat cgccgcgagg cgatcgtccg 140040
   agccgtcgag cacgtttttc gcgtcgacga aaacatgatg caatgttgcg tcagccggat 140100
   gtttgtcgcg cgcgcgcgtg acccgaatcg ataacgatcg agaggtcgac gatcatccgg 140160
   ggtcatcgat tgacgcacgc gtcgcatgaa taatgatgat gatgttgata ttgcccaata 140220
   ttgcatcatg ttttgtaatt ataatgaaac gacaatcgta ttgcatcatc ttttttattt 140280
   ttacgtttcc aacgaatccg cattaaaact cgatgacgca aaatataaac tcgattataa 140340
   tgtgatgtcc aaattcatca attatgaggg taaaaacttt ataatcttgt aaaacgactc 140400
   ggacgcttaa gagacatcga ctacaaaaat tttttgtgat ttttttacaa aattttagtc 140460
   tatcatcaca gacgttttta atgaaattga agtcgttgat ccatataaaa agaattttag 140520
   agacgatttc gatggtattt actttgaatt tatagttatt ggcatgcatg ttgaggatac 140580
   atgaatctgt aaaaattttt aaaatcacgc gcaggccgtg tattgtgtag aaattaataa 140640
   tctaaaaaag agacttttac tcgatgattg atgtgaatca ccacataaat aaagttttcg 140700
   acgttgacag ttttaaaatt ttataaaaat ttttagcaga aaatttttat cgttttgtca 140760
   gagccgaaat catcaataat agcgtcgagt cgattcacga ttaaacgatt atgaatcgac 140820
   acgacatcaa catcgtcgca aatatctttg aaaatagtgt cagtaacgat ttcaacaata 140880
   atggtgtcat ggcgattcat gatcgtttca tcatgaatgg atatgacaaa tctaagaaac 140940
   gatattcaaa aatctaagac tcgaatatct gtaagatata tcaatcgcat gtctgtagcc 141000
   tactatattg gtcgtgtcaa aaatggatca aaacattaaa aaattattta ttaaagaaac 141060
   caatctcaag gaaaaacacg aaagtctgga caaggcctac agaaaaaaac ctaaagaaga 141120
   tagaaacgag agtgcaatcg atgagatgac cacctgcgcc gcctatttgt acggcgtgga 141180
   agagcagctg tttagccttt tgtcgaacgt gagtcaagag cgcagaatgg actttattaa 141240
   cgatctaacc gaactggact ttagtaacta cgaaattgaa ctattattga acactagatc 141300
   cgatgaatat ttattgaaaa aatttcatgt tcacaagaag agcgaaaata ttcaaaagat 141360
   tttcaaaagt aacagtatta gattcgttaa agtcttggag cagtttgtgg tgaagagacg 141420
   ggcatacaag aaaaaaccta aagatactct actcgaagaa ttggtcattt taaaatccat 141480
   tataattaaa catttatgta taatggaaaa acttacacaa aaataaaaat ctataaaatt 141540
   ataatcaatc gttttttatt tacacacaat tatatttaca tacacacaat tatatctata 141600
   taaaaatata gcaacgatac ggacaaaata cattatttcg atgacattag aaacgataat 141660
   ggtggtgctg tcgccgccgc tgtcaaagat tgccgatctg tcgtcggtat agccatatac 141720
   gtgtatgctg catggtccca tttgggtgta ttattggtgg gtacagcata gtatggcgac 141780
   tcgtttttat ccacattaat gtagtgtttc acctcgtcct tgtcgctatc gtcgtcacca 141840
   tcgaagctat catagtcttc ttctttaaaa tttatatatg tttcttgtgt ttcataaaat 141900
   tttttcttgt cctccctgcg gttttcatct tttataacgc cattcaccgg tttaatggcg 141960
   attttttcta cttcatcaat cgtgtcttca gtggatgtcg acttgtttgt tggacagcaa 142020
   ctcgtcgcag ttgactttga atctaatgat ttagtcttgt acgctacgta taaatatgat 142080
   aaatgtatca atagaacgta cgtaggaaat aaaattatag aaaagcgttt ggtttttaga 142140
   aaagtcggtt ctccgacata gtattcgctt ctgcattttg cgcaataaat ttcgaatttg 142200
   ttgttaagcg tgaaacaatt ggcgctttga atcgcattat tgtccatttt gacgattccg 142260
   tcccagcatt ctttgtcgat atcataatct ctctcgttga catcgaggcc gggaacatga 142320
   ccgtatgtga cgaaccagtc cattgaaaat agagcgattg tcgttaaaat agtggtcgcg 142380
   ccgatgagcg gtacagcaat ttgatcatag accaaagtcg agagcacgac aatcaatcca 142440
   taaaccaaca gcacactact aaaattcacc acttgactgc cgtcttcgta atcgatgatg 142500
   agtccaaatc gtccgtcgtt gatcccgacg actcccaaaa cgaaggcggt aaatccgcac 142560
   gcaaaaagca tcactttggt gtaataccgt aaatacgcct gcatcttgta agaaacatgc 142620
   atgtactact aattatagta gttgtgatta tttttctttt tattttatat aaacctatct 142680
   atgccgccca cgtagagata aaaaccgctc aagatgaata caacgccacc gtcgacgacc 142740
   gaatcgagta catggaaggt gtgttaaaaa gacgcaaata tgtaccgctc gaagctctgc 142800
   ctacgatacg attcaatacc gaactaggca ctataaacga tggcgaacgc aagtgtatgt 142860
   cggtgccggt gtacgtcagt tcagataata ttcctaattt tgattgcaca ctcatctgcg 142920
   atgatcccac cgcagtttat tgttttgttg gcgaatttga taaatttctt gtaaacggcc 142980
   agatgctcac gcgcggcggc tattgtacta caaattctgt acctagaaac tgcaatcgcg 143040
   agactagcat cattttacat agcctaaatc aatggacgtg catcgcagag gacccacgct 143100
   actttgccgg ccctatgaac atggtacaaa tcgctgggcg ccagcacgcc actagaatcg 143160
   cacccggcga aatcacaaaa aatattgttt acgacaatct actcaaaatg gaggtcgacg 143220
   tgtctcgaaa cacttttcgt cgggattggg acgaatcgat gccggacggc agccgtagat 143280
   tccaggtcat ttgtaatgca ttagacgatc gattcaatca aatgtttgta aatcccatga 143340
   atccaatcga gtgtttacct aatacatgta cgagtgctag atatgtgcat cgagacgtta 143400
   aacccaattt cgagacgggc gaatgcgaat gtggtgatgt taacgttacc agactaactc 143460
   atatcgtgcc cggtgatcgc acttccatgt gcgcaagtgt tgtcgatacg tttcaaacga 143520
   ccaacaattc acacgtattt agagtgcaat gtgtgaatat gaacacgtta atcagcgact 143580
   atgatccaaa catgcttcta tgtcctggct cgcaattcga ttccaataac gatagcgcgt 143640
   ttagctatga attgccgggc acgtatgttt tttcttcgaa cggcatacac gaacctactc 143700
   atcggttcta tttagacacg cgcagtagaa tcgactataa tatcgtaaat cctgtcccga 143760
   cttgaaacct ataaaagtca gtagcatgta tctttagttt tattgagttg ctgcgaaacg 143820
   atagcaatgg accgacaaac tacacaaaaa ctactcgaaa atgagaataa taaacacaaa 143880
   aatgttggca tacaactcaa caaagaagaa tttggaagcg acattaaaaa ggggaatccg 143940
   aatttttatc atatactaaa taataccgaa gacctcaaaa aacatgccaa atgtcgcaga 144000
   aaactaaact ttgatttcat ataaagatgg aatggaaata aaatcatcat gttattcggg 144060
   atatctatca ataaaaatat aaattcaata aaacattttt atttataaaa cctaatcatt 144120
   atagtttaat cgtactcgat attctcgagt ttattctgta gagctaataa atgctgtgga 144180
   tcgtgatgtt ttttcttgac aggcttcggg ttcatgaatt gcatttcacg atcgacacga 144240
   ataacggtag gatcttcgcg acgacagcaa cacgagaaca tagaagacac ggtgcccgag 144300
   cagcaagtag aataaatttt gaacaccatc agagctagca gactgagtac aatccacacg 144360
   aaaaccattt ttacgacatg gaaatcggct ccaatcgacg agaaaaaatt ggacagccac 144420
   gagttttcag tatcgtctag aatttcatcg ccggcaaaaa ccgtattatt gttcattagc 144480
   cgacgtaaat ctaccaatct atcggtcaat ttgtgtaaat tattgtgctc taaatcatta 144540
   ttcattttca tagtctgcag ctgtagagtg tccagatcct tgagcacagc actcaaatta 144600
   aaggctgtat cgatgggcat ttgtaaattt ttatttagac tgttgtacgt ttcgagtgta 144660
   gttaaaaacg tccgtttcgt gctcaattga caagtgtgct tgccggtagc gttaatgatg 144720
   ccgattcccg ccgacaaaac tagagtgttt gccgacaccg aattatctcc tttctgtaga 144780
   cagtcaaagt tgatgtcgac ctcattttcg aggacataga gccatcgatt aaaatcagta 144840
   attttataga agatttcact ctcaaattta ccgattctaa catcacaatc cttttcaaaa 144900
   ttgatgtctt tgtcgttttt caaaaagata cgaatatcac acagctgcgc ttgattagaa 144960
   tcgtgtacga cctgaggcac gtagcagaga atcatgtcgt gtccgagtcg acaagactcg 145020
   cgagtgtcgt cgaaacgcac aaaattcctg cgatccttcg atacacctag atatttgcta 145080
   tccggtagaa ttatggcaca tttagattta acgcaaaacg gtatgggcac aatttggtag 145140
   acatcatatt tttgctgatt gacgagcggc acttcaatga tgaatagtag ttttctttct 145200
   ttagtgataa aaacgtgaat attaatgatt ctgtcgatga gctcgaacat attgtcgtag 145260
   tgcaatggca caggccacga taaaccttta ggtagataat tgctcacatt cttcatttct 145320
   tccagcaatt tttcggcggt aatgactgcg gaactgagtc gtttgtcttt ggcgtcctcg 145380
   acggcatctt taatctggtc gtaaagactg tctagcctat cgatctcact acagatcaaa 145440
   tgaattttag tgcccacata ctcacatact ttttcttctc ttcgtttggc atccaattca 145500
   tcttccacca tacgttcaaa ctgaatcaaa tgatcggata tctgttttat ctgacgattg 145560
   agtgcgttcg tgccgttggc gagtttgtga agttcgtggg catcgtcact gtccattagg 145620
   ccgaacaaat atttatatag gctaccgaga gggtcgaata gacctctctt gcttctgctc 145680
   agcgtatatt tcgatggagt ttcgtagttg ccagacttgc ggatttttcc atcgatatcg 145740
   ttatgaagat tcaccaagtt gacgattttg ttcatgatag ttttatcgag ttcggtctga 145800
   ataatatttg aactgctaca atttttaaat tgtctacttt tgtagatgaa tttttgtaga 145860
   tttttaacat gatcgaacat gtcttttaat cgcaaaaaca cagcggcatg atccatttct 145920
   acaataaaat gccaagtgtt ttcgacaaac tgcatctcct tgataggttg aaaatacaag 145980
   cccgaagtgt gcggtaaatg ctgtacgtta ataatttcgt tagccgatat tagttcttta 146040
   ttggcgtcgg ttgttgcgca caacaacatc atcgacactg ccaacgagat tagcggctgt 146100
   gacaatgtca tgttgtttcg cgtttctatc gttcacacta tactgaatat gtcagcgaag 146160
   actttgaggc tttatactcg gcaacgcaga atataattaa tagcttacta ctatatattt 146220
   tttaaccgtc gtctcgttgc cgttgctata ctgtcggtcg taaatcaatc gtcacagaca 146280
   agcgacatga ttttcgtggt cattattttc gtgtgtcata cccaaaaata gagtcaataa 146340
   aatacacgtg ttattatttt aaatgcgtca tttatttatc aagttgacct ctaccacaca 146400
   ataacaggat aataaaaaaa taatgaatac aataacaaga taataaaata atacaacaaa 146460
   atgtgatgtc atgtgtctgt tttttaacct tggcagctct cgcaaccgat gtagcatgcg 146520
   tcaacaacat ccggttcatt gtttgcacga ttgatcgccg acgatgaagg cacggtgaat 146580
   tgaatcgtgt ttgctgcggg tttggttctc aaatagtaca tgcccgtttt taaacccttt 146640
   tcccaagaat agtcgtgaat ggatgataaa actttatagg ttggattggc cacaaaaaga 146700
   ttgagcgatt ggctctgatc gatgtaaggt ccccgctctg cggccatgtc caacactact 146760
   ttttgtttaa tttcccaagc ggtcttgaag agcgccttga tattgtgtgg aatttcatca 146820
   atattttgta cagatccgtt gtttttaata atttttatca tgatatctgt cgaatatacg 146880
   ttggtgtcta cgagtttatc gacgagatgc tgattgacca cttgatactc gcccgacaga 146940
   actcttcgta cgtacaaatt actagtaaac ggctcgaacg actcgttgtt acctagaatc 147000
   tgtgcagtcg ttgccgtcgg catgtaagca atcaataatg aattgcgtaa gccgtgcata 147060
   gccatttctc gcttaagctc gtcccagttc caaagatcat tcgattggtt aatattccac 147120
   aaatcaaact gtaaaagtcc acaacttgca gggcttccat cgtaagaatc gtatgttccg 147180
   tgcagtttag ctagttcgat actttgactt agtgcaccat agtaaatagt ttctgcaatc 147240
   tttttattta aatctctagc ctcttggctg tcgtagggca tatccaaaat ggcgaacgta 147300
   tccgcaagtc cttgaatacc gataccgatg ggtcggtgtc tgaaattcga tgcttgagcg 147360
   ctagccaacg gataaaaatt caagtcgata attttattga gattattggt aaccactcta 147420
   gatacattgt agagcgactt aaagtcgaag cttttaaact gtttattggc aaaattattt 147480
   acggcaatcg aggccaagtt acacacggcc gtttctcgat tgtcgctata ttgaacgatt 147540
   tcggcacaca gattgctgtt tcgaatcact ccctcgtttt tttgattatt tttattgtta 147600
   acgtgatctt taaacatcat gtatggagct ccggtctcga cttgagtctc aataattttt 147660
   ctatgaagat ctaaagccga aatttgtttt ttaaacttgc cttcggcctc gtaattttcg 147720
   taaaggtcct cgaattcttg gccatagcaa tcgtttaaac cgggacactc gtcggggcac 147780
   atcaatgacc atttccctcc tgtctttacc ctcttcataa acagatcagg tgtccacatg 147840
   gcaaaaagaa gatcgcgagc cttcatatcc tcggcaccca tattgcgctt tagatccaaa 147900
   aaagtaaaaa cgtctgcgtg ccaaggctcg atgtatacgg tcatggcgcc tttcctttta 147960
   cctccttgat cgacatggcg tagcatgcta ttaaaaacac gcaatgcagg tcccaatccc 148020
   gccgcagttc cgttggtgga ttcgatttta ctattggtgg cgcgaatttg agtgacattc 148080
   aggccgatgc cgcctccgtg tttagatatc atggcacaat cgttgagtgt tttatagatg 148140
   ccgttaatac tgtcctcttg aatgttaagc aggtagcacg atgacatttg aggcattttc 148200
   ataccagacg aaaatagagt tggactagcg tgagtgaaca gctttgcccg catcaatcta 148260
   taggattcga tagcgctttc gatgtcatca ccatgaatct gtagagctac acgcatcagc 148320
   atgtgttgag gcctctcgac gacaatatta tcgactctca tcaaatattt tccttcgatt 148380
   aaagtcatca gaccaaagta agtgtaatgt ttatcgaaat cgtaagaaat gctagcattg    148440
   attgagtcta aatgctttat aactatcgag tagaaagaat cacaaagaat cccatttttc    148500
   cagagaatct ctactacttt agagtaatcg tcctcggttt tggcatgtag cttgttgact    148560
   gcgatcacaa cggccagatc agaataaaca tagtgcttgt aagtctgaga ggcacacaca    148620
   ttagcactgt attcttcaaa ctcatcctcg tgtatattgt catagattcc gcaaaaaatt    148680
   ttatcggcaa tatagttaaa gtcgatgtat tctttatttt ttttcaagtc accacttcga    148740
   gtataaagat aagttttgat gtagtccata tttactttga tcgactctcg gccgttcctc    148800
   aaaatcattt gatatgaaga aaacatttta aaatcactat acaaaagtca cgactcggtg    148860
   tcaaggtgta tgtagtaata ataaaacaca tttgattgtc gagtatattt atatcattta    148920
   atcgctgaat gatgctcgtc ggattgagaa agagataaaa aattctcata ttagatgcgt    148980
   ttgccgtaat catgtttcga ccaaaaattt ttctattgat catgcacaac aaaataagta    149040
   tttttctcct ttcgtaaaag tttgtgaaaa atcaaatata                          149080
<210> 19
   <211> 150079
   <212> DNA
   <213> Chrysodeixis chalcites nucleopolyhedrovirus
<220>
   <221> source
   <222> 1..150079
   <223> /isolate="ChchSNPV-TF1" /isolation_source="Canary Islans, Spain" /mol_type="DNA" /note="Genome of ChchSNPV-TFl-C" /organism="Chrysodeixis chalcites nucleopolyhedrovirus" /strain="ChchSNPV-TF1-C"
<220>
   <221> unsure
   <222> 4372
   <223> /note="a or g or c or t"
<220>
   <221> unsure
   <222> 4387
   <223> /note="a or g or c or t"
<220>
   <221> unsure
   <222> 4389
   <223> /note="a or g or c or t"
<220>
   <221> unsure
   <222> 6870
   <223> /note="a or g or c or t"
<220>
   <221> unsure
   <222> 6873
   <223> /note="a or g or c or t"
<220>
   <221> unsure
   <222> 6874
   <223> /note="a or g or c or t"
<400> 19
   atgtatacac gttacagcta taacccttct ctgggccgca cctacgttta cgacaacaaa      60
   tactacaaaa atttgggtgc cgtaatcaag aatgccaagc gtaagaagca ctacgccgaa     120
   catgaattag aagaaaaaac ccttgatccc ctagacaact acttggtagc tgaagatcct     180
   ttcctgggac ccggtaagaa ccaaaaactc actttgttta aagagatccg taatgtaaag     240
   cccgatacca tgaagcttgt cgttaactgg agcggcaaag agtttctcag ggaaacttgg     300
   acccgcttca tggaggacag cttccccatc gttaacgacc aagaaatcat ggacgttttc     360
   ctagtagtta acatgcgccc tactagacct aacagatgct tcaaattcct tgcccaacac     420
   gctttacgtt gcgaccccga ttatgttcct cacgaggtga ttagaatcgt agagccgtct     480
   tgggtaggca gcaacaacga atacagaatt agtctggcca agaaaggcgg tggctgccca     540
   atcatgaacc ttcactctga gtacaccaac tcgtttgaag agtttattgc tcgcgtgatc     600
   tgggagaact tctacaagcc catagtttac gtaggaaccg attccgccga ggaagaggag     660
   attcttcttg aagtgtcttt agtctttaaa attaaggaat tcgctcccga cgcgcctctg     720
   tattccggcc cagcatacta agccgtagat tcgttgttga ctttttcgtt aatttctgtt     780
   aaaagtcttt ctgcttgttt aatgtcttgt ctgctaacgt gttggctatt tattaatctc     840
   gacacattag cagacatgcg taagatttga tcgttttcgt ttccgctatt ctgaactcga     900
   ttcatagtgt ttaaatactt atttttgacg gtttccacct cattgtcgct gatccaatca     960
   ttattgtctt cggaaaacga atcctcatta ccggtgctct cggctagcgc ttttactctt    1020
   tttgccaggg ccaaatgcaa tactgaaggg gcagtttcaa caatcggttc tttgtccgcg    1080
   ggaatttttt ttacattttt taaaattgta ccagatcgaa tcgcttccat taactgcgaa    1140
   tgtgcgtcta ttggcggtga cgcaactgtt gtgggcttta acgacggttt tgagggcaaa    1200
   ttttcttctc cgacttcttt aaacatttga aacatgggcg gcggtggcgg tggcggtggc    1260
   ggtggaatcg aagtcagtgg catcggcggt ggcggcggtg gcggtggaat cgaagacatc    1320
   ggcggcaaag aaggtggcgg tggtatcgta atatgggttt gcgtcgacga caatggtgat    1380
   attgaatttt tttcctgagt agaatatatt tttgttgttt cttgtatctt ttgtgtctcg    1440
   gtgattggca taggaatcga tggtgtcgac accgattttg gggtagatga cgccaacgac    1500
   ccagcatttt gattatccag agtcaagatt tgattgaaga tcgaatcgat tttggccgtc    1560
   ggtttttcga cttcttcttg atactgttta tagagtttaa aaaaagtttt cattaaattc    1620
   tctatcttta aaagttcgtt ttcgtggtta attttatcga taatgtcgaa taaaatagtt    1680
   ttatagcggc tgttggcgtc gacgcgggtc accatatttt cgagttgcag aattttattt 1740
   ttcatgcgca cgttatctgg tagaacggca ggtccgatag tgtcccggac gttggccgca 1800
   acatcgatag tgattttagc tgtattttgg taaatcgatt ccgccagacg tagatatttt 1860
   atagccagag ctctgtccat gacgactctg tctccgatga cggcattgcc aaagtcgagt 1920
   ctgcgcacga aatctgtcgg aggttgataa ttaagactct gtaagtattc tctcacggtg 1980
   acctgtgtca tttcagtcgt cgatcaatat ggacggtgtg ctcgaagaga tgagtgactt 2040
   tttaagcgaa ctcgtgccag aaactaagat taaagtggcc gaaggtaaat ttggcaaagt 2100
   gtccgtatgg agacacaagg acactcaaaa attattttta cagaaagaaa ttactccaga 2160
   gaatttcaat ggtatcgagg tgatggtcca cgacttaatg aagaataaca agttttttat 2220
   taaactttat tatttcacga cgacacttaa aactcacttg cttattatgg attacatcaa 2280
   aggaggtgac ttatgggatc tccttcgaca agagcataaa ctagaggaaa tggaaacaaa 2340
   attaataatc atgcaattgg tagaagcgct ttattatctt cattcacatc aatttataca 2400
   taacgatata aagctagaga atataatata ctcaagatat agacagattt atctatgtga 2460
   ttatggccta tgtaaatcag tcaaaacgga atcgtgctat gacggaacta tggattattt 2520
   ttcaccagaa aaaatcaaag gacatgaata taattttagt tttgattggt gggctgttgg 2580
   tattttgact tacgagctga ttacaggtca tcatccatat aaaaactatt acgatgaaga 2640
   tttgacagta aaaatgttac atcatagaca gcagcacaaa aaattaaaat ttaactctaa 2700
   attaagtaaa gttgttgtaa tgtttataga tggtatgtta aaatacaact ataactatag 2760
   actgaataat gtttatgaca taatgaaaca agagtttatt aatattgaca ttaatttatt 2820
   aaaataaaat tatattacta catacatttt ttgttgttta aaatttattt actttttttg 2880
   tattttttaa atttaattga tacattttaa atttattaaa atataaaata taacatacac 2940
   attttgttgt ttttattata tacattattc tattttttga actgtagtta taagaggaag 3000
   tattgtggta agaccacaat cctcaacaat ctggacgtcg tcatcgctgt cattggccga 3060
   agcaggaggt aaaatactgg ataatgtgtc tttggccaat tgttgataat ctatagcagt 3120
   ctgctgcagc tgcaccggtt cttctttaac aacgtctaga acgaaatccg taggcacgta 3180
   cggtttaccg cgagcagtta gatttaactt gcgcaaattt tcattattgc aagtatagcg 3240
   ataagtgacc agttttgtcg acggaggagg ctcattagat gaatttattg cctccggtgt 3300
   gattacacgc aacgcccgtg aatgctctgg aacagttgaa ttcataggct ggttaaaatc 3360
   aggattattg aaggaggccg gacttatagt atcgtacatt ggagatataa aactcgaaaa 3420
   ttgatttaac ccggggtatc cgttgttgta tgcagcattg tatccgccat cgtatccgtt 3480
   gtttccgttg ttataaccat tgttgtaatt gttgtagcca ttatatccat tattgtatcc 3540
   attattgtat ctattgttat atcctccgtt attgtatcct ccattattat atcctccatt 3600
   attatatctt ccgttgttgt atccgttatt gtatgaccaa tcagccatcg gcatcataaa 3660
   attatgcatc ggtggtgctg gcatcatcat cggtggcgga gctgacatca tcggcattgg 3720
   ttgagctggc atcggaataa actcagtatc gaacgttaaa gcttctggta tcaaagccgc 3780
   taaagccgga gtcgctggag ctgacgttgg catctgagtc gctggagctg gtatcggagt 3840
   cgctgacgtt ggtatcgtag tcgctggagt cgctggagtc gctgtagctg gagccgccgg 3900
   tgtagctgga gtcgctggag tcgctgtagc tggagccggt gtagcttccg tcggcggctg 3960
   atcagtggtc attagttgtg aagataatga ttctatcggc gaggttggtt gcagtgtgga 4020
   aaccgctgat tcttcaactc ccaattgtgt taattgggat tctatagatt tttctgcagt 4080
   agccatattt actcgcgaag gagacgaaac ttcagtatta ttgttatcaa catcatcgtc 4140
   atcatcatca tcatcactac tattgctact actatcacta ttatcaccat cgttattgtc 4200
   atcatcacta ctatcactac tatcaccact atcactacta ttattattag tttgattatc 4260
   aatctgttcg tcttgttgat tttcgtcttg ttcttcttgt tcttcttgtt cttcttgttc 4320
   ttcttgttct tcttgttctt cttgttcttc ttgttcttct tgttcttctt gntgattttc 4380
   gtcttgntna ttttcttctt gttcttcttg ttcttcttgt tcttcttgtt cttcttgttc 4440
   ttcttgttga ttttcgtctt gttgattttc ttcttgttct tcttgttctt cttgttcttc 4500
   ttgttcttct tgttcttctt gttgattttc gtcttgttga ttttcttctt gttcttcttg 4560
   ttcttcttgt tcttcttgtt cttcttgttc ttcttgttct tcttgttgat tttcgtcttg 4620
   ttgattttct tcttgttctt cttgttcttc ttgttcttct tgttcttctt gttcttcttg 4680
   ttgattttcg tcttgttgat tttcttcttg ttcttcttgt tcttcttgtt cttcttgttc 4740
   ttcttgttct tcttgttctt cttgttgatt ttcgtcttgt tgattttcgt cttgttcttc 4800
   ttgttgattg ttttgttgtt gattttcgtc ttgttgattg tcgtcttgtt tttgttcatt 4860
   ttcttgaact aatactggtc ttgcagcagg ttttattgta gcagttggtt tctcaataat 4920
   tatctcagac tgtggtagta cagcattatt atcattaata tcgccatcat tattactggg 4980
   gccaggcgat tgagatgacg ctggaatatc atcagcgggc gcttgatcat cagcgggtgc 5040
   ttgatcatca acgggcggcg gcgatggcgt ccatcgcatc cgcggcggtg gagattcata 5100
   tcggaatgaa ttaatttgtt tggtttgata gcaaaaaata cattcattgt atttcatcat 5160
   actatcgtag gtacagcata tgcataatat atgttcacag tgtggataca caaatccttt 5220
   atctccattg ttgcaactta tgcacggttc agtcataatc ttcagcttgt tgtttctcat 5280
   gtatgtatat agtacagaac tgagtctaga tgatttttct cgaagtgcaa tggcttcggt 5340
   ttgaagcgca tgagttatta tcgtatccag tagatagata ttctttttta tctccgcatc 5400
   tacggtgata gtgtgaaaag aagagccaat attcttgaat ttgtcgtgca tcagaccaca 5460
   tttgatcatt aattcttgtc tagtgctagt attaacgttt tcgaaaatcg attgtataaa 5520
   cgaaaactta gcattctcat cctcggaaac agtatagaat tttttatatt cagataaaca 5580
   ttgcataata tgctctaaaa cgcattgctt ggttagtatt gtcaagtaag tctctaattg 5640
   cttgcgaaca ggtatatagt ttaacgattg gatatgcaag aataaatctt cataacggtt 5700
   agagaaaaag tgtctagatt tatatgtgat gccagtaaac ttgaattgtt tgttaataca 5760
   gcacacatag cctacgcgct ggttattttt agagtttaaa tttaaataga taaacacttt 5820
   tttctcgacc gtattatggt gcaggtgaat attgttatga ttgttactga tcatatctcc 5880
   agtgggtaat attatagcag tagtattaga tgggttttca ttatctacca catctaccac 5940
   gggagtttct tgtttttgac gcttgctgtt agacgcagca tcactatcac tctcattatc 6000
   gatatcacgc ttaggtgttt ctttttcttt tagtttcttc agtatttttt tagttttttt 6060
   tccctttcct tttcctttac ctttttcaat tttttcattt tcattatcac tgtcactttc 6120
   actaataatt ttacgtttgc gtaattcgat ttcttcttct tcttgatctt gtcctttatt 6180
   tttttctttt ttaatttttt cataatcact gtcattatca ctttcactaa taattttacg 6240
   tttttctttt tcttgatctt gttgttcttg atcttgttgt tgttgatctt gttcttgatc 6300
   ttgttgttgt ttagatgcgc tggcgataac gttatcgcca gcagccgatt ctactgtcac 6360
   gccatttgaa tctacgtcgt gagtcaattc atccatcttc agtatttttt tagtcttttt 6420
   tccctttcct tttcctttac cttttccttt ttcatttttt ttatattcac tgtcattatc 6480
   actttcatta ataattttac gtttgcgtaa ttcgattttt tcttcttgat cttgtttaga 6540
   tgtgctggta atcacgttat cgccagtcac gccatttgaa tctacgtctt gagtcaattc 6600
   atctaattct ttttgaattt cgtttatttt tgacaaatta ttttcattta ttttatcaaa 6660
   attttcatat tctttaaaaa tttcttcatc agattcggta aatggcatgg gttcaagaaa 6720
   acctattaaa ggttttttca tttcacgccg aatctcttct tcagtaaaat taaacctccg 6780
   tagggtttcc tctttcaatt ctgcattcga tagcgacgca gtatatttca ttttctcaaa 6840
   tttagattta aatttccgta tcattatgan gannacaatt aaccagtata gcacgtatag 6900
   aaaaattata ctgaacaagt tttaaataac caacacatat ataataaaag ataaaaaaat 6960
   tatgtgatta gatagagatt tcatatcata tcataaatat gatatagctc gagataaact 7020
   tttttgtcgt gcaaaaaagt actctaaagt gtagtatatt tacagcatat tgtacagtgt 7080
   agactattct agttaaatag tctacgatta gtagattttg ttctgtatat tgatgccgat 7140
   acgataacga acttttttgt agtgcaaaaa aatacgataa cgattatcga tagcctattt 7200
   gattagatat agccatgttt gttatctttc gatataaagt aatatgttta ataaaatata 7260
   ttagtaaaat atatcagtcg tgccgggtaa cacaaatatc gatcgaaagt gataaaatgc 7320
   cgaaactcaa gaaaattcca aaaaaaccag tagatgacca cctaggaatc ctcagatatt 7380
   tcaagattat cagaaagcca gtttgtgcta taaacgaatt acctcctaaa gaagatcaaa 7440
   aaaaagaaga cgacatcatc attcatcttt ctagtgacgc acctgataat tcattcaatt 7500
   ttgattttga acttagtgac gactcaagcg atagtgactc tgacgattca agcgatagtg 7560
   actctgattc gtctaacgca cttagtggcc tagataaggt tttgttgaat tcgtctaata 7620
   ataatgctgc tgacaagaat gctatcttta tatgtcctaa cactcaaaag ttaaacagca 7680
   gtggtagtgc cataataggc atgaatcgtc ctaatttgac tatagaagat tcagataata 7740
   gcaatgatga ggaagtttat gtacctactt cgcggcggcc aataaaacct gcagcaaatt 7800
   atcagaagat aaagcagcaa ctaaagcagc aactcgacca aatattatat cgtttgtgag 7860
   tgataacaat ggaacattat ctaatcaata atcaataaaa gattaatatt gtgtacgtga 7920
   tgtttttatt tgattataac tgatatgtca ggtataaaag cgctcaaatc gcagtcagga 7980
   ccatcacaag tatttgtgcc ttgctcccga gcagtcctcc tctatacttt ttgttgtgca 8040
   aactacaatt ttttttaatt catcatgatg atgtattaca tcaagcaaga cattgaacat 8100
   ctaatgtctg ttttgctgaa ggaaaaaccc aaacgtgaaa tggtgttaaa cgagcggaat 8160
   tcggagaaaa gttacttgct cagcagaata cccaaaccta ctttgttaaa ggataacaaa 8220
   gtgtttaatc tggtggccga tatctttcat gaaagtaaag aagagcttaa tcataaaggg 8280
   gcggaaatcg tgtacacatc atcggcttcg gtgcctagtg tgtcagaagc tgactctgat 8340
   tctgattctg attcggatga cgatgatgac actttagaaa aatttgtgaa gatcaagaaa 8400
   aatgtaaatg ctgttttctc gcggaccgtt aaacaactta aaaaacaaaa caaactaata 8460
   aaaaaatttt attcagccat gaataatgtt ggagagggtg agtcagaaga aacgagacgt 8520
   ttaaataaca gaagaatgat agcctacaaa aacaacggct tgagcattgc caatgttggt 8580
   gatatagtag ataatctatt cagggcttat gttgaaatga cttcaggtat cgtacattta 8640
   ataaataata taaatgaatt tgtgatagat agaccatata gtgatttctt cgatggtaaa 8700
   aaaccatata gtgatttctt cgatggtcaa gaaaatcttc ctgatgagaa taactccaat 8760
   catcaggaag atcaagcggg agatctaaca gatccagaag attcaaatca agaggaagac 8820
   tccaacaatc gtcaggaaga ctccaacaat cgtcaggaag actccaacaa tcgtcaggaa 8880
   gactctaaca attatcaaaa aaatcaagcg gaaaatctaa cagacccaga agattccaac 8940
   tatcaggaag atatagaaaa tcaagctgag tcaagcggct ttgtgccaag ggattcaatt 9000
   ttctataata tgtatggtga tcaagatgat gacaatgaaa gtgtagcata tgatgacatt 9060
   gcagatgtta atgaagtcaa taacatttca gaaaatgatg tttctgaaat aaatattagc 9120
   aaaatgaata tgcaagacga acaccaggaa gatatagagg aagatcaaca agaaaaagat 9180
   aaaaatcaac aagataaaaa ggaaaatcaa caagataaag ataaagatca acaacaagaa 9240
   acaaaacgtg cctatcaaga gtgggctttc aacgattatc agacatcatc acacaggtcc 9300
   gatagcatct caagttctaa tgatgactct ggcagtgtcg acaatacaac tgctaaaaga 9360
   aacagtaaat ttgtatttaa aacttcttct cgtgctcgtc gtgacatacg ccgcgacaac 9420
   acagtgcgtc aagctctcaa attttaatat ttttgttttg tatactttta aatctttttg 9480
   ttgttttata taatttcata taagcttttg ttatgttttt tttgccaatt tattataata 9540
   aacaaacaca aaccaaaact aatttttttc tttagatttt tttagaaata gcattttttc 9600
   tttacacctt tataaacaat agctcacgtc ttgcttaact gacagggtta caattaactt 9660
   aactgccagg gttcgattcc ctaaaactct ataacctttt ttattaagat acatcaaatt 9720
   aaaatgagtt tttttaatcc tctacgaaaa ataaataaaa tttatcctaa tacaacacaa 9780
   tttaatttag acaacttggc tgtagttaat aatgctccaa gtggattcaa gaatgttctt 9840
   aattctaata gcattcaaaa tgcaggaaat aatcgcttta ttcccggcta taatataggc 9900
   aataataatt ttatcagtgc agctgatatg aataaaataa tgcgcaataa tgatgtcacc 9960
   agcattcgta atgtttttgg taatacacca actcaaactg acctaaatgg cctttcattg 10020
   ctgcgtagag cagataatgt tcctgatgcg aaccttcata gtgcggccat gcgtaggaat 10080
   gcagtaaaac aaaattaccc caacaccaat acaaacacac ctgccggtgt aagcaatgtg 10140
   ttagaacaaa atcctcggtt aactaatcat ttacagacat taaaaaatgc tggagtggta 10200
   agtttgattg gtgtcggtgt ttacttgact tttagcgcag ccactctaat tcaagacata 10260
   attgaagccc tcaaccgaac cggaggcagt ttttactata taggaagcga cggcggagat 10320
   aacatcagcg catgtttact catggataga acttgccgat tacaacatcc tgaggcaata 10380
   actgacgtga gaaaatgcga ttttgaccct ttaatatcga acgcaacaga actgcagagt 10440
   atttgtcaag gctttaatta tgaactagaa aaatccgtat gtcgggctag cgaccccaac 10500
   gccaacataa atacacctca atatgtagat atatccgact tagctcccgg tcaaactatc 10560
   gcctgtgtag aaccctacaa tatgggtgat ttaataggag atttaaattt agataattta 10620
   ttgggcgaaa acggcttgat taataaatct tcaaataaaa gtcaaagttt aagcgatagt 10680
   ttattgccgt ttattgtaat gataggagtt attgcattta taattatcat tggatatttt 10740
   gttatcaaaa gactgttcaa taagcaaact gtgagcgtag aaactaatag aactcgttga 10800
   gtggcttaat catgtcactt tgtcgtctag acgatttacg actgttttat tgtcatggcg 10860
   attcataata aaacaattat gtgtagcccg aaggattttt attagacttt caaatgaata 10920
   cacataattt ttatacatta atttttttat aaagattcat atcgtaatag tagatacgtt 10980
   tgggtttgat ggttttaact ttaacacaaa cgctataaaa ttttaaagca gacacgtcga 11040
   gcttgtttct agtgaaaaac atggcatccg taaaaccgca ctgagaacaa tacagcacta 11100
   tatgtttttt tagatacatt ttatgtcgag tgcagagaat gcaaaatcgc ttacacggta 11160
   taggaaaata tagtacaaaa ttaaaagtag ttttgtagac ttttttttcg accgaataga 11220
   aataggtgag cagcttctgt tcgcccatgc tcgcaaaaaa atccatcatt ttgctatcgc 11280
   cgaaatatag ctgaaaagtg ttgtcatcgc agccgatctt gctagaagta gtcttgatga 11340
   tgcaatttct gtcagtttct gtaaatagag ttcgaccata tgttcgcaat aaaatttttg 11400
   tgatttgttc gttgggcttt tttttatcca ctatatcctt tattgtccca aagaaatcgt 11460
   gtcgacccac aaccgaaatc tcttctgtag tacaagtgtg ttcgagatta atattgaaca 11520
   agtagcattt aaaaaagccc aattgacaca gagtctctgc ttcttcgagt ataacatatg 11580
   gatatatctg aaacaggtca aactggtgtt tatatttaaa cagttcgtaa catttagagc 11640
   agatcaattt aaacttcttg tgtctcttta attcatcttc aatgtctatg ctgttgtcga 11700
   tgaggcaaaa gagtgagaaa ttagtctctc gcttaaactg tttcgagcat tggttacatg 11760
   tagccttttc tacaatttct tcttgcttca aatctctaca attcatctgt agcaaattgt 11820
   tcacttttaa tcttccctga acgtaatcgg acgcaaactg aattatcgat tgtagtagac 11880
   aataattcaa atgactcaag aatttaggtc tcaaatcctt cggagggctt tgaatgatgg 11940
   gcggctgtct ataatctgga ttgttggctc tagtttctcg aactatattc atcaggtggt 12000
   ctatcagcat gggtttcctt tccgtttttg aagacatttt gactgttgtc gtggaatacg 12060
   attgggtcgt cgatgacata tgaccgatgt ttagaagcgc acgatagctt atgaaaataa 12120
   atacgagaag cgaacacttt atacacgctc tgttgacctc ggcgcgaccg tagagttata 12180
   aataaacaca taatatgcgc gggaatttga ctctacaaag gcgtcacccg tcaagtttat 12240
   attattacga gacgcgagac aatagcgcga ttcatttaca tgaagatgct catcctgtct 12300
   ggtacgtgat tttgtagtca tggacccagt acaaaagctt atctcatacg atgttgtggt 12360
   cagtcatcat cgggcgattg tcatgaacac ttttgattat caaaacgatc tttataagcg 12420
   tatcgcaatg gaaagtgcgg ctgtggccag cattctattg tcaccgcgaa actctgacga 12480
   tggaaacagt aatagtataa atagtttctt gaacattaga aacaacaatg gagaaaaagg 12540
   ctcgaccgat tttgaaacac aggtatttaa aaattttatt tttaacgacc tgcacaccaa 12600
   cgatttagcg ttgaatccca aggcccaatt tttagttaaa caggcctcat ttaatgtgat 12660
   agacgagatc tttcaacaga catataacga aaaaatcgac aagattctgg cgtttgtaga 12720
   tgacactgac gatattcgta ttcccaacga tagatgttta cattatttga tagcggaaat 12780
   cggtaaaatc gttaatgttt tgcagcacgt taatcgcatg tcaaagtttg aatatagtat 12840
   gtacgtattt atgccgtatc taaaacaaat aagaaaaact atcattctat tcgtcaatga 12900
   cttttgctgc aaaaaactcg tcgaaaacta tttattaacg ctcgatgtga tgattgcgga 12960
   ctctttaaaa ttactagaaa cgattcaaat gatcaataaa cgtgtagatg tgatgaacgt 13020
   gtttctagat cgacccgtgt accggtgcaa catctgcgaa gatactagtt tagaatcgcg 13080
   tttcctcaaa cccaacgaat gctgcggcta tagcatctgt aacatgtgct atgctaattt 13140
   atggaaattt tgcactctct atccagtgtg tcctgtttgt aagaccagtt tcaaatcttc 13200
   aggctctgca gcttcttctt cgtcatcgtc gtcgaccaag catcaagctc tattcgaaga 13260
   ataaaatata taagatttaa atatgagtct aatcggcgag agacagactt tggaaaaaaa 13320
   gcacatgaaa cacttgtttc tggcatcgta ttttaatcta aacaattatg actatttacc 13380
   ggtcgaaacg accgctttta ttggcgatta tttacgcaat aatttcgaaa gactagacga 13440
   ctcgactctg ttgcaatata ttaattattt gaatagtatc gccctcaaaa atctcgtagt 13500
   cgacaacagt gcagacagtt ttaaatatgt aaagccacag tttaagttta tctgcggtcg 13560
   caacaatatc gacattatcg agttcgattc aaaggttttt attaaaccga aaaccccaat 13620
   ctacgctacc aatttattcg taaacgaccc caaacagttt cgattgattc tctatcaaga 13680
   attttcaaag gtgtttaacg atcgattatt tgtcaacaat ggtgacactt actgcataat 13740
   cgacggtggc gaaggctaca ttttcgaaga cgcctacatc gattggtgcg ggattcgcat 13800
   gtgtagtgtg cccaaagtcg aaaacgatca atatccgtat cgattgtatt tgatcggaga 13860
   acccatggct caacatttta tcaggaacaa tatcacgatg ctcgcgggca gcgactacat 13920
   tctcaaaaat tttcacaaag gattgccgct gtttcgcaac aattatcggg tgattaatag 13980
   taaaaaattt gtgacccgaa agcctaatca gctttttgca gaaatgcgag cggaactgga 14040
   cacgcacacg aattatataa aatttattca acgtgactac atttacgatg ccaatttccc 14100
   tgaagatttg ctcgatctcc tcaacgatta catgaccaat acatctcatt acaagtttat 14160
   caccaagttt atcgagccgg gcatcaagat gggcaacagt tatagcgaga ttgtggtcga 14220
   tcgttatgcc gttaataagt atcgaaaatt aaacataaaa agcgagccga acacactctt 14280
   cccggcgctg cgttacaacg atccgtccta tatttttgtg agaccagata tcaaacaaat 14340
   aaagggcact ttgaacgctt ttttcgtgcc gaaagaacgt ttattggtga tcttggccaa 14400
   cagcagcctg ttcggatcca ccgagctgat tcattttaat aaacatcgaa acccaaacga 14460
   gctggtgcat ttcgatagga gtttgctgcc gtatagacag ttttcgcctc cgcagatact 14520
   aacccttgac acgtatatca ttgacgccag tcacaaattg tatttaacta agcacatttt 14580
   tggtaataca gtgcctgcat atttaattat aagaggagat tacgaaagtt ctcaatttca 14640
   agatttgaaa aatctgaaaa actcttgggt tttgaatact ctacttaagc tgtttataga 14700
   tccgcaataa aataaaatat ggaagattta cgttcgccag gaactacgag tcgattcaac 14760
   ccgaacatgt tcaatcccag catgttgatg accattctca tcggtctagt aattattata 14820
   ctgctgatta tgctcttcca atcgagcagc gcgggaggcg gtagttctcc agcagcggcg 14880
   cctcaagcgg gctttacgaa tccgttgaat gccacgatga gagcgaatcc ctttgtgaac 14940
   acaccacaac gagcgatgct gtaaagcgtt ctacgagaat tataagacaa tataaacaaa 15000
   atgaaacgtt tcaagtgtca gacagcacca aaagtgagaa ccgtcaccga aatcatcaac 15060
   agcgatgaca aactacaaaa ggaatacgat ctcaccgagt ttgatgtgaa aaacctcaac 15120
   agtctcgaaa gttatgatac gttaaaaata aagctagtga tcgtcaaata tatggccatg 15180
   ttgaatactt tgcagctaac tcagccgctg ctgaccatct tccgagacag aaacgccact 15240
   cgcgagatcg ttaccgttgt tttggcctca ctcggcttcg tacacaatcg cgtaaatcca 15300
   ctagtgaata attttaatcg gaaaatggag tttataattg tggaatcgaa aaacctgata 15360
   attcccggag aaccgattct atttcgacac aacgagaatg aggatatcgt ttgtattatc 15420
   gatcgagtca gtatcgtcaa gatgctcgaa aagcaattcg acacggacat gaacgtcagc 15480
   aatattattc aagaacatca aaaacttaaa ctcatcaagt cgtttacgag tgtaaagaaa 15540
   cgaaaatcgt ttgacgatca ggacaatagc ctctacatta agctgaacga aatcgaagcc 15600
   acccagtaca cgactttgct gttcatcatg gaacacgctt acggacacta ttatattctc 15660
   aaaaactatg gcatctacaa ttatactcag tcgcttctgg atcataccat ttttactcaa 15720
   aaatacaagc cttctttgaa tgtcaacttt gccaatctac ttttgagtaa attcagattt 15780
   accgtcgagg aatctgaggg caatcgaagc aaatcttgcg gcaacaatag tttgtattct 15840
   tctaaaaatc aactagaaat attaagttat agcgataaat aaaattcatg tggcttttac 15900
   tagcgatttt tattattgtt aaactattag tttttcataa aatgcaaaat atgcatttag 15960
   acgtacacaa tcataaacta tgtccgaatg gatatcacgg cctcgtaaat gatcctttcg 16020
   attgtaacgt ttattacagt tgtcctcaaa aaactcaatt ttattgtcct cccgatgaac 16080
   aattcgatgt cgagcgacaa ttatgcatac caaacgatat tcatgacgat agctgcatgg 16140
   ccagaaaata caaaagctta cttctctaat ttttaataaa actcgacatt cgcacactag 16200
   ttttttttgg ttaaacacat tcgatcctga cgtcttctct gttaatgcag acatttggat 16260
   atacaaaagc tgccatttca atgtctttcg ggttggtgtg agttttcgaa agtgatgcta 16320
   taaaatgcga tggtatgttg ccgcggagaa tgaccagttc tttagcgaaa taattctcaa 16380
   ttccatcgac acatacggtc acgcctcgca cacgctggaa agaagctggc tcgtgatttt 16440
   tattaactgt aaaaatgtgc cacacttgta tttcgccttt acagtcgacg acggccggca 16500
   acactggtct cgaaaaattt tgatgattca ccaatctgac gcaaaaaaac ggtaatctga 16560
   cgcaactaat tacatacgcg tttctaatgg cagtgcgatc gatcggcggc aatagacact 16620
   tcattcgtat tgcaatatta actcggtcag cgcgcccgcg ttcgtagccg cttacgagtc 16680
   ttgtggaagc gaaattgcgg ttattaccat cgccgtcagt gtcatcgctt tcgtttattt 16740
   gaaacggtat gatatttatc gagttgatcg catttatcac gctaaatgta attaggctat 16800
   ctttatcttg aggatagtat aaattgacaa tcattttatt tttacttcca gttctcactt 16860
   acaagcgttg tgatgtcgca tccaaacatc atcagtgcca tggacaacaa cgacgcgtct 16920
   atcaaatata aaaattatat cgacaacgcc atcaacactc ctacgcatac gattctgcag 16980
   aacgtcagta tggatttcga cgacagcaat attctggatt tcggcaacga aaatgatatg 17040
   aatgtctacg acagacgaga cagtaacagc agtaaaattg agaatgatgc ttgcgatgaa 17100
   aactctcaat tttctgatgt caacgtcaac aataatgcgg acaacaatga ttatataaaa 17160
   atcatgaaaa ctgctaccga tgtcgtcgaa aacaagaatg aatatacgaa taaacataaa 17220
   actgcagtcg tttcgactaa accattcaag aaaaatccta aaaaaaggcc atcgtcatcg 17280
   ttgacgacta cgacgatgac gacgacgacg acgacagaaa agaagaacaa gtcaagacct 17340
   aacagacctc ccaattccac tgtaatcgct gagggtagta ttccaccaca acctgtgata 17400
   aagccatcga agaagcagac agtttttgtt tcgcctttaa tcaatagagg aggaaaaaac 17460
   ttgaatgttt tgcgcaacga caataataat aatttcaata atgacagtga cgatagcaac 17520
   ggaagcgaca gtgaagatag cgattctacg catccgccgc cttcgaaaaa gacaaaaatg 17580
   acatcaaaat catctaaaat gtcggtgacg ccgcaacaac aaatgcccga gattttgaaa 17640
   attaatactg ccgacaaaaa taaagtcaat gacgagaaac aaacagtgaa atataacaaa 17700
   aaaaagcaac aatctcaaga cgctggtgcc gtcgtggtcg tgaaacaaca aaaacttgat 17760
   aacgaatcaa caagtcaaac ttccgttaat gatgatcaac aacggtcgaa agattgcgat 17820
   tctccaacaa atgacttgtt tgaaaataaa ataatcccca acatgatgac catggaaaga 17880
   gacaataacc gcaagtttgt gcaatatatt ctcaacgctc acaattatct gtttatagta 17940
   tacgaaaaca agtataatgc caagactttt aacaaaaact ccaacgcatc gatttataaa 18000
   atagagtatg tgaattgcgt ccagtccata tacaagtatt ataacgccaa ttactcgcat 18060
   atcgatagaa catgcaaagt cgtgtctttc aatcgattca gattcgccat atctgtgaac 18120
   cttttaaata aaatgcagat tgaattgcct cctacggaac aatttaaaaa ggaagacctc 18180
   aagaagattt ctccgaagaa cactttttgc ctattaaatg aagtcaaaga tccggatttc 18240
   atttccaagc tcactaacac attcggcttg gacaatattt atattcaggg tcaactcact 18300
   atgctgctct cgtcgattgg tgagaatcgg gcaaagattt tgaatcagca tatcagtgca 18360
   atgattgaag ataaaagcct attcactata cctttgcatt tgtctcgatc caaggaattg 18420
   gaagaaattg ttgatgacga tctgaacccc aacaacagta acgtctcgtc ggcctacatt 18480
   cgagacataa tagaactctc gaacaaactc aagtttaagg ctcctattat tccgtcatat 18540
   gtctataaaa ccaaggaaca aaacattgag aatgttctta gtttttggat caacactcag 18600
   aagaacaaca acgagcgcga taaaactttg gcaaaatctc tgcagtttac atacaagttt 18660
   accagtgtcg ctcgagtgct cttcgacgaa accgatggcg acgtcaataa actgtttaaa 18720
   gtgaagaaag agcctggatc tgtggcaatg attgaagatt atctacaggc ttgtgaaaaa 18780
   atacccaatg gcaacaactt tattatgatc aacacactca acgatgaacg cgtgacaatc 18840
   atcaaggcca aaaatgaatt cttttggatt tgtactaata atcctaataa tttaattcac 18900
   tgtatcgata tcattatggc ctttaaaaac tttaatcatc atttgctctc tttgattccc 18960
   agcaatcgta aggatttgaa caatcgtcac agtggattaa taaagctagt ggcctatcat 19020
   ttaggtggtg atgttgacat taattttgta cgtgccatgg ctgagaagtt taaatgtaat 19080
   tatctatata aaaaatttta aatgtataat ttttcttgtc tactgtaatt tgaataaatt 19140
   tttaaggatc gtattttgta tagtattgta gaattagttt tatgattaat aaaaattttt 19200
   tattcgtata tcattctatg gtttatttat accttcgaaa ttatgtccaa taagaaaata 19260
   aaatcatgtc gactttgacc gctatcgata tcgataacgc attcaagtat tcgactcata 19320
   tgcataggct gatttatatt cacaagtgga gatctaaatt tccaaacatt tttattaatt 19380
   acgaaattag agcagcaaca aatgacgatt attatgtgcc cctcgcccta gccgacaggg 19440
   ccattgctgt taaactcgag tttagtaaag agggatgcga gagtatttct tgctatccct 19500
   ataacgaata tgggcctgtg gattttaaaa cacctacggg ctacactcaa acgtccgatg 19560
   tggccgtaca atacgctcag ccggcatgct atcatttgga cagagcggcg gctacgcgac 19620
   aaggcgcaga gaatgaagtc caagcacccg aattgaggta taccgacggc ggtaaatgta 19680
   ttctagtcga taccctaact aaaatgtact tgaactctcc ctacatacga acggacgagc 19740
   atctgattca aggtgtcgat gatgtgcctg gatttaatgt cgtgcccgac acagatcccc 19800
   tatttcccga gcgattcaaa ggcgaattta atgaagcata ctgtcgacgc ttcggcaggt 19860
   ctctggtgaa cggcggttgc tccctacagt ggtgggagag tttgatagga ttcgttttag 19920
   gcgataccat ttacattaca tttaaaatgc tgatcaataa tgtttttagc gaattgcgag 19980
   atttcgatta tagtcgaccg tcgccagagt tgccgacaaa gcccgtcgtc gattccgaac 20040
   gactcttaaa cgagtggaaa aaccgcatcg acagccgttc taatcttttg gatgaaataa 20100
   aatttgccga atttaaaacc tattccgatt taacgatcag tagaactcga aaattagttt 20160
   atattgcgga aaagggattt catcatgaga cgataccgtt taaacaattg gaatttcgaa 20220
   aggtgtcgac cgctttctca cgtacgaaca tcgacaacac agcgtctttg gacgacatta 20280
   ttactcaatt tctcgaggac aattcgttac tcttgggtct ggcaactagc tacggattcg 20340
   atgtcatttt cgatcaattg aaggccatgc tcaaaagaat taacaccact ctaattccag 20400
   ccatgaaacg gttgcttttg gagacatcga aacgtgtcac ggccaagatg ctcggcgaaa 20460
   cttataaagc tttcgtggcc cacaccttca atcgtatcgc cattaaaact ctatcgaccg 20520
   tagcaaaggc gatgacaaga ataaccatca aagccgcgtc ggtcgtcgga attttgttaa 20580
   ttattttcac aatatctgac ctaattttag ctttttggga tccgttcggt tacagtaata 20640
   tgttccctcg agaatttccg gacgacttgt cgcgttcgtt ccttgcggcc tatttcgagt 20700
   cgatcagcga tggcaatcta gacatgatag aatggctacc cgagtacttt gaagacctca 20760
   tcgaagagga cgatgtcgcc atgtttgaga atctaaaatc cacatttgag tatgtgacct 20820
   ctctcgaaat aaactctgac ggacaactgt tggagttcga tagaggcgaa agcatcgaag 20880
   atttcgacga aatctcgtta attggaaacg ctcttgcgtc cagcgctctc tacacacgac 20940
   tcgaatttct acaatatacg gctcgacaca ataaaatatt atacggcacc gacgacggta 21000
   ttgtcgtcaa caacgccggt aataacgtta ttttgccgac gcttttcatt tttggcgcag 21060
   tgatcatcat atttatacag agtcaagata ctattcaaaa taatattatg gtatcgctat 21120
   tcatcgtatt tctactgatt gctgtttatt taatcataca gaattcttta ttatactact 21180
   tgagtttaag gaaatttacg agtaggacac aggctaaatg gtatgataat ttatacaact 21240
   aaatagagat taaatatatt tttattcaac aaatcgaacg atatgattac ttaggagagg 21300
   acttcttttt tctgagcgga acatcgggca cgtcggggat ctcgggtatc tcggggttga 21360
   gaatctcagt aatgccgtcg acggctgtct gaagagtggc gagactcgcg ctttgagcgt 21420
   cgagtttctc gtttaattct gtgacatcgg gtagattggc gcgaacgtcc tcgactgccg 21480
   actgaagaac atcaactttc tggctgacgg cttggatgtc ggcacgaatc aacaacaaaa 21540
   tattttgaga catgttttaa tttttttaat atttatacga taaatggatt tatacttatt 21600
   atatgtaatt ttattgaggg acaattaacg gaccgtccaa tctaattctg tctatttcgt 21660
   attcgccttc caccaaagag atgcttatct cttttttatt gtgaaaaatg gcaatatttc 21720
   taggtagatc gcgatatgtt aaacgatttg ccgtcgtgga caaagcaaac tttttcacgg 21780
   tcatatacgg cgaagaatag gccatctgtc gtttaccata aaccgacatg ccattacgca 21840
   acaaactgac atatactgtt gaatcgtaat taatttgacc agaaaccaat cctttcggcc 21900
   ttatgcccga cactggaaac agcacaattc cagcgtcata atcgtcatat cgacacgtga 21960
   ttacagagac cagacgatcg ttaaaaaata tcggtgcgcc tatccaaatc ttatcggcaa 22020
   tccacagatc gtctacagca aaagtgcgca actgtccgta gatcaggcga ttggcgtgtc 22080
   tatgaaaatt agtgtagacg tgttccggct gcactctaaa caatatgccg tcgtttagca 22140
   agaccatcaa actgtcgttc gagttgtcga tggcggggaa cacaatatcg ctggccaccc 22200
   cgggaaaatg atgaagaacc gacagcggcg gctgatcgtc accggttttt atgtaattcg 22260
   aatcggaatg cggtcgaatc gtctcaattc tgacagcttt gccgtcgacg gcaactacac 22320
   gaataatttt ttcattttca tcaatactat attctacatt taatttcgtt ctccgagtcg 22380
   tcgtcggtgt cgacgccatt gacgctgacg ctattgtcaa cataaatgac aagataaatt 22440
   gagattgaat cattttatat aataccttat cttgtgtttt ttctcctacc aatctgctca 22500
   ttaatagcat atttaacctc tgacattacg agttgagtat atattaattg agatgaatct 22560
   ttgtcgatta ttaacgaacg aaccgacgaa acgaacgagc caaaatgcct gtttttaaag 22620
   gctctaacag caaaaaaaat ggagtgtcga cgagctgcac ttctacaatc actaaaaata 22680
   catggacgga cactagaaac accatggtgg tcaagagcaa tgacgagaat ctaaggaaaa 22740
   aattaaacga aatccttcaa gccaagaaac aactcagcat tcagatggaa cattgggaaa 22800
   gaatcaagcg cataacgaag gatcccaaag aaatagcagc catcgaatgt aagctcaata 22860
   aaattagaaa tgattttcta aattttgggt caaataattt ctagtcatgg ataaaacaaa 22920
   taaaaagtgt gtgtataatt ttaaaaagga gttttattat aattttcatc attaatacca 22980
   ccaccaccac ctaaatctac atcaataaat gtcgcagaat tataccaatc gtcatcgtgt 23040
   ttgctgactt tggtgtcgcc gacggatgtc gatgaataat tttgttttag atcatcaaaa 23100
   atatgatgac agctctcaaa atcatgtcgt tggcgacgac ggtcattgcg cgatctccgc 23160
   cttggcgcct cagttaattt gcacttgaat aagctcccgt ccggccagta gactctacgg 23220
   ttgacagcaa tcaatttgtt ggcggcataa cgtgatgtca atacgattcg cttgcgggtg 23280
   catttgtcgt gtttgatgtc agccttgatt ctgccgccgc acacatgatt gataaaattt 23340
   ttaataaatt ttttatcaac attctcgcca ttgacataaa agacgaaaca gtcacgatcg 23400
   gtatagtcgt acattttttt ccttattatt cctcatcttc tgcaggtttt atgtgaaaaa 23460
   ataccatcgg aatgatggaa agagacaatg gcttttcctt aagtttagtt ccagtcagag 23520
   tatgtagcga ttcttccttt tggccttcct tcacaccttc tactattgat cccacaaaca 23580
   ttggcacttc ttcagaaggc ttatcattgt cctttagctt taatatctca tcgaagcgtt 23640
   ctatagtaaa agattcacat ttgaccgtct tgtctagttt gccagtgctg aagagcagtt 23700
   gattatgctt tcgagaaata tcaaaaaatt tgttcacatt ctttgcgtat tctttgccca 23760
   tgttgaagta gtaaatgttg ctaattttgc agtctttttc tttaaaatgc atcgatatga 23820
   tattgccaaa aatctcattg tacacatgtc tgttggccga acgtatagct aaagaatcgc 23880
   catattgacc acgagtacgt ttcaccatag cgtaatcgaa atagaaaaac ggttttaagc 23940
   cacccttgac acgcataccg atctcgtata catttttctt gcttgacccg ttgggctcga 24000
   cgacgcaaat ctgattcaga tattccgtag tttccatttg caaatttagc agtttactca 24060
   gatgattttc aatgtgtata ttgtctcgaa tgaatccaaa gcactccgtc aaaatgttat 24120
   agggcgaaat gcaatcgatt attttattat ttacgtaacg ttgagacaca attgtattga 24180
   tcatgtccga catccaagaa ctctcggtct tccataaaca aagggcacta tcagaaacat 24240
   caaattcgat gttttgagcg acatcatcgg aatcgatggc ggctccggcg gtgataatac 24300
   tctcgtactc ttccctaggt ttcttgctgt tcgaatcttg atcgttggta tcgagcttcc 24360
   tcttcatttc gctggcgctg gcgctggcgt tgttgaatcg agatgccatg ttggacgacg 24420
   tgtgtctaaa gactgataca tataaccgat cggtaaacgc tttttatacc atataccatt 24480
   aatctatctt tatatattta tctagtaagc gtaatcatgg agtatcgttc gtgttatctt 24540
   ttgagcaata gcgccttttc ctcgaagagc ggtgtacgtt ttaaacagta caaaaatctg 24600
   atgaatcttg ccaaaggcct tataccatcg tcgatagaac cgatttcgat catagaattg 24660
   gaaaaattta aaatgataat cgagcccgac accaattacg tttccaatat acacgatttc 24720
   catttttata ccgacaataa agatttcact gtcgttcacg ttttagatgc gaacactaga 24780
   cagtttttgg gtaaactcat tattaactat aatatcgatg acgctgatga tcatggcatt 24840
   ttcgggttgg ttgccgatct cgccaacgat aattccgacg ataacgataa cgtttatcaa 24900
   gatagtttag atattgaacc aatataaagt tgaaattcag caagcgttac tttattctag 24960
   tagtggagct cgctttggaa cagaactaaa ttcattattc gacatggagt acctgacaaa 25020
   tcattcgata ttgaatatga tttccgttta ttttgaatca ttcgatgatt ttttgaatct 25080
   gcagacggcc atgcaactgg atcattttac tttttggaat ctattctatc cgggagccat 25140
   caaaaagaat aatatttaca atagtcaata tgccatgtgc tatttcgatt tgctgaacaa 25200
   gcccaaggtt tttagaaaat acaagaaata tgaagataaa tttgatttat tggtaaccga 25260
   cgacgacgag tgtgtgggtc ttcatttata tctgcaaatg atcgattacg attaccagca 25320
   ccaatacaga aaattgttgc cattgatgca cgatcgacag gccacattcg atgccatgta 25380
   tgaattattt tgcaagagtg tcgacattga ggatatgttc agtgatgtgc ccaggaaaaa 25440
   tatttctgat ccggcaagtg aaagacttaa atataaatat ccgattaatg tatgtaaaat 25500
   tttaacatca agagctacat acgtgatctt taaagggacc gatttggagc tacgggtaaa 25560
   ttttaaaaaa tatccaaagc tattggagta tattcagaaa ttgcttacgt tcgaactcga 25620
   tcctgaggag agatgctgtt ttgttaaaag tttactcgga aacaagagta aacaattgtg 25680
   cgattggcat aacatttttc aatattacgg caagtctgaa gtgctgtata aagcttttat 25740
   aaaaaataat gttttttata aattagtgtt tggtatcgaa aaggatattt ttcttcaaca 25800
   atacatcgag agcaccaatc atcttggtaa cttgcgtaaa attctgaaga atgactttaa 25860
   aaaattgaag tttaacactg aagtggggct cgataattca tttccagttt taaaatatgt 25920
   cgatttatat gtactcaaca aaatttgtct tgagtttaag tctgttgaaa aaaacaccaa 25980
   aaagaagatt aattttcttt tacagaatat tttattcatg tataaatcaa aaatattatt 26040
   agataaaaaa atgttgaagg acgtcaaaca tcttacaaac ttagctgtaa atagaatttt 26100
   aatttataaa agagtcgatg gatattataa agacatactt gattataata gaaacgcaat 26160
   gatccaacaa tttaataaca ctttgagtaa aaaaattgac aaaaaaatca ataaacacta 26220
   caatttcaat ttaaaagttt ctcgcaagcg taaattgtct acatgttcag acatttaaca 26280
   aatgattgta atgtggtttt gttacatata attaaatttt tataaaattt ttttttcgtc 26340
   tattcatttg tttcttcatc ctcctcatcc attgtttcga acgaaacgtc atcgacatcg 26400
   tcgagacagt cgtagttata aaaagtgagc aattctttga tactcgaaaa tccggtcaaa 26460
   tccctaaaca agaattgagc gtttaatttc cagaacggca cgctcaaata cgagtaatac 26520
   agttctacaa aactgatcac acccatttcg ctgtttaaaa actcgatata agcggaattg 26580
   ttgacgttcg acgactgatc gaaaacgtat tttcgatgaa aatcacatcg aagttcggca 26640
   tttttatgat aacacaaacg acaatggtaa aatttttcta actgcaaagc gtttaaataa 26700
   tctcgcgctt cgtccgagag aatattgatt tcttcgatgc taaaatcttc gtctttcttt 26760
   cgtttcaaca ataatttgtt tctacccata tgttgaagaa catcgcccag tttcggaggt 26820
   agcgacggca tcggtggcgg tggcggtggc ggcggtgaca atgacgatga tgtcgttaac 26880
   gccaatgttg cggtagctgg tggagttttt ggtcgtatcg atgccatcag ttttgaggtc 26940
   aatcgacgca aatcctcacg aaacgtctcg tttttattca tattgtcgtg cgcgagcgcc 27000
   aataatagag ccttaatgat actatttctc gctaaattat ataagtactt tttagtcaaa 27060
   caaaatgcaa gtgtttgtaa aaacattaac tggaaaatcg atcaccatcg aattagaatc 27120
   aaacgacacc atcgagcaag tcaaacaaaa gattaccgac aaagaaggga tacctcccga 27180
   tcaacaaaga ctcatttacg gcggcaaaca actcgaagat gatagaaacg tcagcgacta 27240
   taacattcaa aaagaatcta ctatccatct ggtcctgaga cttagaggag gaaaaatatg 27300
   attagtttga atcaaatcat aaaaatcgtt ataacaaaat ctaatctaca cggcatacac 27360
   aatgtcaagt tgattgacga aaaaaacgac aaaattgatg atttttacca agccgtttta 27420
   atcgtgcctc cgggcattgg taaggtattt taccagggcc tacatgtcac cggtcgtgta 27480
   ctcagaatcg aaggaaccaa tgtagatttg atatatcaaa aagagtaaaa attttttaga 27540
   tttcgtgtgt gtactgtgat atagtgtaaa atcctttatt tttcgacatt caatatattt 27600
   tgaaatgatg tatagaaaga aataaaaaaa ttgtgtacat ttaagttttt tattttacaa 27660
   ttaaacagat gacatttgac tctcatcaaa atcgtcatcg acaatttgtt gatatgtcga 27720
   tgatgatttc gatgacgact tgtgttgctg tttagccttt tttacattca aattaatatt 27780
   agttttctgg acacgttttc tttgctgttg ctgtttagag ctgccggcgg cgacatgatt 27840
   gtcatcattg ttggaggttt taatcattaa tttatgttca gacgacgacg acgacgacgg 27900
   tacaatcgtt tccaataatt ttttaaagca atcgacacca ttctgtatca tgtttcgatt 27960
   caaacgctca aaaccgacat ccgatccgtt actgttttta gatagcttga tttcgtatag 28020
   aaagctttga ttaggagtcg atccatcaac aaacgtttcg ttcatcactc gataaaactc 28080
   attgtataat tcagcacgct tagcgttgga ggcatcaatt ttactagatt cggcaacatt 28140
   cttgatatca ttttcttcaa tgttggcgtc agaattagtc tgttcggaga tgactggcgc 28200
   cccatcgact tctatacttt tgcctaattt ttccataaaa tcaaaaatca aaacaaacgg 28260
   cttgatgttc gaatggctct tcataatatt ccacatcatg tgactgttgg gccagaccga 28320
   ttgcttgttt ttcgtgtacc agctattgaa taggatatac ttgttattgc tgatcatctt 28380
   ctttgttttc tttgcagtcc gtttgtcgtg caacaaaata atggccacgg taaactcgag 28440
   accgttgtcg ccatacacag gcatcatggt gattctatac ttgattttct tcttctcgag 28500
   cagttgaatt gtggtattga cattttcaaa attgttcctg tcgaaaacta gtttgtcgct 28560
   gtatttgtcg aagatttcga cgagattatt acagtttttg gtggagttgg ccatatcgat 28620
   tcgaagattg tttatattca gtttcgaggg atttatttaa atgaaatatt ctttttaatt 28680
   ttaaatgatg acactgcacg cgcttgcact gcgttcgctg tccgccaacg gttatgagtg 28740
   ctttatcaag attattcctt atatagtcga attgagtagc gaatccatca tcgaacacat 28800
   gatcacaaac attggtcacg tctcccgtat gtttacgctt gttaatggtc tctcgaacca 28860
   aagcgtaaat ctcgcttcta gtcaagcagc attgactata tttttcaggt cttgactgtt 28920
   catcattgga atggtggtgt tgtcgctgct gttcgacgtg atgttggtgc tggaccgacg 28980
   cgtggctgct gtcgatttcg cattcgttgt ggtcctcgaa cgcaaagtcg tcgccattga 29040
   cgatttccat cgttccaatc gacgattcag ttttaaagat aaaagaaaaa atccaaatga 29100
   atttttatta ccaaacttgt cgtagccgtt ttcgtctttt tcgcagttta taacgttact 29160
   ttcgtcgaat ttagctttga caaatttaac gtattcaaaa aactcacgat aattactaga 29220
   atcgtacgtg atcaattgct tctcgttcat gtactgaaaa tagtcttgta acgacacgat 29280
   atatacgtgt cgaggcaatt ctctgttgtg tcgtcgagtc tcgatatcga ctcgatagtc 29340
   gttgcctttg tcgccgccgt tattgagttt gacacaatac gtattcggtt ctcgagatac 29400
   actgattagc aaaccgttta aaatgcccac gtaaatggcg tatttgtacg tgacgcccga 29460
   gtctatccat tgcaaaataa acggccgacg gtagacatag gcgctttcga aaaaagtgcc 29520
   agtctcctcg ataaactcgc gaatagcagt ttcatgatcg aaaatgtctc gattgtcgcg 29580
   ctttcctctg ggtatagaaa ttttttccaa aaagtttgct ctttttagca cggcactgtc 29640
   gctatattcg acggtgctgt cgtacgatcg tcgagcacac agcagcaccg ccttatcggg 29700
   ctccattatc ataaacaggc ctgcacttct catcgtaaat aaaattcaac tactttatat 29760
   attaataata cttttatttt atataaaacc attattatca cataaaatct taacaaacat 29820
   attagtacaa gacatcaatc ctctcgaagc tgagacagat cattacaaat ttttaacaaa 29880
   gactgataat cactaaaaat tagttcaaat gattgtgcgt agtcatgtgg agaaatctca 29940
   atgtcgttta actcgaaaaa agtattcatt gacgttaaat tcttgtgatt gtccaggaaa 30000
   aatttttgaa tcaattttct gttttgatca tcctcgtcgt cgtcgacgcc atctccatct 30060
   tttctagcgt ttgaatcgat gaattcgtcg aaaacgtaaa ggacagccat ctgaactcca 30120
   cgaacgtacg gatttgtgta aatagtttta tttaaacact tgacaatcaa atctgtgccg 30180
   atgtagtcat aaatgtcaga gtcgtcgtac ttatcatgga gaactagacg aattttatta 30240
   caaagctgtc tgatgcaatc caaatttaaa atttgtattc ctggattgtg ctcgaatgtt 30300
   tgcagataat acgcttgctc gtgcagcccg gacagatgta taaaacgaag gaatttggaa 30360
   tacatttcga cttgaaatga cgtacgacaa caggcgaagc accttcgact ttaaatgatg 30420
   taaaacaacg ggtgaagcgt ctatttatat gccactccac tctacacaat catgataacg 30480
   agtctgcatc tgatcgacaa taatatttat tcaatcgttg cttgataaca ccacacaaat 30540
   tgtacgctcg gaataattta gccatcaaca caagaatatg gtttttgtcc gtggattcga 30600
   tgtcggtcac tcgagtcaag tggaaaaagg cgctggcgta attattattc attaatgccg 30660
   tgtagcgttt aaataaatct ctgctgccga ttcttttcaa actcgtagcc aaccatagcg 30720
   tgtacgagag aatcaaccgt ttcttgtggt cgatttccgc attctcatcg tcgccgacgt 30780
   acaattgaag ccaatcgtcc agttctttgc gtatattgtc tcggacgaat cctttaattt 30840
   tattttcatt tgttccgttc acatgaacat ctctcactac cgctatcaac acttttaatt 30900
   tcttctgtat attaacgagt ttgtcgtaga gttggttgga gtcgggtaat gttgtaaaaa 30960
   agtcaatatt tatttgaatc tgcatcacga cactatcgag attatacaat tcgtactgta 31020
   cactgtcagc ggccaaacaa attaatccga aatcaatcat gctgtcgttg acaatctgat 31080
   cagagttcga ctaaacgatg gccgtaatgt gatccggttt tgattcgctt gcctttaatg 31140
   aaatcaatct taatttcgtt atcgttacag agtattcgag attgggcagc agctcgtttg 31200
   atcgaattta ggcgacgggt cgacagctcg agtggattat atatttctga tttctccagg 31260
   gtaaacgagt tgagatggcc acaattgaac cattccagtc caacgattaa attgatcaag 31320
   aaaataattt cataatcaca gtactcgaat acgaatttat tacaaaggca gtaatagtag 31380
   aatttgagtg aattatatag atagaatagg tagtttccct gtttcagata aaattcaaca 31440
   tctgtcacga ataaaatgtt gataattttt tcggaaatta aatgctttag tttttgtaga 31500
   tatatcaaag agtttttgtc ctcgtcgagt ctgatggcac acacgttatc catgtacgcg 31560
   ttattgatgc aaacaaattc ttgtctcttc aattctcttt tcagtgcagc acttttatcg 31620
   ttggcaattt cgatgacgat atatttttga gagctcttgt gtctctttat cacgtccgaa 31680
   atggatgtaa tcgaagcgaa tagtttttca aataaattat tctcccagcc tctggcgccg 31740
   acgctggtct tgtcgttatt gttctgatgt ttaaagtcca gtccgctctg aattagcttt 31800
   gtggtgatgc gcacgctcaa ttgttgtcca agagtataat gatcttcgta gcctctctcc 31860
   cacatgacgt tacaagcgaa agcaatcaaa tcggacacgt tttctttaac caaaatcttg 31920
   tgtaatcggt cgaaatcgcc tttgtaccaa cgatcgcacg ccaacagttt cagcaatctc 31980
   gattgatgtt cggtcaggtc gtttcctcta gtggcatcga tatagtgttc gatgtcgcga 32040
   ggactcgatt tcaacagatt tgtcatgtaa aatacgacca acgtctgttg tatttgcacg 32100
   aatccctcgt cgttgacttg taggcgaaga tctgtcgtgt gatatagatc accatcgcag 32160
   acattgttgt cgtcgacata tgccagataa aaagagattt catccgctaa aaatttacag 32220
   cactgaggca acagctgcga agttgtagta tagtaacgtg caaacgccat gccggtcatg 32280
   aagtaactat ctgtcgattc gatctaatcg ctatgattca aatctatttc aactgaccta 32340
   gataaaagag ggtttaaata tctcgataaa atgcaattga gattattaaa tttgaagcag 32400
   tgtcctcaag acgtttcgaa aatgtttaaa tttcccaacg aaatgatgga agaattcctg 32460
   atgagaccgg tcattcaaca aggatttcaa gaactcgaca ttattggcaa gataaagaat 32520
   acagaacctc aagttttgat ggaaattcaa cgcctcaact cgagcggtat atatggcatg 32580
   ttggccggag gatatgccgc ttttctattg ggcctgaccg acacatacac tgatgtcgat 32640
   tacttttgtg aagatatcga aggtgtctat caatatgtca tcgctaacaa gaatgattac 32700
   cgagaacatt cgcatgtact gagcagaccc gatgccgtca ctttggtttc ggaagggata 32760
   aagttttatg tggtcaatca tgaacattca aagttgcaga taatatcgag gcgcatgtct 32820
   atgcgcagaa gtatcaatta caaaggatac gccttctatg cagattgctt acgctctttc 32880
   gatttgccca tctgtaaaaa gggaatattt ctatgtccca tcgaatgggt cgggcaaatc 32940
   gagtgcatcg aatacacaaa cgattcgctg cgattcatca tacaaaatta ttctccaatt 33000
   aaaccatcgg gaaaatatga agcatcgtca aaggcgataa cacgcaagga aaaatacagc 33060
   gctagaactg tacaccacgg attgcctact actctgaaag aaatttgtca agacgcaata 33120
   ctttatcaca gcgatcaagc aaacactcaa gattttgtca tttattgaaa atattgtaac 33180
   caaccaaaaa tatattagtt tgcttcttta ttatgtttgc ttttcattat tgtttgattt 33240
   tattttgtga ctaataataa acgaaaataa aaaatatgtg tattttttat ttgttgtaat 33300
   taagtcgaat gacattgagt aaaatgaaaa cacaatactc aaactgcatt atttgtaaag 33360
   gaatggttta tgtttataaa aaatacacac aatctttatc gtcacaaata ttttttcata 33420
   attatcgagc catttataag aaaaacattg gatatctatg ttgtaaatgt tatcgtttac 33480
   atgtaataaa aaaataaaaa ataaaaaaat tgtaaattga tatgaaaaaa atttattaaa 33540
   tcaaatacat gcaaaacaat ttattaatct ttcttttcaa tgttggctga agtcgatgct 33600
   ttgtcgtcaa aagtcaattc ggcaattttt ttatttaagc ctttaatcac catcttgagt 33660
   tgcttttcat tttcttttgc cagtttcatc acatcttcca tagcaagaac gttcactttc 33720
   gattgatacg ctagttcgac caaaatttca aatagcgaaa gtccatgcat atccaagtcc 33780
   tttaatacat caaatatcat gtgatcatta aaaaagttgg tatacgcttc tgttgccttt 33840
   tcaaaactac catcttcaga atcgtcttcg tcgacatagt cattgtcgtc atcgtccgag 33900
   tcttcgtcga ccgtttcttt tatgctcacg tcttctttat caacgtcgac tacttcttct 33960
   ttgttcttgt tcgaatcgtc gattgcgttt cgtttaaaaa ttttcttgtt gccggcgaac 34020
   atgttctcga cgtcgtcgtc gtaatcgtca tcgctgctca tgtcttcgta tgcggcggca 34080
   atttcttttt gcaattctat tacgccttct tttttcagaa aaactagttt tgtcttcaac 34140
   gtttccaacc tcagccatat tttatattct tcttcttgga cgaccgaata gtcaataaat 34200
   tcgacacgtc tactttttaa ctttttgaac atatcgttaa attgttgaag taaaatggtc 34260
   cgttcgttga acgttatttt taattgttga gacataatat atttgcgtgc ctttaatatt 34320
   atacattcca tccatttcga gggatgaata ttctctaaat cgagatcgcc caataaaagt 34380
   tcgttagagt gctttgaatt catctttact attgacttgt ttacgcggtc aaaagatcca 34440
   gaagactgtg atattgttcg gctcgaactc ctcttttata tcatttcttt atcgtatcat 34500
   agtattattg cagtgcaata agctgcccat gactctgaat tctccgctat tatcgggcag 34560
   attagaacta ccgcggacac attgcaggct ttcggacagg cgattaccca caaacgattg 34620
   attgatagtc cactgatcta atcgtgttcc ttcgactttt tcgtgtcccg tataattact 34680
   tttgataaat tctttaaaaa tattatctgg agtattattg aacagcatat agggtatatt 34740
   aaagattgga tatcgcttgg cctcgagatc gtggtgattg ccgccgcgga cgacatattt 34800
   agtttctatg tcattaaaat tcgactgtct cgacaagaaa gatatgggcg atagacatat 34860
   ctgcgcgcta gttccgtctt ctgccatcca ttctgtcaaa tcttcggaac cgttcatgac 34920
   tcctttttgt ccgtgtatac cacagatttt gatgccttgc aggtcacttg tcgacgtgat 34980
   catggtgact ttaacgtaca ccgtgtcgtt gtgtacggcc atagtagagt ctaaacactc 35040
   gatcctttga ttgaatatct ctcgaaagta aatgtaaatt ttcgatagaa aaaaattttt 35100
   atttttacat gcctcgattt tatagcgttt accatcgtat atccagccga tttttacatt 35160
   cgagacgaca acgcctgcca tatataaaaa atttcctcct tcaacttgta caaaattgtt 35220
   ttctttactt ttaaaatatt ttataaccgg cgtctcagtt tttgaatgat tgagttttcc 35280
   ctttaatttg ttgattttgt tgttgtacag tctgatcggt aaattgatgt cgggaatata 35340
   tggatcttcg gcagtcatta gtttattgtc tcgaaccagc gtccacagtt tgaacatttt 35400
   attattcact tgtattcgcg gactgacgac cacagaatta cctacgggca aagtcttcaa 35460
   aaaaacatcg gaatcgagtt cttcgtcgta gcagctcacg ggcatcgcat ttttcaaatt 35520
   cgtcagcgaa actatgagtt ttggtacggg aatcgtgcta aagattcgtg tatagccaaa 35580
   gtaataatat tgcaccagct ttgacatgag gctagtgacg tcatcgtttt cctctatctt 35640
   accgattttt gggtatttct tcaagatcga ataagaatta tgatattcga aaggcgttag 35700
   cagagtcgta atttttttgc ccgcgatact aatcactcgc ttgatacaca ccatgccttc 35760
   gtggtgattt acaaagagaa tgcggttggc cagcttgagt tcaatgggac tgtattgccg 35820
   tttgagttga tagaaaatca attgtaaatc gtcaaaacgg catgcgtata tggtcggccg 35880
   gtcgttgaac gcgattctaa tgtttttttc atcgacattg gagggatcgt tggcgtaaat 35940
   taatccgttg gtcagcaata acttgaattt gtccgcgact aactggtaat cgacggcggg 36000
   aagacgtacg tttcggcaga gaaagaattt tttaccggcc actgtcattt cgccatgaaa 36060
   aaaactatcg acaaacttga caaaatcccg ttgttgcatg agcatgtctt ggcgcatcgt 36120
   gtcgttagtg attcgcacga cttcgttgcc gattcgatat ttaagcggag gaggattaat 36180
   ctcaatgttg ttgttgctgc tgttgtcttg ataattaatg aaattcttct tttgcttgct 36240
   aaaagtcttc gagacggaat agattaattt gccgttgacg atggtgtcca caactttttt 36300
   acattctttg acaaatagaa tagtttgtag ttttcttcgc tttatactgc cagttttatt 36360
   gatatcgcca tcgtttttat tgtcgataaa ttcttcattt ttcggtctaa acgcttgagt 36420
   gttgtttctt tttcgtttgt tattgctttt attttcgccc aaatcgttgc catcgtcatc 36480
   gccgccgctg ccgtttactt tattcttttt ctgtttttcg tctttcgatt cttgtctagc 36540
   aatagagtcg ttatcgtcgt cgctgacatt aacacaattt ttgtcgtcga ttttcatcat 36600
   attcgtgtat tctttgagaa tgggcgcata catcaaatcg agtaaataat cgtgtttata 36660
   tatgatttta ttagccaaac tgtcgatggt gtaattgatg tcggatttca ttatattttt 36720
   aatatcctca actaaatgat tgctctggtc cgtattaaaa tcgaatataa aatttaaagg 36780
   ttcccattta ccgctgcgtt tcagatacat ttcgagggcc tcgttcagct ccatcgtaac 36840
   aatatagtct cgggcgaata cgtctcgggc gaaaagcaca ttatcgtctt tgtcgtatac 36900
   cagttgaatg gcgcgattga tctttttttc cttgtcaacg ttgccgtaca ggaacatgcg 36960
   tttgcagttt ttggaatatt gcttgtcgta gaaattatgc agtagaatat tgttgttcat 37020
   caggatgttc ggaaaactca agtttcttcc gtcgatcatg aaagtgccgt tcatattgtt 37080
   gattttatac gtatccttgt ggcgaaattt gaggtcgaga cgagttccaa aaatcaccaa 37140
   tacacatttg tgtaggacgc atcttccttg tacatcgacg gcacagcaaa aataagatct 37200
   acgctcttgt aaatacttta ctgtacattc gttttcagat ttattattac agtttagata 37260
   gaattcaaaa ttatatttat ttttgagttt atcgtagaga ttattgaaat cgacaatcac 37320
   gtccgtcatc ttgaaactat cggtgatcac taatgatgtc gacaacagcc atggatatca 37380
   ggcctacagc ttctattcga cccgtgactc gtgcgtttaa acgtaaaatg gatcaaaaaa 37440
   ctaaaagata tttagaatcg gaagcgatcg aggatgatga ttatgatttg tcgtcgacgc 37500
   caaaagacga aaatttaatg tcatcgacga catctttgtc gccgccaccg gcgaaacaag 37560
   ctaaaataaa acgaaaaaat ttatttaaaa taaatttgga cacgcagagt ttttacgatt 37620
   tgctttcgct aaaaaccgat tcatccgact ttcaaatcaa acaacgcgta aacgaactaa 37680
   tcgactttta ttcgacggca aaagtcgacg acagtagttt ccgagacgat gacgataaag 37740
   ttgaggtcat caattctaca aatcaaattt tattaaaaat ttctcaagct tccatgatac 37800
   tgaccaacgt ccgcgccaaa cgcgtctacg acaagtatat cgagatcaaa tacaagatta 37860
   gattgtggca actcaacgaa ttgcgaccgt taatcgatga gattttcaag catatcgagt 37920
   cgatattcac cgtctcgagt gattttcaag agtttgatga tatagaaccg gcactcagaa 37980
   actttgtagc caacgagcta actcagcgac gaagtcgagg caaacgcctg cgtagcacca 38040
   ccaccaaccg tcttcgcata caatggaatg tcaatctcaa cgatgagaca aacgccggtg 38100
   tcgacgaacg gtatttactg gattatttcg gtaaatatgg tgagattgtc gggatcgtca 38160
   tgtgcagttc taggcccggt tgtgcggtca tcgaatttga tacgcttcgt tcggtcaccg 38220
   aagtcatcag cgaagagagt caactcaaac gatttattgt tcaggatctc tccgaggcgg 38280
   aattgatcag tagcgatagt gctgctcaac tacaaagtaa actagacgaa ctcgaacgtc 38340
   tatccagtga tttactcagt atacaagaga atatcaacaa acaagcgtct taaacacgca 38400
   atttcgttgt caatttcgat aacaaataaa acaaaaggca taactttaat agtattttat 38460
   tttaaccaaa ataaattttg attacattag taaaagagtt tctacatatc ggacaatcgg 38520
   gtacgctcca agcgcactct atacaagcaa cggcatggcc gcaaggaata tagcaggcgt 38580
   caatctctct gctgaaacaa attttacact cggacttaaa cgatgactct tgaacggttt 38640
   cgtttgaaga cgtgctggcg atttcctgag agatcgcagg cggcgtcggt cttggctttg 38700
   gtattggctt cggtgccggt ataacacacg attcactgat tacttgctgc acataatcgg 38760
   gtcccttgac gaacagtaca tagcgacagt tgggatacca tcgggcgtgt tgttgccaag 38820
   gcaccctatc gcaactccaa tcggataggg tacaaccgtc gtagaaacat tttactttgt 38880
   cgccgatgcc tgtgtaaaag aagcccgcct ccgctaaatc tatatagtcg gtgcggttgc 38940
   gaggccagtg acgaaagctg ttgattcgct taactacatt atcgaaatta ggatatttaa 39000
   aaataaactg attctctcgt tcatatgtat cgtatttggc ccgtttcgca tctagttttt 39060
   ttacgaacgg acactgagga gcatattttt tgtgatcacg atcgaccaca tcgttttgtt 39120
   gccatttaca aatctccact ttacagtagg cacatcggac ttgatctcca ctacctaaat 39180
   aataaaatcc gttcatcgcc aactctatcg gcggaacata gggattaatc cacttttcac 39240
   tatcgaaagt cttaagtctg ttgctacagc gctgcatggt ttgctcaaaa ctctccatct 39300
   tcgttgccgt agtgttcgtg gcttcgctta caaagtataa atactcagag ataacgaccg 39360
   tgcgcgtatt atatagctgt gatacgagag cgtgttatct aatcatttta actacgcagt 39420
   tgcttctcag cgaatcatta ggataagtaa acgacaaaaa atgatgacaa ttttttatta 39480
   tatagctttg ccacctacgc ccgcgacaat gcctactatt attttatcgc ctttacaaag 39540
   gaaaaaacga tttaataaaa gacaaaagac tcgttttata cgtatattta aatcaaaaaa 39600
   attacaaaca aatctctacc gagattaaga gttttttaac tgtttgccgt tacgattcta 39660
   aacaatggaa agaaacatcg cttacagaaa agcttaaaac taaaacaata aaaatataca 39720
   cttccagtga ggcgacaagg cttgtcgcac tgtaaacatt tctcataatg aattttatat 39780
   aattttatct tggactcgta actgtaccaa gcgaaacgaa ttctctcgcg ccagacattt 39840
   ttgtctacaa tgatactatt attctcaaca aaatcaacat catcatcatc aacatcatca 39900
   ccataacttt ttttgttttt gtcgtagcca ccaatgttta tagattcata atagcaaaca 39960
   ctaatcacaa tatagtttat gagctcttca ttttcgctac tgtcgtccaa aggaaataag 40020
   cattgagcgc agcacagatg cgattcttta ttgttcaaag taaaacagaa tttacaaaaa 40080
   aaattattgt acactattcg acaattgtct tcgacactag aatcgctacc gccgctgtcg 40140
   cttcttttga tcgtattcat caccaatgtc tttttaattt cacaccacca acgacggtac 40200
   actagttctt ttcgtacagt atcgtcagcc gttcgataca aacgagaata tttgataaag 40260
   ggtttaataa gctccatgat tcatgctcga atgtgagcca aagtcgaata ttaacgtctt 40320
   tatactacag acaaaacatg ttgataaccg tcaatcttca aaacaaagac gaatatattt 40380
   ataaaacatt caaaaggatg tggaacgaat acaaaatcga atgccaaatt tgtttcgatt 40440
   gtatagaaaa cgatggcgtg gttgccgtca ccgaatatcg aacgttaaat ttagaaaaaa 40500
   tgtttcatca atcgtgccta aagcgctggc aacgagaaca caatcgggat ccttttaata 40560
   gaaacgtcaa gtgttggttc gattttccac caaagtcttt ggagcagtgt tcggcgcttc 40620
   tcgagcatat gaaaggattc atcggagacc aacgtcccga caaagactac gccgacgagt 40680
   ataatcgcat cgtaaaagag gaatctgtca tcgatattga actggatttc gaaaaacttt 40740
   tgagttactc ttgacggagg acaagacggt ttactcgaaa acaagattat tgtaaacgtt 40800
   tttgacgtga atatcgatgt ctatgactcg ataatatttg tttgtcgcgg cgacagatgt 40860
   aaattggttc acatcattgt ccggtgtatg tttatagaat attatagata gcagatccaa 40920
   taaacgatag ttgtacatgt ctgttagttt tctttgataa ttgtctatgt gattacgata 40980
   ttcttcggga tgaatataat catagggcaa tcgagactct ttatattcga gcagatctgt 41040
   tatgttgctc acgatgtaat tgacgcacaa attgaaaaaa ttcgtgatgc cgtgcgtgat 41100
   tttagtcttg ccacgagtgt agttgacatt cacgataacg tgagctacgg tcgacacgtt 41160
   atcgaatatt ctcacataat aattgaacaa ggcttcgcta aatttataaa aattcttacg 41220
   actcacatct ttgtcgctca atatggcatt cattatggtg gcataatcga taacattctc 41280
   gatattgtct tcgaaagtta aaaattgttt aagcgtattc gacactcgag aattgatgtt 41340
   attttgaagc gcatccaaat aaagtttaat gctgtcgata cttttttgga tacacagcat 41400
   gttgttacaa tcgctgggta tttgatttag tagaggcgga gtgatgaaaa ccactacggg 41460
   agatgagttc gatccttcgg ttctttgtct tttattcata tcgatgacac tcgagaccgc 41520
   cgtcgttagc gacgccgccg gcaaagtcgt gggatcctgc gaaagctgta tcgttcgaga 41580
   catttcattt tgacgtctca ttatatgttt ttccgcctct tccatttgac gaatatgttg 41640
   aattctctgt tgctcttcat cgatcatcat cgatcgatcg atatccattt ccatttcggc 41700
   ctctacagag ctcgttgccg tagatattgc cgtcgccggc ggcggtttcg tggtagattt 41760
   taattttttc ttagcgcttc catcggtctt tgaagacttt ctaaatccta atattgatga 41820
   cgacgatgaa gacgatgaag gcggaggtga taatggcgtt tccgacgctc ttttactcga 41880
   gcgccgatcc atttcggtac gaagacgagc agtagtttat caatagacca aaaacaaata 41940
   gtcgggataa tcgtttaaag tgactataga cctcgaactg gacacttctg ttcgtgtgta 42000
   cttatcatag taaaaatatt tattatattc cgaatcgaat ttcacgaatt taaatcgcga 42060
   aagctggtga tattcatcgt aagacagtaa ttttatcttc ttacgttcaa aaatgtctgc 42120
   atctccattc gaagcggacg taatcgacat cattttgaaa aataacctcg agctcgtcga 42180
   caacacttat ataattttaa atgtcatcga tggcaagcgc ggtggttcga ttaaaccagt 42240
   gtgtctcgga gagattaact cctttcaaac ccatcaagat tcaaagtgct caatgtccga 42300
   ttcatccgtt acgagcgaac tgcagagcga tcgtacgcta taatgatgtg gtcgaaagca 42360
   agttcattaa tcacattacg attttcgaat cgttatatca aaattatgac cgacggccgt 42420
   actacatgtg cctgctgaat cctgctttcg agatcgacga aagaggcgaa tacctgaacg 42480
   ccaacgaaat gtacgcctat gttcatctgc agcccatcga cgacgaagaa gtcttctacg 42540
   gaatcgacga agccggagag agatctatgg ccacactaaa aaccgcaatt aaagcgatta 42600
   tggactgttt cgatgtttgt cgagacaaat acattttaat ggtggacgaa ctccaaattg 42660
   atgtggttta ctcgatattt cgaactatcg tattaccgca gcgaatggtg tttatttacc 42720
   agacagagca cgcgcccagc aataatgatg ttcaaatatt tagtgtgccg caaactgtcg 42780
   aatcatggga ttcgcatatt atttacaaat cgtttctact ctacaacacg atactcacaa 42840
   tgatgctcaa acaaaagaac cccttcaaca ataacaagaa aaatatttct gtcgtttttc 42900
   gtaatctagg caagtgtcct aataataaag acagagtcaa gtgttgcgac ctaaactatg 42960
   gaggaaatcc acctggacat attatgtgtc ctccgcgtga aatggtcaaa agaattttcc 43020
   attattccaa atgggccaaa actccgaaca attatcgaag gtatttcgag ttgattacaa 43080
   caccacccat caaccgcaga atgtacgagc cggacggaca agtcaatttt aattacaaca 43140
   cgggaatgct aataatactc gactggtata attttatcga agagtttcgc gcatattttg 43200
   gcatcaagta attatgcgca gatcactagt atttattgag acacgttatc agacgatgct 43260
   tactcttaaa aagtgtgtca tcgcagtgtg ttatcttatc acagattccg aacaagtcct 43320
   atcgaaatga ataaggtcaa ttttcaactc aaaaaagtaa tcaacaatat tgttgataca 43380
   aaaatgaata aggaccagtc ctcttcgggc tactcgtcat cacgatcatt atcgtcggcg 43440
   gctacatcga atcagcagaa tgctgcagat ttctatacca aacataagtc ggatgtggcg 43500
   tacgtcggtc gcaacaccac ctacaatgtc gtcggccagc gaaactatgg aaaatatttt 43560
   gatgaaaaaa cctacaaatt ttaaaatcac tggagggacg ggtcttatgc gttgcgtcga 43620
   cgaaaatgac caaattgtat actgtttaaa ctgtaagttt gtagcaccaa tgtctgtgag 43680
   ctatgaacat tttatttggc tccatgaaaa atacaactgt atggtgaaca atcgaggacc 43740
   atgcgcctat acaatcagca ccagcaacca cgatataccc accgccgata ctccgaatct 43800
   aatcaaacta gtcgataaag attctacccg ataattgtgt tcatttcaaa tctaaaatta 43860
   tgtgtattat agagaatttt aataataaag atccaagaaa attttaaaat tttgctttat 43920
   ttattttctg cgaaattttg ttctgcgcat gtgccaagtc tagtttagta aacatacggc 43980
   catataaata gagtaaatgt cgacgacggc gcgttagtcg tttgctcgat tattaaaatg 44040
   acactgtcga cgtctttcag tgctcgtttc ttagagtttg aacaagtttg tatcgacttg 44100
   agatatgtaa cttttatcga tgacatatca tgcgacaata tcaacaattg tgcccacgat 44160
   tatataatat ttttaaatgt aaagaaggca atgttttgca attttaatat aactacagac 44220
   atgtctctgg agactttggc cgaatatatt tacgataatg ttcgggtcac catcgacgga 44280
   cgtttgcacc gtaagcattc gattatggcc gagtgcgtgc gcttcaacgg agcggacaac 44340
   aacaattcca tattcatcaa tatacatgcc gaagctcgag ttattgtggc taaaaaactg 44400
   ttcgacaacg aacgatacca tcaacgcatc agtggctata tagatttcga gaatcgatcg 44460
   gatccaaacg acaaaaaaaa attattgact gacagggaac gagctctact ggatcgtgaa 44520
   tacgaaatca aaatgctcgg atcgacttga atcatttgtc gtcacggcta actaacgatt 44580
   acgagacatg atttttgcct tgtcgtcgtc gtcgccgccc tcgtcttcgt ggtaataatc 44640
   atcatcgtcg cggttatcgt cggtgtcgta atctgtattg tcttcggtgt cgtcttcgat 44700
   gtcagtataa tccgagtctt gtgcttgttg tgtgaatgga acacgatcgt cctcttttat 44760
   gtttctattg tagaaaaata atttttgttt tgccgatgac gacgaagatg ggcgctcggc 44820
   tgcaacgaca ttgccatcat tttcgtcgtc gtcgtcgctt tcgaccatat cgtgccggac 44880
   aatctcttcg gcggcggtct ttggtatcca acggtcgttg agcttgacgt agtctcgctc 44940
   gacggcctgt cgtgccatat gtaaagcgac atcttcatcg ccgccgtcga gtttatggta 45000
   ttttttaaag gtctttacaa ataattgttt agctcgcgcc ggcatttctt ctctaaaaaa 45060
   ggcctcgttc aaatgaaaca ttttaaaaat acttatctaa tctattcggt atcatagtca 45120
   gaagcggatt cggattcgct ttcgcttgta gatgaagtgt cggtggtgtc gtaatcgttg 45180
   gcgttctttc gagccaccca ctcgtcatct ttattgtgaa catatttacg tttgaccgcc 45240
   tgccaggcga ttttgaagga tatcggttca tattttgttt ttgtgtatgc acgattaaaa 45300
   aattttagga atattctttt accgttgtac ggtagactgt atgtcgaagg tggaagttct 45360
   gttatattat tgtacattat gatgtaaata aaaatttttt tgatcgaaat gtttttttat 45420
   ttgtttaaca cagtgtacac aacacagata catatataca aaacatttaa acttaattaa 45480
   tatctaaacc tgaagtaaaa tttaaactaa caacaatata atttgactat gaatagaatg 45540
   agatataaac tagattcaag tagaattcga tttaaatcaa agtcaaatta taatgaaatt 45600
   gaagtgagat tcaactgtaa aactcattta cgataaaatg cgcaatgagc ccattaaatt 45660
   tgcgtttctt cgtttaatat tagagtctgg ggtaaagacg taataacgct atcatcgtag 45720
   ccggcgttat agttgacgat agagtcatta ttgttaccgt taacgataat ggatgaagtg 45780
   gatagaggag gagacgatag acttgtttgt gtcaacagat cataaatatc attttcgttc 45840
   tttactatat aaattttgct acgttcgttt tgtctcacca tgacgccttt tttacacaac 45900
   gaaacgtact tgtaatgcgg caacagggcg tctcgtgttt ttttcaataa ttgtttatgc 45960
   tcgggcgatg cggccacgaa aatcttgaca ggggcatcga aatcaatatc aagatcataa 46020
   tttttcagtc gaacttcacg cgaacgattt tgccactcgc gagccgtcgc ggcatcgttc 46080
   aactttaatt taatgtgatt tttttcgtaa tcgctgttca cgatgagttt atagtttagc 46140
   gttaggagat ggcaaatttt tttaacgtaa tttgtacgaa tctttttatt gtacagtctt 46200
   ttatcgtaaa ttccatagat ttcgactgta tcgttaagat tttcttcctc gagacgcttc 46260
   agtttattaa caatattatc agtgacgttg cgttcaattt catttttaat tagattcttt 46320
   aaaattggaa tattaattag ctcggccatg ttagtttacg tcaaacgtaa aaatttaaat 46380
   taaattccct cttattacta gacaaaaagt cgatttattt cacaatgagc tcgtctacag 46440
   caagcacgtc catcgaattt atcccgaaaa aaccaataaa ttacgattta ttgatcgatc 46500
   cggccgacat cgacaatgtg tatttctgta agattaacga ttttcgcgtg tttctcaaga 46560
   acattatagc tgacgttaag aaaatcaaga ttaatttctt taatagtcta atcgaacagc 46620
   tgatttccgt gtatcaagaa tgcgaatcgc gaaactcgca caccgaaatg ctgagcagaa 46680
   tgctcatggc cagcaccatc attgtcaccg atttgccgtc caatgtatat ttgaaaaaat 46740
   tgaaacgcaa caaattcacc gacaccatcg actatttgac tctgcccaat ttcatactgt 46800
   gggatcacaa ctttataatt ttcctgaaca aagccttcaa ttcgaaacac gacaacggac 46860
   tcgtggacat cagcggatcg attcaaaaga ttaaactgac ccacggcatc ataaaggatc 46920
   agtttcagag taaaaacggt tacgcaggcc agtttttata ctcgactttc ttgaatacgg 46980
   catctttcta tgcaaacgtc caatgtttga atggagccaa cgaaatcata ccgccgaaga 47040
   gcagcatcaa acggtattat ggacgggacg tgagcaacgt acgagcctgg acgacgagac 47100
   atcctaatat atctcagcta agcacccaag tgtcggacgt tcgccagccc gacgactata 47160
   acgactggaa cgttaaagtc ggtttgggca tcttcacggg cgccaatacc gattgtgatg 47220
   gagacaaaaa agttatcacg tttctacctc aacccaattc gctcatcgat ctggaatgtt 47280
   tattatatgg agacccacga tacaacttta tttgtttcga caaaaacaga ttatcgtttg 47340
   tgtcacaaca aatctactat ctataccgca aacagaataa tatcaaagcc attttcaata 47400
   ccatgcctat attaaaacgt ctgtggaagt cgtataaagg tattgtattt tcacagaaat 47460
   tggatccgtt gctaagagat tgtgcgctcg ttttgagttc aaacgctagt tatttactat 47520
   tcaataaact gacagagtta atatccgacg aagagatgat ttgcggtgac gaagaaattc 47580
   ataatctaca agggcaattt acggatatta ttaaatgtgg cgcaaaaggc agtaaagatt 47640
   taataaatag cactcgaacc tacaaaaata ccaaaataac cgacattgaa gaagtcgccg 47700
   acagagcaat aaaaagttta aacaactaca tttcttcgca caatcgagtc aaagtcggag 47760
   gcggagatat ctatcacaat accaccgttt tacagaatgt ctacatgaaa aacgattaca 47820
   tttgctataa aaacgattca gtcaaattgg ctagcatttg cctattaccc tcagaatttc 47880
   tatttcccga gcatttgtta gatctctttt tgtaatatat tttcgttgac aataaaaaag 47940
   atgatgcaat acgattgtcg tttcattata attacaaaac atgatgcaat attgggcaat 48000
   atcaacatca tcatcattat tcatgcgacg cgtgcgtcaa tcgatgaccc cggatgatcg 48060
   tcgacctctc gatcgttatc gattcgggtc acgcgcgcgc gcgacaaaca tccggctgac 48120
   gcaacattgc atcatgtttt cgtcgacgcg aaaaacgtgc tcgacggctc ggacgatcgc 48180
   ctcgcggcga ttcatgatcg atcgatcgcg cgtcgccgcg ggacgatcgt cggcgtggat 48240
   ggagtcgacg atgatgatga tgagatcaat ttgagtaatt cgattatcgg acgatcgaat 48300
   taaattttta cgctcgtgtc agcgttaaaa accataattt tgtagttatt atcaccaata 48360
   tcaagtaaaa tgtctcgtac gttgttaaca tacaagtttc aaaatttttg cagattttta 48420
   caaactttta cagttacaac tatcgcgata atatccacag tttcaatgtt aaaaactcca 48480
   atttaattag aaacgaccgg aacgtgagct cggtaaaatt ataaaactaa aaactacaaa 48540
   tttttcaaaa aatttttgca tcaagtttta ttatatctct aacattgaag ccaaaaacta 48600
   ctagattgat gtcaaaaact ttaatattat ctaaaacaac cgggacgtca agaaataaaa 48660
   tttctaaatt ttttaacaga tttttctaaa attttttatc ctacgtctaa gacaaaatac 48720
   tcgttttcgt cgtcaaaagc atcgacattg tcgaaatcgt ccgcgacaaa agcttcgttt 48780
   ggcgtacggg ctattcatga ttgatcaatc atgcatcgac ccgagacaaa cgtcccgtac 48840
   gatttcgatg atattgaagt cacgaggtgt tagagtaacg tacgggctat tcatgatcgt 48900
   tcaatcatgg atcgactcgg caccgatgtt atcggaatcg tcacgaacaa ttggcatatt 48960
   tgacataatc attggctttg atattgagtc gattcgaacg acaaaaaaaa cagaataaaa 49020
   taaacaaaca aaatccatcg tcactggcac tatttaaacc gctttagcgc gacattcgtt 49080
   agtatctcgt tatctttcgt gtcaactata cacatcaaag aaaaaatgtc tctctcaaaa 49140
   ataaatcatg tcgctgacaa cgaaatgcct gccgctaaga gattcaaagc gtgtgttaat 49200
   caatccgaag aatcgtgtca tgaaaaaaat aaccattccg acgtttgtgc cgacggtaaa 49260
   attaaagtta gatttggcaa cgaagacatt gtagttgtaa caatatttga tgacaacggt 49320
   caattatgga tgttggcgaa tccgtttgcc agaattttgg agtacagtaa tgctccgaaa 49380
   gcgatttcaa cgttcgtcag tgacaaaaat caattgtgtt tcgaaaatct taagtcttcc 49440
   caaagcaggc agacttgtat gacgtcatct tttgtccaag ccaagtcgaa attcatcaat 49500
   cgtgccggtt tatttgagtt gattcaaaac tctaagatgc ccaaggctca agaatttaag 49560
   caatggatca actcggacct gctgcctaca ctgtgtcagc aaagagaata cagcccgcat 49620
   aaaaattacc aaaacatgtc tctaacaaaa attaattttg tagacgataa agaagttgag 49680
   acgtataccg tggatgtcga tggtgaaaaa tggatggctg cgaacccgtt cgctgaagct 49740
   ctcaattaca ctcgtgcgaa ttatgcagtg aatcaacatg tttctataaa taatcaaaaa 49800
   acttatgaag aaatcaagtc gcagggatta ggtgcgaccg atgactcatc cgttctacct 49860
   cggaacattc aggccaagac caagtttatc aatcaagcgg gcgtcttcga gttgatcaac 49920
   gctagcacaa tgcctgccgc gaagcgattc aaagcgtgga acaccaacga tctgctgccc 49980
   actctttgtc aacaaggaga atacagcatg actgcagacg cgcccgtcga aatacaagag 50040
   ggaatgaatg ctgttcacgc agccactaac gaaggccgag aagctccgtg gatggaggac 50100
   ttgcacaaac taagaaactc cgtagtacag aaagacaaaa taattgaagt catatcatac 50160
   gagaacaaag aactctccgt gtcgttgcgc acttcgaacg aaaaattgca agatgctaac 50220
   gacaaactaa tgtactttgc cagcgccttg gtagactcca acaatggatt aatgaaagcc 50280
   aacgaacgga ttgaaaacct tgccaatcgc atggcagaca tcgcccagga cgtgatagcc 50340
   aagccgagtg accctcaact attgcattcg ctagctgtgt gcgccttggg cgaaggccaa 50400
   tatgcctttg tgagacctca aaagcgtagt ctgaaacgta gcttggaccg gctttccatc 50460
   gacgagagtc aaattttatt taaaagtaac tacgtaccca atgcgatgaa cgtactgaac 50520
   aaagtcaaag agagtctgcc taaagacaag tttacagctc gtcacaacaa gattacgttg 50580
   ctagaagatt tgactagaga ggatctagtt gaagcgatta actcgtcgat gaccgagcga 50640
   caggtggcca ttttcgccaa caaagcgtcg ggcagaaaat aaaatgtgat caatcaataa 50700
   aaatcttttt ttatatattt tacatgttga ctattattct aatccctatg tgcgttgaat 50760
   agtttttaat gtttgttgag cagtattcca atccttgcga ccattcatga tcgtttaatc 50820
   atgaatcgcc acgacaccaa cgttaccgtt tttgtcaata ccaatgatag attttaatag 50880
   aaaaattgtg gtcatgtttt aatacaataa taatatatgt taagatcact ctagtctaat 50940
   taagaggttc gaattaaaca acaattgaaa atgttcatgt actttacctt cctagtacta 51000
   attatattgg gattcatttt tgataaaaat gaaggattct ctaatctatt actctttatg 51060
   ctgctgctgt ttgtcatatt tataatgtta ctgcaaatct actacataaa gccagagtct 51120
   acgactgccg atctaccaac cggtaaagct aagagtataa aaaagaaaag agatttagaa 51180
   aaagcgtttg acgcaattat aaataagaac actagttctc tagattgaaa caaaatggac 51240
   ttctttaaaa attttgtcaa tcaaatggtc acatcgatgc cgatcgtgac aaaagtggcc 51300
   tacgtcaatg tgcacatcaa aaactacatg aaggagctgg aacgggacga cacgtttcgc 51360
   cataaattta tcatgatcct acaaatgttt atcaatcgac agatcacagt cgaagacatg 51420
   tgtcaaataa tggatgccgt cgatggtttg aaactgacta gacagcagat tgaatacttg 51480
   gtgaatcgag tctactacaa cgaacatatt atattgattc ttcagagtta tgttcgcaat 51540
   cagcacttgg acgatgacca gattgacgat ttagcacatt tcctagttca cgaaataaat 51600
   aatgcgatta tataccaaaa ataaaaactt tattcatcaa aacaaatcaa caaattttat 51660
   tacattcccc acacaaattg ttaggatcat aacgaaaaac gtcaacgtca gctgacacga 51720
   cgacaggtct cttggtcttt gtcgatttcg gcggcggacg ctttttgtat agagaaaact 51780
   gatactgata gttgacgacg atcgattcat cttcgctttc gtcgtcgtta gagtcgtcac 51840
   tgtcgttatc gtcatcttcg atggcgtcgg tagatttttt tgttgttttt ttggcagcat 51900
   gtttgacggg cttcaattga ggaaatttaa catcgtgctg ttgattctgc atgtaatgct 51960
   gacagattct aataaaataa tactcaaact cggggcgatc gccaaatatc tgaatcagct 52020
   cgttcataaa cgtacacaag atgcccatat gcttgagcca actcatacgc ttggtctgat 52080
   cgaaaagctt gagaggaaag gccttttgag tcacagtgcc gacgacatcg gtcagtaagt 52140
   aggggattct gtctccgttg ccgggtagaa aatcagttcc gcaattagcc aaaatctctc 52200
   gacagtgctt ggcgatggtg atgggtcgct tgcgctctgg cgcgcccggg gccaattctt 52260
   tcttcttttt gtttgtagtg ttttcgttat agctcatgct gaagccataa tcgctatagt 52320
   tatcgtcctt tccgaaattg tcgtaatgag cgatcatgag ctcggtgagc agattgagac 52380
   cgcatgccac gctatgattg cgcagataca tgtcgacgat ctgtctaaaa gacttgcgca 52440
   taaacagagg catatccttt tttatcagcc accccttgta tttgatgcga ttttcgctat 52500
   tcagataaca atatttcttc tttttcaata gaatcagact ggtcattatg ttctctagag 52560
   ccattttata tccgacccaa tcggcattca gtttattgag cacataattg tttataattt 52620
   taccaattgt ctcatagaca ttttctttaa tttcattctc atcgaaggct acgtgaataa 52680
   acgaagagtc cgtgtcgcca tagatcactt tgaacacaat cgatgacagc ttgaaatcgc 52740
   tcaatattct agggtcgtcg ctcatacttt gaatcttttt gatggcctcc atcaattttt 52800
   cacgtccgat ttttgtcaca taattggcta aaggtttaaa gaagataccg aaatagccgt 52860
   agatgctgtt ggcgattcgc tttacagcat tctgagtttt atcatataaa ttatactgaa 52920
   atgtgcccac atcgaacttg tctcgcttcg ttttatatag aattcgcagg tccaacagct 52980
   gtctcaaaag cttgggattg atcgcatttt gattcttcac tagatacacg taattatcgt 53040
   gtccgatgaa cgtgttcgat aggcaaattc cctcttgaat cattatagtt aggtagagcg 53100
   aattaaagtc tagagtggcg acccaacgtt tcagtcccgg ttgaggcgaa agaactttac 53160
   cgcccttgta gatacatttt tgtttttggt gacacagacg aacggcattt tgaggtattt 53220
   cttcgattgg acttctctgc agcagagtca agtcgaccat ttgcgagtct acggcttcaa 53280
   tgttgccttc actaaagcca tcctccctct gtttccatgt gcgcttgcgc ccagacgtta 53340
   ccgataaatc ggctttatta aaaaaatacg catcaggtat ttcgcgcttc aatcgatcat 53400
   cgtaatgagt gttgttcaaa gctcgataaa aaaacactac actcactttg tgtgaaatgt 53460
   tacgaagaac gtcatctgtg ctcagataga gcaacatgca cagcgtatac ataaagtcca 53520
   taatttctag cttgagaaag atatcgatgg gcagcacgct gtcctgtacg ttgtactcga 53580
   tcagtttctt gaggcaattg ttattataga gatcgagcat ttcggcaatg ggcaaatcca 53640
   cctttttttg attcaaataa tgctcggaca cagtgttcaa ttgaaaattt tccacattat 53700
   tctgctcaga atctgtactc aaaaattggt atagatcgac atgagtatag tacgaaaaaa 53760
   aatgagtatt catacgatta ccgaatttat cgcataatat tgacgtgtga atactaatcg 53820
   gctccagatc gtagcggacg atcttggtcc acgattccga tgtggtcaat cccacgctat 53880
   agctgcgact gtgttgtctg ctcgacagaa ttctagcgcg attgatgata aaaggcaaat 53940
   caaatttgtc tccgttgtaa tcgagaagac aatccatatt aatggcgggc agcagtttaa 54000
   aaaaagcctt tagcatattt atttcgtttt tgaagcgaac acacatgatt tccgcagacg 54060
   ggtccttgtc gacattttgc atgtcgttgt cgttattttc aatgtaataa aggcaaattt 54120
   tcgtgttttt gtcgtcgcgc ttaacgacaa ttgatatcga aatgatgaaa ttttcttcgg 54180
   gcgtcgaaaa tctttggccg tttgaatgtg tttctatatc gtaacacgca atgaccggga 54240
   caatctcacg agttaacgag tcgggcgaca cgatttcgag tagttcttcg atgttgtcaa 54300
   tgttattgta ggagcattgc aaacgatttc cgatacatct tagcgttttc ttgaatcgaa 54360
   cgtattgccc ttctttaagt tttgtttgca tatgaacacg attgatgtcg tttagaaacg 54420
   aatctagaca atacttgtca aactgttcat tttctctaca gcgcttgtat ttaatcacgt 54480
   taatgcgttc gctgctcact cctcgcaaac ccggcatcac catcgtctta tagctttcgc 54540
   agttgttgta gcacttggaa aaggcgtgat tgttatagca ttttcgatag gaaaacaaat 54600
   cacacatggt ctccatgtaa aattgataag accgattgtt gttgtggcca gtgaaaaaaa 54660
   ctatcaaacg gttattattg tacatcattc gtgttatacg gtacacgtcc gagggaaaga 54720
   tcttggtatt tccctgtagc cacgtacttc tgacagcact ttctagttcc tcgtacttca 54780
   aaaggctcaa catcatgaac aggtatcgac caaagtataa aaacactgaa gtcaacgctg 54840
   acaccgtgca aaatttattg agaacaatca acactatgag tcagcggtgc aaaactcagt 54900
   cgtcgactga tgatgtgctg cagcgagttc gctctattat actattgcat cgacccgatc 54960
   tgatatcgcg tgctgatctt cagataccgg agctcgtcac cgaggctctg ataccgtcgt 55020
   ctaatcagat tacacacaat ttcaactaca agtacgatta taatagtaac gtgccaaatc 55080
   ctccgattcc gccgccttta aacccattta cgtcgaatac gtccgggacg acgacgacgt 55140
   atcaatccga tcaaaaatta ccgttgcagc cggtgcagat gttcacattc aatcagactc 55200
   cgtcggcgac gatggcaccg ccttcgcagt cgaatgctac tttttatccg caaacgacgt 55260
   cgacaccgcc gccgccgccg caaactttta tatcgatttc cgaggaagat tcggtcgcgc 55320
   tcaacgaatc ataccaacaa actctacgat cgccatcgat cgaatcgtac aaacgattaa 55380
   ttagcgtttt ggtggtgatg tctaaaaaat atattagatc ggacgtgttc gtcattggct 55440
   tgaatcgctt ggaatcgttc gattctctgc tgagtagcga tctgcaggaa ctgatgcagt 55500
   gtattcaacg taacaccaat tggtcgatag gcggcggagg taaagctttt accactccag 55560
   aattatgtca gctaatttcg acgatagtga caggctattg tcgctttgcc ggtaccgtgc 55620
   tcaaatcgtc tagttttaat ctgtcgagtg taggtagcgt cgagagtttg caacaacaga 55680
   tggcttctat tcaaagtcaa ttgtattcaa taatcagtgc gagcgactct gtcggcttga 55740
   ataacgatca aataattaga ttaaataatc agattgaaat gcttcaatcg acattggtcg 55800
   aacgagaatc taccattata actctgaata caaacataaa tattatgact caacgtaatc 55860
   aagccctcga atcgaatctg gcaattatcc agtcatcgta taatagtttg gctagagctt 55920
   tagatgttaa tataaacacg tccgctagac tggaaaatgc cgaaatcgtt gaggcgacaa 55980
   atcgaattat acagaacaaa attatggagg atagaggtct actgaaatca tttgagagcg 56040
   aaaaacaaac atacggtcaa tctgtaaata gattagagag ggaattgaac gattctcgtg 56100
   agtacgcaaa tagattaatg ttggaattga agtcggctcg tgatgatatt atccaattaa 56160
   aagaggaatc gaaccgtaat gcttcgaagc aacaggacga atctactatt aaagaacgcc 56220
   aacaacaaga attgtttgta tcggaattat tcgatttacg cgaagaaaat aaaagtaatc 56280
   aaaatcgaat cgcttcaatg agcatcgaat taatgaatat tgccgaagaa aataaagttt 56340
   taaaagataa attggcaatt cgagagaagc cagtcgctcg cgccaaaccc tacaatagac 56400
   cgtcgcagat agaggcgacg acatcgacgg ccgtagaaaa tcggactcga gctctacaga 56460
   ataccattaa agatctcgaa acgactaatc aacaattgcg caaccaaaac tctagcttaa 56520
   catcgcaaat gaacaaaaac aaacaatcgg ctagcgttca ggcggactgc aatcgaatca 56580
   ggtccgaggt agataacgtt cgtcaaaagg tcgaggaatt aatgaaaaat caggcgacat 56640
   taaacttgca agacattaat gcgctcaatc aaaagactgc agactattac aataccaaat 56700
   tgtccgaatt gaccagtgaa aatcaaagca ttagagacat tctaaacact gaaatcgata 56760
   aacaagtcga tatactcaac gaacgtatcg accgatcaaa atcaaatatt cttgataaaa 56820
   tcgaaagact atcggaagaa tttagtcctg ttttagttcg tgcacaatca tcagaaaaat 56880
   atgttcagaa tttgctcgcc gattatgaat ctctgtctag aaatgtagcc aatcagcagt 56940
   tgtcggaata atacaaaaaa atgtttatta aagtttattt aaacccacaa cccaataata 57000
   gtacaagatt tgtaaattta tgtttattta tacccacaac cctaaaatag tagcaatttt 57060
   aattacatca aaaattataa tacaaaaaca cacattatat agaataatat tgttgttgtt 57120
   cgtgttcata ggcagtgact cctataatat tgtagtttct cgaaacatta tcggcgacaa 57180
   tatagaattc gacttcagtt atattctcag caatcatgtc gttgagttga ttcaaatcca 57240
   ttttaatttt aaccaatttc tgtaatcgat cctcattgtt gcctttgttg ttgttgccac 57300
   agaagaatgt tcctttgagc gggtcattgt tgtcttgttc gaacaaatta aatgaaatac 57360
   tcaatcgatc gttgccatat tgttccgctt gaactctagt aatcttgccg acgatgacac 57420
   gtttgttctg gcgacttacg cttaaaatgc tgttcttgtc ctcctttaac atagcagcat 57480
   ctttggctct ctcgatttga gtaatgtcca taatgataaa agacttgtag ttgttgttgc 57540
   tctgatggca tttgatgcgc atcaaattga tcattctatt ttgattattt acaaaaaatt 57600
   ccaacaaatc agactcgtta tcgaagcttt gtttaaaggc ggcactaatt ttctgtaatg 57660
   ttgcactaca ttcaatctga tataattttc cttcactacg agaactaaaa tcgttggtaa 57720
   acgacactac aaatatcatt ttgtacaagt cattttccat taatttaaag ccaaattgaa 57780
   atttagccaa tacagaaata atttcactgt cgtcaaagtc tttctgacaa acaaatcgtt 57840
   taacattgac tttcttatcg ggctctgaag attctttaaa atcagtaata tagtagcgtt 57900
   tcttctcata tgtaatggtt aaattgtatt tcttgtttag cttcatggca taaaactgac 57960
   tggcatctcc ataatactcc ttgctgacgt tgtcgatgag tactttgaat aagtaaaatg 58020
   attcattgtt gatagataga gtgtttctgg cgaccaactc gcccacgact ctattctgtc 58080
   gcattgggcc cgcactagag cccacacttc cagtcgaaat cgacatagca tcacttttat 58140
   tattagacct tttcagaggt agattaacat caatcttctg gcgtttgccg ccataaatct 58200
   cttcgttatc ttcgacggca cgatcgttct gtttttgatc gcgtttgcgc tttttaattt 58260
   ttttttcttg ttgtttttgt aatagaagct gttgttgacg ttgttgttgt tcatcgtctt 58320
   cgtcttcgac gactttttca actttattat cgatattctc ttcaacgtca gtggtgttgt 58380
   tgtagacgtt ttcgtcttgt tcctcttcat caaagtgaga gggagcagtc accgacatat 58440
   tcttgataga catggccaat aattcgatac gctggcgaat gctaaactcg gcgagggtcg 58500
   aagtgtctcc cgagagtaga gaacacgctt ggaaggattt agttatcgac acgttgatag 58560
   ccagcccgaa agacgattcc tacaggacta tgatcaataa agccaatttc gaaaactttg 58620
   actataagag acccttgatt tatgatatca aaactgatac ggttctgata aacagcgagt 58680
   ctttgaacag agcacttaat cgaccgaccg gttcgctcga tccgataaat gtgatgtctg 58740
   cacacgtatt tctcgctttt atatgctcac tactgttaat tctgttgacg gcactgcttt 58800
   ttgatcaaaa cgacagagac gaatcgccaa ttgaattgaa cgatggttag atcaagtttc 58860
   ggtgtcgtcg atgtcggcgg tcattgttaa ggtcggccaa atggattatt attcgaatat 58920
   aatcaactct cgactcgcgc cgccgtcgtt ctatctcgag tacagcaaca gaatcgagag 58980
   cttcgtcggt agttctttaa ccagagaata tatacaaaat ttagcaaagt ttggtatata 59040
   ctacgaaagc ggcaatggtg tttataaatg tgcattttgt cctctaatta tggtcaaatt 59100
   ggacatgaga acgctaaaat atcacacctt ttcgttgtgt tcgatggcga cgactatact 59160
   cgccataaac gaaactctcc gaaaggaatc gtttcgcaag ttcaagactg ctcgacgcat 59220
   attcaaagaa aacggcaact ctttggccgt aaacggattc tattattatg gaaaaaatat 59280
   cgaaattcga tgcgccggtt gtcgtctcac aatcgtcaaa ttgaatcgaa cagatcgtgc 59340
   cgaagatatt catcgaaaat attcaccaga atgcgaattt aacaacaagc ctacggctcc 59400
   gcccgccagc gacagcgaca cggaaattga tgtcaaaaac gtcaaaaatc accatacaga 59460
   cgtctacacc gaatcgtatg gtaatactgc tgcaaaaatt tatcctgttt tggattcgaa 59520
   taatggtatc gtccatgacg ataaaaccaa caatagcaat gatgtcaacc tgtttttaaa 59580
   atcgaatatt gtcgccgacg gtgataaaaa tacacaatcg gtttcgagcg tcgcaggaga 59640
   ggacgatcgt tattgtaaaa tatgctttga gaacgagaga aatacgtgct ttttaccttg 59700
   taaacacgtt agcacatgca ccgattgtgc ccgcaaatgt aaagtatgtt gtatatgtag 59760
   aatgaagata aaagaacgat tggaggttta tttacagtaa tttattgttt atttgaacta 59820
   tataaccgca acacgatcgt ttcggacatc attcgaaaca aaatgtctat acaaacgggt 59880
   gacatagtcg agtcgaacgt ggtgacccaa tttaattcgg tcgcttttat tgttaatcat 59940
   gagactcgtc gggtcacgat catgtcatat aaaaatgaac ctgtcaaatt ccacgtgttc 60000
   aaccagcacg ataacgatcc tgatcttcta aagcttcaat atcattatcc gggactggcg 60060
   agcagtatcg aactacctaa aatcactcac atgagtaaag ttcgaatcat ggtattcgat 60120
   cagcgacatc ggatgattat cgtcgaagag tttctcctgg aaaaactata ctttgtgcat 60180
   catcattacg ctaaatatta tgtgtacggt ttagtgcctg ttgtcgccaa gaactttaaa 60240
   cataatctgt atagcggtgc gcctgtcttt gatgaaacgg gtaaagtttt gttgtcggtc 60300
   gttagcgact gttatttttc atcgacaaca gacgaaaatt gtattatacc cctgagcgga 60360
   gaactgagcg gatctcgagg agtgttgtgt ctcgatggcc atgtgtggct caacgaaagc 60420
   ggtgacgatt tcacattcga caagacaaac gttgcgaata gaatcgattt gtacgtttcg 60480
   catgataaaa aatttgtgta tttaaacata ttctataata acaatattat aaattttatt 60540
   agagttaaag ccaagtttgt tggaaatgta ctgattcgtt aaacatcgat tagaatgaac 60600
   tgattaattt attactctat taaaacgtgt actgattagt taggaattgt gtactgatac 60660
   gttaaatatt gattgtatat ttaaagtgta atgataataa aataaaatca aattggttgt 60720
   gacaagacta ttttattcgc gccgatgcgg ccaactcgtt gatcataccg cacgaattta 60780
   cgtttctttt gaccctaacg aaaccgttct ctccgtagtc ggatccccac gaatttttca 60840
   acacccagaa cggtacacca ttttctatgc cgtagccgac gagaagaacg gcatgattga 60900
   ggccgtagtt tttacacgaa gtaattattc ctccgtaata atctgtgaga tcgacggcat 60960
   cgaccgccac tgctatagga cccatttcat gaagtacatc tttcaattta tcctcactat 61020
   ataaaacata tctataacaa ttgtccacgg aaacttcaaa tttatcgctt tggagtcgac 61080
   acgggccttg taccgaccga tagggatagt cttcttcgta ttccaaaccg cccatagcca 61140
   ttatttcctc gtacgcagtg tgtagtagtc cgccggcgca tcccatgtcg atggtgtcgc 61200
   aatcgaccaa ttgctgttcg gatagatcga catgttcatt atacttgatg gcatattgac 61260
   tctctaatcc ggctatagta gagaaggccc agcacgatcc gcaaaacccc tggtctttga 61320
   cggaagtgat cttgttaaat tgtctccagt cgaaggtttc ttgcgtatat ttcgagggac 61380
   cgtcgactat gacgggctcg caagagtttt ttaaagctgg cgatcttatg cccagacccg 61440
   tgaacttggc gatgacttca ttcttggtca gatcggcaaa tttgtttata ttgtaaacag 61500
   ccgaatcgtt ttgttgattc ttaagattga tgatttcgag attagtttta aagattttat 61560
   accgataatc gcgttcgttg tcgtcggcgt atactttttt atattttgtt tgaaacgttt 61620
   caaaatattg cggagctcgt tctaaattat attgtaattt aaactgcgga gccgccgtga 61680
   cataatcgat aatggcgtcg acaccattgt caaagccgcc ggacgcaaag acgaaaacaa 61740
   aaaatagtat gatttttttc ataattattc gctactcata caataaacga taatttctta 61800
   tttggacaat acagtcatga tgcattcggt aatgttgcat ttctaggaaa attgatattt 61860
   ataagacaaa atcaaaatgc attttgtatt cattggaatc gtatgctaca tattaaccat 61920
   agacggcgga catgctgcgg ctcccgggat tcctgtgatc gattgggccg acagaaatta 61980
   tgccttggtt caagtcagtc acgaagccac ctcctacgaa aatctagtta aaaaagtcga 62040
   tcgagttcaa gttccggtct cttggaacgt ttggagtggc gaacaaggcg atatcgctta 62100
   tgtgttattc gacagtaaac aagtatggag cggtgacgca tccaacaaac acgcttccat 62160
   atttgttagt acaggcggtc gatttaaaat gactgtaaag ttgtgcaatt cggacggatg 62220
   cacttcgagt cagccggtcg atgtcgtgat cgcagacacg gacggctctc atctagctcc 62280
   attatattat gaatatcaag aattaaatca accgttcgag actcgggtca aaaataaagt 62340
   cgttgccgca tattttgtcg aatggggcgt ttatcctaga aactttcctg tagatcgagt 62400
   gcctactcct aatctatcgc atgttctcta tggatttatt cctatatgcg gcggcgacgg 62460
   cattaacgac agtctcaaga ccataaccgg tagttttgaa gctcttcagc ggtcgtgttc 62520
   gggtcgagac gactttaaag tgtcgattca tgacccgtgg gctgccgtcc agaaaccaca 62580
   aaaacaagta accgcttgga acgaaccata caaaggtaat tttggtcaat tgatggcagc 62640
   caaactggcg aatcctcaca ttaaagtgct accttcgata ggcggctgga cattatccga 62700
   tccgttcttt tattttcacg atgtggccaa acgcaagatt tttgtcgatt ccgtcataga 62760
   atttttacag acctggaaat ttttcgatgg tgtcgacatc gattgggagt ttcccggcgg 62820
   gaaaggcgcc aatcccaacg taggcgacgt cgaacgcgat cgcgaaacgt acacgattct 62880
   gttgagagat ttacgcgaag ccctcgaccg tctctcaatc gaaacgaatc gaacgtacga 62940
   attgactagc gcgatcagcg ccggcgatga caaaattgcc gtagtcgatt acactgaagc 63000
   gcaaaaatat ttggattata tttttttaat gacatacgat ttcaaaggag cttggtcgaa 63060
   tagcgatttg ggatatcaaa cgactctgta cgcgccttcg tttaacccta aagagcatta 63120
   tacgaccgat tttgccgttc gtgctatgct caagcaaaaa gtcgaaaaca aaaagattat 63180
   agtcggcgtg gccatgtacg gtcgaggttg gaccggcgtc ggtgactttg tcgacgaaaa 63240
   taatccattt tccggcacgg ctaagggacc gatcaaaggt acctgggagg acggagtcgt 63300
   cgattataga gagattttta attcttttgt gggtaaagac ggcattaatc gttacgattc 63360
   catcgcaaaa gcggcatacg tctttaataa cgcgaccggc gatctcataa cctacgacag 63420
   tcctcagtcg gttctggata aagcaaaata tgttttggac tataatctgg gtggtctgtt 63480
   cgcgtgggag atcgatgctg acaacggatt gcttctaaac gcaatgaaca gcggtctcgg 63540
   cggtttaaca atttttgcta gtgaaaatga attttataaa gtgaataatc attaatattg 63600
   cgacaacaca ttagtcgcat cgatcataca ccggcttcgg tgaaattaca taaaacagta 63660
   aaggaaataa aaaacaaatg tgtagtaaaa aaattttttt atttaaatat cttcgttggg 63720
   tacataacta gcgatatata gctccatgta gccgtcgtct ctgttttcgt tgtcgagaaa 63780
   atactttaat tttaacaaat gtttctcaat aatggatatc tcgacaaaag aattgatcgg 63840
   atttttagtg taattgtaga aatattttac attaatctcg atcatgtcgg tcggttctgt 63900
   agcactatcg atggcacaat tttcttcaga ttctccttct ttcacaacgt aattgacatt 63960
   aatttcgtcg cctttgctgt taaaaatgat tttatcgaga tcgattttga tgccaacgtt 64020
   aagattgccg agtaaatcca agtgacgaca caggcgtttc aattcgacac tatgcatcat 64080
   ggctctgccg gtcacttgca aatcgttaga catgccatag tcttggctat caaaatgata 64140
   caacttgtat tcataggatt tcagagattt gccagtcttg atcttaaagt tgagataatc 64200
   gtgactgtcg ttttcgccgg accatatagt gagcacgctc gcgtctgtgc acgtttttaa 64260
   aatcttgcac aatccactca tcttgacgga tagagacaag tttttcgtaa acgtaaaact 64320
   cttaaaagct tccttcatca ttgtaagctt gatgaatgaa atcctgctcg tgtccgagca 64380
   ttgcatcgaa attccttcgc tggtgacttc tagactcgag aactcgacca ccgtctcaat 64440
   aacttccatt gcctttttga aagcaatcgg attcttgaat ttagcctcga acatcttgat 64500
   tgtgtcgctg acgctagttg actattgaca gtaacaaaaa acgattgtat ttatactcga 64560
   tgacgatcga gataatcgag atatgaaatg ttatcgcaac aaagttgatt attaagcgtt 64620
   cactatgatt cgttcgattt tcgtgttata tacaattatc aattgtgtta gatcccacgg 64680
   atacttgtcc tatccggcgg cgagacaata caaatgcttt agagacaata atttctggtg 64740
   gcccgaggac ggagaggcga ttcccgatcc cgcctgcaaa cacgcatatc aaacagtgta 64800
   cgccaaatac cgatcagaag gagagccagc cggtgttgcc gccaatgccg cacagtacat 64860
   gtttcagcag tattacgagt acgctgccgt cgccggcgcc aactatgaag atttcgatca 64920
   catcaagcga aatgtagtat cgcataattt atgcgcggcc ggtgccgatt accgttccga 64980
   tcctttcggt gacaaatccg gcatcgacga gcctcataca gagtggcgag ccgatacttt 65040
   ttatgtagat cccaagcaaa aatatagtcg aggcatcgaa acggttctgc atttctgtcc 65100
   gaccgcggtt catcagccca gctacttcga ggtctttatc tctaagcgcg aatatgacta 65160
   ttcccacgaa ttgacttgga acgacctcga gtttataggc ggtaacaact cacagttgat 65220
   cgacaacgac gggtcggatc cattgtgcgc acacgaatcc atatacacta tcaacgtcgc 65280
   catacccttt agaattagga aattcgtatt gtacgttcgc tggcaacgca acgacatagc 65340
   aggcgaagga ttttacaact gtgccgatgt cgtctttgac gattatttac tcacacacag 65400
   acgtgtcagt aagcggagaa aggcatatag agatctttaa tttatcgacc aaactaaaat 65460
   tattttcgac caatcgaaat gtattttcga ccgattaaat gcgttatctg ttaaaacaat 65520
   tttatctaca aaccaaagta tataaagctc gggatggtac gattcgtgca gttattattt 65580
   tcatcatgtc gctgaaagtc aactacgatc aaacgttcgc cgtcagcgct cagcaaactc 65640
   tggccaatca ttttcaagag tgtagaacaa ctaatatcgg aagactctgt gatcctaaac 65700
   gaattctaaa gttgaatcaa accgaagagc ccgaccacga tgtcgtcaag aaaggaggag 65760
   tggtgtattg gatgtggaga gactgtcgcg tacaagacaa ttgggccatg atttatgctc 65820
   agtatttagc gtttaaaacc aaaagtccat tatatatagt ctattgcctg ccgaaatttt 65880
   atctaaattc gactcgacga caatatcagt tttgattgga aggcttgatc gagctgagtg 65940
   aagagtgtgc cgaactcgat atcacttttg tgatactcga cgacagtgcc gatgttgtgc 66000
   tgatcgattg ggtgcgcaag cacgatattt gtgctgtcgt ttgcgacttt aatcctttgc 66060
   agcttcagat aaaatcgacc gttaatattt tacaaaatct gccggccgat gtgtactttg 66120
   ctcaagtcga tgctcataat gtcgtgcctt gttggttgac tttcattgcc gacagacacg 66180
   attatgatga gtttaaaagt aaaattgatg cagaactaga aaacttattg actccgtttt 66240
   ctcttgtcat acaacatccg tataaatcgg tcgtttctat agaatcttcg accaatacgt 66300
   ccatcgactg gagcaatcta ttgtcgtcac gaaatgtcga tcattctgtg aagcgaatta 66360
   aatggaccga agcaggatac aatgcggcaa tactgagatt ggccacgttc attcagtgct 66420
   atatttataa ttataaaaat tcaatacata atccagtctc cagtaaacag agtgatttat 66480
   caccattctt tcattttgga tttatatcag cacaacgagt aatttatcat ttacgctttt 66540
   gtgttacaaa aaaaagtgtc cttcaaaaaa ctgtatttat aagaaaactt aaaaataata 66600
   tagaaaaatt tatcgaaaac tgtttatatc gaagggaatt tgcagacaat ttttgttact 66660
   ttaatttaaa ctacataact tttaatgcgg cttcgccaca aatcaagagg tatatagcaa 66720
   aacatttaag atactataca tacagcttga atgaattgga atattcgcaa acacacgaca 66780
   atatatggaa taaagctcaa gaagatttgc gagaaaacgg caaaatatat ccattcatac 66840
   gcgtatactg ggccaaaaag attttggagt ggacgtcaac accggaagag gctttgaatc 66900
   gtgcgattta cttgaatcaa aagtatgctg tcgacggttg cgatcccagc ggttatgtag 66960
   gatgtttgta cgctatgagc ggtcttttga acgccggcca atctaatgtg tttgtctttg 67020
   gcgaaatcaa caatatcagg tcgaaatggc taataaacac gtgcaaatat aaagatttta 67080
   catatagcta tacttcattt agatttcaat acatgtttga atagattgcc gtcatttaag 67140
   atttaatttt gtattacaat aaaaactctt ttttttcaat tataattttt attttacaac 67200
   aatacatttt aatgattata ataaaaatta atcctataca tgattgtttt attcgttaga 67260
   acttttattg tcgattgcct gcattatgct gctcacatct tgtgctggca tcgagccatt 67320
   aaaaatataa ttgttaccat taaagtagtt ttttatacac tgatcgtgca cttgttgcgg 67380
   tgaataaatt tctaaagcat tttcatatgt ctttggacta ccttcgggct tggtctcatt 67440
   ttttatttcg tcgatgaccg cacgcacgat ttggctgact cgttctttag cttcgatgct 67500
   aggcgtgaga cacttgagaa tggcatcttc ttcgctcatt aatccgagac ttttaaagtg 67560
   ctcgatagcg gtgcgatcga ccagtcgatt tttcttcacc atctcgacgt cggcgagata 67620
   tttctgacgc aactcgtctt cgttgagaat ttccatttcg gtacacaatt tgtttacata 67680
   cttgagtccg tagaacatgt gaggatattt cgatcgtatc acaacccaaa cggccactgg 67740
   attggcgcat ttaatctgta aaaatttttc cgaatccagc agccattcgt agcgtttaga 67800
   tcgctggcgt ttcgacggat tggatcgttg acgctcgagg aatttatcgc attgatcgat 67860
   ctcgtttcgc tggcaacggc tcatacgaat ccgtttttta ccgtcgacgg tgcgttcgta 67920
   tccagtgaca tattcctctt tttcgggtcg attgctcagc gatggcacga cccgttcgct 67980
   cacactttcc aagttatttc tcatttgttc gttctctttt atattgtctt gagccaaaag 68040
   agtagtgaca ccaagttgca tcatattcat ttgggccata tgggacattt tgttcaggtc 68100
   caattgcatc ttgagcgctt gttctttgta ctcggagatt tgacgttcgt aatttatttt 68160
   catttcacag atagtgttat tcttgtcggc aattgtcaga tccttctcgg cgatgactgt 68220
   attcttctcg gcgataatca aatttttctc catcacttcc atttttaatt ccatcaactg 68280
   tttttccttt gcccaaattg ccgatttgcc ttcgtttgtg acttgatgaa ttatatccat 68340
   tcccgcttgt atttcgacgg gcgcgtctgc agtcatgctg tattctcctt gttgacaaag 68400
   agtgggcagc agatcgttgg tgttccacgc tttgaatcgc ttcgcggcag gcatttcgct 68460
   ggcgttgatg agctcgaaaa cgcctgcacg attgatgaat tttgtgttcg atttgatgtt 68520
   ccgaggtaga acggatgagt catcacccgt accgattcgg tacgagttaa tttcttcata 68580
   aattttttga ttctcggtag acactttttc taggattgct ttattaggtt tagaatattt 68640
   taacgcttca gcgaacgggt tcgcagccat ccatttttca ccatcgacat ccacggtata 68700
   cgtctcaact tctttgtcgc caaattgaac tttagacaaa gatattccgt cataattctt 68760
   ttgcgcacaa ttattagcta gcacatccac ttgcatatca tatcttcctt tatcgcagag 68820
   ttttggtagt aaatcaaagt tgatccattg cttaaattct tgagcttttg gcatcttaga 68880
   gttttgaatc aactcgaaca aacccgcttt gttgatgaat ttagtcttcg gatgaagtaa 68940
   tgacgtaatc tgcccaatgt gggaagatcg acaatcttct atacacaatt gatttttatc 69000
   gctgacgtac ttagcgatgg cattgggagc attactgtac tccaaaattt tggcaaacgg 69060
   attcgccaac atccatctct cgccgctttc gtcgacgacg caaacgactt cgagattttc 69120
   gttgccgaag ctagtcatga ttttggatgt agtcatttta aaaaaattaa aaagtgataa 69180
   tgacaaactg acacaatgta tctctaccga tgtcaagcga ctactaattt tttaaaaaat 69240
   aaaaccaaaa tatatatata tatatataaa actgttaatt tggattcatt tcgaaacaat 69300
   attgttacaa aaagcaatca agttgcgtag aagtttacta tccaaactag tgtttaaaaa 69360
   aatatattgt atgtcattaa attacatgac aatcgactca aacattatgt aaaagacatc 69420
   aaataacatg acaatgtaat gaattaaaca aaaagttttt tataaaattt atttatttat 69480
   taaatttata gggtaatctg cttggacagt atgtcgttgt tgttgttgat ctacataatg 69540
   ttgcagttga gttcgttatc atcgcgagac atggttcgcc ttctcttatt ggccaaataa 69600
   ctagctaaat ggctgtcaat gtcatattta ttattctggt tataattttt gttgtcaatt 69660
   ttcgcatctt tattagtctg gttgattatc ttgcccaatt cgttatttct cgacgacaaa 69720
   ttgcgtataa tattttgcat ctgatcttcg cgattttgat attcaagaat tcgcttttga 69780
   taattgttca atatcatatc cttttgtttg ttgtctttca tgacgtttac tgttttttgt 69840
   tctgattcca gtagtttatg cttcatatta cgtataactt gatcttgagc ttctttattt 69900
   ttctgatctt cgacatccat agcgtttcga gtaatttttt cttgattttt gtttacaaaa 69960
   tattgaccat ctgtacacaa tttgatgagc acggtcgaag cgagccattg acgtaaatcg 70020
   atcgcttttt gaatctgaga cccttttatt aactcgagca cgcccgcgag attgacgaaa 70080
   atcgtctcgg ttccgcacca tttcgcacgt tcgggatcct gcttcataaa tgtgccgacg 70140
   ctgatagcaa gcttcaaaaa ctttaacttc ttccggttat cttcatcgac aatcttcaaa 70200
   atgcttctat ttacgccaat ctcgagaatc gaagccagtt gagtaccaca aaaccactcc 70260
   tcttcgtaca aatcgacgac ggagatgacg tgtttaaaat cgtctttaaa aaacttggta 70320
   atgactttag aattcatgat tttctaccga ttattgctga caaattgact gagaaagtga 70380
   atttacacta gctttataca ttagttatct tgaatgatat cgcccgataa cgataacaaa 70440
   tcgtcttctt cagtaggcat gatttcgttt gtttgtaaaa atctcacaca atctctgcaa 70500
   gtaatgattt gatcataatc ttctttttta atgtttttgt cgtgtaatgt tgtcgatttc 70560
   aacggtacac gatctatatt ataatatttg tgtagatgat ctacggtagt ggacggatga 70620
   gattgagaca tttctatcga atacaatctg aatccagtcc aacgaatatt tgtacgtaca 70680
   ctactatcga tcagataaaa agtcgtcgaa gagataacga cagataaaat attctttaaa 70740
   tatactgcac atttgtatat tccattatta ctcgtgtctc gtcgacgaaa acggtagcaa 70800
   taacgatggc agcaaacgat cgattgaagc gaatgttcga agagagtcgt atcgactcat 70860
   ccggtgagac ctgtcacccg tcacgcgttc gtgtcataag ccgattacgc gaattggtcg 70920
   tcgccagcga aggcaaagaa gaaggtggtg ttgtttactg gatgtctcgc gatagcagag 70980
   ttcaagacaa ttgggccctg atctacgctc aagaactggc gcacacggct aaattgcctc 71040
   tttatgtggt gttttgtatg accaagagtt ttaataatgc ttcgatgcgt caatttcatt 71100
   ttctgatcaa aggtctcgaa gaagttcgtg tcgaatgtca aaaactcgac atcacctttg 71160
   tcatgctcga cggcagcgcg gatctagtgt tgaacgactg ggtgcgtgaa catgacattt 71220
   gtgcggtcgt gtgtgatttt aatccgttac gtacggtcag agagtgggtg agccgaatcc 71280
   gtgaccaatt gcccgataca gtatactttg ctcaagtcga tgctcataat gtagtgcctt 71340
   gctgggtcgc atcgaacaaa caagaatata acgctatgtt catgcgcaaa aaactaaatt 71400
   ctaaactaga atgttattta aagccgtttc ctcctgtcgt tcgacacgaa tataaatcca 71460
   gtgttgttct cgatccgtgc actacgacac acatcgattg gcaacaactg ttggcttcgc 71520
   gcgacgctga cgtttccgtg ggacctgtgg attggatcga gcctggttac atcagcgcct 71580
   tgaacgtgtt ggccggattc attcgatgta atctattaaa gtacaaaatg agtcgcaaca 71640
   atcccgtatt aaaaacgcag agcaacatgt cgccctttta ccattttggc caaatatctg 71700
   ttcaacgagt aatgttgcat ctcaatagcc ttaaaatcaa taaaaatgac agtggtatcg 71760
   attcaaacat caacgattac atcgaagagt gttttgttcg tcgtgaatta gcagacaatt 71820
   tctgtttcta caattccaac tatgacactt ttttaggcgc tccgaattgg gccaaggaaa 71880
   ctctggtgat tcacaagtcc gattctaggc aatatattta tagtttattg caattcgagc 71940
   ataatcaaac gcatgatgaa ctatggaatg ctgctcagcg acagctacgc aatgagggta 72000
   aaattcacgg atacctacgc atgtactggg ccaaaaagat tctcgagtgg actcaaactc 72060
   ccgagcaggc gctcgagtat tctatttatc taaacgatag atacagtctc gacggtcgcg 72120
   atcctaacgg ttatgtcggt tgcatgtgga gcatttgcgg tcttcacgat agaagctttt 72180
   tagaacgtca agtgtttggt aaaattcgct atatgaatcg taatggttgc gaacgcaagt 72240
   tcaatgtgaa acaatacatt attaataaca aataaatgat ttataactat tcaattgttt 72300
   tattcgcatc atccttgaaa aatagaatca aactgacgaa tcccatattt atgtattacg 72360
   aaatatacaa tataagtcac aataacgttt gaactcgaac aataaacacg tttcgcgctt 72420
   aatatttttt aataaaatat ggctcaagtc gacgctggcg ttttgactat tcagttcgac 72480
   atcggtagca taagcaaagg atacagcata gactcgagcg acaaagatat cataattgtc 72540
   acaaaatgta cgccggagaa gtttatggat tatttataca accgtactct gctcgtcaat 72600
   aatcacacca agactgttga cggcaattcg actagtgtgg acctgtacaa agctttacat 72660
   ggaatttaca cgggtcgcta ttactatttg ggcgtcttca cgacaaagga agattttctt 72720
   aacaccaatg gaacgtacga catggaattg tatacattta cacgcgagtt gaccaggctg 72780
   cgcataaaca acatactaaa aaccatgatg atgtacaaaa tcaaaggtaa caatccggac 72840
   gcgcccaagt ttttgttgat gaccatgttt catttggcat acacagagta ttggctgaaa 72900
   aataacgatt ttcccgactg tgtaaagcta ccgagtctat tgcaaaagtg taatactcgt 72960
   aagaagaaag tatatcgtca tttgatgcga aacagaagag aaaatacagt accgcttcaa 73020
   agggacattg actacatcaa caattggcaa tacaggttga agattcaagt cagtcaacta 73080
   ccagctacca aaaatcgtta cgatgtcctc aagataataa taatgtacat gctaggtgaa 73140
   tctaatttgt atattcctga aataaataaa gtttaatcat tcaactttta tgtttaattt 73200
   tgtagaataa gtatttcaac aaatttaaat aaaaaaaata cacaatatgc atatacgatc 73260
   agttattcta gccgtagttt tgttaggcta tcaatatgcc atggcttgta cagaaacagg 73320
   tcgtaattgt aaatacagcg atgaatgctg tagcggtgct tgttcggcag tatttggatt 73380
   ttgtttacat agatagatat acaagtcaaa taaaataata caaattagca ttttattaaa 73440
   atcatttatt ttttctggta tataaaacaa tcagaaagac aatttttaca aaaataatct 73500
   atataataat tactaattag ttgttctttg gtcaacacgt gtccccttac gcaacgatct 73560
   aaacgaatcc gattatcgtc atgggttacg tatcttaaag gcagtttgta ttccaacagt 73620
   atagctccat aatttttttg taagttaagc gtcaatcctg catctattat gtgatcacgt 73680
   agcatgcaag ccgtattcag gctttttatc ttgcgaaagg gcctcttgaa attaatcata 73740
   agcactttaa aacacgaatc actcaaagac ttgactgttg gtttggcaca ctcgattcga 73800
   gtataatcgt acactacacg aatatgtctt gatctatcgc ccgtatagaa acgatattcg 73860
   gtccttaacg cttcgggtat atcttctaag cgaacctcaa tattattagt attatatact 73920
   ctagcacgca aactagattt tctaccgcaa atatcgacat caatatgcat gtccctgaac 73980
   ggttccatcg cgtgaataga atcgacccga tcgacaatcg ccgacagaat gaacaaattc 74040
   gattgcatag ctgagtttta ttcgatatac tgatgacatg atttaaaaat aaaaaaatta 74100
   tctaatctta aaagcgttta ttaatttata ctactattac cgctgctctc gctaatttcg 74160
   tccgattgtt cgcgtatttc ctccatatcg tttccattgt tttcatcttc gtcgtcttcg 74220
   tcgtcatctt cgtcattatc gtcatcatcg ctttcgttga ttccgacatc gaatttgttt 74280
   agataatgct gtgtactctt ccacgattcg tggttcatta attttgcgac tttttgcaga 74340
   ggtacaccat tattgtattg attgctgcac aagtagtgtc taatcatatt cgatctgggt 74400
   ctatccatct cgacgccggc ttcttcgaga agtcgtttga aatctttaaa tggggtagaa 74460
   gtgtttttcg agatctgcaa gatcgtcgga tgcttaacgt atatctctcg cgccagctcg 74520
   agtggcttgt ttttaatgtt attcaaagcg tttatacggt tacgttttcg tttcagatta 74580
   atgttgcttc gaatgttgcc cttttttatt agtgtattta ggttttctat ggtcaattga 74640
   cgcgcttcgt tgatgcgcat tccagtgcct agcattatac aaaaaacgat agcgcctctg 74700
   agcagacccc gatcgtgtac atactctccg ttcaaatatt taattttttt ctctatacaa 74760
   acgatcacac tgtcgataat ctttttcaac actatattct tttctttatc tcggatattt 74820
   ttgagttctt tatcgcgggg caacatgacc attttaggaa ttttatactc gggcaagctc 74880
   attgcgttcg aataaaaatt gatggtgagc tggagagttt ctttggtgac cgatctcaat 74940
   tcaagcattc gacgacacag ctcctcgggg tccacgattg cttgttgatt ttcgatagaa 75000
   tcgaactctc tttttagcgt ataattattg atatcatcga gattacgatc gtcgatgaga 75060
   caatagatga gttttatcag acgcgactta tagcttttta atgtggtcgg agcgaagggt 75120
   ttattgaaca tgtactgtga ccacatgctg ttgcttttga cctgatccgg cgagcatcgc 75180
   tggcgttcgg tggcaatctc gaatatctcc tcgaatcttt cgtgactttg aattttacat 75240
   ttccaactat tgaacgagta ctcatttcta agatttgcgg cagcatctac gtcattcatg 75300
   ctagaataaa atagggtggt ttgactatac gtttttctcg ttctcttatt attacaaagt 75360
   aatatatggc atacaatata actagaacac aaaaaataga aagcaatgcc atcaagacca 75420
   catttaaaat tccaccttca gcagcattcg cgtacgcagt ataattagta ttgccgatgt 75480
   cgtttaacaa taacgaagac gatggtcgag ttttattgat accatcgtcc tcggtgtcgt 75540
   tggtcttttg tttaggtttt ttatatccat tttcatcgat atcgttcacg gccaacttca 75600
   acggtatata ctctactttt ttttgattaa gagacatgcg ctcgtaaggc acctctagac 75660
   tcatgatcgt atcacgtgta attatttcag ttgttcaaat tttgtttata acttaactgt 75720
   tgaaaaataa atctacgtaa cgcttcgttt tgaaaggcca attcagtgag gctctgctga 75780
   cagggagttt tatacgattc cgtgtttttc gtcgaagtca atgcagccgc actctcaaaa 75840
   agactggcat tgatgccttt ttctgtgctc agattagcga taaatttaaa aatgtccgtc 75900
   gatgcggaca atggtgccaa ttgacttatg tttcgtttca agctgagcac acgtgatcga 75960
   ttacgttcgt ttagagtata cagataatag tcactgccgt tgccgccaaa caaatcgtca 76020
   ataacgttat tgatgatggt gttgatcatc aacagccgag gatagcgttg cttgtcgttc 76080
   atcccgtatt cgatagcgtt ttgaatgttt tgcggaatag tggctcgctg aagtaacaga 76140
   gtcatgtaat cgtttgcaat cttctggtcg aatggcagcg gaatgggggt gtttgtcgcg 76200
   atcgcttcgg ctatttgata ttcgagggcg aatcccaatt gacgagcggc ctcttcgacg 76260
   ctgctcgagt gtagagctgc tgcattatta ttataaaatt tatgagcgta cgccggcaaa 76320
   acattataga cgaaagaaga tctgaagatg tcgctagtga tctcgctcga tcccaactct 76380
   tgttgcagac gtgcataatg tttgatgaga ttttcatcac tatcgaagcg cttggtcacg 76440
   ttaacgtcga ccggttgggt ttcgatacac acgttacgta tggtactgac cagatctagc 76500
   atttgaggcg agagatgcga catatcgttg gtgctataat aacgaataat tttacccata 76560
   tagtcgacac atttatttaa ccacatgtca ttgacggaag acggcacggc tgccgaggac 76620
   gtcctctgtt gctgctgatt catagtaata aatgttacgg tcgtgactat aataaatttt 76680
   tgattcgcgt ttatgtggac acacgatgat tcgaggatca cttatcatat aattattgtt 76740
   gatatacaaa atcgctataa taaaaataac caaaaaaata aaataccacg aaaacgtaaa 76800
   catattggcc acaatcacag ccaatgccaa agaaacaaat acagtttgca tgctctttcg 76860
   tttacagaga atactctcgc aatttttgaa ggcgacgtta aacttgtttt cgccatcgat 76920
   aaacgctcga agttcgtctt tacagcactc atcgcaaagc accaaagcca ttatcatggt 76980
   gccgtcagag tgcactattt gaaaggtttt cggctgactg cccggatgta attcaaatga 77040
   atatccgttc gatatatcga tgtgagcata gtaatgcgcc aacatggtgc cgtaggtctt 77100
   ttttactcta gttttacaca cttttataat gttcaagtct tcgctatccg ttattccgtc 77160
   gaacagataa tgaatcaata attccggatc atatttaatg cgattgaggg tcgttgtcaa 77220
   gtttttgtcg tttaattgat agttcgccgc tgctgtcgtc atcgctgtcg gaatattcca 77280
   ttgcatctga tccgattcca ttaccgttaa ttttgttgtt ttcgtcacga tcttcacgac 77340
   tatcgactcg tgtgttcttt cttatctcgt cgtcctcgtc gttgctgtct tcgtcgtcgt 77400
   cttcgtcttg agatttattt ccaaaagttt ttagaaataa ttgactgttt gttgattttc 77460
   tcaacggctc aatgttgata ccgttgacgt cgacggcagt gatattattg ttgatgtcct 77520
   cgacaaaatt gttcttcaaa tttatctcgt ttttaagaac agcttccgtc atcttatcag 77580
   tatagactat tttaaaaata caagcacctt tcttgaggat ggtcggttta atgttaaaga 77640
   gaataacgtt aagattgttg ctcatattaa aattatagac tactacataa tcgcctccga 77700
   cgatggtcga cgaacgagac tgattttgaa tcaatttcgc tagcagctga gtgtcgagga 77760
   tgtttaaatt atatgcgcca actgccagtt tttttaagtg atattcatcc tgaacaataa 77820
   acgaaagaat attttcatca ttttctttat cgagtgtcac atagacggca atgtcgactg 77880
   tgccgctgtt gttggtggcc atatttttac ttgtaatatt ttctataata tacttatcaa 77940
   tatacaatga attcgacgaa cgagaattca ataatcgtct caaagtcatg accgagtaca 78000
   taaaaagaac aaatgccgaa catccgctgc cggtttcgtt aagttatgtc tctggcgtcg 78060
   atcaaaattt ttatacagtc actacttttt ccactcaaaa tttaatcgta gaacatatga 78120
   gcatacatga cgatcgacac gaatatttta attttttgac tcaaaaatat gaaaaagtca 78180
   acaattatga tgatgtttta gtcgattcac tcgccaccgt ttcggacaac aacagagtca 78240
   aatctcattc gagtgatccg acaaaatatt tgttgcgagg cgacgacggt tggtttgaaa 78300
   tgaattgccc catagaagag caattcgacg ccaacgcttt tcgttgtgta ccgattcccg 78360
   tgtgtcaaaa taaacaagtc ggagtgcacg gtatgacaga aaatctactc gacagattag 78420
   tgctgaatca tcgaacgccc agagtcgtcg atgacgataa cgttcaatcc gaagaaattc 78480
   atcctactat ttatcttaga tgtgtcgaag gcggatcggc cgtcgtcgaa gagtgtccca 78540
   atggatatct ttttgacgtt tcgcaaggcc gatgtgtaat gcgaaacgac tgtgaaaata 78600
   gacccgacgg ttatgtatta tcacaattcc ctgaatctct aaacatcgac cagtacatga 78660
   tctgtcgtga tggcgataca gtaattcagt cgtgtcctgt cggtcaaata ttcgaccgaa 78720
   ggctgatgtc gtgtgtggag gcgcatccgt gccaatttaa tggagcgggc cacacctaca 78780
   taacgaacga tatcggtcct acgcaatttt ataaatgtca agattccgaa aattcgattc 78840
   taattacatg catcaatcgg gtttttgtca acgacgaata ccaatgttcg ggagattctc 78900
   gatgctctgt tttcgaaaac ggcaccggta cccaaattaa agtttttgac gatgaaatct 78960
   tgtcgtatga ccaaggaatt ctagtttgcg acaactataa cgtcatcaaa aatatcgatt 79020
   gcgatgacac aaatatttta gaaaatcgca tttacaacaa tcgtttcgta ccgatgatca 79080
   atctacctaa agaaatttat tcgatagctg actcgaaatg cgtgtcgttc gattttagtt 79140
   tagttagaca aaataacgac acttttggca tcgaatccgt gcccaatgac tataatataa 79200
   aattcgagac ggcttttacg ggtcgcacca gcagtgcgga cagactaatc gatatcactc 79260
   gaatagatag tgtggtgtct tcatacgccc gcgactttaa tttcgtcgga ttcaattttt 79320
   taaatgaaca agaaattaat tgcgtgcaag gatctttata tgatatcttt gaaggccgac 79380
   gattaaactt gtgcacgagt gatgacaatt ttagacagat tatcattcgt ccaaacgagt 79440
   tttataaatc gaaaaacgtg ctactcgaag tcgatagcga ctatagagag tattgtgccg 79500
   ctcgattaga caataatatg agtcaaatat tcatattcga taattttctc gaaacgttta 79560
   cggtcaatat acgacaattc gacgaatgtc ataatattct cgataaaatt catatcaaat 79620
   atactacgat tgacggtaaa tatactaccg tacagcccga atataacttc aaaagtgtaa 79680
   acgaccaaaa aaatatcgaa agatatgcgg aaaatataca aaattccgag gacactatac 79740
   cggtgctttt cgaccctttc gagcacatcg aaaccgtaaa gcctctgttc aatccgtttg 79800
   cagagaacac gattgctaga gatttacccg attttccaga cttgttcccg ccttatcctc 79860
   ctcctcgtcc tcctccgcca ccaccaccag tcataccaga gcccgaactg actctaagga 79920
   acaaatttct taattattct tgcttctttt cattgcccac gtttaaaatg agtcattgtg 79980
   atgtcgtcga tgaccacata attgaagcta tcgctaatat tagacagaac gtacaggtgc 80040
   acgatgattg tgtcaacgct gaaggcatca aaaacgtgat taatgcctac gtttatctgg 80100
   gaaatgacat cggttgtaga acagaattta ccgatgacgg cactatacat gttaatcgtg 80160
   taaacgaacc cacgaaattt atgaatctga ccactcaatc aaacgacggc ataaaataca 80220
   accattggat acatacaaac gatggaataa attatttggc atgtcctcac aatttgtaca 80280
   atagcaaccg atttacctgt tccgtcgaat cgaacagatt gtattacatg caagatttac 80340
   agaattaaag aaaaaaaact attgtatatt tgtgtaaacg tttaatattt attaaaataa 80400
   atggtcaaaa tagaaattta attttattca ttcattcatt cattcattca ccccgcaata 80460
   aagggttctg taacagtcgc gttggctgga ggaaacggtg gaataacttc tcgcggcacc 80520
   gaaatcggca tgatggatgc ccgagatccg tcgtcgggtc taagaatttc acttcccatg 80580
   atcaacggca ccatcatcgg atattgttcg tatcgggcga gagatctctg tagagcagtg 80640
   gcgttgccct taaacttgag aacgttttcg atctgtaaaa agttttcgtt tatgcccgat 80700
   ctatatttgg gttgaacggg attatagaag ggcacgttgg caacgagacc ggtgctgtcg 80760
   aatcggcacg tggcacaatt tctaaactct aaactctccg tcgatatctt catgatttga 80820
   ggcgcgacgc acttgttgat gagattttga agatagggcg gcatactggc gactactcgt 80880
   tcagagatat cgtcattctc atcgtcccaa ctataggttc gagcgctatt gttcttgacg 80940
   accgaacaga atctcgtggg atccgtctgc gacaggacat gagtagtgat cgaatataga 81000
   tcgtttacta tgttcactag ttccatttga aagctctcgt tgtatcttaa cgattgacac 81060
   acttgttctt gtgcttcata gttgttgtag atgatgtgta acacgagctg ttcgggcaag 81120
   ggtagtgcca taacgttaag cactgtctga tagtgttctt tggtgggaat aagtattcga 81180
   tcgttgccca cggctttgtg attgacgagg cttttgccga cggtacgata gtatgtgttg 81240
   ccttcgccat cgacgatcgg catcgacatt ttctccattt taaatcgaac ggacgcatga 81300
   aaatcgcaaa tgaaccaacc gtcgtcgaca ttcgcatccg gagaacacgg agagccgtac 81360
   gttcgacaca catcgaacgg ccgtaccgct tggaagatac aataattatt catacggttg 81420
   ctgatgctcc cgctcgacac tagtgccatg gctatcgaaa atgaaatctc ttattcaata 81480
   aatttgagcc aagatttgtt gtatattatt ttagattctt atatttctaa aaagtttatt 81540
   gtgacagaag aatacactga tttcgtggac gaaaacaacg tgcgcactcg acgatatgtc 81600
   ggcgaacata gattcgtgag cgtgaagaaa acttgtaaaa gtctcgaaaa aatagttttc 81660
   gtcgacaaaa atattgtggt gccgttcgtg aatcgaatta gtttcgaaga acctccagac 81720
   actacgccca cgaacagctt gcgaagaata ataaagtgta aagtttacaa aagtgataaa 81780
   tgccccgact gtgagataaa gttcgagcac gtctacttta acaagaacaa aattgacaat 81840
   ttagattccc taatggccac gaaacagatg gtgctctata atttactaca aaataaaaac 81900
   gaaactctgg tgaagcagtc tcatttggga tcggatgaaa tactggccaa tctacgcttg 81960
   gaatatgaat acacgacggg atttgtcaat agtgacgttt taaaattcat gtctacaatc 82020
   gtgcgagact ttgacgagat tgtggcgtat aagaacatca gtcctatgct gccttatact 82080
   acgctacaga atcatataat ctacaggaaa tttgaagatg aaaaatgtct aatggataat 82140
   tttgaagaca tcaaagatgt gtataaatgg gcactaaaac tcgacggtat tcgaggcaga 82200
   gggtttatca ccaaaaagca aataataatt tttatggacg atatgcaaat tttttccgga 82260
   ccttttgatt cgccattcag catcaacaac gtgattgcct ttcaatgtga actcatagaa 82320
   aaaaccctat atattactga tcttcttcac gtgttcaaat atacgtataa taatcgtaca 82380
   caatacgaat gttccttgga tccatacaat atagatcccg ccaccgctat cgattgtatc 82440
   aatttaatgg ctaatctata caacaaaccg tctaacgacg acaacgacaa tttcaagatt 82500
   tacacattta aaaatgaaac gatcagcatt aaatttcaaa aattctatga tccgccgatc 82560
   aaatctcttg gatacagcac cttgccgacc gatggatttg tcgtgctcga cacgaacatg 82620
   agctacgtaa aatataaata ctataaaacc gtagaattgg agtacgacaa gaaaaaagat 82680
   gtgtttaaaa ctctcgaaag cgatctcgaa caatacatgg tcaccattaa tccgtgcgtg 82740
   ggtgaattga aacacgagag catctacgaa gctatcgtcc acgataatca tataaatgtc 82800
   atcaaatatc gtcccgatag attgattccg aattgattta gacacaattt cggagtgatc 82860
   gactttgttt ttgaaaaata taaataaata aataaattta caaacagaat taaaattggt 82920
   tttattcgat aatgtattac atgtgtaacg actttttgta ttcggcccat gtcatcctct 82980
   caaagtgttt aacaggcatc atattccttt gtatgtaacg ataatcgttt atatgattgt 83040
   gaaagaccat tgtggcgtat aaagttccgt gtttcattaa tgtgttttga ttagaatttt 83100
   tgatggtaat ctcgtcgacc atgatgattt tctcaccaaa tttctctcga ttcaaactaa 83160
   tttcgatgcg ttctatagaa aatattaaaa atcctttaat acccaaataa tgttctcgac 83220
   acattgcaca gtttaattta aaaaataaat tataaaatat ggtcttcatt tgttggagat 83280
   tggcggtgac aatttcgtaa gttagcttgt ctcggctgtc gatcatgtcg tcgacgacga 83340
   tagcgagaaa atgaatggta tcccagattg tggtgaagga atatgaatat tttttgggtt 83400
   gaaacgcccg caaattgagc tcttcggtct tttcgataaa taacacttgc atctgttgca 83460
   aatcaaaggt gctcggcaac tctaaagccc agttcttcag ctgttcgatc tcgttcgctt 83520
   gttcatcttt gtaggtgatc acgcaagcta tgctatataa ataagtcagt tctgtcgcta 83580
   gaatttcggc aatttctctc gattgacagg ttcgtattcg gtctatgtga cgaaaagcgt 83640
   acagaaaaaa actatcttta taacgagaaa acaaaggagt caacggtatc atgattaacg 83700
   attctgaaaa aactatttat ctatatctat cggatatgcc gaacggtatt cagaacgaca 83760
   aggccgacga tggcgatata atctattttg agggaattat cgagtgtttc gacgacgaga 83820
   ccgccgataa atttggcttt ttcgctgagc tgaagaaaga ggaagcctta tttatgaaaa 83880
   aaacatttta cgatctcatc gaacacaaca acggcaacta ttgcaaaaat catgtactaa 83940
   tcgacgcgct cattatgtat aagacgtatg cggaattggt ggacgaatcg gcatttggcg 84000
   tgaacatact caattattgt gtagaatact tgacacacgt tttcaagtta tttcgcttgc 84060
   agagtcgcat aattgttgta ctgccgacac acgtagactg gcagcaagat aatttaagtg 84120
   ctcttttgaa acatttacta cagtccacgc tgattgaaat cgtttcaaaa tgatcggaac 84180
   tctagtattg ataatcatag tgttggcggt gttgtacttt ttgtttatca acaacaaact 84240
   gaacatgaac tcgatcaacg aatcttcgcc gagtttagcg gacagcagca attccctgca 84300
   gaccgacccg cagaccggac aattgagcgt caagttcaat agtcccaaaa tcaagtccct 84360
   tcgcgtgtta cacggagaaa acaaaattag taaaatctac gtcagcgaaa aaccattgac 84420
   atacaacgaa atcatcgacg aaggcaacag atccgtgggc acgaattgtg tttttctagg 84480
   aactcttctt gatacatcat catcatcatc atcaccatca gcggcaaatg cgactacaac 84540
   gactcgcact actaccaatt tcgatgtgaa acaattcaaa aacgtgttta tagtcttcaa 84600
   aaatctcgaa tcgagcaaga ttaaagagtc tacaaacatg actcgctacg aagccgaagg 84660
   aatggtgtat tgtttaatcg attcaactac aacgaccgtg cccgatctaa gagacgtgtc 84720
   gtacccgatt gtagtgtaca cgaccaatgc aaacgcgcag ctcaagctca aggaatggga 84780
   ctacacacag ctcaacgatg ctggaactct cttcctaaag aatgaaaaat cttttaggct 84840
   acagtaattt accgataaat cctaactatg aattgaaaga tagactactt gtaatttatg 84900
   ataaaggaaa cacatgttaa aataataaaa aaatttttgt ttaatctcta tatttttcta 84960
   ataaatatac aaaattcaaa cacatttttt attcaaatct agcataaaaa ttctggtcta 85020
   tgtatgttga attgttcctt gttccaggcc atgtcgatgc tgtagaagca tttttcatct 85080
   tctttatagt actttttata tttacgttta aaagcactaa acaatgtggc aattcgttgc 85140
   gaaccgttgg cgtttcgttt gctgaccaac agatcgtgta gcatattctc gacgtaggga 85200
   acggcaagct cgatcatctt ctcgatcttt gtttcgtcaa tcattttggc tcccggtttt 85260
   acttcgactt tgagcacgta gattaaagcg ttcaacggac tattgttcac gtccagacac 85320
   agtagattgt gattgtgtat aggatcgtgt ttaatgatat ttttataggg aacataacca 85380
   tctttggcgt ttcgcttgta catgagtacg tgagatagga agattcgaac cggtttcgcc 85440
   aaatcttcat tgtagttttt ctccatcgga tagagtttat ttttaatgtg ccaatatata 85500
   gacccgttga atggtaaatt ttcttcaaag agatgatcag tataaatgac ggcaaatcta 85560
   ttgcgcacac ccttgtcata gtccgagatg tgcagcggtt tattattgac gatgagcaat 85620
   ttataattag cctcatactt ttgactgccc tgaaactttc gacagaccga attgctcttg 85680
   gtcgagtctg cggtactctt gaagaaagaa tcgtcacaca ccttcatttc attgatcacg 85740
   tacagctgag agatcatttt atcggcctcc atttcgttgg tgtccttctt ggatagagtg 85800
   tacttgtcgc tgtcgtgttt atgcactacg actatatact cgagcagttc gaaaaagctc 85860
   gattttcccg agcccggctt gccgttcaag tagatgcagc acttttcata atcagagggt 85920
   atgcccatgc tcgccccgaa atgcatcata accaaagagt ttatatagtt aaaattggta 85980
   aaccctcgga aatagagata tcccctgacg atttgcttta caaaagtcga attatactcg 86040
   tcgagaccga gtttggtcat aaagattctc atataaaatc tgactaacca cgagttgagg 86100
   tcatcgttgg gtctgcgtat gatcaatttg tcccaccata gattccacct ctttaaaatg 86160
   tgccatgtgt tacaatagtt gttgtaaaaa ttaattaaat tctctttaga ttcttcattt 86220
   tcgacatctt tcaaactgtc gtttatccgg ttatcgtcag cgaaatcgtc attatcgtca 86280
   tgattgtttt gatttataca cgatctagac gacaagactt gtgttttgag tgtctcgatg 86340
   aaatcgtctg gtgacgacaa ttccgtaata aaattatcaa taaaatcgat tctatcaatc 86400
   ttcgagtata aataatcgat tattgattcg cgatgttcat tgaaaaattc tagattattg 86460
   tagagtaaaa tgttttcatg gtttttcttc agtgaccatt tcaagttgtc ttttaagact 86520
   ttaaaatata aaccagtcga gtagagtaga gaccaagaca gctcgatgat gcttcgtgtt 86580
   ttcgaaaaca actccatgat tagagcgatt ctaaaagcac gcgaattaat ctcaatattc 86640
   atcagacatt ggcacttttt cttttgttgt gccgattttt gctccagaca ctctgaacac 86700
   tttagattga tcatcagatc ggtcaatttt gtattgctca gatagacacc aatcgtgatc 86760
   aattcgctat gatccgaatg ccaaatttca cgaaatacat cattcagagc actctgttcg 86820
   cggagcttgc atttcaagca attgttgaga gccaatgcta tggacatgtt gttttttaaa 86880
   gtccgaatat ctctacagat tttagccata tgatatgtct tgtaaattac attttcgttc 86940
   ttgccattgt ccatcataaa ttgtatgaca cgttcgggaa gataggtttt ttcctctttt 87000
   cgggtcaacg attttatcag agtattgccc agaagaaaag gacaactgct atggtaatcg 87060
   ttaatgaaca gattgtaaac accctcttca gtgaaatata aatatttcca gttgttaaac 87120
   ttgacgctag gcagtgttat gccgttgaca gactcagtca acttgtggag atcctccttt 87180
   tccttgacgg caatgtaatg ctttccgtta tatacgagaa tggtattggc cgatttgatt 87240
   ttattaaaaa atccactaca taacacttcg gcagtagcgc cacagctttt tgcgagatcg 87300
   tatgtatagt cccaggtttt gtttttgtaa taaatcaaat agtgaatagc gtaataatgg 87360
   gccaacgata cgcacggatc cacattcaag aacacggccc aatgattaca gaaacgaata 87420
   aagacttttg gcgtcaatct gtagtatggc tcgcaacgct tcttcgccaa ctcgaaatta 87480
   attcccttgt ctccgaaaat actctcgacc actaattcta gatacagttt tatgtcggtc 87540
   ttgcaaaact cgagttcttc aaccttggag atggctctcc atataacgat tataatgtaa 87600
   tcgaaattca taaaattgct gtcgataaaa taattttgga gaactctagt atctgccaaa 87660
   tcggtggctg tcaatacttc agccatattt tcgattatag tttccacaca atcgagtatt 87720
   tgctgctgaa atttgtccaa attctcactg ctgggagtga tattcttgtg ggctttcaat 87780
   ttctccttgc caatcaaggc ggctggcgtt tcaaattcct tgtcaaacgc acgtagattg 87840
   tttagattga tctgaatcat gtgtttatac ttttctgccg tgatacattt cgataaattt 87900
   atatcgtctc taatgtagtc gaaaggattt ttattggaaa acactaatct atccttgact 87960
   ctgcagacgc cttcgcctcc gtcgttcatc gccagatgaa acatgatttc gtcactatcg 88020
   tcgaatctac tgcggccgtt gacaaacatg gtcctgttgt cttcgtagct cttgatcgac 88080
   aactccacat tgaggaaatc ttcaggattg aacacaaaca aatttacgtt tcctagcgat 88140
   ttgttgtgaa tcaggggtat tctgttgcca atgtcgatgt cgtgcttcat cagtaaatag 88200
   tggcgccatc catagtaaga tataggaata ttgggccata tgtaatagct gccgactcta 88260
   ttacactcgt tgccgaatcc gttttgattg ctgttaaaaa actcttcaaa gttgatgtca 88320
   tctctgatac gatcgtacaa gtccacgtga ataaagggat tcaccatcaa ataaaagtaa 88380
   ttgtcttcga caaaccagtc gtgcggcttc atcgaagaca gatcgtgagt agtggcgcac 88440
   gagagtcgtt gttgagtttc cgtgtgtccg tcgattaaca tcaacactag tttttcgaaa 88500
   ttttcataat tgtcgatctg caatctttgc tcggtcgatt gatttacaat aacgatacta 88560
   tcgacattca ctgtattgtt gtcgccgata gatttaaaaa tgttatcgaa aatagtatta 88620
   gatctaatcg gtagtgctgc cattttaggt ctactgattt ttttaatggc gcttctgttt 88680
   ataaatcctt atcggcgcga catagaaaga ttagtgagag atcacaaaca aactttacaa 88740
   tttggcgctt acattgacat tttcgatttg agcacacgac aacaaatcga acgactgttt 88800
   ataataaaac ccgaaaacgt aatattgtac aatacgaacg gtagtctatt ttattacttg 88860
   gcatcgacca gtttattgtg tccaaacgag ttttctttgg tgcgcttcac tcgacccgaa 88920
   attgtagcat ttaacgagaa cggtcaagcc aataccgtct gcaccaatgt aaacagttta 88980
   gttatcgttg aacactttat gactctcaag aacaacatcg ccgacgacag aatacttttg 89040
   tccgtagacg aaatccatta cacaattatt gatatcataa atttactcat tcgttcggga 89100
   tatgtatttc tacaataact gcaaaatttt caacaatatc gttgacaatg cccacgacaa 89160
   agtcgtcgac aatcgtttca ataatagttt caataatagt ttcaagtgtt tcgatggtta 89220
   atttttaatt taattacaca atgaccacaa actaaacgat aatgtttgca attttaaaga 89280
   tatttaccac aaataaagcg attgcggcta caatgtcgaa gacatcgaca gatatctaaa 89340
   ccgatgcggt cgtcaaaatt ataaagacta tgaccacgaa tacaacggac gtgatttcaa 89400
   agacagactc gccatttgta atcttttaca aattaggtta gaggcgttag atataaagta 89460
   tttagtctca tttttgctac agtcttaagc gctcagcaat cgagatggga cattttttaa 89520
   tccaacgaga atctatcttt gacgatttga tcgaatctag tgtcggcttt caagatgtgg 89580
   acaaattcga ctttattaaa gacattatcg tcaataccga tgtgttggag agggattgtg 89640
   ccgattttat ggatccgaat ttagaattta atataacggc tccaatctac gaattgtgtc 89700
   atgaaagttt acttaaaatc atttggattg ccaagtacgg accgaactgt tttttaccag 89760
   agttttggac tttttttatt agaaaatata acggaaaatg ctataaatgt gacaatcgtg 89820
   cacagttacg ttatcccgag tcttgtaact attgcgattc cgatatcagg caattttatt 89880
   atcatgacga tggaataata ttcgaagaac tagtcaatga cgataataac tattgttctt 89940
   catgtcgaaa ggctctattc gacatagtgg atgtgtatca atttaatcat tccatctgta 90000
   gatgtgatta ttaaataaaa aattttatat atatcactac cattaacatg cgtttgtcgt 90060
   cgacgattct gatcgatcac ggagaaaaat cttgttggat gttgatcgat gtcaaaaaac 90120
   gtaaaatcga ttctttaaac gacaataacg acaatatcgt caaagacagc ggcgtgcaag 90180
   ataaactctg cgtatttggt gaaaataacc acaaaattat catcaacgac ggcgacgacg 90240
   acgacgacaa caacgatggt agatttgtaa aaaggagaaa aataaaataa ctttttgaaa 90300
   ttttgtgttt tatttatact atatattatt tgatttgaac caattttcgt attcgtcaat 90360
   gttgtcaacg atttcgtcat gataaatgtc ccaatcgttg atgggctcga tacatttgcg 90420
   aactttaata aaataatcat acgaataatc gttcgttttc aaatcgtcga cgagagaaat 90480
   cgtttttatg tagtttattc ccgcttttct gaggtaatat agcactatcc ttggggactt 90540
   gggaagtttg gcatcctcgt cgttgttgac gttaacatct gaataaaaag ccttttccac 90600
   gaagatgtgt ttcgttttat tatcggcaat cactcgtctc gtcattgtcg acgatggcgt 90660
   cgacgagcgt tgaccgccgc agattacgat atcaaaatac ggcacgagtc tagttctatc 90720
   cattgaataa gtcacgtgct ctttgtttcc ataagaccaa aggaccaaga caaatccctt 90780
   ttctttgagg gtaattaagc tgtcgtaaac gtcatcgttt cttatcctga cgtcttcctc 90840
   gtcggtgatg agagtattgt cgagatcgaa gacgagaacg tgcggtatct cccatgaaaa 90900
   gctgtccgtt tgcatctgat aaatttctaa atagttttga acgtaccagt ctttgagaaa 90960
   ccaatacatt ggtatttttt cattaattac atacacgtgg cctagcgcag acgttttgaa 91020
   agccagtttt aaatttactc gaatttcacg catattgtct tcacatcgta ccacgtgcat 91080
   gatgtaattg ttggtgtcga tcgagcgagc gttgaacgac tttttgtcaa gggcgaacac 91140
   gataaactcg aacagatcca aatgtcgaaa agacaccagc gaaaggtcgg cgaagtcgca 91200
   aacgacaaga atgtgtcttt tgacaagagc ggcgcgtctg cgaagcacga cccaggtcgg 91260
   tggcgtcata attgttatga attattttta atttttaaag aatttcgaaa caacaatcaa 91320
   tacaaggagc tgatacaatt tctcgtcgac aactatccgg ccaatgtcaa gaataaaact 91380
   tttaattttg tcaatactga acatttattt cattcattgt acgcttatat accggccatc 91440
   accaatgtgg aaaaagaacg aaagcaaatt cgtctatcgg aagaatgtat ccataaactg 91500
   tttgttaata ctataaacga ttttaaacta tattcagagc tattcgacat gataatcatt 91560
   cagaagatga aagagacctg tccctgtcaa ttgctgctac agagaagaga agaaatcaaa 91620
   gtgtatgtaa acacgattag agataaaaaa ttcgattcta aaccgccaaa gctcaaaaaa 91680
   gagtacatcg acaacatcat gtacaagtac tcgttaaatt ggaaaaatct tttattgaaa 91740
   aaaaaattat cagaaacgag cgacggccgg ggcaacaccg tgggcattgg caagagtaaa 91800
   aatgccaaaa aaaaattaaa actcaaaaag agaacaattt tgaccgatga tttgatttat 91860
   ttaaaaaata ttaaacattc acaaagattg ccagcgataa atggtatgtc gctcaaagtc 91920
   tgtaaacaca gctttatcac tatggagaag cagatgcgag ccggcgacga agccgtttcc 91980
   ttcattagat actgtcaata ctgtggcaaa attggaaaca acggtaataa tcaataacgt 92040
   ctccggctgt atccgtaggg attgcgacta ccgctcgatg agcgtctgcc gcgaggacgt 92100
   ccgggtctac gacgcctgcc tccgctagag cggcgcctgc tggtcgacct gcgtctgtat 92160
   tgtccgccgc ttgagcgccg cctgcctccg cttgaacgac gcctatactg tccgccgctt 92220
   gaacgacgcc tgcctccgct ggagcggcgc ctgcctccgc tggagcgacg acgcccgccc 92280
   gatgatcgtc gacgatatac cattttgttg agttgaatac aatcgtgttt aaagtattgt 92340
   gttttttatt ttacacctta tattattatt ttagttcgat atagccttat tagtattttt 92400
   tacgtttgtt cgaggtgccg ctgctgccca aatagatatt caacgaatcg gtctgctcgg 92460
   gagttttgac gagaattcca catttctcac tatactctcg aatgttgtcc atcaccttga 92520
   aaatgttgtt atagtcgttg acgttgaact tgcaattgga cacggcatag ttttccatgg 92580
   tagtatagaa tatggagttg gccgcattat agaacattcg atgtatagag aaatcatctg 92640
   taatcttgag caactcatta ataaaatctt cgtcgtcgca atacggaatt cgattgcctt 92700
   cgctcgcatt gacatattgc tgcggctcga gcttgctcag ttcgtctcct cgttcggtca 92760
   gcagttcttc gagagtgcat ctcttgtcgc gcgacagagc cgaactctgc agcactatga 92820
   cgcgagcgaa acgagtcaca ggatagttca tggccttgtt catcgtgctc tttagaattt 92880
   taatattgcc aaagtcgaca atcgacgtag gtagatccag caaatttttg agtaaatctg 92940
   ttaaatgatg cacctcatcg ctggtgtagt tgggcataca ttcgtattcg tccaccatcg 93000
   agctctccag tagagcatag agaggtctgt atttaggcga tttggccaga tagatcatgc 93060
   acgagacgag atcgctaact ttgaaatcgg tcgaactcgt attcgtcaat gtatagtgtt 93120
   tgagtatttt ctggcaattt ttgcggaaga gtcccatgtt ggcgctagag ctgtccgccg 93180
   cgatcgatct accgccgccg ctgctgctag agaataaact cagaggattt ctctgtgcag 93240
   tcggcgccat cgtcgtcgtc gacgtcgcag gaaaaggaca tgaactcgcg gccggcggtg 93300
   tcgatcctct ctcgaattga ctagtgagag cagcattatt ttcgacgtaa gacgcttgcg 93360
   acagttgagc cacggtggtg agaaagtctt gaaactcgtc catcgacagg tccatggtgg 93420
   cgtcgggatc ggcgagtaga gtaaaaaatt ttggccaaat ggccatattc atttgtgtgt 93480
   cgattttatt ttttattcgt tcaatttcga gaaataacat tacagaactc atcttgaaaa 93540
   ttgggtactt cttcttattg atttacgatt acaaatttat tatcatatat attcgacatg 93600
   agctggagca cgtttatagt tttgaccgta ttcaaacgaa tagactgttc ttcgttcgag 93660
   atggcctcta atatagtttt agctatcgca gattgcttgc ttattacacg aatcgcgtta 93720
   cgtttttgtg ggttggtgtc gttgagaata acgtccgcca cggtattcgt ctggtttaaa 93780
   ttttgcaaaa attcagccga attcataatc atgtctccat cgtcttcttc gttatcgtcc 93840
   atcgttcgat tcgagtggag gcgacgactc gtggttcttg ccgtgccgat cagactagcc 93900
   gacgacattg tcgttgagta gatgatacag ttcttcgtct agtttatatt tagtcaccag 93960
   ttgtcttata tagcgttcgg ctacaatgta ttcgctcatc gtttcactga ataaatcctg 94020
   tttgatactc tgcagtttga gcataaattt ctcaaattta tcgtcatcat aattatgaaa 94080
   aatgaatcga cagacgtttc tcatttctaa ttctgcagca cttttcgttt tattcgtaga 94140
   cgcttccaag tatagtctca aaaagaagcc ggtgaacact atcgaagcga ttttatttat 94200
   ttttggtgat tttattttcg ttttgagagc cagttcagtt atgaatcgtt tgaacggaga 94260
   gaacagctta gattcgcaag tattgttgtt gcgcaatttc cacaaaagtt gctcgagatc 94320
   gccatcgcca agaccgccgg cgacgagacg acattctttg atgagagaag cgcacgcttt 94380
   aaagttcaaa tagcccgtgg tggctttgtc acataataga ttataaaaga attgagtaaa 94440
   agaatttgta atcaaatcgt cagcattgaa cactccattg gtgtcgtact cagtttttag 94500
   aataatatac aggaaaatgg gcagaccgaa catgggtctt aaaaatatgt cccatccgtc 94560
   ctgtatggcg gtatcgaaaa cggtcaagct ctccgacaaa taattaatct tgcagctgag 94620
   acattcgatt ttgttaacgg agcatacttc acatttcgaa ttcagctgca ttatcgaagg 94680
   cgctggtgtg gatttataat atttttgcaa gtattgcatt atagttctga attttggcac 94740
   ttgactcatg aattcatctt tgagaaaaac ggaaaaaatt tttttgacat cattattgtc 94800
   ctgttttctg tcgaaatcct gtttcaccac gtcgacacat ttattaaatt cattgaagaa 94860
   cgtcaagcct ttgatgttga cgttagtgct ctgatcgaaa tactttgaaa ataagaatgc 94920
   cagtgaatct atttcggccg tgctcaaaag aaccctaaaa ttgacattct gaaaagagtc 94980
   aaatttattg aatctcagag tgtattcgac taggttcatt gcgttggtca tttttcaaat 95040
   actacactta tcagtatacc gaaaacatgg cctccgatga cgattatgat gaaggtgtcg 95100
   acatcaacac cgccgatgca accatcagac ataacacatt taaaataaaa ttcgaattcg 95160
   ccaatctact gtttaaatat ctggcatacg ttagaccgga agaaagaatc aatctcgagt 95220
   cgtttcgctc tcgtctcgac gacttagaca ttggaattga gtttgaaatc tctaatttta 95280
   ctctcgataa cgtgataaat caaatgaaaa atttattaat ttacgtgact cccggcccat 95340
   ctgtaaatgt tgaaccgtcc gcgacgagtg tcaccaccgc gaatttaatt ttcaccaaca 95400
   acgatgacat cgacacgatc aaactatatc tgacaaatct tttgcaaaac gaaaatctaa 95460
   aacctttaac gtatcgatcg ttaaacgaat tggaccagag attgaagacg gaatccatca 95520
   tttatgcgga cattctctcc gaattacggc tggacgccat cgactgtgaa caaaatacga 95580
   tactccagag atttttgaat atttataaaa attatggaat cgccaactgt atcgacacag 95640
   atctccggta ttatgtcgaa aagttgcggg attttgaccg atccacgctg ccgccgacgg 95700
   tggcggatgc gctcaaaacg atcatatata atatggactc gccgtataaa attcaagtta 95760
   cgatggatcg acaggaatat ctcacgggcg cgaccaacgt cgacgtcagg gcgcttttca 95820
   acagatataa cgagatttta cccataaaat ttgtttcgtc agagacgtcg gcggtaacgc 95880
   cgatggacgc gacaacatcg gcatcgttca aacgaaaatt gggatcggat cgaaaaaatt 95940
   attcctcgag ttccgaatac gatgacgata ccgacgctat tggtggcgtc ggacgcatca 96000
   ttagcgatat cggctcgacc gccgccgact cttcgactct tcgaaaaaaa agacgaaaac 96060
   gaaaagccaa aaaaatatct agcgccgcga catcgaagcc gatcgctaca acaaaacctt 96120
   cgacgaccac aaccaccgag ccgatcatct cgtcttcggc caatcaaata tttatcgaag 96180
   gcgttcgaca atcggcaaga ccgacgatgg ctcttccgcc attgttgttg tatatcattc 96240
   gaatcgtacc gaccgatgtg aacgcttcgc tattgacatg tccctcgaac gcgttgagct 96300
   cgaatatcaa cacttttaat ttctacggta aattgtcaag aattaaatcg ttaaatctca 96360
   cgacaatcga cagaaatgtc cattttttcg agttgctcga acctctagcg cattatggtg 96420
   tcactctaaa cgacatcaat cggtcgattt ggttcatttc aaaggccgga aactatttcg 96480
   aggcgaacgc ttttaatttc aataacatca gacggaatct ggctaaagaa acggacgatt 96540
   ccgatcgcat agctctcttc atgatacgct ataattttct atggcactat agacaattta 96600
   taagtaaact agtgtccagt gccatatcac catataaaaa ccagaaaatc attaacgtat 96660
   tgcaggtgtg cagcaattcg gtcaatagag cgtttgataa agccaatatt agactaaaca 96720
   actcaaaaat ttactccggt cccgttgacg atatagttcg tctgatgaac ggcatgtttg 96780
   cggatctgtt gatatgatcg tttatgttgc cttgcttgtt gtggtgtttg tcgtctttct 96840
   gaccgttctc gttatattga gtgttaataa aattcaatta agacaaatgt tgtattatca 96900
   atataaatat ataccggaac cattaataag attcgtcaaa gtcgatcgac tcaaaaccta 96960
   cgatgacgtg tccttttaac attaaagtaa tcatcagcga tagattcttt acgtttcctc 97020
   acgattatgt cgtgccacaa actgacgtag gcggagctcc agtccgcaat ctagtcgtgt 97080
   acgtgccctc cgaagaggac gtgcagtacg tagacaagcg cctgtttacc aatttcgatt 97140
   cggtcctagt ctatagacac gaatggacgg atcgagtcga gagtcgagct cccaaaaaaa 97200
   atggcatcgc caccgtcgtc tactggaatc cgatcctgcc catcgccgag gtcggcgtgg 97260
   gcgaaacacg cgtttttagc gttttattga ccgataaact gttttattgc aacaccatga 97320
   ttgtcgatcc caacgtaccc gtttgtccca ttcagattat gaataaatcc ctacgaaact 97380
   atataccgat cacgggcgag tgtccgcttc agcatttgaa cgtgttactc gacgataata 97440
   aaaataactt tttaatatgt ttcaatagag agacatcgac cgctctcagg ctattgaaca 97500
   tcaaacggat catgaccatt ttcggttata gacgagtctc tgccaagtat tcgttcaata 97560
   tgcaagattc tcatctcgaa cagatttata tcgaactcaa gtacgagctg attcgcagac 97620
   tgatgaaagg cgacaccaca ccgaaatgct tacaattgaa cgtcaacagc ttggagtatg 97680
   tgaagagggc gagagagctt ctgatgatac ccgactctgc tcagactata gtcaatttag 97740
   tcaaaatgtt ttcaaaactt attctgccat atcttttagt gccggacgtg atcatcaaat 97800
   tgaacactat ggaccgacag cgtaaggttc gattgttctg taaaaacgac agttttgcta 97860
   taacttcatt cggacccgtg cccaacaata tggtcgaaga taacccgatg gcgttcgatt 97920
   attcggacat caatacgccc ggacacttga atagcgtcaa agaacagata tttacggcat 97980
   ctcggatcga aaacgtgacc atcacagcgg ctagatacaa ttactttttt taaaccacaa 98040
   ataagtcgac atcatgagac gtatggctag aactctcggc ggcagcgaca caaactcgat 98100
   acttaaccag gatcaactcg aacaattagt caaccgaaat cagtcatttt tacgtgattt 98160
   tcttttggtt atttgtggtg ttgttgtctt tattgtatgt ttattattta tagtgttaat 98220
   tttcaatttg aactcttcga tagaagctag agaacgacag aaacggcaac gaaaacagac 98280
   atttatggct aatctcgatt acagataccg acagccgaac aacagtttta ataaataatt 98340
   ttttttaact tgtattttgt tttaataatt gaatttaaat tgattactgt gtgcattttc 98400
   gaaataaaag ctgttggagg ttttggttac ggtcgagggc acaattaaat cagaaatgga 98460
   catggaacca aaagacaacg gttcttcaat atcttcgaat aaaagctgat gtgttaggcg 98520
   agacctgctc gtcgcgttat tgatagttat ttgacgagtt tcagtgtctt tgagtatgac 98580
   aataggaaaa tctactatac attggatggt gctcgagttt gtggtgatga tgaaggcgtt 98640
   cgtgtcgggc acacgaacaa ccgtcgtgtt atcttcatcg agagcatcct cgaccaacaa 98700
   actgaaacag gttattcctc tgccggtgtt tatcaattca gacgagatga tctgttcgac 98760
   cgtttgcatg ttaatcggtt ctaacaatcg agtatcaaaa ttcgataagc ctcctaggtt 98820
   attgttcaca atgttgtgat gtttggtcac gactccattc gacacttgat tgttagagtt 98880
   actcgtccaa cgagactctt gagttaaatc tttcgttaaa accacacaac gtatggtaga 98940
   tgaagttaca ggacgaatcg cattaatacg ttcgtacata tggaacatgc cgtatcgatg 99000
   atacagtgta tagctgtaat attcgatgga taaagcatcg aaacgagcgt gcatagccat 99060
   gacaccggcg ttttctgtgg cgcacattgc cgtatatgca aacgccggat gaaagctact 99120
   cgtgctcggg cccgtagtcg gtatcaccac ttcgccctgc agattggtca acagcaaaat 99180
   accagactcg agacctagca tttgcgtacg atacggaatt actcgagctc cgttcgccca 99240
   gattttgcga gtcatcgtcc acaacggagc gtgcctatta ttagtgggat cggcctcgta 99300
   ataggcgatt tcgggagatt ggcccactac ggaaccgaaa tatttgtcgt ttcgaatggt 99360
   taaaatttta ctgaagtctc cactgattac accattacga tagtcaataa agaatccgag 99420
   cacattagaa aaatgagctc cgtttctaga tagaacggct ggattggcaa gtccctgtct 99480
   actgccgatc agcgagattg aattataaat attttgcata ttgacagtcg attcaccgaa 99540
   tagatagttg tagtagtcga atgtgaagaa actgttaatc agatatccgt aggccctgac 99600
   gtcggtgtga tcgaagtagg caaaatcttg atggatgccg ttaccggaca ctacaatcgg 99660
   gaaattgatc aatttgagca cgtattgaac ctgatcttcg gctttgattt gagcaaaaga 99720
   atagcctctg agaagctggc cataactgta cggtagacac attcgcatgg cgtttccggc 99780
   cgtgcgccac cagcctaatg caaaagtcgg caacggtaaa tactcatcga agaccttgat 99840
   gaccaactcg tcgagattat aaaagcccct tagcacgata caggtatttt gaaagacctc 99900
   tggcatagta attgagaaat gataccactc tgcttgtatt ccccacgggg cttggttcac 99960
   aggcgccggg aacggtaaat gcgaaacgat cgtgaacatg gccgcataaa gattggcagc 100020
   aagttgcgaa tctaaataga gcgaatcgtt aggattgcgg aacctgactc cgtagcctat 100080
   cagtgtatgt agtacagtgc caaattgagg tacactattc caaggatcta ggcctacgaa 100140
   aatgttacca tcgtcagaaa attggcgagt gggattcgct attttttcgg ccttttgcaa 100200
   aaacttgtat tgaagagttt ttatataata attttcaaac gcatccaaat cggatatgtt 100260
   cctttcttcg gttcctaatg aagacgaaac atttgccatt gtacgggcag gtaaaggatt 100320
   cgatgaaaat tgaaatacga ataatatgac gattactaca ataatgattg cgaatagaaa 100380
   cgcccacatg atgaaatgat tagacgacac aggacaaaca caaaacaaat aaaattattt 100440
   tcttataatc ccatatttat ttatttcaat atccttctaa aataggcagt ttgtagaaaa 100500
   atttccagat gaatacgtcc atatacttga tgataggcat tcgagtccta tatttttcct 100560
   tttttaccac tatgctcgtt tcgtaatcat cacagtcgat atttcctatt ccgatgctat 100620
   tattgaaatc gttgctactg cgatgccagg gcttgacgtc tccataatag ttgatcacgt 100680
   aagcttcgct atttttacgc aaatgatgat acgatcctgc cgtccacgca tacaaaatgg 100740
   tcaaatgcgt tattgtcatg ttcgaaatcg ccaacgcttg taacagcaca ggctcgtcga 100800
   atccgttgtg gaaatgacat ttctgtaaaa atctattcgt cgggaccaaa agcgatttaa 100860
   tcgtatttaa aagcttcaac gacggctcga acagcaccgt gcccgcttta cataaaattt 100920
   tattgtgttt atactctaga tattgattga ttttacaagc gtcaattttg gtaccgaatg 100980
   gcatttttcc atagttgtcg tcggccatgt acagagctgg agtttccaaa gcgaacaaat 101040
   ggtcaatgct tttgattacg atgtgatcgg catcgaggta gagtattttg tcatagtcgg 101100
   tcaacgaaag acactgccat tttgtgaagg catgatcgat ccaaggctta tacatttgat 101160
   tctgtcgatt cgacagcatg ggcggacatg aatacgaaat aaaatcgacc aaaactactc 101220
   tatcgtagac tcgttcgaga gttttgactg cgcgatcgct cacatccctc gttaccatac 101280
   acaccaaatc gtgaacggta ttggtgtaca atatgctttt ggccaaagct aaagctcctt 101340
   tgacatattc atcgccaagc ataaccaaag tcacgtatgc atacattttg gtctttatat 101400
   ttctacctta acacaatacg tccgaaccga atcaatctaa attagcgtgc ggtccattca 101460
   tgatggaaga atcatgaatg gtcgatgcga atcgcatcgg gtcgaacgat catgaatgat 101520
   tccaccgacg taaacgccat cggtatcggc cgacgttaat ataataattc accgcaacat 101580
   tgtcgtaaat ttccatgaca ctaacgttaa taatcattta tacagtttat ataaaaattt 101640
   ttaaattttt gaattttatt gaaagtttgt tttgtgattt tgtctacgac gattatctcg 101700
   aagtcattgc caacaataaa cctgtcgttg acaattttat tgtcgacatt gtcggtgacg 101760
   acatatgtaa tggtaattgc gcatttagta aaaatctgat gcaataattt aggtaatggc 101820
   gcaataaaaa atctgatgca ataattcaag tggtggcgca ataaaaatct gatgtaataa 101880
   tttagatgat gagtagaacc aatcgattct gccgatcacg ttaataatgt ttccaaatcg 101940
   acccaaaaac gattgaaata aacttgagaa taacatcact gacataatcg ataacaaaac 102000
   tttcaaaaag tgcgcaaaaa atttttgtgt ggcgcggcgc aacaatgtgt cgatttacaa 102060
   tcaaataacg gtttttatca tcattctcga cacaaatagc ctcaacttgg ggttttataa 102120
   actctaaaaa tgttataatg tcgcggacac caaaatgaca atccgtaaat taatttttat 102180
   tctttcttca tgaaattcat ataaacgacc agtttattca tgtcgatgat atgtggatat 102240
   aaagattatt ataataatac tgataaacta gggaatataa agaaaagtaa agctgttgat 102300
   tcgttagtat accagttgcc ctcaaatcga atagtttatc cggatcaatc atggtcaacg 102360
   attttaattt cgatgttatc gtcagaaata attccgataa ttctataaat tttgtaaatt 102420
   ttgtagtcga cgacaacaac ccaaaagccg cgagcagcga tttcggtgcc gtcgataaaa 102480
   atttaagagt cgatgactca atcttcggtg tcgccgacaa caatatcgat gtaaacagca 102540
   acaatcttgg cgtcgacaat ataaaaattt ctaatcaaga ctttcaagat ataatagatt 102600
   ttatgaacaa gaatgccgaa cgaaacagag agttgcgcat gaaaccgtat aaaagcgtcg 102660
   aaaatgcgtt gaaaagtttc gaaactaaca taaatggcat gaaagatgat gtgtccaaca 102720
   tacaagaaaa aattaatagc atggaaactc aactgaaatg tatcgaagat gatgcctata 102780
   acaaacgatt ctctatgaga aacaaaaaga ctcgctatct gaataaaagg gttcgtcagc 102840
   tagaatgttt gacacggact cagctgttgg acctgatcga caaatgcatt catcctgtag 102900
   atgttcttcg attgactgaa attgttaaaa acaaattgag ataaataaaa aaattacata 102960
   tatttttgca tttttattta gacacagcat ttacaataaa ttgtttagat tgattacaat 103020
   caagaaagca aaattttaca atacaatctg gattcgaaac gaaacaacat taaacacgta 103080
   aaccaattgt ttcgaataca tcgcgacttt caatcgcttt gtagatgtcg aatccgcgca 103140
   tcttgaggac acgatccaaa taggatttga ttgtaataac aaaagatttg tgagctctct 103200
   caatgtcgtc agagtcaaac gtatcaagtt tagcgatttt gctgaaattt ttgacgatat 103260
   cgtcgagggg aacttgagtg agagcatgaa tcacatctct gagctgtttc accacactca 103320
   tccatgtctc tacattcatc ggactctgca caacaaaaga tttataattg tttaaaagca 103380
   gccgatttct ttcaaatccg atcactctac tcatcttcat cttgacatca atctcgatgt 103440
   acacggcatt ttcatattca tcatcatcat catccaaatc catatagtaa tctctgtcgt 103500
   catcgtcatc gtcgagtcta aggttttgat attcatccgg tatccagcat gtgacataat 103560
   ggaaacaagt ctctaaaaac tcattcgcga cattgtcgct atcaaaacaa tatgttggaa 103620
   taccagcaag ctcttcgtcc acttcattgc gcactcgaac aatatcaaac atgccagtct 103680
   cttcctgcat aacaatttct gccatggtcc acatggcata gtcaatcgac gaattaaact 103740
   tgtgaatggt acctctcgct gctagcagta ttacttctgt gatgatattc ggcacggaag 103800
   tcgcttcgat acacatcatg agtcgtccga tcacacaatc gaaaaatttt tttctattga 103860
   tcaagtttat ggcatagttt tctgtcccga gcccatgtat atcttcggaa gattttaaat 103920
   gttcgtcatt tttatattta acttgaaagg cgtcatgaat ttttttgaat tttaaaaatt 103980
   tgttggcaat ttgaggacca aattggttga gctcttcgtc gacgacgacg agagaattat 104040
   cgatgtgttg ttgttgttgt tgttttgaag acatgttgga taatgataga attactacat 104100
   tttcttgaca tttatactgg gatttcagtg attatcaaag ggatttcaat aattatcagt 104160
   gtgatttcaa tgattaatag agataataaa acgacaggat aggatatcaa aaataatatt 104220
   tatttgcaca ttattcagat tttatgatct ttcgtagttg cttcggagaa atctcgtagc 104280
   aatcgttttc tttgcggcag ttgacatcca atatgttatg caggatttta ttaatagttt 104340
   tttcggcaaa caaactattc ttggcgtaca ccgaataata gtgttcttta gggtttttga 104400
   tgtctttatt gatggattct atatacttta aatgcttctt ggccacatca attttatact 104460
   tgttcgcggc ttgtagtttt ctgttcgtgt agatgaaaac ggtattcagg ctcatcaggc 104520
   gacgagtcga cgacatagaa gacatggaag acaaagacga tgctgaagat atcgaagtca 104580
   atgaagtggt cgatgccatg gagaccatag aatcttcgga atgtgaactt gaaagactca 104640
   tgatgaaaca gagcgaaata gtgcactaat gtttaaattt tggtatatat cattatttat 104700
   acatatcttt aatcttcata atctgataca aacgtacgat ttatctgata taaagggctt 104760
   tatcagatac ggaggtaata taaatgtacg atgttgctgc aacgaatgca gtttatgtac 104820
   tgaacataaa acatcatgtc atacctgttg tctctcggtg gagtggcgtg cacgaccaaa 104880
   actacaattt tgagacagat cgtccaagac aaaataccta atgtcgtggt gcattttgac 104940
   gactacaagg aacttcacga tcgataccag tttgatcatc gaatgggcag cgaactgttt 105000
   gcagcatatc gcaacgccaa cgatacagtg ttcaagaacg attatatgaa tgtgcacatt 105060
   tttgatcgcc agccaatgga atcgctcgtt tattcgagtc ttcgccacaa aatcaacaaa 105120
   aatatcagct actacatgta catcaacagt gcgaacatgg gactgcacga cggctggaag 105180
   agcgtcatat tgcgcacgca cccgagcaac gatgagctgt tcgtcgaaat gatgaagaag 105240
   cgcgacaata aaatcgacat gtataccaga gggtatatac gcgagcaaaa taacaggttc 105300
   aatttgtggt ccaaagctgt tgacgctact gaactgtatg tcaatttgca gaataacgat 105360
   attaaacgat tgactcaaga acagaataac attaaaaatt atatagtcaa tcagatctat 105420
   aatgtcgaat gtttggacgg tctggtggtt tacaagttta gattgcctat aatccaggat 105480
   aggattgctc tattttctta tgattattta ctgtggaatc ttttgtcgtt taatcctcag 105540
   cgacgataca gattcgtggt cgaaaaaatg attaggcttt tggataacaa atttacacta 105600
   attttcatgt gtcatttgaa gctgaacgag gtaaacgagc atataaatta tttgtgcaag 105660
   actctaaatt tacctctaat catgatgatt gccactgaga acaacaaata ttgtgtacct 105720
   cgatcgggct gtgtagatta tttgaccaat ctaaatccta aaatcaatcg gacccaatct 105780
   cgctactacg gaaatttcaa tagtagtatg gaagaagatt atgctaccaa taatagtgta 105840
   caatttttcg atcgacttga cttttttcta aataaataaa tgaatgaata aaactccaaa 105900
   tgtttcatta ttgtgatcat tgtcgaaaat tttaattcca ttatcgtcaa ttacaattct 105960
   ataatcaata ttatcaattt tatatgtcac attatcgatt ccattgtctt caattttgta 106020
   gtggttttct attataattg aaataaaggt tttgcaattt tatacgatgg tgttattttt 106080
   cttttacgac tattcaatgc caccgttaaa agcggtgaag atgctgtaga ttttttcaga 106140
   ttttcgtctg tcgtcgactt tgtcgtcgtc tgtgacgata acgatgatgt tgcggttaaa 106200
   gtgatcggac ttttattatt atggctgtat agatcgatcg tttctataaa aatttttgtc 106260
   gcaatctctt taccaaaagt gacgagatga tgctcgttgg tgcggggatt gtccagattt 106320
   tcgaggcact cgttgtagtg ttggagcatg gctcgcggcg agctcttgag atcgggatcg 106380
   attccgtcga gtcgctttag agccacttcg aggacggctc tgtagttttt gaaatagtct 106440
   ttgcctttgt tgagaccaaa tttgaaggcg atcaacaaga gacgcctgtt gaaatcttta 106500
   tagtcgatcg aatcgtcaac atatttggtt tggctgaata attttttaat agtgtcataa 106560
   ttttttggag atggattctt aaagtattca tcgcgtgcgc ccttgacgat ggcaagaata 106620
   ttgtcgggta gcatgcgctc agattcgatc agagcgctac atttgtcggc gatgagttgc 106680
   ctagcaaagt cgtcaatgtt tatcgaatcc ataaccgcga cgagtactta ataggtagct 106740
   cacaaacaca atctatcgtt atctagtcct gaaattgagc gatccgtcga ggttaaaatt 106800
   gtagttgctc aatttaaagt ataatcgagc cacgtccatc gaaccgattg cttcgataac 106860
   gtcattcaat tcagacgaat gttgaaaact gtagacgcaa tagcacattt tgtagttgag 106920
   cagcgtggac accaacaatt cgatgagaaa ctgaaacgtt ttattttcgg tgcagtcgtt 106980
   gatgccggcg ccggcgtgta acctgataat ggcgtcgggt tcgtcttcga tgtcgttgtt 107040
   gtgtcgtctc gtgattctat taaacagcat gcccgaatag agtttgagta tcatacgatc 107100
   gagatgtccg atatcggatt gacgagaacc cgcatgcgtt ttgatgtcgc aagactcgaa 107160
   catgagtata ttgacgtaga cgatattgta cagacttatc gcgtctgccc agaatttcga 107220
   ttcgtacgaa gcgtttttca cgagattata ctgaatgata ttgctgacga tataagaatc 107280
   gttatcgcta aacaccggtt tattattctt ttcaatgaaa tacgctaccg catacagctc 107340
   gggatatttc ataaaaagta tgtcttcgta ggaagcgcta tcatcgagag cgaataggcc 107400
   gggtcgaatc tcggcatagt cgaggatcag gtcgtgcgac ttgaaagacg ttcgagtatt 107460
   atatgcatgt gtcacatcaa actgctcaat gtttaccata ttggggttga ttttctggtt 107520
   ggatgagcga attttttcta tcgtcggagc cgttgccgca gacggatggt gtacgataat 107580
   cggcttgact aacgattcgg cacttaaacg aataaaatta aacatgttta tgaaatagtt 107640
   gaagaggcat ttgagttttt caatttttag aggagtatcg gccgctttca gagccgtaaa 107700
   atcgagtcca cgaatactat cgaaaaagaa ggcatgaatc aatagaacga ggcaatgata 107760
   gcggctcata tcggtttcgt tgcgtatttc gagagaggcg atgtcagtgt tgaggaccac 107820
   ttcaaactcg acatgaaatt taaaatcttg catctcgaca tcctgtatcc tatttagcag 107880
   ttgaatgtac gcgtcgattc ccaaatagcg attcatttcc gcaataaaat caattttagg 107940
   agttttggtc ggcaacgtcg acgacaacgt cggcgctgtt atcgttgtga ttgtcgacga 108000
   tattgatgga tttttattcg gaatcgatgc ttcggtcgtt ttaatcgcgg ccgttgtcgg 108060
   cgacgatgat tgatttatta tattatttag tagatttagt gtatctttaa tgattttttc 108120
   atcttcgctc atgtaatcta tgattttatt acgaaaatca gcatcgtcga tcacgtttaa 108180
   ttttaatttc aaatcacaaa aggtctgttg atgctgttta aatctattgg aaaaatttat 108240
   aaattgttga gatgtggcat tgaaatcgtt ggcaaacgcc atagtttcgg tgaacgtgac 108300
   cacaatactg ttttgtaatg aaacatattg atcgatgata ctttttttat tcattgtgta 108360
   tcgcagagtg tataatctta tattaggaaa aaaaccaata caatagtaaa ttcacatatt 108420
   ttttatttga taaaatttga tttcgaataa caaacaatac aaatagagaa aagttatttt 108480
   atttgattaa aatcgaacgt atcaattatt taaatttcca taaacatatt gatcgagatc 108540
   gtacatggat tcggcggcag aactacaaaa aggcatggaa tttggaaggc gaggaccttt 108600
   gttttggttt tcgataaaag aagttagaaa ttcttctgca tatttgagca tgtcgtgttg 108660
   agaaaaatgt cgttcttctt cttcttcaat atcatcagca ttcagttgat aaacgtcaat 108720
   tatttgattc ctcaaaatgt ccaattcttt aggcatgatg tcgtgtagat tttccggtaa 108780
   aacaacaaca gtatcgtaga acattaaatg ctccattaga tcatctttaa aagcagacag 108840
   ctctcgatta aagttttttc ttgaatagta gttgagcgtt tcgggtagca tgtcaactat 108900
   acaatctatg agcatgtcga tcaaatggcc ggaattaata aaatgctcac cttttgaatt 108960
   gtaaacgcac aatttattat ctagagcgta gaccggatcc ggtgtgaatc ttttggccgt 109020
   ttgtccgaat tttttaacgt aaacctcaaa atgtttaaag aaatcttcaa ttagcgtatc 109080
   catgtaatta ttatcatcat aatctccttc gtccaaattc aaaatgagcc atacgccacc 109140
   gcattcgatc agacgatcca gtagctcttc aaatctgttt tggtcttcca tcgagacggg 109200
   caacgcgatt ttaaagcaat cttcatcatc gtcatcgatt tttgccgcac attcagcgca 109260
   ataaactgta gaattgagtt tattcttttc aacgactcga aatgtccaaa cgttggcttc 109320
   gtattgttga tagccatatc cacaaacggt acaaacgaaa taaaagtcat tagtgataaa 109380
   cttgtaagca aacatgttga ttagattcgc gagtgcgtcg cgagacacgt cttcggtcga 109440
   gataaacatg atttcgatat cacagagttt ttgtccgtag agtatagact gtaaaaatat 109500
   tacaagtacc ggtgtttata tatatttaca gattatagta gataacaaaa cgactagatg 109560
   atgtcatata tatttttgta gatggaaact agattgttgg gtatgcacac cggtatcgag 109620
   cgcaccaatg atgtttgatt atagtacatt cgagtgtaat tcgagtcaca gacgcaatcc 109680
   tcgacactaa aaggtctgtt gactagatct aaagagagat ttccgttgct tacgacggca 109740
   tctttataac cacaaatata gggtctcaag tcacccgaat cgttgacaat gtcacgataa 109800
   gtactgagac aagtattgta tacgagaatg tcgtcagcga ctatggcgtc gacgagtcct 109860
   cttgatctgt cgcaaatcga ttcgatcgaa tcgggacctc tgtacggtat cgaagctgta 109920
   caaaagccct cgacacaatc aaattgattg atagcaatgg cgcaattgtt agtgcattgt 109980
   cgcttcgaca cgcacggtaa tctagtatgc gaacaatcta caataccgtt ttgttcgtat 110040
   acaaactgta gtgcagattt acctgtattt ttattggctc tctctacatt ttgtattttg 110100
   ttttcagtca tcatgtttat agttttcaat aaataaaaac atactattag taaagcaatc 110160
   aaaattatta ttatacccag cgaaattgat gaaaaggacg acatgattgt tgcctacagg 110220
   cttatttgaa aataaaacga tttatattta taatatagag tttattacaa atcaattcga 110280
   taaaatcacg taaccattgt tcaatccatg attaaacagg ctgagcacag tattgaaaca 110340
   ttcgtcgcat ttagtcgaat cgtacagctt aaaaagccaa gcttcgcaaa cgctgcatcg 110400
   agtctggcca tccagatccg gcgtgtcgaa tccgtgtgat atcgaaaata attgatggtt 110460
   agatttcaat atgacgttgg atagatcttc agcggtaaac tttgaagcgc aattgataca 110520
   gtaggtagcg tctttaaaaa tgacgcattt catcacgaca catttgtacg tgtcggcaat 110580
   gttaatagta atctcatcga catcaactcc ttggtttacg acatcaattt tcgagttgtc 110640
   cgccatgaca aaggtgcgat ggtgtgggaa tacagccgct aacgtactga tgctcgagcg 110700
   acaaacgtat cagcatttat agtcgtctcg acctaatgat aaggcaaaat ttaaatttat 110760
   cgcaacttta tcgacattat cataatcata atcatcggca tgtctggcga agaggatcag 110820
   aatatttttc ttgttatacg cgaagacata aaaaatgtca gtattcaagt tcaagatgtt 110880
   gacgataagg ctagcgattc taatgctagt ctagtagaaa tagaaaactc tataaaagct 110940
   tttccattcg attcgttggc tgctcaattg aatgtgatca acaccggtgt acaaacgaca 111000
   aacaccagta taagtcaggc taatgcgcaa ttaaatacac ttcaaaagaa cgtcgacgat 111060
   aagctaacca gtctaggtac ttcggtgcag ggagtgagcg caaaagtaga cgccggagtt 111120
   gctagtctcg cttctcaatt cactgccacc aacactaaaa tagacggtgt cggcactaaa 111180
   gtagattctg gtgttcaaac cacttcagct caattgacag cctttcaaaa ggatacaaat 111240
   tctaaaattg atactctcgg cactagtgtc gcatccatat cgacacaatt gacagctttt 111300
   cagaatagta caaaagctag tttagacaat attacggctt tgctgaatgc cataatcaaa 111360
   attcttaaac ctttaggact gttgtagtaa tgggcatgga tacgtattaa aagcatcaag 111420
   attattgtgt taaaaattta atagaaatca aaaatacata aaagtaaaaa actattatta 111480
   attatagtaa aatgaaaata aaatcaagaa aaacattaaa gtcaaaatta tataataaaa 111540
   atcaatatag ggttaaaatt attagaaaaa aacaatacat taaaaattaa ttgatttaag 111600
   ttgtgtctgc tgacataaag aatcgaaatg tagtttaatt ctttttacag ctctactttc 111660
   gggtttatac atgtgttcta accaccaatt ggatattttg ttgatgtgac gcttgcgttc 111720
   acaacgcaaa acctgcgtgt taccaggcca caacactatc gaaccgtcaa tagactttat 111780
   ttcgaatgta gtagcattgt tggtgaatag tttctctctc gggatggtgt gcacatgagc 111840
   agcaaattta tgcatgttac aatattcctg tataaagtca tcggggaata tgttttcaaa 111900
   gaaaagcttc acaattaaag ggtcgtggtc ttcgttttct tcatcttcaa tctgttgttt 111960
   attagcacat aatgtatcca aaaccgtatc gaatgtgctg tgatagcggt tcaaaaaagt 112020
   cttggtcaat acatactcgt atgtaaagtt tttattatcg aaaatatgaa tccaatttat 112080
   tttgtagttg atgtcatcat ataggcccgt gaataacacc cttttgttta gcgtataaat 112140
   gttcaataaa tcgataaact tccataattc tggatattta ttaatgtttc cgatgacatg 112200
   ttttctcgtt tgatttttgg tacgatcgac aaaagtaaag ctattattgt gttttgatgc 112260
   gtacgattta atagcatcgg tcaacaacat ggcaggccaa aaattattac acggatcgat 112320
   ggtgacccct tcattattat tttgatacca tttatgtgcg cactcgtcta cagatccttt 112380
   gtcgcaataa aacgtgacct cgtttctatt gatccatttg ggtagattgg tcataaattt 112440
   ctcgacaaaa aaatcgtaag acacatcaaa cttgatttgt tcgatcgttt ccttgagacc 112500
   gggcgtggta aagctgtcca cgtctccatt gtacacgagc cactgttcga cttcattatt 112560
   atcgggatat ttagaagcga ccgcttggta tattagacgt tcgtattcgt cgacgatggg 112620
   attttcatga ataccggtca aaaatctgag aaccttatgg ccatagtatg tgattttgtc 112680
   gcccaaattc aattcctcaa tataattata tagttccata gattcctcgt cgtcttcttt 112740
   tatggtatcg aaaatcagat aattctcatc gagatatttc cagatttgca tgtcagtttt 112800
   tgtagtatct ttaaacagta atttaaattc ttcgaatgat tcgagctctg tcaagttttt 112860
   ttccgaaaga tattgctgct tttttctttc gtgatcaaca ttttccattg ctctatctaa 112920
   taaaggaaaa tcttcgggag gtgcatctaa caattctcca tgatatctat cgaatctttc 112980
   atcatgattc cgatttctat gccatttttt ttctttttga tcgaattcac gggctaaatt 113040
   gggttgaact gccgtcggtt cgtacaactc tactgttgta aattctttct ccagattcat 113100
   gtataataaa taataactgg tgatatagat gccatacctt tttggctcag tcgattcttt 113160
   ctcgacatcg tggtaattgt accgattgat acgtaaaaac gcatttccgt tacaatccgt 113220
   gatgatgtct tctacatata ctttttcgat ttcacacgaa catgacggca caaacacttt 113280
   cagattcttg gcgttgaacg cttcgacctt gtaaatttta ttacaacaca acatagacac 113340
   gaccactcga tggatccaat gtcccccgac attatcgcga ttagcccgtt gaaatttggc 113400
   aatttcggtt atttgctcca tagtcgagtc gcttgacagg gacaataaac atttttagct 113460
   cgcaatcagc tttatataaa ttgataaata tcacatttca cagattagat cagattacgg 113520
   ctaagtacta aagatgctat caactttaga cgcaattatg ttacctgacg agattagata 113580
   ataaagtggg acgacagatt agatgatagt aaagtgagcc agattagtta ttaattttgt 113640
   atattaaata aagtaaaata atatagtata atattaaact cattttactc gacgattggg 113700
   attaaattaa acagaattaa tttttttcta agtgttttat ttgcacatta caaaaactat 113760
   gtcaatcatt tattctatag ccaaggtatt tgaagacatg ggattctcat ttggtggagc 113820
   aacgttgaaa tatctaatat tgaacacgtt tttaacaaaa aagttttgac aattgttcat 113880
   ggtgtgcacc acctgttcag gcgaataagt ttcccctgcg ttagagtgtc gacgctgagg 113940
   ggtatcaggc actaaatcct ttgacattcc ttcgactatt ccctcgacta tcgcgttaat 114000
   acgttcctta gcctcaatgc ttggcgtgag acatttagac acgcaatcat cttcgctagt 114060
   aaagtccaac gccttaaact cttcgatcat cttcgagtgt atgttcttgt tacgtttaca 114120
   catttccatg tcagctttat acttatcgcg aagctcaatt tcatttaaaa cctccatttc 114180
   ggttctcagt ttgttggtgt atcgaaggcc gtaaaatatg tgcggctgat ctgtgcgcac 114240
   cttgagccac acagtcacgg gattaggaca cttgagctgt aaaaactttt tagagtcttt 114300
   gagccaggcg taccgacgcg aattggttag tttgggtcgc ttggcgttta cttcgggcaa 114360
   tgtggcttct tcctggtatc gttgaatggc tctgtcctgt tgttttatct cactgtattg 114420
   gtttcgacct atacgaatgc gttttctgcc gtcgacggtg cgctcgtagc cagtgatgta 114480
   ctcttccttg tcgggtcgct cggtaagcgc aggaactact cttccgctga tgctagtcag 114540
   tgtttgacga agcttttcat tctcatcgat gttgtccttc gccaacaaag cattgactgc 114600
   aaattgagtc atggacatgt tggcttgagt ggccatatcc ttcatgagca gctgcatttt 114660
   gtattcgcgc tctttgtact cgctcatttg attttcgtaa tggcgcttca tctccgaaat 114720
   ggaggcgtcg tacttggcaa tcgtagtgtt tgcctgcgca atcgtggtgt ttgcctgcgc 114780
   aatcgtggtg tttgcctgcg acagctccaa ctttaattgt agcgattcca tttgagcttc 114840
   tgccaattgt ttgtcataat tcacgacatt ggctgtcgtc ggaatccagt tttcctcaat 114900
   tccggcaaca cttcttcgaa tagccagcgt tgaaattctt ccgcagcagg tagtttagac 114960
   ttcataatca aagcgtaaac gcctgcctca ctgatgaata ctgtgtttgg ctgccagttt 115020
   gcaggtaatt gcgattcggc tgatgtcgtt tcatctttag tttgcttcgt ttcaaggggg 115080
   tcctgattta ggattctttg agatgaccgc gaagtcgtag gtatttgacc gttagtcgat 115140
   gccgttgcaa gagagttctg ttttaaaacc tcctttatct cttgccaggt ccgacgccat 115200
   tgaggtttta tatggtcata taaagctttt ccgggtcttt tataccccaa aaattgagca 115260
   atggcatgac cgccgtacat aaatacatct ttctcgactt cgacaatcca cacatcgcaa 115320
   gcgacgctgc cgatcttgca ttttttgttg acgagagaca tgatttggat acgactcgaa 115380
   tatgacgtta gatgcaatcc gacgataaac taatcttatc ggcacgaaac ttgacgaccg 115440
   aatgattaaa tctttgacgc gtaatcgatt tttatagtgc cgtcgaggcg tgatctcgcg 115500
   acttttttgt aagtataata ataatgaaag ccatttgtgt tatccgcggt gacgtgaccg 115560
   gacaagtatt ctttttacaa gagagtcccg agcatctact aaagatcacg ggctacattt 115620
   tgaacctacc caaaggctta cacggatttc atgttcacga attcggcgac acgagcaacg 115680
   gctgcacgtc tgcaggcgag cattttaacc ctacgaacat ggctcacggt gcgcccaact 115740
   ctgccgttcg tcacgtcggt gatcttggca atattgatgc taaaactcac cattctttga 115800
   ctagtatcga taaaatagat tcggtaatgt ctttgtacgg accccactcg atattgggca 115860
   gaagtctagt ggttcatact catcgtgacg atctcggcct gaccgatcat cctttgagta 115920
   aaactacagg caactcggga ggaagattgg gatgcggaat cattggcgta tctcgataaa 115980
   aataattaca cacaaaattt ctcaaacaaa aaatttatta cctccaccaa ataataatga 116040
   taataaagtg aacaaaatta tagttacagg aaatttttac aaaaaatcgt ccatatcact 116100
   atgttcgctt tcataaaact gaagttcgct agattcatcg gagtcagagt ccaagtccat 116160
   agaaaagaaa ctacaagtat aatgattaca atatctgcaa cgtatagaag gcaaatcgtt 116220
   atatctgagg cagcttaggc attttaaatc gtaagcaaag ggtgacaaaa agaaacgact 116280
   gtacaactca atcaactgca cggccatgta atgaggaata tatctagaaa atttagtaac 116340
   gaattcttgt ctaaacgcta cccgttcttc gatgttgtgc atatttatct ttttcttgat 116400
   agaaaaaaaa attttcttgg cacacatcaa cgacaagctg tcttctttgc gctgacgaca 116460
   acgaatatac cgatcacaag ctttagaatt catttcaatg tcccatcgcc agtttaaaac 116520
   gtttatttgc ttgaacattc taaacacatt ttcgtaaatc gggcgtggta aatagggctt 116580
   catcacatct tgaaactttt gttctacttc ttcgagcatc aagtcatcgg gaatatactt 116640
   ttcatagtat tctgtaaaca cactattatt taacgtattc aacaaattct gtgcgcacaa 116700
   gagctgcaga gaagatacgt tttggtgtcc acgatccatg gtggttgaca gacttgttat 116760
   aatgctttct ggcgacgaga cccaatattt atattataat ttatctaatt ttatctaatc 116820
   tatacaataa aacaatcaaa tttatcagga aaaaagttta tttaatcaat aatttttata 116880
   cttgtatccg ggagcatatg aaaagctggt agatttttga aaagccatat ccttactcga 116940
   agacgtggag tagcggcaat tgcaattcca cgatttgttg tccaggcacc gtctacattt 117000
   ttcattgtag ttactattaa agttgttgaa gaaaatcact ctggtcgccc acgaagtcat 117060
   cgaaaatgaa gtttcttccg gtttcttcat agtgccggtg gagctgctgg tgctgggctt 117120
   gttagagccg gtggcgtacg agcctttgcc agtcttgcaa atgaggcaca tgattgagag 117180
   ttatgattgc aactgcactg tttgcaactg tatgcattta gctattcagt atgcgctttt 117240
   atagtgtgaa aaaagattag ataacgagat aacacgagat aacacgagat aactagtaga 117300
   ttagcatttc ctcaacatta aagttttatt agttgaagat ggaatcgaat gcaaaaaatt 117360
   ttgcccaata tctcaagtga ataaaataat taaattatca ggcgtcaact acaaaattaa 117420
   atgtttagcg aaatattgag aatcatcagg cgttcgccca aattatcgat tgattgtttg 117480
   ctacaataaa attatcagga accaaggtaa agagtaaaat aaaacataat ttaaataata 117540
   aaagttttta tttagatggg tacaaatttt attttagtca caaacatatc taagaggaat 117600
   tttatagtgc ccggatagac attttttgtt attaattttg aatccacaga actttttttg 117660
   acggcaaaaa tatatgtagt tctacagatg gggcatgaat tatgattttg agaccaaatc 117720
   ttgagacatt ttgtacaaaa cacgtgctca cagttgttgg gatgtgaata gcttcgacag 117780
   cctcgtcgac gaatcttttg cacacaaata gcacactcgt cgttcatttt cagcacaaca 117840
   tcgcggacga tcgacggttt ttcaattata atatcgctga cttgcaggac atcgaacaag 117900
   attctgttat gtttgtcaaa actaatgttt aaatatttct catgtttctt gtctacgaat 117960
   tgagcagaat caattatttt cgttcttcgt ttaaccacat atccgttggg gttcaaaaaa 118020
   attatatagt caaaattacc aatcttagcc acaaaaccca cgttagacat ggtgactttt 118080
   taatgtacaa atgaatcgat cgttaaagag atcgctttaa atagtgtatc gagatttatt 118140
   tatcggtaat catgtatact acacacacat aaagaataca acaaaatata ggaaaaaaat 118200
   atttattatt tattaaaaac atttcattgg ccggtagagc cgaaaccgcc gctgcccctg 118260
   tcggtgtcgt cgagctcgtc gaccatgatc acgttgggtg tatgatagga ctcgatgatc 118320
   atctgcgcaa tcttgtcgcc tttcttgaaa ttatactctt ttttgcttct attgatgaga 118380
   caaacaccaa cggggccgcg atagtctgtg tcgattacgc ccgcttggat gccgataccg 118440
   tgcttggttg ccaggccaga acgctcagcg atacgaccat atcgtttcgg aggcaattga 118500
   atggcgattc cgatatcgac aactgtccat ttttgaggtt tcagacacac atcggtggga 118560
   gtgtagagat cgtagccggc ggagcccaga gtcgcacgtt gaggagcaat cgcatcttta 118620
   tttagcttct taaagtacat attctcgtgt tgttcttgat gatagtattc gttgtcggaa 118680
   caatcttcaa tgctcaatct cgatcgctta ttggcggtag acatgtttat ttattatttt 118740
   cgcagagagg tgtggtgtga gtgtcgtcgt aagttactat aaatatgact gactgacgac 118800
   cggactctgt ttatatacca agtgaatcga taaatattaa tgatatcgta atatgtcgtc 118860
   attgacgaga tcaactgatt tttagtcatc gtatgttcag gacgttgacg gtacaataaa 118920
   atattttact cgtcgagtcg agagtatcta tcgttcaata agttatttat aaaattaacc 118980
   actattcgtt acgatataat gtcgttgctt tctagaaaat accaagatgt cgacattgcg 119040
   gcatatctcg atcaatcgta tgtgctgtgg gtcagtgccg atgacgtttt acagattttg 119100
   aggttgcccg tgtccgttct tcagaccata gctccgcgac acaaaaaatg ctggacagat 119160
   tttcgatgtc ccactcagtg cagattcgac gcgagcaaag tttttgtaga tctctacggt 119220
   cttgggaacc tatgcaatcg tgtcaattcg agctgttccg attatttgat gactctattc 119280
   gtggccgaag tctatagaga ttgcttgcct cgtcgacgat catcgggatg tcggccgcgc 119340
   agacgatcgt ccggaggtcg cagacgctca tcgggatgcc gacgaaggtc ctcaggcgga 119400
   cgaaggcgtt catccggcgg tcgtaggcgt tcgtctggag gtcgtaggcg ttcgtcttgt 119460
   tgcagacctc ctcaccacca ttatcatcac catcataacg agttactcga gcgtatttcg 119520
   cgacaaaacg agattctgct caccacgctc aaccaactca gtctaaacaa tagtaaccaa 119580
   catctcgagc tgaccaacat tctaaacgcg attcgcctgc aaaatgtggc catagctgct 119640
   caattaacgc aagtcataga aactctcgac agtcagttgg gcgaattgac gacggattta 119700
   agacgattat tgaatgagct cgacgctcgt ttcgctgctc tcacaacggc actcaccaat 119760
   gccatcgctc aacttcaaga ctcgatcaga gccgaactgg tcggtatcaa ttcgatactc 119820
   aacaatctca cgtccagcgt caccaatatt aacgctactc tcaacaatct attacaggcc 119880
   gtaaacggat taaacattga aggtctcacg acactactca ataccatttt agctttgatc 119940
   gaggagattc tagggatatt gacacctgaa attcctttaa ataaaagatc ctaaatatat 120000
   gggaataaaa aacataaatt atttttaaca aaaattttat ttgagcatac taaaaatctt 120060
   cgagaatttc atatgtttcg ttggccgctc cgaaccgctt gtactcgccc acacgttttt 120120
   caaagaaatt agtcttgccc tcaagagata tgttattcat aaactcgaaa ggatttgcag 120180
   tgttgaaatg cttgtcgtag cccaactcga acaacagtcg atctgccaca aactcgatgt 120240
   attcgcacat ggattgcgaa ttcatgccca acatagcgac cggtaaagca ttggtaaaga 120300
   aatccttttc aatgtcgacc gcttctacaa acatactcaa caccttggtc ttggagagct 120360
   tgttctcttc gggaatatat ttattgtagt atagacaagc aaagtcggtg tgtagtcctt 120420
   cgtctctgga aatgagttcg ttgctgtaag tcaagccggg tagagctcct tttttcgagt 120480
   ctttgatata gtagattgca gcaaaactac cagaaaaaaa gacaccttcg acgatagcaa 120540
   aagcgaccat gcgttcggcg aatgtggcag attgattttc gatccatttt tgagcccaag 120600
   ccgccttctg cttgacacta tctatatgct cgatcgcatt caaaatcctg tcgcattctt 120660
   ggggatcttt aatgtattcg acgacaagaa tgttatacat ttccgtgtga acacactcaa 120720
   tgaatgtctg ttgcgcatag aaaaaactag cttccaattc gggcacggcg gattgcatgt 120780
   gctcgatgat gttagtgttc acgatgacgt cggcggcagc gaaaaacgcg agaacgtgct 120840
   tgagaaaaaa tcgttcttta tcgttcagtt tcttgttcca atcgtcaaag tccttggaca 120900
   tgtcgatctc ttcgactttc cagtgacaat cgaccgcttt cttgtacgat tgccagagat 120960
   cttcattcac gatgggcaat aaagtcttac gcgaaacgga cgacatcgtc agtgagtaag 121020
   aagttttctt cgatacgaga cgagtgcttg tgaacaagtg ccgctcgagc aaatgtcaaa 121080
   gtgataatac aaatttaacg atcgttctcg actcttttat acaatcatga tcgaagatga 121140
   taactatgct ctacccgtgc tctccttcga taacgaggct tacaaagtgt cgtggcaaga 121200
   gatatcgttc gatccgtaca gacaagagta tttgtattct atacgagtag aagagctcga 121260
   agttcgttac gtctattggg acgataacgc gcgtcgaatg aatctgctga gcaccggtca 121320
   gtttaagatt cgcatcggaa acgtgtactc cgaaccggag acggtcgagt ttaatgccga 121380
   cgagcaatta ttgaaattga ctgtttattc gccacagaaa aattttaatt cgatcaccgt 121440
   gcacttgaat aaaagatatt ttgtcgacga tcgcagaccg tggcaagttc cacgttggat 121500
   caaacgtgtc tttggtccgt tataagaaat atctaaatta ctttaagcgc aatgaacgaa 121560
   tacagtacat catttcgagc gtaccgagaa ttgttgagca tcaaacgtgc tttgtggctg 121620
   tacgtgtcgt tggtggtttg cggctttgcc attttcgtcg gtttgagtgt ttacgccggc 121680
   ggagtcattt acgcccaaat tgaagacatt tctcaaactg ttggcgcgtc agcccgcagc 121740
   caaaaagaac tacaaagcct cattatagaa tctttcgaca acaatctcga ggccgacata 121800
   caattttctt taataattcg ctcgagtgcc tcggttcaac tgaagatcaa cacttatcca 121860
   gcacgaagat cgataacgtt ttctgaagaa cgagtgacct gtcccggtag tgtccaatat 121920
   gaaaactaca taaaaacact atacgatcga gacactaaac ccgatataca taacatttat 121980
   ctgttctatg gaggccatgg actgggcaaa tcgtttgcat ctcagcaatt gggaaaagcc 122040
   ttgagtaaat ttggaaatgt ggtggtcgtg tcagtaccga tgaccagttt cgtaggagtg 122100
   gtcggcttca acacgatagg caatgtcata caatctatag aatccgtctg tcacggttgc 122160
   tatgtcatat ggatgtttga cgaattggat tcgtatttga tagataatcg cgattcttta 122220
   taccagagca aaagcatcac agaatttgcc gagtacacgg gctttgtcaa gaatcataat 122280
   cgaatcctag cctttacaat gaacaatggt gagattctta agcatgacta ttggacgcat 122340
   caagatgaaa tcgtcaacgg cacatacgaa tacgaacgtg actttagaaa ggctctcgtc 122400
   aaaaccggca tgagttcgac tgattttctt atggacggac agctgagcag actgcgatcg 122460
   ttcgtcggca acaaaatatt taaatttaaa cgattcaata gagagacggc aaagcgattc 122520
   atcaacatgt atctacctga ccataagcag ctatcgtgga acagtcaaat cgaaaataaa 122580
   ctgttcggta acgaaacaac tcagacatat gatgtgagaa cgctggtgat cgctctcgat 122640
   gatgtactaa atttaaagaa aataaataaa acttaacaca ttttataaaa aattttattt 122700
   cattcctcta gattgtataa ttcaaattga acaaatcgtt taaaatacac ataaatctgt 122760
   accaacattt ggtaaaataa ttattttccc acaaatgata ggggaagttg acctgatgtg 122820
   taatatcatc aatattatta actagtctat cgtttagata taaatgtata atgagattcg 122880
   agcccaatgt cggattcaag agatacaaca agtttttgag attcttgatt tcaaagacag 122940
   cattcgagtt ttttatataa taaaaatttt gtagttgcat acgactattg acaggataat 123000
   atttttttcc tagcagtttc ctaagtttta tgattctatt ctttattttg taatcttgat 123060
   agttgaacac gttaaacatt agacattcga tctgatcggc aatgacatta tcgatatatt 123120
   cgacggtaat attcgtatcg taaagcggct tgatgagata cgataaaggt ttgttcatag 123180
   ccgaattcat ggcactataa ttttttatag tcaaatcgag aaaatagaca tcaaacacga 123240
   aatagttcaa ttccttgatg tccgatcgtc ggtataaacg catcgatgac tgcgaagtgg 123300
   cgaccatgtg cacgttaaat gagtatcgca ctaaaaaata ttcttcgttc actttcgaaa 123360
   cggtggtctt gaaaggcatc cggtgattca accgcaattc gacgtcggcc acattgaatt 123420
   cgacggcttc gttgacgtac gatttgaaaa taattacaga tttttgacag tcgtctgtgc 123480
   tgtcgtcgtt gtcatcgtca tccatattat acgtttgttt ggtgtcgttg gccgcatcct 123540
   tgatgagttt tttgatgtca aattctcgta aaattttctc gattacacca aaatattttt 123600
   gtgaaatttt tcgtaaattt ttttcgaggc tagttaacgc cacaaatgac gacaaattgt 123660
   cgatattgac tcgatgattg aagtcgacgt cgtcgacgta aatctcgatg tcgatgtcgg 123720
   cgagaaacgg gtttatttcc tccaaatgtg cggcaatggc agcgccgcct ttcacgacgg 123780
   taaatgtttt cagagaactg tatatttttt ctaaaatttc ttgatgaaat ttcttcgccg 123840
   gtcgattgtc gtctaaaaag ccgccgtcga ctgctttatt gttgttttcg tcgactacga 123900
   cggtcgggct atcgatacga ttcaattttt gaagtactct gtcgctgaga atgtcgttga 123960
   cgagtttttt tgaaatgtaa gggatggttc tatggtagtg aaatcgttcg agtatagact 124020
   ccataatgaa atcaaataac gatcgtatta acgtgttaaa acagcatcgg cagcttacta 124080
   tgccctcgtt aatgcgtatt attaattcga tcgatacgtc taagcgcgac ggacgtcaac 124140
   tattttataa catttgttat aacatcataa ccagaatctt gagtagatcg actgcaatcg 124200
   aaaccatcaa aacgatcgtt tcgatcatta tagaaactga aaaaactatt ttcaaccaga 124260
   gtaaagtttt aaactacatc agaaactttt tgatcgttaa cagtgatggt cataatcttc 124320
   agtgtactat taatttaaaa ttactagatt atattttaaa tagatataac atcgaagaag 124380
   aaaaagaaga acaaaacgat tatcgtcatc aataaaaatg tcatcgctgc tcaccgccga 124440
   acagaaagag atttacgaca aatattgcta tgtcaattat gtggccatgc taaaacacga 124500
   cgacatgaag ctgacgcaaa acgaaatcct gagagtggaa aaaatgactc gtggacaatc 124560
   tgaaaatccc ctctggaggc tgcttcgact caatcgacag accgcttcga acagcaacag 124620
   caccagcata atgatttcgc ccagtgcagc aatgtgtcat ggtatcaaag aagaaaaagt 124680
   agtaaaggac aataaaaatt taatgaatga aattcgtagg ttaatcgaaa taactttaaa 124740
   cacgattgtc acaaaagaga ttttcgactg tggcatgttt ctctccaagt taggtctgca 124800
   tgccgcctct ccagacgcat actatgttac gatcgacaat gtctatgtgc ccatcgaaat 124860
   caagtgtcca tacacgtaca aagacttaac atacgaagat gtacgaaaat ctttacgaaa 124920
   aaatccaaac tcgaaaaaag aacgatatag aatcaaaaat actgcattgt cggtcaacgt 124980
   ggaaggcgtc ccgctgttta ttgtggaaaa aaccgatccc cattatcgcc aaatgcaacg 125040
   acaaatgtat gtcatgaatg ctcccatctg tgtgtatcta gtaaaattta aagattcgtt 125100
   tgtcgcttgt acagtctatc gagattctac ttcatacgat gcagagtcca gagacgaaag 125160
   aaaaattttc aacatgttta tcaatcgaaa caagagaaat ctaatgctaa ccgatcagta 125220
   caagcgaaaa gcgagcttcg aaggcaaaca tctaccgcaa tacagcgagc tcgatattcg 125280
   agcgttgacc agaagcggac tctatttcga ttatggacaa ctcgtctgta tattttgcca 125340
   gatttgcttc gatgtcgaca caccggccaa ggaaattctg gcaaaacata cagattgcgc 125400
   gtcgaatagc tacaacggca tcgactttca cgtcgaacat cgagcttata tcaataactc 125460
   gactcgctat aagactttac catacgaaca caggcacttg gccagtgacg gcttgtttct 125520
   tgatcccatc gattctcaat ataaaatgtt ttgttgtggc ctaataatag aggactttaa 125580
   tgttaaacat gtcgtcgaat gtgaatatag tatgataatt gatgataaat aaaatacatt 125640
   aataaatttt acacaatcgt atttattact ttttccttta ccacatgtac agtatagagc 125700
   ataacataaa tagttaacaa cagtagaata caacaagagg agagtttaaa catttcttgg 125760
   ccatcggacc attcgagaaa tctgtacact ggatttcctg tgtaatagtt ataaaacact 125820
   aaaaagaatc catagaatag tgtaaacaac agcggctgat atacatgttt caggcggacg 125880
   ggcactgacg aaatacaaac ttcaaagagc accacgagcg aattacaaaa atgtatcaca 125940
   ttgttaacgg gcgaactaat gttattgtga tatttataga ctacagtcac tataaaatat 126000
   attgtactag tcagcagatt cgacgaacag gccacattaa agaaaaacca ttgtacgttt 126060
   acgtaccaag gcaatctata actatattcg gcttcggtga atcgtccaat aaatgtggtc 126120
   atagtgccga cgacaaacat gaccaacaat gagatcatcg accaatgtga atagtacaga 126180
   aaaaattccg ctttatctat agctacataa tatcccgacg cgattagcaa cgacatgatc 126240
   aatacggatg ccgttctcgt ggtcagcata atcagttgcg aagaatcgcg cggagaaacc 126300
   aaacagcaag agctaaagcg atctgtaatg tctccgcccc agatcatgtt ctgttgtcgc 126360
   tgccgtttgc cgcccattaa atggatgctg tccattctca accgaatgag actgtatgta 126420
   ttaaacgtaa aaaattccac ttaattgtga ctcgatgttc acgattcgta ccgttttata 126480
   ccacagttcg taggtgtcgt ccattaaaac gattaaaaaa aatctatata agtattattt 126540
   aacataaagt gtgggtgtcg acatcgacaa cgaccttggc accgaaacct atataattcg 126600
   aacggctgcc gacgaccgag acgagtcgca ttcgagcgaa cggacatacg acatcattta 126660
   gcatgtcaaa ttaccgcaaa atgtggaaat cattattagt gtcgttgctt ttgtcgatga 126720
   cggtggcaga gatcgttcat agcgcgccag cgccaatgaa cacgactttg atatccatcg 126780
   atgtggacga tcacgacggc ggcggcggcg gcgacgtcgg cggcggttta ccgttaaagg 126840
   ccggtaccga aagattggta cagtttttta tcaatcacaa gtatctgcac atgcatgccg 126900
   atggcgtgat aaacggctca ctcgacagat ttaataatga gacagtgtgg agaagaatag 126960
   cggtaccaat accagaggaa actacggaaa aaactactgg tattttgatc caatcggcta 127020
   gcaattgccg ttatctgtgt ataaacgagt gtggctacat gtacagtgcc gaaataccca 127080
   ctttcgagtg tattttacaa gaaaaaatcg aagaaagtcg atatcattta atctataagg 127140
   attttaatag acgtcatgcg tacttgtcgc tgaatttaga aggaaaattc cgtcgaagcg 127200
   tattcaaaag caaggaaccg atcggcgatg ccataatacg cacaaacgtg gccatcgaaa 127260
   cttggactgg tgaaaaaatt tcgaatacgt gcggtgagtt gattcgtagc aaactcaatt 127320
   atcgcattcg aaagacatgt cgaagtgcat taaaaaaaag actgatcagc aacgaaattc 127380
   cggccatagg agacaaagtt ggcatcgacg acatcataag cgagatgatt aacgacaaca 127440
   gcactctaga ttctgttgct gttgcgatgc cgccgcctcc gccgataccc actctagaaa 127500
   ttatgaatag tactgtcgag gtggcaacaa taaattcttc gagtgttgcc tcgacgaatg 127560
   cgactaatgc ggtggcggtt gtggattatg tcgacgacgg tgacgacgac gacgacgaca 127620
   gtgaagatgt cgaaatcact atcgaaggcg tcgacccgaa aaatttttat gtgccggatt 127680
   cgtccgattc gacgctgagc attcatttgc taaactcgac tacagctcgg cctccgacta 127740
   ctcctgttcg tcgtcgccag cccgtgatcg ccatcgaaga cataatcgat agtatgatca 127800
   agattcctgt cgaaaccggc ggaacatcgg acgtttcgga tgcacgaaat gttcttttta 127860
   aatatgaaaa taatttctat atcaataaat gtataaacat gtaaatctgt atattgtaga 127920
   tgtatttcaa ttggattgta ataaaggctt tgaaatcaaa tttttattcg tattacattt 127980
   attcgtagcc aaactatact gagtaattac tataggtatt gagtatttga gctaaattat 128040
   tatactggct ttcgggcacc ataccgattg catcgcaaat gtgtatagtt gtagcatttt 128100
   tagtttcaag cacaccaaat actgcgctga ccattaatgc cacaggaaat tggcctctag 128160
   gatatcgagt ggcaggactc caaattttaa tccatcctcc ttctctgcta aaatagcacc 128220
   aagagtttgt aaatgtttct gcggtgccga cttgaacagt gccgtgtctg cgtatacaaa 128280
   gatcgggttg atgtacgatg catctgcctc tacccggata gaagcatggt tgagtcgttc 128340
   gtctggctaa ctttaaaaac ctaacatata tactgtcctt tagagatgag tcgacaaaga 128400
   tttcatgtgg actgtaagcg atcgaaaatt ttattgtaga aagaatcgaa tttggaatcg 128460
   gctgtagcag cctatggtat ctgacatgac taagattatg cctggtaacg tgagcaaaaa 128520
   tatcgtcatc cgacagtaaa ttgtcgcttc ttccccaaaa aaatttatat tgtatttcat 128580
   gtaacgcatc gatgggaata ttaaacggct gtatgcacga gttgacaata gaaatattgt 128640
   ctctcctatt caaacgagac tgtctcagca tactcgctcc gccggacgct attccgaaga 128700
   gattgtcttc gatgggacaa ttacaaaaac tcaattcggt tccgtcttga tcatttttaa 128760
   agtactggag tcgtccagtc gtacgcctgc ctgtgatcgg atctattgaa caaggatcga 128820
   caacacaaat ggaagtgagt cgtagtgtct gtcgatatat cggatcgaga ccaggatgtt 128880
   cgatcgaaac aaatccgtct cgacacgggg cctgctcaaa aaaagtccta taatcggtat 128940
   ctcgcacacg catcggtcgg caaaatggag tctgtgtctg agtatctaac tctgatatga 129000
   atccttcgtc gcagacgcat ctcattggcg attcgttaat gtttagaata cgtccgttcg 129060
   gttgacatcc tacaggcacg ttacaatcag aatagagatt cagctgagtg actagacccg 129120
   gcgacgtgca gttgcagaga agcgaatagc caatttcgtt tctagtcaaa atccaaacgc 129180
   ccgtgttggg attgcaggca cgagcccgtt ctctattgag cgccatgcaa tacgattcgc 129240
   ccggctgtat cgtatactcg atgtcgttca tagtgatttt agtaggttcg tcaaaatatt 129300
   gacagttggc caaaccttca cgacaaatat cacaatcttg atgtgaagtg catggcgtca 129360
   attgagagtg acaagacact ggattatctt cgagtataat ctcggaagga ggttctatta 129420
   gaggaacatt cgagttatcg aagcgttcga gaggatacag gacaggctcg tccgcataca 129480
   ccaatacaga tatataattt gtcaagagta atacaattag aattagtatg atgatagcta 129540
   atattaaata cattttgttg acttacgaat atataaaaat aaaaaaattg taacgtttat 129600
   taatattttt tattgtaatc acaaggaaaa tagacggcag gaggacaaag gttcctaatg 129660
   tttaaatccg tttcaagctc tatacgcaaa gcgttatatg tagaccagct ccttgtgtga 129720
   tgatatgtgc catcgttaaa ctcaacgtgt ctgataaaat actttcttct cacaatcttc 129780
   atgtataata ttttcttgca ccatttagca tgtagaattc cgatcagtcc gttggctata 129840
   ttatgctcgt gggcaggcct catgaacgtg tataaagccc atacgttcct cgaccactcg 129900
   tgcgaagcca gaaaatcgta tctaagcttg gtaatagcac gtcttgttat acgcataccc 129960
   tctttgttac agatatggat ctcgattgcc actattctgt tgaatcgttt ctggtgaaac 130020
   gtaaacagct cgtggccatt ttccaagatc gaaacacttt ccttgttgta gttatgaatc 130080
   gaatacaatt ggtcgatttc aaaaattgaa ggcgaacgca aaggagacaa tatagttgct 130140
   agagaagcca ttgtatcggt cgaaagactg taatatgatc gccaaacgaa agtccattta 130200
   tactaataga ttaaacataa tcagcagata acactttatc tgttgataat ggtatcaatt 130260
   ttttggttta tcatgtcgat tttgttgcca ttggtcatga cgagatcgta agtaaatttt 130320
   gtagtgtttt gaattcggtt caaataggac atggtgtcgt ttctaaattt ggccatatcg 130380
   taaacgatag agtcgaatct gtcttcgatc gctcgataaa taaaagtcaa cagcttttta 130440
   attgcggcca gttcgtaagc tatgctgcct gtctgccata gataagctag caacagaacc 130500
   agagccaacg cagagtagtt cattttcgtt tcccttattt tttttctaat attatattct 130560
   catttttgtt tcccttattt ttttctaatg attatatctt taattgattt aaaactatcg 130620
   accatggtgt cgttactgaa tgcaagttta ttagtcaagt ccgcttgcat gctcttcact 130680
   gaatcgagag ccgatgtaat gttcgagtat agagacgcat tttcggtgcg aatgatttcg 130740
   cgaatggtgt cgacggtgtc gtatccgaca ccattggagc cgagcacatt attattaata 130800
   ttgactttat cgaaatccgg ttgatataat tgtccgctat tggtgtttaa acattcttgt 130860
   agtgtacaga gctgtgtttt tatctcgttc agaggatcaa ccacctgact ctgtgcaccc 130920
   attattaaat cacaaaagag tcgcttcaat acgaaatact gaggcgggtg gactttttta 130980
   ctattcaaaa cgtacgacga gaggtatttt cctaatgaaa acacgtgtat atagttttta 131040
   ttgtttcgcg tcagtttttg ggaagcatta acgttcgtcc agagcactgc cttattgaaa 131100
   tttcttattg tcaccaaggg cgctaacagc ttagtggccg aacctagctc gacataccca 131160
   tctcgatcgt catcgctatt ttcaataatt accacttcca aaacctcatc gtcatagtga 131220
   aattgctgtt gcaatgaatc tgacatcacc acgttattcg tattgtttgg cccgttcatt 131280
   gatgtcgaat cgttgagatt gctcgtttcg tacattgtgt taatggctta attgtagaat 131340
   taaaaatagc actagatgat tacgtttatc gttttaaaaa agtatattag gtcttatctt 131400
   ttaagttaaa ttcgtatttc gaatataatt ttttaaatac attatgaagt ctcaaatgtt 131460
   ggtgtacgac aacggcggcc tcgccaacac caatcatttg tatactaatc atcgacaaaa 131520
   atcttatagt cccgtcgaca gaatgcacac ccgtcgtcaa tataattata caaactacct 131580
   taatgatttg tcctattcgc cggccgcttg gcataaactc acgaccaacg aaagaaccaa 131640
   cgttatctac gaaaaatgta agttatctca atatgatggg gtcgattacc gtagattaca 131700
   gggacgcgat agctttcggt gtgatgatat attaaagatt gtcgctagcc cgcagccttc 131760
   tagttattcc acaactatca atggagacac atcagcagca atcgtctcgt cgggaacaac 131820
   ggcaagaggt gttattatgg acaccattac tgtctaatct cgacgatatc gacaagaatg 131880
   ccgattatag aatcttaatt gacgattttg acatagatat cacgccgtat acagtctttg 131940
   aaaatgatgg agccacaata aaaatatctg gattgcgtct gtattatcta ctcaaaaata 132000
   aacgcttgta cgaagaatcg atggagactc agtgtagtag taataaaact tttaaaaaga 132060
   gtctgaaaaa agtgtgcttt acaaaggcta tccaaggagg aaaaaatagt gtagttgctg 132120
   tcatcaagag taaattacgc ttaccgcctt gtatacagtc tctactatca gacatcgatg 132180
   tgcgaccccg aggaaatagg tttcgaaaga gattcatttt caattgttat attgctaatc 132240
   tcataacgtg tccggcgtgc gacaaacagt gcatcgtaga cgccatggct gttctctacc 132300
   tgcacgacga caagtgcgtt cgtgaatttg aaaaactact caatcgcaat aaggacgtgt 132360
   ataagccgcc cagttgctta aatatgcaaa acaaagaacg tttatgtccc aataaaactg 132420
   gtatgggatg taaaggacgt aatcctctat gtaatttcta atttatatgt attgaataaa 132480
   aaaatttttt gtttaaaaat gatttttatt tacaaatttt cgaatataaa cgatttgact 132540
   gtttctacag aagattcaac ttgtaaagtg cgtcgagttt tctttttgta attaaagttc 132600
   ttcatatcga gctcttcgtt gagacacagc atggccagct gaggattagg atgaatgctg 132660
   tcgtaaacta gttccatatc gctatatttt cgtttcatag ctctgtgtcg tttggattgg 132720
   cccgacagaa aagctatctg agtagaattg ttttcgagag attttacgaa aaccgccaat 132780
   cttggatgtt tagaagaatc gcgtggaaag cgcacgcttt cataacgaca catttgtctg 132840
   tatcctccgt cgtggtcgtc gtcgtcgtcg ctttgattat tacgagattt gcacgataat 132900
   tggcaatcat cggcgacaca attgtcgcta gtcgcttcaa tattgtgacc atcgactcga 132960
   taattgtttc tgttgactcg atgattgtgt ctatccactc gatatttctc aagttgactg 133020
   tacaattctt ctgcagatat ttttttctca attttttcaa aattttcaca catttgagct 133080
   ttgagcgttt cgatcgcttg ataattttta gtcagagttt ctattgtatt agtttcattg 133140
   ttcctgacaa aagcatcaaa aacgcgtaac atttctgtca ttttctcttc tatcggcaac 133200
   gacgccaaag gcaacggctt cttttttgtc gacaaatgtt cttctttcat ggcatcaaaa 133260
   acgttttcaa tcatccatgt ggtaaattca attttatttt taaaattaac attatcgaga 133320
   atctgcatcg cgccatgttt attgatgata aaaatcgttt tattgtcgtc attgtctgcg 133380
   actgctcgag attctttttg aaaactcaac agctctccca cggttttttt atattgatca 133440
   ttctcgataa agtttatggc cgcatcgatg tcgtcaagtc cgatgccttc ggcgaaatcg 133500
   accgccaaaa tccagatttc gttctcgtaa aatagatagc gcaacaaaaa cgaaaattga 133560
   tcatcgaaac ataatctttt tttattgaaa atatacgaga atgatttcat gtctcgctgt 133620
   cgattccttc gattgtggtc actttcgtcg gcataattgt tgtcattgtc gacagaatcg 133680
   cccgaaaata ataatctctt tataaaatta aacattttat tggtaacaca caagacacat 133740
   acacacacac actttacaat atttggaaag aaaacttttg tcctttataa ttgtagctaa 133800
   aaggtgctct tatctgtgat tgagtacaaa acacgtgttt gtcgacagtc ggccagagct 133860
   tgtgtaatgt agcgacatcg gcgctcttgc cacttataga ctcgacggca gatttgtacg 133920
   agtgaataaa gcttccaggc cagattcgat ccagacgaat atctgcaggc ttgacgaata 133980
   tttggtaata gcgctctcgt agatttttgg aggctccgat gcgaaattta tctatacgta 134040
   accacacatg aatgcctcga ttgcccgagt gcatgattcg agctatgctg cttttgaaaa 134100
   aatttctaaa tgtctccgtc gccactttaa ttttcaagtc taaaaagtct gcatcgtcgg 134160
   cttcgatgtc cacgtcgatg acccattctc gaccgtcttc gagcgccttg acgtgaatat 134220
   cgctgacgtt ttgatcttga ataaattcaa atagatctcg agcgttcgta aagtattgtt 134280
   taggatgata ccaattgacg ccatcggtga acgaatactg tctgctgtcg ttgtatgcca 134340
   cgctattcca cagcttttcg acttgctcga atgtgtagag tccaggctga gcgtacatct 134400
   cgttagaatt gctgtcgacg agtggacgat aagtaacatg gcgttacatt tattgtttgc 134460
   cgttctgata tcgaatgcgc cacgcctcaa cagcaccgcg catgacgata tcgatagagt 134520
   attcgatttg attttggacg agatcaccaa aattgaggag aatgaagctg acgattttaa 134580
   ttatactcgt ttgattttct ttattctttt actgataata attattttga tcaagacacg 134640
   actatatcgc tatttgctat gctgtaaaaa ttgtcggaaa aaagcgatga cgattgaacc 134700
   aaaaacgaag gaatctccac cgacatcgcc gactacggcg gcgccgacag aaccgccgat 134760
   tgttaaactt gaattttcga aaaaaaatat ttccgacgac ataagcgtcg tcgacgataa 134820
   aggccgcgaa cctgcgatta aattaatcaa tttcaagtct attgtcgata ccgactagat 134880
   tatgataatt atgatagaaa ttgcgacatc aattgtcttt tgaatgccgg attcggattt 134940
   attacgctac gtctgctcgt cacataatta tagatttcat cgagattata gcccgtacgt 135000
   ttggtcaaat agtgtatgac tatggtggca gatcgagaga ttccggcgtg acaatgcacg 135060
   taaacgcgtc ttttctccag gtcaatcttt tgtaacaaaa aattgaaaga tttcaaaaaa 135120
   tgagattgta tgtcggctcg ttcgttgtca tcgatattaa tgtatagata gttgtcgatc 135180
   gcgtcgatta tgtcggtttt gcccacaaaa acgggaggat cgttccagac ggtcaacact 135240
   gcagtaatat tttcttggtg taaaaatttt tcaaatagat ttcgatcgta gatggcaccg 135300
   cccaaataca gattgtcggt gattttggac gcattaatca gtattccgtc tccgtcaccc 135360
   gctccgactc taatgtaatt catattgcgt acgctaaccc gtttccgtgc aaagattcga 135420
   ataaacacac cgtgtcagtg ggtcagtgtg ccatatttaa acaggggccg gacgacacac 135480
   gacacacatc atcgggtaac ctaaatatcc ttaaattaac cacacgaaaa aagtagagaa 135540
   agtgcacaat gaaaactata atagtgctgt cgttaatgat ggtactagtc attctaccgt 135600
   cgcaaggcgc cagaattttg gcagtgtttc caacaccggc gtacagccat cagaccgtct 135660
   tcaaaaccta cattcgagct ctggccgaaa gaggtcatga gattttcgtg atcaaaccat 135720
   cgaccagagt aaattacgta gatcggtact ctgatttgaa tcttttggga aatatcacag 135780
   agatcgatgc cagtctatcg gaagaatact ttaggcggct ggtcaaggcg tcgagcgttt 135840
   ttcgcaagag aggactcatc tccgatagta gcacagtgac ggcacacaac tatctcggct 135900
   tggtgaggat gatcagcgat cagttcgagt tgcccgaagt gaaaaacttt atagcaaacc 135960
   gcaaggaatt gcaattcgat ctactcatta ccgaagcatt tatggactat tctctagtgt 136020
   tttctcatct ttttgatgat ttgccagtga ttcaaatatc ttcaggctat ggagtggcgg 136080
   aaaattttga aacgatgggt gccgtcagta ggcatcctct atactatccg aatctttggc 136140
   gagacaagtt caccgacttg aacgtctggg aaacgatcaa cgaaatctat atggaattac 136200
   gattgcaaaa cgagtttagt aaactggccg acgaacagaa taaaatgctt aaagatcagt 136260
   tcggcgggag cactcccact gtacaggaat tgagaaatcg agtagaatta ctctttatca 136320
   acacacatgc cgttttcgat aacaatcgac cggtaccgcc gagcgtccag tatctgggcg 136380
   gattgcatct gaaaggaaat acgcaccgtc gtcatctcca aggattcgta aaggaatatc 136440
   tggacaattc tactcagggc gtcgtgtatg tcagtttcgg ttcgggtatc aacagtgctg 136500
   acatggaaaa tgaattcctt caaatgttcc tcgatgtgtt tgttaaatta ccctataata 136560
   ttttatggaa gtacgacggc atgatcgagt catccagact gccagataac gtgtttatac 136620
   aggcgtggtt cgatcagttt gaagtattga aacatccaaa cgtgaaggca ttcgtgactc 136680
   aaggcggagt acaatcgacg gacgaggcca tagaagcact cgtgccgctg atcggactac 136740
   caatgatggg cgatcaagca tttaacacta ataaatatgc tgaactaggc atcggccgga 136800
   atttggacac gctcactgta aatacaaaac aattggttga cgcaatcgtt gacgcatgcc 136860
   aaaatccttc gtaccgcaaa catttatcac aattgcgcca tattattcgt catcaagcaa 136920
   tttcaccttt acagaaagcc atatattata cggagcacgt gattaacaac cgcggacgta 136980
   ccatgctcaa aacgaaagct gccaacgtta gctatggcga ttacgtcatg tcatatataa 137040
   ttgttccttt cgtgactgtt tctattatga atcatttgcg tcaattgttg agaataaatt 137100
   tattttaata tgataactgt atttttttta tgttccaaat gtaaaatact acattttgta 137160
   ctctattaca agggaataaa acctgttatt tcgttgatcg agtaatctgt cgacactcga 137220
   tttgcgcagg cgttgtcggc tgcgcggatt tggtttataa acgactcgtg ttcatagtca 137280
   tgtacactac aactcgaact agcgccaaat catgtcgaca ttgaggaata aatgcttgat 137340
   acgaagcata caaatattag agaaaaatta tcgacgggtg tctgtagggg acttgaaaaa 137400
   aatatcagag gccatggcga tactacaaga gtccaacggt aaactaacga gaaccctaca 137460
   aaagatgtct tcgtattacg cagaacgcta caagaacaag atacagaacc ttcaaaatac 137520
   gatcgataaa aaagatcgtc acattaaaga tttatacaag caaatcgacg agaaaaacca 137580
   aaaaggagtt tttatagttc gactcgataa atctgtatgt ttcttttctt ctctcgagtc 137640
   cctgaataaa acaatcaagc aatctgcgtc cgtacagaat ataaagattt tattatatcg 137700
   aagcgcatgc gacatcaaat ctgaacgatc ggtctgcatc gcaatagcaa aggcgaaata 137760
   caagaattac gtgagcgttg agaatcgaaa gattatattc gtcgagagtg ccgatgtgga 137820
   ctcgttcgaa ttagatatta aacagatgct cacctaaaat atgtaaaata tgtagtaaaa 137880
   ttttttttag ttaattttta gtggtgtttg tgtgtaaaga ggctcgattt tcctagtact 137940
   ttaaacaatc ttgcctatac aagttaacta ttgcttcttc ggatacgctt gggtgttgaa 138000
   ataatttact cgaatactag aataaaatta tttattgata ttaatcgaat agacggacat 138060
   tacattcgtt gctactatta gaataaaatt tatttttgtt aaaatattta tcgtgtttat 138120
   tcatcctatt agctttatta tttttgttat tgttagaaat atatttgact aatgctatcg 138180
   ttaaagctat gttaaccagt attaaagata tacataaaac tcctaaaaat acaataaaat 138240
   actttaaaat aatactgacg atcgttttat tattttcctc attctcgctt ttgttgtggt 138300
   catcattttc tttgctaaag atgtcatttt catcgactat aatcaaagtc gatggaggat 138360
   taccagtgga aactattaaa ggaacaacag tactggtacg aggtaaatga gtagtaggaa 138420
   ttattgttaa aggtgttgtt gttgtacgag aataaggagt aataattttt cttgttacat 138480
   ttaatatcac atttataggc ttatatacta gagatgattt cttgactaca taactaaagc 138540
   aatacttgtt tggattaaaa ttaaagtgtt cgatatactt tttggtataa taatataaat 138600
   ctaaaggagt attctgatcg atatttagca gagacgtaaa caattttttt gaggtctcac 138660
   cgcctctgct caaaagccgt tcgaaatacg gttgattata catggcggcg agatgcacga 138720
   gtgtgttgtt acgcaaatcg acaggcttca ataaattttg taaagaaatc ttctcgtttc 138780
   gcagtctggg tatgttgttg gcgaacgaac gcaacgacag gttgctgttg atgctaggcg 138840
   gggccacgaa cacttgacat gcatccactc gattcatcag attctttaac gtctctgctt 138900
   cttgtacttc gactgctggt gctgatggag acgaacgaat cgaggcgaac ggcagtaatc 138960
   tcgaccaaaa cgatgaggcg cgacccaatc gaattaaagc gctcgtatcg tttcggcaag 139020
   acgacacccc attataccag taatcgttat cgatattgaa aaatttttcc aagacattgt 139080
   ggtgatcgac agcattgctg ggcatcagat atcgaactgc caactttact agtcctttct 139140
   gtagatattc aaaatcaaat tgcgacaccg gttcgtgatt agtctttatt tcgtattgcg 139200
   ctacaatttt ttcgatcgta ttatctggtc gcctgtttct attctgtttg atcagacgga 139260
   atggtgtcaa aataaatgca caatcgtcaa agtcgatctg tatatagttg tggcacacgg 139320
   taaacgttcg attatcgatc atctgcaaat actgttcctg taccgtattc ttggcgatga 139380
   tcgactcgtc tttgctgttt attttgtcgc attttgcgat tctgtttaat ttaaaaacgt 139440
   taaaatcatt ttctaaagcg gaatcgacat cgatcgtata gttttgcttt agaatcatat 139500
   ccctgaatat gctgggcttc atttcgtaaa gaaaagacac gagagcgctt cccatcgagt 139560
   ataatatatt ggaatcgtaa tcggctttga caatctcggc gatggtggcg tttcgatatg 139620
   cctttaaacc ctcatgatcg cgccaaaaac aatcacgatt accgaatctg tcggcggcgc 139680
   cttcgatata ccacttgggc atgaatcttg cttgtagcgt aaacaaaaga cagtgatgaa 139740
   attcatgacc atatgcgttc ggtagatcgt catgttcata gtctctgaag aagacatgag 139800
   ctcgaatcac acgtttctcg tgcacgtatt gagtgatgcc gccgttgttg acgccataat 139860
   tccagtaagg tcccaatcga acgtattgtt tacgatcgtt atgcacgtac atgtcaatgt 139920
   cggtgaggtt gtggcgatac tgaagctgta gtcgtttgta gaaatcgata aaatctttat 139980
   agacggtatt catttcgagc gaaatcttca tgagtacatt atttctagta atgttgtgat 140040
   gtaaaaaaac tcgtacgggt cccacgtcaa atctcgtgtt gaagggatag atgacatctt 140100
   caattttttc atagagattt ttatcaaact tggagtcatc gtaactgatc ttgttgccgg 140160
   agacgaccaa atttttaaat ttcgacacat aacacacgta aatgtcttgt ttgatctcgg 140220
   ccttcggcat aatcatcagc ggatgctcga gggctattcg agacaactga acggcagcgc 140280
   gatcgatgag ttcggttatt ttgatgtttt tctcgaatgt ctctctgatc gaagcgtaag 140340
   cgactagaca cgttttaaac tgtctatcgt tccacatgat gaatgaactg cacaattcgt 140400
   gaaacgatac aaaagttctt cgcatgttgg ccgagttcac gtagcgagta tacaaatcgg 140460
   cggtcatttc gaatagattc gaggcgacaa tctgcatttt gcgagaaatc cgacggtcga 140520
   cgagagcaat ttttctatta aatttatcta gcgaatccag tagagaggct tgagcatcgt 140580
   actcttgcat ccgagccatt agctgcaacc acaccatata tctctctatt tcggatggtg 140640
   tgttgaatgc aaaatcttga tctttaaaat tatctacaag ttttttggca ttacactcga 140700
   aattgatgaa tttggctcgt atacacgata tcaaaagttt attcattttc actttatcat 140760
   aatggctgta atcgatacac aaaaatgtcg gcagaaaaat tattgtcgtg attatcgcgt 140820
   acttgaaaaa caccaccatt gttacgagcc aatacacttt gactgccaat tgaaataaat 140880
   tttttcgaca catttgtctg ttttccttta aaactgataa tcattctaca agccattcga 140940
   tcgtccgaga atcgatcatc gtcgactcca tccacgccga cgatcgtccc gcggcgacgc 141000
   gcgatcgatc gatcatgaat cgccgcgagg cgatcgtccg agccgtcgag cacgtttttc 141060
   gcgtcgacga aaacatgatg caatgttgcg tcagccggat gtttgtcgcg cgcgcgcgtg 141120
   acccgaatcg ataacgatcg agaggtcgac gatcatccgg ggtcatcgat tgacgcacgc 141180
   gtcgcatgaa taatgatgat gatgttgata ttgcccaata ttgcatcatg ttttgtaatt 141240
   ataatgaaac gacaatcgta ttgcatcatc ttttttattt ttacgtttcc aacgaatccg 141300
   cattaaaact cgatgacgca aaatataaac tcgattataa tgtgatgtcc aaattcatca 141360
   attatgaggg taaaaacttt ataatcttgt aaaacgactc ggacgcttaa gagacatcga 141420
   ctacaaaaat tttttgtgat ttttttacaa aattttagtc tatcatcaca gacgttttta 141480
   atgaaattga agtcgttgat ccatataaaa agaattttag agacgatttc gatggtattt 141540
   actttgaatt tatagttatt ggcatgcatg ttgaggatac atgaatctgt aaaaattttt 141600
   aaaatcacgc gcaggccgtg tattgtgtag aaattaataa tctaaaaaag agacttttac 141660
   tcgatgattg atgtgaatca ccacataaat aaagttttcg acgttgacag ttttaaaatt 141720
   ttataaaaat ttttagcaga aaatttttat cgttttgtca gagccgaaat catcaataat 141780
   agcgtcgagt cgattcacga ttaaacgatt atgaatcgac acgacatcaa catcgtcgca 141840
   aatatctttg aaaatagtgt cagtaacgat ttcaacaata atggtgtcat ggcgattcat 141900
   gatcgtttca tcatgaatgg atatgacaaa tctaagaaac gatattcaaa aatctaagac 141960
   tcgaatatct gtaagatata tcaatcgcat gtctgtagcc tactatattg gtcgtgtcaa 142020
   aaatggatca aaacattaaa aaattattta ttaaagaaac caatctcaag gaaaaacacg 142080
   aaagtctgga caaggcctac agaaaaaaac ctaaagaaga tagaaacgag agtgcaatcg 142140
   atgagatgac cacctgcgcc gcctatttgt acggcgtgga agagcagctg tttagccttt 142200
   tgtcgaacgt gagtcaagag cgcagaatgg actttattaa cgatctaacc gaactggact 142260
   ttagtaacta cgaaattgaa ctattattga acactagatc cgatgaatat ttattgaaaa 142320
   aatttcatgt tcacaagaag agcgaaaata ttcaaaagat tttcaaaagt aacagtatta 142380
   gattcgttaa agtcttggag cagtttgtgg tgaagagacg ggcatacaag aaaaaaccta 142440
   aagatactct actcgaagaa ttggtcattt taaaatccat tataattaaa catttatgta 142500
   taatggaaaa acttacacaa aaataaaaat ctataaaatt ataatcaatc gttttttatt 142560
   tacacacaat tatatttaca tacacacaat tatatctata taaaaatata gcaacgatac 142620
   ggacaaaata cattatttcg atgacattag aaacgataat ggtggtgctg tcgccgccgc 142680
   tgtcaaagat tgccgatctg tcgtcggtat agccatatac gtgtatgctg catggtccca 142740
   tttgggtgta ttattggtgg gtacagcata gtatggcgac tcgtttttat ccacattaat 142800
   gtagtgtttc acctcgtcct tgtcgctatc gtcgtcacca tcgaagctat catagtcttc 142860
   ttctttaaaa tttatatatg tttcttgtgt ttcataaaat tttttcttgt cctccctgcg 142920
   gttttcatct tttataacgc cattcaccgg tttaatggcg attttttcta cttcatcaat 142980
   cgtgtcttca gtggatgtcg acttgtttgt tggacagcaa ctcgtcgcag ttgactttga 143040
   atctaatgat ttagtcttgt acgctacgta taaatatgat aaatgtatca atagaacgta 143100
   cgtaggaaat aaaattatag aaaagcgttt ggtttttaga aaagtcggtt ctccgacata 143160
   gtattcgctt ctgcattttg cgcaataaat ttcgaatttg ttgttaagcg tgaaacaatt 143220
   ggcgctttga atcgcattat tgtccatttt gacgattccg tcccagcatt ctttgtcgat 143280
   atcataatct ctctcgttga catcgaggcc gggaacatga ccgtatgtga cgaaccagtc 143340
   cattgaaaat agagcgattg tcgttaaaat agtggtcgcg ccgatgagcg gtacagcaat 143400
   ttgatcatag accaaagtcg agagcacgac aatcaatcca taaaccaaca gcacactact 143460
   aaaattcacc acttgactgc cgtcttcgta atcgatgatg agtccaaatc gtccgtcgtt 143520
   gatcccgacg actcccaaaa cgaaggcggt aaatccgcac gcaaaaagca tcactttggt 143580
   gtaataccgt aaatacgcct gcatcttgta agaaacatgc atgtactact aattatagta 143640
   gttgtgatta tttttctttt tattttatat aaacctatct atgccgccca cgtagagata 143700
   aaaaccgctc aagatgaata caacgccacc gtcgacgacc gaatcgagta catggaaggt 143760
   gtgttaaaaa gacgcaaata tgtaccgctc gaagctctgc ctacgatacg atttaatacc 143820
   gaactaggca ctataaacaa tggcgaacgc aagtgtatgt cggtgccggt gtacgtcagt 143880
   tcagataata ttcctaattt tgattgcaca ctcatctgcg atgatcccac cgcggtttat 143940
   ttttttgttg gcgaatttga taaatttctt gtaaacggcc agatgctcac gcgcggcggc 144000
   tattgtacta caaattctgt acctagaaac tgcaatcgcg agactagcat cattttacat 144060
   agcctaaatc aatggacgtg catcgcagag gacccacgct actttgccgg ccctatgaac 144120
   atggtacaaa ttgctgggcg acagcacgcc accagaatcg cacccggcga aatcacaaaa 144180
   aatattctct acgacaatct actcaaaatg gaggtcgacg tgtctcgaaa cacttttcgt 144240
   cgggattggg acgaatcgat gccggacggc agccgtagat tccaggtcat ttgtaatgca 144300
   ttagacgatc gattcaatca gatgtttgta aatcccatga atccaatcga gtgtttaccc 144360
   aatacatgta cgagtgctag atatgtgcat cgagacgtta aacccaattt cgagacgggc 144420
   gaatgcgaat gtggtgatgt taacgttacc agactaactc atatcgtgcc cggtgatcgc 144480
   acttccatgt gcgcaagtgt tgtcgatacg tttcaaacga ccaacaattc acacgtattt 144540
   agggtgcaat gtgtgaatat gaacacgtta atcagcgact atgatccaaa catgcttcta 144600
   tgtcctggct cgcaattcga ttccaataac gatagcgcgt ttagctatga attgccgggc 144660
   acgtatgttt tttcttcgaa cggcatacac gaacctactc atcggttcta tttagacacg 144720
   cgcagtagaa tcgactataa tatcgtaaat cctgtcccga cttgaaacct ataaaagtca 144780
   gtagcatgta tctttagttt tattgagttg ctgcgaaacg atagcaatgg accgacaaac 144840
   tacacaaaaa ctactcgaaa atgagaataa taaacacaaa aatgttggca tacaactcaa 144900
   caaagaagaa tttggaagcg acatcaaaaa gggaaatcca aatttttatc atatactaaa 144960
   taataccgag gacctcaaaa aacatgccaa atgtcgcaga aaactaaact ttgatttcat 145020
   ataaagatgg aatggaaata aaatcatcat gttattcggg atatctatca ataaaaatat 145080
   aaattcaata aaacattttt atttataaaa cctaatcatt atagtttaat cgtactcgat 145140
   attctcgagt ttattctgta gagctaataa atgctgtgga tcgtgatgtt ttttcttgac 145200
   aggcttcggg ttcatgaatt gcatttcacg atcgacacga ataacggtag gatcttcgcg 145260
   acgacagcaa cacgagaaca tagaagacac ggtgcccgag cagcaagtag aataaatttt 145320
   gaacaccatc agagctagca gactgagtat aatccacacg aaaaccattt ttacgacatg 145380
   gaaatcggct ccaatcgacg agaaaaaatt gtaaagccac gagttttcag tatcgtctag 145440
   aatttcatcg ccggcaaaaa ccgtattatt gttcattagc cgacgtaaat ctaccaatct 145500
   atcggtcaat ttgtgtaaat tattgtgctc taaatcatta ttcattttca tagtctgcag 145560
   ctgtagagtg tccagatcct tgagcacagc actcaaatta aaggctgtat cgatgggcat 145620
   ttgtaaattt ttatttagac tgttgtacgt ttcgagtgta gttaaaaacg tccgtttcgt 145680
   gctcaattga caagtgtgct tgccggtagc gttaatgatg ccgattcccg ccgacaaaac 145740
   tagagtgttt gccgacaccg aattatctcc tttctgtaga cagtcaaagt tgatgtcgac 145800
   ctcattttcg aggacataga gccatcgatt aaaatcagta attttataga agatttcact 145860
   ctcaaattta ccgattctaa catcacaatc cttttcaaaa ttgatgtctt tgtcgttttt 145920
   caaaaagata cgaatatcac acagctgcgc ttgattagaa tcgtgtacga cctgaggcac 145980
   gtagcagaga atcatgtcgt gtccgagtcg acaagactcg cgagtgtcgt cgaaacgcac 146040
   aaaattcctg cgatccttcg atacacctag atatttgcta tccggtagaa ttatggcaca 146100
   tttagattta acgcaaaacg gtatgggcac aatttggtag acatcatatt tttgctgatt 146160
   gacgagcggc acttcaatga tgaatagtag ttttctttct ttagtgataa aaacgtgaat 146220
   attaatgatt ctgtcgatga gctcgaacat attgtcgtag tgcaatggca caggccacga 146280
   taaaccttta ggtagataat tgctcacatt cttcatttct tccagcaatt tttcggcggt 146340
   aatgactgcg gaactgagtc gtttgtcttt ggcgtcctcg acggcatctt taatctggtc 146400
   gtaaagactg tctagcctat cgatctcact acagatcaaa tgaattttag tgcccacata 146460
   ctcacatact ttttcttctc ttcgtttggc atccaattca tcttccacca tacgttcaaa 146520
   ctgaatcaaa tgatcggata tctgttttat ctgacgattg agtgcgttcg tgccgttggc 146580
   gagtttgtga agttcgtggg catcgtcact gtccattagg ccgaacagat atttatatag 146640
   gctaccgaga gggtcgaata gacctctctt gcttctgctc agcgtatatt tcaatggagt 146700
   ttcgtagttg ccagacttgc ggatttttcc atcgatatcg ttatgaagat tcaccaagtt 146760
   gacgattttg ttcatgatag ttttatcgag ttcggtctga ataatatttg aactgctaca 146820
   atttttaaat tgtctacttt tgtagatgaa tttttgtaga tttttaacat gatcgaacat 146880
   ttcttttaat cgcaaaaaca cagcggcatg atccatttct acaataaaat gccaagtgtt 146940
   ttcgacaaac tgcatctcct tgataggttg aaaatacaag cccgaagtgt gcggtaaatg 147000
   ctgtacgtta ataatttcgt tagccgatat tagttcttta ttggcgtcgg ctgttgcgca 147060
   caacaacatc atcgacactg ccaacgagat tagcggctgt gacaatgtca tgttgtttcg 147120
   cgtttctatc gttcacacta tactgaatat gtcagcgaag actttgaggc tttatactcg 147180
   gcaacgcaga atataattaa tagcttacta ctatatattt tttaaccgtc gtcaagttgc 147240
   cgttgctata ttgtcggtcg taaatcaatc gtcacagaca agcgacatga ttttcgtggt 147300
   cattattttc gtgtgtcata cccaaaaata gagtcaataa aatacacgtg ttattatttt 147360
   aaatgcgtca tttatttatc aagttgaccc ctaccacaca ataacaggat aataaaaaat 147420
   aatgaataca ataacaagat aataaaataa tacaacaaaa tgtgatgtca tgtgtctgtt 147480
   ttttaacctt ggcagctctc gcaaccgatg tagcatgcgt caacaacatc cggttcattg 147540
   tttgcacgat tgatcgccga cgatgaaggc acggtgaatt gaatcgtgtt tgctgcgggt 147600
   ttggttctca aatagtacat gcccgttttt aaaccctttt cccaagaata gtcgtgaatg 147660
   gatgataaaa ctttataggt aggattggcc acaaaaagat tgagcgattg gctctgatcg 147720
   atgtaaggtc cccgctctgc ggccatgtcc aacactactt tttgtttaat ttcccaagcg 147780
   gtcttgaaga gcgccttgat attgtgtgga atttcatcaa tattttgtac agatccgttg 147840
   tttttaatga tttttatcat gatatctgtc gagtatacgt tggtgtctac gagtttatcg 147900
   acgagatgct gattgaccac ttgatactcg cccgacagaa ctcttcgtac gtacaaatta 147960
   ctagtaaacg gctcgaacga ctcgttgttg cctagaatct gtgcagtcgt tgcggtcggc 148020
   atgtaagcaa tcaataatga attgcgtaag ccgtacatag ccatttctcg cttaagctcg 148080
   tcccagttcc aaagatcatt cgattggtta atattccaca aatcaaactg taaaagtcca 148140
   gaacttgcag ggcttccatc gtaagaatcg tatgttccgt gtagtttagc tagttcgata 148200
   ctttgactta gtgcaccata gtaaatagtt tctgcaatct ttttatttaa atctctagcc 148260
   tcttggctgt cgtagggcat atccaaaatg gcgaacgtat cggcaagtcc ttgaataccg 148320
   ataccgatgg gtctgtgtct gaaattcgat gcttgagcgc tagccaacgg ataaaaattc 148380
   aagtcgataa ttttattgag attattggta accactctag atacattgta gagcgactta 148440
   aagtcgaagc ttttaaattg tttattggca aaattattta cggcaatcga ggccaagtta 148500
   cacacggccg tttctcgatt gtcgctatat tgaacgattt cggcacacag attgctgttt 148560
   cgaatcactc cctcgttttt ttgattattt ttattgttaa cgtgatcttt aaacatcatg 148620
   tatggagctc cggtttcgac ttgagtctca ataatttttc tatgaagatc taaagccgaa 148680
   atttgttttt taaacttgcc ttcggcctcg taattttcgt aaaggtcctc gaattcttgg 148740
   ccatagcaat cgtttaaacc gggacactcg tcggggcaca tcaatgacca tttccctcct 148800
   gtctttaccc tcttcataaa cagatcaggt gtccacatgg caaagagaag atcgcgagcc 148860
   ttcatatcct cggcacccat attgcgcttt agatccaaaa aagtaaaaac gtctgcgtgc 148920
   caaggctcga tgtatacggt catggcgcct ttccttttac ctccttgatc gacatggcgt 148980
   agcatgctat taaaaacacg caatgcaggt cccaatcccg ccgcagttcc gtttgtggat 149040
   tcaattttac tattggtggc gcgaatttga gtgacattca ggccgatgcc gcctccgtgt 149100
   ttagatatca tggcacaatc gttgagtgtt ttatagatgc cgttaatact gtcctcttga    149160
   atgttaagca ggtagcacga tgacatttga ggcattttca tgccagacga aaatagagtt    149220
   ggactagcgt gagtgaacag ctttgcccgc atcaatctat aggattcgat agcgctttcg    149280
   atgtcatcac catgaatctg tagagctaca cgcatcagca tgtgttgagg cctctcgacg    149340
   acaatattat cgactctcat caaatatttt ccttcgatta aagtcatcag accaaagtaa    149400
   gtgtaatgtt tatcgaaatc gtaagaaatg ctagcattga ttgagtctaa atgctttata    149460
   actatcgaat agaaagaatc acaaagaatc ccatttttcc agagaatctc tactacttta    149520
   gagtaatcgt cctcggtttt ggcatgtagc ttgttgactg cgatcacaac ggccagatca    149580
   gaataaacat agtgcttgta agtctgagag gcacacacat tagcactgta ttcttcaaac    149640
   tcatcctcgt gtatattgtc atagattccg caaaaaattt tatcggcaat atagttaaag    149700
   tcgatgtatt ctttattttt tttcaagtca ccacttcgag tataaagata agttttgatg    149760
   tagtccatat ttactttgat cgactctcgg ccgttcctca aaatcatttg atatgaagaa    149820
   aacattttaa aatcactata caaaagtcac gactcggtgt caaggtgtat gtagtaataa    149880
   taaaacacat ttgattgtcg agtatattta tatcatttaa tcgctgaatg atgctcgtcg    149940
   gattgagaaa gagataaaaa attctcatat tagatgcgtt tgccgtaatc atgtttcgac    150000
   caaaaatttt tctattgatc atgcacaaca aaataagtat ttttctcctt tcgtaaaagt    150060
   ttgtgaaaaa tcaaatata                                                 150079

## Description

### Field of the invention

The invention refers to the technical sector of biological pesticides used in the control of insect pests.

Specifically, the invention refers to three novel genotypes of a nucleopolyhedrovirus that is capable of infecting larvae of the lepidopteran *Chrysodeixis chalcites*, to compositions that comprise one or various of these novel genotypes, to a procedure for the production of the virus genotypes and their use for the control of infestations of the pest insect.

### Background to the invention

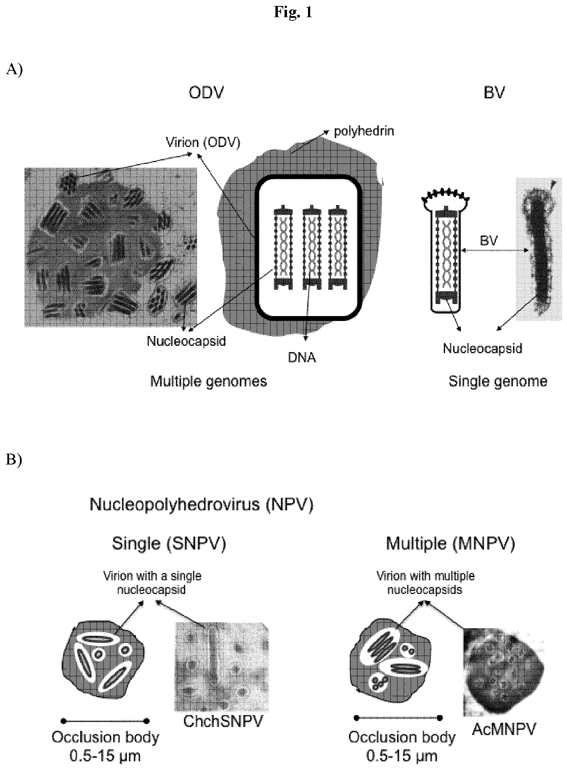

Baculoviruses (Baculoviridae) are a family of viruses that specifically infect invertebrates and that have been isolated from numerous phytophagous species, among which many of the the most important species of insect pests are found, principally those of the order Lepidoptera. These viruses have a double-stranded DNA genome packed in a protein capsid to form the nucleocapsid. During the replication of the virus, the nucleocapsid acquires a trilaminar envelope comprising a protein layer between two layers of lipids (Caballero et al., 2001). Some nucleocapsids remain in the cell in which they were produced, where they acquire a membrane that is synthesized *de novo.* These occlusion derived virions (ODV) are subsequently occluded in a protein matrix to form the polyhedral occlusion body. Other nucleocapsids, once produced, leave the host cell. In this case, the nucleocapsid acquires a membrane derived from the host cell membrane at specific points at which a virus-encoded glycoprotein (GP64 or F protein, depending on the virus), is present in the cell membrane. These virions are called budded virus (BV) and are found freely in the haemocelic cavity of the host, and are responsible for propagating the infection to the different host tissues. Consequently, baculoviruses possess two types of virions or viral particles, that are morphologically complete and infectious: the occlusion derived virions (ODV) of the occlusion bodies are responsible for primary infection in the digestive tract, whereas the budded virions (BV) are responsible for secondary infection that is propagated to all the organs and tissues in the haemocelic cavity of the insect (Fig. 1A).

Morphologically, ODVs can be one of two types; the single type (SNPV, single nucleopolyhedrovirus) contain a single nucleocapsid, whereas the multiple type (MNPV, multiple ncucelopolyhedrovirus) contain a variable number of nucleocapsids within each virion (Fig. 1B). The ODVs found in all known baculoviruses are the infectious entities responsible for transmission of the virus to susceptible individuals and the initiation of the primary infection of the epithelial cells of the insect midgut. In contrast, BVs are morphologically similar and each contain a single nucleocapsid. These virions are the infectious entities responsible for diseminating the infection to organs and tissues in the haemocelic cavity of the insect, and for diseminating the infection in cell culture *in vitro* (Caballero et al., 2001).

In the final stages of the infectious process, all baculoviruses synthesize large quantities of polyhedrin (in the case of nucleopolyhedroviruses, NPVs) or granulin (granulovirus, GVs), which crystalize to form a matrix or occlusion body (OB) in the shape of irregular polyhedra (polyhedrin) or granules (granulin). Due to their shape, the occlusion bodies of NPVs are also known as polyhedric occlusion bodies or, simply, as polyhedra. Various ODVs are occluded within each occlusion body of NPVs, whereas a single ODV is occluded within each granule of GVs. The matrix preserves the infectious characteristics of these viruses outside the host, as OBs are insoluable in water, resistant to putrefaction and breakdown by chemical agents, and also resist physical treatments such as freezing, dessication or freeze-drying, which allows them to persist in the environment. In contrast, OBs are soluable in alcaline solutions, such as occur in the digestive tract of some insects (pH 9 - 11), which facilitates the release of ODV virions from the OBs and allows them to initiate infection (Caballero et al., 2001).

Historically, the Baculoviridae family has been divided in two groups or genera, based on on occlusion body morphology: *Nucleopolyhedrovirus* (NPV) with polyhedral occlusion bodies and *Granulovirus* with granular forms. However, since 2006 the classification has been based on phylogenetic analyses of the genomes of these viruses (Jehle et al., 2006). Currently, four genera are recognized depending on the species of host infected: *Alphabaculovirus* are lepidopteran-specific NPVs, *Betabaculovirus* are GVs that infect lepidopterans, *Gammabaculovirus* are hymenopteran-specific NPVs and finally *Deltabaculovirus* are NPVs that infect dipterans.

Natural populations of baculoviruses have high levels of genetic heterogeneity. This diversity is observed following the characterization of different geographical isolates of the same virus, and is also present within individual isolates, each of which often comprises different genotypic variants. Analysis of viral DNA using restriction enzymes has been shown to be a very useful method for characterizing and differentiating isolates and genotypes present in each isolate, as it results in profiles that are characteristic for each isolate or genotype (Erlandson et al., 2007; Harrison y Bonning, 1999; Figueiredo et al., 1999).

Closely related isolates do not usually shown large differences in phenotypic traits such as insecticidal properties, although occasionally minimal changes in the genomes of these variants can affect important biological characteristics, such as the quanity of inoculum required to kill a percentage of the host population (pathogenicity), the speed at which the virus kills the insect (mean time to death, MTD), the yield of occlusion bodies (production of OBs), or even the host range, occlusion body size, or liquefaction characteristics of larvae (Cory et al., 2005; Harrison et al., 2012). The geographical origen of the virus and the host have also been observed to affect the dose-mortality curves and the period of survival of the infected host (Erlandson, 2009; Erlandson et al., 2007).

*Chrysodeixis chalcites* (first described by Esper in 1789), is a lepidopteran of the family Noctuidae, whose members are often referred to as nocuids in the scientific literature and whose larvae are eruciform caterpillars with a cilindrical body, well developed head and abdominal pseudopodia (false legs). This moth is considered one of the most important polyphagous pests in many countries, principally in southern Europe, Africa, Oceania, Asia and some parts of the Americas. The caterpillars consume many species of plants including ornalmental, vegetable and industrial crops, as well as plants of no commercial value. In The Netherlands, the pest principally affects tomatoes, peppers and chrysanthmums grown in greenhouses. In mainland Spain, this insect causes economically important damage in Valle del Guadalquivir, Vega de Granada and in the greenhouses of Almería province. In the Canary Islands (that are a Spanish territory located off the coast of Africa, some 100 km from Morroco), populations of these moths have undergone marked increases over the past decade and currently cause losses of up to 30% of the banana harvest.

This pest is usually controlled by the use of synthetic chemical insecticides. However, chemical based control measures require multiple applications, that tend to increase production costs, hinder the commercialization of products that may contain pesticide residues, and may lead to resistance in the pest insect population (Horowitz et al., 1998; Perera y Molina, 2007; Cabello y Belda, 1994).

The growing tendancy to minimize the negative impacts associated with the continued use of chemical insecticdes has driven the search for alternative methods of control, including the use of viruses and other entomopathogenic microorganisms.

*Bacillus thuringiensis* (Bt) is a Gram positive bacterium that is frequently used as a biological alternative to synthetic chemical-based pesticides. The abundant literature on this organism, and the review of Roh et al. (Roh et al., 2007), reveal that the bacterium is abundant in the soil, on the surface of the leaves of plants, in the gut of diseased or dead insects and even in stored products.

The insecticidal properties of this bacterium is due to protein crystals, known as δ-endotoxins or Cry proteins, that develop during sporulation, and which must be ingested by the susceptible insect in order to exert their effects. The Cry toxins are specifically active against insects of the orders Lepidoptera (butterflies and moths), Diptera (flies and mosquitoes), Coleoptera (beetles) and Hymenoptera (such as some ants), and also nematode worms. Specifically, each Cry toxin has a defined spectrum of activity, which is usually restricted to a few species within one of the aforementioned orders, although some of them have a spectum of activity that covers two or three orders of insects. The *cry* genes that encode the Cry proteins, are usually located on plasmids. Each strain of Bt usually contains between 1 and 6 *cry* genes. Thus, depending on the gene content of the plasmids present, each strain of *Bacillus thuringiensis* can synthetize more than one type of Cry protein. The combination of toxins of each strain defines its spectrum of activity.

Bt-based insecticides began to be commercialized in the 1930's and since then its use as a biopesticide has grown markely, specially in the form of sprays that contain endospores, Cry protein crystals or their mixtures. Moreover, transgenic plants (principally potato, maize and cotton), known as Bt crops, have been developed that express Cry protiens and that act as producers of their own insecticide, thus reducing or eliminating the need for insecticide applications on these crops.

The use of *Bacillus thuringiensis* based insecticides is generally considered environmentally safe, although some studies have identified a possible risk of hepatic damage in rats or changes in the fertility of mice. Moreover, studies have been published that indicate that some Cry proteins could kill the Monarch butterfly (*Danaus plexippus,* a lepidopteran of the family Nymphalidae), particularly if the Cry proteins are present in the pollen of Bt maize. The validity of these studies has been questioned, although these and other studies suggest that resistance can develop in insect populations that were previously susceptible to certain toxic Cry proteins. The search for alternative bioinsecticdes therefore appears to be warented.

Nucleopolyhedroviruses (NPVs) have generated interest for insect pest control, as much for their insecticidal capacity, as for their specificity and safety, specifically because they only infect invertebrates. Various NPV isolates from many places in the world have been characterized biochemically and biologically (Gelernter y Federici, 1990; Caballero et al., 1992; Hara et al., 1995) and some of them are currently registered as insecticides in various parts of the world. where they are used for pest control (Moscardi, 1999).

Among other entomopathogens, larvae of *C. chalcites* can sucumb to nucleopolyhedrovirus infection which is known in its abreviated form as ChchSNPV (*Chrysodeixis chalcites* single nucleopolyhedrovirus, genus *Alphabaculovirus*). This is an SNPV that can also infect larvae of other members of the family Noctuidae, such as *Trichoplusia ni* larvae.

To date, two isolates of ChchSNPV have been described:
- One of these originates from larvae of *C. chalcites* in Netherlands' greenhouses, which in the present document will be referred to as ChchSNPV-NL, or the more abbreviated form, ChchNL. The genome of this virus was described by van Oers and colleagues (van Oers et al., 2004, 2005). Specifically, Table 2 in the paper by van Oers et al. published in 2005 lists the open reading frames (ORFs) predicted in this genome, in which ORF4 (comprising nucleotides 2965 to 6504 according to the table) corresponding to the *hoar* gene, and ORF114 (comprising nucleotides 113,517 to 114,806) corresponding to the gene *bro-d.* Van Oers et al. compared this genome with those of other viruses of the genus *Alphabaculovirus.* They observed a close relationship between the genome of ChchSNPV-NL and the genomes of viruses that infect the noctuids *Spodoptera exigua* (SeMNPV, sequenced by Ijkel et al., 1999) and *Mamestra configurata* (MacoNPV, sequenced by Li et al., 2002a, 2002b). The complete genome of ChchSNPV-NL is available in the GenBank database, with accession number AY864330.
- The second isolate was obtained from larvae present on vegetable and fruit crops in the greenhouses of El Ejido (Almeria, Spain); in the present document this isolate will be referred to as ChchSNPV-SP1, and also in the more abbreviated form as ChchSP1.

This isolate was characterized by Murrillo and colleagues (Murillo et al., 2000), that are members of the present group of inventors. ChchSNPV-SP1 was described in their study as quite host specific, as it was only infective to larvae of *C. chalcites, Plusia gamma* and *Trichoplusia ni,* that are considered species that are phylogenetically very closely related. Consequently, these authors suggested that the virus could be of interest as the basis for biological insecticides in situations where there exists an overwhelming need to conserve other species present in the ecosystem. In their paper, additional information on the insecticidal capacity of the isolate, such as its pathogenicity (LD₅₀) or virulence (LT₅₀), was not given. Said paper also presents a study on the proteins and the genome of ChchSNPV-SP1, in comparison to that of the NPV that infects *Autographa californica* (AcMNPV-wt) and other NPVs from the area around Almería, such as the virus of *Spodoptera exigua* (SeMNPV-SP2, abbreviated to Se-SP2 in their paper) or Morocco such as the virus of *Spodoptera littoralis* (SIMNPV-M2, abbreviated to SI-M2 in their paper). Studies on protein electrophoresis (structural proteins from the occlusion body and the lipoprotein membrane) and the study of the profiles of genomic DNA obtained using restriction enzymes, revealed that ChchSNPV-SP1 is a different virus, that is distinguishable from those mentioned previously using the aforementioned techniques. Specifically, Table 1 of their paper presents the estimated sizes of restriction fragments obtained using the enzymes *Bgl*II, *Eco*RI and *Pst*I*,* and the total genome size estimated from the sum of the fragment sizes, as shown below (Table 1):

**Table 1.- Estimated fragment sizes of C. chalcites NPV obtained by digestion with BglII, EcoRI and PstI and total genome size in kpb (Murillo et al., 2000)**

| **Fragment** | ***Bgl*II** | ***Eco*RI** | ***Pst*I** |
|---|---|---|---|
| A | 15.0 | 17.8 | 15.1 |
| B | 12.5 | 17.6 | 14.9 |
| C | 11.8 | 15.1 | 14.6 |
| D | 10.3 | 10.9 | 11.4 |
| E | 10.2 | 10.9 | 9.8 |
| F | 9.2 | 8.6 | 9.1 |
| G | 7.9 | 7.4 | 7.4 |
| H | 7.0 | 7.0 | 5.1 |
| I | 6.7 | 6.4 | 4.7 |
| J | 5.1 | 3.9 | 4.7 |
| K | 3.8 | 3.8 | 3.5 |
| L | 3.6 | 3.5 | 3.3 |
| M | 3.5 | 3.2 | 2.7 |
| N | 3.5 | 2.8 | 2.6 |
| O | 0.5 | 2.7 | 2.5 |
| P | 0.4 | 2.6 | 1.8 |
| Q | 0.2 | 2.0 | 1.6 |
| R | - | 1.9 | 1.4 |
| S | - | 1.8 | 1.3 |
| T | - | 1.8 | 1.2 |
| U | - | - | 0.9 |
| V | - | - | 0.8 |
| Genome size | 123.8 | 131.7 | 123.7 |

It would be useful to have an alternative to chemical insecticides or products based on *Bacillus thuringiensis,* with a high insecticidal activity against *Chrysodeixis chalcites.* This alternative should preferably have a limited host range, as often ocurrs in baculoviruses, so that other organisms, particularly other insects, would not be adversely affected, with the exception of those that are phylogenetically closely related to *Chrysodeixis chalcites.*

The invention provides a solution to this problem.

### Description of the invention

The present invention is based on a new natural isolate of *C. chalcites* single nucleopolyhedrovirus originally from the Canary Islands, herein named ChchSNPV-TF1 (or in the more abbreviated form, ChchTF1), and the discovery that the isolate contained several distinct genotypes of ChchSNPV, that differed from one another and which were also distinct from the known viruses, ChchSNPV-NL and ChchSNPV-SP1.

Assays performed using genotypes obtained from the ChchTF1 isolate demonstrated that three of the novel cloned genotypes (ChchTF1-A (CNCM 1-4690), ChchTF1-B (CNCM 1-4620), ChchTF1-C (CNCM I-4621)) and in particular, their mixture in a determined proportion, results in one of the most active nucleopolyhedroviruses that have been developed as a bioinsecticide to date.

The product represents a clean and safe technology that does not leave toxic residues on crops and is not toxic to man or other animals, including insect natural enemies such as parasitoids or predators.

Moreover, these nucleopolyhedroviruses have the additional advantage that they are easy and abundantly produced.

Thus, the first aspect of the present invention refers to a single nucleopolyhedrovirus of *C. chalcites* (ChchSNPV) characterized in that is selected from the group:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype characterized by a genome as depicted in SEQ ID NO: 17 (ChchTF1-A), SEQ ID NO: 18 (ChchTF1-B) or SEQ ID NO: 19 (ChchTF1-C).

This nucleopolyhedrovirus can be in different forms, such as a virus particle (virion), or in the most complete form which is the form that is ingested by larvae in nature, the occlusion bodies (polyhedra) that contain various co-occluded ODVs.

An occlusion body can contain virions of one of the previously mentioned ChchSNPV or genotypes or mixtures of ODVs of the previously mentioned ChchSNPVs or genotypes.

The virions of the present invention can be occlusion derived virions (ODV) (the form of propagation which is occluded in occlusion bodies and which is released in the intestine following the breakdown of the polyhedrin structure), or as budded virions (BV) (the form in which the infection is propagated to the different tissues of an infected insect, and that may also be present in cell cultures).

An additional aspect of the present invention is an occlusion body that contains various virions in which at least one of the virions is a virion of single nucleopolyhedrovirus of *C. chalcites* selected from the group of:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype characterized by a genome as depicted in SEQ ID NO: 17 (ChchTF1-A), SEQ ID NO: 18 (ChchTF1-B) or SEQ ID NO: 19 (ChchTF1-C).

In this aspect of the invention, the occlusion body can contain various virions comprising the genome of the same genotype or of different genotypes that are co-occluded.

In the case in which the virions comprise the genome of the same genotype, the genotype can be any of the genotypes characterized by the genome of ChchTF1-A (SEQ ID NO:17), ChchTF1-B (SEQ ID NO:18) or ChchTF1-C (SEQ ID NO:19). In the second case, in which the co-occluded virions can be mixtures of virions comprising the genomes of any of the genotypes ChchTF1-A (SEQ ID NO:17), ChchTF1-B (SEQ ID NO:18) and ChchTF1-C (SEQ ID NO:19) in different proportions, with two or three of these genotypes present in the mixture.

Additionally, virions comprising the genomes of other genotypes of *C. chalcites* single nucleopolyhedrovirus can be present in the mixtures, or all virions can have the genome characterizing one of the genotypes of the group ChchTF1-A, ChchTF1-B and ChchTF1-C.

In whichever case, it it perfectly understood that virions within occlusion bodies are occlusion derived virions, ODVs.

As will be described later in this document, the mentioned genotypes can be distinguished by the specific sequence of the genome characterizing each one in certain variable zones of their respective genomes, such as the *hoar* or *bro-d* genes, as described in the Examples in the present document.

Possible embodiments of this aspect of the invention are occlusion bodies that contain at least one virion (ODV) with a genome that contains a fragment of DNA whose sequence is represented by:
- SEQ ID NO:5, SEQ ID NO:6 or SEQ ID NO:7 (the specific sequences of the *hoar gene* amplified by PCR using primers F-Hoar and R-Hoar in Examples of the present invention, which characterized ChchTF1-A (CNCM I-4690), ChchTF1-B (CNCM I-4620) and ChchTF1-C (CNCM I-4621), respectively),
- SEQ ID NO:8, SEQ ID NO:9 or SEQ ID NO:10 (the specific sequences of the *bro-d* gene amplified by PCR using primers F-Brod and R-Brod in Examples of the present invention, which characterized ChchTF1-A (CNCM I-4690), ChchTF1-B (CNCM I-4620) and ChchTF1-C (CNCM I-4621), respectively),
- SEQ ID NO:11, SEQ ID NO:12 or SEQ ID NO:13 (the complementary sequences to the coding regions of the *hoar gene* of ChchTF1-A (CNCM I-4690), ChchTF1-B (CNCM I-4620) and ChchTF1-C (CNCM I-4621), respectively, in which the previously mentioned variable regions are present in this gene),
- SEQ ID NO:14, SEQ ID NO:15 or SEQ ID NO:16 (the complementary sequences to the coding regions of the *bro-d* gene, of ChchTF1-A (CNCM I-4690), ChchTF1-B (CNCM I-4620) and ChchTF1-C (CNCM I-4621), respectively, in which the previously mentioned variable regions are present in this gene that can be used to differentiate the genotypes).

An additional aspect of the invention is a composition comprising nucleopolyhedrovirus of at least one of the previously mentioned ChchSNPVs: ChchTF1-A (CNCM I-4690), ChchTF1-B (CNCM I-4620) and ChchTF1-C (CNCM I-4621), or those having the genotypes characterized by a genome as depicted in SEQ ID NO:17 (ChchTF1-A), SEQ ID NO:18 (ChchTF1-B), SEQ ID NO:19 (ChchTF1-C), or combinations thereof.

As in the previous case, nucleopolyhedroviruses can exist in different forms, such as free virions, or more preferably, in the form of occlusion bodies that can occlude a variable number of co-occluded virions (virions which will be ODVs, occlusion derived virions, as described previously in this document). In this second case, the virions contained in the occlusion body can comprise the genome of one of the previously mentioned genotypes, or more than one genotype, as long as at least one of the genotypes is characterized by a genome as depicted in SEQ ID NO: 17 (ChchTF1-A), SEQ ID NO: 18 (ChchTF1-B) or SEQ ID NO: 19 (ChchTF1-C), that is, the genome of ChchTF1-A (CNCM I-4690), ChchTF1-B (CNCM I-4620) or ChchTF1-C (CNCM I-4621). Consequently, this aspect of the invention refers to a composition that comprises a nucleopolyhedrovirus of the invention or an occlusion body of the invention.

Specifically, possible embodiments of the invention are those comprising the mixtures of virions of distinct genotypes that were used in assays described later in the Examples of the present invention, preferentially those compositions that comprise a mixture of virions of :
i) ChchTF1-A (CNCM 1-4690) and ChchTF1-B (CNCM I-4620),
ii) ChchTF1-A (CNCM 1-4690) and ChchTF1-C (CNCM I-4621),
iii) ChchTF1-B (CNCM 1-4620) and ChchTF1-C (CNCM I-4621), or
iv) ChchTF1-A (CNCM 1-4690), ChchTF1-B (CNCM 1-4620) and ChchTF1-C (CNCM I-4621),
   or virions of the genotypes comprising the genomes as depicted in
v) SEQ ID NO: 17 (ChchTF1-A) and SEQ ID NO: 18 (ChchTF1-B),
vi) SEQ ID NO: 17 (ChchTF1-A) and SEQ ID NO: 19 (ChchTF1-C),
vii) SEQ ID NO: 18 (ChchTF1-B) and SEQ ID NO: 19 (ChchTF1-C), or
viii) SEQ ID NO: 17 (ChchTF1-A), SEQ ID NO: 18 (ChchTF1-B) or SEQ ID NO: 19 (ChchTF1-C).

In principle, the genotypes can be present in any relative proportions. It is specially preferred the composition that was assayed and shown to produce the best results, namely the mixture of the genotypes ChchTF1-A, ChchTF1-B and ChchTF1-C, in the ratio ChchTF1-A : ChchTF1-B : ChchTF1-C 36:26:14, respectively.

The compositions of the invention can additionally comprise any excipient, adjuvant or vehicle appropriate to the agricultural sector, preferibly those that facilitate its application by common methods used in agriculture: spraying at ground level, aerial sprays, applications in solution, application as powders, via any type of watering or irrigation system, or as a solid.

The composition can be in any form, such as an aqueous form or a solid form. The composition can contain any other component, with special preference for those of agricultural interest. Thus, the single nucleopolyhedroviruses of *C. chalcites* can be mixed, for example, with a compost, fertilizer, pesticide or mixtures of these.

In the specific case of this possible embodiment, the composition of the invention could additionally comprise an insecticide based on the bacterium *Bacillus thuringiensis* including endospores, Cry protein crystals, or mixtures thereof.

Additional possible embodiments of the compositions of the present invention include those that comprise an agent that potentiates the pathogenic effect of the nucleopolyhedrovirus in the lepidopteran.

An additional aspect of the invention is the use of at least one of the nucleopolyhedroviruses of the invention, or a composition comprising at least one of them, as an insecticide. Preferably, the insect to be controlled should be *C. chalcites,* and preferably when it is in the larval or caterpillar stage. It is also preferred that the composition to be used as an insecticide comprises a mixture of genotypes ChchTF1-A (CNCM I-4690), ChchTF1-B (CNCM I-4620) and ChchTF1-C (CNCM I-4621), with particular preference for mixtures in which these genotypes are present in a ratio 36:26:14 of ChchTF1-A : ChchTF1-B : ChchTF1-C, respectively.

An additional aspect of the invention is a procedure for the production of the product object of the invention, i.e., a procedure for the production of occlusion bodies of the present invention that comprises a step wherein *C. chacites* larvae feed on an artificial diet containing *C. chalcites* nucleopolyhedrovirus occlusion bodies selected of the group of oclussion bodies that contain virions of any or all of the group of ChchSNPV of:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype characterized by a genome as depicted in SEQ ID NO: 17 (ChchTF1-A), SEQ ID NO: 18 (ChchTF1-B) or SEQ ID NO: 19 (ChchTF1-C),
or mixtures thereof.

Moreover, an additional aspect of the invention is a method for identifying the presence of *C. chalcites* single nucleopolyhedrovirus selected of the group of:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype characterized by a genome as depicted in SEQ ID NO: 17 (ChchTF1-A), SEQ ID NO: 18 (ChchTF1-B) or SEQ ID NO: 19 (ChchTF1-C)
in a sample, that comprises the following steps:
a) amplifying by PCR the DNA extracted from the sample, using a pair of primers selected from those formed by the oligonucleotides of:
   i) SEQ ID NO:1 (F-Hoar) and SEQ ID NO:2 (R-Hoar), or
   ii) SEQ ID NO:3 (F-Brod) and SEQ ID NO:4 (R-Brod);
b) analysing the amplified fragment to determine its length and sequence;
c) conclude that one of the ChchSNPV ChchTF1-A, ChchTF1-B, or ChchTF1-C is present if:
   i) the fragment amplified using the primer pair SEQ ID NO:1 and SEQ ID NO:2 has:
      a. a length of 756 (ChchTF1-A), 921 (ChchTF1-B) or 651 (ChchTF1-C) nucleotides, respectively; or
      b. the nucleotide sequence represented by SEQ ID NO:5 (ChchTF1-A), SEQ ID NO:6 (ChchTF1-B) or SEQ ID NO:7 (ChchTF1-C), respectively,
      or, alternatively,
   ii) the fragment amplified using the primer pair SEQ ID NO:3 and SEQ ID NO:4 has:
      a. a length of 1743 (ChchTF1-A), 962 (ChchTF1-B) or 1725 nucleotides (ChchTF1-C), respectively; or
      b. the nucleotide sequence represented by SEQ ID NO:8 (ChchTF1-A), SEQ ID NO:9 (ChchTF1-B) or SEQ ID NO:10 (ChchTF1-C), respectively.

The invention will now be explained in greater detail using the Figures and Examples that are given further on in the document.

### Brief description of the Figures

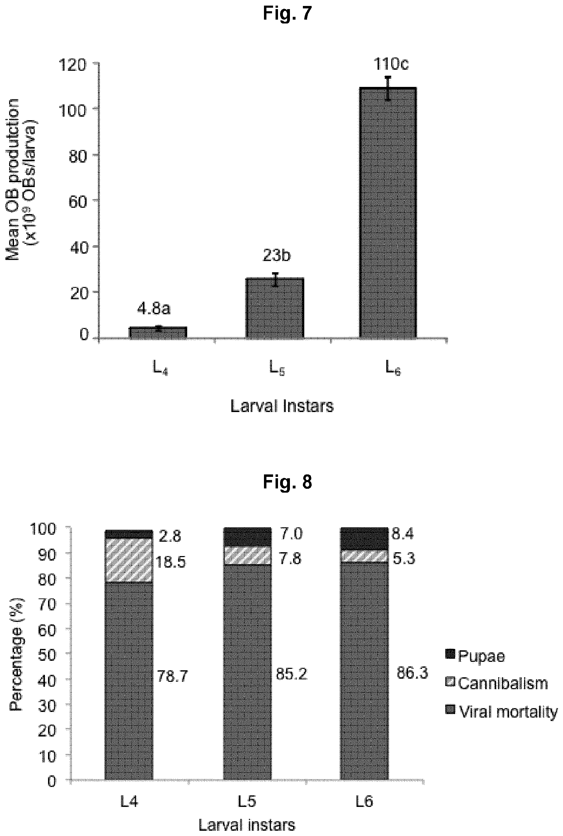

**Fig. 1****.** Schematic representation and electron microscope images of A): occlusion derived virions (left section of panel A) and budded virions (right section of panel A), and B): single type nucleopolyhedrovirus (SNPV), with virions of a single nucleocapsid (left section of panel B), and the multiple type (MNPV) with virions comprising multiple nucleocapsids (right section of panel B).

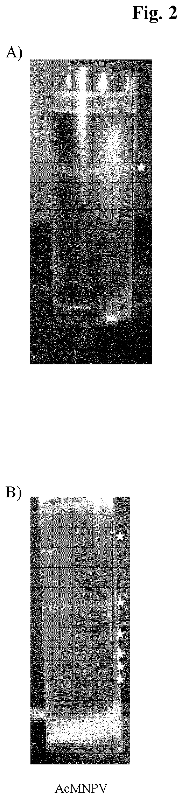

**Fig. 2****.** Banding pattern observed following continuous gradient centrifugation of virions obtained from ChchSNPV (panel A) and AcMNPV (panel B), MNPV type viruses. White asterisks indicate bands that represent ODVs. Panel A: a single visible band is observed, indicating that all virions have the same morphology and contain a single nucleocapsid. Panel B: various bands are observed each of which represents ODVs with a varying number of nucleocapsids; depending on virion density bands appear at a greater or lesser height.

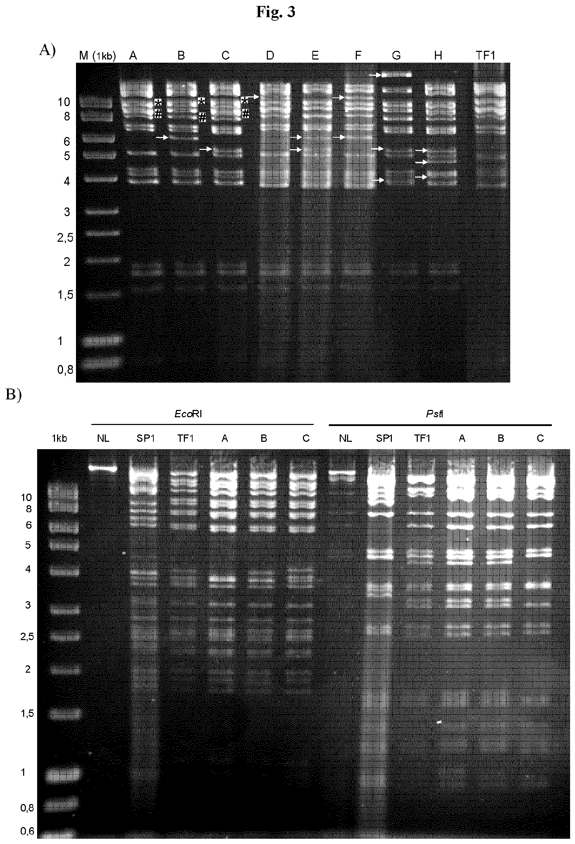

**Fig. 3****.** Restriction profiles of genomic DNA of the genotypes of isolate ChchSNPV-TF1 (abbriviated as ChchTF1) of *C. chalcites* nucleopolyhedrovirus from Tenerife (isolate abbreviated as TF1 in Figure):
   - Panel A: Image of electrophoresis gel of restriction fragments obtained following treatment of viral DNA of the genotypes of the wild-type isolate ChchTF1: ChchTF1-A (A), ChchTF1-B (B), ChchTF1-C (C), ChchTF1-D (D), ChchTF1-E (E), ChchTF1-F (F), ChchTF1-G (G) and ChchTF1-H (H) with the restriction enzyme *Bgl*II*.* Arrow (→) indicates the characteristic restriction fragments of each genotype. Asterisk (*) indicates the fragments on which the *hoar* gene is located. Hash (#) indicates restriction fragments on which the *bro-d* genes is located. The profile of the natural isolate ChchTF1 appears in the final lane. The 1kb (Stratagene) marker of fragment sizes in kilobases appears on the left of the figure.
   - Panel B: Image of electrophoresis gel of restriction fragments obtained using the restriction enzymes EcoRI (lanes to the left) or *Pst*I (lanes to the right) for isolates of C. *chalcites* nucleopolyhedrovirus ChchSNPV-NL (lanes marked NL) and ChchSNPV-SP1 (lanes marked SP1), ChchSNPV-TF1 (lanes marked TF1) and the genotypes of ChchTF1-A (lanes marked A), ChchTF1-B (lanes marked B), ChchTf1-C (lanes marked C).

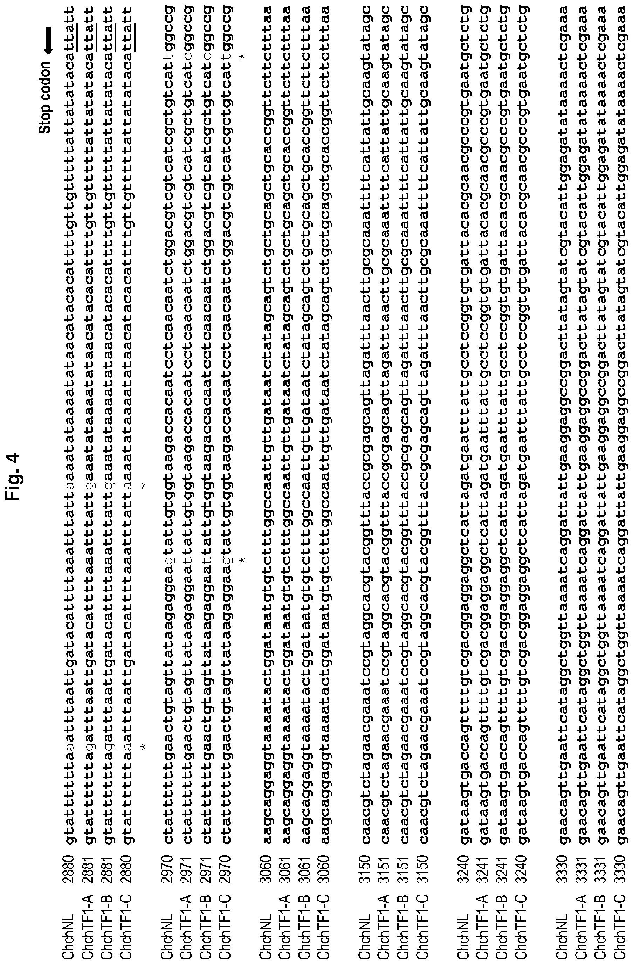

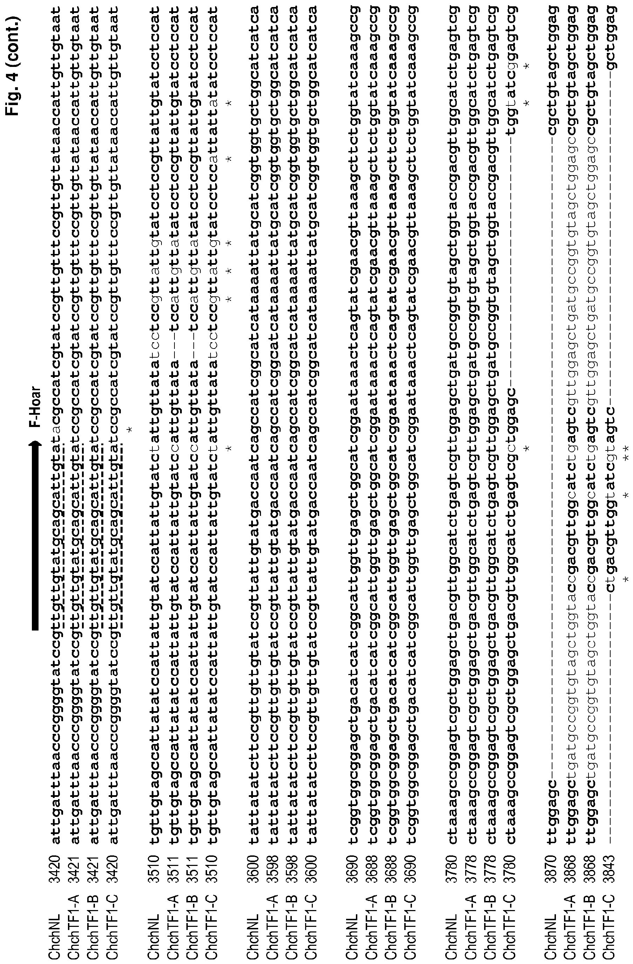

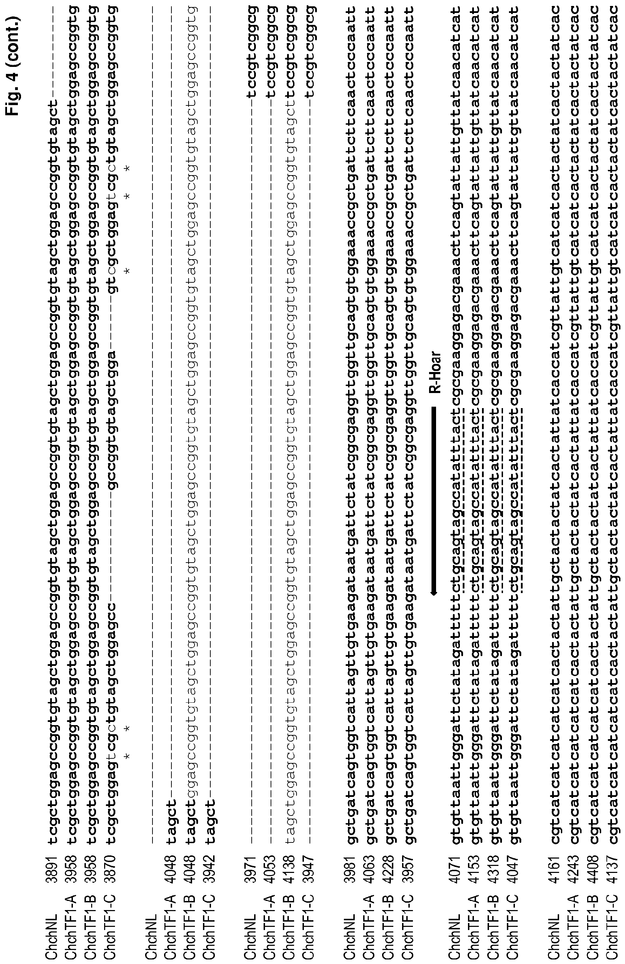

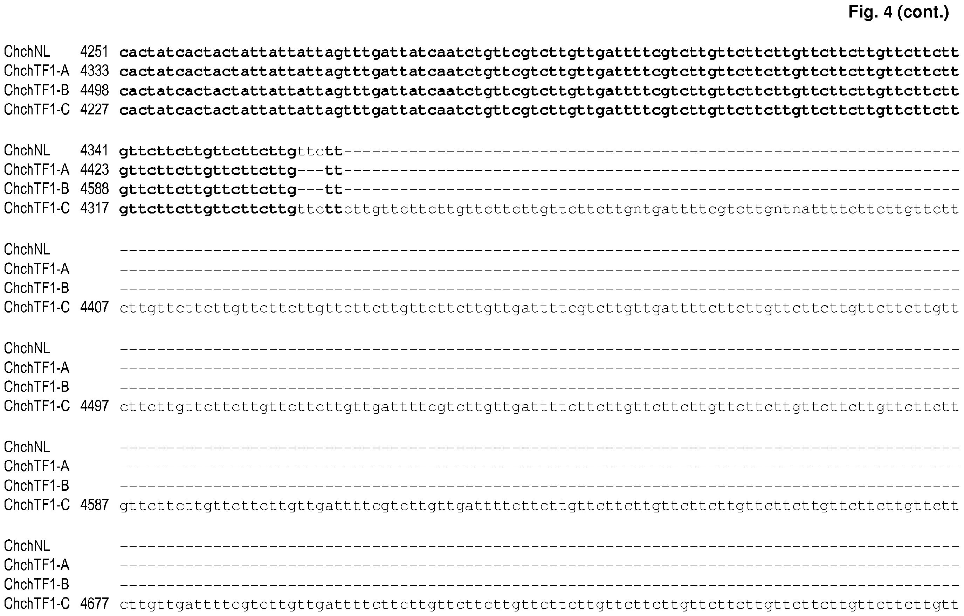

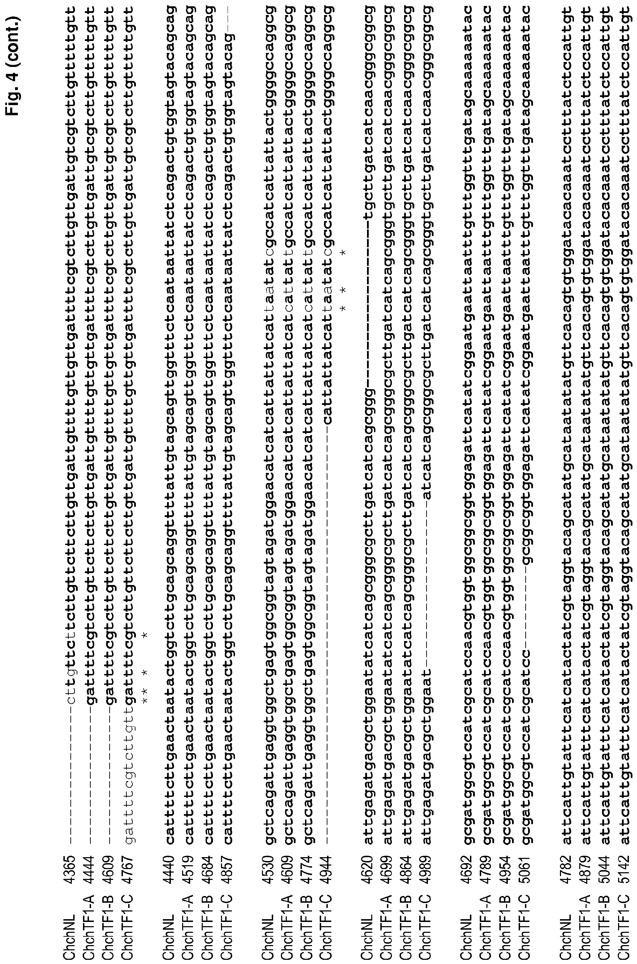

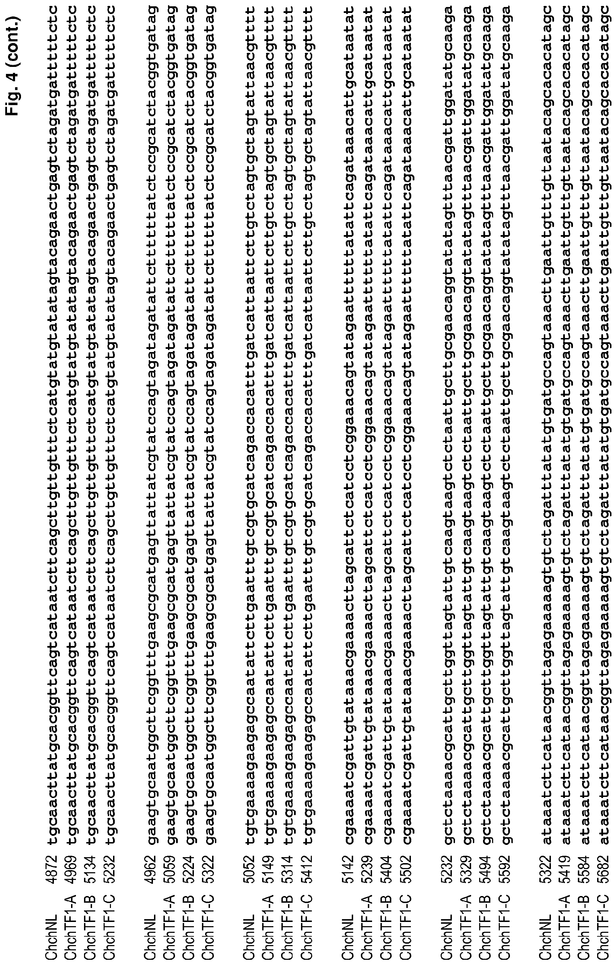

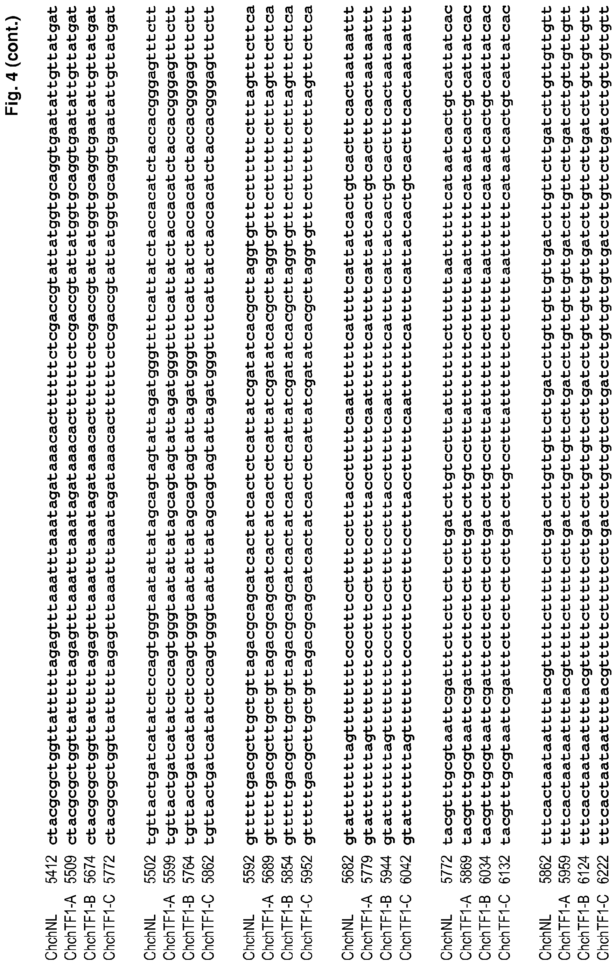

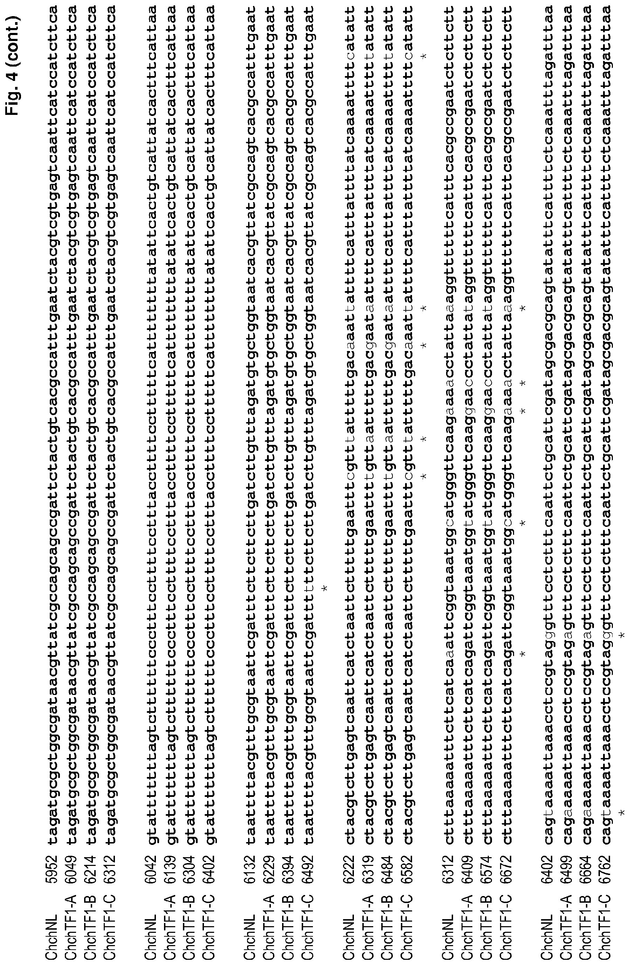

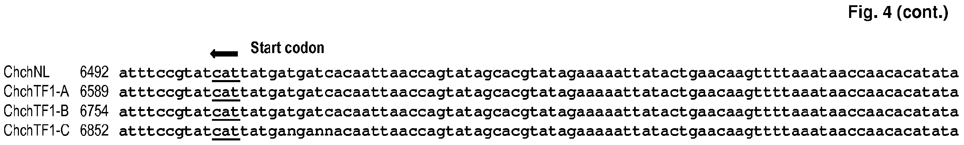

**Fig. 4****.** Alignment of nucleotide sequences of ORF4 (*chch4*: *hoar*) corresponding to the genomes of the *C. chalcites* nucleopolyhedrovirus isolate from The Netherlands (abbriviated as ChchNL), and the genotypes ChchTF1-A, ChchTF1-B and ChchTF1-C from the Tenerife isolate ChchSNPV-TF1. Shading indicates nucleotides that are common to all four genomes. In the same figure, the cat triplet, complementary to the start codon ("Start codon", nucleotides 6599-6601) and the stop codon ("Stop codon"), is highlighted with a box in the nucleotide sequence of genotype ChchTF1-A, and is the same for the different genotypes. Sites of primer hybridization used for PCR amplification of the region between these primers: F-Hoar (forward primer) and R-Hoar (reverse primer), are also shown.

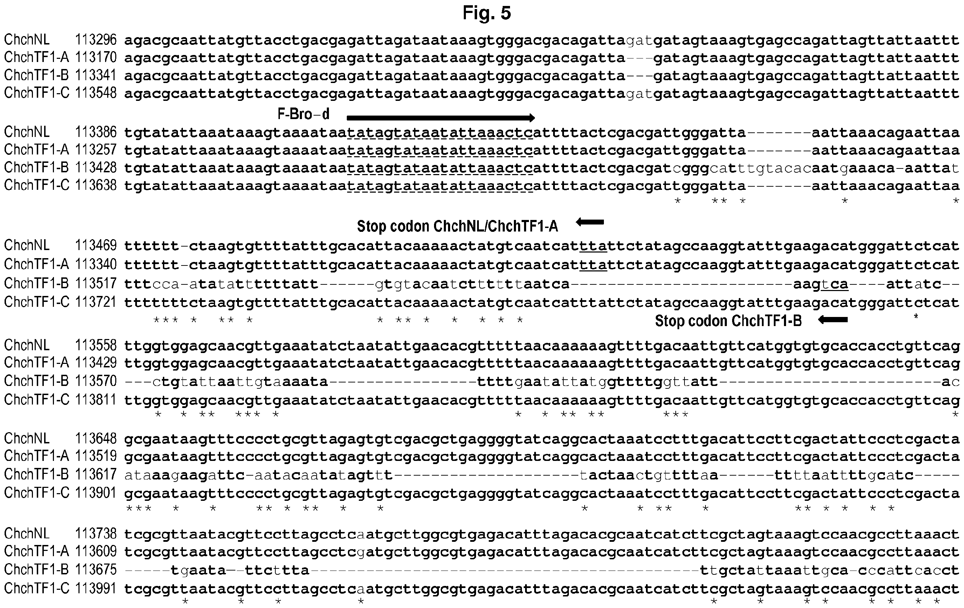

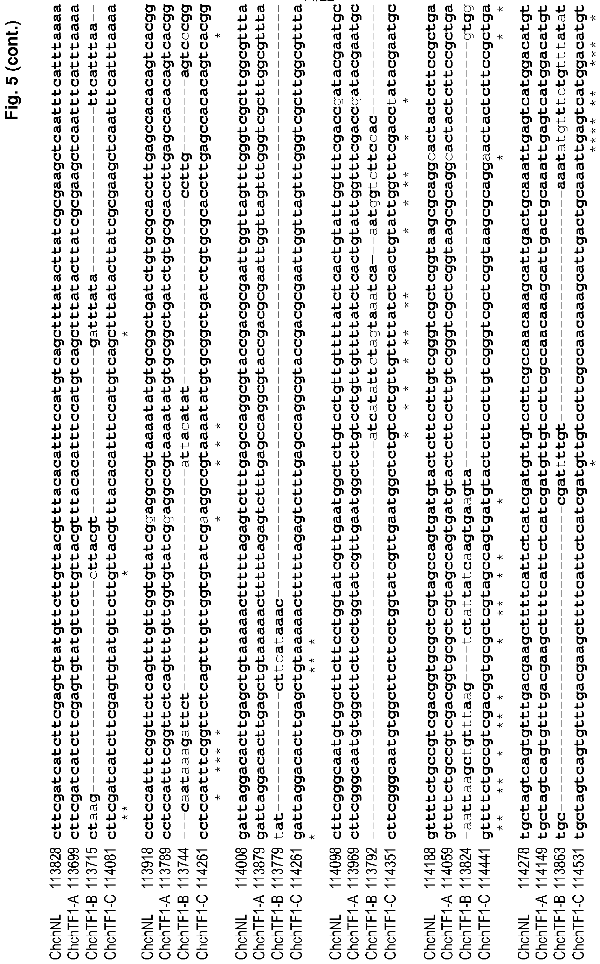

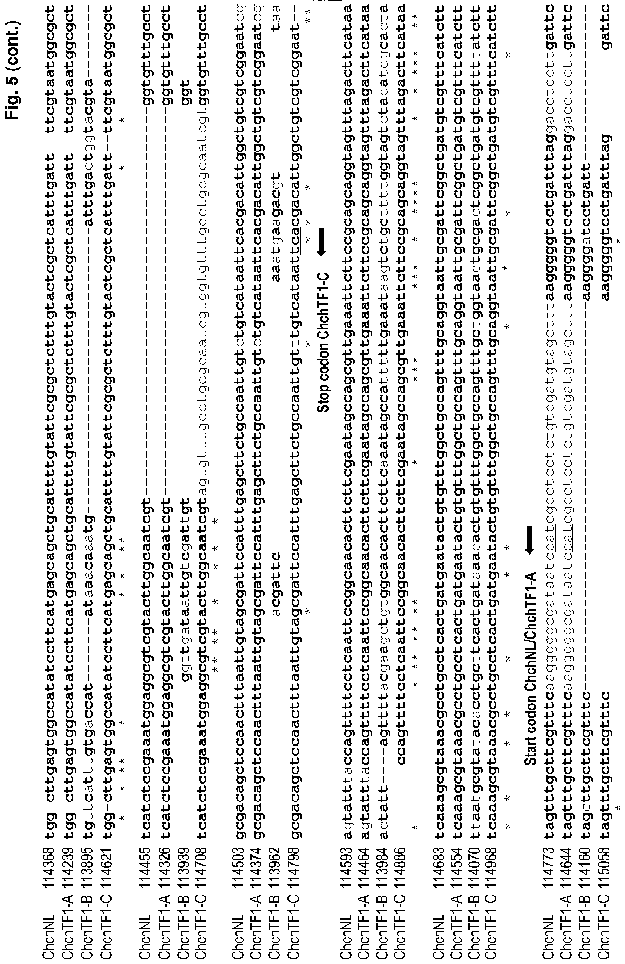

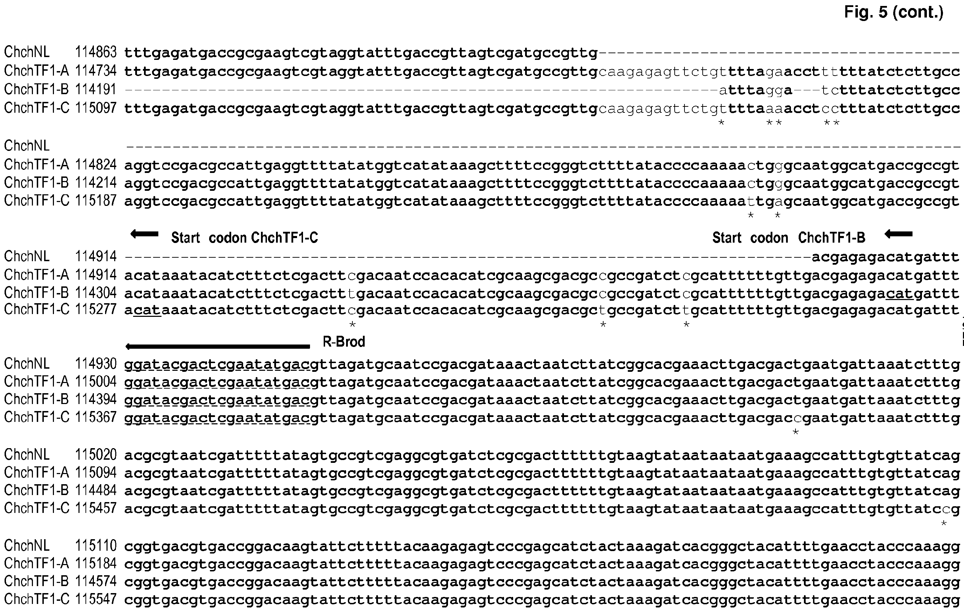

**Fig. 5****.** Alignment of nucleotide sequences of ORF114 (*chch114: bro-d*) corresponding to the genomes of the *C. chalcites* nucleopolyhedrovirus isolate from The Netherlands (abbreviated as ChchNL), and the genotypes ChchTF1-A, ChchTF1-B and ChchTF1-C from the Tenerife isolate ChchSNPV-TF1. In the same figure, the triplet complementary to the start codon ("Start codon") and the stop codon ("Stop codon"), are highlighted in boxes in the nucleotide sequence of the different genotypes. As shown in Fig. 5, the ChchTF1-C genome has a stop codon (TGA) which results in a shorter gene or encoded protein. Sites of primer hybridization used for PCR amplification of the region between these primers: F-Brod (forward primer) and R-Brod (reverse primer), are also shown.

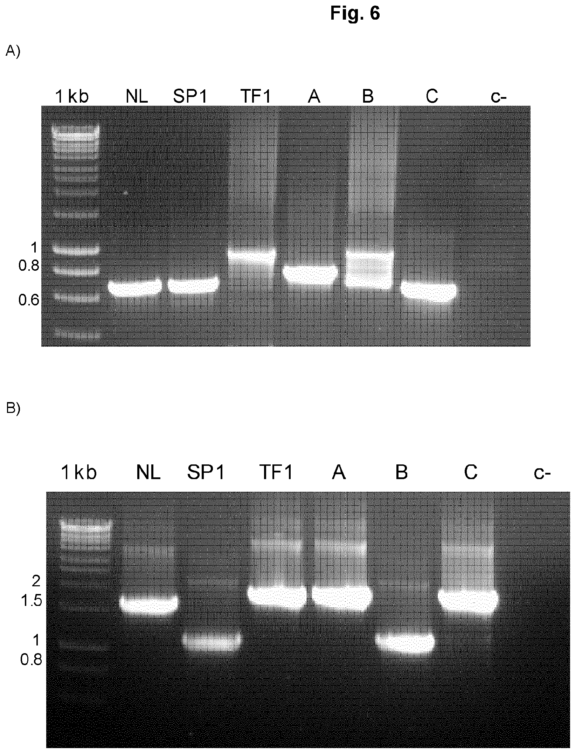

**Fig. 6****.** Fragments obtained by PCR amplification of variable zones of different genotypes of *C. chalcites* nucleopolyhedrovirus. Lanes: "1 kb": 1 kilobase marker (Stratagene); NL: Netherlands strain, ChchSNPV-NL; SP1: Almería strain, ChchSNPV-SP1; TF1: Tenerife strain, ChchSNPV-TF1; A, B, C: genotypes ChchTF1-A, ChchTF1-B and ChchTF1-C of the Tenerife isolate ChchSNPV-TF1; c-: negative control without viral DNA). Images present photographic evidence of agarose geles following electrophoresis of fragments obtained by PCR amplification of genomic DNA from the genotypes indicated above each lane, namely:
   - Panel A: amplification of a fragment of the *chch4 (hoar)* gene between the primer hybridization sites complimentary to SEQ ID NO:1 and SEQ ID NO:2
   - Panel B: amplification of a fragment of the *chch114 (bro-d)* gene between the primer hybridization sites complimentary to SEQ ID NO:3 y SEQ ID NO:4
**Fig. 7****.** Average production of occlusion bodies (x10⁹ OBs/larva) in *C. chalcites* in L4, L5 and L6 larvae reared at a density of 50 larvae/container. The columns of the figure indicate the values of the mean production of occlusion bodies. Numerical values labeled with different letters (a, b, c) indicate statistically significant differences between the values (p<0.05).
**Fig. 8****.** Percentages of cannibalism (area of columns filled with diagonal hatching, //), virus-killed larvae (area of columns filled with solid dark shading, 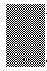 ) and larvae that have reached the pupal stage (upper area of columns filled with dotted shading, 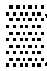 ) in *C. chalcites* L4, L5 and L6 larvae inoculated with the corresponding LC₉₀ (a concentration that results in 90% mortality in each group of different larval stages) of ChchSNPV and reared in groups of 50 larvae in 1.5 litre plastic containers.

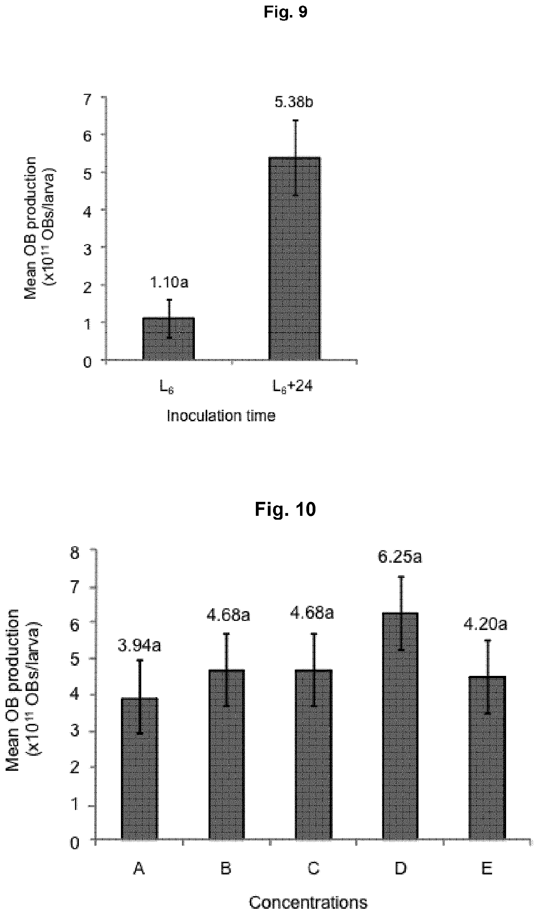

**Fig. 9****.** Average production of occlusion bodies (x10¹¹ OBs/larva) in recently-moulted sixth stage (L6) *C. chalcites* larvae, or at one day after moulting (L6+24). The columns in the figure indicate the values of the mean production of occlusion bodies. Numerical values labeled with different letters indicate statistically significant differences between the values (p<0.05).
**Fig. 10****.** Average production of occlusion bodies (x10¹¹ OBs/larva) in *C. chalcites* larvae L6+24 stage larvae inoculated with one of the following five concentrations ChchSNPV: 9.08x10⁸ OBs/ml (A), 6.86x10⁸ OBs/ml (B), 4.64x10⁸ OBs/ml (C), 2.42x10⁸ OBs/ml (D) and 2.05x10⁷ OBs/ml (E). The columns of the figura indicate the values of the mean production of occlusion bodies at each concentration. Numerical values labeled with identical letters ("a") indicate that values do not differ significantly from one another (p>0.05).

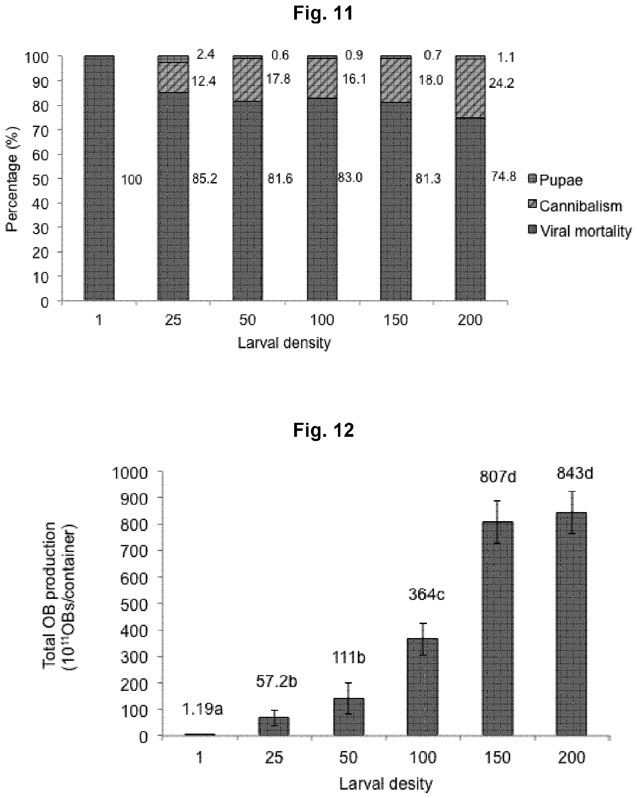

**Fig. 11****.** Percentages of cannibalism (area of columns filled with diagonal hatching, //), virus-killed larvae (area of columns filled with solid dark shading, 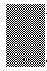 ) and larvae that have reached the pupal stage (upper area of columns filled with dotted shading, 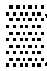 ) in larvae reared at densities of 1, 25, 50, 100, 150 and 200 larvae in 1.5 litre containers, as indicated beneath each column. Numbers within each area of the columns (or located above columns in the case of pupae) indicate the corresponding specific values. In all cases L6 stage larvae were inoculated 24 hours after moulting (L6+24) with the LC₉₀ (9.08x10⁸ OBs/ml).
**Fig. 12****.** Total production of occlusion bodies (x10¹¹ OBs/larva) obtained per 1.5 litre container as a function of the density of *C. chalcites* larvae (L6+24) inoculated with ChchSNPV. The columns of the figure indicate the values of the production of occlusion bodies for each of the larval densities studied. Numerical values labeled with different letters indicate statistically significant differences between the values (p<0.05).

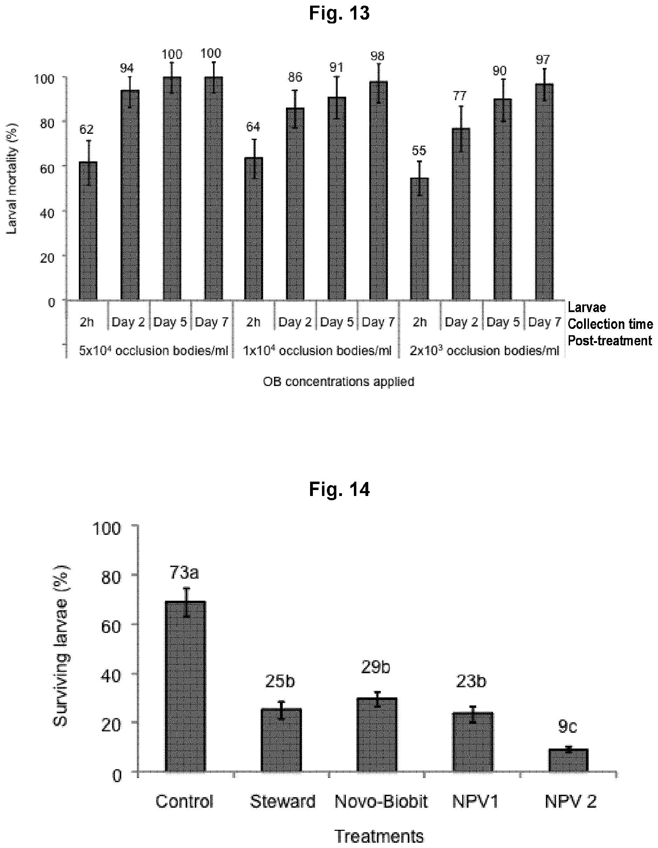

**Fig. 13****.** Percentage of mortality of second stage larvae of *C. chalcites* collected from tomato plants at different intervals following the application of the treatments indicated below the columns: two hours (2 h) or two, five or seven days post-application. Below each group of four columns the final concentrations of virus used in the laboratory experiment with tomato plants is given: 5x10⁴, 1x10⁴ and 2x10³ OBs/ml. The exact percentage of mortality values are shown above each corresponding column.
**Fig. 14****.** Percentage of survival of *C. chalcites* larvae on banana plants treated with one of the following products: water+Agral® (column labelled: Control, corresponding to the commercial product by Syngenta that comprises iso-tridecyl alcohol ethoxylate), the chemical insecticide indoxacarb (column labelled: Steward, corresponding to the product Steward by DuPont), a biological insecticide based on *Bacillus thuringiensis* (column labelled: Novo-Biobit, corresponding to the name of the commercial product used), *C. chalcites* nucleopolyhedrovirus at a concentration of 1x10⁵ OBs/ml (equivalent to 1x10¹¹ OBs/Ha) (column labelled: NPV1), *C. chalcites* nucleopolyhedrovirus at a concentration of 1x10⁶ OBs/ml (1x10¹² OBs/Ha) (column labelled: NPV2). Numerical values labeled with different letters (a, b, c) indicate statistically significant differences between the values (p<0.05).

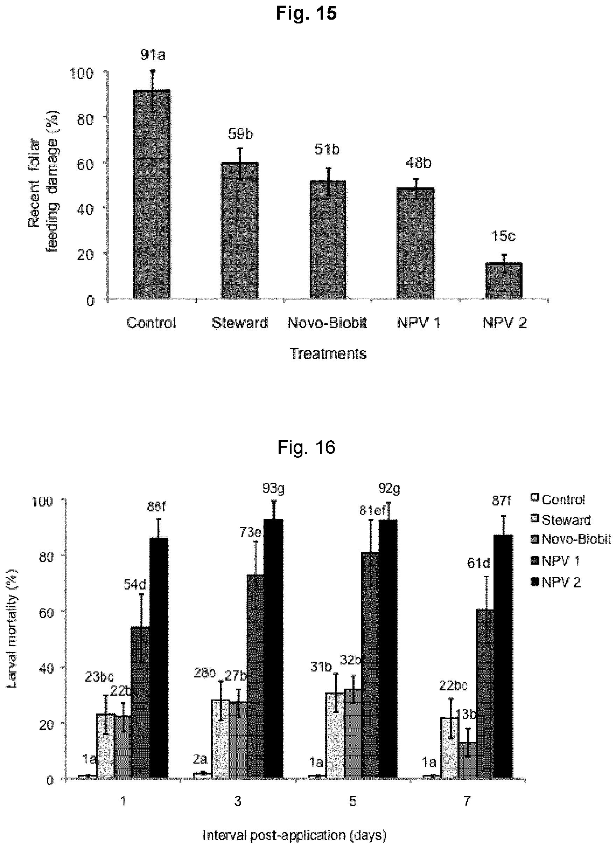

**Fig. 15****.** Percentage of recent damage of banana plants at 7 days following the application of the following products: water+Agral® (column labelled: control, corresponding to the commercial product by Syngenta that comprises iso-tridecyl alcohol ethoxylate), the chemical insecticide indoxacarb (column labelled: Steward, corresponding to the product by DuPont Steward), a biological insecticide based on *Bacillus thuringiensis* (column labelled: Novo-Biobit, corresponding to the name of the commercial product used), *C. chalcites* nucleopolyhedrovirus at a concentration of 1x10⁵ OBs/ml (equivalent to 1x10¹¹ OBs/Ha) (column labelled: NPV1), *C. chalcites* nucleopolyhedrovirus at a concentration of 1x10⁶ OBs/ml (1x10¹² OBs/Ha) (column labelled: NPV2). Numerical values labeled with different letters (a, b, c) indicate statistically significant differences between the values (p<0.05).
**Fig. 16****.** Percentage of mortality of *C. chalcites* larvae collected from banana plants as indicated beneath each group of 5 columns at 1, 3, 5, and 7 days following treatment of plants with one of the following products as indicated in the side key: control, Steward, Novo-Biobit, NPV1, NPV2 (as described in the legends to Fig. 14 and 15), indicated in the same order as shown in the columns of each group. Numerical values labeled with different letters (a, b, c, d, e, f, g) indicate statistically significant differences between the values for comparisons within each day of sampling (p<0.05).

### Detailed description of the invention

As mentioned previously, the invention is based on the isolation of a new *C*. *chalcites* single nucleopolyhedrovirus isolate originating from the Canary Islands, named ChchSNPV-TF1, and the discovery that this isolate comprised various genotypes of ChchSNPV, that differed from one another and that differed from known genotypes, ChchSNPV-NL and ChchSNPV-SP1.

Analysis of the natural isolate ChchSNPV-TF1 demonstrated that it comprised several different genotypes that were cloned and characterized; the restriction (endonuclease) profiles were obtained by digestion of genomic DNA for each of these genotypes.

Of the different genotypes, three were selected, ChchNPV-TF1-A (ChchSNPV-TF1-A), ChchNPV-TF1-B (ChchSNPV-TF1-B) and ChchNPV-TF1-C (ChchSNPV-TF1-C), also named ChchTF1-A, ChchTF1-B and ChchTF1-C, respectively, that can be readily distinguished from known genotypes of ChchSNPV (ChchSNPV-NL and ChchSNPV-SP1), by treating each genome with restriction enzymes (for eample *Bgl*II, *Eco*RI, or *Pst*I). The restriction profiles obtained for these novel genotypes and for the previously known genotypes ChchSNPV-NL (NL) and ChchSNPV-SP1 (SP1) are shown in Fig. 3A (restriction enzyme: *Bgl*II), and Fig. 3B (restriction enzymes: *Eco*RI (left side of image) and *Pst*I (lanes on right side of image)). Genotype differentiation is based on the presence of characteristic polymorphic fragments in the restriction profiles of each genotype or isolate.

The ability to differentiate between the genotypes ChchTF1-A, ChchTF1-B and ChchTF1-C and to differentiate them from the known isolates of ChchSNPV (ChchSNPV-NL and ChchSNPV-SP1) by examination of the profiles obtained using restriction enzymes, can also be clearly achieved with reference to the numerical values of the fragment lengths that comprise these profiles.

Consequently Table 2, provides the estimated lengths of the restriction fragments of the genomes of ChchSNPV-NL (NL) isolated in The Netherlands, ChchSNPV-SP1 (SP1) from Almería, Spain, and the cloned genotypes ChchTF1-A (A), ChchTF1-B (B) and ChchTF1-C (C) obtained following plaque purification from the natural isolate ChchSNPV-TF1 (also abbreviated as ChchTF1), generated following digestion with the enzyme B*gl*II. DNA fragments are named alphabetically, with fragment A being the largest in size:

**Table 2.- Estimated fragment sizes (kpb) of DNA generated following digestion of genomic DNA of the distinct isolates and genotypes using the BglII enzyme.**

| Fragments | ChchSNPV isolates | | ChchSNPV-TF1 genotypes | | | |
|---|---|---|---|---|---|---|
| | NL | SP1 | TF1 | A | B | C |
| A | 27.71 | 14.41 | 14.41 | 14.41 | 14.41 | 14.41 |
| B | 12.78 | 13.29 | 13.29 | 13.29 | 13.29 | 13.30 |
| C | **9.83*** | 12.79 | 12.79 | 12.79 | 12.79 | 12.78 |
| D | 9.76 | 11.90 | 12.12 | 12.12 | 12.78 | **10.19*** |
| E | 9.06 | **9.93*** | **9.93*** | **9.93*** | **10.19*** | 9.77 |
| F | 8.97 | 9.79 | 9.79 | 9.79 | 9.79 | 9.04 |
| G | **8.52#** | 9.60 | 8.97 | 8.97 | 9.04 | 8.96 |
| H | 8.00 | 9.06 | 8.93 | 8.93 | 8.93 | **8.72#** |
| I | 7.02 | **8.97#** | **8.72#** | **8.72#** | **8.20#** | 8.00 |
| J | 6.80 | 8.00 | 8.00 | 8.00 | 7.02 | 7.02 |
| K | 6.69 | 7.02 | 7.02 | 7.02 | 6.69 | 6.82 |
| L | 5.34 | 6.69 | 6.69 | 6.69 | 6.20 | 6.68 |
| M | 5.07 | 5.07 | 5.07 | 5.07 | 5.06 | 5.34 |
| N | 4.31 | 4.31 | 4.29 | 4.29 | 4.29 | 5.07 |
| O | 4.14 | 4.14 | 4.13 | 4.13 | 4.13 | 4.29 |
| P | 3.94 | 3.94 | 3.86 | 3.86 | 3.86 | 4.13 |
| Q | 3.84 | 3.84 | 3.84 | 3.84 | 3.84 | 3.88 |
| R | 1.87 | 1.87 | 1.87 | 1.87 | 1.87 | 3.84 |
| S | 1.75 | 1.75 | 1.75 | 1.75 | 1.75 | 1.87 |
| T | 1.75 | 1.75 | 1.75 | 1.75 | 1.75 | 1.75 |
| U | 1.53 | 1.53 | 1.53 | 1.53 | 1.53 | 1.75 |
| V | 0.78 | 0.78 | 0.78 | 0.78 | 0.78 | 1.53 |
| W | 0.15 | 0.15 | 0.15 | 0.15 | 0.74 | 0.78 |
| X | | | | | 0.15 | 0.15 |
| Total size (kb) | 149.61 | 150.58 | 149.68 | 150.58 | 149.08 | 150.07 |

| | | | | | | |
|---|---|---|---|---|---|---|
| Restriction fragments on which the *hoar* gene is located. # Restriction fragments on which the *bro-d* gene is located (indicated in Fig. 3A using the same symbol) The underlined fragment indicates the characteristic fragment(s) (indicated in Fig. 3A using an arrow →) | | | | | | |

As can be observed in Table 2, the restriction fragments obtained for ChchTF1 and genotype ChchTF1-A are identical, as ChchTF1-A is the dominant genotype in the ChchTF1 isolate, and has an identical restriction profile.

The column headed SP1 corresponds to the results obtained following *Bgl*II digestion of the genome of the Almerian isolate ChchSNPV-SP1, as in the second column of Table 1 that reflects the results obtained by Murillo and collaborators (Murillo et al., 2000). Note that the difference in the number of fragments is due to the more detailed analysis performed to generate the results given in Table 2. Comparison of the information obtained for genotypes ChchTF1-A, ChchTF1-B and ChchTf1-C with the information in Table 1 or Table 2, reveal that differences in fragments can be observed that reflect differences in the genomes, which indicate that ChchTF1-A, ChchTF1-B and ChchTf1-C differ from the known isolates and are therefore novel. In the case of the profile generated following *Bgl*II treatment of ChchTF1 and ChchTF1-A (the most similar to SP1), differences can be observed in at least 4 fragments whose size differs from one profile to another: namely fragments D, G, H and I. The fragments that are characteristic of genotypes ChchTF1-B and ChchTF1-C are indicated with an arrow in Fig. 3A.

Genotypes ChchTF1-A, ChchTF1-B and ChchTf1-C can also be differentiated from one another, or from other ChchSNPV isolates described in the literature, by the specific nucleotide sequences of each genotype or isolate in specific regions of the genome.

For example, the region in which the open reading frame number 4 of ChchSNPV (ORF4; *chch4;* gen *hoar*) is located, with reference to the genome sequence of the Netherlands isolate of ChchSNPV (ChchSNPV-NL) (van Oers et al., 2004, 2005), with GenBank accession number AY864330, can be used. The same applies to the región in which the *bro-d* is located (ORF114 of the ChchSNPV-NL genome, which is why the gene is also known as *chch114*).

The rapid and precise differentiation of each of these three genotypes can be achived using the PCR technique with specific primers that target, for example, one of the following alternative regions:
a) In the region of the *hoar* gene, particularly the region located between nucleotides 3,443 and 4,117 of the viral genome with GenBank accession number AY864330, that corresponds to a fragment located between nucleotides 3444 and 4199 of the genome of ChchTF1-A. In this region of the virus genome, it has been demonstrated that specific primers F-Hoar (5'-TTGTTGTATGCAGCATTGTA-3') (SEQ ID NO:1) and R-Hoar (5'-AGTAAATATGGCTACTGCAG3') (SEQ ID NO:2) amplify a fragment 675 nucleotides in length of the genotype from the Netherlands (ChchSNPV-NL), whereas the amplicon is of a length of 756, 921 and 651 nucleotides for genotypes ChchTF1-A, ChchTF1-B and ChchTF1-C, respectively.
b) In the region of the *bro-d* gene, specifically amplifying the region located between nucleotides 113,410 and 114,949 of the viral genome with GenBank accession number AY864330. In this region of the genome, it has been demonstrated that specific primers F-Brod (5'-TATAGTATAATATTAAACTC-3') (SEQ ID NO:3) and R-Brod (5'-GTCATATTCGAGTCGTATCC-3') (SEQ ID NO:4) can be used to amplify a fragment of 1540 nucleotides in the case of the genotype from The Netherlands (ChchSNPV-NL), whereas the amplicon has a length of 1743, 962 and 1725 nucleotides for the genotypes ChchTF1-A, ChchTF1-B and ChchTF1-C, respectively. Consequently, these primers can also be used to differentiate between the different genotypes resulting in different amplicon sizes and sequences in each case. However, as the fragment size is greater than that obtained with the *hoar*-specific primers the cost of amplicon sequencing will be greater. As such, the use of the *hoar*-specific primers is the preferred option.

Fig. 6, panels A and B present photographic evidence obtained following electrophoresis of fragments amplified by PCR of the mentioned *hoar* (panel a) or *bro-d* (panel b) gene fragments. It can been seen in these photographs that the fragments obtained for each genotype are different and distinguishable from one another. Due to the absence of the complete genome sequence of the Spanish isolate ChchSNPV-SP1 it was not possible to determine exactly the size of the fragments obtained, but a visual inspection of the fragments obtained following PCR amplification using specific primers for the *hoar gene* (Fig. 6A) or *bro-d* gene (Fig. 6B), indicated that the ChchSNPV-SP1 amplicon was similar to that of ChchNL in the case of the *hoar* gene, or to that of ChchTF1-B in the case of the *bro-d* gene, suggesting that the amplicon sizes were approximately 674 bp and 945 bp, respectively.

In this manner, a single PCR reaction can be used to differentiate between the distrinct genotypes with respect to any other genotype of this virus described in the scientific literature (see Tables 4 and 5 below, and Example 2).

In the case of natural isolates, or artificial mixtures that can comprise mixtures of genotypes, the proportion of the three genotypes ChchTF1-A, ChchTF1-B and ChchTF1-C in the mixture can be determined by semiquantitative PCR, which is based on a densitometric analysis of the PCR amplified fragment of each genotype, as described below in the materials and methods section (see Examples). In this case, the density of each band is proportional to the quantity of each of the genotypes present.

Similarly, determination of the sequence of these fragments allows their identification in artificial mixtures or as components of the natural isolate. Specifically the sequences represented by SEQ ID NO:5, SEQ ID NO:6 and SEQ ID NO:7 correspond to the sequences of the amplified fragments of the *chch4* (*hoar*) gene in genotypes ChchTF1-A, ChchTF1-B and ChchTF1-C, whereas SEQ ID NO:8, SEQ ID NO:9 and SEQ ID NO:10 represent the corresponding sequences of the amplified fragments of the *chch114* (*bro-d*) gene.

As mentioned below, the complete genome sequence has been obtained for each of the three genotypes ChchTF1-A, ChchTF1-B y ChchTF1-C, which are given as SEQ ID NO:17, SEQ ID NO:18 and SEQ ID NO:19, respectively, and which can be used to differentate these genotypes from others by comparing other regions. Specifically, due to its variability, the complementary sequences to the coding regions of the *chch4 (hoar)* gene (SEQ ID NO:11, SEQ ID NO:12, SEQ ID NO:13, corresponding to genotypes ChchTF1-A, ChchTF1-B and ChchTF1-C, respectively), and *chch114 (bro-d)* gene (SEQ ID NO:14, SEQ ID NO:15, SEQ ID NO:16, corresponding to genotypes ChchTF1-A, ChchTF1-B and ChchTF1-C, respectively). In the case of both the *chch4 (hoar)* and *chch114 (bro-d)* genes the sequences are shown in the sense in which they appear in the complete genome, that is to say, the complementary sequence to the coding sequence.

The fact that the complete genome sequence has been obtained for each of the genotypes ChchTF1-A (SEQ ID NO:17), ChchTF1-B (SEQ ID NO:18) and ChchTF1-C (SEQ ID NO:19), representing the definitive and characteristic feature of each of them, means that each of the genotypes has been described in the present document such that an expert could reproduce the invention. This is so, particularly given that the sequence of each genome is complemented by information that the nucleopolyhedrovirus of *Chrysodeixis chalcites* is an SNPV. As such, each complete particle or virion contains a single nucleocapsid which implies that each complete particle or virion contains a single copy of the nucleopolyhedrovirus genome. Additional information is also provided on the genotypes, such as the profile obtained with distrinct restriction enzymes, or the sequence or length of the fragments obtained by amplification of a variable region of the *hoar* or *bro-d* genes using, primer pairs SEQ ID NO:1 and SEQ ID NO:2 or SEQ ID NO:3 and SEQ ID NO:4, respectively. Additionally, in the Examples section, information is given on the insecticidal activity of each genotype alone and in mixtures, and the possible differences in insecticidal activity between occlusion bodies that contain co-occluded virions of distrinct genotypes and occlusion bodies that contain virions of a single genotype, in addition to the method of producing such occlusion bodies. Differences in insecticidal activity are not statistically significantly different (P>0.05) between genotypes but do differ statistically with respect to the natural isolate, or mixtures of the genotypes of the invention, in which the pure single genotypes have statistically lower insecticidal activity.

Moreover, information is provided on the deposit of the theree genotypes accoring to the terms of the Budapest Treaty under the following deposit reference numbers CNCM I-4690 (ChchTF1-A), CNCM I-4620 (ChchTF1-B), CNCM I-4621 (ChchTF1-C).

With respect to the possible applications of the nucleopolyhedrovirus of the present invention, it has been shown that the each of the novel genotypes ChchTF1-A, ChchTF1-B and ChchTF1-C (A, B, C) have a specific insecticidal activity in *C. chalcites* larvae that can be considered comparable to that of chemical insecticides, or biological type insecticides, based on the properties of *Bacillus thuringiensis* commonly used to control *C. chalcites.* In addition, it has been determined that the mixture of the three genotypes ChchTF1-ABC in a specific ratio (36:26:14), in the form of occlusion bodies that contain co-occluded ODVs, such that a single occlusion body can contain different genotypes of ChchSNPV-TF1, possess an insecticidal activity that is significantly higher than any of the component individual genotypes, or any of the natural isolates of ChchSNPV already known. This is evidenced by a significant reduction in the LC₅₀ value of the three-genotype mixture, which indicates that a lower quantity of inoculum is required to kill the pest (which implies reduced costs), in addition to a 33 hour reduction in the mean time to death (MTD) of infected larvae.

This indicates a significant advantage given that one of the principal disadvantages of baculovirus-based bioinsecticides is the generally slow speed of kill, which tends to be slower than that of a chemical insecticide. Also, this virus is readily produced by inoculation of groups of 150 larvae at 24 h following moulting to the sixth stage (L6+24), which results in the production of approximately 8.07x10¹³ occlusion bodies.

In addition, assays using tomato plants in the laboratory and banana plants in the field, have revealed that concentrations of the nucleopolyhedrovirus of the invention in the order of 5x10¹⁰ OBs/Ha (5x10⁷ OBs/litre of spray) on tomato and 10¹² OBs/Ha (10⁹ OBs/litre of spray) on banana plants, are capable of controlling infestations of larvae of this pest. Virus applications were even more effective than the chemical insecticide Steward®, that contains the active ingredient indoxacarb: (S)-methyl-7-chloro-2,5-dihydro-2-[[(methoxy-carbonyl) [4(trifluoromethoxy) phenyl]amino]-carbonyl]indane[1,2-e][1,3,4]oxadiazyn-4A-(3H)-carboxylate) and other biological insecticides (Novo-Biobit™; based on *Bacillus thuringiensis* var. *kurstaki*) commonly used in banana plantations. Based on the production of occlusion bodies and the most effective concentration for control of *C. chalcites* in each crop, it should be possible to protect approximately 1614 Ha of tomato plants or 80 Ha of banana plants using the occlusion bodies obtained from a container of 150 *C. chalcites* L6+24 larvae (see Example 4).

These results demonstrate that the mixtures of these three cloned genotypes (ChchTF1-A, ChchTF1-B, ChchTF1-C) are among the most potent nucleopolyhedroviruses that have been developed as bioinsecticides to date. Moreover, as the host range of baculoviruses is restricted to invertebrates, and due to the high specificity of ChchSNPV in particular (that only affects a few species of moths of the Noctuidae family all of which are closely related phylogenetically), means that this is a clean and safe technology which does not leave toxic residues in crop products and is not toxic to man, other animals or insect natural enemies such as parasitoids or predators.

The nucleopolyhedroviruses have the additional advantage of their ease of production and the high yields that can be obtained. The occlusion bodies that habour the insecticidal activity can be mass-produced *in vivo* by inoculating *C. chalcites* larvae with occlusion bodies obtained beforehand. The occlusion bodies can be used to contaminate the artificial diet used to feed the insects. The occlusion bodies will contain virions of any of the genotypes ChchTF1-A, ChchTF1-B and ChchTF1-C, or their mixtures, depending on whether occlusion bodies are desired that contain a single genotype, or virions of different genotypes that are co-occluded in the same occlusion body.

The specific procedure used to obtain the new occlusion bodies can the the same as that described in the Examples of the present invention, that are described below. An example of the specific artificial diet that is compatible with the procedure for the production of occlusion bodies of this invention is also described. For example, the procedure for the production of occlusion bodies can comprise the following stages:
a) feeding *Chysodeixis chalcites* larvae on an artificial diet with *C. chalcites* nucleopolyhedrovirus occlusion bodies that contain virions of any of the genotypes ChchTF1-A (CNCM I-4690), ChchTF1-B (CNCM I-4620) and ChchTF1-C (CNCM I-4621), or mixtures thereof;
b) rearing larvae at a temperature of 25-28ºC until death;
c) purifying the occlusion bodies produced in the infected larvae by triturating the cadavers in water, filtering the resulting suspension, sedimenting the occlusion bodies, washing them and sedimenting them one more;
d) resuspend the sedimented pellet in water at neutral pH;
e) optionally, the resulting suspension can be stored under one of the following conditions:
   i. at environmental temperature
   ii. in refrigeration (between 0ºC and 4ºC)
   iii. freeze-drying the suspension and storing it at room temperature.

The artificial diet can be administered in the form of a liquid, such as an aqueous solution, optionally with 10% sucrose in which the occlusion bodies are suspended and which preferably also contains a colorant that indicates those larvae that have ingested the diet, such as the blue food dye Fluorella blue. In this case the suspension is preferably administered as droplets that the larvae drink, following the droplet-feeding method described by Hughes and Wood (Hughes and Wood, 1986).

The artificial diet used to administer occlusion bodies to larvae can also be a solid, in the form of plugs or pieces that contain *Chrysodeixis chalcites* nucleopolyhedrovirus occlusion bodies and the following ingredients: 5% wheatgerm, 5% soya flour, 5% brewer's yeast, 1.5% agar, 1.3% sugar, 1% mixed salts, 0.5% cellulose, 0.45% ascorbic acid, 0.4% sorbic acid, 0.18% nipagin, 0.18% benzoic acid, 0.14% wheatgerm oil, 0.094% choline chloride, 0.05% chloramphenicol, 0.009% vitamins and 80% distilled water. Both methods of administering the diet can be combined such that the occlusion bodies can first be administered in the droplets of an aqueous suspension and susbsequently inoculated larvae are permitted access to solid artificial diet in the form of plugs that can be of different shapes, such as a cube.

The assays decribed in Example 4 of the present document, demonstrate that different variables can be modified in the occlusion body production procedure of the invention. There exist different means of achieving the production procedure, although the preferred conditions tend to produce the best results. Specifically, evidence is presented to show that the procedure can be applied for the production of occlusion bodies under the following conditions:
- *C. chalcites* larvae of stages/instars 4 (L4), 5 (L5) and 6 (L6), although sixth stage larvae are preferred;
- L6 larvae recently moulted or larvae treated with the virus one day (24 hours) after moulting (L6+24 larvae); this second alternative results in an increase in the production of occlusion bodies per larva of approximately 5-fold compared to the use of larvae at other stages of development;
- different concentrations of occlusion bodies (OBs) are administered in artificial diet, as demonstrated in assays with different concentrations in the range 2.00x10⁷ to 1.00x10⁹ OBs/ml, although the use of L6+24 larvae inoculated with a concentration of 9.08x10⁸ OBs/ml is preferred;
- different densities of larvae, in a single container, that can vary from 1 to 200 larvae in each 1.5 litre capacity container, as shown in Example 4. The highest production ocurrs at densities of 150-200 larvae, with 150 larvae in each 1.5 litre container being the preferred density, as it reduces the need for starting materials.

The study of the distinct larval densities per container, and the prevalence of acts of cannibalism that occur, demonstrate that the optimal production of occlusion bodies is obtained when groups of 150 sixth stage larvae, that moulted to the sixth stage 24 hours prior to their inoculation, are reared in a 1.5 litre capacity plastic container, having been inoculated with a concentration of occlusion bodies that results in approximately 90% of lethal infection (LC₉₀) in the larvae of this stage, which in this case is a concentration of 9.08x10⁸ OBs/ml. As a result, the occlusion bodies produced in a 1.5 litre plastic container represent sufficient active material to treat 1614 Ha of tomato plants or 81 Ha of banana plants.

The occlusion bodies produced in larvae can be extracted, formulated in solid or liquid (e.g., aqueous) and sprayed as aqueous suspensions, which very effectively protect tomato and banana crops from infestions of *C. chalcites* pest larvae.

The occlusion body compositions can also be applied by other different methods, such as ground spraying, aerial spraying, application in suspension, application as powder, or during watering or irrigation. As mentioned previously, the occlusion bodies can be mixed with excipients and this mixture can be used with suitable adjuvants or vehicles for the agricultural sector, especially those that aid in the preparation of the composition to be applied in a form suitable for the application method that is desired. The same composition can also include for example, a compost, a fertilizer or a pesticide. The composition can also comprise a agent that potentiates the pathogenic effect of the nucleopolyhedrovirus on *C. chalcites.*

Of the compounds or products that can be used in mixtures with occlusion bodies, the use of wetting agents is of particular interest, such is the case of the commercial product Agral®, commercialized by Syngenta, that is used in the Examples of the present document. The chacteristics of this product are described in the web page (http://www.syngenta.com/country/es/sp/productos/proteccion cultivos/mojantes/Pagin as/agral.aspx). The product is a non-ionic tensoactive wetter dispersant which is designed to be mixed with all clases of insecticides, fungicides and other agrochemicals. The wetting agent present in the product is iso-tridecyl alcohol ethoxylate, a non-ionic tenoactive substance that improves the biological action of insecticides, herbicides, fungicides and pesticides in general, as it improves crop coverage and penetration. Among other applications, the product is designed to be employed in spray applications on waxy leaf surfaces that are difficult to wet, among which are the principal crops attacked by *C. chalcites,* such as banana plants, and treatments targeted at other caterpillar pests.

Another case of particular interest is that of a composition that contains another pesticide, which results in an increase in the spectrum of action to include other pests present on the crop, that not restricted to *C. chalcites* and other closely related noctuids, such as *Trichoplusia ni.* The pesticide may be, for example, another biological insecticide, such as those based on *Bacillus thuringiensis* (Bt) previously mentioned in this document, such as the product Novo-Biobit® that was employed in Example 5, further on in the present document, that is used to control infestations of *C. chalcites* on crops. The mixture with Bt-based insecticides provides a particularly interesting case, as synergistic interactions have been described in the insecticidal activity of these products and baculoviruses that attack noctuids such as *Trichoplusia ni* (Granados et al., 2001).

Moreoever, the assays described in the Examples section described below show that each of the three genotypes possess a characteristic insecticidal activity against larvae of *C. chalcites,* which is determined as the statistical relationship between the number of virus particles (occlusion bodies) and the mortality that ocurrs in treated larvae, which can be expressed as the lethal concentration (e.g., LC₅₀).

In previous studies by the research group of the present inventors, it has been observed that on certain ocasions mixtures of occlusion bodies or co-occluded mixtures of virions possess an insecticidal activity that differs from that of mixtures of occlusion bodies each of a single genotype alone (López-Ferber et al., 2003; Simón et al., 2005) or of the natural isolate (Muñoz et al., 1998). Similarly, the co-occluded mixtures that contain distinct genotypes occluded in the same occlusion body can have an insecticidal activity that differs from the activity of mixtures of occlusion bodies of single genotypes alone (López-Ferber et al., 2003). This is because certain genotypes can have synergistic or antagonistic activities. In the present invention, the study of the insecticidal activity of mixtures has been performed to determine whther they possess activities that differ from occlusion bodies of a single genotype, whether or not differences exist that indicate antagonisms or synergisms according to different mixtures, and the variations that could exist between the different combinations and proportions thereof.

The mixture of the three genotypes ChchTF1-ABC in the ratio 36:26:14, in the form of ODVs of distinct genotypes co-ocluded in the same occlusion body, possess an insecticidal activity significantly greater, in terms of pathogenicity and virulence, than any of the individual component genotypes, and greater than any other mixture of genotypes or mixtures of the natural isolates that are currently known. For that reason, these genotypes were selected for application in the form of co-occluded genotypes within the same occlusion body, compared to other genotypes present in the same natural isolate ChchSNPV-TF1, or in other isolates obtained from the same region.

The assays of insecticidal activity described in Example 3 of the present invention demonstrate that the novel nucleopolyhedrovirus isolates are among the most active biological insecticides for control of insect pests. Consequently, their use as insecticides is proposed, particularly for the control of *C. chalcites,* but also for control of other noctuids that are closely related to this moth, in which ChchSNPV has previously been shown to have insecticidal activity, such as *Trichoplusia ni* or *Plusia gamma.* Insect pests can be controlled on any plant that is damaged by the attack of these insects, whether grown or cultivated in greenhouses or open fields, particularly in the case of tomato, pepper and banana crops. In this last case, the possible application of the invention in the place of origen of the isolate, the Canary Islands, where its efficacy has been demonstrated, deserves special consideration.

Taking into account the aforementioned information, the following statements can be made:
- each of the new genotypes isolated, ChchTF1-A, ChchTF1-B and ChchTF1-C (A, B, C) is novel, as each of the genotypes is distinct from other genotypes, and different from the *C. chalcites* nucleopolyhedroviruses described to date, ChchSNPV-NL and ChchSNPV-SP1, which can be distinguished by the differences in the genomic sequences (particularly in the regions of the *hoar* and *bro-d* genes), and differences in the profiles generated by digestion of the genomes with restriction enzymes, in particular *Bgl*II, *Eco*RI and/or *Pst*I.
- the three novel genotypes share the following technical characteristics:
   - they were all obtained from a natural isolate, ChchSNPV-TF1, indicating that all three exist together in nature in the same genographical region in which crops are affected by infestations of *C. chalcites;*
   - their insecticidal activity, of each one is lower or equal to that of any of the natural isolates known previously;
   - a synergistic interaction ocurrs between when they are present in mixtures, in particular the mixture of the three genotypes together, especially when co-occluded, and particularly in co-occluded mixture A:B:C in the ratio 36:26:14, in which their insecticidal activity against *C*. *chalcites* larvae is greater than that of the natural isolates of this virus and comparable to that of other insecticdes that are used to control this pest, such as the commercial chemical-type insecticide, commercialized under the name Steward®, or the commercial Bt-based insecticide known as Novo-Biobit®,
   - given that the three genotypes were isolated in the same geographical zone and from the same natural isolate, they may be expected to be highly active against populations of *C. chalcites* from the same geographical region, the Canary Islands, and particularly those of the island of Tenerife,
   - the high insecticidal capacity, especially of the co-occluded mixture of genotypes A:B:C was not expected; nor was it expected that the ratio of genotypes of 36:26:14 would prove to have an outstanding insecticidal activity in terms of both pathogenicity and virulence.

### Examples

The assays that are described in the Examples section below were performed using the following materials and techniques:

### - Insects

The *C. chalcites* larvae used to produce the virus were obtained from a laboratory-reared colony maintained at the Universidad Pública de Navarra (Spain). The colony was established in 2007 using pupae received from the Instituto Canario de Investigaciones Agrarias (ICIA), Tenerife, Spain, and was periodically refreshed with pupae from the Canary Islands. The larvae were reared at a temperature of 25ºC, 70±5% relative humidity, and a photoperiod of 16:8 (light:dark), on a semisynthetic diet based on wheatgerm, yeast and soya described by Cabello et al. (1984). The adults fed *ad libitum* on 30% (weight/volume) honey solution.

### - Isolation and production of occlusion bodies

Occlusion bodies (OBs) were extracted from dead larvae by homogenizing cadavers in water and purifying them by filtration and differential centrifugation (Caballero et al., 1992). The OBs were then suspended in double-distilled water and their concentration was determined by counting samples in triplicate using a Neubauer haemocytometer (Hawksley, Lancing, United Kingdom) using a phase contrast microscope at x400 magnification.

Occlusion bodies of the different field-collected isolates were produced by a single passage in fourth stage *C. chalcites* larvae. Groups of 25 larvae from the laboratory colony were held overnight without access to food. They were then allowed to drink a suspension of occlusion bodies (10⁶ OBs/ml), obtained from field-collected infected insects, following the droplet feeding method (Hughes y Wood, 1981). This method involves the *per os* infection of larvae that have been starved for a short period (∼8 h). The great advantage of this method is that the dose or concentration of the virus is ingested in a very brief period of time, which is particularly important when calculating parameters such as the time-mortality relationship. Moreover, using a food colouring agent (Fluorella Blue, Hilton-Davis, Cincinati, Ohio) in the occlusion body suspension, allows the identification of those larvae that have ingested the dose. Larvae that drank the suspension were reared individually on semisynthetic diet until death.

Purified OBs from the laboratory-reared larvae were stored at 4ºC for up to one month prior to use in molecular or biological characterization assays.

### - DNA extraction and analysis using restriction endonucleases

To extract DNA, virions were released from occlusion bodies by mixing an OB suspension containing 10⁹ OBs/ml with 100 µl of 0.5 M sodium carbonate (Na₂CO₃) and 50 µl of 10% (weight/volume) sodium dodecyl sulphate (SDS) in a final volume of 500 µl followed by inclubation at 60ºC for 10 minutes. Undissolved OBs and other debris were removed by low speed centrifugation (3800 *x g*, 5 min). The supernatant containing virions was treated with 25 µl of 20 mg/ml proteinase K for 1 hour at 50ºC. Viral DNA was extracted twice with saturated phenol and once with chloroform, and was precipitated by addition of alcohol to the aqueous phase. The precipitate was resuspended in 50 to 100 µl of x0.1 TE buffer (Tris-EDTA, pH 8) at 60ºC for 10 minutes. The DNA concentration was estimated by measuring optical absorbance at 260 nm.

For restriction endonuclease analysis, 2 µg of viral DNA was mixed with 10 U of one of the following enzymes: *Bam*HI, *Bgl*II, *Pst*I (Takara Bio Inc., Japan) and incubated for 4 to 12 hours at 37ºC. Reactions were stopped by addition of 4 µl of loading buffer (0.25% [weight/volume] bromophenol blue, 40% [weight/volume] sucrose). Electrophoresis was performed in 1% agarose horizontal gels under TAE buffer (0.04M Tris-acetate, 0.001 M EDTA, pH 8.0) at 20 V during 10 to 24 hours. DNA fragments were stained with ethidium bromide and visualized on an ultraviolet transiluminator (Chemi-Doc, BioRad, California, USA).

### - Nucleocapsid packing

To determine whether the ODV virions of the Canary Islands' *C. chalcites* nucleopolyhedrovirus isolates were single or multiple type, ODVs were released from 5x10⁸ occlusion bodies by treatment with alcaline buffer (0.1 M Na₂CO₃) at 28ºC for 30 minutes. Polyhedrin and other debris were removed by low speed centrifugation (2500 x g, 2 minutes). Supernatant containing virions was separated into layers by centrifugation (90000 x *g,* 1 h) through a continuous sucrose gradient (30-60% weight/volume). The resulting bands were inspected visually and photographed.

### - Complete genome sequencing

To completely sequence the genomes of the three genotypes ChchTF1-A, ChchTF1-B and ChchTF1-C, DNA was purified by caesium chloride (CICs) centrifugation (King and Possee, 1997). Initially, ODVs were released and purified as decribed in the section on nucleocapsid packaging. For this, 500 µl of occlusion body suspension (10⁹ OBs/ml) was mixed with an identical volume (500 µl) of 0.1 M sodium carbonate (Na₂CO₃), and following continuous sucrose gradient centrifugation a single band was obtained for each of the three genotypes. A 1 ml syringe and needle was used to pierce the centrifige tube at the level of the band of ODVs, which was entirely removed into the syringe. These virions were diluted 1:3 in TE buffer (Tris-EDTA pH 8) and sedimented by centrifugation at 24000 rpm for 1 hour. Virions were concentrated by resuspension in 400 µl of 1x TE buffer. For DNA extraction, 400 µl of purified virion suspension was mixed with 100 µl of 20% sarcosyl (sodium lauroyl sarcosinate or sodium N-lauroylsarcosinate solution, Sigma) and incubated for 30 minutes at 60ºC. This resulted in the lysis of the virions and rupture of the nucleocapsids releasing DNA into solution. The lysate was immediately treansferred to 5 ml of 50% (weight/weight) caesium chloride solution in TE buffer which also contained 12.5 µl of 10 mg/ml ethidium bromide, that stained DNA and permited its visualization. Samples were centrifuged at 35000 rpm in a Beckman SW41 rotor for a minimum of 18 h at 20ºC.

Following centrifugation DNA was visualized as two orange-coloured layers (due to ethidium bromide staining). These bands corresponded to supercoiled DNA (lower band) and open circular DNA (upper band). A 1 ml syringe and needle was used to pierce the tube and extract both layers. Following extraction, ethidium bromide was removed by repeated washing with butanol. For this each sample was mixed with an equal volume of butanol, centrifuged, and the phase containing butanol and ethidium bromide was eliminated. This process was repeated serveral times until the sample appeared colourless. Finally the sample was dialyzed in a beaker containing 500 ml of 1x TE buffer that was continuously stired at 4ºC. The TE buffer was changed 2 or 3 times at 8 h intervals. Following dialysis, DNA was transferred to a tube, quantified in a spectrophotometer and was stored at 4ºC until required. A restriction enzyme analysis was performed using *Bgl*II, as were PCR amplifications using R-Hoar/F-Hoar and R-Brod-/R-Brod primers to confirm the identity and the quality of the DNA.

DNA sequencing of the three genotypes was performed by Lifesequencing S.L. (Paterna, Valencia, Spain). Between 5 and 10 µg of CICs gradient purified DNA was used for sequencing. For this, a genomic library was constructed in a sequencing vector for each of the DNAs. Between 2568 and 3185 sequencing reactions were performed that corresponded to between 1758 and 2015 clones. The sequencing reactions were performed in a 9600 PE thermocycler using an ABI Prism Big Dye Terminator Cycle Sequencing Reaction. Possible gaps and zones between problematic sequences were resequenced by amplification of each zone using primers designed to target sequences outside these zones. Finally the information was assembled using the Newbler v 2.3 program. The complete genome sequences of each of the genotypes were compared to the known sequence of ChchSNPV-NL and with one another, using the Clone Manager program (Scientific & Educational Software, 1994-2007).

### - Construction of occlusion body mixtures and co-occluded mixtures

To determine the minimal mixture of genotypes with the best insecticidal properties for control of *C. chalcites,* mixtures of occlusion bodies and co-occluded mixtures with different genotypes were constructed. The most abundant genotypes present, ChchTF1-A (isolated at a frequency of 36%), ChchTF1-B (26%) y ChchTF1-C (14%) were selected, as were genotypes that were present at low abundance: ChchTF1-G (1%) and ChchTF1-H (1%). Distinct combinations of genotypes were prepared in various proportions in the range from 10% to 90%. In this manner the contribution of the most abundant and least abundant genotypes to the insecticidal activity of the natural isolate were determined. Initially, uniform concentrations of the distinct genotypes were prepared from samples of 1x10⁹ OBs/ml. To construct occlusion body mixtures, the occlusion bodies were mixed in the desired proportions.

Only those combinations of genotypes that resulted in pathogenicity equal or greater than that of the natural isolate ChchTF1 are considered in this document. In this way, mixtures were selected that included the distinct genotypes in the proportions that they were isolated; ChchTF1-AB (in a ratio of 36:26; that was obtained by mixing 36 µl of ChchTF1-A suspension with 26 µl of ChchTF1-B suspension to produce a total volume of 62 µl), ChchTF1-ABC (in a ratio 36:26:14; produced by mixing 36 µl ChchTF1-A, 26 µl ChchTF1-B and 14 µl ChchTF1-C, in a total volume of 76 µl), ChchTF1-ABCG (in a ratio 36:26:14:1; produced by mixing 36 µl ChchTF1-A, 26 µl ChchTF1-B, 14 µl ChchTF1-C and 1 µl ChchTF1-G, in a total volume of 77 µl), and ChchTF1-ABCGH (in a ratio 36:26:14:1:1; prepared by mixing 36 µl ChchTF1-A, 26 µl ChchTF1-B, 14 µl ChchTF1-C, 1 µl ChchTF1-G and ChchTF1-H, in a total volume of 78 µl). In these mixtures, the occlusion bodies contained virions of the same genotype.

To produce ODVs of different genotypes that were co-occluded in the same occlusion body, in the proportions mentioned in each mixture, fourth stage *C. chlacites* larvae were orally infected with occlusion bodies at a concentration of 1x10⁶ OBs/ml (the mixture of occlusion bodies produced as described above was diluted 1000-fold prior to inoculation of the larvae). In this manner, the mixture of occlusion bodies containing ODVs of the same genotype entered the digestive tract and following the release of the virions a mixture of virions of different genotypes was produced

(originating from the distinct occlusion bodies) and upon entering and replicating in the same cell, the genotypically diverse virions are co-occluded in the same occlusion body. This results in the production of virions of different genotypes that are co-occluded in the same occlusion body.

Semiquantitative PCR, described in the methodology below, was used to confirm the proportion of each genotype in the mixtures and compare these proportions with the initial proportions in the inoculum.

### - PCR and semiquantitative PCR

To determine the nature of the different pure genotypes, in addition to restriction enzyme analyses, viral DNA obtained from each genotype was amplified by PCR using F-Hoar/R-Hoar and F-Brod-/R-Brod primer pairs. For each PCR reaction, 2.5 µl polymerase buffer (10x) was mixed with 1.25 µl magnesium chloride (50 mM MgCl₂), 0.25 µl dNTPs (diphosphate nucleotides), 0.25 µl of each of the respective primers (R-Hoar/F-Hoar or F-Brod/R-Brod), 0.25 µl of Taq polymerase and 0.25 µl of DNA diluted 1:100. The reaction conditions were denaturation at 94ºC during 2 minutes, followed by 35 cycles that included denaturation at 94ºC for 1 minute, hybridation at 50ºC for 1 minute, extension at 72ºC for 1 minute, followed finally by a final elongation step at 72ºC for 10 minutos.

To determine the relative porportions of each of the genotypes in the mixture, the intensity of each PCR fragment was measured and compared in each case. For semiquantitative PCR it is important not to reach the stationary phase of the reaction, given that at this point the intensity of the fragments tends to be similar, and consequently it is difficult to observe differences between the different fragments. For this, initial PCR reactions were performed to determine the number of amplification cycles required prior to reaching product saturation point (normally between 15 and 20 cycles). PCR reactions were stopped at cycle number 17, just prior to the decrease in the amplification ratio (stationary phase). At this point the density of the resulting PCR fragment is proportional to the initial quantity of DNA present in the mixture. The intensity of bands was compared using the image analysis program Scion Image PC (Scion Corp., Frederick, USA) and by this means the relative proportions of each of the fragments, and consequently each of the genotypes, can be estimated.

### - Bioassays in insects

The insecticidal activities of the ChchSNPV isolates from Almería (ChchSNPV-SP1), The Netherlands (ChchSNPV-NL) and the Canary Islands (ChchSNPV-TF1) were compared with those of the pure genotypes obtained from the ChchTF1 isolate and mixtures of genotypes. The concentration-mortality curves and mean time to death values (MTD) were determined by *per os* (peroral) bioassay, performed using the droplet feeding method, described above.

Second, fourth, fifth and sixth stage larvae of *C. chalcites* from the laboratory colony were placed in petri dishes without food at 26ºC for 8 to 12 hours and were then allowed to drink from an aqueous suspension comprising 10% (weight/volume) sucrose, 0.001% (weight/volume) Fluorella blue and occlusion bodies at one of five concentrations: 1x10⁵, 2x10⁴, 4x10³, 8x10² y 1,6x10² OBs/ml for second stage larvae, that had previously been determined to kill between 95 and 5% of experimental insects. Larvae that ingested the suspension in a 10 minute period were individually transferred to the wells of a 25-well cell culture plate containing a cube of semisynthetic diet. Bioassays using groups of 25 larvae per virus concentration and 25 larvae as negative controls were performed on three ocasions. Larvae were reared at 25ºC and mortality was checked at 12 hour intervals until death or pupation of the insects. Virus induced mortality results were subjected to logit analysis using the POLO-PC (LeOra Software, 1987) program.

For the fourth and fifth stage larvae the concentrations tested were those that resulted in 90% mortality, i.e., 1.16x10⁵ OBs/ml in the fourth stage (L4) and 9.16x10⁵OBs/ml in the fifth stage (L5). For the fifth stage larvae the concentrations used were between 9.08x10⁸ and 2.05x10⁷ OBs/ml, that killed between 90 and 50% of the experimental population.

The analysis of mortality over time, expressed as mean time to death (MTD), was performed for the assays of isolates and mixtures in groups of 25 second stage larvae. These insects drank a suspension of occlusion bodies during a 10 minute period, as described in the concentration-mortality assays. The concentration of occlusion bodies used for the time-mortality assay was 1.0x10⁵ OBs/ml for the ChchSNPV-SP1 and ChchSNPV-NL isolates, and 3.31x10⁴ OBs/ml for ChchSNPV-TF1. For the assays of mixtures, the concentrations used for the pure genotypes were 2.22x10⁵, 2.33x10⁵, 3.01x10⁵, 2.36x10⁵, 2.91x10⁵, 1.60x10⁵, 1.90x10⁵ and 1.87x10⁵ OBs/ml for genotypes ChchTF1-A, -B, -C, -D, -E, -F, -G y -H respectively, and were 4.37x10⁴, 3.49x10⁴, 4.22x10⁴, 4.37x10⁴ OBs/ml for the mixtures ChchTF1-AB, ChchTF1-ABC, ChchTF1-ABCG and ChchTF1-ABCGH, respectively. Finally, 5.31x10⁴, 3.73x10⁴, 4.91x10⁴ and 5.19x10⁴ OBs/ml were used for the co-occluded mixtures. These concentrations resulted in ∼90% mortality in all cases. Larvae were individually reared at 25ºC and mortality was checked at intervals of 8 hours. Time-mortality results were subjected to Weibull survival analysis using the Generalized Linear Interactive Modeling program (GLIM) (Crawley, 1993). Survival models such as the Weibull are preferred for time-mortality analyses in which the variance tends to increase with the mean. The time-mortality distribution of different isolates was also analyzed graphically. Only those individuals that died from polyhedrovirus disease, confirmed by microscopic analysis of occlusion bodies, were included in these analyses.

The production of occlusion bodies by pure genotypes and mixtures of genotypes was determined in second stage larvae. In contrast, mass-porduction studies were performed using fourth, fifth and sixth stage larvae of *C. chalcites* infected with occlusion bodies by the droplet feeding method at concentrations that resulted in 90% mortality. These concentrations varied according to the virus inoculum and insect stage (the same concentrations were used as described in the time-mortality assays). All the larvae that died from NPV disease were collected and stored at -20ºC until used for counting occlusion bodies (OBs). For this, each larva was homogenized in 100 µl distilled water and the total number of OBs per larva was estimated by counting samples in triplicate using an improved Neubauer haemocytometer. Results were analyzed by non-parametric Krustal Wallis and the Mann Whitney U test using the SPSS v12 package. Critical *P* values were subjected to false positive correction for multiple comparisons (Benjamini and Hochberg, 1995).

### - Example 1: In vitro cloning of individual genotypes present in the natural isolate of Chrysodeixis chalcites nucleopolvhedrovirus (ChchSNPV-TF1)

During various periods of sampling carried out in different greenhouses planted with banana crops in the Santa Cruz province of Tenerife (La Palma, El Hierro and Tenerife), Canary Islands, a large number of *C. chalcites* larvae showing signs of polyhedrosis disease were collected individually. The occlusion bodies were purified from each dead larva by ultracentrifugation through a discontinuous sucrose gradient. The occlusion bodies purified from each individual larva were considered to be an isolate. On occasions the quanity of occlusion bodies obtained from an infected larva was not sufficient to perform a DNA restriction profile characterization. In such cases it was necesary to amplify the isolate in larvae. Similarly, as the interval increases between death in the field and collection of the insect cadaver, the presence of bacteria and fungi that degrade the sample make it difficult to extract good quality DNA for restriction profile analysis. In these cases, the samples were also multiplied in larvae under laboratory conditions (for greater sterility, resulting in greater purity of occlusion bodies).

The amplification of occlusion bodies purified from field-collected larvae was perfomed by a single passage in fourth stage *C. chalcites* larvae from the laboratory colony maintained at the Universidad Pública de Navarra as mentioned in the previous section on materials and methods.

To determine the nature of the isolates, whether they were of the single or multiple type, a continuous sucrose gradiant was used. Virions were obtained from occlusion bodies of different isolates and their centrifugation through a sucrose gradient revealed that all the virions contained a single nucleocapsid as indicated by the single band visible in the sucrose gradient (Fig. 2A). In the case of a multiple type virus, various bands would have been observed, each one containing ODVs with different numbers of nucleocapsids, which determine the weight of the virions (Fig. 2B).

As such all the isolates correspond to single type NPV (SNPV). Thus, larvae appear to be infected by a single type nucleopolyhedrovirus, named ChchSNPV.

Of the isolates obtained, specifically the natural isolate corresponding to larva number 33, collected from a greenhouse in southern Tenerife was infected by a mixture of different genotypes. This isolate was selected for cloning of the component genotypes. The isolate was named ChchSNPV-TF1, or also in the more abbreviated form ChchTF1. Other isolates from other larvae from the same zone were numbered similarly (ChchTF2, ChchTF3, ChchTF3, ChchTF4, ChchTF5...).

To purify the individual genotypes present in the ChchTF1 isolate, *in vitro* purification techniques were employed (Muñoz et al., 2001; Simón et al., 2004). The fact that a specific *C. chalcites* cell line has not been developed made it necesary to determine the susceptibility of different cell lines to infection by the ChchTF1 isolate. Among the cell lines analyzed, the cell line BTI-TN-5B1-4, that is commonly known as High Five, was selected as it was the most susceptible. This cell line was developed from a clone of the parental cell line of *Trichoplusia ni* originating from embryonic tissue of this species which is available commercially (Invitrogen).

Plaque purification was based on the cell culture infection *in vitro* with low titres of virus particles obtained following the extraction of insect haemolymph 48 h after oral infection. At this moment, the haemolymph contains abundant BVs (budded virions) that each contain a single nucleocapsid, and as such contain a single genotype. In this way, a few isolated cells become infected by a single infective particle (virion). The cell culture is subsequently covered with a solid layer that limits the spread of virus particles so that the cells that were initially infected release their progeny virus particles, and only the neighbouring cells become infected. The resulting plaque is generated from a single BV and consequently represents a clonal population.

Following this protocol, groups of 25 fifth stage (L5) *C. chalcites* larvae were inoculated with a concentration of 10⁷ OBs/ml of ChchTF1. At 48 hours later a small incision was made in the final pair of pseudopodos of the larvae to extract haemolymph, that contains large quantities of BVs. The haemolymph was filtered through a 0.45 µm filter to eliminate possible contaminants such as bacteria. The haemolymph was then serially diluted in TNM-FH (Gibco, Invitrogen) medium. Batches of 2x10⁶ High Five (Invitrogen) cells were incubated in 6-well 30 mm culture plates at 27ºC for three hours to allow their attachment. After 3 hours, the culture medium was replaced with 100 µl of the medium with diluted haemolymph and the BVs that it contained. After one hour the viral inoculum contained in the haemolymph was substituted with new TNM-FH medium containing antibiotics (1% penicillin-streptomycin (Gibco®, Life Technologies Ltd, UK), to eliminate contaminants, and agarose to prevent excessive dissemination of the infection. After 5 days cells were stained with neutral red in order to differentiate healthy cells from infected cells. Healthy cells stained reddish, in contrast to infected cells that produce a uncoloured zone, called a plaque, that comprises a group of dead cells that arrise from the infection of a single BV. Each clone or plaque was taken with the aid of a sterile Pasteur pipette. Plaques were diluted individually in 100 µl TNM-FH medium. A total of 245 clones were obtained. Each suspension was injected into fourth stage (L4) larvae for *in vivo* multiplication and the production of large quantities of occlusion bodies. Of the 245 plaques or clones, only 117 resulted in lethal infection of injected larvae.These 117 clones were analyzed at the molecular level to determine the number of different genotypes present.

### - Example 2: Molecular characterization of individual genotypes cloned in vitro. 2.1. Restriction profiles

Using molecular techniques based on the use of restriction endonucleases, of the 117 clones analyzed 8 different genotypes were present in the ChchTF1 isolate: ChchTF1-A, ChchTF1-B, ChchTF1-C, ChchTF1-D, ChchTF1-E, ChchTF1-F, ChchTF1-G y ChchTF1-H (Fig. 3).

Genotype ChchTF1-A was taken as the reference genotype, given that its profile is identical to that of ChchTF1, which suggests that this genotype is present at high abundance in the natural isolate, and was also the genotype that was isolated at the highest frequency.

As can be seen in Fig. 3, panel A, the digestion of genomic DNA of the distinct cloned genotypes using the restriction endonuclease *Bgl*II, results in a characteristic profile which is unique for each genotype. Certain fragments generated by this restriction enzyme can be used as markers to differentiate between the distinct genotypes. Thus genotypes ChchTF1-B, -C y -D present a unique characteristic fragment of 6203 bp (*Bgl*II-L), 5339 bp (*Bgl*II-M) and 10503 bp (*Bgl*II-D), respectively, compared to ChchTF1-A. The genotypes ChchTF1-E and -F, also present two polymorphic fragments of 6220 bp (*Bgl*II-M) and 5339 bp (*Bgl*II-N) in the case of ChchTF1-E, compared to 10500 bp (*Bgl*II-D) and 6220 bp (*Bgl*II-N) in the case of ChchTF1-F. Finally, the restriction profiles of ChchTF1-G and ChchTF1-H present three polymorphic fragments of 27705 bp (*Bgl*II-A), 5338 bp (*Bgl*II-L; as also observed in ChchTF1-C and -H), and of 3923 bp (*Bgl*II-P) in the case of ChchTF1-G, and of 5338 bp (*Bgl*II-L; as also observed in ChchTF1-C and -H), and of 4769 (*Bgl*II-N) and 4208 bp (*Bgl*II-P) for ChchTF1-H. Submolar fragments were not observed in the restriction profiles of these genotypes following one passage of multiplication in larvae. The restriction profiles were stable following various passages indicating their genetic stability and purity.

The restriction profiles were also shown to allow the differentiation of these genotypes with those known previously; specifically the isolate from The Netherlands ChchSNPV-NL (van Oers et al., 2004, 2005) and the isolate from Almería ChchSNPV-SP1 (Murillo et al., 2000). Table 3 that includes information reflected in Table 2, also permits comparison of the restriction profiles generated from other isolates of ChchSNPV obtained during sampling performed in the Canary Islands referred to in the present document (namely isolates ChchSNPV-TF2, ChchSNPV-TF3, ChchSNPV-TF4, ChchSNPV-TF5, indicated in abbreviated from in the Table as TF2, TF3, TF4 and TF5, respectively). This proves that the restriction enzyme profile is valuable in the identification of the presence of genotypes in the isolates.

**Table 3.- Estimated fragment sizes (kpb) generated following BglII digestion of genomic DNA from distrinct isolates and genotypes.**

| Fragments | ChchSNPV isolates | | | | | | | ChchSNPV-TF1 genotypes | | |
|---|---|---|---|---|---|---|---|---|---|---|
| | NL | SP1 | TF1 | TF2 | TF3 | TF4 | TF5 | A | B | C |
| A | 27.71 | 14.41 | 14.41 | 14.41 | 14.41 | 14.41 | 14.41 | 14.41 | 14.41 | 14.41 |
| B | 12.78 | 13.29 | 13.29 | 13.29 | 13.30 | 13.29 | 13.29 | 13.29 | 13.29 | 13.30 |
| C | **9.83*** | 12.79 | 12.79 | 12.79 | 12.78 | 12.79 | 12.79 | 12.79 | 12.79 | 12.78 |
| D | 9.76 | 11.90 | 12.12 | 9.78 | **10.19*** | 12.12 | 12.11 | 12.12 | 12.78 | **10.19*** |
| E | 9.06 | **9.93*** | **9.93*** | **9.69*** | 9.77 | **9.93*** | **9.93*** | **9.93*** | **10.19*** | 9.77 |
| F | 8.97 | 9.79 | 9.79 | 9.06 | 9.04 | 9.79 | 9.79 | 9.79 | 9.79 | 9.04 |
| G | **8.52#** | 9.60 | 8.97 | **8.72#** | 8.96 | 8.97 | 8.97 | 8.97 | 9.04 | 8.96 |
| H | 8.00 | 9.06 | 8.93 | 8.00 | **8.72#** | 8.93 | 8.93 | 8.93 | 8.93 | **8.72#** |
| I | 7.02 | **8.97#** | **8.72#** | 7.02 | 8.00 | **8.72#** | **8.72#** | **8.72#** | **8.20#** | 8.00 |
| J | 6.80 | 8.00 | 8.00 | 6.82 | 7.02 | 8.00 | 8.00 | 8.00 | 7.02 | 7.02 |
| K | 6.69 | 7.02 | 7.02 | 6.68 | 6.82 | 7.02 | 7.02 | 7.02 | 6.69 | 6.82 |
| L | 5.34 | 6.69 | 6.69 | 5.34 | 6.68 | 6.69 | 6.69 | 6.69 | 6.20 | 6.68 |
| M | 5.07 | 5.07 | 5.07 | 5.07 | 5.34 | (5.34) | (5.34) | 5.07 | 5.06 | 5.34 |
| N | 4.31 | 4.31 | 4.29 | 4.77 | 5.07 | 5.07 | 5.06 | 4.29 | 4.29 | 5.07 |
| 0 | 4.14 | 4.14 | 4.13 | 4.29 | 4.29 | (4.77) | 4.29 | 4.13 | 4.13 | 4.29 |
| P | 3.94 | 3.94 | 3.86 | 4.21 | 4.13 | 4.29 | 4.13 | 3.86 | 3.86 | 4.13 |
| Q | 3.84 | 3.84 | 3.84 | 4.15 | 3.88 | 4.13 | 3.88 | 3.84 | 3.84 | 3.88 |
| R | 1.87 | 1.87 | 1.87 | 3.88 | 3.84 | 3.86 | 3.83 | 1.87 | 1.87 | 3.84 |
| S | 1.75 | 1.75 | 1.75 | 3.84 | 1.87 | 3.84 | 1.86 | 1.75 | 1.75 | 1.87 |
| T | 1.75 | 1.75 | 1.75 | 1.87 | 1.75 | 1.87 | 1.75 | 1.75 | 1.75 | 1.75 |
| U | 1.53 | 1.53 | 1.53 | 1.75 | 1.75 | 1.75 | 1.74 | 1.53 | 1.53 | 1.75 |
| V | 0.78 | 0.78 | 0.78 | 1.74 | 1.53 | 1.75 | 1.52 | 0.78 | 0.78 | 1.53 |
| W | 0.15 | 0.15 | 0.15 | 1.53 | 0.78 | 1.53 | 0.77 | 0.15 | 0.74 | 0.78 |
| X | | | | 0.77 | 0.15 | 0.78 | 0.15 | | 0.15 | 0.15 |
| Y | | | | 0.15 | | 0.15 | | | | |
| Total size (kb) | 149.61 | 150.58 | 149.68 | 149.61 | 150.07 | 149.68 | 149.56 | 150.58 | 149.08 | 150.07 |

| | | | | | | | | | | |
|---|---|---|---|---|---|---|---|---|---|---|
| Restriction fragments on which the *hoar gene* is located. # Restriction fragments on which the *bro-d* gene is located (indicated in Fig. 3A using the same symbol) The underlined frag indicates the characteristic fragment of each profile (indicated in Fig. 3A using an arrow →) | | | | | | | | | | |

As can be observed in Table 3, the fragments obtained from ChchTF1 and those of genotype ChchTF1-A are identical, as genotype ChchTF1-A is dominant in the ChchTF1 isolate and its restriction profile is identical. The same situation is observed in the fragments of isolate ChchTF3 and genotype ChchTF1-C, which also presented an identical profile, which suggests that genotype ChchTF1-C is dominant in the ChchTF3 isolate. Isolates ChchTF4 and ChchTF5 comprise a heterogenous mixture of two isolates. In the profile of ChchTF4 the fragments *Bgl*II-M (5.34 kpb) and *Bgl*II-O (4.76 kbp), characteristic of ChchTF2, appear as submolar bands, and as no other characteristic bands appear with low intensity, this suggests that ChchTF4 is mainly composed of ChchTF1 and ChchTF2. In the profile of ChchTF5 the *Bgl*II-M (5,34 kbp) fragment, characteristic of ChchTF3, appears at a low intensity which indicates that ChchTF5 is mainly composed of two isolates: ChchTF1 and ChchTF3.

Concerning the previously described isolates, ChchSNPV-NL and the isolate from Almería ChchSNPV-SP1, the information in the previous Table reveals differences in the profiles of the novel isolates with respect to the known isolates. For example, the *Bgl*II restriction profile of ChchSNPV-NL presents a fragment of 27.71 kbp, that does not appear in any other the other profiles, in which the largest sized fragment is 14.71 kbp. The profile within the zone of large fragments differs from those obtained from the ChchTF1 isolates that present four fragments in the zone between 10 kbp and 15 kbp (of 14.41, ∼13.29, ∼12.79 y ∼12.12 kbp for ChchTF1-A, the final fragment being of 12.78 kbp for ChchTF1-B or 10.19 kbp for ChchTF1-C), whereas ChchSNPV-NL only presents a fragment of 12.78 kbp in this range of fragment sizes. The profiles of the new genotypes of the invention obtained with *Bgl*II, with the profile corresponding to isolate ChchSNPV-SP1 are very similar, although clear differences are also present. For example, as mentioned previously, the profile of ChchTF1-A, presents at least four fragments whose size differs from that of the profile of ChchSNPV-SP1, namely the fragments D (11.90 for ChchSNPV-SP1 and 12.12 ChchTF1-A) G (9.60 compared to 8.97), H (9.06 compared to 8.93) and I (8.97 compared to 8.72). Digestion of ChchSNPV-SP1 results in four fragments of over 9 kbp (E, F, G y H) and one fragment of almost 9 kbp (fragment I: 8.97 kbp), whereas ChchTF1-A presents two bands with a size of over 9 kpb (E and F), and two fragments with a size very close to 9 kbp (G and H, of 8.97 and 8.93 kbp, respectively). As such, the profiles obtained with *Bgl*II can be used for the identification of genotypes, and to distinguish them from known genotypes, although profiles obtained from other restriction enzymes can also be used or may provide complementary information to that of *Bgl*II.

The photograph shown in Fig. 3., panel B shows the restriction profiles obtained with enzymes *Eco*RI and *Pst*I from strains already known, those of isolate ChChTF1 and three of the component genotypes, namely ChchTF1-A, ChchTF1-B and ChchTF1-C.

Comparison of the profiles obtained using *Pst*I, for example, demonstrates that isolate ChchTF1, and the genotypes A and B from that isolate, shows a triplet of bands in the size range between 5 and 4 kb, which is present as a doublet in the isolates ChchSNPV-NL and ChchSNPV-SP1, which is also seen in genotype C of ChchTF1; these last three genomes do not produce the smallest sized band of the triplet observed in genotypes A and B.

Digestion using *Pst*I also allows the Almería isolate ChchSNPV-SP1 to be distinguished from any of the genotypes A, B or C of the Tenerife isolate. The restriction profile of strain ChchSNPV-SP1 shows a triplet of bands greater than 3 kb and less than 4 kb, that are almost equidistant. Genotypes A and B of ChchTF1, similar to ChchTF1, give rise to two bands in this same zone, of which the upper pair coincide with the first two bands of the triplet observed for ChchSNPV-SP1, whereas the lower pair is absent in the profile obtained for ChchSNPV-SP1. The lower band of the triplet of ChchSNPV-SP1 in the size zone between 3 and 4 kb is absent in the profile of ChchTF1 and of any of the genotypes A, B or C. Genotype C, moreover, can be distinguished from genotypes A and B because in the 3 - 4 kb zone it does not present a pair of upper bands and a pair of lower bands, but rather a pair of upper bands and a single lower band, which coincides with a band of smaller size than the lower pair of bands observed in genotypes A and B.

Digestion with *Eco*RI, also allows genotypes A, B and C of the ChchTF1 to be distinguished, particularly with reference to the bands close to (longer or shorter than) 4 kb. Genotype C presents 4 bands close to 4 kb: one pair of bands (shown in the photograph in panel A) at the height of the 4 kb commercial marker band, and two other bands, one larger and one smaller, of which the larger band (size greater than 4 kb) that appears to be characteristic of genotype C, is not present in genotypes A or B. Genotype B presents three bands in this zone (of which the intermediate band, due to its intensity, may be possible to separate into two distinct fragments following a more extended electrophoresis). Of these three bands, the two lower bands coincide in size with the corresponding bands of genotype C. In contrast, the upper band presents an intermediate size between the upper band of genotype C and the following band obtained for this genotype. Genotype A shows two lower bands in this zone. The profile corresponding to isolate ChchSNPV-SP1 shows three bands in this same zone, as does genotype B, but the upper band appears lower (is smaller) than the upper band of the triplet observed in genotype B.
As such, the study of restriction profiles allows us to reach three conclusions:
- Genotypes A, B, C, D, E, F, G, H of the isolate ChchTF1 differ from one another and can be differentiated by their restriction profiles.
- Genotypes A, B and C, are not only distinguishable from one another, but can also be differentiated, by their restriction profiles, from known strains of ChchSNPV: ChchSNPV-NL and ChchSNPV-SP1.
- Given that the restriction profiles differ, none of the genotypes A, B or C of ChchTF1 has a genome that is identical to one of the previously isolated strains from The Netherlands (ChchSNPV-NL) or Almería (ChchSNPV-SP1). As such, they can be considered as novel genotypes that are distinct from, and that can be distinguished from, known strains of ChChSNPV.

### 2.2. Differentiation by PCR amplification

A more precise differentitation is achieved by amplification of regions of the genome that are characteristic of each of the genotypes. For this, the PCR (polymerase chain reaction) technique is employed using specific primers designed to target regions of variability.

To this end the complete genome sequence of three (ChchTF1-A, ChchTF1-B, ChchTF1-C) of the eight genotypes was determined. The sequence of each of these genomes is given further on as indicated by SEQ ID NO:17 (ChchTF1-A), SEQ ID NO:18 (ChchTF1-B) and SEQ ID NO:19 (ChchTF1-C).

The complete sequence of these three genotypes has revealed great similarity among them, with 99% of the genome shared among them, and clear similarity to the genome sequence of the isolate from The Netherlands (98%).

Two principal zones of variability have been observed:
- One zone is localized in ORF4 of ChchSNPV (chch4; gen *hoar,* between nucleotides 2965 and 6504 in the genome sequence of ChchSNPV-NL: van Oers et al., 2005). Fig. 4 shows the alignment of this region of the genome of ChchSNPV-NL with the analogous regions of the genomes of ChchTF1-A, ChchTF1-B and ChchTF1-C. The complete sequences of the *hoar* gene corresponding to each of these genotypes, specifically the complementary sequences to the CDS (coding sequence), are represented by SEQ ID NO: 11, SEQ ID NO:12 and SEQ ID NO:13, respectively.
- The second zone is located in ORF114 (chch114, *bro-d,* between nucleotides 113517 and 114806 of the genome sequence of ChchSNPV-NL). Fig. 5 shows the alignment of this region of the genome of ChchSNPV-NL with the analogous regions of the genomes of ChchTF1-A, ChchTF1-B and ChchTF1-C. The complementary sequences of the *bro-d* gene corresponding to each of these genotypes, specifically the complementary sequences to the CDS (coding sequence), are represented by SEQ ID NO: 14, SEQ ID NO:15 y SEQ ID NO:16, respectively.
The following primers were designed.

For the *hoar* gene:
- forward F-Hoar: 5'-TTGTTGTATGCAGCATTGTA-3' (SEQ ID NO:1), and
- reverse R-Hoar: 5'-AGTAAATATGGCTACTGCAG-3' (SEQ ID NO:2)
and their use in the amplification of the indicated zone of the chch4 (*hoar*) gene was demonstrated by PCR as described in the section on the techniques used, under the subheading "PCR and semiquantitative PCR". The results obtained following electrophoresis of the amplified fragments are shown in Fig. 6, panel A.

For the *bro-d* gene:
- forward: F-Brod: 5'-TATAGTATAATATTAAACTC-3' (SEQ ID NO:3)
- reverse: R-Brod: 5'-GTCATATTCGAGTCGTATCC-3' (SEQ ID NO:4)
and their use in the amplification of the indicated zone of the *bro-d* gene was demonstrated by PCR as described in the section on the techniques used, under the subheading "PCR and semiquantitative PCR". The results obtained following electrophoresis of the amplified fragments are shown in Fig. 6, panel A.

Information on the specific primers designed to target ORF4 (chch4, *hoar*) and ORF114 (chch114, *bro-d*), nuceotide sequences, direction of genome amplification ("F/R": forward (>) or reverse (<)), genotype, the position in the genome targeted by each primer for each of the genotypes, length of the amplified fragment and reference identification number of the PCR amplified sequence obtained, are sumarized below in Tables 4 and 5.

**Table 4: Primers targeted at chch4 (hoar) and the amplified fragments for each genotype**

| Primer (sequence no.) | F/R* | Genotype | Position | Fragment length | SEQ ID NO. |
|---|---|---|---|---|---|
| F-Hoar (SEQ ID NO:1) | > | ChchNL | 3443-3463 | 675 | - |
| R-Hoar (SEQ ID NO:2) | < | ChchNL | 4098-4117 | | |
| F-Hoar (SEQ ID NO:1) | > | ChchTF1-A | 3444-3463 | 756 | 5 |
| R-Hoar (SEQ ID NO:2) | < | ChchTF1-A | 4180-4199 | | |
| F-Hoar (SEQ ID NO:1) | > | ChchTF1-B | 3444-3463 | 921 | 6 |
| R-Hoar (SEQ ID NO:2) | < | ChchTF1-B | 4345-4364 | | |
| F-Hoar (SEQ ID NO:1) | > | ChchTF1-C | 3443-3462 | 651 | 7 |
| R-Hoar (SEQ ID NO:2) | < | ChchTF1-C | 4074-4093 | | |

| | | | | | |
|---|---|---|---|---|---|
| *F/R: F forward (>), R reverse (<) | | | | | |

**Table 5: Primers targeted at chch114 (bro-d) and fragments amplified from each genotype**

| Primer | (sequence) | F/R* | Genotype | Position | Fragment length | SEQ ID NO: |
|---|---|---|---|---|---|---|
| F-Brod | (SEQ ID NO:3) | > | ChchNL | 113.410-113.429 | 1540 | - |
| R-Brod | (SEQ ID NO:4) | < | ChchNL | 114.930-114.949 | | |
| F-Brod | (SEQ ID NO:3) | > | ChchTF1-A | 113.281-113.300 | 1743 | 8 |
| R-Brod | (SEQ ID NO:4) | < | ChchTF1-A | 115.004-115.023 | | |
| F-Brod | (SEQ ID NO:3) | > | ChchTF1-B | 113.542-113.471 | 962 | 9 |
| R-Brod | (SEQ ID NO:4) | < | ChchTF1-B | 114.394-114.413 | | |
| F-Brod | (SEQ ID NO:3) | > | ChchTF1-C | 113.662-116.681 | 1725 | 10 |
| R-Brod | (SEQ ID NO:4) | < | ChchTF1-C | 115.367-115.386 | | |

| | | | | | | |
|---|---|---|---|---|---|---|
| *F/R: F forward (>), R reverse (<) | | | | | | |

### - Example 3: Insecticidal activity of individual genotypes and mixtures of genotypes

To perform the biological characterization and select the mimimal mixture of genotypes with the best insecticidal properties, the insecticidal activity of the individual genotypes and the mixtures of occlusion bodies containing identical genomes, and occlusion bodies that contained co-occluded virions comprising the genomes characterizingdistinct genotypes was determined.

For simplicity, in this example, the term "mixtures of occlusion bodies" will be used to refer to mixtures of occlusion bodies of different genotypes in which each of the occlusion bodies contains one or more virions of a single genotype. In contrast, the term "co-occluded mixtures" or "co-occluded virions" will be used to refer to those cases in which each of the occlusion bodies of the mixture contains virions of more than one distinct genotype. In whichever case, it is understood that the virions are ODVs, occlusion derived virions.

To construct mixtures, distinct combinations of genotypes in various proportions were used, as described in the previous section on materials and techniques under the subheading "Construction of occlusion body mixtures and co-occluded mixtures". Briefly, mixtures of occlusion bodies were obtained simply by mixing occlusion bodies of each genotype in the desired proportions. In contrast, to obtain co-occluded mixtures, fourth stage (L4) larvae of *C. chalcites* were inoculated with mixtures of occlusion bodies and, following the infection process, co-occluded mixtures were obtained. The true proportions of the genotypes present in the mixtures were compared and with the proportions used to inoculate the larvae by semiquantitative PCR. This verification confirmed that genotypes maintained their relative proportions following a single passage in larvae.

Pathogenicity was expressed as the mean lethal concentration (LC₅₀). These values were determined by bioassay in second stage (L2) *C. chalcites* larvae. Larvae were inoculated by ingestion following the droplet feeding method described by Hughes and Wood (1981) using the following concentrations: 1.0x10⁵, 2.0x10⁴, 4.0x10³, 8.0x10² and 1.6x10² OBs/ml estimated in previous studies to result in between 95% and 5% mortality. A group of larvae treated with the same solution, but without occlusion bodies, was employed as a control group. Treated larvae were reared on artificial diet at 26 ºC. The number of larvae that died from virus infection was registered at 8 hour intervals.

Concentration-mortality results were analyzed by fitting a regression of the logarithm of concentration against mortality, which permitted the calculation of the LC₅₀ value for the individual genotypes and the mixtures, using the program Polo-PC (LeOra-Software, 1987).

Bioassays were used to compare the insecticidal activity of the natural isolates from Almería (ChchSNPV-SP1, abbreviated to ChchSP1 in this example), The Netherlands (ChchSNPV-NL, abbriviated in this example as ChchNL) and the Canary Islands (ChchSNPV-TF1, abbreviated in this example as ChchTF1), of pure genotypes cloned from ChchTF1 (ChchTF1-A, ChchTF1-B, ChchTF1-C, ChchTF1-D, ChchTF1-E, ChchTF1-F, ChchTF1-G and ChchTF1-H), and occlusion body mixtures and co-occluded mixtures (ChchTF1-ABC, ChchTF1-ABCG and ChchTF1-ABCGH, where the different letters following the abbreviation ChchTF1 refer to each of the genotypes of the ChchTF1 isolate that were present in the mixture).

Table 6 shows the LC₅₀ values and the relative potency of the different natural isolates and the individual genotypes (A, B and C), and the mixture of genotypes ABC, taking the isolate from Almería, ChchSP1 as the reference isolate. The relative potencies were calculated as the ratio of each LC₅₀ value relative to that of the ChchSP1 isolate.

**Table 6. Insecticidal activity of the natural isolates and individual genotypes A, B and C.**

| Treatment nent | | LC₅₀ (OBs/ ml) | Relative potency | 95% confidence limits | | MTD (h) | 95% confidence limits | |
|---|---|---|---|---|---|---|---|---|
| | | | | upper | lower | | upper | lower |
| Natural Isolates | ChchSP1 | 1.89x10⁴ | 1 | - | | 126 | 121 | 131 |
| | ChchNL | 2.08x10⁴ | 0.91 | 0.39 | 2.11 | 126 | 123 | 127 |
| | ChchTF1 | 1.35x10³ | 14.00 | 6.97 | 28.07 | 127 | 125 | 129 |
| Individual genotypes | ChchTF1-A | 9.13x10³ | 2.07 | 1.03 | 4.12 | 125 | 122 | 128 |
| | ChchTF1-B | 5.24x10³ | 3.60 | 1.75 | 7.37 | 117 | 115 | 119 |
| | ChchTF1-C | 1.11x10⁴ | 1.70 | 0.84 | 3.41 | 115 | 113 | 117 |
| Co-occluded mixtures of virions | ChchTF1-ABC | 6.44x10² | 2.09 | 1.05 | 4.13 | 93 | 90 | 113 |

From these results it is clear that the relative potency of the natural isolate ChchTF1 is greater than that of any of the natural isolates known previously. The known natural isolates ChchNL and ChchSP1, have similar relative potencies and the differences observed between them are not statistically significant (P>0.05). The individual genotypes A, B and C, present relative potencies that are higher than that of the natural isolate ChchSP1. This suggests that interactions among the genotypes present in ChchTF1 result in a mixture (or natural isolate ChchTF1) that is more insecticidally active.

The information given in Table 7 shows that the mixtures of occlusion bodies and the co-occluded mixtures have relative potencies higher than that of any of the individual genotypes or the natural isolates, ChchNL or ChchSP1, or the isolate from which they originate, namely ChchTF1. However, the mixture of co-occluded genotypes ChchTF1-ABC not only presents higher pathogenicity, but also greater virulence than any of the isolates, pure genotypes, or mixtures, as reflected in the mean time to death which is ∼33 h shorter than that of the natural isolate. One of the main limitations to the use of these viruses as biological insecticdes is that they are slow to kill the pest insect, but the use of this mixture would reduce the mortality time by more than one day, resulting in a reduction in the feeding period of the larvae and consequently a reduction in the damage to the crop.

The information in Table 7 shows the results obtained for each of the individual genotypes of the ChchTF1 isolate, the occlusion body mixtures, and co-occluded mixtures, that were subjected to bioassay. The co-occluded mixture of genotypes ChchTF1-ABC in the ratio 36:26:14 was selected given that it was the more pathogenic and virulent than any of the natural isolates, pure genotypes or other combinations of genotypes (Table 7).

**Table 7. LC₅₀ and relative potency values of the individual genotypes and mixtures of genotypes in the proportions mentioned in the methods section compared to the natural isolate ChchTF1. In this case the relative potencies are calculated as the ratio of each LC₅₀ value relative to that of the natural isolate ChchTF1.**

| Treatment | | LC₅₀ (OBs/ml) | Relative Potency | 95% confidence limits | | MTD (h) | 95% confidence limits | |
|---|---|---|---|---|---|---|---|---|
| | | | | lower | upper | | lower | upper |
| Natural isolate | ChchTF1 | 1.61x10³ | 1 | - | - | 126 | 121 | 131 |
| Individual genotypes | ChchTF1-A | 9.13x10³ | 0.14 | 0.08 | 0.25 | 123 | 120 | 126 |
| | ChchTF1-B | 5.24x10³ | 0.25 | 0.14 | 0.46 | 116 | 114 | 118 |
| | ChchTF1-C | 1.11x10⁴ | 0.12 | 0.06 | 0.21 | 113 | 109 | 117 |
| | ChchTF1-D | 1.72x10⁴ | 0.07 | 0.04 | 0.16 | 120 | 118 | 122 |
| | ChchTF1-E | 6.24x10³ | 0.21 | 0.11 | 0.36 | 119 | 117 | 121 |
| | ChchTF1-F | 1.39x10⁴ | 0.11 | 0.07 | 0.17 | 126 | 121 | 129 |
| | ChchTF1-G | 1.69x10⁴ | 0.09 | 0.06 | 0.15 | 127 | 121 | 133 |
| | ChchTF1-H | 1.35x10⁴ | 0.11 | 0.07 | 0.18 | 125 | 120 | 130 |
| Mixtures of occlusion bodies | ChchTF1-A | 1.34x10³ | 1 | 0.55 | 1.80 | 115 | 110 | 120 |
| | ChchTF1-ABC | 5.75x10² | 2.34 | 1.16 | 4.70 | 112 | 105 | 119 |
| | ChchTF1-ABCG | 9.08x10² | 1.48 | 0.82 | 2.65 | 113 | 106 | 120 |
| | ChchTF1-ABCGH | 1.95x10³ | 0.68 | 0.39 | 1.19 | 111 | 104 | 120 |
| Co-occluded mixtures of virions | ChchTF1-AB | 2.13x10³ | 0.63 | 0.36 | 1.10 | 105 | 97 | 113 |
| | ChchTF1-ABC | 6.44x10² | 2.09 | 1.05 | 4.13 | 93 | 90 | 96 |
| | ChchTF1-ABCG | 1.93x10³ | 0.69 | 0.39 | 1.22 | 99 | 96 | 102 |
| | ChchTF1-ABCGH | 1.77x10³ | 0.76 | 0.42 | 1.34 | 98 | 95 | 101 |

The pathogenicity of the ChchTF1-ABC mixture, both as mixtures of occlusion bodies and co-occluded mixtures, was greater than that of the individual genotypes or that of the other mixtures of genotypes. However, the co-occluded mixture was much more virulent than the mixture of occlusion bodies (20 h difference in mean time to death; the co-occluded mixture killed larvae on average 20 h faster than the mixture of occlusion bodies, or 33 h faster than the natural isolate ChchTF1). Consequently, the co-occluded mixture ChchTF1-ABC, in the ratio 36:26:14, was selected as the preferred option because in this manner the amount of active material necessary for field applications to crops is reduced (as it is more pathogenic), and because larvae die more quickly, by approximately one day. By dying sooner, the period for which the crop plants are exposed to insect defoliation, and consequently the magnitude of damage to the plants, are reduced. The co-occluded mixture not only presents better characteristics (in terms of pathogenicity and virulence), but is also easier to produce and cheaper to produce. Because of this the co-occluded mixture ChchTF1-ABC (36:26:14) was selected for mass-production assays and field experiments.

### - Example 4: Mass production of ChchSNPV

### 4.1. Effect of larval stage used for inoculation.

For mass production of ChchSNPV the number of occlusion bodies produced by lethally infected larvae was determined. In the case of other species of lepidopterans, the last possible stage that can be productively infected is usually taken as the most suitable for mass production of the virus (Kalia et al., 2001; Lasa et al., 2007). For *C. chalcites* this would be the sixth stage (L6), as this is the stage with the largest body size. This means that the number of cells in which the virus can replicate, and thus the number of occlusion bodies that can be produced in each infected insect, is expected to be highest in sixth stage larvae. However, this variable can differ significantly depending on the host-baculovirus system and therefore requires detailed study in each case.

The final three stages (L4, L5, L6) were selected for study to determine the production of occlusion bodies/larva in each of these stages. Larvae were inoculated individually following the droplet feeding technique described by Hughes and Wood (1981), and were individually placed in cups containing artificial diet until death by polyhedrosis disease or pupation. Each larval stage was inoculated with the corresponding LC₉₀ calculated previously in concentration-mortality studies. These were 1.16x10⁵ OBs/ml for the L4 stage, 9.16x10⁵ OBs/ml for the L5 stage and 9.08x10⁸ OBs/ml for the L6 stage. Results were analyzed using the statistical package SPSS v12.

The L6 stage was significantly more productive than the other stages (χ²=73,277; d.f.=2; p<0.001, (χ²: Chi-squared value following Kruskal-Wallis test; results were not normally distributed and were therefore subjected to a non-parametric analysis; d.f.: degrees of freedom). The mean occlusion body production in the L6 stage was 1.10x10¹¹ OBs/larva (Fig. 7).

### 4.2. Effect of stage and cannibalism on the production of occlusion bodies/larva

Mass production of virus can be acheived by treating larvae individually using cups of 20 ml capacity in which a single larva is placed in each cup, or in larger containers (1.5 litre capacity), in which a larger number of larvae are placed. However, the latter opion may led to problems depending on the frequency of cannibalism observed in each larval stage. Consequently the percentage of cannibalism was determined in groups of larvae of different stages.

For this, groups of 50 larvae of stages L4, L5 and L6 were inoculated as indicated in the experiment described in point 4.1, and were placed in groups in containers of 1.5 litre capacity and monitored for cannibalism. Results were analyzed using contingency tables (that results in the calculation of a χ² statistic). Cannibalism was highest among fourth stage larvae and was reduced in later stage larvae (χ²=98.266; d,f.=4; p<0.001) (Fig. 8).

Based on these results the sixth stage (L6) was selected as the most suitable for mass production of ChchSNPV in *C. chalcites* larvae, given that it is the most productive stage and shows the lowest percentage of cannibalism.

### 4.3. Optimal timing of inoculation of larvae.

Preliminary studies revealed that *C. chalcites* larvae are significantly larger one day after moulting to the sixth stage, compared to their size on the day of the moult. This led us to consider that if larvae were inoculated one day after moulting, the production of virus might be increased. For this reason, the production of occlusion bodies in recently-moulted L6 larvae was compared with that of larvae inoculated 24 h after moulting (L6+24). For this, 50 larvae of each physiological stage (L6 and L6+24) were inoculated with 9.08x10⁸ OBs/ml that was previously determined to represent an LC₉₀ concentration.

The L6+24 larvae produced 5-fold more occlusion bodies (Mann Whitney U = 105.500, p<0.001; Mann Whitney U: a non-parametric statistic used to compare the median values of two independent groups), than recently-moulted L6 larvae (Fig. 9). Consequently, to produce a greater number of occlusion bodies/larva, *C. chalcites* larvae must be inoculated with ChchSNPV at 24 hours after having moulted to L6.

### 4.4. Effect of viral inoculum concentration.

In another assay, the optimal concentration of virus inoculum for the production of occlusion bodies in the sixth larval stage (L6) selected previously. It is known that high concentrations of inoculum tend to kill the larva quickly and therefore reduce the production of occlusion bodies. Consequently, L6+24 larvae were inocluated with different concentrations: 9.08x10⁸, 6.86x10⁸, 4.64x10⁸, 2.42x10⁸ and 2.05x10⁷ OBs/ml.

The number of occlusion bodies produced per larva did not differ significantly between treatments (F=0.283, d.f.=4, p=0.886; where F is Fisher's F statistic, used to compare two or more independent groups following an analysis of variance (ANOVA), as results were distributed normally) (Fig. 10). However, for a given number of larvae inoculated with a low concentration of virus, the total quantity of occlusion bodies was reduced because a greater number of larvae did not die from infection, and as such, did not produce occlusion bodies. Consequently, the concentration of 9.08x10⁸ OBs/ml was chosen because it results in the greatest total number of occlusion bodies.

### 4.5. Effect of larval density on occlusion body production.

An experiment was performed to determine the optimal density of larvae. Groups of 1, 25, 50, 100, 150 and 200 larvae were placed in each 1.5 litre container. L6+24 larvae were first inoculated with an LC₉₀ and then transferred to the containers. Distinct parameters were measured, specifically final weight, cannibalism and the total number of occlusion bodies produced in each container.

The weight of larvae did not differ significantly between larval densities (F=1.003, d.f.=5, 54, p=0.425, where F is Fisher's F statistic calculated following ANOVA). Therefore larval density did not significantly affect final larval weight.

The percentage of cannibalism was determined at different larval densities. Cannibalism was more prevalent at higher larval densities. Cannibalism was lowest at the density of 25 larvae/container. The percentage of cannibalism did not differ significantly with density in the treatments involving 50, 100 or 150 larvae per container (χ²=0.859, d.f.=2, p=0.651; following a contingency table analysis) (Fig. 11).

The final production of occlusion bodies per container was determined and significant differences were detected between the different treatments (χ²=247.69; d.f.=5; p<0.001, following Kruskal Wallis test) (Fig. 12). The most productive containers were those that contained 150 and 200 larvae, which did not differ significantly from one another (Mann Whitney U=45, p=0.089, where the Mann Whitney U statistic is obtained by comparing two independent populations). The container of 100 larvas was significantly less productive than the 150 or 200 larvae containers (Mann Whitney U, p<0,05). Finally, the containers of 25 and 50 larvae produced similar numbers of occlusion bodies (Mann Whitney U =27, p=0.089), but were significantly less productive than the containers with higher densities of larvae (Mann Whitney U, p<0.05).

As such, the inoculation of 150 larvae/container results in an average production of 8.07x10¹³ occlusion bodies, similar to the number of occlusion bodies obtained by inoculating 200 larvae/container (8.43x10¹³ occlusion bodies). However, the cost of setting up and starting each container is higher if an additional 50 larvae are included. In contrast, the total production of occlusion bodies would be similar to that when rearing 150 larvae separately, but for mass-production, gregarious rearing is not only easier to manage but also less costly.

Based on these results, the preferred method for mass production of ChchSNPV occlusion bodies involves the inoculation of L6+24 stage *C. chalcites* larvae with a concentration of 9.08x10⁸ occlusion bodies/ml followed by rearing at a density of 150 larvae in a 1.5 litre container (preferably of plastic).

### - Example 5: Studies on the efficacy of ChchSNPV for the control of C. chalcites on plants treated under laboratory conditions.

These studies were performed using tomato plants treated with occlusion bodies and maintained under laboratory conditions. Tomato plants were treated by spraying an aqueous suspension of ChchSNPV at different concentrations (1x10⁶, 5x10⁵, 1x10⁵, 5x10⁴, 1x10⁴ and 2x10³ OBs/ml) and the agricultural wetting agent Agral® (Syngenta), at a concentration of 0.2%. Control plants were treated with a solution containing water and 0.2% Agral®, but without occlusion bodies. Once treated, plants were placed in glass chambers of 10 liters in volume and were infected with groups of 200 second-stage *C*. *chalcites* larvae (L2).

The efficacy of each treatment was determined by quantifying the percentage of mortality of larvae. For this, 25 larva were collected from each of the treatments at day 0 (prior to treatment), and were also collected at 2 hours post-treatment and at 2, 5 and 7 days following the treatment. These larvae were individually placed in cups containing artificial diet and larval mortality was recorded daily. The results of this assay are shown in Fig. 13.

Virus mortality was not observed in larvae collected at day 0 prior to treatment, which indicates that tomato plants were not contamined with occlusion bodies prior to the experiment. From the results obtained it is clear that increasing concentrations of occlusion bodies, and increasing the period during which larvae were exposed to contaminated plants, resulted in an increase in the percentage of larval mortality (Fig. 13).

The concentration of 5x10⁴ OBs/ml (5x10¹⁰ OBs/Ha) was the minimum concentration that produced mortalities of around 100% in larvae collected between the fifth and seventh day after the start of the experiment. Lower concentrations resulted in lower percentages of mortality. As such, the concentration of 5x10⁴ OBs/ml (5x10¹⁰ OBs/Ha) was selected as being optimal for control of *C. chalcites* larvae on tomato crops.

### - Example 6: Efficacy of ChchSNPV for the control of C. chalcites on banana crops in the Canary Islands

Field trials were performed in banana crops planted in the Canary Islands. Based on the results obtained in the laboratory studies, and knowning that to achive a similar level of mortality, field applications invariably involve higher concentrations than that used in the laboratory, ChchSNPV applications were performed at two concentrations: 1x10⁵ OBs/ml (1x10¹¹ OBs/Ha) and 1x10⁶ OBs/ml (1x10¹² OBs/Ha). The efficacy of the virus treatments were compared with that of the following treatments:
- a chemical insecticide used to control lepidopteran larvae (Steward®, DuPont™, that contains 30% weight/weight indoxacarb as the active ingredient), at the rate of application recomended by the manufacturer (http://www2.dupont.com/Crop Protection/es ES/assets/downloads/pdfs/Ste ward Horticolas int.pdf). For treatment of banana plants, the recommended rate of 0.04 g active ingredient/litre was applied (Perera y Molina, 2007); and
- a biological insecticide based on the bacterial entomopathogen *Bacillus thuringiensis* var. kurstaki (Novo-Biobit®, Cequisa-Agroindustrial, Barcelona, Spain, that contains 16% *Bacillus thuringiensis* in the form of a wettable powder [WP], that is frequently used in banana crops (see product technical information at http://www.guiafitos.com/ficha_formulado/4408). This product is commonly used at a rate of 0.25-0.5 Kg/Ha (0.25-0.5 g/litre) (Perera y Molina, 2007) and was applied at a concentration of 0.5 g/litre in the present example.

A 0.2% Agral solution in water without occlusion bodies was applied as a control. Treatments were applied by spraying aqueous suspensions or solutions. Each experimental plot was sprayed with 2 liters of each treatment directed at the upper and lower surfaces of the plant using a conventional sprayer.

The experimental design consisted on eight blocks with five treatments per block randomly distributed plots within each block. Each treatment was applied to a total of 28 banana plants per plot, 10 in a central position and 18 around the edges. The 10 central plants were examined to determine the initial and final levels of defoliation (number of sites of feeding damage) and the initial and final numbers of larvae or those that survived each of the treatments. Larvae were collected from the plants at the edge of the plot to determine treatment-induced mortalities.

Analysis of the survival of larvae on the 10 central plants was determined by counting the number of larvae immediately prior to treatment and at 10 days post-treatment. The percentage of larvae that survived differed significantly between treatments (χ²=218.241; d.f.=4; p<0.001; following contingency table analysis) (Fig. 14). Larval survival was highest in the control treatment (73%), which differed significantly from the remaining treatments. The percentage of survival at 10 days post-treatment in the plots treated with Steward, Bt or ChchSNPV at low dose (NPV1), was 25, 29 and 23%, respectively, and survival in these treatments did not differ significantly from one another (χ²=6.004; d.f.=2; p=0.05, following contingency table analysis). In contrast, survival of larvae was significantly lower in in plots treated with ChchSNPV at high dose (NPV2) in which just 9% of larvae survived to 10 days post-treatment.

As such, ChchSNPV applied at a rate of 1x10¹¹ OBs/Ha was as efective as the chemical insecticide treatment or the biological insecticide that is commonly used. In contrast, the application of 1x10¹² OBs/Ha was approximately five-fold more effective than chemical or Bt-based insecticides. This dose is equivalent or inferior to that of other commercial baculovirus-based insecticides.

An analysis of the incidence of defoliation was performed by counting the initial and final number of sites of insect feeding damage on the leaves. The prevalence of accumulated damage differed significantly between the different treatments (χ²=64.824; d.f.=4; p<0.001, following contingency table analysis) (Fig. 15). The higest prevalence of new damage was observed in the control treatment (91%), which differed significantly from the remaining treatments. In plots treated with Steward, Bt or ChchSNPV at a rate of 1x10¹¹ OBs/Ha (NPV1, low dose), the prevalence of new damage was 59, 51 and 48%, respectively and this did not differ significantly among these treatments (χ²=1.828, d.f.=2, p=0.401; following contingency table analysis). In contrast plants treated with ChchSNPV at a rate of 1x10¹² OBs/Ha (NPV2, high dose) had the lowest prevalence of new feeding damage (15%) that was significantly lower than observed in the other treatments. It can therefore be concluded that the baculovirus (ChchSNPV) applied at a dose of 1x10¹¹ OBs/Ha was as effective at protecting banana plants as a chemical insecticide or biological insecticide based on Bt. In contrast, at the dose of 1x10¹² OBs/Ha the virus was approximately four times more effective than either the chemical insecticide or Bt based insecticide.

Finally, the rate of acquisition of a lethal dose of virus by *C. chalcites* larvae was determined. For this, larvae were collected from plants in treated plots at 0, 1, 2, 3, 5 and 7 days post-treatment. These larvae were placed individually in cups with diet and mortality was registered daily during a 10 day period.

The percentage of mortality differed significantly between treatments and at different collection times (χ²=130.418, d.f.=19, p<0.001; following contingency table analysis) (Fig. 16). On day 0, prior to application of the treatments, the percentage of mortality was 0% in all treatments (information not shown in Fig. 16). Between 1% and 2% mortality was observed in larvae from control plants on the different days of collection, probably due to a very low level of contamination by virus from other treatments. At 1 day post-application the percentage of mortality in other treatments varied from 22% in the Bt based insecticide plots, to 86% in the ChchSNPV plots treated with 1x10¹² OBs/Ha (NPV2). The percentages of mortality in larvae collected from Bt and chemical insecticide treated plots were similar, reaching a maximum of 32% at 5 days post-application and falling to 13% at 7 days post-application. In contrast, the percentages of mortality in larvae from plants treated with ChchSNPV, at both low dose (NPV1) and high dose (NPV2) were significantly higher than observed in the chemical or Bt based insecticide treatments (Mann Whitney U, p<0.05).

In the light of these results, it can be concluded that the application of ChchSNPV treatments at a concentration of 1x10⁶ OBs/ml protects banana plants from *C. chalcites* pest infestations and that the virus is more effective than the chemical insecticide or *B. thuringiensis* based biological insecticide that is currently used to control this pest in this crop.

### Biological material deposits

The novel genotypes ChchTF1-A (ChchNPV-TF1-A), ChchTF1-B (ChchNPV-TF1-B) and ChchTF1-C (ChchNPV-TF1-C) were deposited in the Collection Nationale de Cultures de Microorganismes (CNCM), Institut Pasteur, 25 Rue du Docteur Roux, F-75724 Paris Cedex 15, France, following the rules of the Treaty of Budapest. The deposit reference (accession) numbers and the dates of the deposits were as follows:

| Genotype | Deposit number | Deposit date |
|---|---|---|
| ChchNPV-TF1-A | CNCM I-4690 | 5 November 2012 |
| ChchNPV-TF1-B | CNCM I-4620 | 27 April 2012 |
| ChchNPV-TF1-C | CNCM I-4621 | 27 April 2012 |

All three genotypes were deposited by one of the inventors, Prof. Dr. Primitivo Caballero (Instituto de Agrobiotecnología y Recursos Naturales, Universidad Pública de Navarra, Campus de Arrosadía, Mutilva Baja, E-31006, Pamplona, Navarra, Spain), as employee of the first applicant, for and on behalf of the four applicants (Universidad Pública de Navarra, Consejo Superior de Investigaciones Científicas, Instituto Canario de Investigaciones Agrarias (ICIA), Instituto de Ecología A.C.).

### References

Benjamini, Y., Hochberg, Y., 1995. Controlling the false discovery rate: a practical and powerful approach to multiple testing. Journal of the Royal Statistical Society Series B 57, 289-300.
Caballero, P., Williams, T., López-Ferber, M., 2001. Estructura y clasificación de los baculovirus, pp. 15-46. En: Caballero, P., Williams, T., López-Ferber, M. (Eds.). Los baculovirus y sus aplicaciones como bioinsecticidas en el control biológico de plagas. Phytoma-España, Valencia, España.
Caballero, P., Zuidema, D., Santiago-Alvarez, C., Vlak, J.M., 1992. Biochemical and biological characterization of four isolates of Spodoptera exigua nuclear polyhedrosis virus. Biocontrol Science and Technology, 2, 145-157.
Cabello, T., Belda, J., 1994. Noctuidos plaga (Lepidoptera: Nuctuidae) en cultivos hortícolas de invernadero, pp. 179-211. En: Moreno, R. (Ed.). Sanidad vegetal en la horticultura protegida. Consejería de Agricultura y Pesca, Junta de Andalucía, Sevilla.
Cabello, T., Rodriguez, H., Vargas, P., 1984. Utilización de una dieta artificial simple en la cría de Heliothis armigera (Hübner), Spodoptera littoralis (Boisduval) y Trigonophor meticulosa (Hübner) (Lepidoptera: Noctuidae). Anales INIA, Serie Agrícola 27, 101-107.
Cory, J.S., Green, B.M., Paul, R.K., Hunter-Fujita, F., 2005. Genotypic and phenotypic diversity of a baculovirus population within an individual insect host. Journal of Invertebrate Pathology 89, 101-111.
Crawley, M.J., 1993. GLIM for ecologists. Blackwell Science, Oxford, UK.
Erlandson, M.A., Newhouse, S., Moore, K., Janmaat, A., Myers, J., Theilmann, D., 2007. Characterization of baculovirus isolates from Trichoplusia ni populations from vegatable greenhouses. Biological Control 41, 256-263.
Erlandson, M.A., 2009. Genetic variation in field population of baculoviruses: Mechanism for generating variation and its potential role in baculovirus epizootiology. Virológica Sinica 24, 458-469.
Figueiredo, E., Muñoz, D., Escribano, A, Mexía, A, Vlak J.M., Caballero P., 1999. Biochemical identification and comparative insecticidal activity of nucleopolyhedrovirus isolates pathogenic ofr Heliothis armigera larvae. Journal of Applied Entomology 123, 165-169.
Gelernter, W.D., Federici, B.A., 1990. Virus epizootics in Californian populations of Spodoptera exigua: dominance of a single viral genotype. Biochemical Systematcis and Ecology 18, 461-466.
Granados, R., Fu, Y., Corsaro, B., Hughes, P., 2001. Enhancement of Bacillus thuringiensis toxicity to lepidopterous species with the enhancin from Trichoplusia ni granulovirus. Biological Control 20, 153-159.
Hara, K., Funakoshi, M., Kawarabata, T., 1995. In vivo and in vitro characterization of several isolates of Spodoptera exigua nuclear polyhedrosis virus. Acta Virologica 39, 215-222.
Harrison, R.L., Bonning, B.C., 1999. The nucleopoliedrovirus of Rachoplusia ou and Anagrapha falcifera are isolates of the same virus. Journal of General Virology 80, 2793-2798.
Harrison, R.L., Popham, H.J.R., Breitenbach, J.E., Rowley, D.L., 2012. Genetic variation and virulence of Autographa californica multiple nucleopolyhedrovirus and Trichoplusia ni single nucleopolyhedrovirus isolates. Journal of Invertebrate Pathology 110, 33-47.
Horowitz, A.R., Weintraub, P.G., Ishaaya, I., 1998. Status of pesticide resistance in arthropod pests in Israel. Phytoparasitica 26, 231-240.
Hughes, P.R., Wood, H.A., 1981. A synchronous per oral technique for the bioassay of insect viruses. Journal of Invertebrate Pathology 37, 154-159.
IJkel, W.F., van Strien, E.A., Heldens, J.G., Broer, R., Zuidema, D., Goldbach, R.W., Vlak, J. M., 1999. Sequence and organization of the Spodoptera exigua multicapsid nucleopolyhedrovirus genome. Journal of General Virology 80, 3289-3304.
Jehle, J.A., Blissard, G.W., Bonning, B.C., Cory, J.S., Herniou, E.A., Rohrmann, G.F., Theilmann, D.A., Thiem, S.M., Vlak, J.M., 2006. On the classification and nomenclature of baculoviruses: a proposal for revision. Archives of Virology 151, 1257-1266.
Kalia, V., Chaudhari, S., Gujar, G., 2001. Optimization of production of nucleopolyhedrovirus of Helicoverpa armigera throughout larval stages. Phytoparasitica 29, 23-28.
King, L.A., Possee, R.D., 1992. The baculovirus expression system. A laboratory guide. Chapman & Hall, London.
Lasa R., Ruiz-Portero, C., Alcázar, D., Belda, J.E., Caballero, P., Williams, T., 2007. Efficacy of optical brightener formulations of Spodoptera exigua multiple nucleopolyhedrovirus (SeMNPV) as a biological insecticide in greenhouses in southern Spain. Biological Control 40, 89-96.
LeOra-Software, 1987. POLO-PC a user's guide to Probit or Logit analysis. Berkeley, CA.
Li, L., Donly, C., Li, Q., Willis, L.G., Keddie, B.A., Erlandson, M.A, Theilmann, D.A. 2002a. Identification and genomic analysis of a second species of nucleopolydrovirus isolated from Mamestra configurata. Virology 297, 226-244.
Li, Q., Donly, C., Li, L., Willis, L.G., Theilmann, D.A. & Erlandson, M., 2002b. Sequence and organization of the Mamestra configurata nucleopolydrovirus genome. Virology 294, 106-121.
López-Ferber, M., Simón, O., Williams, T., Caballero, P., 2003. Defective or effective? Mutualistic interactions between virus genotypes. Proceedings of the Royal Society of London B 270, 2249-2255.
Moscardi, F., 1999. Assessment of the application of baculoviruses for control of Lepidoptera. Annual Review of Entomology 44, 257-289.
Muñoz, D., Castillejo, J.I., Caballero, P., 1998. Naturally occurring deletion mutants are parasitic genotypes in a wild-type nucleopolyhedrovirus population of Spodoptera exigua. Applied and Environmental Microbiology 64, 4372-4377.
Muñoz, D, Martinez, A.M., Murillo, R., Ruiz de Escudero, I., Vilaplana, L., 2001. Técnicas básicas para la caracterización de baculovirus, pp. 479-518. En: Caballero, P., Williams, T., López-Ferber, M. (Eds.). Los baculovirus y sus aplicaciones como bioinsecticidas en el control biológico de plagas. Phytoma-España, Valencia, España.
Murillo, R, Jerzy, J.L. Muñoz D, Amate, J., Barranco, P., Cabello, T., Caballero, P., 2000. Caracterización bioquímica de un nucleopoliedrovirus de Chrysodeixis chalcites autóctono de España. Bol. San. Veg. Plagas, 26, 637-644
Perera, S., Molina, M.J., 2007. Plagas y enfermedades en el cultivo ecológico de la platanera, pp. 77-118. En: Nogueroles, C., Líbano, J. (Eds.). El cultivo ecológico de la platanera en Canarias. Gabinete de Proyectos Agroecológicos S.L. Tenerife.
Roh, J.Y., Choi, J.Y., Li, M.S., Jin, B.R., Je, Y.H., 2007. Bacillus thuringiensis as a specific, safe, and effective tool for insect pest control. J. Microbial. Biotechnol. 4, 547-559.
Simón, O., Williams, T., López-Ferber, M., Caballero, P., 2004. Genetic structure of a Spodoptera frugiperda nucleopolyhedrovirus population: High prevalence of deletion genotypes. Applied and Environmental Microbiology 70, 5579-5588.
Simón, O., Williams, T., López-Ferber, M., Caballero, P., 2005. Functional importance of deletion mutant genotypes in an insect nucleopolyhedrovirus population. Applied and Environmental Microbiology 71, 4254-4262.
van Oers, M.M., Herniou, E.A., Usmany, M., Messelink, G.J., Vlak, J.M., 2004. Identification and characterization of a DNA photolyase-containing baculovirus from Chrysodeixis chalcites. Virology 330, 460-470.
van Oers, M.M., Abma-Henkens, M.H., Herniou, E.A., de Groot, J.C., Peters, S., Vlak, J.M., 2005. Genome sequence of Chrysodeixis chalcites nucleopolyhedrovirus, a baculovirus with two DNA photolyase genes. Journal of General Virology 86, 2069-2080.

## Claims

1. A *Chrysodeixis chalcites* single nucleopolyhedrovirus (ChchSNPV), **characterized in that** it is selected from the group of:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype **characterized by** a genome as depicted in SEQ ID NO: 17 (ChchTF1-A), SEQ ID NO: 18 (ChchTF1-B) or SEQ ID NO: 19 (ChchTF1-C).

2. A nucleopolyhedrovirus according to Claim 1, that is in the form of:
a) a complete virus particle (virion)
b) an occlusion body.

3. An occlusion body that contains various virions in which at least one of the virions is a virion of *Chrysoideixis chalcites* single nucleopolyhedrovirus selected from the group of:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype **characterized by** a genome as depicted in SEQ ID NO: 17 (ChchTF1-A), SEQ ID NO: 18 (ChchTF1-B) or SEQ ID NO: 19 (ChchTF1-C).

4. An occlusion body according to Claim 3, that
a) contains virions comprising the genome of different genotypes; or
b) contains virions comprising the genome of a single genotype **characterized by** a genome as depicted in SEQ ID NO: 17 (ChchTF1-A), SEQ ID NO: 18 (ChchTF1-B) or SEQ ID NO: 19 (ChchTF1-C)..

5. An occlusion body according to any one of the Claims 3 or 4 that contains virions in which the genome of at least one of the virions comprises a DNA fragment whose sequence is represented, over the entire length, by:
a) SEQ ID NO:5, SEQ ID NO:6, or SEQ ID NO:7;
b) SEQ ID NO:8, SEQ ID NO:9 or SEQ ID NO:10;
c) SEQ ID NO:11, SEQ ID NO:12 or SEQ ID NO:13; or
d) SEQ ID NO:14, SEQ ID NO:15 or SEQ ID NO:16.

6. A composition that comprises at least a nucleopolyhedrovirus as claimed in any one of Claims 1 or 2, or at least one occlusion body as claimed in any one of Claims 3 to 5.

7. A composition according to Claim 6, that comprises occlusion bodies as claimed in any one of Claims 3 to 5 that contain co-occluded virions, and in which the virions that are co-occluded in the same nucleopolyhedrovirus have the same genotype or different genotypes.

8. A composition according to any one of Claims 6 or 7, which comprises a mixture of virions
of:
i) ChchTF1-A (CNCM deposit reference number CNCM I-4690) and ChchTF1-B (CNCM deposit reference number CNCM I-4620),
ii) ChchTF1-A (CNCM deposit reference number CNCM I-4690) and ChchTF1-C (CNCM deposit reference number CNCM I-4621),
iii) ChchTF1-B (CNCM deposit reference number CNCM I-4620) and ChchTF1-C (CNCM deposit reference number CNCM I-4621), or
iv) ChchTF1-A (CNCM deposit reference number CNCM I-4690), ChchTF1-B (CNCM deposit reference number CNCM I-4620) and ChchTF1-C (CNCM deposit reference number CNCM I-4621),
or
of the genotypes comprising the genomes as depicted in:
v) SEQ ID NO: 17 (ChchTF1-A) and SEQ ID NO: 18 (ChchTF1-B),
vi) SEQ ID NO: 17 (ChchTF1-A) and SEQ ID NO: 19 (ChchTF1-C),
vii) SEQ ID NO: 18 (ChchTF1-B) and SEQ ID NO: 19 (ChchTF1-C), or
viii) SEQ ID NO: 17 (ChchTF1-A), SEQ ID NO: 18 (ChchTF1-B) or SEQ ID NO: 19 (ChchTF1-C).

9. A composition according to Claim 8, wherein the genomes of ChchTF1-A, ChchTF1-B and ChchTF1-C are present in the ratio ChchTF1-A : ChchTF1-B : ChchTF1-C 36:26:14, respectively.

10. A composition according to any one of Claims 6 to 9, that additionally contains an excipient, adjuvant or vehicle appropriate to use in the agricultural sector.

11. A procedure for the production of occlusion bodies as claimed in any one of the Claims 3 to 5, that comprises a step in which *Chysodeixis chalcites* larvae feed on artificial diet containing occlusion bodies according to claims 3 to 5.

12. A procedure according to Claim 11, wherein *C. chalcites* larvae are larvae of the sixth stage or instar.

13. A method for identifying the presence of *C. chalcites* single nucleopolyhedrovirus selected from the group of:
i) the ChchSNPV deposited in the Collection Nationale de Cultures de Microorganismes (CNCM) with deposit reference numbers CNCM I-4690 (ChchTF1-A), CNCM I-4620 (ChchTF1-B), CNCM I-4621 (ChchTF1-C), or
ii) ChchSNPV having a genotype **characterized by** a genome as depicted in SEQ ID NO: 17 (ChchTF1-A), SEQ ID NO: 18 (ChchTF1-B) or SEQ ID NO: 19 (ChchTF1-C)
in a sample, comprising the following steps:
a) amplifying by PCR the DNA extracted from the sample, using a pair of primers selected from those formed by:
i) SEQ ID NO:1 (F-Hoar) and SEQ ID NO:2 (R-Hoar), or
ii) SEQ ID NO:3 (F-Brod) and SEQ ID NO:4 (R-Brod);
b) analysing the amplified fragment to determine its size or sequence;
c) concluding that one of ChChSNPV ChchTF1-A, ChchTF1-B or ChchTF1-C is present if:
i) the fragment amplified by the primer pair SEQ ID NO:1 and SEQ ID NO:2 has:
a. a length of 756 (ChchTF1-A), 921 (ChchTF1-B) or 651 (ChchTF1-C) nucleotides respectively; or
b. the sequence represented by SEQ ID NO:5 (ChchTF1-A), SEQ ID NO:6 (ChchTF1-B) or SEQ ID NO:7 (ChchTF1-C), respectively or alternatively,
ii) the fragment amplified by the primer pair SEQ ID NO:3 and SEQ ID NO:4 has:
a. a length of 1743 (ChchTF1-A), 962 (ChchTF1-B) or 1725 (ChchTF1-C), nucleotides, respectively; or
b. the sequence represented by SEQ ID NO:8 (ChchTF1-A), SEQ ID NO:9 (ChchTF1-B) o SEQ ID NO:10 (ChchTF1-C), respectively.

14. The use of a composition as claimed in any one of Claims 6 to 10 as an insecticide to protect plants.

15. The use according to Claim 14, to control:
a) *C. chalcites* pests; and/or
b) pests of the family Noctuidae, specifically the species *Trichoplusia ni* and/or *Plusia gamma.*

16. The use according to any one of Claims 14 or 15, wherein the crop plant is tomato, pepper or banana.

## Patentansprüche

1. Single-Nucleopolyhedrovirus von *Chrysodeixis chalcites* (ChchSNPV), **dadurch gekennzeichnet, dass** es ausgewählt wird aus der Gruppe:
i) der in der Collection Nationale de Cultures de Microorganismes (CNCM, Nationale Sammlung von Mikroorganismenkulturen) unter den Hinterlegungsnummern CNCM I-4690 (ChchTF1-A), CNCM I-4620 (ChchTF1-B), CNCM I-4621 (ChchTF1-C) hinterlegten ChchSNPV, oder
ii) der ChchSNPV mit einem Genotyp, der durch ein Genom wie in SEQ ID NO: 17 (ChchTF1-A), SEQ ID NO: 18 (ChchTF1-B) oder SEQ ID NO: 19 (ChchTF1-C) dargestellt gekennzeichnet ist.

2. Nucleopolyhedrovirus nach Anspruch 1, das in Form:
a) eines vollständigen Viruspartikels (Virion),
b) eines Okklusionskörpers
vorliegt.

3. Okklusionskörper, der mehrere Virionen enthält und in dem mindestens eines der Virionen ein Virion des Single-Nucleopolyhedrovirus von *Chrysodeixis chalcites* ist, ausgewählt aus der Gruppe:
i) der in der Collection Nationale de Cultures de Microorganismes (CNCM, Nationale Sammlung von Mikroorganismenkulturen) unter den Hinterlegungsnummern CNCM I-4690 (ChchTF1-A), CNCM I-4620 (ChchTF1-B), CNCM I-4621 (ChchTF1-C) hinterlegten ChchSNPV, oder
ii) der ChchSNPV mit einem Genotyp, der durch ein Genom wie in SEQ ID NO: 17 (ChchTF1-A), SEQ ID NO: 18 (ChchTF1-B) oder SEQ ID NO: 19 (ChchTF1-C) dargestellt gekennzeichnet ist.

4. Okklusionskörper nach Anspruch 3, der
a) Virionen enthält, die das Genom verschiedener Genotypen umfassen; oder
b) Virionen enthält, die das Genom eines einzigen Genotyps umfassen, **gekennzeichnet durch** ein Genom wie in SEQ ID NO: 17 (ChchTF1-A), SEQ ID NO: 18 (ChchTF1-B) oder SEQ ID NO: 19 (ChchTF1-C) dargestellt.

5. Okklusionskörper nach einem der Ansprüche 3 oder 4, der Virionen enthält und in dem das Genom von mindestens einem der Virionen ein DNA-Fragment umfasst, dessen Sequenz über die gesamte Länge dargestellt ist durch:
a) SEQ ID NO:5, SEQ ID NO:6 oder SEQ ID NO:7;
b) SEQ ID NO:8, SEQ ID NO:9 oder SEQ ID NO:10;
c) SEQ ID NO:11, SEQ ID NO:12 oder SEQ ID NO:13; oder
d) SEQ ID NO:14, SEQ ID NO:15 oder SEQ ID NO:16.

6. Zusammensetzung, die mindestens ein Nucleopolyhedrovirus nach einem der Ansprüche 1 oder 2 oder mindestens einen Okklusionskörper nach einem der Ansprüche 3 bis 5 umfasst.

7. Zusammensetzung nach Anspruch 6, die Okklusionskörper nach einem der Ansprüche 3 bis 5 umfasst, die miteingeschlossene Virionen enthalten und in denen die im gleichen Nucleopolyhedrovirus mit eingeschlossenen Virionen denselben oder unterschiedliche Genotypen aufweisen.

8. Zusammensetzung nach einem der Ansprüche 6 oder 7, umfassend eine Virionen-Mischung aus:
i) ChchTF1-A (CNCM-Hinterlegungsnummer CNCM I-4690) und ChchTF1-B (CNCM-Hinterlegungsnummer CNCM I-4620),
ii) ChchTF1-A (CNCM-Hinterlegungsnummer CNCM I-4690) und ChchTF1-C (CNCM-Hinterlegungsnummer CNCM I-4621),
iii) ChchTF1-B (CNCM-Hinterlegungsnummer CNCM I-4620) und ChchTF1-C (CNCM-Hinterlegungsnummer CNCM I-4621) oder
iv) ChchTF1-A (CNCM-Hinterlegungsnummer CNCM I-4690), ChchTF1-B (CNCM-Hinterlegungsnummer CNCM I-4620) und ChchTF1-C (CNCM-Hinterlegungsnummer CNCM I-4621),
oder aus den Genotypen, die die in:
v) SEQ ID NO: 17 (ChchTF1-A) und SEQ ID NO: 18 (ChchTF1-B),
vi) SEQ ID NO: 17 (ChchTF1-A) und SEQ ID NO: 19 (ChchTF1-C),
vii) SEQ ID NO: 18 (ChchTF1-B) und SEQ ID NO: 19 (ChchTF1-C) oder
viii) SEQ ID NO: 17 (ChchTF1-A), SEQ ID NO: 18 (ChchTF1-B) oder SEQ ID NO:19 (ChchTF1-C)
dargestellten Genome umfassen.

9. Zusammensetzung nach Anspruch 8, wobei die Genome von ChchTF1-A, ChchTF1-B und ChchTF1-C jeweils in einem Verhältnis ChchTF1-A : ChchTF1-B : ChchTF1-C von 36:26:14 vorliegen.

10. Zusammensetzung nach einem der Ansprüche 6 bis 9, die zusätzlich einen für die Verwendung im Agrarsektor geeigneten Hilfsstoff, Adjuvant oder Träger enthält.

11. Verfahren zur Herstellung von Okklusionskörpern nach einem der Ansprüche 3 bis 5, das einen Schritt umfasst, in dem *Chrysodeixis*-*chalcites*-Larven mit Kunstnahrung, die Okklusionskörper nach den Ansprüchen 3 bis 5 enthält, gefüttert werden.

12. Verfahren nach Anspruch 11, wobei die *C.-chalcites-Larven* Larven im sechsten Larvenstadium sind.

13. Verfahren zum Nachweis der Anwesenheit von Single-Nucleopolyhedroviren von *C. chalcites,* ausgewählt aus der Gruppe:
i) der in der Collection Nationale de Cultures de Microorganismes (CNCM, Nationale Sammlung von Mikroorganismenkulturen) unter den Hinterlegungsnummern CNCM I-4690 (ChchTF1-A), CNCM I-4620 (ChchTF1-B), CNCM I-4621 (ChchTF1-C) hinterlegten ChchSNPV oder
ii) der ChchSNPV mit einem Genotyp, der durch ein Genom wie in SEQ ID NO: 17 (ChchTF1-A), SEQ ID NO: 18 (ChchTF1-B) oder SEQ ID NO: 19 (ChchTF1-C) dargestellt gekennzeichnet ist,
in einer Probe, das folgende Schritte umfasst:
a) Amplifikation der aus der Probe extrahierten DNA durch PCR unter Verwendung eines Primerpaars, das ausgewählt wird aus:
i) SEQ ID NO:1 (F-Hoar) und SEQ ID NO:2 (R-Hoar), oder
ii) SEQ ID NO:3 (F-Brod) und SEQ ID NO:4 (R-Brod);
b) Analyse des amplifizierten Fragments, um dessen Größe oder Sequenz zu bestimmen;
c) Schlussfolgern, dass eines der ChchSNPV ChchTF1-A, ChchTF1-B oder ChchTF1-C anwesend ist, wenn:
i) das mit dem Primerpaar SEQ ID NO:1 und SEQ ID NO:2 amplifizierte Fragment
a. eine Länge von jeweils 756 (ChchTF1-A), 921 (ChchTF1-B) oder 651 (ChchTF1-C) Nukleotiden aufweist; oder
b. die jeweils durch SEQ ID NO:5 (ChchTF1-A), SEQ ID NO:6 (ChchTF1-B) oder SEQ ID NO:7 (ChchTF1-C) gegebene Sequenz aufweist,
oder alternativ
ii) das mit dem Primerpaar SEQ ID NO:3 und SEQ ID NO:4 amplifizierte Fragment
a. eine Länge von jeweils 1743 (ChchTF1-A), 962 (ChchTF1-B) oder 1725 (ChchTF1-C) Nukleotiden aufweist; oder
b. die jeweils durch SEQ ID NO:8 (ChchTF1-A), SEQ ID NO:9 (ChchTF1-B) oder SEQ ID NO:10 (ChchTF1-C) gegebene Sequenz aufweist.

14. Verwendung einer Zusammensetzung nach einem der Ansprüche 6 bis 10 als Insektizid zum Pflanzenschutz.

15. Verwendung nach Anspruch 14 zur Bekämpfung von:
a) *C*.-*chalcites*-*Plagen*; und/oder
b) Plagen der Familie Noctuidae, insbesondere der Arten *Trichoplusia ni* und/oder *Plusia gamma.*

16. Verwendung nach einem der Ansprüche 14 oder 15, wobei die Nutzpflanze Tomate, Pfeffer oder Banane ist.

## Revendications

1. Un nucléopolyédrovirus à enveloppement simple de *Chrysodeixis chalcites* (ChchSNPV), **caractérisé en ce qu'**il est choisi dans le groupe composé de :
i) le ChchSNPV déposé à la Collection Nationale de Cultures de Microorganismes (CNCM) sous les numéros de référence de dépôt CNCM I-4690 (ChchTF1-A), CNCM I-4620 (ChchTF1-B), CNCM I-4621 (ChchTF1-C), ou
ii) ChchSNPV ayant un génotype **caractérisé par** un génome tel que représenté dans SEQ ID NO: 17 (ChchTF1-A), SEQ ID NO: 18 (ChchTF1-B) ou SEQ ID NO: 19 (ChchTF1-C).

2. Un nucléopolyédrovirus selon la revendication 1, qui se présente sous la forme de :
a) une particule virale complète (virion)
b) un corps d'occlusion.

3. Un corps d'occlusion qui contient plusieurs virions dans lequel au moins l'un des virions est un virion du nucléopolyédrovirus à enveloppement simple de *Chrysodeixis chalcites* choisi dans le groupe composé de :
i) le ChchSNPV déposé à la Collection Nationale de Cultures de Microorganismes (CNCM) sous les numéros de référence de dépôt CNCM I-4690 (ChchTF1-A), CNCM I-4620 (ChchTF1-B), CNCM I-4621 (ChchTF1-C), ou
ii) ChchSNPV ayant un génotype **caractérisé par** un génome tel que représenté dans SEQ ID NO: 17 (ChchTF1-A), SEQ ID NO: 18 (ChchTF1-B) ou SEQ ID NO: 19 (ChchTF1-C).

4. Un corps d'occlusion selon la revendication 3, qui
a) contient des virions comprenant le génome de différents génotypes ; ou
b) contient des virions comprenant le génome d'un unique génotype **caractérisé par** un génome tel que représenté dans SEQ ID NO: 17 (ChchTF1-A), SEQ ID NO: 18 (ChchTF1-B) ou SEQ ID NO: 19 (ChchTF1-C).

5. Un corps d'occlusion selon l'une quelconque des revendications 3 ou 4 qui contient des virions dans lequel le génome d'au moins l'un des virions comprend un fragment d'ADN dont la séquence est représentée, sur toute la longueur, par :
a) SEQ ID NO: 5, SEQ ID NO: 6, ou SEQ ID NO: 7 ;
b) SEQ ID NO: 8, SEQ ID NO: 9, ou SEQ ID NO: 10 ;
c) SEQ ID NO: 11, SEQ ID NO: 12, ou SEQ ID NO: 13 ; ou
d) SEQ ID NO: 14, SEQ ID NO: 15, ou SEQ ID NO: 16.

6. Une composition qui comprend au moins un nucléopolyédrovirus tel que revendiqué dans l'une quelconque des revendications 1 ou 2, ou au moins un corps d'occlusion tel que revendiqué dans l'une quelconque des revendications 3 à 5.

7. Une composition selon la revendication 6, qui comprend des corps d'occlusion tels que revendiqués dans l'une quelconque des revendications 3 à 5 qui contiennent des virions co-occlus, et dans laquelle les virions qui sont co-occlus dans le même nucléopolyédrovirus ont le même génotype ou des génotypes différents.

8. Une composition selon l'une quelconque des revendications 6 ou 7, qui comprend un mélange de virions de :
i) ChchTF1-A (numéro de référence de dépôt à la CNCM : CNCM I-4690) et ChchTF1-B (numéro de référence de dépôt à la CNCM : CNCM I-4620),
ii) ChchTF1-A (numéro de référence de dépôt à la CNCM : CNCM I-4690) et ChchTF1-C (numéro de référence de dépôt à la CNCM : CNCM I-4621),
iii) ChchTF1-B (numéro de référence de dépôt à la CNCM : CNCM I-4620) et ChchTF1-C (numéro de référence de dépôt à la CNCM : CNCM I-4621), ou
iv) ChchTF1-A (numéro de référence de dépôt à la CNCM : CNCM I-4690), ChchTF1-B (numéro de référence de dépôt à la CNCM : CNCM I-4620) et ChchTF1-C (numéro de référence de dépôt à la CNCM : CNCM I-4621),
ou des génotypes comprenant les génomes tels que représentés dans :
v) SEQ ID NO: 17 (ChchTF1-A) et SEQ ID NO: 18 (ChchTF1-B),
vi) SEQ ID NO: 17 (ChchTF1-A) et SEQ ID NO: 19 (ChchTF1-C),
vii) SEQ ID NO: 18 (ChchTF1-B) et SEQ ID NO: 19 (ChchTF1-C), ou
viii) SEQ ID NO: 17 (ChchTF1-A), SEQ ID NO: 18 (ChchTF1-B) ou SEQ ID NO: 19 (ChchTF1-C).

9. Une composition selon la revendication 8, dans laquelle les génomes de ChchTF1-A, ChchTF1-B et ChchTF1-C sont présents dans la proportion ChchTF1-A:ChchTF1-B:ChchTF1-C 36:26:14, respectivement.

10. Une composition selon l'une quelconque des revendications 6 à 9, qui contient en outre un excipient, adjuvant ou véhicule approprié à une utilisation dans le secteur agricole.

11. Une procédure pour la production de corps d'occlusion tels que revendiqués dans l'une quelconque des revendications 3 à 5, qui comprend une étape dans laquelle des larves de *Chrysodeixis chalcites* sont nourries avec un régime artificiel contenant des corps d'occlusion selon les revendications 3 à 5.

12. Une procédure selon la revendication 11, dans laquelle des larves de *C. chalcites* sont des larves du sixième stade ou instar.

13. Un procédé pour identifier la présence d'un nucléopolyédrovirus à enveloppement simple de *C. chalcites* choisi dans le groupe composé de :
i) le ChchSNPV déposé à la Collection Nationale de Cultures de Microorganismes (CNCM) sous les numéros de référence de dépôt CNCM I-4690 (ChchTF1-A), CNCM I-4620 (ChchTF1-B), CNCM I-4621 (ChchTF1-C), ou
ii) ChchSNPV ayant un génotype **caractérisé par** un génome tel que représenté dans SEQ ID NO: 17 (ChchTF1-A), SEQ ID NO: 18 (ChchTF1-B) ou SEQ ID NO: 19 (ChchTF1-C)
dans un échantillon, comprenant les étapes suivantes :
a) amplifier par PCR l'ADN extrait de l'échantillon, en utilisant une paire d'amorces sélectionnées parmi celles formées par :
i) SEQ ID NO: 1 (F-Hoar) et SEQ ID NO: 2 (R-Hoar), ou
ii) SEQ ID NO: 3 (F-Brod) et SEQ ID NO: 4 (R-Brod) ;
b) analyser le fragment amplifié pour déterminer sa taille ou séquence ;
c) conclure que l'un des ChchSNPV ChchTF1-A, ChchTF1-B ou ChchTF1-C est présent si :
i) le fragment amplifié par la paire d'amorces SEQ ID NO: 1 et SEQ ID NO: 2 présente :
a. une longueur de 756 (ChchTF1-A), 921 (ChchTF1-B) ou 651 (ChchTF1-C) nucléotides respectivement ; ou
b. la séquence représentée par SEQ ID NO: 5 (ChchTF1-A), SEQ ID NO: 6 (ChchTF1-B), ou SEQ ID NO: 7 (ChchTF1-C), respectivement ou bien,
ii) le fragment amplifié par la paire d'amorces SEQ ID NO: 3 et SEQ ID NO: 4 présente :
a. une longueur de 1743 (ChchTF1-A), 962 (ChchTF1-B) ou 1725 (ChchTF1-C) nucléotides, respectivement; ou
b. la séquence représentée par SEQ ID NO: 8 (ChchTF1-A), SEQ ID NO: 9 (ChchTF1-B) ou SEQ ID NO: 10 (ChchTF1-C), respectivement.

14. L'utilisation d'une composition telle que revendiquée dans l'une quelconque des revendications 6 à 10 comme insecticide pour protéger les plantes.

15. L'utilisation selon la revendication 14, pour contrôler:
a) les organismes nuisibles *C. chalcites* ; et/ou
b) les organismes nuisibles de la famille Nouctuidae, spécifiquement les espèces *Trichoplusia ni* et/ou *Plusia gamma.*

16. L'utilisation selon l'une quelconque des revendications 14 ou 15, dans laquelle la plante cultivée est la tomate, le poivron ou la banane.
